# FASCICULE DE BREVET EUROPEEN

(11) **EP 2 658 844 B1**
(45) Date de publication et mention de la délivrance du brevet: **26.10.2016**
(21) Numéro de dépôt: 11805849.4
(22) Date de dépôt: 22.12.2011
(51) Int. Cl.: C07D 209/30, C07D 403/06, C07D 401/14, C07D 403/04, C07D 413/04, C07D 413/14, C07D 471/10, C07D 493/10, A61K 31/513, A61P 35/00

(54) **NOUVEAUX DERIVES DE PYRIMIDINES, LEUR PREPARATION ET LEUR UTILISATION PHARMACEUTIQUE COMME INHIBITEURS DE PHOSPHORYLATION D'AKT(PKB)**
NEUE PYRIMIDINDERIVATE, IHRE HERSTELLUNG UND IHRE PHARMAZEUTISCHE VERWENDUNG ALS AKT (PKB)-PHOSPHORYLIERUNGSINHIBITOREN
NOVEL PYRIMIDINE DERIVATIVES, PREPARATION THEREOF, AND PHARMACEUTICAL USE THEREOF AS AKT(PKB) PHOSPHORYLATION INHIBITORS

(30) Priorité: 28.12.2010 FR 1061297; 28.12.2010 FR 1061298; 28.12.2010 FR 1061300; 28.12.2010 FR 1061301; 28.12.2010 FR 1061303; 14.10.2011 FR 1159313; 14.10.2011 FR 1159315; 14.10.2011 FR 1159316; 14.10.2011 FR 1159317
(43) Date de publication de la demande: 06.11.2013
(73) Titulaire: SANOFI, 75008 Paris (FR)
(72) Inventeur: BROLLO, Maurice, F-75013 Paris (FR); CARRY, Jean-Christophe, F-75013 Paris (FR); CERTAL, Victor, F-75013 Paris (FR); DIDIER, Eric, F-75013 Paris (FR); DOERFLINGER, Gilles, F-75013 Paris (FR); EL AHMAD, Youssef, F-75013 Paris (FR); FILOCHE-ROMME, Bruno, F-75013 Paris (FR); HALLEY, Frank, F-75013 Paris (FR); KARLSSON, Karl Andreas, F-75013 Paris (FR); SCHIO, Laurent, F-75013 Paris (FR); THOMPSON, Fabienne, F-75013 Paris (FR)
(74) Mandataire: Lavoix
(86) Numéro de dépôt international: PCT/EP2011/073875
(87) Numéro de publication internationale: WO 2012/089633

(56) Documents cités:
- WO-A1-2008/013322
- WO-A1-2010/056631
- WO-A1-2011/001114
- WO-A2-2008/148074
- US-A1- 2007 083 053
- QUN LI: "Recent progress in the discovery of Akt inhibitors as anticancer agents", EXPERT OPINION ON THERAPEUTIC PATENTS, INFORMA HEALTHCARE, GB, vol. 17, no. 9, 1 janvier 2007 (2007-01-01), pages 1077-1130, XP002500200, ISSN: 1354-3776, DOI: DOI:10.1517/13543776.17.9.1077
- AN T VU ET AL: "1-(Indolin-1-yl)-1-phenyl-3-propan-2-olam ines as Potent and Selective Norepinephrine Reuptake Inhibitors", JOURNAL OF MEDICINAL CHEMISTRY, AMERICAN CHEMICAL SOCIETY, US, vol. 53, no. 5, 1 mars 2010 (2010-03-01), pages 2051-2062, XP002652405, ISSN: 0022-2623, DOI: 10.1021/JM901559E [extrait le 2010-02-02]
- ZHAO HE ET AL: "Indoline and piperazine containing derivatives as a novel class of mixed D2/D4 receptor antagonists. Part 1: Identification and structure-activity relationships", BIOORGANIC & MEDICINAL CHEMISTRY LETTERS, PERGAMON, ELSEVIER SCIENCE, GB, vol. 12, no. 21, 1 janvier 2002 (2002-01-01), pages 3105-3109, XP002323244, ISSN: 0960-894X, DOI: 10.1016/S0960-894X(02)00655-8
- BATT D G ET AL: "Heteroatom- and carbon-linked biphenyl analogs of Brequinar as immunosuppressive agents", BIOORGANIC & MEDICINAL CHEMISTRY LETTERS, PERGAMON, ELSEVIER SCIENCE, GB, vol. 8, no. 13, 7 juillet 1998 (1998-07-07), pages 1745-1750, XP004137121, ISSN: 0960-894X, DOI: 10.1016/S0960-894X(98)00308-4
- PASQUIER C ET AL: "Synthesis and application in enantioselective hydrogenation of new free and chromium complexed aminophosphine-phosphinite ligands", TETRAHEDRON ASYMMETRY, PERGAMON PRESS LTD, OXFORD, GB, vol. 9, no. 2, 30 janvier 1998 (1998-01-30), pages 193-196, XP004131167, ISSN: 0957-4166, DOI: 10.1016/S0957-4166(97)00642-3

## Description

La présente invention concerne de nouveaux composés chimiques dérivés de pyrimidines, leur application à titre de médicaments, les compositions pharmaceutiques les renfermant et la nouvelle utilisation de tels dérivés.

La présente invention concerne ainsi également l'utilisation desdits dérivés pour la préparation d'un médicament destiné au traitement de l'homme.

Plus particulièrement, l'invention concerne, de nouveaux dérivés de pyrimidines et leur utilisation pharmaceutique pour la prévention et le traitement d'affections capables d'être modulées par l'inhibition de la voie PI3K/AKT/mTOR. AKT est un acteur clé dans cette voie de signalisation. Un niveau élevé de phosphorylation d'AKT est le marqueur de l'activation de la voie qui est retrouvée dans de nombreux cancers humains.

Les produits de la présente invention peuvent ainsi notamment être utilisés pour la prévention ou le traitement d'affections capables d'être modulées par l'inhibition de la phosphorylation d'AKT (P-AKT). L'inhibition de P-AKT peut être notamment obtenue par l'inhibition de la voie PI3K/AKT/mTOR, et en particulier par l'inhibition de kinases appartenant à cette voie comme les récepteurs à activité tyrosine kinase tels EGFR, IGFR, ErbB2, la 3'-phosphoinositide-dependent protein kinase-1 (PDK1), la phosphoinositide kinase PI3K, la serine-threonine kinase AKT, la kinase mTOR.

L'inhibition et la régulation de la voie PI3K/AKT/mTOR constitue notamment un nouveau et puissant mécanisme d'action pour le traitement d'un grand nombre de maladies cancéreuses incluant des tumeurs solides et liquides.

De telles affections que peuvent traiter les produits de la présente demande sont les tumeurs humaines solides ou liquides.

### Rôle de la voie PI3K/AKT/mTOR

La voie de signalisation PI3K/AKT/mTOR est un réseau complexe qui régule de multiples fonctions cellulaires, comme la croissance, la survie, la prolifération et la motilité cellulaire, qui sont des processus clés de la tumorigénèse.

Cette voie de signalisation est une cible importante dans le traitement du cancer car la plupart de ses effecteurs sont altérés dans les tumeurs humaines. Les principaux effecteurs contribuant à l'activation de la voie sont i) les oncogènes tels que ErbB1 (EGFR), ErbB2 (HER2), PIK3CA et AKT activés par mutation, amplification ou surexpression ; ii) la déficience des gènes suppresseurs de tumeurs tels que PTEN, TSC1/2, LKB et PML qui sont inactivés suite à des mutations ou à des délétions (Jiang L-Z & Liu L-Z, Biochim Biophys Acta, 2008, 1784 :150 ; Vivanco I & Sawyers CL, 2002, Nat Rev Cancer, 2 :489 ; Cully M et al., Nature Rev. Cancer, 2006, 6 :184).

L'activation des oncogènes de cette voie de signalisation est retrouvée dans de nombreuses maladies cancéreuses humaines.
- les mutations activatrices de PIK3CA sont présentes dans 15-30% des cancers du colon, du sein, de l'endomètre, du foie, de l'ovaire et de la prostate (TL Yuan and LC Cantley, Oncogene, 2008, 27:5497; Y. Samuels et al. Science, 2004, 304:554; KE. Bachman et al. Cancer Biol Ther, 2004, 3:772; DA Levine et al. Clin Canc Res. 2005, 11:2875; C. Hartmann et al. Acta Neuropathol. 2005, 109:639).
- les amplifications, mutations activatrices et surexpressions des RTKs tels qu'EGFR et HER2 dans les cancers du cerveau, du sein et du poumon (NSCLC)
- l'amplification et la surexpression activatrice d'AKT dans les cancers du cerveau, du poumon (NSCLC), du sein, du rein, de l'ovaire et du pancréas (Testa JR. and Bellacosa A., Proct. Natl. Acad. Sci. USA 2001, 98:10983 ; Cheng et al., Proct. Natl. Acad. Sci. USA 1992, 89: 9267 ; Bellacosa et al., Int. J. Cancer, 1995, 64:280 ; Cheng et al., Proct. Natl. Acad. Sci. USA 1996, 93 :3636 ; Yuan et al., Oncogene, 2000, 19 :2324).

La déficience des gènes suppresseurs de tumeurs de cette voie de signalisation est également retouvée dans de nombreuses maladies cancéreuses humaines:
∘ la délétion de *PTEN* dans 50% des cancers du poumon (NSCLC), du foie, du rein, de la prostate, du sein, du cerveau, du pancréas, de l'endomètre et du colon (Maxwell GL et al. Canc. Res. 1998, 58 :2500 ; Zhou X-P et al. Amer. J. Pathol., 2002, 161 :439 ; Endersby R & Baker SJ, Oncogene, 2008, 27 :5416 ; Li et al. Science, 1997, 275:1943; Steack PA et al., Nat. Genet., 1997, 15 :356)
o les mutations de *TSC1*/*2* dans plus de 50% des scléroses tubéreuses
o les mutations ou délétions de *LKB1* (or *STK11*) qui prédisposent aux cancers du tractus gastro-intestinal et au cancer du pancreas et qui sont retouvées en particulier dans 10-38% des adenocarcinomes du poumon (Shah U. et al. Cancer Res. 2008, 68 :3562)
∘ les modifications de *PML* notament par translocation dans les tumeurs humaines (Gurrieri C et al, J. NAtl Cancer Inst. 2004, 96 :269).

De plus cette voie de signalisation est un facteur majeur de résistance à la chimiothérapie, la radiothérapie et à des thérapies ciblées tels que les inhibiteurs d'EGFR et HER2 par exemple (C. Sawyers et al. Nat Rev 2002).

### Rôle d'AKT

AKT (protéine kinase B ; PKB) est une sérine-thréonine kinase qui occupe une place centrale dans une des voies majeures de signalisation cellulaire, la voie PI3K/AKT. AKT est notamment impliquée dans la croissance, la prolifération et la survie des cellules tumorales. L'activation d'AKT se fait en deux étapes (i) par phosphorylation de la thréonine 308 (P-T308) par PDK1 et (2) par phosphorylation de la sérine 473 (P-S473) par mTORC2 (ou complexe mTOR-Rictor), résultant en une activation totale. AKT à son tour régule un grand nombre de protéines dont mTOR (mammalian target of Rapamycin), BAD, GSK3, p21, p27, FOXO ou FKHRL1 (Manning BD & Cantley LC, Cell, 2007 129 :1261). L'activation d'AKT promeut l'internalisation des nutriments, ce qui déclenche un processus de métabolisation anabolisante soutenant la croissance et la prolifération cellulaire. En particulier, AKT contrôle l'initiation de la synthèse protéique à travers une cascade d'interactions qui procède par l'intermédiaire de TSC1/2 (complexe de sclérose tubéreuse), Rheb, et TOR pour aboutir à deux cibles critiques de la voie de signalisation, p70S6K et 4EBP. AKT induit également une phosphorylation inhibitrice du facteur de transcription Forkhead et l'inactivation de GSK3β qui conduisent à l'inhibition de l'apoptose et à la progression du cycle cellulaire (Franke TF, Oncogene, 2008, 27 :6473). AKT est donc une cible pour la thérapie anti-cancéreuse et l'inhibition de l'activation d'AKT par l'inhibition de sa phosphorylation peut induire l'apoptose des cellules malignes et par la même, présenter un traitement pour le cancer.

### Les récepteurs à activité tyrosine kinase comme IGF1 R

Des niveaux anormalement élevés d'activité protéine kinase ont été impliqués dans de nombreuses maladies résultant de fonctions cellulaires anormales. Ceci peut provenir soit directement soit indirectement, d'un disfonctionnement dans les mécanismes de contrôle de l'activité kinase, lié par exemple à une mutation, une sur-expression ou une activation inappropriée de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc). Le récepteur activé par son ligand induit une activité mitogènique dans les cellules normales. Cependant IGF-I-R joue un rôle important dans la croissance dite anormale.

Plusieurs rapports cliniques soulignent le rôle important de la voie IGF-I dans le développement des cancers humains :
IGF-I-R est souvent trouvé sur-exprimé dans de nombreux types tumoraux (sein, colon, poumon, sarcome, prostate, myelome multiple) et sa présence est souvent associée à un phénotype plus agressif.

De fortes concentrations d'IGF1 circulant sont fortement corrélées à un risque de cancer de la prostate, poumon et sein.

De plus, il a été largement documenté que IGF-I-R est nécessaire à l'établissement et au maintient du phénotype transformé in vitro comme in vivo [Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie, radiation, et l'apoptose induite par des cytokines. De plus, l'inhibition d'IGF-I-R endogène par un dominant négatif, la formation de triple hélice ou l'expression d'un antisens provoque une suppression de l'activité transformante in vitro et la diminution de la croissance de tumeurs dans les modèles animaux.

### PDK1

La 3'-phosphoinositide-dependent protein kinase-1 (PDK1) est une des composantes essentielles de la voie de signalisation PI3K-AKT. C'est une sérine-thréonine (Ser/Thr) kinase dont le rôle est de phosphoryler et d'activer d'autres Ser/Thr kinases de la famille des AGC impliquées dans le contrôle de la croissance, la prolifération, la survie cellulaire et dans la régulation du métabolisme. Ces kinases incluent la protéine kinase B (PKB ou AKT), SGK (ou serum and glucocorticoïd regulated kinase), RSK (ou p90 ribosomal S6 kinase), p70S6K (ou p70 ribosomal S6 kinase) ainsi que diverses isoformes de la protéine kinase C (PKC) (Vanhaesebroeck B. & Alessi DR., Biochem J, 2000, 346:561). Un des rôles clés de PDK1 est donc l'activation d'AKT : en présence de PIP3, le second messager généré par PI3K, PDK-1 est recruté à la membrane plasmique via son domaine PH (plekstrin homology) et phosphoryle AKT sur la thréonine 308 situé dans la boucle d'activation, une modification essentielle de l'activation d'AKT. PDK1 est exprimée de façon ubiquitaire et est une kinase constitutivement active. PDK1 est un élément clé dans la voie de signalisation PI3K/AKT pour la régulation de processus clés dans la tumorigénèse comme la prolifération et la survie cellulaire. Cette voie étant activée dans plus de 50% des cancers humains, PDK1 représente une cible pour la thérapie anticancéreuse. L'inhibition de PDK1 devrait résulter en une inhibition effective de la prolifération et de la survie des cellules cancéreuses et donc apporter un bénéfice thérapeutique pour les cancers humains (Bayascas JR, Cell cycle, 2008, 7 :2978 ; Peifer C. & Alessi DR, ChemMedChem, 2008, 3 :1810).

### Les phosphoinositides-3 kinases (PI3Ks)

La lipide kinase PI3K est une cible importante dans cette voie de signalisation pour l'oncologie. Les PI3Ks de la classe I sont réparties en classe la (PI3Kα,β,δ) activée par les récepteurs à activité tyrosine kinase (RTKs), les récepteurs couplés aux protéines G (GPCRs), les GTPases de la famille Rho, p21-Ras et en classe Ib (PI3Kγ) activé par les GPCRs et par p21-Ras. Les PI3Ks de la classe la sont des hétérodimères qui consistent en une sous unité catalytique p110α, β ou δ et une sous unité régulatrice p85 ou p55□ La classe Ib (p110γ) est monomérique. Les PI3Ks de la classe I sont des lipides/protéines kinases qui sont activées par les RTKs, les GPCRs ou Ras après recrutement à la membrane. Ces PI3Ks de la classe I phosphorylent le phosphatidylinositol 4,5 diphosphate (PIP2) sur la position 3 de l'inositol pour donner le phosphatidylinositol 3,4,5 triphosphate (PIP3), messager secondaire clé de cette voie de signalisation. A son tour, PIP3 recrute AKT et PDK1 à la membrane où ils se fixent par leur domaine homologue à la pleckstrine (domaine PH), conduisant à l'activation d'AKT par phosphorylation de PDK1 sur la thréonine 308. AKT phosphoryle de nombreux substrats, jouant ainsi un rôle clé dans de nombreux processus aboutissant à la transformation cellulaire comme la prolifération, la croissance et la survie cellulaire ainsi que l'angiogénèse.

Les PI3Ks de classe I sont impliquées dans les cancers humains : des mutations somatiques du gène PIK3CA qui code pour PI3Kαβ se retrouvent dans 15-35% des tumeurs humaines avec notamment deux mutations oncogéniques principales H1047R (dans le domaine kinase) et E545K/E542K (dans le domaine hélical) (Y. Samuels et al. Science, 2004, 304:554; TL Yuan and LC Cantley, Oncogene, 2008, 27:5497). Des inhibiteurs de PI3K sont attendus efficaces pour le traitement de nombreux cancers humains présentant des altérations génétiques aboutissant à l'activation de la voie PI3K/AKT/mTOR (Vogt P. et al., Virology, 2006, 344 :131 ; Zhao L & Vogt PK, Oncogene, 2008, 27 :5486).

Des dérivés Morpholino pyrimidinones inhibiteurs de kinases sont connus de l'homme de l'art.

L'application WO2008/148074 décrit des produits qui possèdent une activité inhibitrice de mTOR. Ces produits sont des pyrido[1,2-a]pyrimidin-4-ones qui diffèrent des produits de la présente invention en raison de leur caractère entièrement aromatique et de leurs substitutions.

L'application WO2008/064244 décrit l'application des produits TGX-221 et TGX-155 inhibiteurs de P13Kβ utiles dans le traitement du cancer et notamment dans le cancer du sein. Ces produits sont des pyrido[1,2-a]pyrimidin-4-ones précédemment décrits dans les applications WO2004/016607 et WO2001/053266 qui diffèrent des produits de la présente invention en raison de leur caractère entièrement aromatique et de leurs substitutions.

Les applications WO2006/109081, WO2006/109084 et WO2006/126010 décrivent des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

L'application WO2003/024949 décrit des produits inhibiteurs de DNA-PK utiles pour le traitement des cancers ATM déficients. Ces produits sont des pyrido[1,2-a]pyrimidin-4-ones qui diffèrent des produits de la présente invention en raison de leur caractère entièrement aromatique et de leurs substitutions

Des dérivés morpholino pyrimidine inhibiteurs de kinase sont également connus de l'homme de l'art.

Les applications WO2009/007748, WO2009/007749, WO2009/007750 et WO2009/007751 décrivent des produits qui possèdent une activité inhibitrice de mTOR et/ou de PI3K pour le traitement des cancers. Ces produits sont des pyrimidines substituées en 2, 4 et 6 et les produits de la présente invention en différent en raison de la présence du groupement carbonyle ou thiocarbonyle sur la pyrimidine ainsi que par les différent substituants.

WO 2008/148074 décrit des composés inhibiteurs de la protéine kinase mTOR de formules générales :

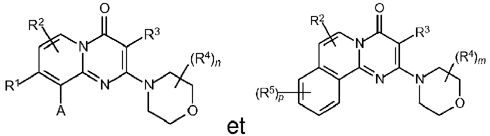

Des inhibiteurs de la protéine AKT sont décrits dans l'article « Recent progress in the discovery of AKT inhibitors as anticancer agents" (Expert Opin. Ther. Patents, Qun Li, (2007) 17(9) :1077-1130) tels que des dérivés de la staurosporine, des dérivés d'isoquinolines sulfonamides ou encore des pyridines disubstituées en positions 3 et 5.

WO 2011/001114 décrit des dérivés de (6-oxo-1,6-dihydro-pyrimidin-2-yl)-amide en tant qu'inhibiteurs de la voie PI3K/AKT/mTOR.

### Formules générales des composés selon l'invention:

La présente invention a pour objet des composés de formules (la), (Ib), (Ic), (Id) et (le).

### La présente invention a pour objet les produits de formule (Ia):

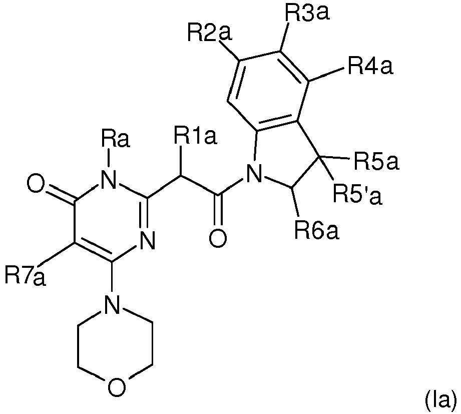

dans laquelle :
Ra représente un atome d'hydrogène ou un radical alkyle ;
R1 a représente un atome d'hydrogène ou un radical méthyle ;
R2a représente un atome d'hydrogène ou un atome de fluor ;
R3a représente un atome d'hydrogène ou un atome d'halogène ;
R4a représente un atome d'hydrogène, un atome d'halogène ou un radical hydroxyle, alkyle ou alcoxy, les radicaux alkyle étant éventuellement substitués par un ou plusieurs radicaux identiques ou différents choisis parmi les atomes d'halogène et le radical hydroxyle, les radicaux alcoxy étant éventuellement substitués par un ou plusieurs atomes d'halogène ;
R5a et R5'a, identiques ou différents, représente un atome d'hydrogène ou un radical alkyle ;
R6a représente un atome d'hydrogène ou un radical alkyle éventuellement substitué par un ou plusieurs radicaux identiques ou différents choisis parmi les atomes d'halogène et le radical hydroxyle ;
R7a représente un atome d'halogène ;
lesdits produits de formule (la) étant sous toutes les formes isomères possibles racémiques, énantiomères et diastéréoisomères, ainsi que les sels d'addition avec les acides minéraux et organiques ou avec les bases minérales et organiques desdits produits de formule (la).

La présente invention a ainsi pour objet les produits de formule (la) tels que :
Ra représente un atome d'hydrogène ou un radical méthyle ;
R1 a représente un atome d'hydrogène ou un radical méthyle ;
R2a représente un atome d'hydrogène ou un atome de fluor ;
R3a représente un atome d'hydrogène ou un atome de fluor ;
R4a représente un atome d'hydrogène, un atome d'halogène ou un radical alkyle éventuellement substitué par un ou plusieurs radicaux identiques ou différents choisis parmi les atomes d'halogène ;
R5a et R5'a représentent un atome d'hydrogène ;
R6a représente un atome d'hydrogène ou un radical alkyle éventuellement substitué par un ou plusieurs radicaux identiques ou différents choisis parmi les atomes de fluor et le radical hydroxyle;
R7a représente un atome de fluor, brome ou chlore ;
lesdits produits de formule (la) étant sous toutes les formes isomères possibles racémiques, énantiomères et diastéréoisomères, ainsi que les sels d'addition avec les acides minéraux et organiques ou avec les bases minérales et organiques desdits produits de formule (la).

Dans les produits de formule (la) tels que définis ci-dessus, R4a représente par exemple un atome d'hydrogène ou un atome d'halogène tel que fluor, chlore ou brome ou encore le radical CF3 , les autres substituants Ra, R1a, R2a, R3a, R5a, R5'a et R6a ayant l'une quelconque de leurs significations indiquées ci-dessus pour lesdits produits de formule (la).

Dans les produits de formule (la), R6a représente par exemple un atome d'hydrogène ou un radical méthyle éventuellement substitué par un atome de fluor ou le radical hydroxyle. les autres substituants Ra, R1 a , R2a, R3a, R4a, R5a et R5'a ayant l'une quelconque de leurs significations indiquées ci-dessus pour lesdits produits de formule (la).

### La présente invention a également pour objet les produits de formule (Ib):

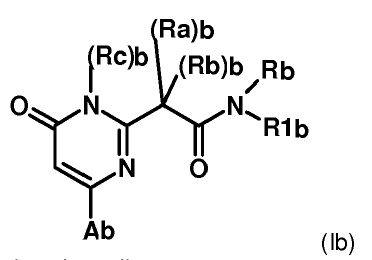

dans laquelle :
Ab représente un radical morpholine ou un radical pyridyle définis comme suit :
   le radical morpholine, que peut représenter Ab, étant substitué par un ou plusieurs radicaux choisis parmi l'atome de deutérium,et les radicaux alkyles eux-mêmes éventuellement substitués par un ou plusieurs radicaux choisis parmi les atomes d'halogène et le radical hydroxyle, étant entendu que deux substituants adjacents de la morpholine peuvent former ensemble un radical cyclique avec les atomes de carbone auxquels ils sont liés ;
   le radical pyridyle, que peut représenter Ab, étant éventuellement substitué par un atome d'halogène ou un radical alkyle ou alcoxy;
   R1b représente un radical aryle ou hétéroaryle éventuellement substitués par un ou plusieurs radicaux identiques ou différents choisis parmi les atomes d'halogène et les radicaux hydroxyle, CN, nitro, -COOH, -COOalk, -N(Rx)b(Ry)b, -CON(Rx)b(Ry)b, - N(Rx)bCO(Ry)b, -CO(Ry)b, -N(Rx)bCO₂(Rz)b, alcoxy, phénoxy, alkylthio, alkyle, alkényle, alkynyle, cycloalkyle, O-cycloalkyle, hétérocycloalkyle ; aryle et hétéroaryle;
   ces derniers radicaux alcoxy, phénoxy, alkylthio, alkyle, alkényle, alkynyle, hétérocycloalkyle, aryle et hétéroaryle étant eux-mêmes éventuellement substitués par un ou plusieurs radicaux identiques ou différents choisis parmi les atomes d'halogène et les radicaux hydroxyle, alcoxy et N(Rv)b(Rw)b ;
   les radicaux aryle et hétéroaryle étant de plus éventuellement substitués par un ou plusieurs radicaux alkyle et alcoxy eux-mêmes éventuellement substitués par un ou plusieurs atomes d'halogène ;
   les radicaux hétérocycloalkyle et hétéroaryle pouvant de plus renfermer un radical oxo,
   Rb représente un atome d'hydrogène ou bien forme avec R1 b un cycle
   à 5 ou 6 chaînons saturé ou partiellement ou totalement insaturé fusionné à un reste aryle ou hétéroaryle et renfermant éventuellement un ou plusieurs autres hétéroatomes choisi(s) parmi O, S, N, NH et Nalk, ce radical bicyclique étant éventuellement substitué par un ou plusieurs radicaux identiques ou différents choisis parmi les atomes d'halogène et les radicaux hydroxyle, alkyle et alcoxy;
   (Ra)b et (Rb)b, identiques ou différents représentent indépendamment un atome d'hydrogène, un atome d'halogène ou un radical alkyle éventuellement substitué par un ou plusieurs atomes d'halogène ;
   (Rc)b représente un atome d'hydrogène ou un radical alkyle éventuellement substitué par un ou plusieurs atomes d'halogène;
   N(Rx)b(Ry)b étant tel que (Rx)b représente un atome d'hydrogène ou un radical alkyle et (Ry)b représente un atome d'hydrogène, un radical cycloalkyle ou un radical alkyle éventuellement substitué par un ou plusieurs radicaux identiques ou différents choisis parmi les radicaux hydroxyle, alcoxy, N(Rv)b(Rw)b et hétérocycloalkyle ; soit (Rx)b et (Ry)b forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
   N(Rv)b(Rw)b étant tel que (Rv)b représente un atome d'hydrogène ou un radical alkyle et (Rw)b représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit (Rv)b et (Rw)b forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
   les radicaux cycliques que peuvent former (Rx)b et (Ry)b ou (Rv)b et (Rw)b respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
   (Rz)b représente les valeurs de (Ry)b à l'exception de hydrogène ;
   (Rx)b, (Ry)b et (Rz)b dans les radicaux N(Rx)bCO(Ry)b, -CO(Ry)b et N(Rx)bCO₂(Rz)b étant choisis parmi les significations indiquées ci-dessus pour (Rx)b, (Ry)b, et (Rz)b ;
   tous les radicaux alkyle (alk), alcoxy et alkylthio ci-dessus étant linéaires ou ramifiés et renfermant de 1 à 6 atomes de carbone,
   lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

On note particulièrement les produits de formule (Ib) tels que définis ci-dessus dans lesquels lorsque R1b représente un radical phényle et Ab représente un radical morpholine, alors R1 b ne porte pas comme substituant un radical pyrrole.

On note également particulièrement les produits de formule (Ib) tels que définis ci-dessus dans lesquels lorsque R1 b représente un radical phényle et Ab représente un radical pyridyle, alors R1b est forcément substitué par au moins un substituant tel que défini ci-dessus (cad R1b ne représente pas phényle non substitué) et lorsque R1b n'a qu'un seul substituant en position méta, alors ce substituant n'est pas l'atome de fluor.

### La formule générale suivante (1c) est décrite :

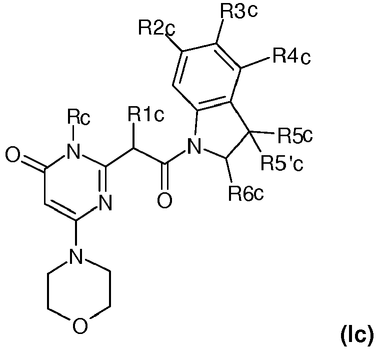

dans laquelle :
Rc représente un atome d'hydrogène ou un radical alkyle ;
R1c représente un atome d'hydrogène ou un radical méthyle ;
R2c représente un atome d'hydrogène ou un atome de fluor ;
R3c représente un atome d'hydrogène ou un atome d'halogène ;
R4c représente un atome d'hydrogène, un atome d'halogène ou un radical hydroxyle, alkyle ou alcoxy, les radicaux alkyle étant éventuellement substitués par un ou plusieurs radicaux identiques ou différents choisis parmi les atomes d'halogène et le radical hydroxyle, les radicaux alcoxy étant éventuellement substitués par un ou plusieurs atomes d'halogène ;
R5c et R5'c, identiques ou différents, représentent un atome d'hydrogène ou un radical alkyle;
R6c représente un atome d'hydrogène ou un radical alkyle éventuellement substitué par un ou plusieurs radicaux identiques ou différents choisis parmi les atomes d'halogène (F) et les radicaux hydroxyle et alcoxy ;
lesdits produits de formule (Ic) étant sous toutes les formes isomères possibles racémiques, énantiomères et diastéréoisomères, ainsi que les sels d'addition avec les acides minéraux et organiques ou avec les bases minérales et organiques desdits produits de formule (Ic).

La formule générale (Ic) décrite comprend des produitspour lesquels :
Rc représente un atome d'hydrogène ou un radical méthyle ;
R1c représente un atome d'hydrogène ;
R2c représente un atome d'hydrogène ou un atome de fluor ;
R3c représente un atome d'hydrogène ou un atome de fluor;
R4c représente un atome d'hydrogène, un atome de fluor, chlore ou brome ou un radical hydroxyle ;
R5c et R5'c, identiques ou différents, représente un atome d'hydrogène ou un radical méthyle ;
R6c représente un atome d'hydrogène ou un radical méthyle, éthyle ou isopropyle ;
lesdits produits de formule (Ic) étant sous toutes les formes isomères possibles racémiques, énantiomères et diastéréoisomères, ainsi que les sels d'addition avec les acides minéraux et organiques ou avec les bases minérales et organiques desdits produits de formule (Ic).

Les produits de formule (Ic) sont tels que :
- soit R4c ne représente pas F, Cl ou Br lorsque R2c, R3c, R5c et R6c représentent un atome d'hydrogène
- soit R6c ne représente pas méthyle lorsque R2c, R3c, R4c et R5c représentent un atome d'hydrogène.

### La formule générale suivante (Id) est décrite:

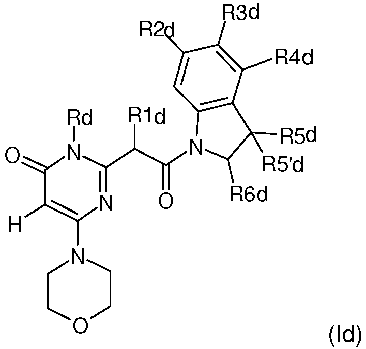

dans laquelle :
Rd représente un atome d'hydrogène ou un radical alkyle, cycloalkyle, aryle ou hétéroaryle, NH2, CN ;
R1 d représente un atome d'hydrogène ou un radical méthyle ;
R2d représente un atome d'hydrogène ou un atome de fluor ;
R3d représente un atome d'hydrogène ou un atome d'halogène ;
R4d représente un atome d'hydrogène, les atomes d'halogène et les radicaux hydroxyle, alkyle, alcoxy ; hétérocycloalkyle, aryle hétéroaryle et N(Rx)d(Ry)d ; tous ces radicaux alkyle, alcoxy, hétérocycloalkyle, aryle, hétéroaryle étant eux-mêmes éventuellement substitués par un ou plusieurs radicaux identiques ou différents choisis parmi les atomes d'halogène et les radicaux hydroxyle, oxo, alkyle, hétérocycloalkyle, alcoxy, N(Rv)d(Rw)d, et -SO2Alk;
R5d et R5'd, identiques ou différents, représente un atome d'hydrogène ou un radical alkyle ou forment ensemble avec l'atome de carbone auquels ils sont liés un radical cyclique renfermant de 3 à 10 chaînons (spirocycloalkyle) renfermant éventuellement un ou plusieurs hétéroatomes (spirohétérocycloalkyle); choisi(s) parmi O, S, NH, ces radicaux cycliques étant éventuellement substitués par un ou plusieurs radicaux identiques ou différents choisis parmi les atomes d'halogène, les radicaux alkyle, cycloalkyle, hydroxyle, oxo, alcoxy, NH2; NHalk et N(alk)2 ;
R6d représente un atome d'hydrogène ; un radical alkyle lui-même éventuellement substitué par un ou plusieurs radicaux identiques ou différents choisis parmi les atomes d'halogène, des atomes de deutérium et les radicaux hydroxyle et alcoxy ; un radical cycloalkyle ou un radical phényle lui-même éventuellement substitué par un ou plusieurs radicaux identiques ou différents choisis parmi les atomes d'halogène ou les radicaux alcoxy ;
R5d et R6d pouvant éventuellement former avec les atomes de carbone auquels ils sont liés un radical cyclique de 3 à 10 chaînons (cycloalkyle) renfermant éventuellement un ou plusieurs hétéroatomes (hétérocycloalkyle) choisi(s) parmi O, S, NH, ces radicaux cycliques étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
N(Rx)d(Ry)d étant tel que (Rx)d représente un atome d'hydrogène ou un radical alkyle et (Ry)d représente un atome d'hydrogène, un radical cycloalkyle ou un radical alkyle éventuellement substitué par un ou plusieurs radicaux identiques ou différents choisis parmi les radicaux hydroxyle, alcoxy, N(Rv)d(Rw)d et hétérocycloalkyle ; soit (Rx)d et (Ry)d forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué par un ou plusieurs radicaux identiques ou différents choisis parmi les atomes d'halogène et les radicaux hydroxyle, oxo, alkyle, hétérocycloalkyle, alcoxy, N(Rv)d(Rw)d et -SO2Alk;
N(Rv)d(Rw)d étant tel que (Rv)d représente un atome d'hydrogène ou un radical alkyle et (Rw)d représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit (Rv)d et (Rw)d forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué; par un ou plusieurs radicaux identiques ou différents choisis parmi les atomes d'halogène et les radicaux hydroxyle, oxo, alkyle, hétérocycloalkyle, alcoxy, NH2; NHalk et N(alk)2 et -SO2Alk;
lesdits produits de formule (Id) étant sous toutes les formes isomères possibles racémiques, énantiomères et diastéréoisomères, ainsi que les sels d'addition avec les acides minéraux et organiques ou avec les bases minérales et organiques desdits produits de formule (Id).

Les produits de formule (Id) sont tels que définis ci-dessus.

La formule générale (Id) dans laquelle Rd représente un atome d'hydrogène ou un radical alkyle, cycloalkyle, aryle ou hétéroaryle; et les autres substituents R1d, R2d, R3d, R4d, R5d, R5'd et R6d ont l'une quelconque des définitions indiquées ci-dessus ou ci-après pour lesdits produits de formule (Id) est décrite.

La formule générale (Id) dans laquelle:
- soit l'un au moins de R2d, R3d, R4d, R5d, R5'd et R6d est différent de halogène, hydroxyle, alkyle et alcoxy; les autres substituants Rd et R1d desdits produits de formule (Id) ayant l'une quelconque des définitions indiquées ci-dessus ou ci-après.
- soit Rd ne représente pas un atome d'hydrogène ou un radical alkyle éventuellement substitué par un ou plusieurs atomes d'halogène, les autres substituants R1d, R2d, R3d, R4d , R5d, R5'd et R6d desdits produits de formule (Id) ayant l'une quelconque des définitions indiquées ci-dessus ou ci-après est décrite.

### La présente invention a également pour objet les produits de formule (Ie):

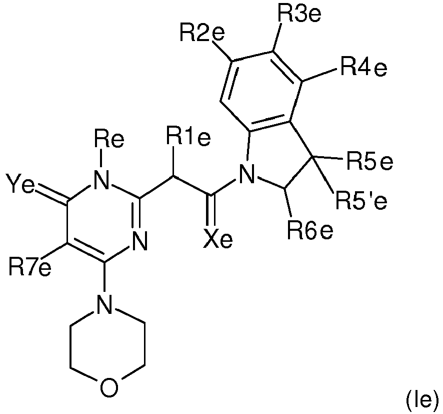

dans laquelle :
Xe et Ye, identiques ou différents, sont tels que :
Xe représente O ou S et Ye représente S,
Re représente un atome d'hydrogène ou un radical alkyle;
R1 e représente un atome d'hydrogène ou un radical méthyle ;
R2e représente un atome d'hydrogène ou un atome de fluor ;
R3e représente un atome d'hydrogène ou un atome d'halogène ;
R4e représente un atome d'hydrogène, un atome d'halogène ou un radical hydroxyle, alkyle ou alcoxy, les radicaux alkyle étant éventuellement substitués par un ou plusieurs radicaux identiques ou différents choisis parmi les atomes d'halogène et le radical hydroxyle, les radicaux alcoxy étant éventuellement substitués par un ou plusieurs atomes d'halogène ;
R5e et R5'e, identiques ou différents, représente un atome d'hydrogène ou un radical alkyle ;
R6e représente un atome d'hydrogène ou un radical alkyle éventuellement substitué par un ou plusieurs radicaux identiques ou différents choisis parmi les atomes d'halogène et le radical hydroxyle ;
R7e représente un atome d'hydrogène ou un atome d'halogène;
lesdits produits de formule (le) étant sous toutes les formes isomères possibles racémiques, énantiomères et diastéréoisomères, ainsi que les sels d'addition avec les acides minéraux et organiques ou avec les bases minérales et organiques desdits produits de formule (le).

La présente invention concerne ainsi les produits de formule (le) tels que définis ci-dessus dans laquelle :
Xe et Ye, identiques ou différents, sont tels que :
Xe représente O ou S et Y représente S,
Re représente un atome d'hydrogène;
R1 e représente un atome d'hydrogène ou un radical méthyle ;
R2e représente un atome d'hydrogène ou un atome de fluor ;
R3e représente un atome d'hydrogène ou un atome d'halogène ;
R4e représente un atome d'hydrogène, un atome d'halogène ou un radical hydroxyle, alkyle ou alcoxy, les radicaux alkyle étant éventuellement substitués par un ou plusieurs radicaux identiques ou différents choisis parmi les atomes d'halogène et le radical hydroxyle, les radicaux alcoxy étant éventuellement substitués par un ou plusieurs atomes d'halogène ;
R5e et R5'e, identiques ou différents, représente un atome d'hydrogène ou un radical alkyle ;
R6e représente un atome d'hydrogène ou un radical alkyle éventuellement substitué par un ou plusieurs radicaux identiques ou différents choisis parmi les atomes d'halogène et le radical hydroxyle ;
R7e représente un atome d'hydrogène;
lesdits produits de formule (le) étant sous toutes les formes isomères possibles racémiques, énantiomères et diastéréoisomères, ainsi que les sels d'addition avec les acides minéraux et organiques ou avec les bases minérales et organiques desdits produits de formule (le).

### Dans les formules (Ia), (Ib), (Ic), (Id) et (Ie):

- le terme radical alkyle (ou alk) désigne les radicaux, linéaires et ramifiés, renfermant de 1 à 10 atomes de carbone, tels que méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : on préfère les radicaux alkyles renfermant de 1 à 6 atomes de carbone et plus particulièrement les radicaux alkyle renfermant de 1 à 4 atomes de carbone de la liste ci-dessus ;
- le terme radical alcoxy désigne les radicaux linéaires et ramifiés, renfermant de 1 à 10 atomes de carbone, tels que méthoxy, éthoxy, propoxy, isopropoxy, butoxy linéaire, secondaire ou tertiaire, pentoxy ou hexoxy ainsi que leurs isomères de position linéaires ou ramifiés : on préfère les radicaux alkoxy renfermant de 1 à 4 atomes de carbone de la liste ci-dessus ;
- le terme atome d'halogène désigne les atomes de chlore, de brome, d'iode ou de fluor et de préférence l'atome de chlore, de brome ou de fluor.
- le terme radical cycloalkyle désigne un radical carbocyclique saturé renfermant 3 à 10 atomes de carbone et désigne ainsi notamment les radicaux cyclopropyle, cyclobutyle, cyclopentyle et cyclohexyle et tout particulièrement les radicaux cyclopropyle, cyclopentyle et cyclohexyle ;
- le terme radical alkylthio désigne les radicaux linéaires et le cas échéant ramifiés, méthylthio, éthylthio, propylthio , isopropylthio, butylthio linéaire, secondaire ou tertiaire, pentylthio ou hexylthio ainsi que leurs isomères de position linéaires ou ramifiés : on préfère les radicaux alkylthio renfermant de 1 à 4 atomes de carbone de la liste ci-dessus ;
- dans le radical -O-cycloalkyle, cycloalkyle est tel que défini ci-dessus ;
- le terme radical hétérocycloalkyle désigne ainsi un radical carbocyclique monocyclique ou bicyclique, renfermant de 3 à 10 chaînons interrompu par un ou plusieurs hétéroatomes, identiques ou différents, choisis parmi les atomes d'oxygène, d'azote ou de soufre : on peut citer par exemple les radicaux morpholinyle, thiomorpholinyle, homomorpholinyle, aziridyle, azétidyle, pipérazinyle, pipéridyle, homopipérazinyle, pyrrolidinyle, imidazolidinyle, pyrazolidinyle, tétrahydrofuryle, tétrahydrothiényle, tétrahydropyranne, oxodihydropyridazinyle, ou encore oxétanyle tous ces radicaux étant éventuellement substitués ; on peut citer notamment les radicaux morpholinyle, thiomorpholinyle, homomorpholinyle, pipérazinyle, pipéridyle, homopipérazinyle ou encore pyrrolidinyle,
- les termes aryle et hétéroaryle désignent des radicaux insaturés ou partiellement insaturés, respectivement carbocycliques et hétérocycliques, monocycliques ou bicycliques, renfermant au plus 12 chaînons, pouvant éventuellement contenir un chaînon -C(O), les radicaux hétérocycliques contenant un ou plusieurs hétéroatomes identiques ou différents choisis parmi O, N, ou S avec N, le cas échéant, éventuellement substitué ;
le terme radical aryle désigne ainsi des radicaux monocycliques ou bicycliques renfermant 6 à 12 chaînons tels que par exemple les radicaux phényle, naphtyle, biphényle, indényle, fluorényle et anthracényle, plus particulièrement les radicaux phényle et naphtyle et encore plus particulièrement le radical phényle. On peut noter qu'un radical carbocyclique contenant un chaînon -C(O) est par exemple le radical tétralone ;
le terme radical hétéroaryle désigne ainsi des radicaux monocycliques ou bicycliques renfermant 5 à 12 chaînons : des radicaux hétéroaryles monocycliques tels que par exemple les radicaux thiényle tel que 2-thiényle et 3-thiényle, furyle tel que 2-furyle, 3-furyle, pyrannyle, pyrrolyle, pyrrolinyle, pyrazolinyle, imidazolyle, pyrazolyle, pyridyle tel que 2-pyridyle, 3-pyridyle et 4-pyridyle, pyrazinyle, pyrimidinyle, pyridazinyle, oxazolyle, thiazolyle, isothiazolyle, diazolyle, thiadiazolyle, thiatriazolyle, oxadiazolyle, isoxazolyle tel que 3- ou 4-isoxazolyle, furazannyle, tétrazolyle libre ou salifié, tous ces radicaux étant éventuellement substitués parmi lesquels plus particulièrement les radicaux thiényle tel que 2-thiényle et 3-thiényle, furyle tel que 2-furyle, pyrrolyle, pyrrolinyle, pyrazolinyle, imidazolyle, pyrazolyle, oxazolyle, isoxazolyle, pyridyle, pyridazinyle, ces radicaux étant éventuellement substitués ; des radicaux hétéroaryles bicycliques tels que par exemple les radicaux benzothiényle tel que 3-benzothiényle, benzothiazolyle, quinolyle, isoquinolyle, dihydroquinolyle, quinolone, tétralone, adamentyl, benzofuryle, isobenzofuryle, dihydrobenzofuranne, éthylènedioxyphényle, thianthrényle, benzopyrrolyle, benzimidazolyle, benzoxazolyle, thionaphtyle, indolyle, azaindolyle, indazolyle, purinyle, thiénopyrazolyle, tétrahydroindazolyle, tétrahydrocyclopentapyrazolyle, dihydrofuropyrazolyle, tétrahydropyrrolopyrazolyle, oxotétrahydropyrrolopyrazolyle, tétrahydropyranopyrazolyle, tétrahydropyridinopyrazolyle ou oxodihydropyridino-pyrazolyle, tous ces radicaux étant éventuellement substitués ;

Comme exemples de radicaux hétéroaryles ou bicycliques, on peut citer plus particulièrement les radicaux pyrimidinyle, pyridyle, pyrrolyle, azaindolyle, indazolyle ou pyrazolyle, benzothiazolyle ou benzimidazolyle éventuellement substitués par un ou plusieurs substituants identiques ou différents comme indiqué ci-dessus.

Le ou les radicaux carboxy des produits de formules (la) à (le) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formules (la) à (le) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

### Selon certains modes préférés de l'invention:

### Pour les produits de formule (Ib):

Dans les produits de formule (Ib) tels que définis ci-dessus, R1b peut ainsi par exemple représenter un radical phényle éventuellement substitué par un ou plusieurs radicaux identiques ou différents choisis parmi les atomes d'halogène et les radicaux CN, nitro, -COOH, -COOalk, -N(Rx)b(Ry)b, alcoxy, alkyle, alkynyle et cycloalkyle;
ces derniers radicaux alcoxy, alkyle et alkynyle, étant eux-mêmes éventuellement substitués par un ou plusieurs radicaux identiques ou différents choisis parmi les atomes d'halogène et les radicaux hydroxyle alcoxy et N(Rv)b(Rw)b ,
avec (Rx)b,(Ry)b,(Rv)b,(Rw)b tels que définis ci-dessus ou ci-après.

Dans les produits de formule (Ib) tels que définis ci-dessus, N(Rx)b(Ry)b peut être tel que soit (Rx)b représente un atome d'hydrogène ou un radical alkyle et (Ry)b représente un atome d'hydrogène ou un radical alkyle; soit (Rx)b et (Ry)b forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
et N(Rv)b(Rw)b peut être tel que (Rv)b représente un atome d'hydrogène ou un radical alkyle et (Rw)b représente un atome d'hydrogène ou un radical alkyle;
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

Notamment, lorsque N(Rx)b(Ry)b ou N(Rv)b(Rw)b forme un cycle comme défini ci-dessus, un tel cycle aminé peut être choisi notamment parmi les radicaux pyrrolidinyle, pyrazolidinyle, pyrazolinyle, pipéridyle, azépinyle, morpholinyle, homomorpholinyle, pipérazinyle ou homopipérazinyle, ces radicaux étant eux-mêmes éventuellement substitués comme indiqué ci-dessus ou ci-après.

Le cycle N(Rx)b(Ry)b ou N(Rv)b(Rw)b peut plus particulièrement être choisi parmi les radicaux pyrrolidinyle, morpholinyle éventuellement substitué par un ou deux radicaux alkyle ou pipérazinyle éventuellement substitué sur le second atome d'azote par un radical alkyle, phényle, ou et CH2-phényle, eux-mêmes éventuellement substitués par un ou plusieurs radicaux identiques ou différents choisis parmi les atomes d'halogène et les radicaux alkyle, hydroxyle et alcoxy.

La présente invention a ainsi pour objet les produits de formule (Ib) tels que définis ci-dessus dans laquelle :
Ab représente un radical pyridyle ou un radical morpholine tels que définis ci-après: 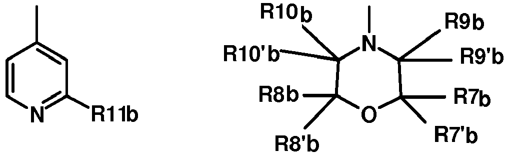 dans lesquels:
   R11 b représente un atome d'hydrogène, un atome d'halogène, un radical alcoxy ou un radical alkyle;
   R7b, R7'b, R8b et R8'b, identiques ou différents représentent un atome d'hydrogène ou un atome de Deutérium D, ou un radical alkyle éventuellement substitué par un ou plusieurs radicaux choisis parmi l'atome de fluor et le radical hydroxyle,
   R9b, R9'b, R10b et R10'b, identiques ou différents, représentent un atome d'hydrogène ou de Deutérium D,
   étant entendu que l'un au moins de R7b, R7'b, R8b, R8'b, R9b, R9'b, R10b et R10'b est différent de l'atome d'hydrogène,
   étant entendu que R7b avec R9b ou bien R8b avec R10b peuvent former ensemble un radical cyclique avec les atomes de carbone auxquels ils sont liés ;
   R1b représente un radical phényle, éventuellement substitués par un ou plusieurs radicaux identiques ou différents choisis parmi les atomes d'halogène et les radicaux CN, NH2, NHalk, N(alk)2, alcoxy et alkyle, ces derniers radicaux alcoxy et alkyle étant eux-mêmes éventuellement substitués par un ou plusieurs radicaux identiques ou différents choisis parmi les atomes d'halogène et les radicaux hydroxyle et alcoxy;
   Rb représente un atome d'hydrogène ou bien forme avec R1 b un cycle
   2,3-dihydro-indol-1-yle défini comme suit : 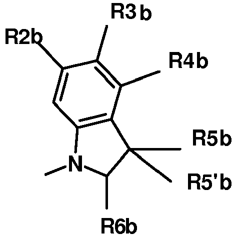 dans lequel:
   R2b représente un atome d'hydrogène ou un atome de fluor ;
   R3b représente un atome d'hydrogène ou un atome d'halogène ;
   R4b représente un atome d'hydrogène, un atome d'halogène ou un radical hydroxyle, alkyle ou alcoxy, les radicaux alkyle étant éventuellement substitués par un ou plusieurs radicaux identiques ou différents choisis parmi les atomes d'halogène et le radical hydroxyle, les radicaux alcoxy étant éventuellement substitués par un ou plusieurs atomes d'halogène ;
   R5b et R5'b, identiques ou différents, représentent un atome d'hydrogène ou un radical alkyle ;
   R6b représente un atome d'hydrogène ou un radical alkyle éventuellement substitué par un ou plusieurs radicaux identiques ou différents choisis parmi les atomes d'halogène (F) et les radicaux hydroxyle et alcoxy ;
   (Ra)b et (Rb)b, identiques ou différents représentent indépendamment un atome d'hydrogène ou un radical alkyle;
   (Rc)b représente un atome d'hydrogène ou un radical alkyle;
   lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

La présente invention a particulièrement pour objet les produits de formule (Ib) tels que définis ci-dessus dans laquelle :
Ab représente un radical morpholine ou un radical pyridyle tels que définis ci-après: 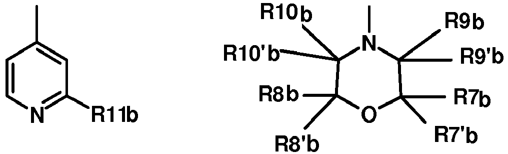 dans lesquels:
   R11 b représente un atome d'hydrogène, un atome de fluor, un radical alcoxy ou un radical alkyle ;
   et R7b,R7'b,R8b, R8'b , R9b, R9'b, R10b et R10'b sont tels que :
   soit R7b, R7'b, R8b, R8'b, R9b, R9'b, R10b et R10'b représentent tous un atome de Deutérium D ;
   soit R7b, R7'b, R8b, R8'b représentent tous un atome d'hydrogène et R9b, R9'b, R10b et R10'b représentent tous un atome de Deutérium D ;
   soit R7b représente un radical alkyle éventuellement substitué par un ou plusieurs radicaux choisis parmi l'atome de fluor et le radical hydroxyle, et R7'b, R8b, R8'b, R9b, R9'b, R10b et R10'b représentent tous un atome d'hydrogène ;
   soit R7b avec R9b ou bien R8b avec R10b forment ensemble un radical cyclique avec les atomes de carbone auxquels ils sont liés ;
   R1b représente un radical phényle éventuellement substitué par un ou plusieurs radicaux choisis parmi les atomes de fluor et de chlore ;
   Rb représente un atome d'hydrogène ou bien forme avec R1 b le bicycle défini ci-après : 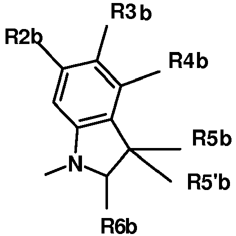 dans lequel
      R2b représente un atome d'hydrogène ou un atome de fluor ;
      R3b représente un atome d'hydrogène ou un atome de fluor ;
      R4b représente un atome d'hydrogène ou un atome de fluor ou de chlore ;
      R5b et R5'b représentent un atome d'hydrogène ;
      R6b représente un atome d'hydrogène ou un radical méthyle;
      (Ra)b et (Rb)b représentent un atome d'hydrogène;
      (Rc)b représente un atome d'hydrogène;
   lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

### Pour les composés de formule (Id):

Dans les produits de formule (Id) tels que définis ci-dessus, N(Rx)d(Ry)d peut par exemple être tel que soit (Rx)d représente un atome d'hydrogène ou un radical alkyle et (Ry)d représente un atome d'hydrogène ou un radical alkyle; soit (Rx)d et (Ry)d forment avec l'atome d'azote auquel ils sont liés un radical cyclique renfermant de 3 à 7 chaînons et éventuellement un ou plusieurs autres hétéroatomes choisi(s) parmi O, S, NH, ce radical cyclique étant éventuellement substitué comme indiqué ci-dessus ou ci-après ;
et N(Rv)d(Rw)d peut être tel que (Rv)d représente un atome d'hydrogène ou un radical alkyle et (Rw)d représente un atome d'hydrogène ou un radical alkyle; soit (Rv)d et (Rw)d forment avec l'atome d'azote auquel ils sont liés un radical cyclique renfermant de 3 à 7 chaînons et éventuellement un ou plusieurs autres hétéroatomes choisi(s) parmi O, S, NH, ce radical cyclique étant éventuellement substitué comme indiqué ci-dessus ou ci-après;
lesdits produits de formule (Id) étant sous toutes les formes isomères possibles racémiques, énantiomères et diastéréoisomères, ainsi que les sels d'addition avec les acides minéraux et organiques ou avec les bases minérales et organiques desdits produits de formule (Id).

Notamment, lorsque N(Rx)d(Ry)d ou N(Rv)d(Rw)d forme un cycle respectivement avec l'atome auquel ils sont liés comme défini ci-dessus, un tel cycle aminé peut être choisi notamment parmi les radicaux pyrrolidinyle, pyrazolidinyle, pyrazolinyle, pipéridyle, azépinyle, morpholinyle, homomorpholinyle, pipérazinyle ou homopipérazinyle, ces radicaux étant eux-mêmes éventuellement substitués comme indiqué ci-dessus ou ci-après.

Le cycle N(Rx)d(Ry)d ou N(Rv)d(Rw)d peut plus particulièrement être choisi parmi les radicaux pyrrolidinyle, morpholinyle lui-même éventuellement substitué par un ou deux radicaux alkyle ou pipérazinyle lui-même éventuellement substitué sur le second atome d'azote par un radical alkyle, phényle, -CH2-phényle ou
- SO2-Alk , tous ces derniers radicaux alkyle, Alk et phényle étant eux-mêmes éventuellement substitués par un ou plusieurs radicaux identiques ou différents choisis parmi les atomes d'halogène et les radicaux alkyle, hydroxyle et alcoxy.

Dans les produits de formule générale (Id) telle que définie ci-dessus:
Rd représente un atome d'hydrogène ou un radical alkyle, cycloalkyle, phényle, NH2 ou CN ;
R1d représente un atome d'hydrogène ou un radical méthyle ;
R2d représente un atome d'hydrogène ou un atome de fluor ;
R3d représente un atome d'hydrogène ou un atome de fluor;
R4d représente un atome d'hydrogène ; un atome d'halogène; ou un radical hydroxyle, alkyle, ou alcoxy, hétérocycloalkyle, N(Rx)d(Ry)d, phényle ou hétéroaryle; les radicaux alkyle étant éventuellement substitués par un ou plusieurs radicaux identiques ou différents choisis parmi les atomes d'halogène et les radicaux hydroxyle, hétérocycloalkyle et N(Rv)d(Rw)d ; les radicaux alcoxy étant éventuellement substitués par un ou plusieurs atomes d'halogène ;
R5d et R5'd, identiques ou différents, représentent un atome d'hydrogène ou un radical alkyle ou forment ensemble avec l'atome de carbone auquels ils sont liés un radical cyclique de 3 à 6 chaînons renfermant éventuellement un ou plusieurs hétéroatomes choisis parmi O, S, NH, éventuellement substitué par un radical alkyle ou cycloalkyle ;
R6d représente un atome d'hydrogène ; ou un radical alkyle (lui-même éventuellement substitué par un ou plusieurs radicaux identiques ou différents choisis parmi les atomes de fluor, des atomes de deutérium et les radicaux hydroxyle et alcoxy ; un radical cycloalkyle ou un radical phényle lui-même éventuellement substitué par un ou plusieurs radicaux identiques ou différents choisis parmi les atomes de fluor ou les radicaux alcoxy
R5d et R6d pouvant éventuellement former avec les atomes de carbone auquel ils sont liés, un radical cyclique renfermant de 3 à 7 chaînons;
lesdits produits de formule (Id) étant sous toutes les formes isomères possibles racémiques, énantiomères et diastéréoisomères, ainsi que les sels d'addition avec les acides minéraux et organiques ou avec les bases minérales et organiques desdits produits de formule (Id).

La formulé générale (Id) dans laquelle:
R2d représente un atome d'hydrogène ou un atome de fluor ;
R3d représente un atome d'hydrogène ou un atome de fluor , chlore ou brome;
R4d représente un atome d'hydrogène ; un atome d'halogène choisi parmi chlore, fluor et brome; et un radical hydroxyle, alkyle ; alcoxy ; un radical pyrrolidinyle, ou pipéridyle éventuellement substitués par un radical alkyle ; morpholinyle ; pipérazinyle éventuellement substitué par alk sur N ; phényle éventuellement substitué par un ou plusieurs radicaux choisis parmi les atomes de Cl, F ou OCH3 ; et pyridyle ; étant entendu que les radicaux alkyle sont éventuellement substitués par un ou plusieurs radicaux identiques ou différents choisis parmi les atomes de fluor, le radical hydroxyle, et le radical pipérazinyle lui-même éventuellement substitué sur N par un radical alkyle ou SO2-alk ; étant entendu que les radicaux alcoxy sont éventuellement substitués par un ou plusieurs atomes de fluor;
R5d et R5'd, identiques ou différents, représente un atome d'hydrogène ou un radical alkyle ou forment ensemble avec l'atome de carbone auquels ils sont liés un radical spirocyclopropyle, spirotétrahydropyranne ou spiropipéridyle éventuellement substitué par un radical alkyle ou cycloalkyle sur N ;
R6d représente un atome d'hydrogène ; ou un radical alkyle lui-même éventuellement substitué par un ou plusieurs radicaux identiques ou différents choisis parmi les atomes de fluor; un radical cyclopropyle ; ou un radical phényle lui-même éventuellement substitué par un ou plusieurs radicaux identiques ou différents choisis parmi les atomes de fluor ;
R5d et R6d pouvant éventuellement former avec les atomes de carbone auquels ils sont liés un radical cyclopentyle ;
lesdits produits de formule (Id) étant sous toutes les formes isomères possibles racémiques, énantiomères et diastéréoisomères, ainsi que les sels d'addition avec les acides minéraux et organiques ou avec les bases minérales et organiques desdits produits de formule (Id) est décrite.
Dans les produits de formule (Id) tels que définis ci-dessus, lorsque R4d représente un radical alkyle ou alcoxy éventuellement substitués par un ou plusieurs atomes d'halogène, R4d peut notamment représenter un radical -CF3, -OCF3 ou encore - OCHF2

### Pour les composés de formule (Ie):

La présente invention a ainsi pour objet les produits de formule (le) tels que définis ci-dessus dans laquelle:
Xe et Ye, identiques ou différents, sont tels que :
Xe représente O ou S et Ye représente S,
Re représente un atome d'hydrogène ou un radical méthyle;
R1 e représente un atome d'hydrogène ou un radical méthyle ;
R2e représente un atome d'hydrogène ou un atome de fluor ;
R3e représente un atome d'hydrogène ou un atome de fluor ;
R4e représente un atome d'hydrogène, un atome d'halogène ou un radical alkyle éventuellement substitué par un ou plusieurs radicaux identiques ou différents choisis parmi les atomes d'halogène ;
R5e et R5'e représentent un atome d'hydrogène ;
R6e représente un atome d'hydrogène ou un radical alkyle éventuellement substitué par un ou plusieurs radicaux identiques ou différents choisis parmi les atomes de fluor et le radical hydroxyle;
R7e représente un atome d'hydrogène ou un atome de fluor;
lesdits produits de formule (le) étant sous toutes les formes isomères possibles racémiques, énantiomères et diastéréoisomères, ainsi que les sels d'addition avec les acides minéraux et organiques ou avec les bases minérales et organiques desdits produits de formule (le).

La présente invention a ainsi pour objet les produits de formule (le) tels que définis ci-dessus dans laquelle:
Xe et Ye, identiques ou différents, sont tels que :
Xe représente O ou S et Ye représente S,
Re représente un atome d'hydrogène;
R1 e représente un atome d'hydrogène ou un radical méthyle ;
R2e représente un atome d'hydrogène ou un atome de fluor ;
R3e représente un atome d'hydrogène ou un atome de fluor ;
R4e représente un atome d'hydrogène, un atome d'halogène ou un radical alkyle éventuellement substitué par un ou plusieurs radicaux identiques ou différents choisis parmi les atomes d'halogène ;
R5e et R5'e représentent un atome d'hydrogène ;
R6e représente un atome d'hydrogène ou un radical alkyle éventuellement substitué par un ou plusieurs radicaux identiques ou différents choisis parmi les atomes de fluor et le radical hydroxyle;
R7e représente un atome d'hydrogène;
lesdits produits de formule (le) étant sous toutes les formes isomères possibles racémiques, énantiomères et diastéréoisomères, ainsi que les sels d'addition avec les acides minéraux et organiques ou avec les bases minérales et organiques desdits produits de formule (le).

Dans les produits de formule (le) tels que définis ci-dessus, R4e représente par exemple un atome d'hydrogène ou un atome d'halogène tel que fluor, chlore ou brome ou encore le radical CF3, les autres substituants Re, R1e, R2e, R3e, R5e, R5'e et R6e ayant l'une quelconque de leurs significations indiquées ci-dessus pour lesdits produits de formule (le).

Dans les produits de formule (le), R6e représente par exemple un atome d'hydrogène ou un radical méthyle éventuellement substitué par un atome de fluor ou le radical hydroxyle. Les autres substituants Re, R1e, R2e, R3e, R4e, R5e et R5'e ayant l'une quelconque de leurs significations indiquées ci-dessus pour lesdits produits de formule (le).

### Procédés de préparation:

Leprocédé de préparation des produits de formules (la), (Ib), (Ic), (Id) et (le) tels que définis ci-dessus sont décrits.

Les produits selon l'invention peuvent être préparés à partir de méthodes conventionnelles de chimie organique.

### Préparation générale:

### Préparation de composés de formule (Ia) :

Les produits de formule générale (la) selon la présente invention peuvent notamment être préparés comme indiqué dans les Schémas Généraux (1 A)a-(1 E)a ci-dessous. Les préparations des exemples de la présente invention donnent des illustrations des schémas ci-dessous.

Les procédés de préparation des produits de formule Ca à (la) sont tels que définis dans les Schémas Généraux (1A)a-(1 E)a ci-dessous.

Les schémas (1 A)a-(1 E)a ci-dessous sont illustratifs des méthodes utilisées pour la préparation des produits de formule (la). Les produits de formule (la) tels que définis ci-dessus selon la présente invention peuvent ainsi notamment être préparés selon les procédés décrits dans les schémas (1A)a-(1E)a.

Le procédé de préparation de produits de formule (la) selon le schéma (1A)a est tel que défini ci-après.

Le procédé de préparation de produits de formule (la) selon le schéma (1B)a est tel que défini ci-après.

Le procédé de préparation de produits de formule (la) selon le schéma (1 C)a est tel que défini ci-après.

Le procédé de préparation de produits de formule (la) selon le schéma (1 D)a est tel que défini ci-après.

Le procédé de préparation de produits de formule (la) selon le schéma (1 E)a est tel que défini ci-après.

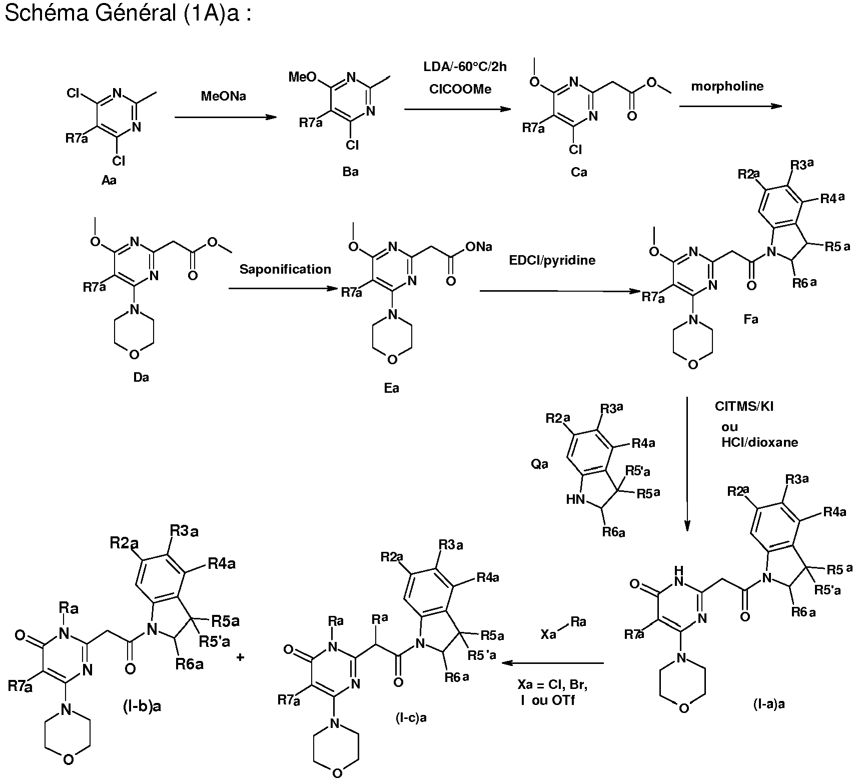

Dans lequel les substituants Ra, R1 a, R2a, R3a, R4a, R5a, R5'a, R6a et R7a ont les significations indiqués ci-dessus.

Dans le schéma général (1 A)a :
Les dérivés méthoxypyrimidines Ba, peuvent être préparées à partir des composés Aa par réaction avec le méthylate de sodium dans un solvant tel que le THF ou le méthanol à une température comprise entre 0°C et 25°C, selon, par exemple, les conditions décrites par loannidis, S. et coll. (Bioorganic and Médicinal Chemistry Letters, (2010), 20(5), 1669 - 1673).

Les composés Ca, peuvent être obtenus par traitement des composés Ba en présence du chloroformiate de methyle avec la LDA (lithium diisopropylamide) dans un solvant tel que le THF à une température comprise entre -78°C et 25°C, selon par exemple, les conditions décrites par Tomioka K. et coll. (Tetrahedron, (1988), 44(14), 4351 - 4356).

Les composés Da, peuvent être obtenus à partir d'un composé Ca par réaction avec la morpholine, en l'absence de solvant, à une température comprise entre 0°C et 25°C, comme décrit par exemple par Aliabiev S.B. (Lett. Org. Chem. (2007), 4(4), 273-280).

Le carboxylate Ea peut être obtenu par hydrolyse de l'ester Da en présence d'une base telle que la soude ou la lithine, dans un solvant tel que le tétrahydrofuranne ou le méthanol, à une température comprise entre 0°C et 30°C

Les amides Fa peuvent être obtenus à partir du carboxylate Ea par condensation d'une indoline Qa en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Les pyrimidones (I-a)a peuvent être obtenus à partir des composés Fa par réaction avec le dioxane chlorhydrique, à une température comprise entre 22°C et la température d'ébullition du solvant, comme, par exemple, dans les conditions décrites par Harnden M. R. and Coll. (J. Med. Chem. (1993), 36(10), 1343 - 1355).

Quand R7a = F, Les pyrimidones (I-a)a peuvent également être obtenus à partir des composés Fa, par réaction avec le chlorotrimethylsilane et l'iodure de potassium ou l'iodotrimethylsilane, dans un solvant tel que l'acétonitrile à une température comprise entre 22°C et la température d'ébullition du solvant, comme, par exemple, dans les conditions décrites par Shiao M.J. (J. Org. Chem. (1993), 58(17), 4742 - 4744).

Les produits (I-b)a et (I-c)a peuvent être obtenus à partir des produits (I-a)a par réaction avec un composé Ra-Xa (Xa= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans (J. Med. Chem. (2008), 51, 5766-5779).

Alternativement, les composés (la) peuvent être obtenus selon le schéma général (1B)a.

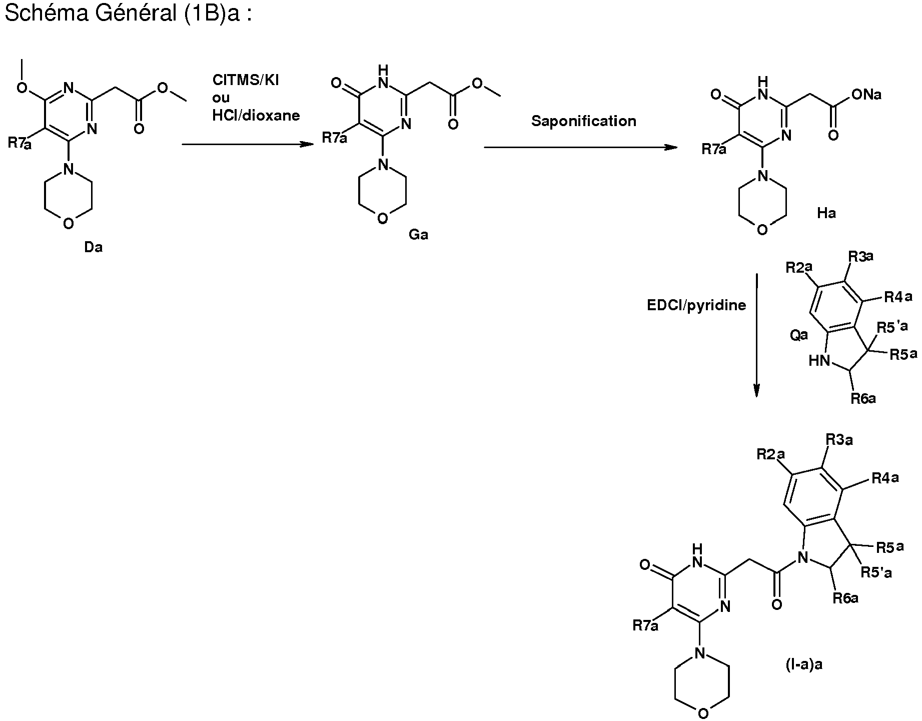

Dans lequel les substituants R2a, R3a, R4a, R5a, R5'a, R6a et R7a ont les significations indiqués ci-dessus.

Les pyrimidones Ga peuvent être obtenus à partir des composés Da par réaction avec le dioxane chlorhydrique, à une température comprise entre 22°C et la température d'ébullition du solvant, comme, par exemple, dans les conditions décrites par Harnden M. R. and Coll. (J. Med. Chem. (1993), 36(10), 1343 - 1355).

Quand R7a = F, Les pyrimidones Ga peuvent également être obtenus à partir des composés Da, par réaction avec le chlorotrimethylsilane et l'iodure de potassium ou l'iodotrimethylsilane, dans un solvant tel que l'acétonitrile à une température comprise entre 22°C et la température d'ébullition du solvant, comme, par exemple, dans les conditions décrites par Shiao M.J. (J. Org. Chem. (1993), 58(17), 4742 - 4744).

Le carboxylate Ha peut être obtenu par hydrolyse de l'ester Ga en présence d'une base telle que la soude ou la lithine, dans un solvant tel que le tétrahydrofuranne ou le méthanol, à une température comprise entre 0°C et 30°C

Les amides (I-a)a peuvent être obtenus à partir du carboxylate Ha par condensation d'une indoline Qa en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Alternativement, les composés Ga peuvent être obtenus selon le schéma général (1 C)a.

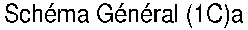

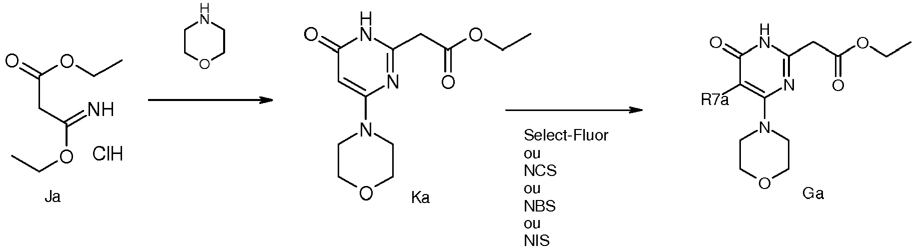

Dans lequel le substituant R7a a les significations indiqués ci-dessus.

L'ester Ka peut être obtenu par réaction « one-pot » entre la morpholine et un excès (par exemple 3 équivalents) d'imino-éther malonique Ja (ou de son tautomère amino-acrylate), dans un solvant tel que l'éthanol, à une température comprise entre 20°C et le point d'ébullition du solvant.

Les pyrimidones Ga peuvent être obtenus à partir des composés Ka, quand R7a = F, avec un réactif de fluoration tel que le Selectfluor, dans un solvant tel que l'acétonitrile à une température comprise entre 22°C et la température d'ébullition du solvant, comme, par exemple, dans les conditions décrites par Banks R. E. (Journal of fluorine Chemestry (1998), 87, 1-17).

Quand R7a = Cl ; Br, I, les pyrimidones Ga peuvent être obtenus à partir des composés Ka par réaction avec le N-chlorosuccimide, N-bromosuccimide ou le N-iodosuccimide, dans un solvant tel le chloroforme, à une température comprise entre 22°C et la température d'ébullition du solvant, comme, par exemple, dans les conditions décrites par Kanojia R. M. et Coll. (J. Med. Chem. (1988), 31(7), 1363 - 1368).

Alternativement, les composés (I-a)a peuvent être obtenus selon le schéma général (1 D)a.

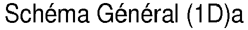

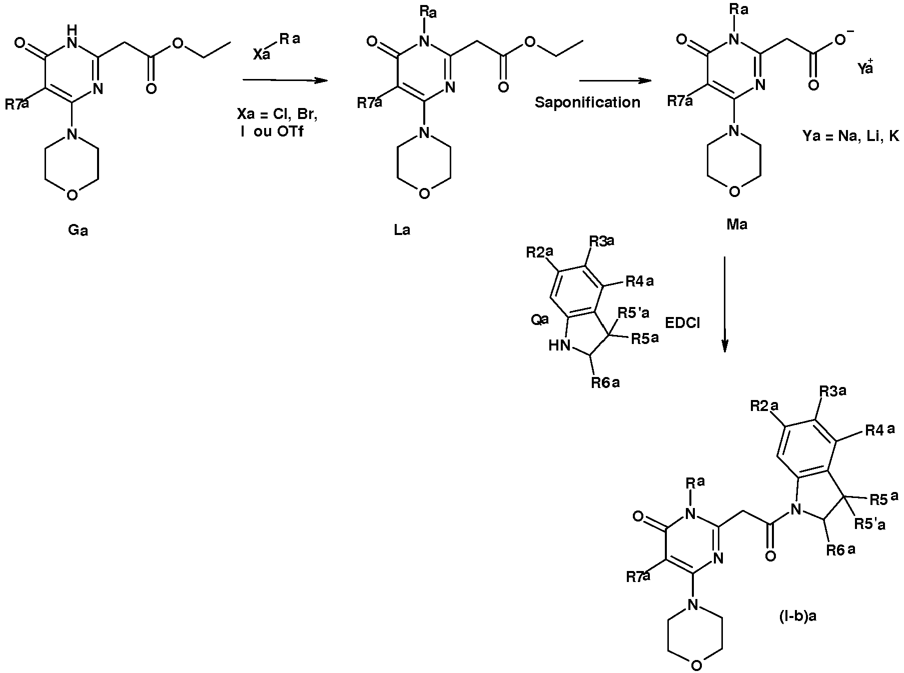

Dans lequel les substituants Ra, R2a, R3a, R4a, R5a, R5'a, R6a et R7a ont les significations indiqués ci-dessus.

Dans le Schéma Général (1 D)a :
Les esters La peuvent être obtenus à partir de l'ester Ga par réaction avec un composé Ra-Xa (Xa= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans (J. Med. Chem. (2008), 51, 5766-5779).

Les carboxylates Ma peuvent être obtenus par hydrolyse des esters La, en présence d'une base telle que la soude ou la lithine, dans un solvant tel que le tétrahydrofuranne ou le méthanol, à une température comprise entre 0°C et 30°C

Les amides (I-b)a peuvent être obtenus à partir des carboxylates Ma par condensation d'une indoline Qa en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Alternativement, les composés (I-a)a peuvent être obtenus selon le schéma général (1 E)a.

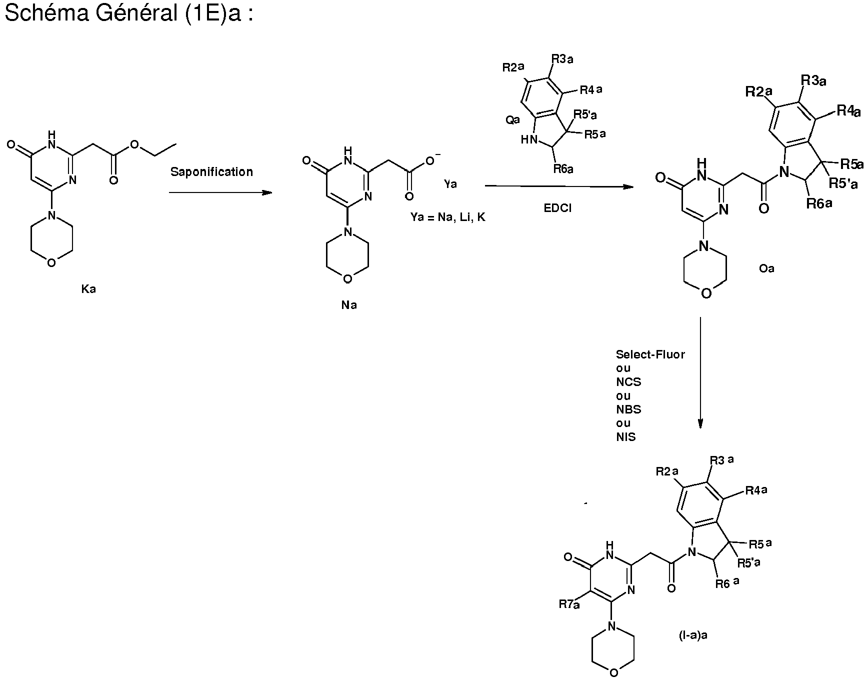

Dans lequel les substituants R2a, R3a, R4a, R5a, R5'a, R6a et R7a ont les significations indiqués ci-dessus.

Les carboxylates Na peuvent être obtenus par hydrolyse des esters Ka, en présence d'une base telle que la soude ou la lithine, dans un solvant tel que le tétrahydrofuranne ou le méthanol, à une température comprise entre 0°C et 30°C

Les amides Oa peuvent être obtenus à partir des carboxylates Na par condensation d'une indoline Qa en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Les pyrimidones (I-a)a peuvent être obtenus à partir des composés Oa, quand R7a = F, avec un réactif de fluoration tel que le Selectfluor, dans un solvant tel que l'acétonitrile à une température comprise entre 22°C et la température d'ébullition du solvant, comme, par exemple, dans les conditions décrites par Banks R. E. (Journal of fluorine Chemestry (1998), 87, 1-17).

Quand R7a = Cl ; Br, I, les pyrimidones (I-a)a peuvent être obtenus à partir des composés Oa par réaction avec le N-chlorosuccimide, N-bromosuccimide ou le N-iodosuccimide, dans un solvant tel le chloroforme, à une température comprise entre 22°C et la température d'ébullition du solvant, comme, par exemple, dans les conditions décrites par Kanojia R. M. et Coll. (J. Med. Chem. (1988), 31(7), 1363 - 1368).

Les indolines Qa, exemples de références, peuvent être obtenues selon le schéma général (1 F)a.

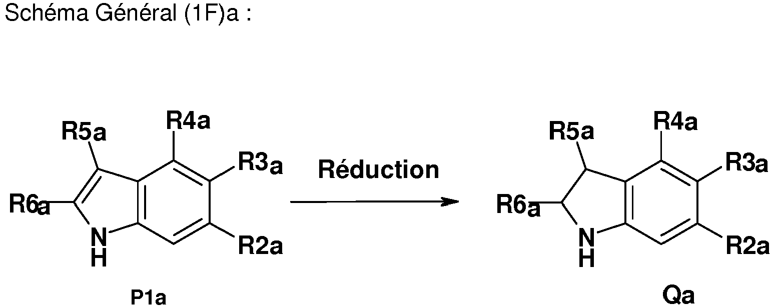

Dans lequel les substituants R2a, R3a, R4a, R5a et R6a ont les significations indiqués ci-dessus.

Les indolines, exemples de références, de formule générale Qa, lorsqu'elles ne sont pas commerciales, peuvent être généralement obtenues à partir des indoles correspondants P1a par réduction en présence d'un agent réducteur tel qu'un hydrure (le cyanoborhydrure de sodium par exemple) au sein d'un solvant tel que l'acide acétique ou l'acide trifluoroacétique, à une température comprise entre -5°C et 25°C, comme décrit par exemple par Kumar, Y. (Synth. Commun., 1983, 13(6), 489-494).

Les indolines énantiomériquement enrichies peuvent être obtenues par exemple par dédoublement chimique des énantiomères à l'aide d'une copule chirale énantiomériquement pure, tel que décrit dans le Schéma Général (1 G)a.

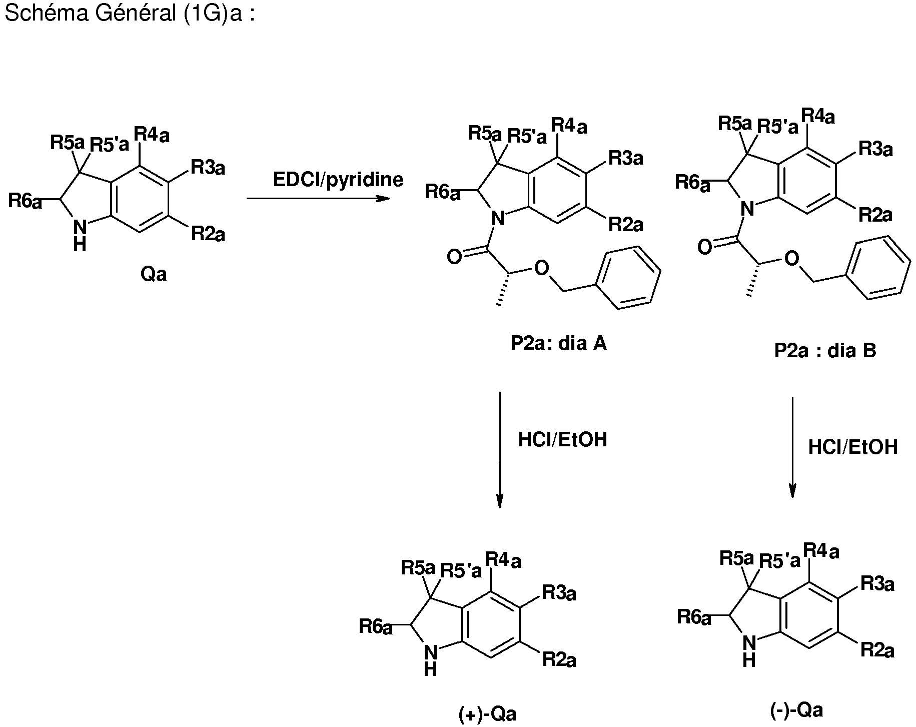

Dans lequel les substituants R2a, R3a, R4a, R5a, R5'a et R6a ont les significations indiqués ci-dessus.

Par exemple, le dédoublement des énantiomères de l'indoline Qa peut être réalisé par séparation chromatographique des deux diastéréoisomères formés par couplage de type peptidique avec l'acide o-benzyl-D-lactique en présence d'un agent de couplage peptidique tel que, par exemple, l'EDCI (N-[3-(diméthylamino)propyl]-N'-éthylcarbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que le N,N-diméthylformamide, la pyridine, l'éthanol, l'eau ou le méthanol, à une température comprise entre 20°C et 100°C, comme par exemple dans les conditions décrites par Kunishima, M. et Coll. (Tetrahedron, 2001, 57, 1551-1558). Les énantiomères (+)-Qa et (-)-Qa peuvent être respectivement obtenus à partir des composés P2a : dia A et P2a : dia B par réaction en présence d'un acide tel que l'acide chlorhydrique concentré, au sein d'un solvant tel qu'un alcool (l'éthanol par exemple), à une température comprise entre 20°C et la température d'ébullition du solvant, comme, par exemple, dans les conditions décrites par Krasnov, V.P. et Coll. (Mendeleev Commun. 2002, 12(1), 27-28).

Les indoles de formule générale (P1-a)a peuvent notamment être préparés comme indiqué dans le Schéma Général (1 H)a ci-dessous.

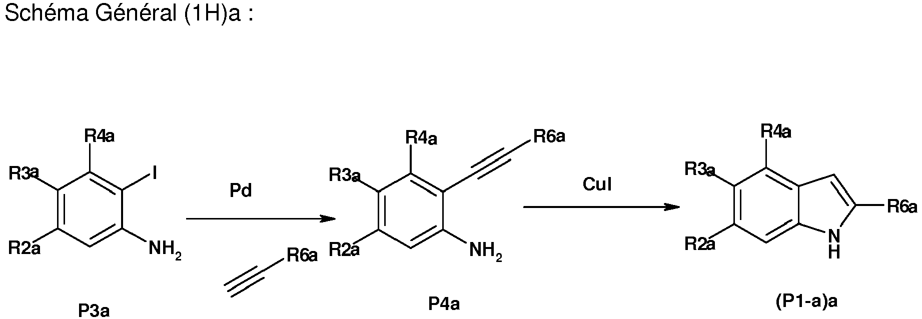

Dans lequel les substituants R2a, R3a, R4a, et R6a ont les significations indiqués ci-dessus.

Dans le schéma général (1 H)a :
Les dérivés P4a peuvent être préparés à partir des composés P3a par couplage de type Sonogashira avec un alcyne en présence d'un catalyseur à base de palladium et d'iodure de cuivre dans un solvant tel que la triéthylamine à une température comprise entre 0°C et le point d'ébullition du solvant selon, par exemple, les conditions décrites par Kuyper, F. et coll. (J. Med. Chem., 1996, 39(4), 892-903).

Les indoles (P1-a)a peuvent être obtenus par cyclisation des composés P4a en présence d'iodure de cuivre dans un solvant tel que le DMF à une température comprise entre 22°C et la température d'ébullition du solvant selon, par exemple, les conditions décrites par Kuyper, F. et coll. (J. Med. Chem., 1996, 39(4), 892-903).

Alternativement, les dérivés de formule générale (P1-b)a peuvent être obtenus selon le schéma général (1J)a ci-dessous, par analogie avec les conditions décrites dans le brevet US 2004/0224973 A1.

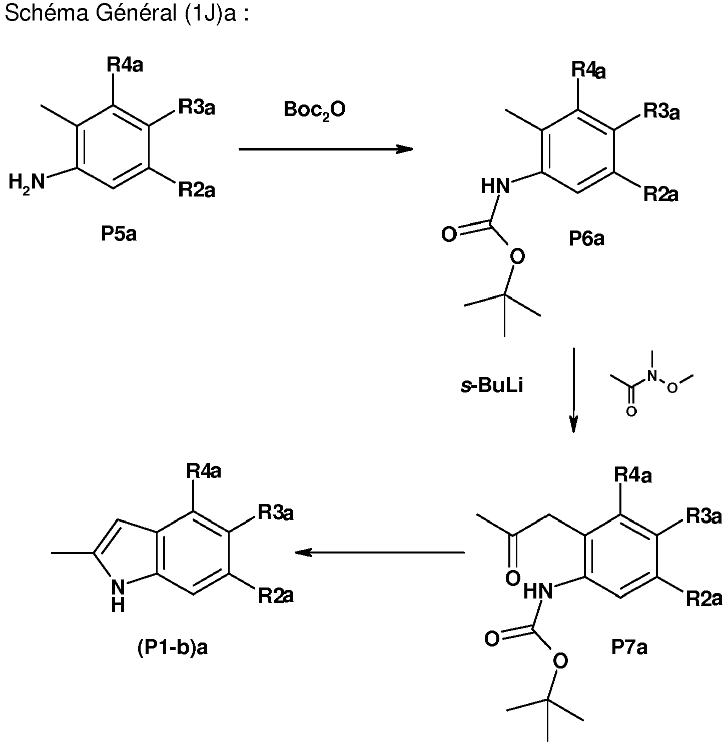

Dans lequel les substituants R2a, R3a et R4a ont les significations indiqués ci-dessus.

Dans le schéma général (1 J)a :
Les dérivés P6a peuvent être préparés à partir des dérivés anilines commerciales correspondantes P5a, par réaction d'acylation avec le di-*tert-*butyl-dicarbonate, au sein d'un solvant tel que le tetrahydrofuranne à une température comprise entre 20°C et la température d'ébullition du solvant.
Les dérivés P7a peuvent être préparés à partir des dérivés carbamates P6a, par réaction en présence d'une base telle que le sec-butyllithium, puis de N-méthoxy-N-méthyl-acétamide, au sein d'un solvant tel que le tetrahydrofuranne à une température comprise entre -50°C et -10°C.
Les dérivés (P1-b)a peuvent être préparés à partir des dérivés P7a, par réaction en présence d'un acide tel que l'acide trifluoroacétique, au sein d'un solvant tel que le dichlorométhane, à une température comprise entre 0°C et 20°C.

Parmi les produits de départs de formules Aa, Ja et Qa, certains sont connus et peuvent être obtenus soit commercialement, soit selon les méthodes usuelles connues de l'homme du métier, par exemple à partir de produits commerciaux.

### Préparation de composés de formule (Ib):

Les produits de formule générale (Ib) selon la présente invention peuvent notamment être préparés comme indiqué dans les Schémas Généraux (1A)b-(1C)b ci-dessous. Les préparations des exemples de la présente invention donnent des illustrations des schémas ci-dessous.

Les procédés de préparation des produits de formule Jb à (Ib) sont tels que définis dans les Schémas Généraux (1A)b-(1C)b ci-dessous 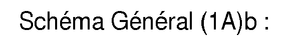 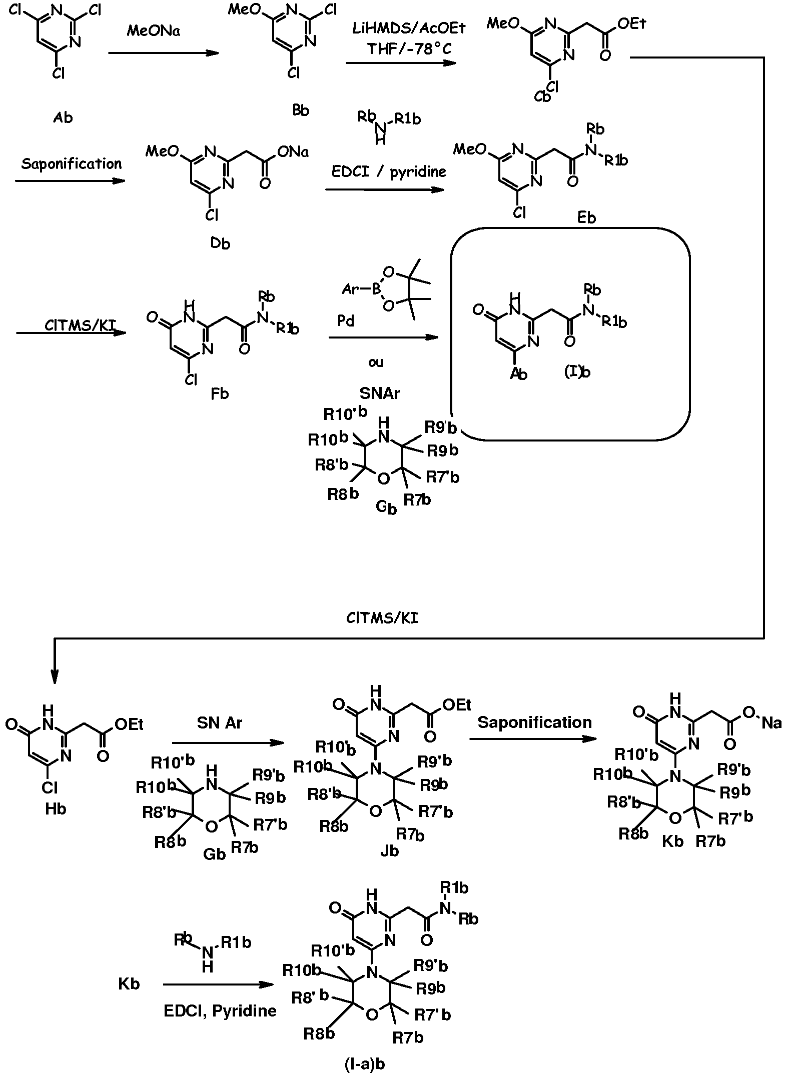 dans lequel les substituants Rb, R1b, R7b, R7'b, R8b, R8'b, R9b, R9'b, R10b, R10'b ont les significations indiquées ci-dessus.

Dans le schéma général (1 A)b :
Les dérivés méthoxypyrimidines Bb sont préparés à partir des composés Ab par réaction avec le méthylate de sodium dans un solvant tel que le THF ou le méthanol à une température comprise entre 0°c et 25°C, selon, par exemple, les conditions décrites par loannidis, S. et coll. (Bioorganic and Medicinal Chemistry Letters, (2010), 20(5), 1669-1673).

Les composés Cb peuvent être obtenus par traitement des composés Bb en présence de l'acétate d'éthyle avec la LiHMDS (lithium bis(trimethylsilyl)amide) dans un solvant tel que le THF à une température comprise entre -78°C et 25°C, selon, par exemple, les conditions décrites par Chekmarev D.S. et coll. (Tetrahedron, (2006), 62(42), 9919-9930).

Le carboxylate Db peut être obtenu par hydrolyse de l'ester Cb en présence d'une base telle que la soude ou la lithine, dans un solvant tel que le tétrahydrofuranne ou le méthanol, à une température comprise entre 0°C et 30°C

Les amides Eb peuvent être obtenus à partir du carboxylate Db par condensation d'une amine NHRbR1b en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Les pyrimidones Fb peuvent être obtenus à partir des composés Eb par réaction avec le chlorotrimethylsilane et l'iodure de potassium ou l'iodotrimethylsilane, dans un solvant tel que l'acétonitrile à une température comprise entre 22°C et la température d'ébullition du solvant, comme, par exemple, les conditions décrites par Shiao M.J. (J. Org. Chem. (1993), 58(17), 4742 - 4744).

Les composés (Ib), quand le groupement Ab est une morpholine substituée, peuvent être obtenus à partir d'un composé Fb par réaction avec une morpholine substituée Gb, en l'absence de solvant, ou en présence d'un solvant à une température comprise entre 25°C et la température d'ébullition du solvant, comme décrit par exemple par Aliabiev S.B. (Lett. Org. Chem. (2007), 4(4), 273-280). Quand le groupement Ab est un hétérocycle, ces composés (Ib) peuvent être obtenus à partir d'un composé Fb par couplage au avec un acide boronique ou une boronate en présence d'un catalyseur au palladium tel que tetrakis-triphenylphosphinepalladium et d'une base telle que le carbonate de césium dans un solvant tel que le dioxane et à une température comprise entre 25°C et la température d'ébullition du solvant ou sous irradiation micro-ondes à une température comprise entre 60°C et 150°C comme décrit par exemple par Diemer V. (European Journal of Organic Chemistry, (2006), 12, 2727-2738).

Les pyrimidones Hb peuvent être obtenus à partir des composés Cb par réaction avec le chlorotrimethylsilane et l'iodure de potassium ou l'iodotrimethylsilane, dans un solvant tel que l'acétonitrile à une température comprise entre 22°C et la température d'ébullition du solvant, comme, par exemple, les conditions décrites par Shiao M.J. (J. Org. Chem. (1993), 58(17), 4742-4744.

Les composés Jb peuvent être obtenus à partir d'un composé Hb par réaction avec une morpholine substituée Gb, en l'absence de solvant, ou en présence d'un solvant à une température comprise entre 25°C et la température d'ébullition du solvant, comme décrit par exemple par Aliabiev S.B. (Lett. Org. Chem. (2007), 4(4), 273-280).

Le carboxylate Kb peut être obtenu par hydrolyse de l'ester Jb en présence d'une base telle que la soude ou la lithine, dans un solvant tel que le tétrahydrofuranne ou le méthanol, à une température comprise entre 0°C et 30°C

Les amides (I-a)b peuvent être obtenus à partir du carboxylate Kb par condensation d'une amine NHRbR1b en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Alternativement, les composés (Ib), quand le groupement Ab est une morpholine substituée peuvent être obtenus selon le schéma général (1 B)b.

Schéma Général (1 B)b :
dans lequel les substituants Rb, R1b, R7b, R7'b, R8b, R8'b, R9b R9'b, R10b, R10'b ont les significations indiquées ci-dessus.

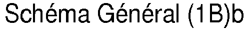

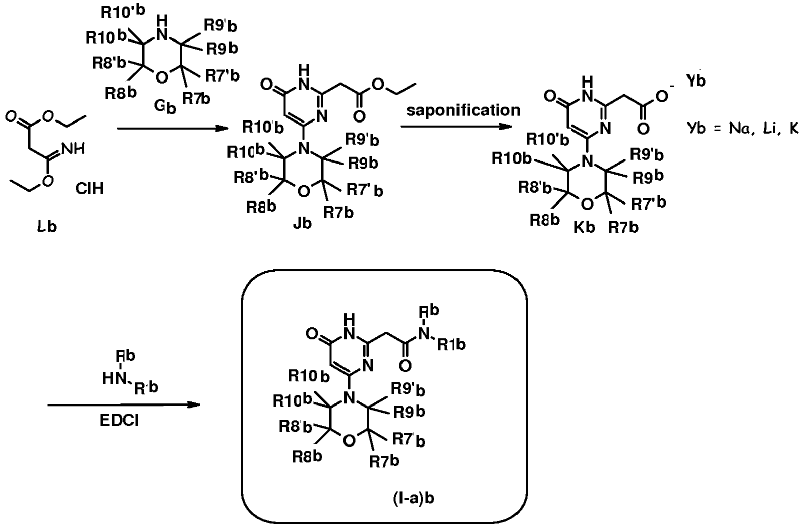

L'ester Jb peut être obtenu par réaction « one-pot » entre la morpholine substituée Gb et un excès (par exemple 3 équivalents) d'imino-éther Lb (ou de son tautomère amino-acrylate), dans un solvant tel que l'éthanol, à une température comprise entre 20°C et le point d'ébullition du solvant.

Le carboxylate Kb peut être obtenu par hydrolyse de l'ester Jb en présence d'une base telle que la soude ou la lithine, dans un solvant tel que le tétrahydrofuranne ou le méthanol, à une température comprise entre 0°C et 30°C

Les amides (I-a)b peuvent être obtenus à partir du carboxylate Kb par condensation d'une amine NHRbR1b en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Alternativement, Les composés (Ib) peuvent être obtenus selon le schéma général (1C)b. 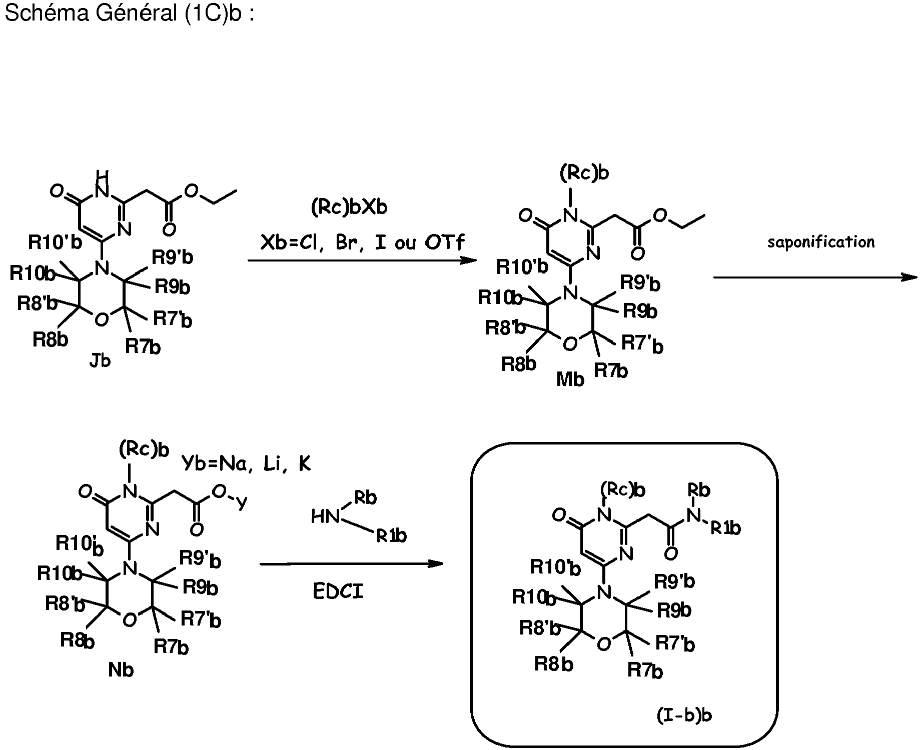 dans lequel les substituants (Rc)b, Rb R1b, R7b, R7'b, R8b, R8'b, R9b, R9'b, R10b, R10'b ont les significations indiquées ci-dessus.

Dans le Schéma Général (1 C)b:
Les esters Mb peuvent être obtenus à partir de l'ester Jb par réaction avec un composé (Rc)b-Xb (Xb= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans (J. Med. Chem. (2008), 51, 5766-5779).

Les carboxylates Nb peuvent être obtenus par hydrolyse des esters Mb, en présence d'une base telle que la soude ou la lithine, dans un solvant tel que le tétrahydrofuranne ou le méthanol, à une température comprise entre 0°C et 30°C

Les amides (I-b)b peuvent être obtenus à partir des carboxylates Nb par condensation d'une amine NHbRbR1b en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Parmi les produits certains sont connus et peuvent être obtenus soit commercialement, soit selon les méthodes usuelles connues de l'homme du métier, par exemple à partir de produits commerciaux.

Lorsque Rb et R1 b forment ensemble un cycle indoline de formule générale (Pb), ces dernières, lorsqu'elles ne sont pas commerciales, peuvent être généralement obtenues à partir des indoles correspondants O1b par réduction en présence d'un agent réducteur tel qu'un hydrure (le cyanoborhydrure de sodium par exemple) au sein d'un solvant tel que l'acide acétique ou l'acide trifluoroacétique, à une température comprise entre -5°C et 25°C, comme décrit par exemple par Kumar, Y. (Synth. Commun., 1983, 13(6), 489). 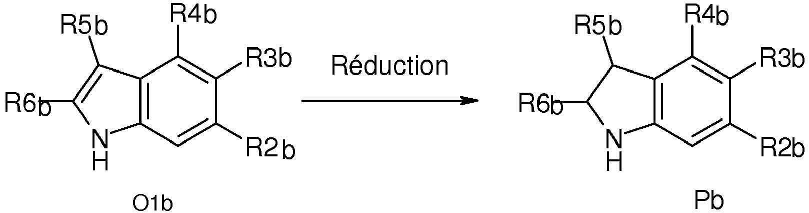 dans lequel les substituants R2b, R3b, R4b, R5b et R6b ont les significations indiquées ci-dessus.

Les indolines énantiomériquement enrichies peuvent être obtenues par exemple par dédoublement chimique des énantiomères à l'aide d'une copule chirale énantiomériquement pure, tel que décrit dans le Schéma Général (1 D)b. Par exemple, le dédoublement des énantiomères de l'indoline Pb peut être réalisé par séparation chromatographique des deux diastéréoisomères formés par couplage de type peptidique avec l'acide *o*-benzyl-D-lactique en présence d'un agent de couplage peptidique tel que, par exemple, l'EDCI (N-[3-(diméthylamino)propyl]-N'-éthylcarbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que le N,N-diméthylformamide, la pyridine, l'éthanol, l'eau ou le méthanol, à une température comprise entre 20°C et 100°C, comme par exemple dans les conditions décrites par Kunishima, M. et Coll. (Tetrahedron, 2001, 57, 1551). Les énantiomères (+)-Pb et (-)-Pb peuvent être respectivement obtenus à partir des composés O2b : dia A et O2b : dia B par réaction en présence d'un acide tel que l'acide chlorhydrique concentré, au sein d'un solvant tel qu'un alcool (l'éthanol par exemple), à une température comprise entre 20°C et la température d'ébullition du solvant, comme, par exemple, dans les conditions décrites par Krasnov, V.P. et Coll. (Mendeleev Commun. 2002, 12(1), 27). 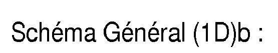 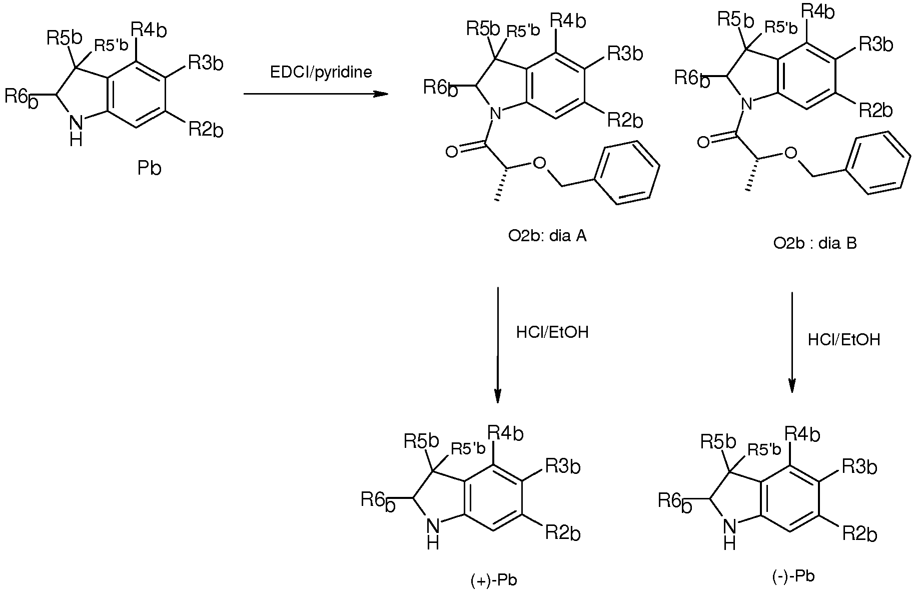 dans lequel les substituants R2b, R3b, R4b, R5b, R5'b et R6b ont les significations indiquées ci-dessus.

Les produits de formule générale (P1b) peuvent notamment être préparés comme indiqué dans le Schéma Général (1 E)b ci-dessous. 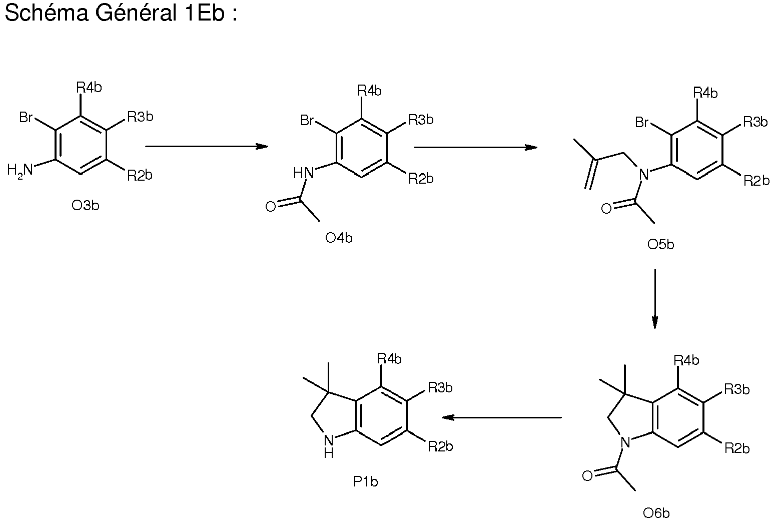 dans lesquels les substituants R2b, R3b et R4b ont les significations indiquées ci-dessus.

Dans le schéma général (1E)b :
Les dérivés O4b peuvent être préparés à partir des dérivés anilines commerciales correspondantes O3b, par réaction d'acétylation avec le chlorure d'acétyle, en présence d'une base telle qu'une amine (la triéthylamine de préférence), au sein d'un solvant tel que le dichlorométhane à une température comprise entre 0°C et la température d'ébullition du solvant.
Les dérivés O5b peuvent être préparés à partir des dérivés anilides O4b, par réaction avec le 3-bromo-2-méthyl-propène, en présence d'une base telle que le carbonate de potassium et d'un hydrure tel que l'hydrure de sodium, au sein d'un solvant tel que le toluène à une température comprise entre 20°C et la température d'ébullition du solvant, par analogie avec les conditions décrites par Edwards, J.P. et coll. (Bioorg. Med. Chem. Lett., 1998, 8, 745).
Les dérivés O6b peuvent être préparés par réaction de cyclisation des dérivés O5b, par exemple en présence d'un catalyseur tel qu'un dérivé de palladium (le diacétate de palladium de préférence), de chlorure de tetrabutylammonium, en présence d'une base telle que la triéthylamine, au sein d'un solvant tel que le N,N-diméthylformamide à une température comprise entre 20°C et la température d'ébullition du solvant, par analogie avec les conditions décrites par Larock, R.C. et coll. (Tetrahedron Lett., 1987, 28, 5291).
Les dérivés P1b peuvent être préparés à partir des dérivés O6b, par réaction en présence d'un acide tel que l'acide chlorhydrique concentré, à une température comprise entre 20°C et la température d'ébullition du solvant, selon les conditions connues de l'homme de l'art.

### Préparation de composés de formule (Ic)

Les produits de formule générale (Ic) selon la présente invention peuvent notamment être préparés comme indiqué dans les Schémas Généraux (1A)c-(1D)c ci-dessous. Les préparations des exemples de la présente invention donnent des illustrations des schémas ci-dessous.

Les procédés de préparation des produits de formule Cc à (I-d)c sont tels que définis dans les Schémas Généraux (1A)c - (1 D)c ci-dessous.

Les procédés de préparation des produits de formule Cc, Dc, Ec et Fc sont tels que définis dans les Schémas Généraux (1A)c - (1 D)c ci-dessous. 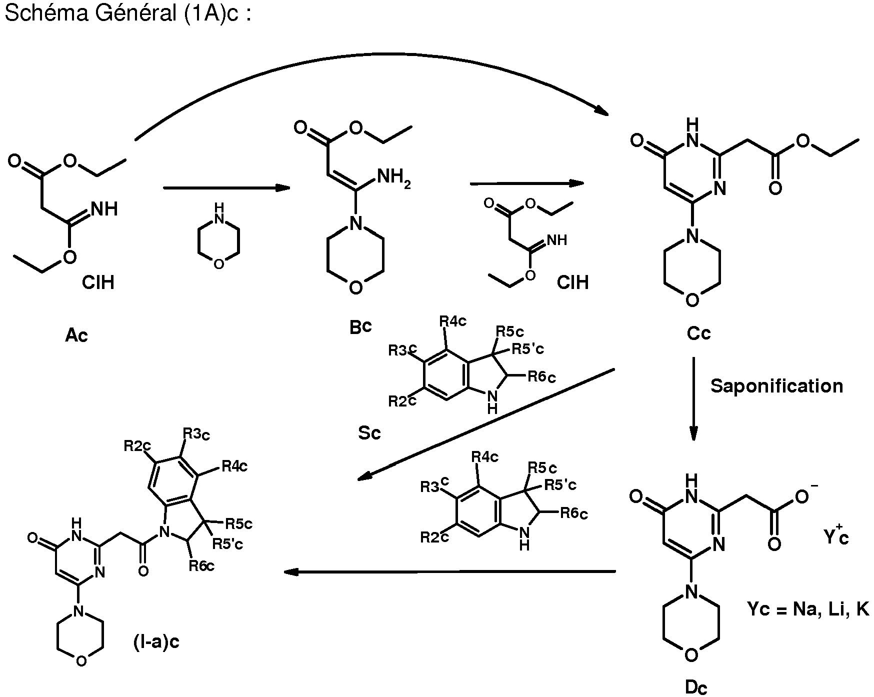 dans lequel les substituants R2c, R3c, R4c, R5c, R5'c et R6c ont les significations indiquées ci-dessus.

Dans le Schéma Général (1 A)c :
Le cétène aminal Bc peut être obtenu à partir de l'imino-éther Ac ou de son tautomère amino-acrylate commercial, par réaction avec la morpholine dans un solvant tel que l'éthanol, à une température comprise entre 0°C et la température d'ébullition du solvant, selon le procédé décrit par Landwehr J. et coll. dans J. Med. Chem. 2006, 49, 4327-4332.

L'ester Cc peut être obtenu par réaction du cétène aminal Bc avec l'imino-éther Ac, ou son tautomère amino-acrylate, dans un solvant tel que l'éthanol, à une température comprise entre 20°C et le point d'ébullition du solvant.

Alternativement, l'ester Cc peut être obtenu par réaction « one-pot » entre la morpholine et un excès (par exemple 3 équivalents) d'imino-éther Ac (ou de son tautomère amino-acrylate), dans un solvant tel que l'éthanol, à une température comprise entre 20°C et le point d'ébullition du solvant.

Le carboxylate Dc peut être obtenu par hydrolyse de l'ester Cc en présence d'une base telle que la soude ou la lithine, dans un solvant tel que le tétrahydrofuranne ou le méthanol, à une température comprise entre 0°C et 30°C

Les amides (I-a)c peuvent être obtenus à partir du carboxylate Dc par condensation d'une indoline Sc en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans Tetrahedron 2001, 57, 1551-1558.

Les amides (I-a)c peuvent également être obtenus à partir de l'ester Cc par réaction d'une indoline Sc en présence d'un agent tel que le triméthyl aluminium ou le tertiobutylate de potassium, dans un solvant tel que le toluène, le tétrahydrofuranne ou la N,N-diméthylformamide, à une température comprise entre 20°C et 150°C, comme par exemple dans les conditions décrites par Perreux L. et Coll. Dans Tetrahedron 2003 (59) 2185-2189 et par Auzeloux, P et coll. dans J. Med. Chem. 2000,43 (2), 190-197. 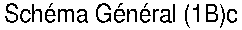 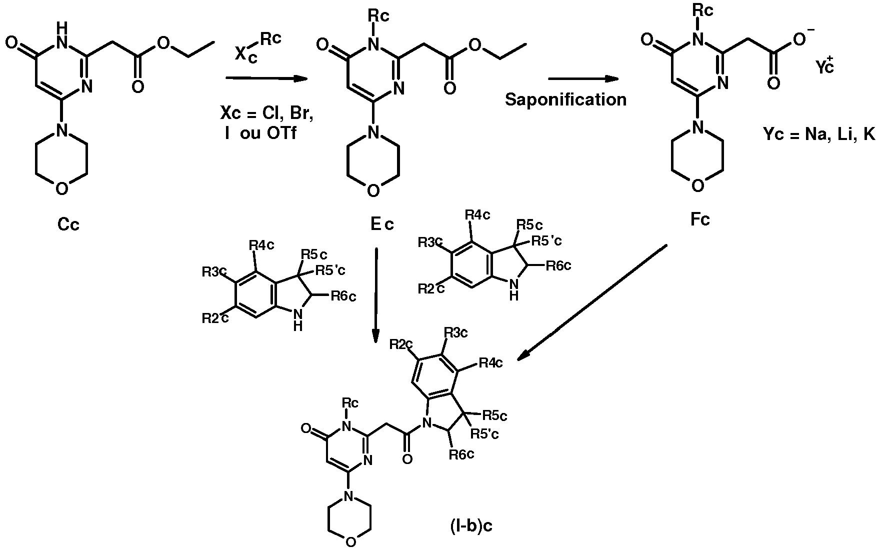 dans lequel les substituants Rc, R2c, R3c, R4c, R5c, R5'c et R6c ont les significations indiquées ci-dessus.

Dans le Schéma Général (1 B)c :
Les esters Ec peuvent être obtenus à partir de l'ester Cc par réaction avec un composé Rc-Xc (Xc= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans J. Med. Chem. 2008, 51, 5766-5779.

Les carboxylates Fc peuvent être obtenus par hydrolyse des esters Ec, en présence d'une base telle que la soude ou la lithine, dans un solvant tel que le tétrahydrofuranne ou le méthanol, à une température comprise entre 0°C et 30°C

Les amides (I-b)c peuvent être obtenus à partir des carboxylates Fc par condensation d'une indoline Sc en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans Tetrahedron 2001, 57, 1551-1558.

Les amides (I-b)c peuvent également être obtenus à partir des esters Ec par réaction d'une indoline Sc, en présence d'un agent tel le triméthyl aluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197. 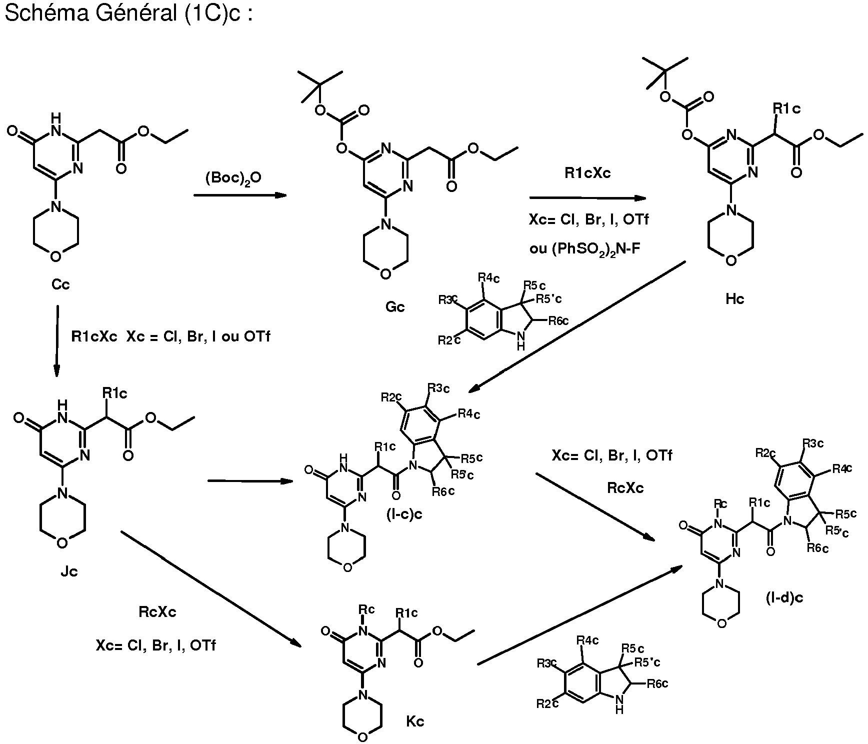 dans lequel les substituants Rc, R1c, R2c, R3c, R4c, R5c, R5'c et R6c ont les significations indiquées ci-dessus.

Dans le Schéma Général (1 C)c :
l'ester Gc peut être obtenu à partir de l'ester Cc par réaction avec le (Boc)2O (dicarbonate de tertiobutyle), dans un solvant tel que la N,N-diméthylformamide, le dioxanne, l'acétonitrile ou le dichlorométhane, en présence d'une base comme par exemple, l'hydrure de sodium, la triéthylamine, la N,N-diisopropyléthylamine ou la pyridine, à une température comprise entre 0°C et 60°C, selon par exemple le procédé décrit par Hioki K. et Coll. Synthesis 2006, 12, 1931-1933

Les produits Hc peuvent être obtenus à partir de l'ester Gc par réaction avec R1c-Xc (Xc = Cl, Br, I ou OTf et R2c et R3c sont des groupes alkyles), en présence d'une base telle que la soude, le tertiobutylate de potassium ou le carbonate de césium, dans un solvant tel que le méthanol, l'éthanol ou le dioxanne, à une température comprise entre 0°C et 100°C, selon par exemple le procédé décrit par Noël D. D'Angelo et coll. dans J. Med. Chem. 2008, 51, 5766-5779.

Le produit Hc où R1c=F peut être obtenu par réaction du produit Gc avec la N-fluorobenzènesulfonimide, en présence d'une base telle que le sel de potassium de l'héxaméthyl-disilylazane, dans un solvant tel que le tétrahydrofuranne, à une température comprise entre -78°C et 20°C, selon par exemple le procédé décrit par Christopher S. Burgey et Coll. Dans J. Med. Chem. 2003, 46, 461-473.

Les esters Jc où le groupe R1c est un radical alkyle peuvent être obtenus à partir de l'ester Cc de la même façon que les produits Hc, en présence d'une base telle que le butyllithium, l'hydrure de sodium, le tertiobutylate de potassium ou le carbonate de césium dans un solvant tel que le méthanol, l'éthanol, le tétrahydrofurane, le N,N-diméthylformamide ou le dioxanne, à une température comprise entre 0°C et 50°C.

Les amides (I-c)c peuvent être obtenus à partir des esters Hc ou Jc par réaction d'une indoline Sc, en présence d'un agent tel le triméthyl aluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197.

Les amides (I-d)c peuvent être obtenus à partir des amides (I-c)c par réaction avec un composé Rc-Xc (Xc= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noel D. D'Angelo et coll. dans J. Med. Chem. 2008, 51, 5766-5779.

Alternativement, les amides (I-d)c peuvent être obtenus à partir des esters Kc par réaction d'une indoline Sc, en présence d'un agent tel le triméthylaluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197.

Les esters Kc peuvent être obtenus à partir des esters Jc par réaction avec un composé Rc-Xc (Xc= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans J. Med. Chem. 2008, 51, 5766-5779. 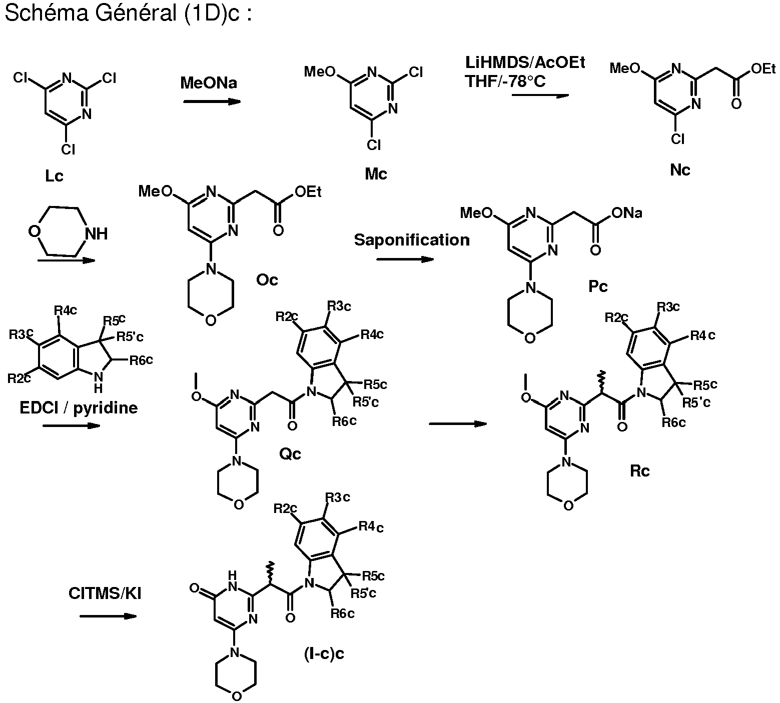 dans lequel les substituants R2c, R3c, R4c, R5c, R5'c et R6c ont les significations indiquées ci-dessus.

Dans le Schéma Général (1 D)c :
Le dérivé méthoxypyrimidine Mc est préparé à partir du composé Lc par réaction avec le méthylate de sodium dans un solvant tel que le THF ou le méthanol à une température comprise entre 0°C et 25°C, selon, par exemple, les conditions décrites par loannidis, S. et coll. (Bioorganic and Medicinal Chemistry Letters, (2010), 20(5), 1669 - 1673).

Le composé Nc peut être obtenu par traitement du composé Mc en présence de l'acétate d'éthyle avec la LiHMDS (lithium bis(trimethylsilyl)amide) dans un solvant tel que le THF à une température comprise entre -78°C et 25°C, selon, par exemple, les conditions décrites par Chekmarev D.S. et coll. Tetrahedron, (2006), 62(42), 9919-9930.

Le composé Oc peut être obtenu à partir du composé Nc par réaction avec la morpholine, en l'absence de solvant, ou en présence d'un solvant à une température comprise entre 25°C et la température d'ébullition du solvant, comme décrit par exemple par Aliabiev S.B. (Lett. Org. Chem. (2007), 4(4), 273-280).

Le carboxylate Pc peut être obtenu par hydrolyse de l'ester Oc en présence d'une base telle que la soude ou la lithine, dans un solvant tel que le tétrahydrofuranne ou le méthanol, à une température comprise entre 0°C et 30°C

L' amide Qc peut être obtenu à partir du carboxylate Ec par condensation d'une indoline Sc en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Le composé Rc peut-être obtenu à partir de Qc en utilisant une base telle que de l'hydrure de sodium dans du tétrahydrofuranne entre 0°C et 40°C.

Les amides (I-c)c peuvent être obtenus à partir du composé Rc par réaction avec le chlorotrimethylsilane et l'iodure de potassium ou l'iodotrimethylsilane, dans un solvant tel que l'acétonitrile à une température comprise entre 22°C et la température d'ébullition du solvant, comme, par exemple, les conditions décrites par Shiao M.J. (J. Org. Chem. (1993), 58(17), 4742-4744.

Parmi les produits de départs de formule Lc ou Mc certains sont connus et peuvent être obtenus soit commercialement, soit selon les méthodes usuelles connues de l'homme du métier, par exemple à partir de produits commerciaux.

Les indolines de formule générale Sc, lorsqu'elles ne sont pas commerciales, peuvent être généralement obtenues à partir des indoles correspondants T1c par réduction en présence d'un agent réducteur tel qu'un hydrure (le cyanoborhydrure de sodium par exemple) au sein d'un solvant tel que l'acide acétique ou l'acide trifluoroacétique, à une température comprise entre -5°C et 25°C, comme décrit par exemple par Kumar, Y. (Synth. Commun., 1983, 13(6), 489). 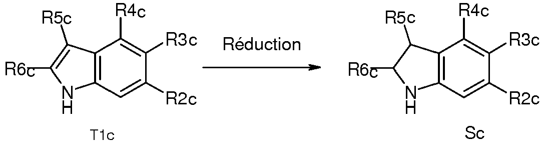 dans lequel les substituants R2c, R3c, R4c, R5c et R6c ont les significations indiquées ci-dessus.

Les indolines énantiomériquement enrichies peuvent être obtenues par exemple par dédoublement chimique des énantiomères à l'aide d'une copule chirale énantiomériquement pure, tel que décrit dans le Schéma Général (1 E)c. Par exemple, le dédoublement des énantiomères de l'indoline Sc peut être réalisé par séparation chromatographique des deux diastéréoisomères formés par couplage de type peptidique avec l'acide *o*-benzyl-D-lactique en présence d'un agent de couplage peptidique tel que, par exemple, l'EDCI (N-[3-(diméthylamino)propyl]-N'-éthylcarbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que le N,N-diméthylformamide, la pyridine, l'éthanol, l'eau ou le méthanol, à une température comprise entre 20°C et 100°C, comme par exemple dans les conditions décrites par Kunishima, M. et Coll. (Tetrahedron, 2001, 57, 1551). Les énantiomères (+)-Sc et (-)-Sc peuvent être respectivement obtenus à partir des composés T2c : dia A et T2 : dia Bc par réaction en présence d'un acide tel que l'acide chlorhydrique concentré, au sein d'un solvant tel qu'un alcool (l'éthanol par exemple), à une température comprise entre 20°C et la température d'ébullition du solvant, comme, par exemple, dans les conditions décrites par Krasnov, V.P. et Coll. (Mendeleev Commun. 2002, 12(1), 27). 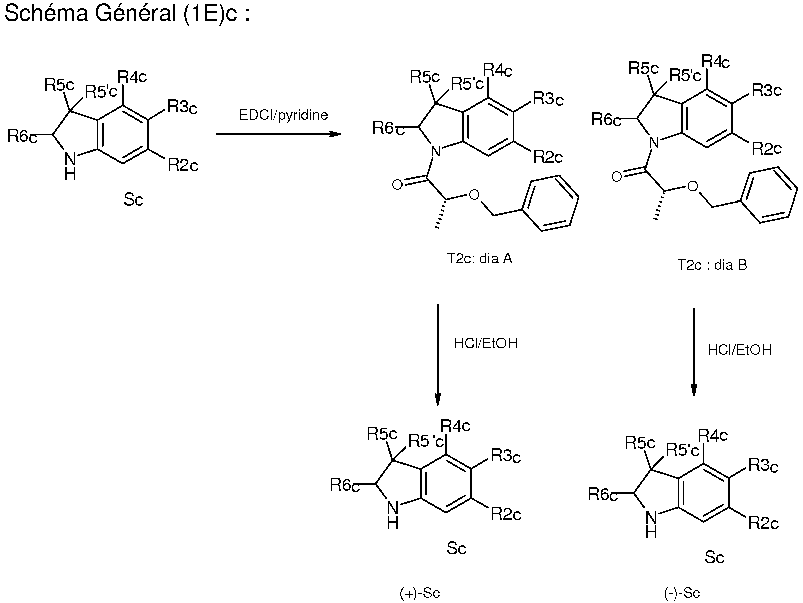 dans lequel les substituants R2c, R3c, R4c, R5c, R5'c et R6c ont les significations indiquées ci-dessus.

Les produits de formule générale (T1-a)c peuvent notamment être préparés comme indiqué dans le Schéma Général (1 F)c ci-dessous. 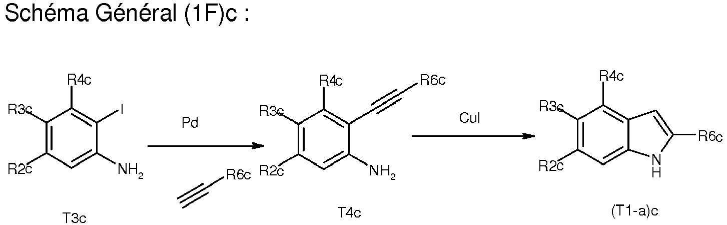 dans lequel les substituants R2c, R3c, R4c et R6c ont les significations indiquées ci-dessus.

Dans le schéma général (1 F)c :
Les dérivés T4c peuvent être préparés à partir des composés T3c par couplage de type Sonogashira avec un alcyne vrai en présence d'un catalyseur à base de palladium et d'iodure de cuivre dans un solvant tel que la triéthylamine à une température comprise entre 0°C et le point d'ébullition du solvant selon, par exemple, les conditions décrites par Kuyper, F. et coll. (J. Med. Chem., 1996, 39(4), 892).

Les indoles (T1-a)c peuvent être obtenus par cyclisation des composés T4c en présence d'iodure de cuivre dans un solvant tel que le DMF à une température comprise entre 22°C et la température d'ébullition du solvant selon, par exemple, les conditions décrites par Kuyper, F. et coll. (J. Med. Chem., 1996, 39(4), 892).

Alternativement, les dérivés de formule générale (T1-b)c peuvent être obtenus selon le schéma général (1 G)c ci-dessous, à partir des acides indole-2-carboxyliques commerciaux correspondants T5c, par réaction de décarboxylation. Cette réaction est effectuée préférentiellement à une température comprise entre 200°C et 240°C, en présence de cuivre(0) et de quinoléine selon, par exemple, les conditions décrites par Tapia, R.A. et coll. (Bioorg. Med. Chem., 2003, 11, 3407). 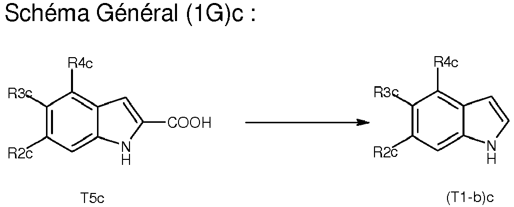 dans lequel les substituants R2c, R3c et R4c ont les significations indiquées ci-dessus.

Alternativement, les dérivés de formule générale (T1-c)c peuvent être obtenus selon le schéma général (1 H)c ci-dessous, par analogie avec les conditions décrites dans le brevet US 2004/0224973 A1. 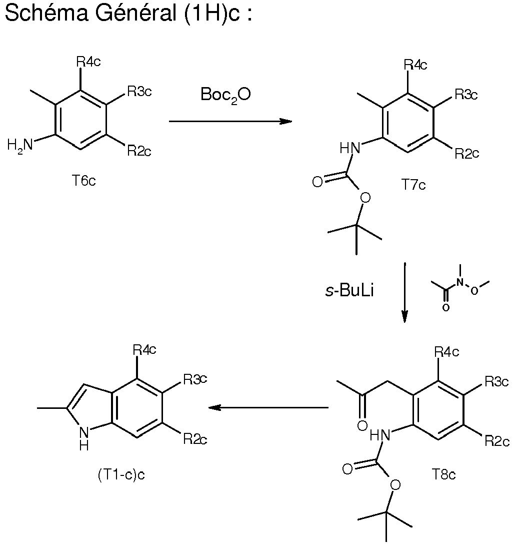 dans lequel les substituants R2c, R3c et R4c ont les significations indiquées ci-dessus.

Dans le schéma général (1H)c :
Les dérivés T7c peuvent être préparés à partir des dérivés anilines commerciales correspondantes T6c, par réaction d'acylation avec le di-*tert*-butyl-dicarbonate, au sein d'un solvant tel que le tetrahydrofuranne à une température comprise entre 20°C et la température d'ébullition du solvant.
Les dérivés T8c peuvent être préparés à partir des dérivés carbamates T7c, par réaction en présence d'une base telle que le sec-butyllithium, puis de N-méthoxy-N-méthyl-acétamide, au sein d'un solvant tel que le tetrahydrofuranne à une température comprise entre -50°C et -10°C.
Les dérivés (T1-c)c peuvent être préparés à partir des dérivés T8c, par réaction en présence d'un acide tel que l'acide trifluoroacétique, au sein d'un solvant tel que le dichlorométhane, à une température comprise entre 0°C et 20°C.

Alternativement, les dérivés de formule générale S1c peuvent être obtenus selon le schéma général (1I)c ci-dessous. 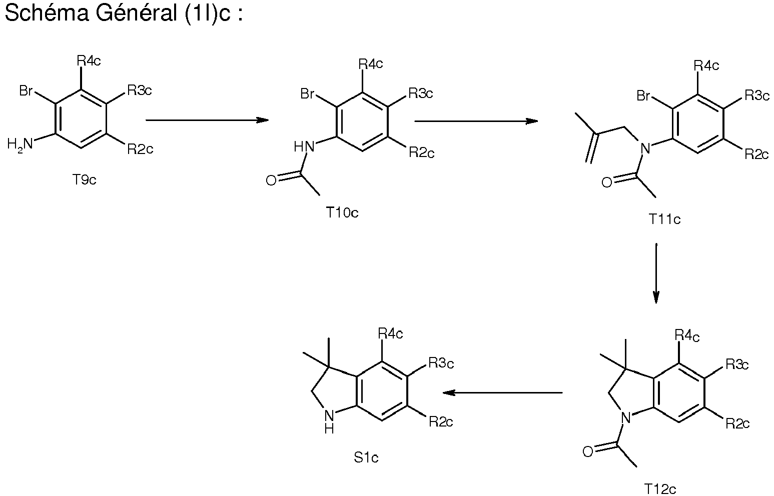 dans lequel les substituants R2c, R3c et R4c ont les significations indiquées ci-dessus.

Dans le schéma général (1I)c :
Les dérivés T10c peuvent être préparés à partir des dérivés anilines commerciales correspondantes T9c, par réaction d'acétylation avec le chlorure d'acétyle, en présence d'une base telle qu'une amine (la triéthylamine de préférence), au sein d'un solvant tel que le dichlorométhane à une température comprise entre 0°C et la température d'ébullition du solvant.
Les dérivés T11 c peuvent être préparés à partir des dérivés anilides T10c, par réaction avec le 3-bromo-2-méthyl-propène, en présence d'une base telle que le carbonate de potassium et d'un hydrure tel que l'hydrure de sodium, au sein d'un solvant tel que le toluène à une température comprise entre 20°C et la température d'ébullition du solvant, par analogie avec les conditions décrites par Edwards, J.P. et coll. (Bioorg. Med. Chem. Lett., 1998, 8, 745).
Les dérivés T12c peuvent être préparés par réaction de cyclisation des dérivés T11 c, par exemple en présence d'un catalyseur tel qu'un dérivé de palladium (le diacétate de palladium de préférence), de chlorure de tetrabutylammonium, en présence d'une base telle que la triéthylamine, au sein d'un solvant tel que le N,N-diméthylformamide à une température comprise entre 20°C et la température d'ébullition du solvant, par analogie avec les conditions décrites par Larock, R.C. et coll. (Tetrahedron Lett., 1987, 28, 5291).
Les dérivés S1c peuvent être préparés à partir des dérivés T12c, par réaction en présence d'un acide tel que l'acide chlorhydrique concentré, à une température comprise entre 20°C et la température d'ébullition du solvant, selon les conditions connues de l'homme de l'art.

### Préparation de composés de formule (Id)

Les produits de formule générale (Id) selon la présente invention peuvent notamment être préparés comme indiqué dans les Schémas Généraux (1A)d-(1C)d ci-dessous. Les préparations des exemples de la présente invention donnent des illustrations des schémas ci-dessous.

Les procédés de préparation des produits de formule Cd à (I-d)d sont tels que définis dans les Schémas Généraux (1 A)d, (1 B)d, (1C)d et (1 K)d ci-dessous. 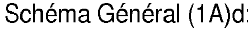 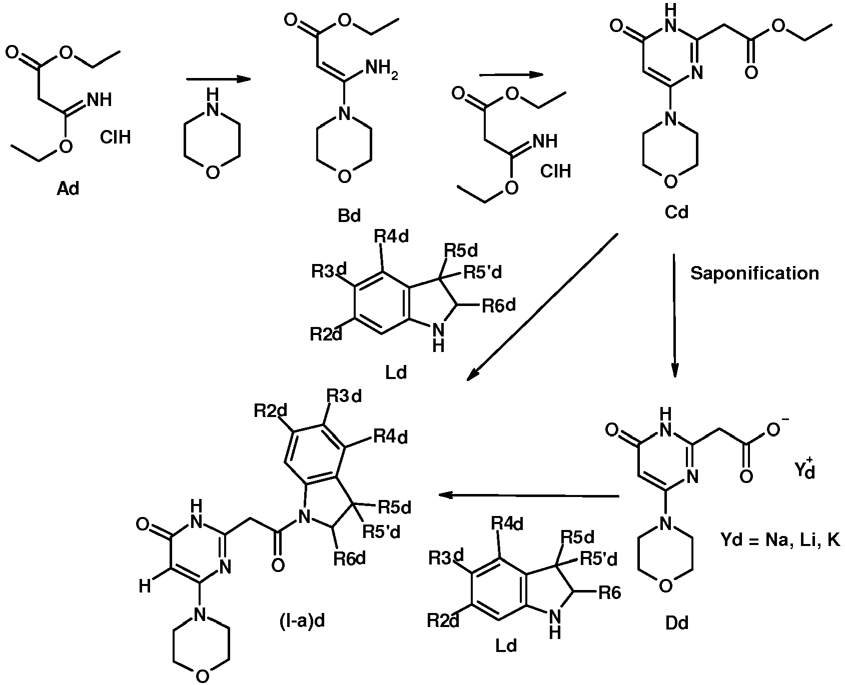 dans lequel les substituants R2d, R3d, R4d, R5d, R5'd et R6d ont les significations indiquées ci-dessus.

Dans le Schéma Général (1A)d :
Le cétène aminal Bd peut être obtenu à partir de l'imino-éther Ad ou de son tautomère amino-acrylate commercial, par réaction avec la morpholine dans un solvant tel que l'éthanol, à une température comprise entre 0°C et la température d'ébullition du solvant, selon le procédé décrit par Landwehr J. et coll. dans J. Med. Chem. 2006, 49, 4327-4332.

L'ester Cd peut être obtenu par réaction du cétène aminal Bd avec l'imino-éther Ad, ou son tautomère amino-acrylate, dans un solvant tel que l'éthanol, à une température comprise entre 20°C et le point d'ébullition du solvant.

Alternativement, l'ester Cd peut être obtenu par réaction « one-pot » entre la morpholine et un excès (par exemple 3 équivalents) d'imino-éther Ad (ou de son tautomère amino-acrylate), dans un solvant tel que l'éthanol, à une température comprise entre 20°C et le point d'ébullition du solvant.

Le carboxylate Dd peut être obtenu par hydrolyse de l'ester Cd en présence d'une base telle que la soude ou la lithine, dans un solvant tel que le tétrahydrofuranne ou le méthanol, à une température comprise entre 0°C et 30°C

Les amides (I-a)d peuvent être obtenus à partir du carboxylate Dd par condensation d'une indoline Ld en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans Tetrahedron 2001, 57, 1551-1558.

Les amides (I-a)d peuvent également être obtenus à partir de l'ester Cd par réaction d'une indoline Ld en présence d'un agent tel que le triméthyl aluminium ou le tertiobutylate de potassium, dans un solvant tel que le toluène, le tétrahydrofuranne ou la N,N-diméthylformamide, à une température comprise entre 20°C et 150°C, comme par exemple dans les conditions décrites par Perreux L. et Coll. Dans Tetrahedron 2003 (59) 2185-2189 et par Auzeloux, P et coll. dans J. Med. Chem. 2000,43 (2), 190-197. 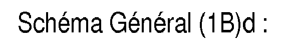 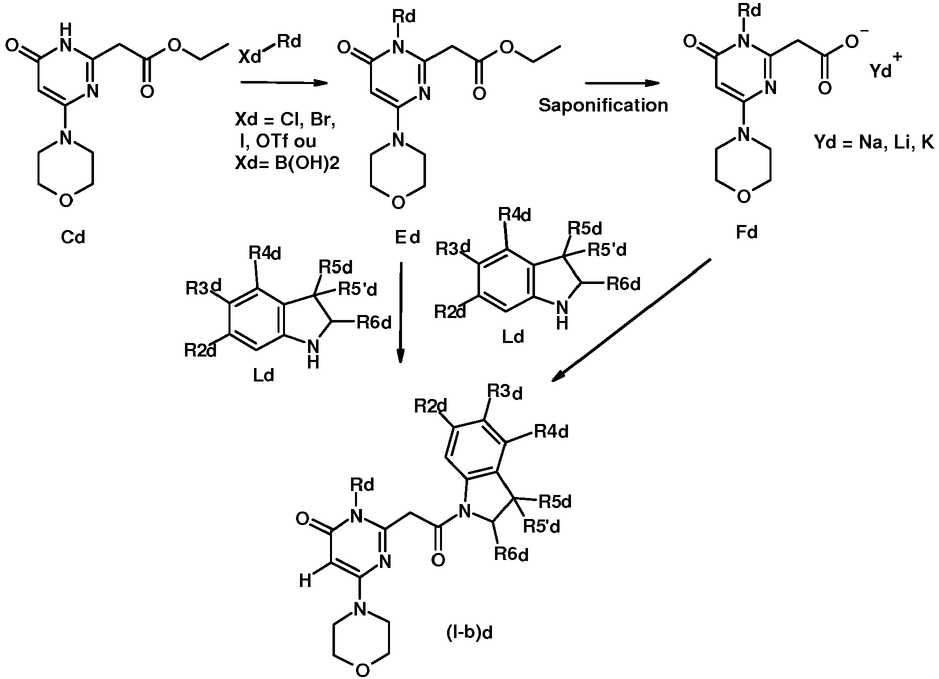 dans lequel les substituants Rd, R2d, R3d, R4d, R5d, R5'd et R6d ont les significations indiquées ci-dessus.

Dans le Schéma Général (1B)d :
Les esters Ed peuvent être obtenus à partir de l'ester Cd par réaction avec un composé Rd-Xd (Xd= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans J. Med. Chem. 2008, 51, 5766-5779.

Les esters Ed peuvent également être obtenus à partir de l'ester Cd par réaction avec un composé R5d-Xd (Xd= B(OH)2), en présence d'une base telle que le DMAP (diméthyl-pyridin-4-yl-amine) et NaHMDS (sel de soudium de 1,1,1,3,3,3-héxamethyl-disilazane), d'un sel de cuivre (II) tel que l'acétate de cuivre (Cu(OAc)₂) dans un solvant tel que le toluène, le dioxanne ou le tétrahydrofuranne, à une température comprise entre 0°C et la température d'ébullition du solvant, selon par exemple le procédé décrit par Takayuki Tsuritani et coll. (Organic Letters 2008, 10(8), 1653-1655).

Les carboxylates Fd peuvent être obtenus par hydrolyse des esters Ed, en présence d'une base telle que la soude ou la lithine, dans un solvant tel que le tétrahydrofuranne ou le méthanol, à une température comprise entre 0°C et 30°C

Les amides (I-b)d peuvent être obtenus à partir des carboxylates Fd par condensation d'une indoline Ld en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans Tetrahedron 2001, 57, 1551-1558.

Les amides (I-b)d peuvent également être obtenus à partir des esters Ed par réaction d'une indoline Ld, en présence d'un agent tel le triméthyl aluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197. 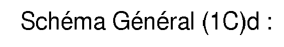 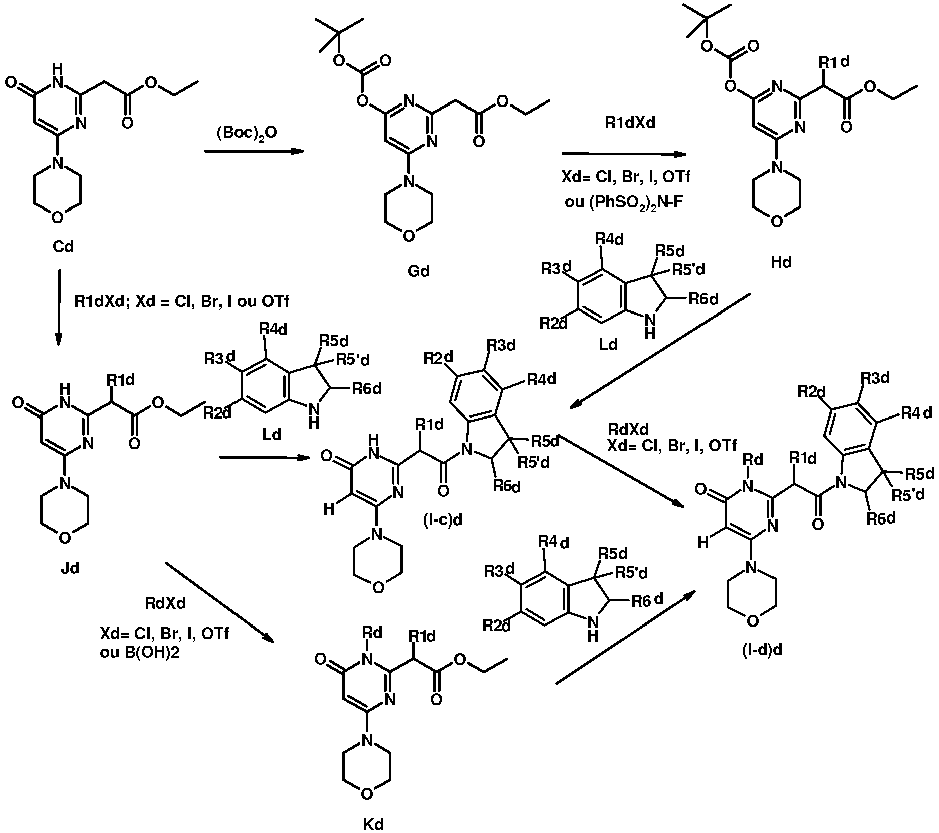 dans lequel les substituants Rd, R1d, R2d, R3d, R4d, R5d, R5'd et R6d ont les significations indiquées ci-dessus.

Dans le Schéma Général (1C)d :
L'ester Gd peut être obtenu à partir de l'ester Cd par réaction avec le (Boc)₂O (dicarbonate de tertiobutyle), dans un solvant tel que la N,N-diméthylformamide, le dioxanne, l'acétonitrile ou le dichlorométhane, en présence d'une base comme par exemple, l'hydrure de sodium, la triéthylamine, la N,N-diisopropyléthylamine ou la pyridine, à une température comprise entre 0°C et 60°C, selon par exemple le procédé décrit par Hioki K. et Coll. Synthesis 2006, 12, 1931-1933

Les produits Hd peuvent être obtenus à partir de l'ester Gd par réaction avec R1d-Xd (Xd = Cl, Br, I ou OTf et R1d est un groupe alkyle), en présence d'une base telle que la soude, le tertiobutylate de potassium ou le carbonate de césium, dans un solvant tel que le méthanol, l'éthanol ou le dioxanne, à une température comprise entre 0°C et 100°C, selon par exemple le procédé décrit par Noël D. D'Angelo et coll. dans J. Med. Chem. 2008, 51, 5766-5779.

Le produit Hd où R1d = F peut être obtenu par réaction du produit Gd avec la N-fluorobenzènesulfonimide, en présence d'une base telle que le sel de potassium de l'héxaméthyl-disilylazane, dans un solvant tel que le tétrahydrofuranne, à une température comprise entre -78°C et 20°C, selon par exemple le procédé décrit par Christopher S. Burgey et Coll. Dans J. Med. Chem. 2003, 46, 461-473.

Les esters Jd où le groupe R1d est un radical alkyle peut être obtenu à partir de l'ester Cd de la même façon que les produits Hd, en présence d'une base telle que le butyllithium, l'hydrure de sodium, le tertiobutylate de potassium ou le carbonate de césium dans un solvant tel que le méthanol, l'éthanol, le tétrahydrofurane, le N,N-diméthylformamide ou le dioxanne, à une température comprise entre 0°C et 50°C.

Les amides (I-c)d peuvent être obtenus à partir des esters Hd ou Jd par réaction d'une indoline Ld, en présence d'un agent tel le triméthyl aluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197.

Les amides (I-d)d peuvent être obtenus à partir des amides (I-c)d par réaction avec un composé Rd-Xd (Xd= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noel D. D'Angelo et coll. dans J. Med. Chem. 2008, 51, 5766-5779.

Alternativement, les amides (I-d)d peuvent être obtenus à partir des esters Kd par réaction d'une indoline Ld, en présence d'un agent tel le triméthylaluminium, dans un solvant tel que le toluène, à une température comprise entre 20°C et le point d'ébullition du solvant, comme par exemple dans les conditions décrites par Auzeloux, P et coll. dans J. Med. Chem. 2000,43 (2), 190-197.

Les esters Kd peuvent être obtenus à partir des esters Jd par réaction avec un composé Rd-Xd (Xd= Cl, Br, I, triflate ou boronate B(OH)2), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans J. Med. Chem. 2008, 51, 5766-5779.

Parmi les produits de départs de formule Ad ou Bd certains sont connus et peuvent être obtenus soit commercialement, soit selon les méthodes usuelles connues de l'homme du métier, par exemple à partir de produits commerciaux.

Les indolines de formule générale (Ld), lorsqu'elles ne sont pas commerciales, peuvent être généralement obtenues à partir des indoles correspondants M1d par réduction en présence d'un agent réducteur tel qu'un hydrure (le cyanoborhydrure de sodium par exemple) au sein d'un solvant tel que l'acide acétique ou l'acide trifluoroacétique, à une température comprise entre -5°C et 25°C, comme décrit par exemple par Kumar, Y. (Synth. Commun., 1983, 13(6), 489). 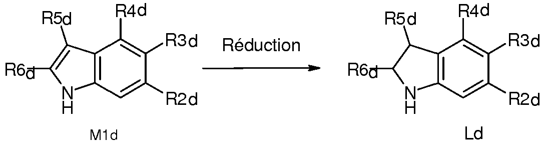 dans lequel les substituants R2d, R3d, R4d, R5d, et R6d ont les significations indiquées ci-dessus.

Les indolines énantiomériquement enrichies peuvent être obtenues par exemple par dédoublement chimique des énantiomères à l'aide d'une copule chirale énantiomériquement pure, tel que décrit dans le Schéma Général (1 D)d. Par exemple, le dédoublement des énantiomères de l'indoline Ld peut être réalisé par séparation chromatographique des deux diastéréoisomères formés par couplage de type peptidique avec l'acide *o*-benzyl-D-lactique en présence d'un agent de couplage peptidique tel que, par exemple, l'EDCI (N-[3-(diméthylamino)propyl]-N'-éthylcarbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que le N,N-diméthylformamide, la pyridine, l'éthanol, l'eau ou le méthanol, à une température comprise entre 20°C et 100°C, comme par exemple dans les conditions décrites par Kunishima, M. et Coll. (Tetrahedron, 2001, 57, 1551). Les énantiomères (+)-Ld et (-)-Ld peuvent être respectivement obtenus à partir des composés M2d : dia A et M2d : dia B par réaction en présence d'un acide tel que l'acide chlorhydrique concentré, au sein d'un solvant tel qu'un alcool (l'éthanol par exemple), à une température comprise entre 20°C et la température d'ébullition du solvant, comme, par exemple, dans les conditions décrites par Krasnov, V.P. et Coll. (Mendeleev Commun. 2002, 12(1), 27). 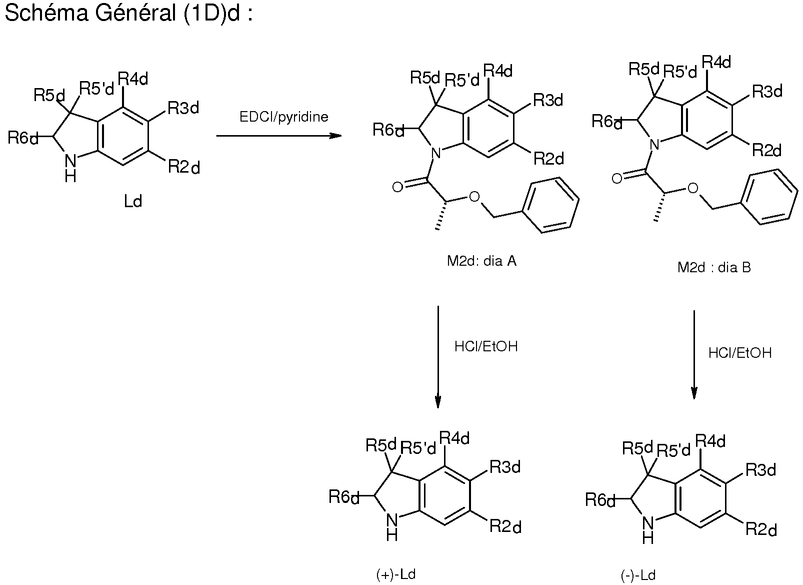 dans lequel les substituants R2d, R3d, R4d, R5d, R5'd et R6d ont les significations indiquées ci-dessus.

Les produits de formule générale (M1-a)d peuvent notamment être préparés comme indiqué dans le Schéma Général (1 E)d ci-dessous, à partir des composés M3d par couplage de type Suzuki avec un acide arylboronique ou un arylboronate en présence d'un catalyseur à base de palladium (le dichlorobis(tri-*o*-tolylphosphine)palladium(II) par exemple), d'une base telle que le carbonate de potassium dans un solvant tel qu'un mélange de dioxanne et d'eau à une température comprise entre 20°C et le point d'ébullition du solvant, ou alternativement avec un acide arylboronique ou un arylboronate en présence d'un catalyseur tel que le [1,1'-bis(diphénylphosphino)ferrocène]dichloropalladium(II), de chlorure de cuivre(I), d'une base telle que le carbonate de césium dans un solvant tel que le N,N-diméthylformamide à une température comprise entre 20°C et le point d'ébullition du solvant (éventuellement sous irradiation par micro-ondes), selon par exemple les conditions décrites dans le brevet US 2007/0072897 A1. 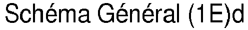 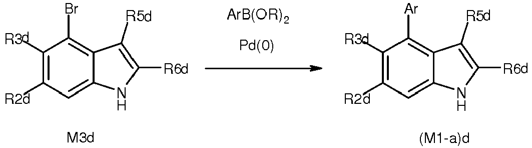 dans lequel les substituants R2d, R3d, R5d et R6d ont les significations indiquées ci-dessus.

Alternativement, les dérivés de formule générale (M1-b)d peuvent être obtenus selon le schéma général (1F)d ci-dessous, à partir des acides indole-2-carboxyliques commerciaux correspondants M4d, par réaction de décarboxylation. Cette réaction est effectuée préférentiellement à une température comprise entre 200°C et 240°C, en présence de cuivre(0) et de quinoléine selon, par exemple, les conditions décrites par Tapia, R.A. et coll. (Bioorg. Med. Chem., 2003, 11, 3407). 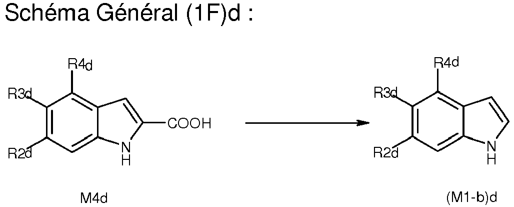 dans lequel les substituants R2d, R3d et R4d ont les significations indiquées ci-dessus.

Alternativement, les dérivés de formule générale (M1-c)d peuvent être obtenus selon le schéma général (1 G)d ci-dessous, par analogie avec les conditions décrites par Katayama, M. et coll. (Biosci. Biotechnol. Biochem., 2008, 72(8), 2025). 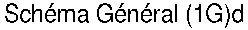 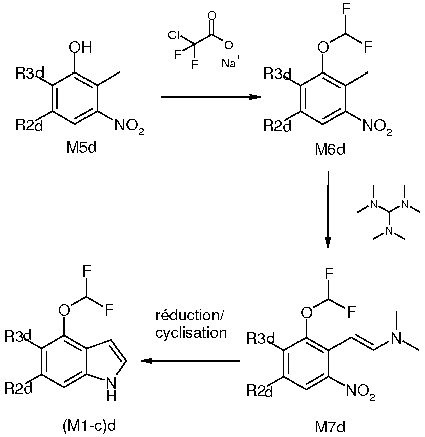 dans lequel les substituants R2d et R3d ont les significations indiquées ci-dessus.

Dans le schéma général (1G)d :
Les dérivés M6d peuvent être préparés à partir des dérivés commerciaux correspondants M5d, par réaction avec le chlorodifluoroacétate de sodium, en présence d'une base telle que le carbonate de potassium, au sein d'un solvant tel qu'un mélange de N,N-diméthylformamide et d'eau à une température comprise entre 20°C et la température d'ébullition du solvant, par analogie avec les conditions décrites dans le brevet WO2009/92590.

Les dérivés M7d peuvent être préparés à partir des dérivés M6d, par réaction avec le tris(diméthylamino) méthane, au sein d'un solvant tel que le N,N-diméthylformamide, à une température comprise entre 20°C et la température d'ébullition du solvant, par analogie avec les conditions décrites par Hume, W.E. et coll. (Tetrahedron, 2002, 58, 3605).

Les dérivés (M1-c)d peuvent être préparés à partir des dérivés M7d, par réduction et cyclisation en présence d'hydrate d'hydrazine, d'un catalyseur tel que le nickel de Raney, au sein d'un solvant tel qu'un mélange de méthanol et de tetrahydrofuranne, à une température comprise entre 20°C et la température d'ébullition du solvant, par analogie avec les conditions décrites par Hume, W.E. et coll. (Tetrahedron, 2002, 58, 3605)*.*

Alternativement, les dérivés de formule générale (M1-d)d dans lesquels Xd représente un groupement SO2Alk ou Alk peuvent être obtenus selon le schéma général (1 H)d ci-dessous, à partir des aldéhydes commerciaux correspondants M8d, par réaction d'amination réductrice en présence des pipérazines commerciales correspondantes et d'un agent réducteur tel que le triacétoxyborhydrure de sodium, au sein d'un solvant tel que le tetrahydrofuranne, à une température comprise entre 20°C et et la température d'ébullition du solvant, selon par exemple les conditions décrites dans le brevet WO2007/113249 A2. 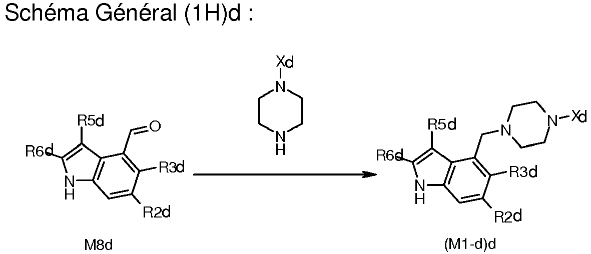 dans lequel les substituants R2d, R3d, R5d et R6d ont les significations indiquées ci-dessus.

Alternativement, les dérivés de formule générale (M1-e)d peuvent être obtenus selon le schéma général (1I)d ci-dessous, à partir des dérivés 4-hydroxy-2-méthyl-indoles commerciaux correspondants M9d, par réaction avec du fréon-22, en présence d'un agent de transfert de phase tel que le bromure de tetrabutylammonium et d'une base telle que la soude, au sein d'un solvant tel que le dichlorométhane, à une température comprise entre 0°C et et la température ambiante, selon par exemple les conditions décrites dans le brevet WO2006/019831. 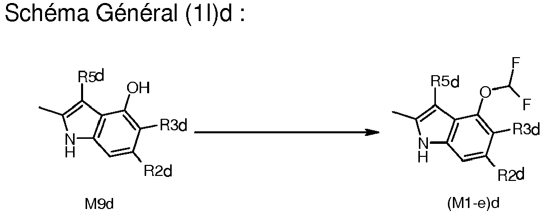 dans lequel les substituants R2d, R3d et R5d ont les significations indiquées ci-dessus.

Les produits de formule générale (M1-f)d peuvent notamment être préparés comme indiqué dans le Schéma Général (1J)d ci-dessous. 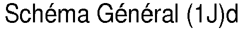 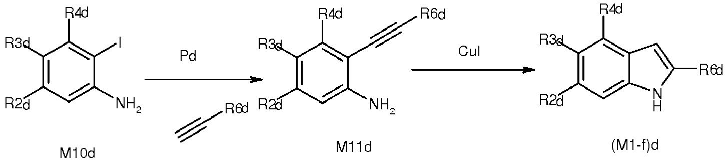 dans lequel les substituants R2d, R3d, R4d et R6d ont les significations indiquées ci-dessus.

Dans le schéma général (1J)d :
Les dérivés M11d peuvent être préparés à partir des composés M10d par couplage de type Sonogashira avec un alcyne vrai en présence d'un catalyseur à base de palladium tel que le dichlorure de bis(triphénylphosphine)palladium(II) et d'iodure de cuivre dans un solvant tel que la triéthylamine et optionnellement en présence d'un co-solvant tel que le N,N-diméthylformamide, à une température comprise entre 0°C et le point d'ébullition du solvant selon, par exemple, les conditions décrites par Kuyper, F. et coll. (J. Med. Chem., 1996, 39(4), 892).

Les indoles (M1-f)d peuvent être obtenus par cyclisation des composés M11d en présence d'iodure de cuivre dans un solvant tel que le DMF à une température comprise entre 22°C et la température d'ébullition du solvant selon, par exemple, les conditions décrites par Kuyper, F. et coll. (J. Med. Chem., 1996, 39(4), 892). 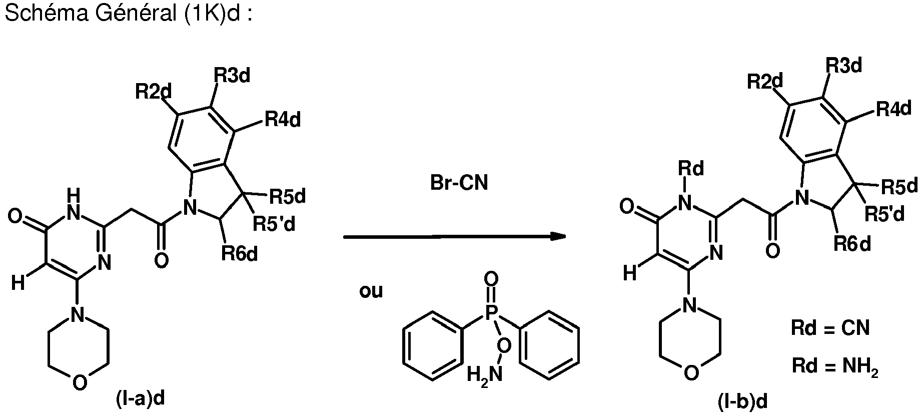 dans lequel les substituants Rd, R2d, R3d, R4d, R5d, R5'd et R6d ont les significations indiquées ci-dessus.

Dans le Schéma Général (1K)d :
Les amides (I-b)d peuvent être obtenus à partir des amides (I-a)d par réaction avec le bromure de cyanogène (Br-CN) en présence d'une base telle que la soude, le tertiobutylate de potassium ou le carbonate de césium, dans un solvant tel que le méthanol, l'éthanol ou le dioxanne, à une température comprise entre 0°C et 50°C, en s'inspirant du procédé décrit par Noël D. D'Angelo et coll. dans J. Med. Chem. 2008, 51, 5766-5779 et en remplaçant les agents alkylants par du bromure de cyanogène.
Les amides (I-b)d peuvent également être obtenus à partir des amides (I-a)d par réaction avec l'o-diphénylphosphinylhydroxylamine en présence d'une base telle le carbonate de césium, dans un solvant tel que le diméthylformamide, à une température comprise entre 0°C et 50°C, selon par exemple le procédé décrit par S. Hanessian et coll. dans Bioorg. Med. Chem. Lett. 2008, 18, 1972-1976

### Préparation de composés de formule (Ie)

Les produits de formule générale (le) selon la présente invention peuvent notamment être préparés comme indiqué dans les Schémas Généraux (IA)e, (IB)e et (IC)e ci-dessous. Les préparations des exemples de la présente invention donnent des illustrations des schémas ci-dessous.

Les procédés de préparation des produits de formule Be à (le) sont tels que définis dans les Schémas Généraux (IA)e, (IB)e et (IC)e ci-dessous.

Les schémas (1A)e, (1B)e et (1C)e ci-dessous sont illustratifs des méthodes utilisées pour la préparation des produits de formule (le). Les produits de formule (le) tels que définis ci-dessus selon la présente invention peuvent ainsi notamment être préparés selon les procédés décrits dans le schéma (1 A)e, (1 B)e et (1C)e.

Le procédé de préparation de produits de formule (le) selon le schéma (1A)e est tel que défini ci-après.

Le procédé de préparation de produits de formule (le) selon le schéma (1 B)e est tel que défini ci-après.

Le procédé de préparation de produits de formule (le) selon le schéma (1A)e est tel que défini ci-après. 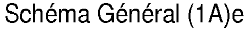 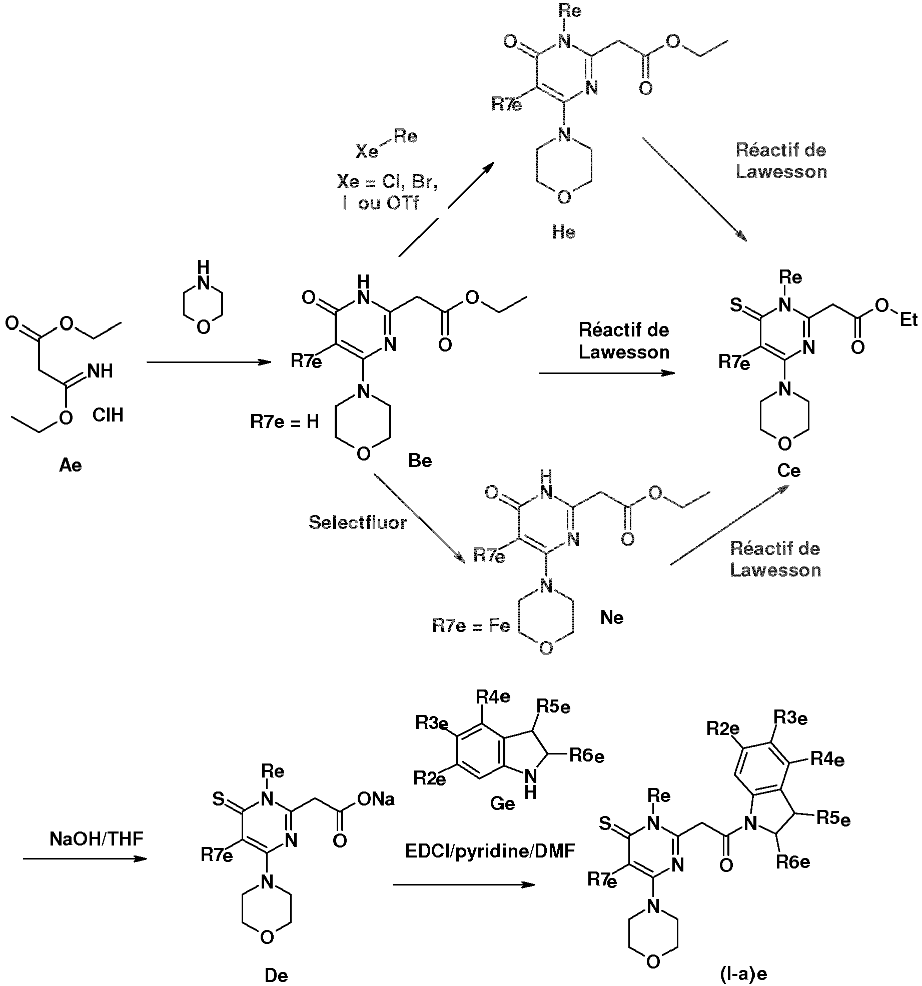 dans lequel les substituants Re, R2e, R3e, R4e, R5e, R6e et R7e ont les significations indiquées ci-dessus.

L'ester Be peut être obtenu par réaction « one-pot » entre la morpholine et un excès (par exemple 3 équivalents) d'imino-éther Ae (ou de son tautomère amino-acrylate), dans un solvant tel que l'éthanol, à une température comprise entre 20°C et le point d'ébullition du solvant.

Les esters He peuvent être obtenus à partir de l'ester Be par réaction avec un composé Re-Xe (Xe= Cl, Br, I ou triflate), en présence d'une base telle que la soude, le tertiobutylate de potassium ou le carbonate de césium, dans un solvant tel que le méthanol, l'éthanol ou le dioxanne, à une température comprise entre 0°C et 50°C, selon par exemple le procédé décrit par Noël D. D'Angelo et coll. dans (J. Med. Chem. (2008), 51, 5766-5779).

Les pyrimidones Ne peuvent être obtenus à partir des composés Be, avec un réactif de fluoration tel que le Selectfluor, dans un solvant tel que l'acétonitrile à une température comprise entre 22°C et la température d'ébullition du solvant, comme, par exemple, dans les conditions décrites par Banks R. E. (Journal of fluorine Chemestry (1998), 87, 1-17).

Les thiopyrimidones Ce peuvent être obtenus à partir des pyrimidones Be, He ou Ne par réaction avec un réactif de sulfuration tel que le réactif de Lawesson, dans un solvant tel que le toluène à une température comprise entre 22°C et la température d'ébullition du solvant, comme, par exemple, dans les conditions décrites par Jones G. (Journal of the Chemical Society, Perkin Transactions 1: Organic and Bio-Organic Chemistry (1972-1999), (1983), 11, 2645 - 2648.

Les carboxylates De peuvent être obtenus par hydrolyse des esters Ce, en présence d'une base telle que la soude ou la lithine, dans un solvant tel que le tétrahydrofuranne ou le méthanol, à une température comprise entre 0°C et 30°C

Les amides (I)e peuvent être obtenus à partir des carboxylates De par condensation d'une indoline Ge en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Alternativement, les composés (le) peuvent être obtenus selon le schéma général (1 B)e. 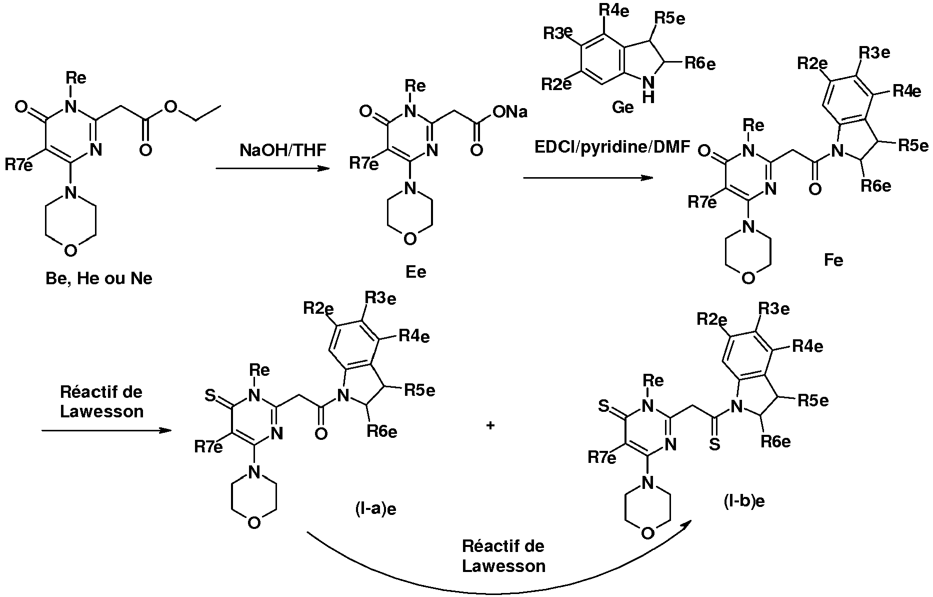 dans lequel les substituants Re, R2e, R3e, R4e, R5e, R6e et R7e ont les significations indiquées ci-dessus.

Les carboxylates Ee peuvent être obtenus par hydrolyse des esters Be, He ou Ne en présence d'une base telle que la soude ou la lithine, dans un solvant tel que le tétrahydrofuranne ou le méthanol, à une température comprise entre 0°C et 30°C

Les amides Fe peuvent être obtenus à partir des carboxylates Ee par condensation d'une indoline Ge en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris-diméthylamino phosphonium], le PyBOP [hexafluorophosphate de benzotriazol-1-yloxy tris-pyrrolidino phosphonium], le PyBROP [hexafluorophosphate de bromo tris pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Les thiopyrimidones (I-a)e et les thiopyrimidones-thioamides (I-b)e peuvent être obtenus à partir des composés Fe par réaction avec un réactif de sulfuration tel que le réactif de Lawesson, dans un solvant tel que le toluène à une température comprise entre 22°C et la température d'ébullition du solvant, comme, par exemple, dans les conditions décrites par Jones G. (Journal of the Chemical Society, Perkin Transactions 1: Organic and Bio-Organic Chemistry (1972-1999), (1983), 11, 2645 - 2648

Les thiopyrimidones-thioamides (I-b)e peuvent être obtenus à partir des thiopyrimidones (I-a)e par réaction avec un réactif de sulfuration tel que le réactif de Lawesson, dans un solvant tel que le toluène à une température comprise entre 22°C et la température d'ébullition du solvant, comme, par exemple, dans les conditions décrites par Jones G. (Journal of the Chemical Society, Perkin Transactions 1: Organic and Bio-Organic Chemistry (1972-1999), (1983), 11, 2645 - 2648

Alternativement, les composés (le) peuvent être obtenus selon le schéma général (IC)e.

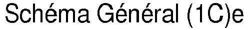

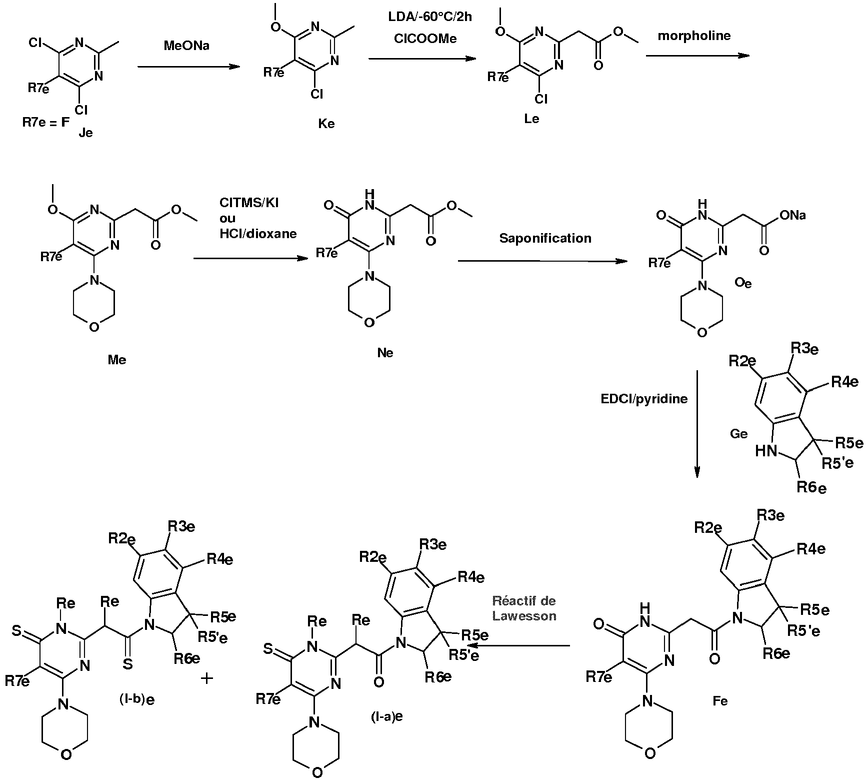

Dans lequel les substituants Re, R1e, R2e, R3e, R4e, R5e, R5'e, R6e et R7e ont les significations indiqués ci-dessus.

Dans le schéma général (1C)e :
Les dérivés méthoxypyrimidines Ke, peuvent être préparées à partir des composés Je par réaction avec le méthylate de sodium dans un solvant tel que le THF ou le méthanol à une température comprise entre 0°C et 25°C, selon, par exemple, les conditions décrites par loannidis, S. et coll. (Bioorganic and Médicinal Chemistry Letters, (2010), 20(5), 1669 - 1673).

Les composés Le, peuvent être obtenus par traitement des composés Ke en présence du chloroformiate de methyle avec la LDA (lithium diisopropylamide) dans un solvant tel que le THF à une température comprise entre -78°C et 25°C, selon par exemple, les conditions décrites par Tomioka K. et coll. (Tetrahedron, (1988), 44(14), 4351 - 4356).

Les composés Me, peuvent être obtenus à partir d'un composé Le par réaction avec la morpholine, en l'absence de solvant, à une température comprise entre 0°C et 25°C, comme décrit par exemple par Aliabiev S.B. (Lett. Org. Chem. (2007), 4(4), 273-280).

Les composés Ne peuvent être obtenus à partir des composés Me par réaction avec le chlorotrimethylsilane et l'iodure de potassium ou l'iodotrimethylsilane, dans un solvant tel que l'acétonitrile à une température comprise entre 22°C et la température d'ébullition du solvant, comme, par exemple, dans les conditions décrites par Shiao M.J. (J. Org. Chem. (1993), 58(17), 4742 - 4744).

Le carboxylate Oe peut être obtenu par hydrolyse de l'ester Ne en présence d'une base telle que la soude ou la lithine, dans un solvant tel que le tétrahydrofuranne ou le méthanol, à une température comprise entre 0°C et 30°C

Les amides Fe peuvent être obtenus à partir du carboxylate Oe par condensation d'une indoline Ge en présence d'un agent de couplage peptidique tel que, par exemple, l'EDCI (éthyl diméthylaminopropyle carbodiimide), le DMT-MM [chlorure de 4-(4,6-diméthoxy-1,2,3-triazin-2-yl) 4-méthylmorpholinium], le BOP [hexafluorophosphate de benzotriazol-1-yloxy tris- diméthylamino phosphonium], le PyBOP [hexafluorophosphate de benzotriazol-1-yloxy tris pyrrolidino phosphonium], le PyBROP [hexafluorophosphate de bromo tris-pyrrolidino phosphonium], le HATU [hexafluorophosphate de O-(7-azabenzotriazol-1-yl)-1,1,3,3-tétraméthyluronium)] ou un mélange HOBT/EDCI [hydroxybenzotriazole / éthyl diméthylaminopropyle carbodiimide], dans un solvant tel que la N,N-diméthylformamide, la pyridine, l'éthanol, l'eau ou le méthanol, à une température comprise entre 20°C et 50°C, comme par exemple dans les conditions décrites par Kunishima M. et Coll. dans (Tetrahedron (2001), 57, 1551-1558).

Les thiopyrimidones (I-a)e et les thiopyrimidones-thioamides (I-b)e peuvent être obtenus à partir des composés Pe par réaction avec un réactif de sulfuration tel que le réactif de Lawesson, dans un solvant tel que le toluène à une température comprise entre 22°C et la température d'ébullition du solvant, comme, par exemple, dans les conditions décrites par Jones G. (Journal of the Chemical Society, Perkin Transactions 1: Organic and Bio-Organic Chemistry (1972-1999), (1983), 11, 2645 - 2648

Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyles, trityle, benzyle, tert-butoxycarbonyle, BOC, benzyloxycarbonyle, phtalimido ou d'autres radicaux connus dans la chimie des peptides,

Les fonctions acide peuvent être protégées par exemple sous forme d'esters formés avec les esters facilement clivables tels que les esters benzyliques ou ter butyliques ou des esters connus dans la chimie des peptides.

On trouvera une liste de différents groupements protecteurs utilisables dans les manuels connus de l'homme du métier et par exemple dans le brevet BF 2 499 995.

On peut noter que l'on peut soumettre, si désiré et si nécessaire, des produits intermédiaires ou des produits de formules (la) à (le) ainsi obtenus par les procédés indiqués ci-dessus, pour obtenir d'autres intermédiaires ou d'autres produits de formules (la) à (le), à une ou plusieurs réactions de transformations connues de l'homme du métier telles que par exemple :
a) une réaction d'estérification de fonction acide,
b) une réaction de saponification de fonction ester en fonction acide,
c) une réaction de réduction de la fonction carboxy libre ou estérifié en fonction alcool,
d) une réaction de transformation de fonction alcoxy en fonction hydroxyle, ou encore de fonction hydroxyle en fonction alcoxy,
e) une réaction d'élimination des groupements protecteurs que peuvent porter les fonctions réactives protégées,
f) une réaction de salification par un acide minéral ou organique ou par une base pour obtenir le sel correspondant,
g) une réaction de dédoublement des formes racémiques en produits dédoublés,
lesdits produits de formules (la) à (le) ainsi obtenus étant sous toutes les formes isomères possibles racémiques, énantiomères et diastéréoisomères.

Les réactions a) à g) peuvent être réalisées dans les conditions usuelles connues de l'homme du métier telles que, par exemple, celles indiquées ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
   La réaction de saponification peut être réalisée selon les méthodes usuelles connues de l'homme du métier, telles que par exemple dans un solvant tel que le méthanol ou l'éthanol, le dioxanne ou le diméthoxyéthane, en présence de soude ou de potasse.
c) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxanne ou l'éther éthylique.
   Les éventuelles fonctions carboxy libre des produits décrits ci-dessus peuvent être, si désiré, réduites en fonction alcool notamment par de l'hydrure de bore.
d) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
e) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
f) Les produits décrits ci-dessus peuvent, si désiré, faire l'objet de réactions de salification par exemple par un acide minéral ou organique ou par une base minérale ou organique selon les méthodes usuelles connues de l'homme du métier : une telle réaction de salification peut être réalisée par exemple en présence d'acide chlorhydrique par exemple ou encore d'acide tartrique, citrique ou méthane sulfonique, dans un alcool tel que par exemple l'éthanol ou le méthanol.
g) Les éventuelles formes optiquement actives des produits décrits ci-dessus peuvent être préparées par dédoublement des racémiques selon les méthodes usuelles connues de l'homme du métier.

Les produits de formules (la), (Ib), (Ic), (Id) ou (le) tels que définis ci-dessus ainsi que leurs sels d'addition avec les acides présentent d'intéressantes propriétés pharmacologiques notamment en raison de leurs propriétés inhibitrices de kinases ainsi qu'il est indiqué ci-dessus.

Les produits de la présente invention sont notamment utiles pour la thérapie de tumeurs.

Les produits de l'invention peuvent également ainsi augmenter les effets thérapeutiques d'agents anti-tumoraux couramment utilisés.

Ces propriétés justifient leur application en thérapeutique et l'invention a particulièrement pour objet à titre de médicaments, les produits de formules (la), (Ib), (Ic), (Id) ou (le) telles que définies ci-dessus, lesdits produits de formules (la), (Ib), (Ic), (Id) ou (le) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formules (la), (Ib), (Ic), (Id) ou (le).

### L'invention a tout particulièrement pour objet, les produits répondant aux formules suivantes, et plus particulièrement les produits répondants aux formules suivantes à titre de médicaments :

### Composés de formule (Ia):

- 5-fluoro-2-[2-(4-fluoro-2,3-dihydro-1H-indol-1-yl)-2-oxoéthyl]-6-(morpholin-4-yl)pyrimidin-4(3H)-one
- 5-fluoro-2-[2-(4-fluoro-2,3-dihydro-1H-indol-1-yl)-2-oxoéthyl]-3-méthyl-6-(morpholin-4-yl)pyrimidin-4(3H)-one
- 5-fluoro-2-[1-(4-fluoro-2,3-dihydro-1H-indol-1-yl)-1-oxopropan-2-yl]-3-méthyl-6-(morpholin-4-yl)pyrimidin-4(3H)-one
- 5-fluoro-2-{2-[(2R)-2-méthyl-2,3-dihydro-1H-indol-1-yl]-2-oxoéthyl}-6-(morpholin-4-yl)pyrimidin-4(3H)-one
- 5-fluoro-2-{2-[(2S)-2-méthyl-2,3-dihydro-1H-indol-1-yl]-2-oxoéthyl}-6-(morpholin-4-yl)pyrimidin-4(3H)-one
- 5-fluoro-2-{2-[(2R)-5-fluoro-2-méthyl-2,3-dihydro-1H-indol-1-yl]-2-oxoéthyl}-6-(morpholin-4-yl)pyrimidin-4(3H)-one
- 5-fluoro-2-{2-[(2S)-5-fluoro-2-méthyl-2,3-dihydro-1H-indol-1-yl]-2-oxoéthyl}-6-(morpholin-4-yl)pyrimidin-4(3H)-one
- 5-fluoro-3-méthyl-2-{2-[(2S)-2-méthyl-2,3-dihydro-1H-indol-1-yl]-2-oxoéthyl}-6-(morpholin-4-yl)pyrimidin-4(3H)-one
- 5-fluoro-3-méthyl-2-{2-[(2R)-2-méthyl-2,3-dihydro-1H-indol-1-yl]-2-oxoéthyl}-6-(morpholin-4-yl)pyrimidin-4(3H)-one
- 5-fluoro-2-{2-[(2R)-4-fluoro-2-méthyl-2,3-dihydro-1H-indol-1-yl]-2-oxoéthyl}-6-(morpholin-4-yl)pyrimidin-4(3H)-one
- 5-fluoro-2-{2-[(2S)-4-fluoro-2-méthyl-2,3-dihydro-1H-indol-1-yl]-2-oxoéthyl}-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-5-fluoro-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-5-fluoro-6-(morpholin-4-yl)pyrimidin-4(3H)-one
- 5-fluoro-2-{2-[(2S)-5-fluoro-2-méthyl-2,3-dihydro-1H-indol-1-yl]-2-oxoéthyl}-3-méthyl-6-(morpholin-4-yl)pyrimidin-4(3H)-one
- 5-fluoro-2-{2-[(2R)-5-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-5-fluoro-6-(morpholin-4-yl)pyrimidin-4(3H)-one
- 2-{2-[(2S)-4-bromo-2-méthyl-2,3-dihydro-1H-indol-1-yl]-2-oxoéthyl}-5-fluoro-6-(morpholin-4-yl)pyrimidin-4(3H)-one
- 2-{2-[(2R)-4-chloro-2-méthyl-2,3-dihydro-1H-indol-1-yl]-2-oxoéthyl}-5-fluoro-6-(morpholin-4-yl)pyrimidin-4(3H)-one
- 2-{2-[(2S)-4-chloro-2-méthyl-2,3-dihydro-1H-indol-1-yl]-2-oxoéthyl}-5-fluoro-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-5-fluoro-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-5-fluoro-6-(morpholin-4-yl)pyrimidin-4(3H)-one
- 5-fluoro-2-{2-[(2S)-4-fluoro-2-méthyl-2,3-dihydro-1H-indol-1-yl]-2-oxoéthyl}-3-méthyl-6-(morpholin-4-yl)pyrimidin-4(3H)-one
- 5-fluoro-2-{2-[(2R)-4-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2R)-4-chloro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-4-chloro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-4-bromo-2-méthyl-2,3-dihydro-1H-indol-1-yl]-2-oxoéthyl}-5-fluoro-3-méthyl-6-(morpholin-4-yl)pyrimidin-4(3H)-one
- 5-fluoro-2-{2-[(2S)-6-fluoro-2-méthyl-2,3-dihydro-1H-indol-1-yl]-2-oxoéthyl}-6-(morpholin-4-yl)pyrimidin-4(3H)-one
- 5-fluoro-2-{2-[(2R)-6-fluoro-2-méthyl-2,3-dihydro-1H-indol-1-yl]-2-oxoéthyl}-6-(morpholin-4-yl)pyrimidin-4(3H)-one
- 5-chloro-2-{2-[(2R)-2-méthyl-2,3-dihydro-1H-indol-1-yl]-2-oxoéthyl}-6-(morpholin-4-yl)pyrimidin-4(3H)-one
- 5-chloro-2-{2-[(2S)-2-méthyl-2,3-dihydro-1H-indol-1-yl]-2-oxoéthyl}-6-(morpholin-4-yl)pyrimidin-4(3H)-one
- 2-{2-[-4-chloro-5-fluoro-2-méthyl-2,3-dihydro-1H-indol-1-yl]-2-oxoéthyl}-5-fluoro-3-méthyl-6-(morpholin-4-yl)pyrimidin-4(3H)-one
- 5-bromo-2-[2-((S)-2-methyl-2,3-dihydro-indol-1-yl)-2-oxo-ethyl]-6-morpholin-4-yl-3H-pyrimidin-4-one
- 5-Fluoro-2-[2-((+)-2-hydroxymethyl-2,3-dihydro-indol-1-yl)-2-oxo-ethyl]-6-morpholin-4-yl-3H-pyrimidin-4-one
- 5-Fluoro-2-[2-((-)-2-hydroxymethyl-2,3-dihydro-indol-1-yl)-2-oxo-ethyl]-6-morpholin-4-yl-3H-pyrimidin-4-one
ainsi que les sels d'addition avec les acides minéraux et organiques ou avec les bases minérales et organiques desdits produits de formule (la).

### Composés de formule (Ib):

N-(4-Fluoro-phényl)-2-[4-(2-méthyl-morpholin-4-yl)-6-oxo-1,6-dihydro-pyrimidin-2-yl]-acétamide
N-(4-Fluoro-phényl)-2-[4-((S)-2-méthyl-morpholin-4-yl)-6-oxo-1,6-dihydro-pyrimidin-2-yl]-acétamide
2-[4-(2-Fluorométhyl-morpholin-4-yl)-6-oxo-1,6-dihydro-pyrimidin-2-yl]-N-(4-fluoro-phényl)-acétamide
(+)-2-[4-(2-Fluorométhyl-morpholin-4-yl)-6-oxo-1,6-dihydro-pyrimidin-2-yl]-N-(4-fluoro-phényl)-acétamide
(+)-N-(3-Chloro-4-fluoro-phényl)-2-[4-(2-fluorométhyl-morpholin-4-yl)-1-méthyl-6-oxo-1,6-dihydro-pyrimidin-2-yl]-acétamide
2-[2-(4-Fluoro-2,3-dihydro-indol-1-yl)-2-oxo-éthyl]-6-(2-fluorométhyl-morpholin-4-yl)-3H-pyrimidin-4-one
(+)-2-[2-(4-Fluoro-2,3-dihydro-indol-1-yl)-2-oxo-éthyl]-6-(2-fluorométhyl-morpholin-4-yl)-3H-pyrimidin-4-one
2-[2-(4-Fluoro-2,3-dihydro-indol-1-yl)-2-oxo-éthyl]-6-(2-méthyl-morpholin-4-yl)-3H-pyrimidin-4-one
(-)-2-[2-(4-Fluoro-2,3-dihydro-indol-1-yl)-2-oxo-éthyl]-6-(2-méthyl-morpholin-4-yl)-3H-pyrimidin-4-one
(+)-2-[2-(4-Fluoro-2,3-dihydro-indol-1-yl)-2-oxo-éthyl]-6-(2-méthyl-morpholin-4-yl)-3H-pyrimidin-4-one
2-[2-(4-Fluoro-2,3-dihydro-indol-1-yl)-2-oxo-éthyl]-6-(hexahydro-cyclopenta[1,4]oxazin-4-yl)-3H-pyrimidin-4-one
2-[2-(4-Fluoro-2,3-dihydro-indol-1-yl)-2-oxo-éthyl]-6-pyridin-4-yl-3H-pyrimidin-4-one
2-[2-(4-Fluoro-2,3-dihydro-indol-1-yl)-2-oxo-éthyl]-6-(2-methoxy-pyridin-4-yl)-3H-pyrimidin-4-one
(±)-2-[4-(2-Éthyl-morpholin-4-yl)-6-oxo-1,6-dihydro-pyrimidin-2-yl]-N-(4-fluoro-phényl)-acétamide
(+)-2-[4-(2-Éthyl-morpholin-4-yl)-6-oxo-1,6-dihydro-pyrimidin-2-yl]-N-(4-fluoro-phényl)-acétamide
2-[2-(4-fluoro-2,3-dihydro-indol-1-yl)-2-oxo-éthyl]-6-(2,2,3,3,5,5,6,6-D8-morpholin)-4-yl-3h-pyrimidin-4-one
2-[2-(4-Fluoro-2,3-dihydro-indol-1-yl)-2-oxo-éthyl]-6-(3,3,5,5-D4-morpholin)-4-yl-3H-pyrimidin-4-one
2-(4-Chloro-6-methoxy-pyrimidin-2-yl)-1-(3,3-diméthyl-2,3-dihydro-indol-1-yl)-ethanone
(+)-2-[2-(2-méthyle-2,3-dihydro-indol-1-yl)-2-oxo-éthyl]-6-(3,3,5,5-D4-morpholin)-4-yl-3H-pyrimidin-4-one
(-)-2-[2-(2-méthyle-2,3-dihydro-indol-1-yl)-2-oxo-éthyl]-6-(3,3,5,5-D4-morpholin)-4-yl-3H-pyrimidin-4-one
(+)-2-[2-(2-méthyle-2,3-dihydro-indol-1-yl)-2-oxo-éthyl]-6-(2,2,3,3,5,5,6,6-D8-morpholin)-4-yl-3H-pyrimidin-4-one
(-)-2-[2-(2-méthyle-2,3-dihydro-indol-1-yl)-2-oxo-éthyl]-6-(2,2,3,3,5,5,6,6-D8-morpholin)-4-yl-3H-pyrimidin-4-one
2-[2-(4-Fluoro-2,3-dihydro-indol-1-yl)-2-oxo-éthyl]-6-(2-méthyl-pyridin-4-yl)-3H-pyrimidin-4-one
2-[2-(4-Fluoro-2,3-dihydro-indol-1-yl)-2-oxo-éthyl]-6-(2-fluoro-pyridin-4-yl)-3H-pyrimidin-4-one
(-)-2-[2-(4-Chloro-2,3-dihydro-indol-1-yl)-2-oxo-éthyl]-6-(2-hydroxyméthyl-morpholin-4-yl)-3H-pyrimidin-4-one
(+)-2-[2-(4-Chloro-2,3-dihydro-indol-1-yl)-2-oxo-éthyl]-6-(2-hydroxyméthyl-morpholin-4-yl)-3H-pyrimidin-4-one
(±)-2-[2-(4-Fluoro-2,3-dihydro-indol-1-yl)-2-oxo-éthyl]-6-(2-hydroxyméthyl-morpholin-4-yl)-3H-pyrimidin-4-one
(±)-2-[2-(4-Chloro-2,3-dihydro-indol-1-yl)-2-oxo-éthyl]-6-(2-méthyl-morpholin-4-yl)-3H-pyrimidin-4-one
(+)-2-[2-((S)-2-Méthyl-2,3-dihydro-indol-1-yl)-2-oxo-éthyl]-6-(-2-méthyl-morpholin-4-yl)-3H-pyrimidin-4-one
(+)-6-(2-Fluorométhyl-morpholin-4-yl)-2-[2-((S)-2-méthyl-2,3-dihydro-indol-1-yl)-2-oxo-éthyl]-3H-pyrimidin-4-one
(+)-6-(2-Hydroxyméthyl-morpholin-4-yl)-2-[2-((S)-2-méthyl-2,3-dihydro-indol-1-yl)-2-oxo-éthyl]-3H-pyrimidin-4-one
ainsi que les sels d'addition avec les acides minéraux et organiques ou avec les bases minérales et organiques desdits produits de formule (Ib).

### Composés de formule (Ic):

- 2-[2-(4-chloro-2-méthyl-2,3-dihydro-1H-indol-1-yl)-2-oxoéthyl]-6-(morpholin-4-yl)pyrimidin-4(3H)-one
- 2-{2-[(2S)-4-chloro-2-méthyl-2,3-dihydro-1H-indol-1-yl]-2-oxoéthyl}-6-(morpholin-4-yl)pyrimidin-4(3H)-one
- 2-{2-[(2R)-4-chloro-2-méthyl-2,3-dihydro-1H-indol-1-yl]-2-oxoéthyl}-6-(morpholin-4-yl)pyrimidin-4(3H)-one
- 2-[2-(4-chloro-3,3-diméthyl-2,3-dihydro-1H-indol-1-yl)-2-oxoéthyl]-3-méthyl-6-(morpholin-4-yl)pyrimidin-4(3H)-one
- 2-[2-(4-chloro-3,3-diméthyl-2,3-dihydro-1H-indol-1-yl)-2-oxoéthyl]-6-(morpholin-4-yl)pyrimidin-4(3H)-one
- 2-[2-(4-hydroxy-2-méthyl-2,3-dihydro-1H-indol-1-yl)-2-oxoéthyl]-6-(morpholin-4-yl)pyrimidin-4(3H)-one
- 2-[2-(4-fluoro-3,3-diméthyl-2,3-dihydro-1H-indol-1-yl)-2-oxoéthyl]-3-méthyl-6-(morpholin-4-yl)pyrimidin-4(3H)-one
- 2-[2-(4-fluoro-3,3-diméthyl-2,3-dihydro-1H-indol-1-yl)-2-oxoéthyl]-6-(morpholin-4-yl)pyrimidin-4(3H)-one
- 2-{2-[(2S)-4-hydroxy-2-méthyl-2,3-dihydro-1H-indol-1-yl]-2-oxoéthyl}-6-(morpholin-4-yl)pyrimidin-4(3H)-one
- 2-{2-[(2R)-4-hydroxy-2-méthyl-2,3-dihydro-1H-indol-1-yl]-2-oxoéthyl}-6-(morpholin-4-yl)pyrimidin-4(3H)-one
- 2-{2-[(3R)-4-hydroxy-3-méthyl-2,3-dihydro-1H-indol-1-yl]-2-oxoéthyl}-6-(morpholin-4-yl)pyrimidin-4(3H)-one
- 2-{2-[(3S)-4-hydroxy-3-méthyl-2,3-dihydro-1H-indol-1-yl]-2-oxoéthyl}-6-(morpholin-4-yl)pyrimidin-4(3H)-one
- 2-{2-[(2S)-5-fluoro-2-méthyl-2,3-dihydro-1H-indol-1-yl]-2-oxoéthyl}-6-(morpholin-4-yl)pyrimidin-4(3H)-one
- 2-{2-[(2R)-5-fluoro-2-méthyl-2,3-dihydro-1H-indol-1-yl]-2-oxoéthyl}-6-(morpholin-4-yl)pyrimidin-4(3H)-one
- 2-{2-[(2S)-5-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-5-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-6-(morpholin-4-yl)pyrimidin-4(3H)-one
- 2-{2-[(2S)-4-bromo-2-méthyl-2,3-dihydro-1H-indol-1-yl]-2-oxoéthyl}-6-(morpholin-4-yl)pyrimidin-4(3H)-one
- 2-{2-[(2R)-4-fluoro-2-méthyl-2,3-dihydro-1H-indol-1-yl]-2-oxoéthyl}-6-(morpholin-4-yl)pyrimidin-4(3H)-one
- 2-{2-[(2S)-4-fluoro-2-méthyl-2,3-dihydro-1H-indol-1-yl]-2-oxoéthyl}-6-(morpholin-4-yl)pyrimidin-4(3H)-one
- 2-{2-[(2R)-4-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-bromo-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-chloro-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-chloro-2-méthyl-2,3-dihydro-1H-indol-1-yl]-2-oxoéthyl}-3-méthyl-6-(morpholin-4-yl)pyrimidin-4(3H)-one
- 2-{2-[(2R)-6-fluoro-2-méthyl-2,3-dihydro-1H-indol-1-yl]-2-oxoéthyl}-6-(morpholin-4-yl)pyrimidin-4(3H)-one
- 2-{2-[(2S)-6-fluoro-2-méthyl-2,3-dihydro-1H-indol-1-yl]-2-oxoéthyl}-6-(morpholin-4-yl)pyrimidin-4(3H)-one
- 2-{2-[(2R)-4-chloro-5-fluoro-2-méthyl-2,3-dihydro-1H-indol-1-yl]-2-oxoéthyl}-6-(morpholin-4-yl)pyrimidin-4(3H)-one
- 2-{2-[(2S)-4-chloro-5-fluoro-2-méthyl-2,3-dihydro-1H-indol-1-yl]-2-oxoéthyl}-6-(morpholin-4-yl)pyrimidin-4(3H)-one
- 2-{2-[(2R)-2-isopropyl-2,3-dihydro-indol-1-yl]-2-oxo-éthyl}-6-morpholin-4-yl-3H-pyrimidin-4-one
- 2-{2-[(2S)-2-isopropyl-2,3-dihydro-indol-1-yl]-2-oxo-éthyl}-6-morpholin-4-yl-3H-pyrimidin-4-one
- 2-[1-(4-fluoro-2,3-dihydro-1H-indol-1-yl)-1-oxopropan-2-yl]-6-(morpholin-4-yl)pyrimidin-4(3H)-one
- 2-[2-((R)-2-éthyl-2,3-dihydro-indol-1-yl)-2-oxo-éthyl]-6-morpholin-4-yl-3H-pyrimidin-4-one
- 2-[2-((S)-2-éthyl-2,3-dihydro-indol-1-yl)-2-oxo-éthyl]-6-morpholin-4-yl-3H-pyrimidin-4-one
ainsi que les sels d'addition avec les acides minéraux et organiques ou avec les bases minérales et organiques desdits produits de formule (Ic).

Sont également décrits les composés suivants :
- 2-[(2R)-2-fluoro-1-(4-fluoro-2,3-dihydro-1*H*-indol-1-yl)-1-oxopropan-2-yl]-6-(morpholin-4-yl)pyrimidin-4(3*H*)-one
- 2-[(2S)-2-fluoro-1-(4-fluoro-2,3-dihydro-1*H*-indol-1-yl)-1-oxopropan-2-yl]-6-(morpholin-4-yl)pyrimidin-4(3*H*)-one
- 1-{[4-(morpholin-4-yl)-6-oxo-1,6-dihydropyrimidin-2-yl]acétyl}-2,3-dihydro-1H-indole-4-carbonitrile.

### Composés de formule (Id):

- 6-(morpholin-4-yl)-2-[2-oxo-2-(spiro[cyclopropane-1,3'-indol]-1'(2'H)-yl)éthyl]pyrimidin-4(3H)-one
- 6-(morpholin-4-yl)-2-[2-oxo-2-(4-phényl-2,3-dihydro-1H-indol-1-yl)éthyl]pyrimidin-4(3H)-one
- 6-(morpholin-4-yl)-2-{2-oxo-2-[4-(trifluorométhoxy)-2,3-dihydro-1H-indol-1-yl]éthyl}pyrimidin-4(3H)-one
- 3-méthyl-6-(morpholin-4-yl)-2-{2-oxo-2-[4-(trifluorométhyl)-2,3-dihydro-1H-indol-1-yl]éthyl}pyrimidin-4(3H)-one
- 6-(morpholin-4-yl)-2-{2-oxo-2-[4-(trifluorométhyl)-2,3-dihydro-1H-indol-1-yl]éthyl}pyrimidin-4(3H)-one
- 2-{2-[4-(2-méthoxyphényl)-2,3-dihydro-1H-indol-1-yl]-2-oxoéthyl}-6-(morpholin-4-yl)pyrimidin-4(3H)-one
- 6-(morpholin-4-yl)-2-{2-oxo-2-[4-(1-propylpipéridin-3-yl)-2,3-dihydro-1H-indol-1-yl]éthyl}pyrimidin-4(3H)-one
- 2-{2-[4-(difluorométhoxy)-2,3-dihydro-1H-indol-1-yl]-2-oxoéthyl}-3-méthyl-6-(morpholin-4-yl)pyrimidin-4(3H)-one
- 2-{2-[4-(difluorométhoxy)-2,3-dihydro-1H-indol-1-yl]-2-oxoéthyl}-6-(morpholin-4-yl)pyrimidin-4(3H)-one
- 6-(morpholin-4-yl)-2-{2-oxo-2-[4-(pyridin-4-yl)-2,3-dihydro-1H-indol-1-yl]éthyl}pyrimidin-4(3H)-one
- 2-[2-(1'-méthylspiro[indole-3,4'-pipéridin]-1(2H)-yl)-2-oxoéthyl]-6-(morpholin-4-yl)pyrimidin-4(3H)-one
- 6-(morpholin-4-yl)-2-{2-oxo-2-[4-(pyridin-2-yl)-2,3-dihydro-1H-indol-1-yl]éthyl}pyrimidin-4(3H)-one
- 6-(morpholin-4-yl)-2-{2-oxo-2-[4-(pyridin-3-yl)-2,3-dihydro-1H-indol-1-yl]éthyl}pyrimidin-4(3H)-one
- 2-{2-[4-(2-chlorophényl)-2,3-dihydro-1H-indol-1-yl]-2-oxoéthyl}-6-(morpholin-4-yl)pyrimidin-4(3H)-one
- 2-[2-(4-chloro-2,3-dihydro-1H-indol-1-yl)-2-oxoéthyl]-3-cyclopropyl-6-(morpholin-4-yl)pyrimidin-4(3H)-one
- 6-(morpholin-4-yl)-2-[2-oxo-2-(2,3,3a,8b-tétrahydrocyclopenta[b]indol-4(1H)-yl)éthyl]pyrimidin-4(3H)-one
- 2-[2-(4-{[4-(méthylsulfonyl)pipérazin-1-yl]méthyl}-2,3-dihydro-1H-indol-1-yl)-2-oxoéthyl]-6-(morpholin-4-yl)pyrimidin-4(3H)-one
- 2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one
- 2-{2-[4-(2-fluorophényl)-2,3-dihydro-1H-indol-1-yl]-2-oxoéthyl}-6-(morpholin-4-yl)pyrimidin-4(3H)-one
- 3-méthyl-2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one
- 6-(morpholin-4-yl)-2-[2-oxo-2-(2',3',5',6'-tétrahydrospiro[indole-3,4'-pyran]-1(2H)-yl)éthyl]pyrimidin-4(3H)-one
- 3-méthyl-6-(morpholin-4-yl)-2-[2-oxo-2-(2',3',5',6'-tétrahydrospiro[indole-3,4'-pyran]-1(2H)-yl)éthyl]pyrimidin-4(3H)-one
- 3-méthyl-6-(morpholin-4-yl)-2-[2-oxo-2-(spiro[indole-3,4'-pipéridin]-1(2H)-yl)éthyl]pyrimidin-4(3H)-one
- 6-(morpholin-4-yl)-2-{2-oxo-2-[(2R)-2-phényl-2,3-dihydro-1H-indol-1-yl]éthyl}pyrimidin-4(3H)-one
- 6-(morpholin-4-yl)-2-{2-oxo-2-[(2S)-2-phényl-2,3-dihydro-1H-indol-1-yl]éthyl}pyrimidin-4(3H)-one
- 2-{2-[4-(4-méthylpipérazin-1-yl)-2,3-dihydro-1H-indol-1-yl]-2-oxoéthyl}-6-(morpholin-4-yl)pyrimidin-4(3H)-one
- 2-{2-[(2R)-2-(4-fluorophényl)-2,3-dihydro-1H-indol-1-yl]-2-oxoéthyl}-6-(morpholin-4-yl)pyrimidin-4(3H)-one
- 2-{2-[(2S)-2-(4-fluorophényl)-2,3-dihydro-1H-indol-1-yl]-2-oxoéthyl}-6-(morpholin-4-yl)pyrimidin-4(3H)-one
- 2-{2-[(2R)-4-(difluorométhoxy)-2-méthyl-2,3-dihydro-1H-indol-1-yl]-2-oxoéthyl}-6-(morpholin-4-yl)pyrimidin-4(3H)-one
- 2-{2-[(2S)-4-(difluorométhoxy)-2-méthyl-2,3-dihydro-1H-indol-1-yl]-2-oxoéthyl}-6-(morpholin-4-yl)pyrimidin-4(3H)-one
- 2-{2-[(2R)-4-(difluorométhoxy)-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-(difluorométhoxy)-2-méthyl-2,3-dihydro-1H-indol-1-yl]-2-oxoéthyl}-3-méthyl-6-(morpholin-4-yl)pyrimidin-4(3H)-one
- 6-(morpholin-4-yl)-2-{2-[4-(morpholin-4-yl)-2,3-dihydro-1H-indol-1-yl]-2-oxoéthyl}pyrimidin-4(3H)-one
- 2-{2-[(2R)-2-cyclopropyl-2,3-dihydro-1H-indol-1-yl]-2-oxoéthyl}-6-(morpholin-4-yl)pyrimidin-4(3H)-one
- 2-{2-[(2S)-2-cyclopropyl-2,3-dihydro-1H-indol-1-yl]-2-oxoéthyl}-6-(morpholin-4-yl)pyrimidin-4(3H)-one
- 2-{2-[(2R)-2-méthyl-4-(trifluorométhyl)-2,3-dihydro-1H-indol-1-yl]-2-oxoéthyl}-6-(morpholin-4-yl)pyrimidin-4(3H)-one
- 2-{2-[(2S)-2-méthyl-4-(trifluorométhyl)-2,3-dihydro-1H-indol-1-yl]-2-oxoéthyl}-6-(morpholin-4-yl)pyrimidin-4(3H)-one
- 2-[2-((+)-2-Fluoromethyl-2,3-dihydro-indol-1-yl)-2-oxo-ethyl]-6-morpholin-4-yl-3H-pyrimidin-4-one
- 2-[2-(2,3-Dihydro-indol-1-yl)-2-oxo -ethyl]-6-morpholin-4-yl-3-phenyl-3H-pyrimidin-4-one
ainsi que les sels d'addition avec les acides minéraux et organiques ou avec les bases minérales et organiques desdits produits de formule (Id).

Sont également décrits les composés suivants :
- 2-[2-((-)-2-fluoromethyl-2,3-dihydro-indol-1-yl)-2-oxo-ethyl]-6-morpholin-4-yl-3H-pyrimidin-4-one
- 3-amino-2-[2-(2,3-dihydro-1*H*-indol-1-yl)-2-oxoéthyl]-6-(morpholin-4-yl)pyrimidin-4(3*H*)-one
- 2-[2-(2,3-dihydro-1*H*-indol-1-yl)-2-oxoéthyl]-4-(morpholin-4-yl)-6-oxopyrimidine-1(6*H*)-carbonitrile.

### Composés de formule (Ie):

- 1-((S)-2-Methyl-2,3-dihydro-indol-1-yl)-2-(4-morpholin-4-yl-6-thioxo-1,6-dihydro-pyrimidin-2-yl)-ethanone
- 1-((R)-2-Methyl-2,3-dihydro-indol-1-yl)-2-(4-morpholin-4-yl-6-thioxo-1,6-dihydro-pyrimidin-2-yl)-ethanone
- 2-[2-((S)-2-Methyl-2,3-dihydro-indol-1-yl)-2-thioxo-ethyl]-6-morpholin-4-yl-3H-pyrimidine-4-thione
- 1-((S)-2-Methyl-2,3-dihydro-indol-1-yl)-2-(1-methyl-4-morpholin-4-yl-6-thioxo-1,6-dihydro-pyrimidin-2-yl)-ethanone
- 1-(R)-2-Methyl-2,3-dihydro-indol-1-yl)-2-(1-methyl-4-morpholin-4-yl-6-thioxo-1,6-dihydro-pyrimidin-2-yl)-ethanone
- 1-(4-Fluoro-2,3-dihydro-indol-1-yl)-2-(1-methyl-4-morpholin-4-yl-6-thioxo-1,6-dihydro-pyrimidin-2-yl)-ethanone
- 1-(4-Chloro-2,3-dihydro-indol-1-yl)-2-(1-methyl-4-morpholin-4-yl-6-thioxo-1,6-dihydro-pyrimidin-2-yl)-ethanone
- 1-((+)-4,5-Difluoro-2-methyl-2,3-dihydro-indol-1-yl)-2-(5-fluoro-4-morpholin-4-yl-6-thioxo-1,6-dihydro-pyrimidin-2-yl)-ethanone
- 2-[2-((+)-4,5-Difluoro-2-methyl-2,3-dihydro-indol-1-yl)-2-thioxo-ethyl]-5-fluoro-6-morpholin-4-yl-3H-pyrimidine-4-thione
- 2-(5-Fluoro-4-morpholin-4-yl-6-thioxo-1,6-dihydro-pyrimidin-2-yl)-1-((S)-2-methyl-2,3-dihydro-indol-1-yl)-ethanone
- 2-(5-Fluoro-4-morpholin-4-yl-6-thioxo-1,6-dihydro-pyrimidin-2-yl)-1-((R)-2-methyl-2,3-dihydro-indol-1-yl)-ethanone
ainsi que les sels d'addition avec les acides minéraux et organiques ou avec les bases minérales et organiques desdits produits de formule (le).

La présente invention a également pour objet les composés listés ci-dessus à titre de médicaments.

La présente invention a encore pour objet tout procédé de préparation des produits de formule (la) à (le) tels que définis ci-dessus.

Les produits selon l'invention peuvent être préparés à partir de méthodes conventionnelles de chimie organique.

L'invention concerne aussi des compositions pharmaceutiques contenant à titre de principe actif l'un au moins des produits de formule (la), (Ib), (Ic), (Id) ou (le) tels que définis ci-dessus ou un sel pharmaceutiquement acceptable de ce produitet, le cas échéant, un support pharmaceutiquement acceptable.

L'invention s'étend ainsi aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

Un tel médicament peut notamment être destiné au traitement ou à la prévention d'une maladie chez un mammifère.

La présente invention a notamment pour objet l'utilisation d'un produit de formule (la), (Ib), (Ic), (Id) ou (le) tel que défini ci-dessus pour la préparation d'un médicament destiné à la prévention ou au traitement de maladies liées à une prolifération non contrôlée.

La présente invention a ainsi tout particulièrement pour objet l'utilisation d'un produit de formule (la), (Ib), (Ic), (Id) ou (le) tel que défini ci-dessus pour la préparation d'un médicament destiné au traitement ou à la prévention de maladies en oncologie et notamment destiné au traitement de cancers.

Parmi ces cancers, on s'intéresse au traitement de tumeurs solides ou liquides, au traitement de cancers résistant à des agents cytotoxiques

Les produits de la présente invention cités peuvent notamment être utilisés pour le traitement de tumeurs primaires et/ou de métastases en particulier dans les cancers gastriques, hépatiques, rénaux, ovariens, du colon, de la prostate, de l'endomètre, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas, dans les hamartomes.

La présente invention a aussi pour objet l'utilisation des produits de formule (la), (Ib), (Ic), (Id) ou (le) telle que définie ci-dessus pour la préparation de médicaments destinés à la chimiothérapie de cancers.

De tels médicaments destinés à la chimiothérapie de cancers peuvent être utilisés seuls ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

Comme inhibiteurs de kinases, on peut citer la butyrolactone, le flavopiridol et la 2(2-hydroxyéthylamino)-6-benzylamino-9-méthylpurine appelée olomucine.

Ainsi la présente demande concerne notamment les produits de formule (la), (Ib), (Ic), (Id) ou (le) tel que définis ci-dessus pour leur utilisation pour le traitement de cancers.

Ainsi la présente demande concerne notamment les produits de formule (la), (Ib), (Ic), (Id) ou (le) tel que définis ci-dessus pour leur utilisation pour le traitement de tumeurs solides ou liquides.

Ainsi la présente demande concerne notamment les produits de formule (la), (Ib), (Ic), (Id) ou (le) tel que définis ci-dessus pour leur utilisation pour le traitement de cancers résistant à des agents cytotoxiques.

Ainsi la présente demande concerne notamment les produits de formule (la), (Ib), (Ic), (Id) ou (le) tel que définis ci-dessus pour leur utilisation pour le traitement de tumeurs primaires et/ou de métastases en particulier dans les cancers gastriques, hépatiques, rénaux, ovariens, du colon, de la prostate, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas. dans les hamartomes.

Ainsi la présente demande concerne notamment les produits de formule (la), (Ib), (Ic), (Id) ou (le) telle que définie ci-dessus, pour leur utilisation pour la chimiothérapie de cancers.

Ainsi la présente demande concerne notamment les produits de formule (la), (Ib), (Ic), (Id) ou (le) telle que définie ci-dessus, pour leur utilisation pour la chimiothérapie de cancers seul ou en en association.

Les produits de la présente demande peuvent notamment être administrés seuls ou en association avec de la chimiothérapie ou de la radiothérapie ou encore en association par exemple avec d'autres agents thérapeutiques.

De tels agents thérapeutiques peuvent être des agents anti-tumoraux couramment utilisés.

Comme inhibiteurs de kinases, on peut citer la butyrolactone, le flavopiridol et la 2(2-hydroxyéthylamino)-6-benzylamino-9-méthylpurine appelée olomucine.

Ainsi la présente demande concerne notamment les produits de formule (la), (Ib), (Ic), (Id) ou (le) tel que définis ci-dessus pour leur utilisation pour le traitement de cancers.

Ainsi la présente demande concerne notamment les produits de formule (la), (Ib), (Ic), (Id) ou (le) tel que définis ci-dessus pour leur utilisation pour le traitement de tumeurs solides ou liquides.

Ainsi la présente demande concerne notamment les produits de formule (la), (Ib), (Ic), (Id) ou (le) tel que définis ci-dessus pour leur utilisation pour le traitement de cancers résistant à des agents cytotoxiques.

Ainsi la présente demande concerne notamment les produits de formule (la), (Ib), (Ic), (Id) ou (le) tel que définis ci-dessus pour leur utilisation pour le traitement de tumeurs primaires et/ou de métastases en particulier dans les cancers gastriques, hépatiques, rénaux, ovariens, du colon, de la prostate, du poumon (NSCLC et SCLC), les glioblastomes, les cancers de la thyroïde, de la vessie, du sein, dans le mélanome, dans les tumeurs hématopoietiques lymphoïdes ou myéloïdes, dans les sarcomes, dans les cancers du cerveau, du larynx, du système lymphatique, cancers des os et du pancréas. dans les hamartomes.

Ainsi la présente demande concerne notamment les produits de formule (la), (Ib), (Ic), (Id) ou (le) telle que définie ci-dessus, pour leur utilisation pour la chimiothérapie de cancers.

Ainsi la présente demande concerne notamment les produits de formule (la), (Ib), (Ic), (Id) ou (le) telle que définie ci-dessus, pour leur utilisation pour la chimiothérapie de cancers seul ou en en association.

### Produits intermédiaires:

### Produits intermédiaires dans la préparation des composés de formule (Ia):

Les intermédiaires de synthèse de formules Ga, La, Ha, Ma et Qa tels que définis ci-dessus sont rappelés ci-après : 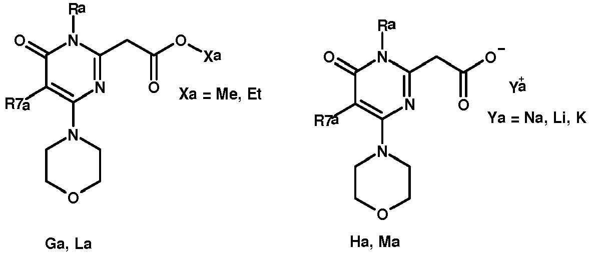 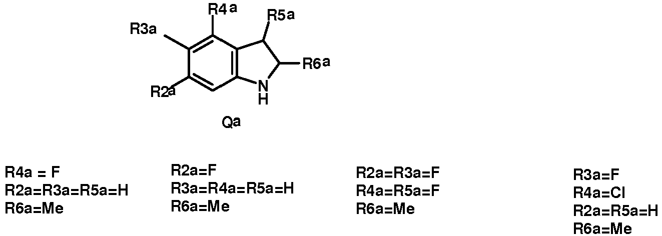 dans lesquels R7a a la définition indiquée ci-dessus, et Ra représente hydrogène dans les produits Ga et Ha et Ra représente alkyle dans les produits La et Ma. ces produits étant sous toutes les formes isomères possibles racémiques, énantiomères et diastéréoisomères.

### Produits intermédiaires dans la préparation des composés de formule (Ib):

Les intermédiaires de synthèse de formules Jb, Mb, Kb, Nb et certaines indolines Pb tels que définis ci-dessus sont rappelés ci-après : 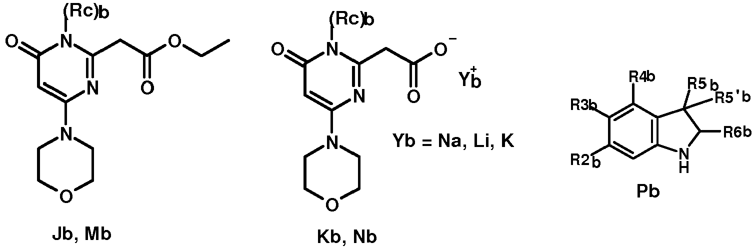 dans lesquels (Rc)b, R2b, R3b, R4b, R5b, R5'b et R6b ont l'une quelconque des définitions indiquées ci-dessus, ces produits étant sous toutes les formes isomères possibles racémiques, énantiomères et diastéréoisomères.

### Produits intermédiaires dans la préparation des composés de formule (Ic):

Les intermédiaires de synthèse de formules Cc, Dc, Ec, Fc et certaines indolines Sc tels que définis ci-dessus sont rappelés ci-après : 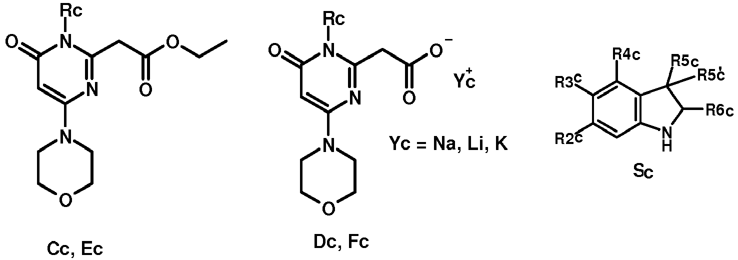 dans lesquels Rc, R2c, R3c, R4c, R5c, R5'c et R6c ont l'une quelconque des définitions indiquées ci dessus, ces produits étant sous toutes les formes isomères possibles racémiques, énantiomères et diastéréoisomères,

### Produits intermédiaires dans la préparation des composés de formle (Id):

Les intermédiaires de synthèse de formules Cd, Dd, Ed, Fd ainsi que certaines indolines Ld tels que définis ci-dessus sont rappelés ci-après : 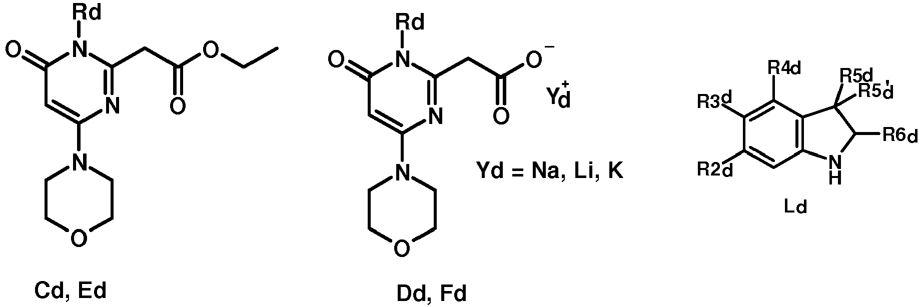 dans lesquels Rd, R2d, R3d, R4d, R5d, R5'd et R6d ont l'une quelconque des définitions ci-dessus, ces produits étant sous toutes les formes isomères possibles racémiques, énantiomères et diastéréoisomères,

### Produits intermédiaires dans la préparation des composés de formule (Ie):

Les intermédiaires de synthèse de formules Ce et De tels que définis ci-dessus sont rappelés ci-après : 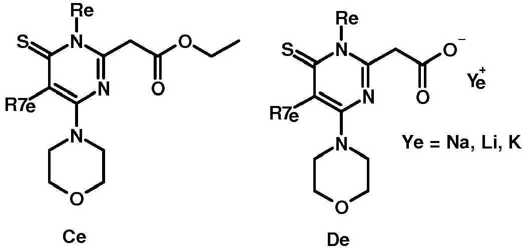 dans lesquels Re représente un atome d'hydrogène dans les produits de formule Ce et De.

La présente invention a encore pour objet tout procédé de préparation des produits de formule (la) à (le) tels que définis ci-dessus.

Les produits selon l'invention peuvent être préparés à partir de méthodes conventionnelles de chimie organique.

### Partie expérimentale

La nomenclature des composés de cette présente invention a été effectuée avec le logiciel ACDLABS version 10.0.

Le four à microondes utilisé est un appareil Biotage, Initiator ™ 2.0, 400W max, 2450 MHz.

Les spectres de RMN 1 H à 400 MHz et 1 H à 500 MHz ont été effectués sur spectromètre BRUKER AVANCE DRX-400 ou BRUKER AVANCE DPX-500 avec les déplacements chimiques (δ en ppm) dans le solvant diméthylsulfoxide-d₆ (DMSO-d₆) référencé à 2,5 ppm à la température de 303K, sauf lorsqu'un autre solvant est spécifié (chloroforme -d : référencé à 7,26 ppm).

Les spectres de masse (SM) ont été obtenus soit par la méthode A, soit par la méthode B.

### Méthode A :

Appareil WATERS UPLC-SQD ; Ionisation : électrospray en mode positif et/ou négatif (ES+/-) ; Conditions chromatographiques : Colonne : ACQUITY BEH C₁₈ 1,7 µm - 2,1 x 50 mm ; Solvants : A : H₂O (0,1 % acide formique) B : CH₃CN (0,1 % acide formique) ; Température de colonne : 50 °C ; Débit : 1 ml/min ; Gradient (2 min) : de 5 à 50 % de B en 0,8 min ; 1,2 min : 100 % de B ; 1,85 min : 100 % de B ; 1,95 : 5 % de B ; Temps de rétention = Tr (min).

### Méthode B :

Appareil WATERS ZQ ; Ionisation : électrospray en mode positif et/ou négatif (ES+/-) ; Conditions chromatographiques : Colonne : XBridge C₁₈ 2,5 µm - 3 x 50 mm ; Solvants : A : H₂O (0,1 % acide formique) B : CH₃CN (0,1 % acide formique) ; Température de colonne : 70°C ; Débit : 0,9 ml/min ; Gradient (7 min) : de 5 à 100 % de B en 5,3 min ; 5,5 min : 100 % de B ; 6,3 min : 5 % de B ; Temps de rétention = Tr (min).

Les pouvoirs rotatoires (PR) ont été effectués sur un polarimètre modèle 341 de chez Perkin Elmer. longueur d'onde: raie α du sodium (589 nanomètres)

### Synthèse des composés de formule (Ia):

### Exemple 1a: Synthèse du 5-fluoro-2-[2-(4-fluoro-2,3-dihydro-1H-indol-1-yl)-2-oxoéthyl]-6-(morpholin-4-yl)pyrimidin-4(3H)-one

### Etape 1a :

### 4-Chloro-5-fluoro-6-méthoxy-2-méthyl-pyrimidine

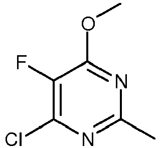

A une solution de 9.8 g du 2-méthyl-4,6-dichloro-5-fluoropyrimidine dans 80 mL de THF refroidit à 5°C dans un bain de glace sont ajoutés 3.21 g de méthylate de sodium. Le bain de glace est retiré. La suspension est agitée à la température ambiante pendant 3 heures. Le milieu réactionnel est refroidit à 5°C dans un bain de glace. Addition de 20 mL d'eau et 100 mL d'acétate d'éthyle. Après décantation, la phase organique est séchée sur sulfate de magnésium, filtrée, concentrée sous pression réduite pour donner 9 g de 4-chloro-5-fluoro-6-méthoxy-2-méthyl-pyrimidine sous forme d'une huile incolore qui cristallise dont les caractéristiques sont les suivantes :
Spectrométrie de Masse : méthode A
Temps de rétention Tr (min) = 0,82 ; [M+H]+ : m/z 177

### Etape 2a :

### (4-Chloro-5-fluoro-6-methoxy-pyrimidin-2-yl)-acetic acid methyl ester

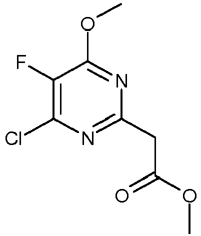

A une solution de 6.7 g du 4-chloro-5-fluoro-6-méthoxy-2-méthyl-pyrimidine et 4.83 mL de chloroformiate de méthyle dans 100 mL de THF anhydre refroidit à -60°C dans un bain de carboglace / MeOH, sont additionnés, goutte à goutte, 95 mL de LDA 2M (THF).

Le milieu réactionnel est agité à -60°C pendant une heure.

Le bain refroidissant est baissé pour laisser la température monter à 22°C. Le milieu réactionnel est agité à 22°C pendant deux heures.

Addition de 20 mL d'eau et 150 mL d'acétate d'éthyle. Après décantation, la phase organique est séchée sur sulfate de magnésium, filtrée puis concentrée sous pression réduite. Le résidu obtenu est purifié sur cartouche de silice : éluant : DCM pour donner 8.36 g de (4-chloro-5-fluoro-6-methoxy-pyrimidin-2-yl)-acetic acid methyl ester sous forme d'une huile jaune vif dont les caractéristiques sont les suivantes :
Spectrométrie de Masse : méthode A
Temps de rétention Tr (min) = 0,84 ; [M+H]+ : m/z 235 ;

### Etape 3a :

### (5-Fluoro-4-methoxy-6-morpholin-4-yl-pyrimidin-2-yl)-acetic acid methyl ester

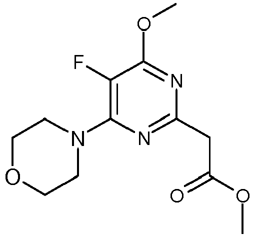

La solution de 8.36 g du (4-chloro-5-fluoro-6-methoxy-pyrimidin-2-yl)-acetic acid methyl ester dans 76 mL de morpholine est agitée à la température ambiante pendant une heure et demie. Le milieu réactionnel est concentré sous pression réduite. Le résidu obtenu est repris avec 50 mL d'eau et 200 mL d'acétate d'éthyle. Après décantation, la phase organique est séchée sur sulfate de magnésium, filtrée puis concentrée sous pression réduite pour donner 8.86 g de (5-fluoro-4-methoxy-6-morpholin-4-yl-pyrimidin-2-yl)-acetic acid methyl ester sous forme d'un solide beige dont les caractéristiques sont les suivantes :
Spectrométrie de Masse : méthode A
Temps de rétention Tr (min) = 0,84 ; [M+H]+ : m/z 286 ;

### Etape 4a :

### (5-Fluoro-4-methoxy-6-morpholin-4-yl-pyrimidin-2-yl)-acetate de sodium

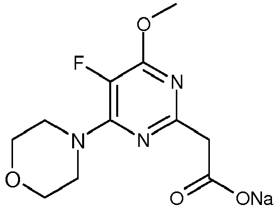

A une solution de 3.46 g du (5-fluoro-4-methoxy-6-morpholin-4-yl-pyrimidin-2-yl)-acetic acid methyl ester dans 36 mL de THF est ajouté 7.9 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 3.7 g de (5-fluoro-4-methoxy-6-morpholin-4-yl-pyrimidin-2-yl)-acetate de sodium dont les caractéristiques sont les suivantes :
Spectrométrie de Masse : méthode A
Temps de rétention Tr (min) = 0,66 ; [M+H]+ : m/z 272 ;
[M-H]- : m/z 270 ; pic de base : m/z 226

### Etape 5a:

### 1-(4-Fluoro-2,3-dihydro-indol-1-yl)-2-(5-fluoro-4-methoxy-6-morpholin-4-yl-pyrimidin-2-yl)-ethanone

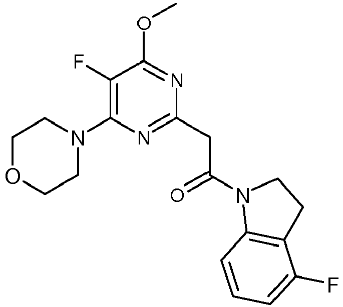

A une solution de 100 mg du (5-fluoro-4-methoxy-6-morpholin-4-yl-pyrimidin-2-yl)-acetate de sodium dans 0.7 mL de DMF et 0.06 mL de pyridine sont ajoutés 59 mg de 4-fluoro-2,3-dihydro-1H-indole et 78 mg de N-[3-(diméthylamino)propyl]-N'-éthylcarbodiimide chlorhydrate.

Le milieu réactionnel est agité à la température ambiante pendant 18 heures.

Addition de 10 mL d'acétate d'éthyle et 5 mL d'eau. Après décantation, la phase organique est séchée sur sulfate de magnésium, filtrée puis concentrée sous pression réduite. Le solide obtenu est repris à l'éther éthylique puis filtré pour donner 70 mg de 1-(4-fluoro-2,3-dihydro-indol-1-yl)-2-(5-fluoro-4-methoxy-6-morpholin-4-yl-pyrimidin-2-yl)-ethanone sous forme d'un solide blanc dont les caractéristiques sont les suivantes :
Spectrométrie de Masse : méthode A
Temps de rétention Tr (min) = 1,01; [M+H]+ : m/z 391 ;

### Etape 6a:

### 5-Fluoro-2-[2-(4-fluoro-2,3-dihydro-indol-1-yl)-2-oxo-ethyl]-6-morpholin-4-yl-3H-pyrimidin-4-one

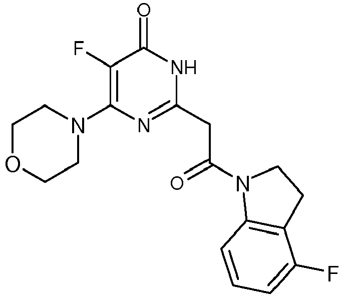

Dans un ballon, 70 mg du 1-(4-fluoro-2,3-dihydro-indol-1-yl)-2-(5-fluoro-4-methoxy-6-morpholin-4-yl-pyrimidin-2-yl)-ethanone avec 0.9 mL d'acétonitrile.

Addition de 89 mg de KI et de 0.07 mL de trimethylchlorosilane. La suspension est agitée à la température ambiante pendant la nuit.

Le milieu réactionnel est concentré sous pression réduite.

Le résidu obtenu est repris avec de l'eau et de l'éther éthylique. Le solide formé est filtré, lavé avec de l'eau et de l'éther éthylique puis séché sous vide pour donner 53 mg du 5-fluoro-2-[2-(4-fluoro-2,3-dihydro-indol-1-yl)-2-oxo-ethyl]-6-morpholin-4-yl-3H-pyrimidin-4-one sous forme d'un solide blanc.
Spectre RMN 1 H (400 MHz, d en ppm, DMSO-d6) : 3,19 (t, *J*=8,3 Hz, 2 H) ; 3,55 (m, 4 H) ; 3,63 (m, 4 H) ; 3,77 (s, 2 H) ; 4,19 (t, J=8,3 Hz, 2 H) ; 6,87 (t, J=8,6 Hz, 1 H) ; 7,22 (m, 1 H) ; 7,83 (d, *J*=8,1 Hz, 1 H) ; 12,29 (m étalé, 1 H)
Spectrométrie de Masse : méthode A
Temps de rétention Tr (min) = 0,72 ; [M+H]+ : m/z 377 ; [M-H]- : m/z 375

### Exemple 2a et Exemple 3a: Synthèse du 5-fluoro-2-[1-(4-fluoro-2,3-dihydro-1H-indol-1-yl)-1-oxopropan-2-yl]-3-méthyl-6-(morpholin-4-yl)pyrimidin-4(3H)-one et du 5-fluoro-2-[2-(4-fluoro-2,3-dihydro-1H-indol-1-yl)-2-oxoéthyl]-3-méthyl-6-(morpholin-4-yl)pyrimidin-4(3H)-one

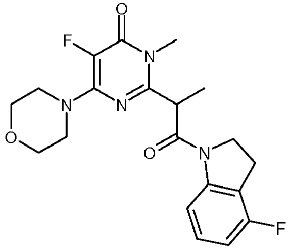

Dans un ballon, 70 mg du 5-fluoro-2-[2-(4-fluoro-2,3-dihydro-indol-1-yl)-2-oxo-ethyl]-6-morpholin-4-yl-3H-pyrimidin-4-one avec 5 mL de DMF.

Addition de 180 mg de carbonate de césium et 0.015 mL d'iodomethane. La suspension est agitée à la température ambiante pendant une heure.

Addition de 20 mL d'eau et 30 mL d'acétate d'éthyle. Après décantation, la phase organique est séchée sur sulfate de magnésium, filtrée puis concentrée sous pression réduite. Le résidu obtenu est purifié sur colonne de silice : éluant dichlorométhane/méthanol 98/02 pour donner 6 mg du 5-fluoro-2-[1-(4-fluoro-2,3-dihydro-1H-indol-1-yl)-1-oxopropan-2-yl]-3-méthyl-6-(morpholin-4-yl)pyrimidin-4(3H)-one dont les caractéristiques sont les suivants :.
Spectre RMN 1 H (400 MHz, d en ppm, DMSO-d6) pour ce lot, nous observons un mélange 50%-50% dez conformères avec : 1,44 (d, *J*=6,8 Hz, 3 H) ; 3,18 (t, *J*=8,6 Hz, 2 H) ; 3,39 (s, 3 H) ; 3,55 (m, 8 H) ; 3,98 (m, 1 H) ; 4,30 (m, 1 H) ; 4,39 (q, *J*=6,8 Hz, 1 H) ; 6,87 (t, *J*=8,3 Hz, 1 H) ; 7,22 (dt, *J*=5,9 et 8,3 Hz, 1 H) ; 7,87 (d, *J*=8,3 Hz, 1 H)
Spectrométrie de Masse : méthode A
Temps de rétention Tr (min) = 0,80 ; [M+H]+ : m/z 405 ; [M-H]- : m/z 403

Et 24 mg du 5-fluoro-2-[2-(4-fluoro-2,3-dihydro-indol-1-yl)-2-oxo-ethyl]-3-methyl-6-morpholin-4-yl-3H-pyrimidin-4-one dont les caractéristiques sont les suivants :
Spectre RMN 1 H (400 MHz, d en ppm, DMSO-d6) : 3,19 (t, *J*=8,6 Hz, 2 H) ; 3,36 (s, 3 H) ; 3,51 à 3,63 (m, 8 H) ; 4,11 (s, 2 H) ; 4,24 (t, *J*=8,6 Hz, 2 H) ; 6,88 (t, *J*=8,3 Hz, 1 H) ; 7,22 (dt, *J*=5,9 et 8,3 Hz, 1 H) ; 7,83 (d, *J*=8,3 Hz, 1 H)
Spectrométrie de Masse : méthode A
Temps de rétention Tr (min) = 0,77 ; [M+H]+ : m/z 391 ; [M-H]- : m/z 389

### Exemple 4a et Exemple 5a: Synthèse du 5-fluoro-2-{2-[(2R)-2-méthyl-2,3-dihydro-1H-indol-1-yl]-2-oxoéthyl}-6-(morpholin-4-yl)pyrimidin-4(3H)-one et du 5-fluoro-2-{2-[(2S)-2-méthyl-2,3-dihydro-1H-indol-1-yl]-2-oxoéthyl}-6-(morpholin-4-yl)pyrimidin-4(3H)-one

### Etape 1a:

### 2-(5-Fluoro-4-methoxy-6-morpholin-4-yl-pyrimidin-2-yl)-1-(2-methyl-2,3-dihydro-indol-1-yl)-ethanone

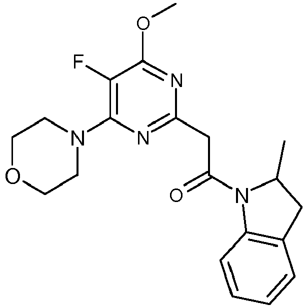

Le produit est préparé en suivant le mode opératoire décrit à l'exemple 1 a (Etape 5a) à partir de 600 mg du (5-fluoro-4-methoxy-6-morpholin-4-yl-pyrimidin-2-yl)-acetate de sodium obtenu à l'exemple 1a (Etape 4a) et de 272 mg de 2-methyl-2,3-dihydro-1H-indole. Après purification par chromatographie sur colonne de silice (éluant : dichlorométhane/acétate d'éthyle 97/03), on obtient 366 mg de 2-(5-fluoro-4-methoxy-6-morpholin-4-yl-pyrimidin-2-yl)-1-(2-methyl-2,3-dihydro-indol-1-yl)-ethanone sous forme d'une poudre blanche dont les caractéristiques sont les suivantes :
Spectrométrie de Masse : méthode A
Temps de rétention Tr (min) = 1,02 ; [M+H]+ : m/z 387 ;

### Etape 2a:

### 5-fluoro-2-{2-[(2R)-2-méthyl-2,3-dihydro-1H-indol-1-yl]-2-oxoéthyl}-6-(morpholin-4-yl)pyrimidin-4(3H)-one et du 5-fluoro-2-{2-[(2S)-2-méthyl-2,3-dihydro-1H-indol-1-yl]-2-oxoéthyll-6-(morpholin-4-yl)pyrimidin-4(3H)-one

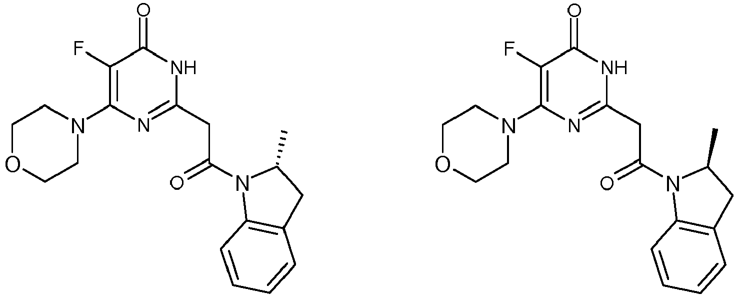

Le 5-fluoro-2-[2-(-2-methyl-2,3-dihydro-indol-1-yl)-2-oxo-ethyl]-6-morpholin-4-yl-3H-pyrimidin-4-one est préparé en suivant le mode opératoire décrit à l'exemple 1a (Etape 6a) à partir de 366 mg du 2-(5-fluoro-4-methoxy-6-morpholin-4-yl-pyrimidin-2-yl)-1-(2-methyl-2,3-dihydro-indol-1-yl)-ethanone, 472 mg d'iodure de potassium et 0.36 de trimethylchlorosilane. Après purification par chromatographie sur colonne de silice (éluant : dichlorométhane/méthanol 98/02), on obtient 75 mg du 5-fluoro-2-[2-(-2-methyl-2,3-dihydro-indol-1-yl)-2-oxo-ethyl]-6-morpholin-4-yl-3H-pyrimidin-4-one sous forme d'un solide dont les caractéristiques sont les suivantes :
Spectrométrie de Masse : méthode A
Temps de rétention Tr (min) = 0,73 ; [M+H]+ : m/z 373 ; [M-H]- : m/z 371

Les énantiomères sont séparés par chromatographie chirale sur colonne:
Phase stationnaire : Chiralpak T304 20µ; phase mobile : EtOH (40%) / MeOH (25%) / Heptane (35%) / triethylamine (0.1%) ; débit : 250 mL/mn.

L'énantiomère dextrogyre est concentré pour obtenir 102 mg de la 5-fluoro-2-[2-((R)-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1H (400 MHz, d en ppm, DMSO-d6) : 1,25 (d large, *J*=6,3 Hz, 3 H) ; 2,69 (d, *J*=16,3 Hz, 1 H) ; 3,20 à 3,42 (m partiellement masqué, 1 H) ; 3,48 à 3,65 (m, 8 H) ; 3,71 (d, *J*=15,8 Hz, 1 H) ; 3,91 (d, *J*=15,8 Hz, 1 H) ; 4,70 (m, 1 H) ; 7,04 (t, *J*=8,1 Hz, 1 H) ; 7,18 (t, *J*=8,1 Hz, 1 H) ; 7,29 (d, *J*=8,1 Hz, 1 H) ; 7,96 (d, *J*=8,1 Hz, 1 H) ; 12,28 (m étalé, 1 H)
Spectrométrie de Masse : méthode A
Temps de rétention Tr (min) = 0,73 ; [M+H]+ : m/z 373 ; [M-H]- : m/z 371
Pouvoir rotatoire : α_{D} = +90 ; C=0.845mg/0.5ML DMSO

L'énantiomère lévogyre est concentré pour obtenir 88 mg de la 5-fluoro-2-[2-((S)-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (400 MHz, d en ppm, DMSO-d6) : 1,25 (d, *J*=6,3 Hz, 3 H) ; 2,69 (d, *J*=16,3 Hz, 1 H) ; 3,22 à 3,43 (m partiellement masqué, 1 H) ; 3,52 à 3,66 (m, 8 H) ; 3,72 (d, *J*=15,8 Hz, 1 H) ; 3,93 (d, *J*=15,8Hz, 1 H) ; 4,69 (m, 1 H) ; 7,05 (t, *J*=8,1 Hz, 1 H) ; 7,18 (t, *J*=8,1 Hz, 1 H) ; 7,29 (d, *J*=8,1 Hz, 1 H) ; 7,96 (d, *J*=8,1 Hz, 1 H) ; 12,25 (m étalé, 1 H)
Spectrométrie de Masse : méthode A
Temps de rétention Tr (min) = 0,73 ; [M+H]+ : m/z 373 ; [M-H]- : m/z 371
Pouvoir rotatoire : α_{D} = -35 ; C=0.910mg/0.5ML DMSO.

### Exemple 6a et Exemple 7a: Synthèse du 5-Fluoro-2-[2-((+)-5-fluoro-2-methyl-2,3-dihydro-indol-1-yl)-2-oxo-ethyl]-6-morpholin-4-yl-3H-pyrimidin-4-one et du 5-Fluoro-2-[2-((-)-5-fluoro-2-methyl-2,3-dihydro-indol-1-yl)-2-oxo-ethyl]-6-morpholin-4-yl-3H-pyrimidin-4-one

### Etape 1a:

### (5-Fluoro-4-morpholin-4-yl-6-oxo-1,6-dihydro-pyrimidin-2-yl)-acetic acid methyl ester

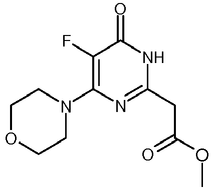

Dans un ballon, à une solution de 386 mg du (5-fluoro-4-methoxy-6-morpholin-4-yl-pyrimidin-2-yl)-acetic acid methyl ester obtenu à l'exemple 1a (Etape 3a) dans 4.7 mL d'acétonitrile, sont ajoutés 722 mg de KI et de 0.56 mL de trimethylchlorosilane. La suspension est agitée à la température ambiante pendant 24 heures.

Le milieu réactionnel est concentré sous pression réduite.

Le résidu obtenu est repris avec de l'eau et de l'acétate d'éthyle. Après décantation, la phase organique est séchée sur sulfate de magnésium, filtrée puis concentrée sous pression réduite. Le résidu obtenu est purifié chromatographie sur colonne de silice (éluant : dichlorométhane/méthanol 98/02) pour donner 155 mg du (5-fluoro-4-morpholin-4-yl-6-oxo-1,6-dihydro-pyrimidin-2-yl)-acetic acid methyl ester sous forme d'un solide beige dont les caractéristiques sont les suivantes :
Spectrométrie de Masse : méthode A
Temps de rétention Tr (min) = 0,43 ; [M+H]+ : m/z 272 ;
[M-H]- : m/z 270 ; pic de base 238

### Etape 2a :

### (5-Fluoro-4-morpholin-4-yl-6-oxo-1,6-dihydro-pyrimidin-2-yl)-acetate de sodium

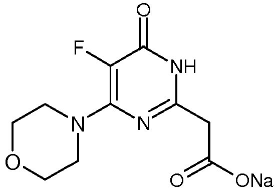

A une solution de 116 mg du (5-fluoro-4-morpholin-4-yl-6-oxo-1,6-dihydro-pyrimidin-2-yl)-acetic acid methyl ester dans 1.2 mL de THF est ajouté 0.43 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 110 mg de (5-fluoro-4-morpholin-4-yl-6-oxo-1,6-dihydro-pyrimidin-2-yl)-acetate de sodium dont les caractéristiques sont les suivantes :
Spectrométrie de Masse : méthode A
Temps de rétention Tr (min) = 0,32 ; [M+H]+ : m/z 258 ;
[M-H]- : m/z 256 ; pic de base : m/z 212

### Etape 2'a:

Alternativement, le composé (5-fluoro-4-morpholin-4-yl-6-oxo-1,6-dihydropyrimidin-2-yl)-acetate de sodium peut être obtenu en trois étapes :

### Etape (2'a)a[4-(morpholin-4-yl)-6-oxo-1,6-dihydropyrimidin-2-yl]acétate d'éthyle

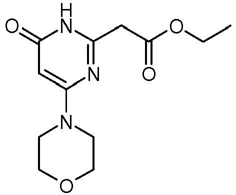

A une solution de 25 g de morpholine dans 400 mL d'éthanol chauffée à 95 °C sont ajoutés 168.5 mL de chlorhydrate de 3-éthoxy-3-iminopropanoate d'éthyle, puis 155 mL de N,N-diisopropyléthylamine dans 200 mL d'éthanol. Le mélange réactionnel est chauffé à 95 °C pendant 30 heures puis laissé revenir à température ambiante. Le précipité formé est filtré sur verre fritté puis lavé avec 100 mL d'éthanol, 2 fois 500 mL d'eau et enfin 500 mL d'éther éthylique. Le solide est séché sous vide pour donner 35 g de [4-(morpholin-4-yl)-6-oxo-1,6-dihydropyrimidin-2-yl]acétate d'éthyle sous forme de solide blanc dont les caractéristiques sont les suivantes :
Spectre RMN 1H (400MHz) : 1,19 (t, *J*=7,1 Hz, 3 H); 3,38 à 3,44 (m, 4 H); 3,56 (s, 2H); 3,61 (dd, J=4,0 et 5,7 Hz, 4 H); 4,12 (q, J=7,1 Hz, 2 H); 5,20 (s, 1 H); 11,69 (s large, 1 H)
Spectrométrie de Masse : méthode A
Temps de rétention Tr (min) = 0.48;
[M+H]+ : m/z 268 ; [M-H]- : m/z 266

### Etape (2'b)a:

### (5-Fluoro-4-morpholin-4-yl-6-oxo-1,6-dihydro-pyrimidin-2-yl)-acetic acid éthyl ester

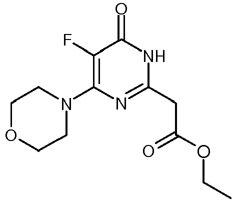

Dans un ballon, 5 g du [4-(morpholin-4-yl)-6-oxo-1,6-dihydropyrimidin-2-yl]acétate d'éthyle obtenu à l'étape précédente avec 50 mL d'acétonitrile sont chauffés à 74°C. Sur cette solution, et à 74°C, est ajouté, goutte à goutte, une solution de 7.67 g de Select fluor solubilisés dans un mélange de 25 mL d'eau et 25 mL d'acétonitrile.

Le milieu réactionnel est chauffé à 75°C pendant 90 minutes.

Après refroidissement, addition de 200 mL d'acétate d'éthyle puis 100 mL d'une solution saturée de bicarbonate de sodium. Après décantation, la phase organique est séchée sur sulfate de magnésium, filtrée puis concentrée sous pression réduite. Le résidu obtenu est purifié par chromatographie sur colonne de silice (éluant : gradient dichlorométhane/méthanol de 100/0 à 95/05) pour donner 0.8 g du (5-fluoro-4-morpholin-4-yl-6-oxo-1,6-dihydro-pyrimidin-2-yl)-acetic acid éthyl ester sous forme d'un solide beige dont les caractéristiques sont les suivantes :
Spectre RMN 1H (400 MHz, d en ppm, DMSO-d6) : 1,19 (t, *J*=7,1 Hz, 3 H) ; 3,56 (m, 6 H) ; 3,63 (m, 4 H) ; 4,12 (q, *J*=7,1 Hz, 2 H) ; 12,32 (m étalé, 1 H)

### Etape (2'c)a :

### (5-Fluoro-4-morpholin-4-yl-6-oxo-1,6-dihydro-pyrimidin-2-yl)-acetate de sodium

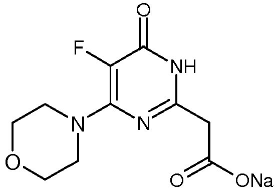

A une solution de 800 mg du (5-fluoro-4-morpholin-4-yl-6-oxo-1,6-dihydropyrimidin-2-yl)-acetic acid éthyl ester dans 10 mL de THF est ajouté 2.9 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 780 mg de (5-fluoro-4-morpholin-4-yl-6-oxo-1,6-dihydro-pyrimidin-2-yl)-acetate de sodium dont les caractéristiques sont les suivantes :
Spectrométrie de Masse : méthode A
Temps de rétention Tr (min) = 0,32 ; [M+H]+ : m/z 258 ;
[M-H]- : m/z 256 ; pic de base : m/z 212

### Etape 3a:

### 5-Fluoro-2-[2-((+)-5-fluoro-2-methyl-2,3-dihydro-indol-1-yl)-2-oxo-ethyl]-6-morpholin-4-yl-3H-pyrimidin-4-one et 5-Fluoro-2-[2-((-)-5-fluoro-2-methyl-2,3-dihydro-indol-1-yl)-2-oxo-ethyl]-6-morpholin-4-yl-3H-pyrimidin-4-one

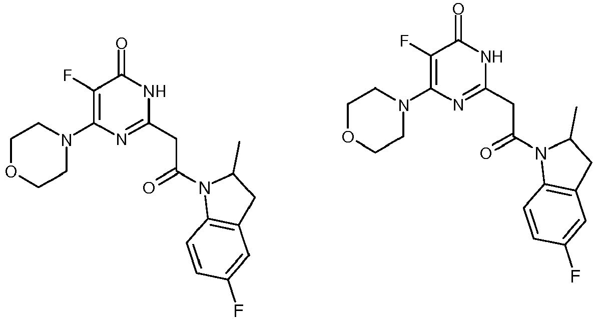

Le 5-fluoro-2-[2-(5-fluoro-2-methyl-2,3-dihydro-indol-1-yl)-2-oxo-ethyl]-6-morpholin-4-yl-3H-pyrimidin-4-one est préparé en suivant le mode opératoire décrit à l'exemple 1a (Etape 5a) à partir de 182 mg du (5-fluoro-4-morpholin-4-yl-6-oxo-1,6-dihydro-pyrimidin-2-yl)-acetate de sodium (obtenu à l'étape 2a) et de 197 mg de 5-fluoro-2-methyl-2,3-dihydro-1H-indole (exemple de référence 1a). Après purification sur colonne de silice, éluant : dichloromethane/methanol 98/02, on obtient 85 mg de 5-fluoro-2-[2-(5-fluoro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectrométrie de Masse : méthode A
Temps de rétention Tr (min) = 0,75 ; [M+H]+ : m/z 391 ; [M-H]- : m/z 389

Les énantiomères sont séparés par chromatographie chirale sur colonne:
Phase stationnaire : Chiralpak AY 20µm lot KLB001; phase mobile : acétonitrile (98%) / isopropanol (02%) ; débit : 180 mL/mn.

L'énantiomère dextrogyre est concentré pour obtenir 66 mg de la 5-fluoro-2-[2-((+)-5-fluoro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (400 MHz, d en ppm, DMSO-d6) : 1,25 (d, *J*=6,4 Hz, 3 H) ; 2,68 (d, *J*=16,3 Hz, 1 H) ; 3,37 (m, 1 H) ; 3,51 (m, 4 H) ; 3,58 à 3,70 (m, 5 H) ; 3,89 (m, 1 H) ; 4,73 (m, 1 H) ; 7,00 (dt, *J*=3,0 et 8,4 Hz, 1 H) ; 7,15 (d large, *J*=9,5 Hz, 1 H) ; 7,94 (dd, *J*=5,0 et 8,4 Hz, 1 H) ; 12,32 (m étalé, 1 H)
Spectrométrie de Masse : méthode A
Temps de rétention Tr (min) = 0,75 ; [M+H]+ : m/z 391 ; [M-H]- : m/z 389
Pouvoir rotatoire : α_{D} = +70.8+/-1.3 ; C=1.773mg/0.5ML DMSO

L'énantiomère lévogyre est concentré pour obtenir 58 mg de la 5-fluoro-2-[2-((-)-5-fluoro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (400 MHz, d en ppm, DMSO-d6) pour ce lot, tous les signaux sont larges avec : 1,25 (d, *J*=6,3 Hz, 3 H) ; 2,69 (d, *J*=16,3 Hz, 1 H) ; 3,36 (m, 1 H) ; 3,52 (m, 4 H) ; 3,58 à 3,70 (m, 5 H) ; 3,86 (m, 1 H) ; 4,74 (m, 1 H) ; 7,00 (m, 1 H) ; 7,15 (d, *J*=9,5 Hz, 1 H) ; 7,93 (m, 1 H) ; 12,28 (m étalé, 1 H)
Spectrométrie de Masse : méthode A
Temps de rétention Tr (min) = 0,76 ; [M+H]+ : m/z 391 ; [M-H]- : m/z 389
Pouvoir rotatoire : α_{D} = -72.4+/-1.4 ; C=1.662mg/0.5ML DMSO.

### Exemple 8a: Synthèse du 5-fluoro-3-méthyl-2-{2-[(2S)-2-méthyl-2,3-dihydro-1H-indol-1-yl]-2-oxoéthyl}-6-(morpholin-4-yl)pyrimidin-4(3H)-one

### Etape 1 a :

### (5-Fluoro-1-methyl-4-morpholin-4-yl-6-oxo-1,6-dihydro-pyrimidin-2-yl)-acetic acid methyl ester

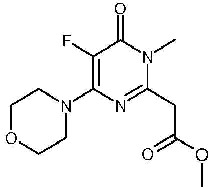

Dans un ballon, 2 g du (5-fluoro-4-morpholin-4-yl-6-oxo-1,6-dihydro-pyrimidin-2-yl)-acetic acid methyl ester avec 50 mL d'acétonitrile.

Addition de 3.12 g de carbonate de césium et 0.6 mL d'iodomethane. La suspension est agitée à la température ambiante pendant 18 heures.

Le milieu réactionnel est filtré. Le filtrat est concentré sous pression réduite. Le résidu obtenu est purifié sur colonne de silice : éluant dichlorométhane/méthanol 98/02 pour donner 759 mg du (5-fluoro-1-methyl-4-morpholin-4-yl-6-oxo-1,6-dihydro-pyrimidin-2-yl)-acetic acid methyl ester dont les caractéristiques sont les suivants :.
Spectrométrie de Masse : méthode A
Temps de rétention Tr (min) = 0,47 ; [M+H]+ : m/z 286 ; [M-H]- : m/z 284

### Etape 2a :

### (5-Fluoro-1-methyl-4-morpholin-4-yl-6-oxo-1,6-dihydro-pyrimidin-2-yl)-acetate de sodium

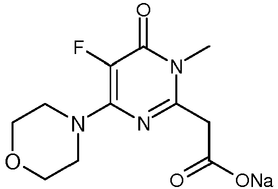

A une solution de 759 mg du (5-Fluoro-1-methyl-4-morpholin-4-yl-6-oxo-1,6-dihydro-pyrimidin-2-yl)-acetic acid methyl ester dans 8 mL de THF est ajouté 2 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 695 mg de (5-fluoro-1-methyl-4-morpholin-4-yl-6-oxo-1,6-dihydro-pyrimidin-2-yl)-acetate de sodium sous forme d'un solide blanc qui est utilisé tel quel dans l'étape suivante.
Spectrométrie de Masse : méthode A
[M+H]+ : m/z 272 ; [M-H]- : m/z 270 ; pic de base : m/z 226

### Etape 3a:

### Synthèse du 5-fluoro-3-méthyl-2-{2-[(2S)-2-méthyl-2,3-dihydro-1H-indol-1-yl]-2-oxoéthyl}-6-(morpholin-4-yl)pyrimidin-4(3H)-one

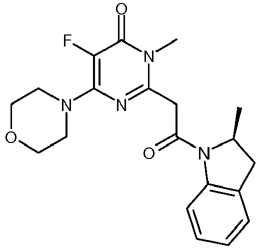

Le produit est préparé en suivant le mode opératoire décrit à l'exemple 1 a (Etape 5a) à partir de 88 mg du (5-fluoro-1-methyl-4-morpholin-4-yl-6-oxo-1,6-dihydro-pyrimidin-2-yl)-acetate de sodium décrit à l'étape 2a et de 40 mg de (S)-2-methyl-2,3-dihydro-1H-indole (qui peut être préparée selon Krasnov, V.P. et Coll. (Mendeleev Commun. (2002), 12(1), 27-28). Après purification sur colonne de silice: éluant dichlorométhane/méthanol 98/02, on obtient 60 mg du 5-fluoro-3-methyl-2-[2-((S)-2-methyl-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1 H (400 MHz, d en ppm, DMSO-d6) : 1,27 (d, *J*=6,4 Hz, 3 H) ; 2,69 (d, *J*=16,3 Hz, 1 H) ; 3,38 (s, 3 H) ; 3,39 (m partiellement masqué, 1 H) ; 3,50 à 3,/ 1 H) ; 7,06 (t, *J*=8,1 Hz, 1 H) ; 7,18 (t, J=8,1 Hz, 1 H) ; 7,29 (d, *J*=8,1 Hz, 1 H) ; 7,94 (d, *J*=8,1 Hz, 1 H)
Spectrométrie de Masse : méthode A
Temps de rétention Tr (min) = 0,77 ; [M+H]+ : m/z 387 ; [M-H]- : m/z 385
Pouvoir rotatoire : α_{D} = -36.0+/-1.0. C= 1.608mg/0.5ML DMSO

### Exemple 9a: Synthèse du 5-fluoro-3-méthyl-2-{2-[(2R)-2-méthyl-2,3-dihydro-1H-indol-1-yl]-2-oxoéthyl}-6-(morpholin-4-yl)pyrimidin-4(3H)-one

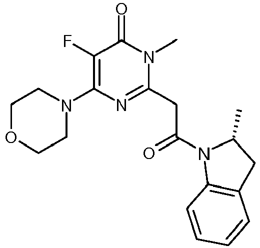

Le produit est préparé en suivant le mode opératoire décrit à l'exemple 1 a (Etape 5a) à partir de 88 mg du (5-fluoro-1-methyl-4-morpholin-4-yl-6-oxo-1,6-dihydro-pyrimidin-2-yl)-acetate de sodium obtenu à l'étape 2a de l'exemple 8a et de 40 mg de (R)-2-methyl-2,3-dihydro-1H-indole (qui peut être préparée selon Krasnov, V.P. et Coll. (Mendeleev Commun. (2002), 12(1), 27-28). Après purification sur colonne de silice: éluant dichlorométhane/méthanol 98/02, on obtient 62 mg du 5-fluoro-3-methyl-2-[2-((R)-2-methyl-2,3-dihydro-indol-1-yl)-2-oxo-ethyl]-6-morpholin-4-yl-3H-pyrimidin-4-one
sous forme d'un solide dont les caractéristiques sont les suivantes :
Spectre RMN 1 H (400 MHz, d en ppm, DMSO-d6) : 1,27 (d, *J*=6,4 Hz, 3 H) ; 2,69 (d, *J*=16,3 Hz, 1 H) ; 3,38 (s, 3 H) ; 3,40 (m, 1 H) ; 3,51 à 3,62 (m, 8 H) ; 4,01 (d, *J*=16,9 Hz, 1 H) ; 4,28 (d, *J*=16,9 Hz, 1 H) ; 4,69 (m, 1 H) ; 7,05 (t, *J*=8,1 Hz, 1 H) ; 7,18 (t, *J*=8,1 Hz, 1 H) ; 7,29 (d, *J*=8,1 Hz, 1 H) ; 7,94 (d, *J*=8,1Hz, 1 H)
Spectrométrie de Masse : méthode A
Temps de rétention Tr (min) = 0,77 ; [M+H]+ : m/z 387 ; [M-H]- : m/z 385
Pouvoir rotatoire :α_{D} = +57. C=1.469mg/0.5ML DMSO

### Exemple 10a: Synthèse du 5-fluoro-2-[2-((+)-2-methyl-4-fluoro-2,3-dihydro-indol-1-yl)-2-oxo-ethyl]-6-morpholin-4-yl-3H-pyrimidin-4-one

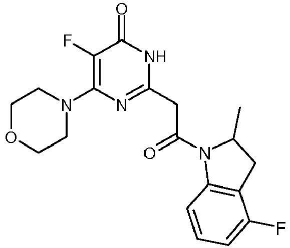

Le produit est préparé en suivant le mode opératoire décrit à l'exemple 1 a (Etape 5a) à partir de 124 mg du (5-fluoro-4-morpholin-4-yl-6-oxo-1,6-dihydro-pyrimidin-2-yl)-acetate de sodium obtenu à l'étape 2a de l'exemple 6a et de 73 mg de (-)-2-methyl-4-fluoro-2,3-dihydro-1H-indole (Exemple de référence 5a). Après purification sur colonne de silice: éluant dichlorométhane/méthanol 98/02, on obtient 70 mg du 5-fluoro-2-[2-((+)-2-methyl-4-fluoro-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1 H (400 MHz, d en ppm, DMSO-d6) pour ce lot, tous les signaux sont larges avec : 1,28 (d, *J*=6,6 Hz, 3 H) ; 2,76 (d, *J*=16,3 Hz, 1 H) ; 3,36 (m partiellement masqué, 1 H) ; 3,52 à 3,64 (m, 8 H) ; 3,74 (d, J=16,9 Hz, 1 H) ; 3,93 (d, J=16,9 Hz, 1 H) ; 4,78 (m, 1 H) ; 6,90 (t, *J*=8,6 Hz, 1 H) ; 7,23 (m, 1 H) ; 7,78 (d, *J*=8,6 Hz, 1 H) ; 12,33 (m étalé, 1 H)
Spectrométrie de Masse : méthode A
Temps de rétention Tr (min) = 0,77 ; [M+H]+ : m/z 391 ; [M-H]- : m/z 389
Pouvoir rotatoire : α_{D} = +75.1+/-1.3. C= 1.998mg/0.5ML DMSO

### Exemple 11a: Synthèse du 5-fluoro-2-[2-((-)-2-methyl-4-fluoro-2,3-dihydro-indol-1-yl)-2-oxo-ethyl]-6-morpholin-4-yl-3H-pyrimidin-4-one

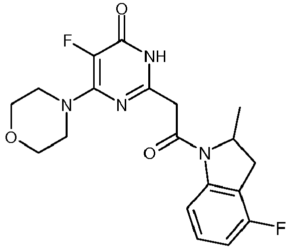

Le produit est préparé en suivant le mode opératoire décrit à l'exemple 1 a (Etape 5a) à partir de 125 mg du (5-fluoro-4-morpholin-4-yl-6-oxo-1,6-dihydro-pyrimidin-2-yl)-acetate de sodium obtenu à l'étape 2a de l'exemple 6a et de 74 mg de (+)-2-methyl-4-fluoro-2,3-dihydro-1H-indole (Exemple de référence 5a). Après purification sur colonne de silice: éluant dichlorométhane/méthanol 98/02, on obtient 105 mg du 5-fluoro-2-[2-((-)-2-methyl-4-fluoro-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1H (400 MHz, d en ppm, DMSO-d6) : 1,28 (d large, *J*=6,6 Hz, 3 H) ; 2,76 (d, *J*=16,3 Hz, 1 H) ; 3,37 (m partiellement masqué, 1 H) ; 3,51 à 3,66 (m, 8 H) ; 3,74 (d, *J*=16,1 Hz, 1 H) ; 3,94 (d, *J*=16,1Hz, 1 H) ; 4,78 (m, 1 H) ; 6,90 (t, *J*=8,6 Hz, 1 H) ; 7,23 (m, 1 H) ; 7,78 (d large, *J*=8,6 Hz, 1 H) ; 12,30 (m étalé, 1 H)
Spectrométrie de Masse : méthode A
Temps de rétention Tr (min) = 0,77 ; [M+H]+ : m/z 391 ; [M-H]- : m/z 389
Pouvoir rotatoire :α_{D} = -83.4+/-1.5. C= 1.719mg/0.5ML DMSO

### Exemple 12a: Synthèse du 2-[2-((+)-5,6-difluoro-2-methyl-2,3-dihydro-indol-1-yl)-2-oxo-ethyl]-5-fluoro-6-morpholin-4-yl-3H-pyrimidin-4-one

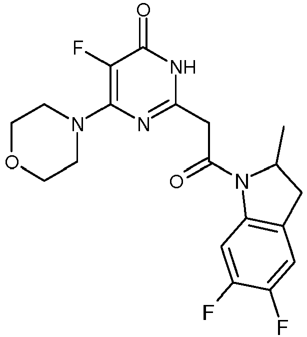

Le produit est préparé en suivant le mode opératoire décrit à l'exemple 1 a (Etape 5a) à partir de 131 mg du (5-fluoro-4-morpholin-4-yl-6-oxo-1,6-dihydro-pyrimidin-2-yl)-acetate de sodium obtenu à l'étape 2a de l'exemple 6a et de 87 mg de (-)-2-methyl-5,6-fluoro-2,3-dihydro-1H-indole (Exemple de référence 3a). Après purification sur colonne de silice: éluant dichlorométhane/méthanol 98/02, on obtient 119 mg du 2-[2-((+)-5,6-difluoro-2-methyl-2,3-dihydro-indol-1-yl)-2-oxo-ethyl]-5-fluoro-6-morpholin-4-yl-3H-pyrimidin-4-one sous forme d'un solide dont les caractéristiques sont les suivantes :
Spectre RMN 1 H (400 MHz, d en ppm, DMSO-d6) : 1,26 (d, *J*=6,4 Hz, 3 H) ; 2,68 (d, J=16,3 Hz, 1 H) ; 3,35 (m partiellement masqué, 1 H) ; 3,55 (m, 4 H) ; 3,62 (m, 4 H) ; 3,73 (d, *J*=16,1 Hz, 1 H) ; 3,92 (d, *J*=16,1 Hz, 1 H) ; 4,73 (m, 1 H) ; 7,39 (dd, *J*=8,5 et 10,5 Hz, 1 H) ; 7,89 (dd, J=7,2 et 12,3 Hz, 1 H) ; 12,34 (m étalé, 1 H)
Spectrométrie de Masse : méthode A
Temps de rétention Tr (min) = 0,79 ; [M+H]+ : m/z 409 ; [M-H]- : m/z 407
Pouvoir rotatoire : α_{D} = +65.9+/-1.2. C=1.931 mg/0.5ML DMSO

### Exemple 13a: Synthèse du 2-[2-((-)-5,6-difluoro-2-methyl-2,3-dihydro-indol-1-yl)-2-oxo-ethyl]-5-fluoro-6-morpholin-4-yl-3H-pyrimidin-4-one

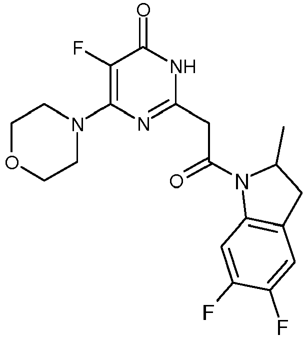

Le produit est préparé en suivant le mode opératoire décrit à l'exemple 1 a (Etape 5a) à partir de 131 mg du (5-fluoro-4-morpholin-4-yl-6-oxo-1,6-dihydro-pyrimidin-2-yl)-acetate de sodium obtenu à l'étape 2a de l'exemple 6a et de 93 mg de (+)-2-methyl-5,6-difluoro-2,3-dihydro-1H-indole (Exemple de référence 3a). Après purification sur colonne de silice: éluant dichlorométhane/méthanol 98/02, on obtient 64 mg du 2-[2-((-)-5,6-difluoro-2-methyl-2,3-dihydro-indol-1-yl)-2-oxo-ethyl]-5-fluoro-6-morpholin-4-yl-3H-pyrimidin-4-one sous forme d'un solide dont les caractéristiques sont les suivantes :
Spectre RMN 1 H (400 MHz, d en ppm, DMSO-d6) : 1,26 (d, *J*=6,4 Hz, 3 H) ; 2,68 (d, *J*=16,3 Hz, 1 H) ; 3,35 (m partiellement masqué, 1 H) ; 3,55 (m, 4 H) ; 3,62 (m, 4 H) ; 3,73 (d, *J*=16,1 Hz, 1 H) ; 3,92 (d, *J*=16,1 Hz, 1 H) ; 4,73 (m, 1 H) ; 7,39 (dd, *J*=8,5 et 10,5 Hz, 1 H) ; 7,89 (dd, *J*=7,2 et 12,3 Hz, 1 H) ; 12,34 (m étalé, 1 H)
Spectrométrie de Masse : méthode A
Temps de rétention Tr (min) = 0,79 ; [M+H]+ : m/z 409 ; [M-H]- : m/z 407
Pouvoir rotatoire : α_{D} = -60.0+/-1.2. C=1.748mg/0.5ML DMSO

### Exemple 14a: Synthèse du 5-fluoro-2-[2-((-)-5-fluoro-2-methyl-2,3-dihydro-indol-1-yl)-2-oxo-ethyl]-3-methyl-6-morpholin-4-yl-3H-pyrimidin-4-one

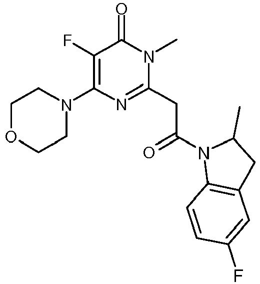

Le produit est préparé en suivant le mode opératoire décrit à l'exemple 1 a (Etape 5a) à partir de 126 mg du (5-fluoro-1-methyl-4-morpholin-4-yl-6-oxo-1,6-dihydro-pyrimidin-2-yl)-acetate de sodium obtenu à l'étape 2a de l'exemple 8a et de 64 mg de (+)-2-methyl-5-fluoro-2,3-dihydro-1H-indole (Exemple de référence 1a). Après purification sur colonne de silice: éluant dichlorométhane/méthanol 98/02, on obtient 80 mg du 5-fluoro-2-[2-((-)-5-fluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,27 (d, *J*=6,6 Hz, 3 H) ; 2,70 (d, *J*=16,3 Hz, 1 H) ; 3,38 (s, 3 H) ; 3,40 (m partiellement masqué, 1 H) ; 3,53 à 3,62 (m, 8 H) ; 4,01 (d, *J*=16,6 Hz, 1 H) ; 4,26 (d, *J*=16,6 Hz, 1 H) ; 4,72 (m, 1 H) ; 7,00 (t large, *J*=9,1 Hz, 1 H) ; 7,16 (d large, *J*=7,8 Hz, 1 H) ; 7,93 (dd, *J*=5,2 et 9,1 Hz, 1 H)
Spectrométrie de Masse : méthode A
Temps de rétention Tr (min) = 0,80 ; [M+H]+ : m/z 405 ; [M-H]- : m/z 403
Pouvoir rotatoire : α_{D} = -44.4+/-1.0. C=1.822mg/0.5ML DMSO

### Exemple 15a: Synthèse du 5-fluoro-2-[2-((+)-5-fluoro-2-methyl-2,3-dihydro-indol-1-yl)-2-oxo-ethyl]-3-methyl-6-morpholin-4-yl-3H-pyrimidin-4-one

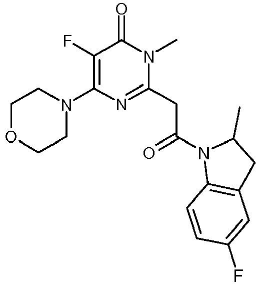

Le produit est préparé en suivant le mode opératoire décrit à l'exemple 1 a (Etape 5a) à partir de 118 mg du (5-fluoro-1-methyl-4-morpholin-4-yl-6-oxo-1,6-dihydro-pyrimidin-2-yl)-acetate de sodium obtenu à l'étape 2a de l'exemple 8a et de 60 mg de (-)-2-methyl-5-fluoro-2,3-dihydro-1H-indole (Exemple de référence 1a). Après purification sur colonne de silice: éluant dichlorométhane/méthanol 98/02, on obtient 89 mg du 5-fluoro-2-[2-((+)-5-fluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,27 (d, *J*=6,4 Hz, 3 H) ; 2,70 (d, *J*=16,3 Hz, 1 H) ; 3,38 (s, 3 H) ; 3,40 (m partiellement masqué, 1 H) ; 3,52 à 3,62 (m, 8 H) ; 4,01 (d, *J*=16,6 Hz, 1 H) ; 4,26 (d, *J*=16,6 Hz, 1 H) ; 4,72 (m, 1 H) ; 7,00 (dt, *J*=2,0 et 9,1 Hz, 1 H) ; 7,16 (d large, *J*=9,1 Hz, 1 H) ; 7,93 (dd, *J*=5,2 et 9,1 Hz, 1 H)
Spectrométrie de Masse : méthode A
Temps de rétention Tr (min) = 0,80 ; [M+H]+ : m/z 405 ; [M-H]- : m/z 403
Pouvoir rotatoire : α_{D} = +57.8+/-1.2. C= 1.840mg/0.5ML DMSO

### Exemple 16a et Exemple 17a: Synthèse du (+)-2-{2-[-4-bromo-2-méthyl-2,3-dihydro-1H-indol-1-yl]-2-oxoéthyl}-5-fluoro-6-(morpholin-4-yl)pyrimidin-4(3H)-one et du (-)-2-{2-[-4-bromo-2-méthyl-2,3-dihydro-1H-indol-1-yl]-2-oxoéthyl}-5-fluoro-6-(morpholin-4-yl)pyrimidin-4(3H)-one

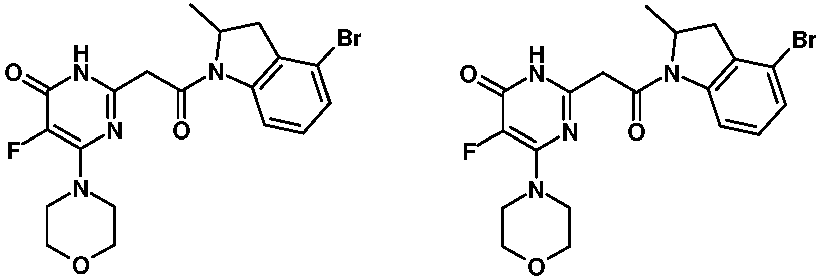

A une solution de 217 mg de (5-fluoro-4-morpholin-4-yl-6-oxo-1,6-dihydro-pyrimidin-2-yl)-acetate de sodium (obtenu à l'étape 2a de l'Exemple 6a) dans 7 ml de N,N-diméthylformamide et 7 ml de pyridine sont ajoutés 150 mg de 4-bromo-2-méthyl-2,3-dihydro-1H-indole [Exemple de référence 4a] et 197 mg de N-[3-(diméthylamino)propyl]-N'-éthylcarbodiimide chlorhydrate. Le mélange réactionnel est agité à température ambiante pendant 72 heures, puis 50 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est trituré dans du dichlorométhane, filtré et lavé avec de l'éther diéthylique pour donner 163 mg de 2-{2-[-4-bromo-2-méthyl-2,3-dihydro-1H-indol-1-yl]-2-oxoéthyl}-5-fluoro-6-(morpholin-4-yl)pyrimidin-4(3H)-one.

Les énantiomères sont séparés par chromatographie chirale sur colonne :
Colonne chirale : phase Whelk 01 SS, 10 µm (10 µm, 77x350 mm), en éluant avec un mélange de : heptane/dichlorométhane/éthanol/méthanol : 69/20/5/6 ; débit : 250 mL/min.

On obtient comme premier énantiomère, 71 mg de (+)-2-{2-[-4-bromo-2-méthyl-2,3-dihydro-1H-indol-1-yl]-2-oxoéthyl}-5-fluoro-6-(morpholin-4-yl)pyrimidin-4(3H)-one, sous forme d'un solide blanc dont les caractéristiques sont les suivantes:
Spectre RMN 1 H (400MHz): 1,29 (d, *J*=6,4 Hz, 3 H); 2,67 (s, 1 H); 3,31 à 3,40 (m, 1 H); 3,51 à 3,64 (m, 8 H); 3,73 (d, J=16,1 Hz, 1 H); 3,93 (d, J=16,1 Hz, 1 H); 4,68 à 4,80 (m, 1 H); 7,16 (t, J=8,2 Hz, 1 H); 7,26 (d, J=8,2 Hz, 1 H); 7,95 (d, J=8,2 Hz, 1 H); 12,30 (d, J=3,2 Hz, 1 H)
Spectrométrie de Masse : méthode B
Temps de rétention Tr (min) = 3,50 ;
[M+H]+ : m/z 452 ; [M-H]- : m/z 450;
Pouvoir rotatoire : α_{D} = +134° (c=1,784 mg/0,5 mL DMSO)

Puis le deuxième énantiomère, 74 mg de (-)-2-{2-[-4-bromo-2-méthyl-2,3-dihydro-1H-indol-1-yl]-2-oxoéthyl}-5-fluoro-6-(morpholin-4-yl)pyrimidin-4(3H)-one sous forme d'un solide blanc dont les caractéristiques sont les suivantes :
Spectre RMN 1 H (400MHz): 1,29 (d, J=6,4 Hz, 3 H) ; 2,67 (m, 1 H) ; 3,32 (m, 1 H) ; 3,50 à 3,65 (m, 8 H) ; 3,73 (d, J=15,9 Hz, 1 H) ; 3,92 (d, J=15,9 Hz, 1 H) ; 4,74 (m, 1 H) ; 7,16 (t, J=8,1 Hz, 1 H) ; 7,26 (d, J=8,1 Hz, 1 H) ; 7,95 (d large, J=8,1 Hz, 1 H) ; 12,32 (m étalé, 1 H)
Spectrométrie de Masse : méthode B
Temps de rétention Tr (min) = 3,50 ;
[M+H]+ : m/z 452 ; [M-H]- : m/z 450;
Pouvoir rotatoire : α_{D} = -109° (c=1,861 mg/0,5 mL DMSO)

### Exemple 18a et Exemple 19a: Synthèse du 5-Fluoro-2-[2-((+)-4-chloro-2-methyl-2,3-dihydro-indol-1-yl)-2-oxo-ethyl]-6-morpholin-4-yl-3H-pyrimidin-4-one et du 5-Fluoro-2-[2-((-)-4-chloro-2-methyl-2,3-dihydro-indol-1-yl)-2-oxo-ethyl]-6-morpholin-4-yl-3H-pyrimidin-4-one

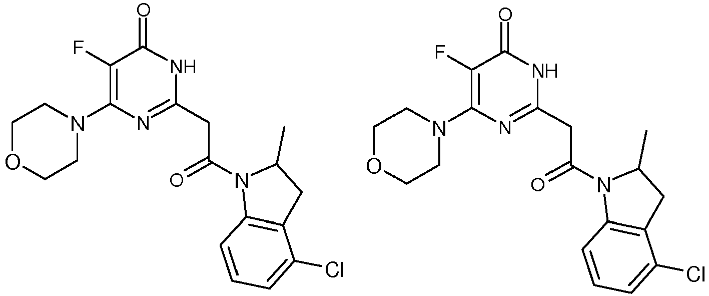

Le 5-fluoro-2-[2-(5-fluoro-2-methyl-2,3-dihydro-indol-1-yl)-2-oxo-ethyl]-6-morpholin-4-yl-3H-pyrimidin-4-one est préparé en suivant le mode opératoire décrit à l'exemple 1a (Etape 5a) à partir de 206 mg du (5-fluoro-4-morpholin-4-yl-6-oxo-1,6-dihydro-pyrimidin-2-yl)-acetate de sodium obtenu à l'étape 2a de l'exemple 6a et de 197 mg de 4-chloro-2-methyl-2,3-dihydro-1H-indole [qui peut être préparée selon le brevet US 4,416,884 (1983)]. Après purification on obtient 151 mg de 5-fluoro-2-[2-(4-chloro-2-methyl-2,3-dihydro-indol-1-yl)-2-oxo-ethyl]-6-morpholin-4-yl-3H-pyrimidin-4-one sous forme d'un solide qui sera séparé sur colonne chirale en ses deux énantiomères.

Les énantiomères sont séparés par chromatographie chirale sur colonne:

Phase stationnaire : Chiralpak AY 20µm (T304); phase mobile : acétonitrile (95%) / isopropanol (05%) ; débit : 250 mL/mn.

L'énantiomère dextrogyre est concentré pour obtenir 58 mg de la 5-fluoro-2-[2-((+)-4-chloro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (400 MHz, d en ppm, DMSO-d6) : 1,28 (d, *J*=6,4 Hz, 3 H) ; 2,72 (d, *J*=16,3 Hz, 1 H) ; 3,37 (dd, *J*=8,6 et 16,3 Hz, 1 H) ; 3,51 à 3,65 (m, 8 H) ; 3,73 (d, *J*=16,4 Hz, 1 H) ; 3,93 (d, *J*=16,4 Hz, 1 H); 4,76 (m, 1 H) ; 7,12 (d, *J*=8,3 Hz, 1 H) ; 7,24 (t, J=8,3 Hz, 1 H) ; 7,91 (d large, *J*=8,3 Hz, 1 H) ; 12,24 (m étalé, 1 H)
Spectrométrie de Masse : méthode A
Temps de rétention Tr (min) = 0,85 ; [M+H]+ : m/z 407 ; [M-H]- : m/z 405
Pouvoir rotatoire : α_{D} = +143. C=0.569mg/0.5ML DMSO

L'énantiomère lévogyre est concentré pour obtenir 64 mg de la 5-fluoro-2-[2-((-)-4-chloro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1H (400 MHz, d en ppm, DMSO-d6) : 1,29 (d large, *J*=6,4 Hz, 3 H) ; 2,72 (d, *J*=16,3 Hz, 1 H) ; 3,37 (dd, *J*=8,6 et 16,3 Hz, 1 H) ; 3,50 à 3,66 (m, 8 H) ; 3,73 (d, J=15,9 Hz, 1 H) ; 3,93 (d, *J*=15,9 Hz, 1 H) ; 4,75 (m, 1 H) ; 7,12 (d, *J*=8,6 Hz, 1 H) ; 7,24 (t, *J*=8,6 Hz, 1 H) ; 7,91 (d large, *J*=8,6 Hz, 1 H) ; 12,32 (m étalé, 1 H)
Spectrométrie de Masse : méthode A
Temps de rétention Tr (min) = 0,85 ; [M+H]+ : m/z 407 ; [M-H]- : m/z 405
Pouvoir rotatoire : α_{D} = -171. C= 0.764mg/0.5ML DMSO.

### Exemple 20a: Synthèse du 2-[2-((-)-4,5-difluoro-2-methyl-2,3-dihydro-indol-1-yl)-2-oxo-ethyl]-5-fluoro-6-morpholin-4-yl-3H-pyrimidin-4-one

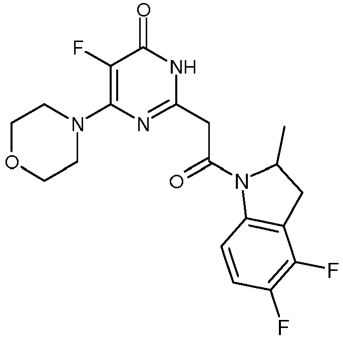

Le produit est préparé en suivant le mode opératoire décrit à l'exemple 1 a (Etape 5a) à partir de 144 mg du (5-fluoro-4-morpholin-4-yl-6-oxo-1,6-dihydro-pyrimidin-2-yl)-acetate de sodium obtenu à l'étape 2a de l'exemple 6a et de 87 mg de (+)-2-methyl-4,5-fluoro-2,3-dihydro-1H-indole (Exemple de référence 2a). Après purification sur colonne de silice: éluant dichlorométhane/méthanol 98/02, on obtient 102 mg du 2-[2-((-)-4,5-difluoro-2-methyl-2,3-dihydro-indol-1-yl)-2-oxo-ethyl]-5-fluoro-6-morpholin-4-yl-3H-pyrimidin-4-one sous forme d'un solide dont les caractéristiques sont les suivantes :
Spectre RMN 1 H (400 MHz, d en ppm, DMSO-d6) : 1,29 (d, *J*=6,4 Hz, 3 H) ; 2,81 (d, *J*=16,6 Hz, 1 H) ; 3,40 (dd, *J*=8,6 et 16,6 Hz, 1 H) ; 3,52 à 3,66 (m, 8 H) ; 3,73 (d, *J*=16,1 Hz, 1 H) ; 3,92 (d, *J*=16,1 Hz, 1 H); 4,794 (m, 1 H) ; 7,24 (m, 1 H) ; 7,74 (d large, *J*=9,0 Hz, 1 H) ; 12,29 (m étalé, 1 H)
Spectrométrie de Masse : méthode A
Temps de rétention Tr (min) = 0,81 ; [M+H]+ : m/z 409 ; [M-H]- : m/z 407
Pouvoir rotatoire : α_{D} =-72.4+/-1.4. C=1.731mg/0.5ML DMSO

### Exemple 21a: Synthèse du 2-[2-((+)-4,5-difluoro-2-methyl-2,3-dihydro-indol-1-yl)-2-oxo-ethyl]-5-fluoro-6-morpholin-4-yl-3H-pyrimidin-4-one

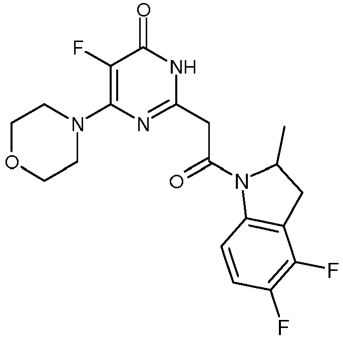

Le produit est préparé en suivant le mode opératoire décrit à l'exemple 1 a (Etape 5a) à partir de 140 mg du (5-fluoro-4-morpholin-4-yl-6-oxo-1,6-dihydro-pyrimidin-2-yl)-acetate de sodium obtenu à l'étape 2a de l'exemple 6a et de 93 mg de (-)-2-methyl-4,5-difluoro-2,3-dihydro-1H-indole (Exemple de référence 2a). Après purification sur colonne de silice: éluant dichlorométhane/méthanol 98/02, on obtient 117 mg du 2-[2-((+)-4,5-difluoro-2-methyl-2,3-dihydro-indol-1-yl)-2-oxo-ethyl]-5-fluoro-6-morpholin-4-yl-3H-pyrimidin-4-one sous forme d'un solide dont les caractéristiques sont les suivantes :
Spectre RMN 1 H (400 MHz, d en ppm, DMSO-d6) : 1,29 (d, *J*=6,4 Hz, 3 H) ; 2,82 (d, *J*=16,6 Hz, 1 H) ; 3,40 (dd, *J*=8,6 et 16,6 Hz, 1 H) ; 3,53 à 3,64 (m, 8 H) ; 3,73 (d, J=16,1 Hz, 1 H) ; 3,92 (d, *J*=16,1 Hz, 1 H); 4,79 (m, 1 H) ; 7,24 (m, 1 H) ; 7,74 (d, *J*=9,0 Hz, 1 H) ; 12,31 (m étalé, 1 H)
Spectrométrie de Masse : méthode A
Temps de rétention Tr (min) = 0,81 ; [M+H]+ : m/z 409 ; [M-H]- : m/z 407
Pouvoir rotatoire : α_{D} = +76.3+/-1.3. C=2.144mg/0.5ML DMSO

### Exemple 22a: Synthèse du 5-fluoro-2-[2-((+)-4-fluoro-2-methyl-2,3-dihydro-indol-1-yl)-2-oxo-ethyl]-3-methyl-6-morpholin-4-yl-3H-pyrimidin-4-one

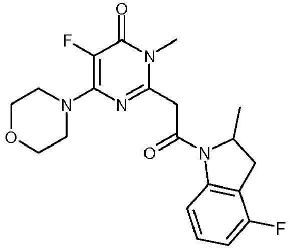

Le produit est préparé en suivant le mode opératoire décrit à l'exemple 1 a (Etape 5a) à partir de 133 mg du (5-fluoro-1-methyl-4-morpholin-4-yl-6-oxo-1,6-dihydro-pyrimidin-2-yl)-acetate de sodium obtenu à l'étape 2a de l'exemple 8a et de 75 mg de (-)-2-methyl-4-fluoro-2,3-dihydro-1H-indole (Exemple de référence 5a). Après purification sur colonne de silice: éluant dichlorométhane/méthanol 98/02, on obtient 78 mg du 5-fluoro-2-[2-((+)-4-fluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,30 (d, *J*=6,3 Hz, 3 H) ; 2,77 (d, *J*=16,1 Hz, 1 H) ; 3,38 (m, 4 H) ; 3,50 à 3,67 (m, 8 H) ; 4,03 (d, *J*=16,9 Hz, 1 H) ; 4,28 (d, *J*=16,9 Hz, 1 H) ; 4,79 (m, 1 H) ; 6,91 (t, *J*=8,6 Hz, 1 H) ; 7,25 (m, 1 H) ; 7,78 (d, *J*=8,6 Hz, 1 H)
Spectrométrie de Masse : méthode A
Temps de rétention Tr (min) = 0,82 ; [M+H]+ : m/z 405 ; [M-H]- : m/z 403
Pouvoir rotatoire :α_{D} = +71.3+/-1.3. C=1.947mg/0.5ML DMSO

### Exemple 23a: Synthèse du 5-fluoro-2-[2-((-)-4-fluoro-2-methyl-2,3-dihydro-indol-1-yl)-2-oxo-ethyl]-3-methyl-6-morpholin-4-yl-3H-pyrimidin-4-one

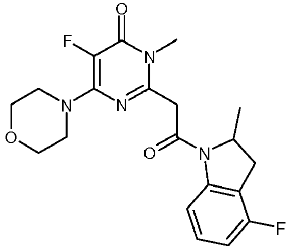

Le produit est préparé en suivant le mode opératoire décrit à l'exemple 1 a (Etape 5a) à partir de 133 mg du (5-fluoro-1-methyl-4-morpholin-4-yl-6-oxo-1,6-dihydro-pyrimidin-2-yl)-acetate de sodium obtenu à l'étape 2a de l'exemple 8a et de 75 mg de (+)-2-methyl-4-fluoro-2,3-dihydro-1H-indole (Exemple de référence 5a). Après purification sur colonne de silice: éluant dichlorométhane/méthanol 98/02, on obtient 90 mg du 5-fluoro-2-[2-((-)-4-fluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,30 (d, *J*=6,6 Hz, 3 H) ; 2,77 (d, *J*=16,3 Hz, 1 H) ; 3,38 (m, 4 H) ; 3,51 à 3,63 (m, 8 H) ; 4,03 (d, *J*=16,6 Hz, 1 H) ; 4,28 (d, *J*=16,6 Hz, 1 H) ; 4,78 (m, 1 H) ; 6,91 (t, *J=*8,6 Hz, 1 H) ; 7,24 (m, 1 H) ; 7,78 (d, *J*=8,6 Hz, 1 H)
Spectrométrie de Masse : méthode A
Temps de rétention Tr (min) = 0,82 ; [M+H]+ : m/z 405 ; [2M+Na]+ : m/z 831
(pic de base); [M-H]- : m/z 403
Pouvoir rotatoire α_{D} = -54.6+/-1.1. C=2.168mg/0.5ML DMSO

### Exemple 24a: Synthèse du 5-fluoro-2-[2-((-)-4,5-difluoro-2-methyl-2,3-dihydro-indol-1-yl)-2-oxo-ethyl]-3-methyl-6-morpholin-4-yl-3H-pyrimidin-4-one

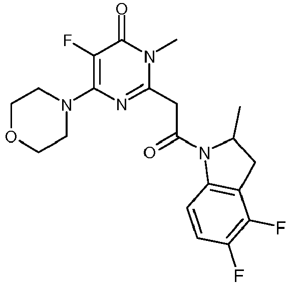

Le produit est préparé en suivant le mode opératoire décrit à l'exemple 1 a (Etape 5a) à partir de 80 mg du (5-fluoro-1-methyl-4-morpholin-4-yl-6-oxo-1,6-dihydro-pyrimidin-2-yl)-acetate de sodium obtenu à l'étape 2a de l'exemple 8a et de 51 mg de (+)-2-methyl-4,5-difluoro -2,3-dihydro-1H-indole (Exemple de référence 2a). Après purification sur colonne de silice: éluant dichlorométhane/méthanol 98/02, on obtient 54 mg du 5-fluoro-2-[2-((-)-4,5-difluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,31 (d, *J*=6,4 Hz, 3 H) ; 2,83 (d, *J*=16,3 Hz, 1 H) ; 3,37 (s, 3 H) ; 3,42 (dd, *J*=8,6 et 16,3 Hz, 1 H) ; 3,51 à 3,66 (m, 8 H) ; 4,02 (d, *J*=16,9 Hz, 1 H) ; 4,27 (d, *J*=16,9 Hz, 1 H) ; 4,80 (m, 1 H) ; 7,25 (m, 1 H) ; 7,73 (dd, *J*=4,1 et 9,1 Hz, 1 H)
Spectrométrie de Masse : méthode A
Temps de rétention Tr (min) = 0,86 ; [M+H]+ : m/z 423 ; [M-H]- : m/z 421
Pouvoir rotatoire : α_{D} = -56.0+/-1.1. C= 2.060mg/0.5ML DMSO

### Exemple 25a: Synthèse 5-fluoro-2-[2-((+)-4,5-difluoro-2-methyl-2,3-dihydro-indol-1-yl)-2-oxo-ethyl]-3-methyl-6-morpholin-4-yl-3H-pyrimidin-4-one

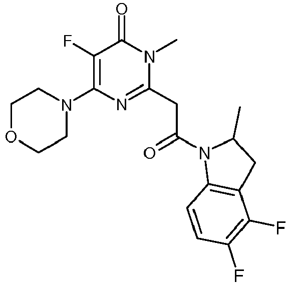

Le produit est préparé en suivant le mode opératoire décrit à l'exemple 1 a (Etape 5a) à partir de 80 mg du (5-fluoro-1-methyl-4-morpholin-4-yl-6-oxo-1,6-dihydro-pyrimidin-2-yl)-acetate de sodium obtenu à l'étape 2a de l'exemple 8a et de 46 mg de (-)-2-methyl-4,5-difluoro-2,3-dihydro-1H-indole (Exemple de référence 2a). Après purification sur colonne de silice: éluant dichlorométhane/méthanol 98/02, on obtient 53 mg du 5-fluoro-2-[2-((+)-4,5-difluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,31 (d, *J*=6,4 Hz, 3 H) ; 2,83 (d, *J*=16,3 Hz, 1 H) ; 3,37 (s, 3 H) ; 3,42 (dd, *J*=8,6 et 16,3 Hz, 1 H) ; 3,52 à 3,65 (m, 8 H) ; 4,02 (d, *J*=16,9 Hz, 1 H) ; 4,27 (d, *J*=16,9 Hz, 1 H) ; 4,75 à 4,85 (m, 1 H) ; 7,25 (m, 1 H) ; 7,73 (dd, *J*=4,0 et 9,0 Hz, 1 H)
Spectrométrie de Masse : méthode A
Temps de rétention Tr (min) = 0,86 ; [M+H]+ : m/z 423 ; [M-H]- : m/z 421
Pouvoir rotatoire : α_{D} = +62.4+/-1.2. C=2.051mg/0.5ML DMSO

### Exemple 26a: Synthèse du 5-fluoro-2-[2-((+)-5,6-difluoro-2-methyl-2,3-dihydro-indol-1-yl)-2-oxo-ethyl]-3-methyl-6-morpholin-4-yl-3H-pyrimidin-4-one

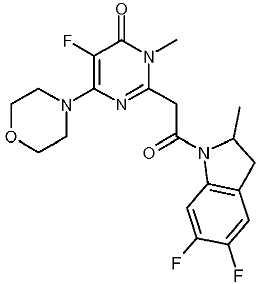

Le produit est préparé en suivant le mode opératoire décrit à l'exemple 1 a (Etape 5a) à partir de 80 mg du (5-fluoro-1-methyl-4-morpholin-4-yl-6-oxo-1,6-dihydro-pyrimidin-2-yl)-acetate de sodium obtenu à l'étape 2a de l'exemple 8a et de 46 mg de (-)-2-methyl-5,6-difluoro -2,3-dihydro-1H-indole (Exemple de référence 3a). Après purification sur colonne de silice: éluant dichlorométhane/méthanol 98/02, on obtient 47 mg du 5-fluoro-2-[2-((+)-5,6-difluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,28 (d, *J*=6,4 Hz, 3 H) ; 2,68 (d, *J*=16,3 Hz, 1 H) ; 3,38 (m, 4 H) ; 3,51 à 3,67 (m, 8 H) ; 4,02 (d, *J*=16,9 Hz, 1 H) ; 4,27 (d, *J*=16,9 Hz, 1 H) ; 4,74 (m, 1 H) ; 7,40 (t, *J*=8,5 Hz, 1 H) ; 7,89 (dd, *J*=7,5 et 12,1 Hz, 1 H)
Spectrométrie de Masse : méthode A
Temps de rétention Tr (min) = 0,85 ; [M+H]+ : m/z 423 ; [M-H]- : m/z 421
Pouvoir rotatoire : α_{D} = +51.5+/-0.9. C=2.396mg/0.5ML DMSO

### Exemple 27a: Synthèse du 5-fluoro-2-[2-((-)-5,6-difluoro-2-methyl-2,3-dihydro-indol-1-yl)-2-oxo-ethyl]-3-methyl-6-morpholin-4-yl-3H-pyrimidin-4-one

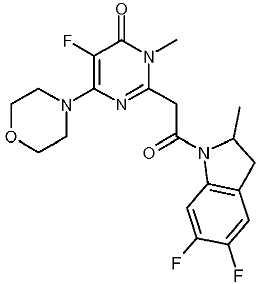

Le produit est préparé en suivant le mode opératoire décrit à l'exemple 1 a (Etape 5a) à partir de 80 mg du (5-fluoro-1-methyl-4-morpholin-4-yl-6-oxo-1,6-dihydro-pyrimidin-2-yl)-acetate de sodium obtenu à l'étape 2a de l'exemple 8a et de 51 mg de (+)-2-methyl-5,6-difluoro-2,3-dihydro-1H-indole (Exemple de référence 3a). Après purification sur colonne de silice: éluant dichlorométhane/méthanol 98/02, on obtient 55 mg du 5-fluoro-2-[2-((-)-5,6-difluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,28 (d, *J*=6,6 Hz, 3 H) ; 2,69 (d, *J*=16,3 Hz, 1 H) ; 3,38 (m, 4 H) ; 3,52 à 3,66 (m, 8 H) ; 4,02 (d, *J*=16,9 Hz, 1 H) ; 4,27 (d, J=16,9 Hz, 1 H) ; 4,74 (m, 1 H) ; 7,40 (t, *J*=8,5 Hz, 1 H) ; 7,89 (dd, *J*=7,6 et 12,2 Hz, 1 H)
Spectrométrie de Masse : méthode A
Temps de rétention Tr (min) = 0,85 ; [M+H]+ : m/z 423 ; [M-H]- : m/z 421
Pouvoir rotatoire : α_{D} = -44.9+/-0.9. C=2.387mg/0.5ML DMSO

### Exemple 28a et Exemple 29a: Synthèse du 5-Fluoro-2-[2-((+)-4-chloro-2-methyl-2,3-dihydro-indol-1-yl)-2-oxo-ethyl]-3-methyl-6-morpholin-4-yl-3H-pyrimidin-4-one et du 5-Fluoro-2-[2-((-)-4-chloro-2-methyl-2,3-dihydro-indol-1-yl)-2-oxo-ethyl]-3-methyl-6-morpholin-4-yl-3H-pyrimidin-4-one

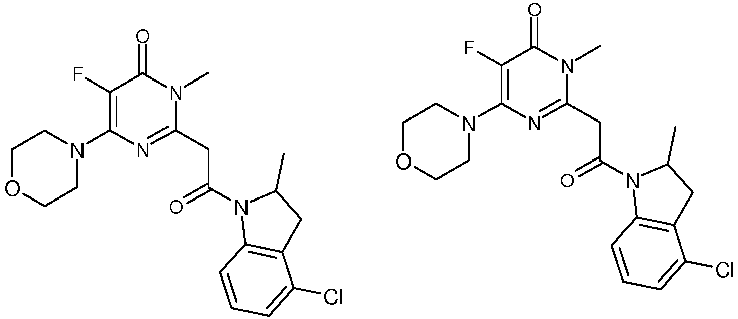

Le 5-Fluoro-2-[2-(4-chloro-2-methyl-2,3-dihydro-indol-1-yl)-2-oxo-ethyl]-3-methyl-6-morpholin-4-yl-3H-pyrimidin-4-one est préparé en suivant le mode opératoire décrit à l'exemple 1a (Etape 5a) à partir de 220 mg du (5-fluoro-1-methyl-4-morpholin-4-yl-6-oxo-1,6-dihydro-pyrimidin-2-yl)-acetate de sodium obtenu à l'étape 2a de l'exemple 8a et de 120 mg de 4-chloro-2-methyl-2,3-dihydro-1H-indole [qui peut être préparée selon le brevet US 4,416,884 (1983)]. Après purification on obtient 151 mg de 5-Fluoro-2-[2-(4-chloro-2-methyl-2,3-dihydro-indol-1-yl)-2-oxo-ethyl]-3-methyl-6-morpholin-4-yl-3H-pyrimidin-4-one sous forme d'un solide qui sera séparer sur colonne chirale en ses deux énantiomères.

Spectre RMN 1 H (400 MHz, d en ppm, DMSO-d6) : 1,31 (d, *J*=6,4 Hz, 3 H) ; 2,74 (d, *J*=16,3 Hz, 1 H) ; 3,37 (m, 4 H) ; 3,50 à 3,62 (m, 8 H) ; 4,03 (d, *J*=16,9 Hz, 1 H) ; 4,28 (d, J=16,9 Hz, 1 H) ; 4,75 (m, 1 H) ; 7,13 (d, *J*=8,3 Hz, 1 H) ; 7,24 (t, *J*=8,3 Hz, 1 H) ; 7,90 (d, *J*=8,3 Hz, 1 H)

Les énantiomères sont séparés par chromatographie chirale sur colonne:
Phase stationnaire : phase Whelk 01 SS, 5 µm ; phase mobile : heptane (50%) / dichlorométhane (35%) / méthanol (15%) ; débit : 43 mL/mn.

L'énantiomère dextrogyre est concentré pour obtenir 81 mg de la 5-fluoro-2-[2-((+)-4-chloro-2-methyl-2,3-dihydro-indol-1-yl)-2-oxo-ethyl]-3-methyl-6-morpholin-4-yl-3H-pyrimidin-4-one sous forme d'un solide blanc dont les caractéristiques sont les suivantes :
Spectre RMN 1 H (400 MHz, d en ppm, DMSO-d6) : 1,31 (d, *J*=6,4 Hz, 3 H) ; 2,74 (d, *J*=16,3 Hz, 1 H) ; 3,39 (m, 4 H) ; 3,51 à 3,62 (m, 8 H) ; 4,03 (d, *J*=17,1 Hz, 1 H) ; 4,28 (d, *J*=17,1 Hz, 1 H) ; 4,76 (m, 1 H) ; 7,13 (d, *J*=8,1 Hz, 1 H) ; 7,24 (t, *J*=8,1 Hz, 1 H) ; 7,90 (d large, *J*=8,1 Hz, 1 H)
Spectrométrie de Masse : méthode A
Temps de rétention Tr (min) = 0,90 ; [M+H]+ : m/z 421 ; [M-H]- : m/z 419
Pouvoir rotatoire : α_{D} = +85. C=0.806mg/0.5ML DMSO

L'énantiomère lévogyre est concentré pour obtenir 81 mg de la 5-fluoro-2-[2-((-)-4-chloro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (400 MHz, d en ppm, DMSO-d6) : 1,31 (d, *J*=6,4 Hz, 3 H) ; 2,73 (d, *J*=16,3 Hz, 1 H) ; 3,39 (m, 4 H) ; 3,51 à 3,62 (m, 8 H) ; 4,03 (d, *J*=17,1 Hz, 1 H) ; 4,28 (d, J=17,1 Hz, 1 H) ; 4,76 (m, 1 H) ; 7,13 (d, *J*=8,1 Hz, 1 H) ; 7,24 (t, *J*=8,1 Hz, 1 H) ; 7,90 (d large, *J*=8,1 Hz, 1 H)
Spectrométrie de Masse : méthode A
Temps de rétention Tr (min) = 0,90 ; [M+H]+ : m/z 421 ; [M-H]- : m/z 419
Pouvoir rotatoire : α_{D} = -83. C=1.340mg/0.5ML DMSO.

### Exemple 30a et Exemple 31a: Synthèse du 5-Fluoro-2-[2-((+)-4-bromo-2-methyl-2,3-dihydro-indol-1-yl)-2-oxo-ethyl]-3-methyl-6-morpholin-4-yl-3H-pyrimidin-4-one et du 5-Fluoro-2-[2-((-)-4-bromo-2-methyl-2,3-dihydro-indol-1-yl)-2-oxo-ethyl]-3-methyl-6-morpholin-4-yl-3H-pyrimidin-4-one

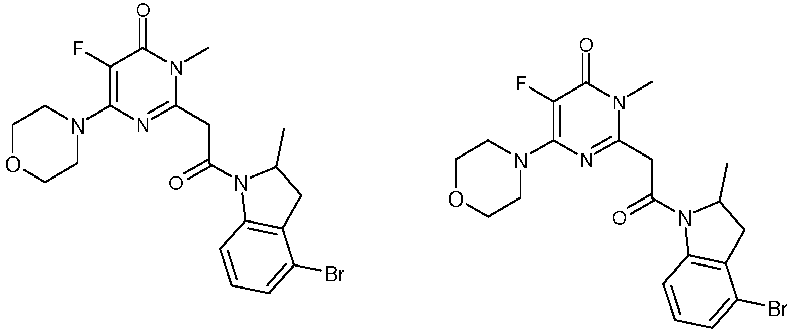

Le 5-fluoro-2-[2-(4-bromo-2-methyl-2,3-dihydro-indol-1-yl)-2-oxo-ethyl]-3-methyl-6-morpholin-4-yl-3H-pyrimidin-4-one est préparé en suivant le mode opératoire décrit à l'exemple 1a (Etape 5a) à partir de 200 mg du (5-fluoro-1-methyl-4-morpholin-4-yl-6-oxo-1,6-dihydro-pyrimidin-2-yl)-acetate de sodium obtenu à l'étape 2a de l'exemple 8a et de 104 mg de 4-bromo-2-methyl-2,3-dihydro-1H-indole (Exemple de référence 4a). Après purification on obtient 154 mg de 5-fluoro-2-[2-(4-bromo-2-methyl-2,3-dihydro-indol-1-yl)-2-oxo-ethyl]-3-methyl-6-morpholin-4-yl-3H-pyrimidin-4-one sous forme d'un solide qui sera séparer sur colonne chirale en ses deux énantiomères.
Spectrométrie de Masse : méthode A
Temps de rétention Tr (min) = 0,93 ; [M+H]+ : m/z 465 ; [M-H]- : m/z 463

Les énantiomères sont séparés par chromatographie chirale sur colonne:
Phase stationnaire : Whelk greffée Kromasil 5um; phase mobile : heptane (50%) / dichloromethane (30%) / méthanol (10%) / éthanol (10%) ; débit : 43 mL/mn.

L'énantiomère dextrogyre est concentré pour obtenir 45 mg de la 5-fluoro-2-[2-((+)-4-bromo-2-methyl-2,3-dihydro-indol-1-yl)-2-oxo-ethyl]-3-methyl-6-morpholin-4-yl-3H-pyrimidin-4-one sous forme d'un solide blanc dont les caractéristiques sont les suivantes :
Spectre RMN 1H (500 MHz, d en ppm, DMSO-d6) : 1,31 (t, *J*=6,4 Hz, 3 H) ; 2,68 (d, *J*=16,3 Hz, 1 H) ; 3,38 (m, 4 H) ; 3,50 à 3,62 (m, 8 H) ; 4,03 (d, *J*=16,6 Hz, 1 H) ; 4,28 (d, J=16,6 Hz, 1 H) ; 4,74 (m, 1 H) ; 7,17 (t, *J*=8,3 Hz, 1 H) ; 7,27 (d, *J*=8,3 Hz, 1 H) ; 7,94 (d, *J*=8,3 Hz, 1 H)
Spectrométrie de Masse : méthode A
Temps de rétention Tr (min) = 0,93 ; [M+H]+ : m/z 465 ; [M-H]- : m/z 463

L'énantiomère lévogyre est concentré pour obtenir 54 mg de la 5-fluoro-2-[2-((-)-4-bromo-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (500 MHz, d en ppm, DMSO-d6) : 1,31 (d, *J*=6,3 Hz, 3 H) ; 2,68 (d, *J*=16,3 Hz, 1 H) ; 3,37 (m, 4 H) ; 3,50 à 3,63 (m, 8 H) ; 4,03 (d, *J*=16,6 Hz, 1 H) ; 4,28 (d, *J*=16,6 Hz, 1 H) ; 4,74 (m, 1 H) ; 7,17 (t, *J*=8,3 Hz, 1 H) ; 7,27 (d, *J*=8,3 Hz, 1 H) ; 7,94 (d, *J*=8,3 Hz, 1 H)
Spectrométrie de Masse : méthode A
Temps de rétention Tr (min) = 0,93 ; [M+H]+ : m/z 465 ; [M-H]- : m/z 463

### Exemple 32a: 5-fluoro-2-[2-((-)-6-fluoro-2-methyl-2,3-dihydro-indol-1-yl)-2-oxo-ethyl]-6-morpholin-4-yl-3H-pyrimidin-4-one

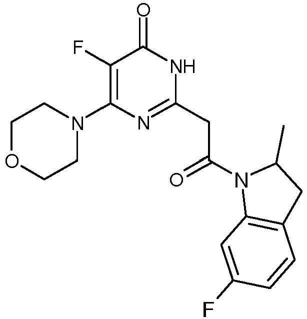

Le produit est préparé en suivant le mode opératoire décrit à l'exemple 1 a (Etape 5a) à partir de 123 mg du (5-fluoro-4-morpholin-4-yl-6-oxo-1,6-dihydro-pyrimidin-2-yl)-acetate de sodium obtenu à l'étape 2a de l'exemple 6a et de 62 mg de (+)-2-methyl-6-fluoro-2,3-dihydro-1H-indole (Exemple de référence 6a). Après purification sur colonne de silice: éluant dichlorométhane/méthanol 98/02, on obtient 84 mg du 5-fluoro-2-[2-((-)-6-fluoro-2-methyl-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1 H (400 MHz, d en ppm, DMSO-d6) : 1,26 (d, *J*=6,4 Hz, 3 H) ; 2,67 (d, *J*=16,3 Hz, 1 H) ; 3,27 à 3,38 (m partiellement masqué, 1 H) ; 3,53 à 3,65 (m, 8 H) ; 3,74 (d, *J*=15,9 Hz, 1 H) ; 3,94 (d, *J*=15,9 Hz, 1 H) ; 4,74 (m, 1 H) ; 6,87 (ddd, *J*=2,6 et 8,3 et 9,2 Hz, 1 H) ; 7,29 (dd, *J*=5,7 et 8,3 Hz, 1 H) ; 7,71 (d large, *J*=11,0 Hz, 1 H) ; 12,31 (m étalé, 1 H)
Spectrométrie de Masse : méthode A
Temps de rétention Tr (min) = 0,76 ; [M+H]+ : m/z 391 ; [M-H]- : m/z 389
Pouvoir rotatoire α_{D} = -65.5+/-1.3. C=1.798mg/0.5ML DMSO

### Exemple 33a: 5-fluoro-2-[2-((+)-6-fluoro-2-methyl-2,3-dihydro-indol-1-yl)-2-oxo-ethyl]-6-morpholin-4-yl-3H-pyrimidin-4-one

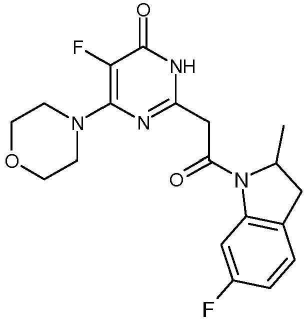

Le produit est préparé en suivant le mode opératoire décrit à l'exemple 1 a (Etape 5a) à partir de 123 mg du (5-fluoro-4-morpholin-4-yl-6-oxo-1,6-dihydro-pyrimidin-2-yl)-acetate de sodium obtenu à l'étape 2a de l'exemple 6a et de 62 mg de (-)-2-methyl-6-fluoro-2,3-dihydro-1H-indole (Exemple de référence 6a). Après purification sur colonne de silice: éluant dichlorométhane/méthanol 98/02, on obtient 74 mg du 5-Fluoro-2-[2-((+)-6-fluoro-2-methyl-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1 H (400 MHz, d en ppm, DMSO-d6) : 1,26 (d, *J*=6,6 Hz, 3 H) ; 2,67 (d, *J*=16,3 Hz, 1 H) ; 3,25 à 3,38 (m partiellement masqué, 1 H) ; 3,52 à 3,66 (m, 8 H) ; 3,73 (d, *J*=16,4 Hz, 1 H) ; 3,94 (d, *J*=16,4 Hz, 1 H) ; 4,74 (m, 1 H) ; 6,87 (ddd, *J*=2,6 et 8,3 et 9,2 Hz, 1 H) ; 7,29 (dd, *J*=5,7 et 8,3 Hz, 1 H) ; 7,71 (d large, *J*=11,0 Hz, 1 H) ; 12,30 (m étalé, 1 H)
Spectrométrie de Masse : méthode A
Temps de rétention Tr (min) = 0,76 ; [M+H]+ : m/z 391 ; [M-H]- : m/z 389
Pouvoir rotatoire: α_{D} = +72.1+/-1.3. C=2.018mg/0.5ML DMSO

### Exemple 34a: 5-Fluoro-2-[2-(-4-chloro-5-fluoro-2-methyl-2,3-dihydro-indol-1-yl)-2-oxo-ethyl]-6-morpholin-4-yl-3H-pyrimidin-4-one

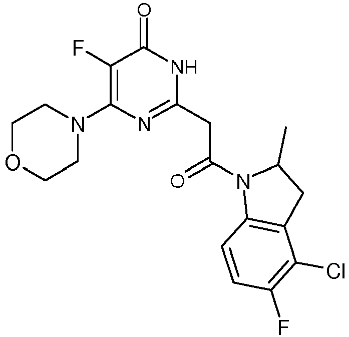

Le produit est préparé en suivant le mode opératoire décrit à l'exemple 1 a (Etape 5a) à partir de 91 mg (5-fluoro-4-morpholin-4-yl-6-oxo-1,6-dihydro-pyrimidin-2-yl)-acetate de sodium et de 55 mg de 4-chloro-5-fluoro-2-methyl-2,3-dihydro-1H-indole. Après purification on obtient 44 mg de 2-[2-(4-chloro-5-fluoro-2-methyl-2,3-dihydro-indol-1-yl)-2-oxo-ethyl]-3-methyl-6-morpholin-4-yl-3H-pyrimidin-4-one sous forme d'un solide dont les caractéristiques sont les suivantes :
Spectre RMN 1 H (400 MHz, d en ppm, DMSO-d6) : 1,30 (d, *J*=6,4 Hz, 3 H) ; 2,76 (d, J=17,4 Hz, 1 H) ; 3,41 (dd, *J*=8,6 et 17,4 Hz, 1 H) ; 3,52 à 3,65 (m, 8 H) ; 3,73 (d, J=16,1 Hz, 1 H) ; 3,92 (d, *J*=16,1 Hz, 1 H) ; 4,78 (m, 1 H) ; 7,24 (t, *J*=9,4 Hz, 1 H) ; 7,90 (m large, 1 H) ; 12,31 (m étalé, 1 H)
Spectrométrie de Masse : méthode A
Temps de rétention Tr (min) = 0,87 ; [M+H]+ : m/z 425 ; [M-H]- : m/z 423

### Exemple 35a: Synthèse du 5-Chloro-2-[2-((S)-2-methyl-2,3-dihydro-indol-1-yl)-2-oxo-ethyl]-6-morpholin-4-yl-3H-pyrimidin-4-one

### Etape 1 a :

### 4,5-Dichloro-6-méthoxy-2-méthyl-pyrimidine

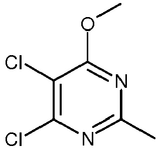

A une solution de 5 g du 2-méthyl-4,5,6-trichloro-pyrimidine dans 41 mL de THF refroidit à 5°C dans un bain de glace sont ajoutés 1.37 g de méthylate de sodium. Le bain de glace est retiré. La suspension est agitée à la température ambiante pendant 18 heures. Le milieu réactionnel est refroidit à 5°C dans un bain de glace. Addition de 20 mL d'eau et 100 mL d'acétate d'éthyle. Après décantation, la phase organique est séchée sur sulfate de magnésium, filtrée, concentrée sous pression réduite pour donner 4.73 g de 4,5-dichloro-6-méthoxy-2-méthyl-pyrimidine sous forme d'une huile incolore qui cristallise dont les caractéristiques sont les suivantes :
Spectrométrie de Masse : méthode B
Cl : [M+H]+ : m/z 193

### Etape 2a :

### (4,5-dichloro-6-methoxy-pyrimidin-2-yl)-acetic acid methyl ester

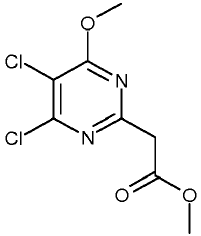

A une solution de 4.7 g du 4,5-dichloro-6-méthoxy-2-méthyl-pyrimidine et 3.1 mL de chloroformiate de méthyle dans 65 mL de THF anhydre refroidit à -60°C dans un bain de carboglace / MeOH, sont additionnés, goutte à goutte, 61 mL de LDA 2M (THF).

Le milieu réactionnel est agité à -60°C pendant une heure.

Le bain refroidissant est baissé pour laisser la température monter à 22°C. Le milieu réactionnel est agité à 22°C pendant deux heures.

Addition de 20 mL d'eau et 150 mL d'acétate d'éthyle. Après décantation, la phase organique est séchée sur sulfate de magnésium, filtrée puis concentrée sous pression réduite. Le résidu obtenu est purifié sur cartouche de silice : éluant : DCM pour donner 4.08 g de (4,5-dichloro-6-methoxy-pyrimidin-2-yl)-acetic acid methyl ester sous forme d'une huile jaune vif dont les caractéristiques sont les suivantes :
Spectrométrie de Masse : méthode A
Temps de rétention Tr (min) = 0,88 ; [M+H]+ : m/z 251 ; [M-H]- : m/z 249

### Etape 3a :

### (5-Chloro-4-methoxy-6-morpholin-4-yl-pyrimidin-2-yl)-acetic acid methyl ester

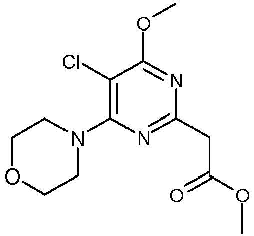

La solution de 4.08 g du (4,5-dichloro-6-methoxy-pyrimidin-2-yl)-acetic acid methyl ester dans 35 mL de morpholine est agitée à la température ambiante pendant une heure et demie. Le milieu réactionnel est concentré sous pression réduite. Le résidu obtenu est repris avec 50 mL d'eau et 200 mL d'acétate d'éthyle. Après décantation, la phase organique est séchée sur sulfate de magnésium, filtrée puis concentrée sous pression réduite pour donner 4.61 g de (5-chloro-4-methoxy-6-morpholin-4-yl-pyrimidin-2-yl)-acetic acid methyl ester sous forme d'un solide beige dont les caractéristiques sont les suivantes :
Spectrométrie de Masse : méthode A
Temps de rétention Tr (min) = 0,87 ; [M+H]+ : m/z 302

### Etape 4a :

### (5-Chloro-4-morpholin-4-yl-6-oxo-1,6-dihydro-pyrimidin-2-yl)-acetic acid methyl ester

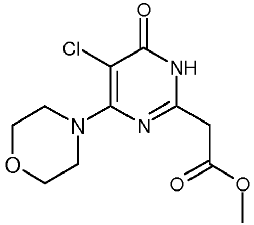

Dans un tube micro-onde, sont introduits, 500 mg du (5-chloro-4-methoxy-6-morpholin-4-yl-pyrimidin-2-yl)-acetic acid methyl ester et 5 mL de dioxane chlorhydrique 4N. Après 30 minutes d'irradiation aux micro-ondes à une température de 110°C sous irradiation micro-ondes, le milieu réactionnel est concentré sous pression réduite. Le résidu obtenu est repris dans l'éther éthylique. Le solide formé est filtré pour donner 414 mg de (5-Chloro-4-morpholin-4-yl-6-oxo-1,6-dihydro-pyrimidin-2-yl)-acetic acid methyl ester dont les caractéristiques sont les suivantes :
Spectrométrie de Masse : méthode A
Temps de rétention Tr (min) = 0,47 ; [M+H]+ : m/z 288 ; [M-H]- : m/z 286 ; pic de base : m/z 254

### Etape 5a :

### (5-Chloro-4-morpholin-4-yl-6-oxo-1,6-dihydro-pyrimidin-2-yl)-acetate de sodium

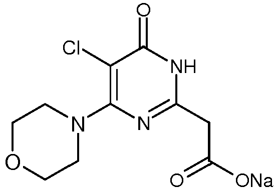

A une solution de 410 mg du (5-chloro-4-morpholin-4-yl-6-oxo-1,6-dihydro-pyrimidin-2-yl)-acetic acid methyl ester dans 3.6 mL de THF est ajouté 1.4 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 400 mg de (5-chloro-4-morpholin-4-yl-6-oxo-1,6-dihydro-pyrimidin-2-yl)-acetate de sodium dont les caractéristiques sont les suivantes :
Spectrométrie de Masse : méthode A
Temps de rétention Tr (min) = 0,37 ; [M+H]+ : m/z 274 ; [2M+H]+ : m/z 547 (pic de base) [M-H]- : m/z 272 ; pic de base : m/z 228

### Alternativement, le composé (5-Chloro-4-morpholin-4-yl-6-oxo-1,6-dihydro-pyrimidin-2-yl)-acetate de sodium peut être obtenu en deux étapes:

### Etape (5'a)a :

### (5-Chloro-4-morpholin-4-yl-6-oxo-1,6-dihydro-pyrimidin-2-yl)-acetic acid ethyl ester

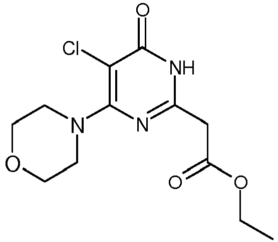

A une solution de 3.75 g du [4-(morpholin-4-yl)-6-oxo-1,6-dihydropyrimidin-2-yl]acétate d'éthyle décrit à l'exemple 6a (Etape (2'a)a),dans 50 mL de chloroforme est ajouté 1.87 g de N-chlorosuccinimide. Le milieu réactionnel est agité à la température ambiante pendant 2 heures. Addition de 20 mL d'eau et 50 mL de dichlorométhane. Après décantation, la phase organique est séchée sur sulfate de magnésium, filtrée, puis concentrée sous pression réduite. Le résidu obtenu est purifié sur colonne de silice, éluant : dichlorométhane/méthanol 99/01 puis 98/02 puis 97/03 pour donner 2.81 g de (5-Chloro-4-morpholin-4-yl-6-oxo-1,6-dihydro-pyrimidin-2-yl)-acetic acid ethyl ester dont les caractéristiques sont les suivantes :
Spectrométrie de Masse : méthode A
Temps de rétention Tr (min) = 0,56 ; [M+H]+ : m/z 302 ; [M-H]- : m/z 300 ; pic de base : m/z 254

### Etape (5'b)a :

### (5-Chloro-4-morpholin-4-yl-6-oxo-1,6-dihydro-pyrimidin-2-yl)-acetate de sodium

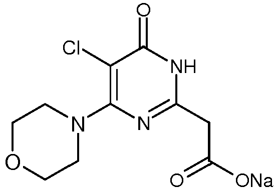

A une solution de 800 mg du (5-chloro-4-morpholin-4-yl-6-oxo-1,6-dihydro-pyrimidin-2-yl)-acetic acid ethyl ester dans 7.2 mL de THF est ajouté 2.8 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 750 mg de (5-chloro-4-morpholin-4-yl-6-oxo-1,6-dihydro-pyrimidin-2-yl)-acetate de sodium dont les caractéristiques sont les suivantes :
Spectrométrie de Masse : méthode A
Temps de rétention Tr (min) = 0,37 ; [M+H]+ : m/z 274 ; [2M+H]+ : m/z 547 (pic de base) [M-H]- : m/z 272 ; pic de base : m/z 228

### Etape 6a:

### 5-Chloro-2-[2-((S)-2-methyl-2,3-dihydro-indol-1-yl)-2-oxo-ethyl]-6-morpholin-4-yl-3H-pyrimidin-4-one

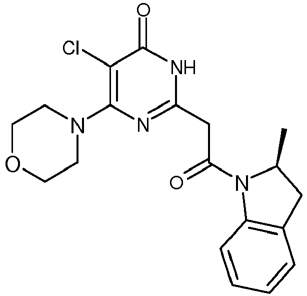

Le produit est préparé en suivant le mode opératoire décrit à l'exemple 1 a (Etape 5a) à partir de 220 mg du (5-chloro-4-morpholin-4-yl-6-oxo-1,6-dihydro-pyrimidin-2-yl)-acetate de sodium et de 110 mg de (S)-2-methyl-4-fluoro-2,3-dihydro-1H-indole (qui peut être préparée selon Krasnov, V.P. et Coll. (Mendeleev Commun. 2002, 12(1), 27-28). Après purification sur colonne de silice: éluant dichlorométhane/méthanol 99/01 puis 98/02 puis 97/03, on obtient 99 mg du 5-chloro-2-[2-((S)-2-methyl-4-fluoro-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1H (400 MHz,) : 1,26 (d large, *J*=6,4 Hz, 3 H) ; 2,69 (d, *J*=16,3 Hz, 1 H) ; 3,38 (m, 1 H) ; 3,50 à 3,65 (m, 8 H) ; 3,76 (d, *J*=15,9 Hz, 1 H) ; 3,97 (d, *J*=15,9 Hz, 1 H) ; 4,70 (m, 1 H) ; 7,05 (t, *J*=8,1 Hz, 1 H) ; 7,18 (t, *J*=8,1 Hz, 1 H) ; 7,29 (d, *J*=8,1 Hz, 1 H) ; 7,95 (d, *J*=8,1 Hz, 1 H) ; 12,42 (m étalé, 1 H)
Spectrométrie de Masse : méthode A
Temps de rétention Tr (min) = 0,77 ; [M+H]+ : m/z 389 ; [M-H]- : m/z 387
Pouvoir rotatoire :α_{D} = +74.1+/-1.4. C=1.696mg/0.5ML DMSO

### Alternativement, le composé 5-Chloro-2-[2-((S)-2-methyl-2,3-dihydro-indol-1-yl)-2-oxo-ethyl]-6-morpholin-4-yl-3H-pyrimidin-4-one peut être obtenu en trois étapes:

### Etape (6'a)a:

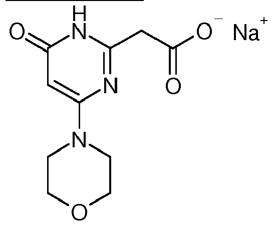

A une solution de 10 g de [4-(morpholin-4-yl)-6-oxo-1,6-dihydropyrimidin-2-yl]acétate d'éthyle décrit à l'exemple 6a (Etape (2'a)a) dans 300 mL de tétrahydrofuranne, est ajouté 18.7 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif pour donner 8.7 g de [4-(morpholin-4-yl)-6-oxo-1,6-dihydropyrimidin-2-yl]acétate de sodium sous forme de solide blanc dont les caractéristiques sont les suivantes:
Spectre RMN 1 H (400MHz, d en ppm , DMSO-d6) : 3,08 (s, 2 H); 3,38 (t, J=4,6 Hz, 4 H); 3,61 (t, J=4,6Hz, 4 H); 5,08 (s, 1 H); 13,16 (s large, 1 H)
Spectrométrie de Masse : méthode A
Temps de rétention Tr (min) = 0.29;
[M+H]+ : m/z 240 ; [M-H]- : m/z 238

### Etape (6'b)a:

### Synthèse de 2-{2-[(2S)-2-méthyl-2,3-dihydro-1H-indol-1-yl]-2-oxoéthyl}-6-(morpholin-4-yl)pyrimidin-4(3H)-one et de la 2-{2-[(2R)-2-méthyl-2,3-dihydro-1H-indol-1-yl]-2-oxoéthyl}-6-(morpholin-4-yl)pyrimidin-4(3H)-one

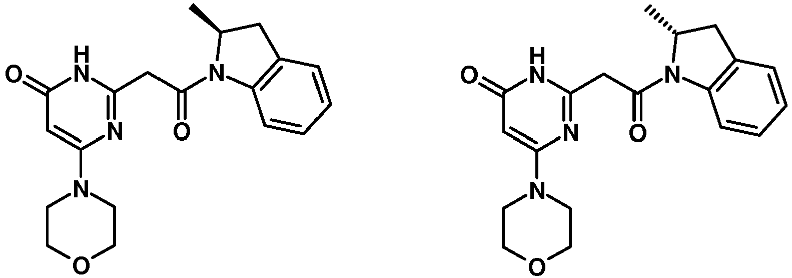

Le 2-[2-(2-méthyl-2,3-dihydro-1H-indol-1-yl)-2-oxoéthyl]-6-(morpholin-4-yl)pyrimidin-4(3H)-one est préparé en suivant le mode opératoire décrit à l'exemple 1a (Etape 5a) à partir de 500 mg de [4-(morpholin-4-yl)-6-oxo-1,6-dihydropyrimidin-2-yl]acétate de sodium, de 510 mg de 2-méthyl-2,3-dihydro-1H-indole, de 487 mg de N-[3-(diméthylamino)propyl]-N'-éthylcarbodiimide chlorhydrate dans un mélange de 0.308 mL de pyridine et de 8 mL de N,N-diméthylformamide . On obtient 400 mg de 2-[2-(2-méthyl-2,3-dihydro-1H-indol-1-yl)-2-oxoéthyl]-6-(morpholin-4-yl)pyrimidin-4(3H)-one sous forme d'une poudre blanche dont les caractéristiques sont les suivantes :
Spectre RMN 1 H (400MHz):
   1,26 (d, J=6,1 Hz, 3 H) ; 2,65 à 2,72 (m, 1 H) ; 3,18 à 3,44 (m partiellement masqué, 5 H) ; 3,54 à 3,63 (m, 4 H) ; 3,72 (d, J=15,7 Hz, 1 H) ; 3,92 (d, J=15,7 Hz, 1 H) ; 4,71 (m, 1 H) ; 5,20 (s, 1 H) ; 7,04 (t, J=7,8 Hz, 1 H) ; 7,18 (t, J=7,8 Hz, 1 H) ; 7,29 (d, J=7,8 Hz, 1 H) ; 7,96 (d, J=7,8 Hz, 1 H) ; 11,69 (m étalé, 1 H)
Spectrométrie de Masse : méthode A
Temps de rétention Tr (min) = 0,70 ;
[M+H]+ : m/z 355 ; [M-H]- : m/z 353
Point de fusion (Kofler) : 172°C

Les énantiomères sont séparés par chromatographique chirale sur colonne : colonne chirale Chiralpak T304 20 µm (1080 g, 20 µm, 8/35 cm), éluant : Acétonitrile/lsopropanol : 90/10 ; débit :185 mL/min. Après purification, on obtient comme premier énantiomère, 160 mg de (+)-2-{2-[(2-méthyl-2,3-dihydro-1H-indol-1-yl]-2-oxoéthyl}-6-(morpholin-4-yl)pyrimidin-4(3H)-one, sous forme d'un solide amorphe rose dont les caractéristiques sont les suivantes:
Spectre RMN 1 H (400MHz): pour ce lot, les signaux sont larges avec : 1,26 (d, J=6,8 Hz, 3 H) ; 2,44 (m partiellement masqué, 1 H) ; 2,69 (d, J=15,2 Hz, 1 H) ; 3,42 (m, 4 H) ; 3,60 (m, 4H) ; 3,72 (d, J=15,7 Hz, 1 H) ; 3,92 (d, J=15,7 Hz, 1 H) ; 4,72 (m, 1 H) ; 5,20 (s, 1 H) ; 7,04 (t, J=7,8 Hz, 1 H) ; 7,18 (t, J=7,8 Hz, 1 H) ; 7,28 (d, J=7,8 Hz, 1 H) ; 7,96 (d, J=7,8 Hz, 1 H) ; 11,67 (m étalé, 1 H)
Spectrométrie de Masse : méthode A
Temps de rétention Tr (min) = 0,70 ;
[M+H]+ : m/z 355 ; [M-H]- : m/z 353;
Pouvoir rotatoire : α_{D} = +65,0° +/-1,3 (c=1,736 mg dans 0,5 mL de méthanol)

Puis le deuxième énantiomère, soit : 143 mg de (-)-2-{2-[(2-méthyl-2,3-dihydro-1H-indol-1-yl]-2-oxoéthyl}-6-(morpholin-4-yl)pyrimidin-4(3H)-one sous forme d'un solide amorphe blanc dont les caractéristiques sont les suivantes :
Spectre RMN 1 H (400MHz): pour ce lot, les signaux sont larges avec : 1,26 (d, J=6,8 Hz, 3 H) ; 2,45 (m partiellement masqué, 1 H) ; 2,69 (m, 1 H) ; 3,41 (m, 4 H) ; 3,61 (m, 4 H) ; 3,72 (d, J=15,7 Hz, 1 H) ; 3,92 (d, J=15,7 Hz, 1 H) ; 4,70 (m, 1 H) ; 5,20 (s, 1 H) ; 7,04 (t, J=7,8 Hz, 1 H) ; 7,18 (t, J=7,8 Hz, 1 H) ; 7,28 (d, J=7,8 Hz, 1 H) ; 7,96 (d, J=7,8 Hz, 1 H) ; 11,64 (m étalé, 1 H)
Spectrométrie de Masse : méthode A
Temps de rétention Tr (min) = 0,70 ;
[M+H]+ : m/z 355 ; [M-H]- : m/z 353;
Pouvoir rotatoire : α_{D} = -72,8° +/-1,2 (c=2,338 mg dans 0,5 mL de méthanol)

### Etape (6'c)a:

### 5-Chloro-2-[2-((S)-2-methyl-2,3-dihydro-indol-1-yl)-2-oxo-ethyl]-6-morpholin-4-yl-3H-pyrimidin-4-one

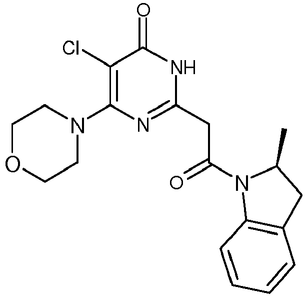

A une solution de 100 mg du 2-{2-[(2S)-2-méthyl-2,3-dihydro-1H-indol-1-yl]-2-oxoéthyl}-6-(morpholin-4-yl)pyrimidin-4(3H)-one dans 4 mL de chloroforme est ajouté 37.6 mg de N-chlorosuccinimide. Le milieu réactionnel est agité à la température ambiante pendant 2 heures. Addition de 5 mL d'eau et 20 mL de dichlorométhane. Après décantation, la phase organique est séchée sur sulfate de magnésium, filtrée, puis concentrée sous pression réduite. Le résidu obtenu est purifié sur colonne de silice, éluant : dichlorométhane/méthanol 99/01 puis 98/02 puis 97/03 pour donner 69 mg de 5-Chloro-2-[2-((S)-2-methyl-2,3-dihydro-indol-1-yl)-2-oxo-ethyl]-6-morpholin-4-yl-3H-pyrimidin-4-one dont les caractéristiques sont les suivantes :
Spectre RMN 1H (400 MHz,) : 1,26 (d large, *J*=6,4 Hz, 3 H) ; 2,69 (d, *J*=16,3 Hz, 1 H) ; 3,38 (m, 1 H) ; 3,50 à 3,65 (m, 8 H) ; 3,76 (d, *J*=15,9 Hz, 1 H) ; 3,97 (d, *J*=15,9 Hz, 1 H) ; 4,70 (m, 1 H) ; 7,05 (t, *J*=8,1 Hz, 1 H) ; 7,18 (t, *J*=8,1 Hz, 1 H) ; 7,29 (d, *J*=8,1 Hz, 1 H) ; 7,95 (d, *J*=8,1 Hz, 1 H) ; 12,42 (m étalé, 1 H)
Spectrométrie de Masse : méthode A
Temps de rétention Tr (min) = 0,77 ; [M+H]+ : m/z 389 ; [M-H]- : m/z 387
Pouvoir rotatoire : α_{D} = +74.1+/-1.4. C=1.696mg/0.5ML DMSO

### Exemple 36a : Synthèse du 5-Chloro-2-[2-((R)-2-methyl-2,3-dihydro-indol-1-yl)-2-oxo-ethyl]-6-morpholin-4-yl-3H-pyrimidin-4-one

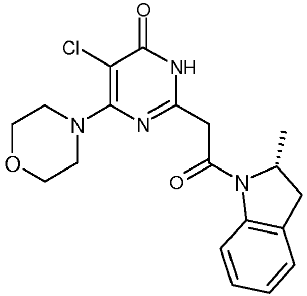

Le produit est préparé en suivant le mode opératoire décrit à l'exemple 1 a (Etape 5a) à partir de 220 mg du (5-chloro-4-morpholin-4-yl-6-oxo-1,6-dihydro-pyrimidin-2-yl)-acetate de sodium et de 110 mg de (R)-2-methyl-4-fluoro-2,3-dihydro-1H-indole (qui peut être préparée selon Krasnov, V.P. et Coll. (Mendeleev Commun. 2002, 12(1), 27-28). Après purification sur colonne de silice: éluant dichlorométhane/méthanol 99/01 puis 98/02 puis 97/03, on obtient 100 mg du 5-chloro-2-[2-((R)-2-methyl-4-fluoro-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1H (400 MHz,) : 1,26 (d large, *J*=6,4 Hz, 3 H) ; 2,69 (d, *J*=16,3 Hz, 1 H) ; 3,38 (m, 1 H) ; 3,50 à 3,65 (m, 8 H) ; 3,76 (d, *J*=15,9 Hz, 1 H) ; 3,97 (d, *J*=15,9 Hz, 1 H) ; 4,70 (m, 1 H) ; 7,05 (t, *J*=8,1 Hz, 1 H) ; 7,18 (t, *J*=8,1 Hz, 1 H) ; 7,29 (d, *J*=8,1 Hz, 1 H) ; 7,95 (d, *J*=8,1 Hz, 1 H) ; 12,43 (m étalé, 1 H)
Spectrométrie de Masse : méthode A
Temps de rétention Tr (min) = 0,77 ; [M+H]+ : m/z 389 ; [M-H]- : m/z 387
Pouvoir rotatoire : α_{D} = -79.4+/-1.4. C=1.858mg/0.5ML DMSO

### Exemple 37a :

### Synthèse 5-bromo-2-[2-((S)-2-methyl-2,3-dihydro-indol-1-yl)-2-oxo-ethyl]-6-morpholin-4-yl-3H-pyrimidin-4-one

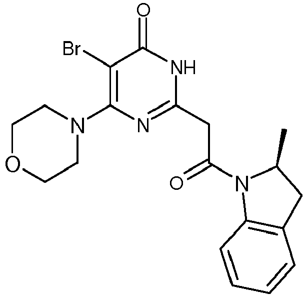

A une solution de 200 mg du 2-{2-[(2S)-2-méthyl-2,3-dihydro-1H-indol-1-yl]-2-oxoéthyl}-6-(morpholin-4-yl)pyrimidin-4(3H)-one dans 4 mL de chloroforme est ajouté 100 mg de N-bromosuccinimide. Le milieu réactionnel est agité à la température ambiante pendant 2 heures. Addition de 5 mL d'eau et 30 mL de dichlorométhane. Après décantation, la phase organique est séchée sur sulfate de magnésium, filtrée, puis concentrée sous pression réduite. Le résidu obtenu est purifié sur colonne de silice, éluant : gradient dichlorométhane/méthanol 99/01 à 96/04 pour donner 93 mg de 5-bromo-2-[2-((S)-2-methyl-2,3-dihydro-indol-1-yl)-2-oxo-ethyl]-6-morpholin-4-yl-3H-pyrimidin-4-one dont les caractéristiques sont les suivantes :
Spectre RMN 1H (400MHz, d en ppm , DMSO-d6) : 1,27 (d, *J*=5,9 Hz, 3 H); 2,63 à 2,75 (m, 2 H); 3,53 (m, 4 H); 3,61 (m, 4 H); 3,77 (d, *J*=16,1 Hz, 1 H); 3,97 (d, *J*=15,7 Hz, 1 H); 4,71 (m, 1 H); 7,05 (t, *J*=7,3 Hz, 1 H); 7,18 (t, *J*=7,5 Hz, 1 H); 7,29 (d, *J*=7,6 Hz, 1 H); 7,95 (d, *J*=7,8 Hz, 1 H); 12,42 (s large, 1 H)
Spectrométrie de Masse : méthode A
Temps de rétention Tr (min) = 0.78;
[M+H]+ : m/z 433 ; [M-H]- : m/z 431

### Exemple 38a : 5-Fluoro-2-[2-((+)-2-hydroxymethyl-2,3-dihydro-indol-1-yl)-2-oxo-ethyl]-6-morpholin-4-yl-3H-pyrimidin-4-one

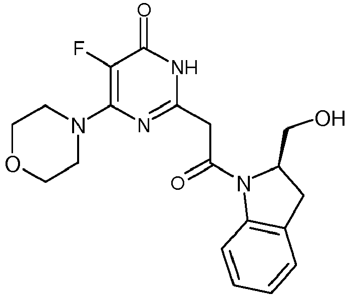

A une solution de 207 mg du (5-fluoro-4-morpholin-4-yl-6-oxo-1,6-dihydro-pyrimidin-2-yl)-acetate de sodium dans 6 mL de DMF et 6 mL de pyridine sont ajoutés 198 mg de N-[3-(diméthylamino)propyl]-N'-éthylcarbodiimide chlorhydrate et 105 mg de (-)-(2,3-dihydro-1H-indol-2-yl)-methanol (Exemple de référence 8a). Le milieu réactionnel est agité à la température ambiante pendant 18 heures puis on ajoute un mélange d"eau et de dichlorométhane. Après décantation, la phase organique est séchée sur sulfate de magnésium, filtrée puis concentrée sous pression réduite. Le résidu obtenu est purifié sur colonne de silice, éluant : dichlorométhane/méthanol 95/05 pour donner 77 mg de 5-fluoro-2-[2-((+)-2-hydroxymethyl-2,3-dihydro-indol-1-yl)-2-oxo-ethyl]-6-morpholin-4-yl-3H-pyrimidin-4-one dont les caractéristiques sont les suivantes :
Spectre RMN 1H (400MHz, d en ppm , DMSO-d6) : 2,86 (d, *J*=16,4 Hz, 1 H); 3,23 (m, 1 H); 3,38 (m, 1 H); 3,45 à 3,57 (m, 5 H); 3,63 (m, 4 H); 3,82 (d, *J*=16,1 Hz, 1 H); 4,01 (d, *J*=16,1 Hz, 1 H); 4,61 (m, 1 H); 5,13 (s large, 1 H); 7,03 (t, *J*=6,4 Hz, 1 H); 7,16 (t, *J*=7,7 Hz, 1 H); 7,26 (d, *J*=7,1 Hz, 1 H); 7,93 (d, *J*=7,6 Hz, 1 H); 12,25 (s large, 1 H)
Spectrométrie de Masse : méthode A
Temps de rétention Tr (min) = 0.58;
[M+H]+ : m/z 389 ; [M-H]- : m/z 387
Pouvoir rotatoire : α_{D} = PR= +79.1 +/1.4. C=1.925mg/0.5ML DMSO

### Exemple 39a : 5-Fluoro-2-[2-((-)-2-hydroxymethyl-2,3-dihydro-indol-1-yl)-2-oxo-ethyl]-6-morpholin-4-yl-3H-pyrimidin-4-one

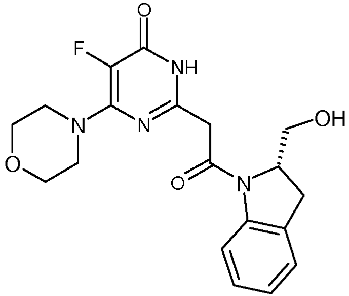

A une solution de 470 mg du (5-fluoro-4-morpholin-4-yl-6-oxo-1,6-dihydro-pyrimidin-2-yl)-acetate de sodium dans 14 mL de DMF et 14 mL de pyridine sont ajoutés 471 mg de N-[3-(diméthylamino)propyl]-N'-éthylcarbodiimide chlorhydrate et 258 mg de (+)-(2,3-dihydro-1H-indol-2-yl)-methanol (Exemple de référence 8a). Le milieu réactionnel est agité à la température ambiante pendant 18 heures puis on ajoute un mélange d"eau et de dichlorométhane. Après décantation, la phase organique est séchée sur sulfate de magnésium, filtrée puis concentrée sous pression réduite. Le résidu obtenu est purifié sur colonne de silice, éluant : dichlorométhane/méthanol 95/05 pour donner 280 mg de 5-fluoro-2-[2-((-)-2-hydroxymethyl-2,3-dihydro-indol-1-yl)-2-oxo-ethyl]-6-morpholin-4-yl-3H-pyrimidin-4-one dont les caractéristiques sont les suivantes :
Spectre RMN 1H (400MHz, d en ppm , DMSO-d6) : 2,86 (d, *J*=15,9 Hz, 1 H); 3,23 (m, 1 H); 3,36 (m, 1 H); 3,50 (dd, *J*=6,8 et 11,0 Hz, 1 H); 3,55 (m, 4 H); 3,61 (m, 4 H); 3,82 (d, *J*=15,9 Hz, 1 H); 4,01 (d, *J*=16,1Hz, 1 H); 4,60 (m, 1 H); 5,13 (s large, 1 H); 7,02 (m, 1 H); 7,16 (t, *J*=7,6 Hz, 1 H); 7,26 (d, *J*=7,1 Hz, 1 H); 7,93 (d, J=8,1 Hz, 1 H); 12,25 (s large, 1 H)
Spectrométrie de Masse : méthode A
Temps de rétention Tr (min) = 0.58;
[M+H]+ : m/z 389 ; [M-H]- : m/z 387

### Exemples de référence pour la préparation des composés de formule (la)

### Exemple de référence 1a : 5-fluoro-2-méthyl-2,3-dihydro-1H-indole

### Etape 1a :

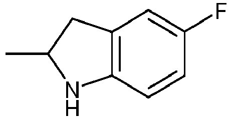

A une solution de 5 g du 5-fluoro-2-méthylindole dans 60 mL d'acide trifluoroacétique refroidit à 5°C, sont ajoutés en quatre fois 6.22 g de cyanoborohydrure de sodium. Le milieu réactionnel est agité à 0°C pendant 30 minutes puis cinq heures à la température ambiante.

Le milieu réactionnel est refroidit de nouveau à 5°C. Addition de 700 mL d'eau glacée, 150 mL de soude 31%, puis 300 mL d'acétate d'éthyle. Agitation à la température ambiante pendant une heure. Après décantation, la phase organique est séchée sur sulfate de magnésium, filtrée puis concentrée sous pression réduite. Le résidu obtenu est purifié sur colonne de silice, éluant : heptane/acétate d'éthyle 90/10 pour donner 2.14 g de 5-fluoro-2-methyl-2,3-dihydro-1H-indole dont les caractéristiques sont les suivantes :
Spectrométrie de Masse : méthode A
Temps de rétention Tr (min) = 0,24 ; [M+H]+ : m/z 152 ;

### Etape 2a :

### (R)-1-(5-Fluoro-2-methyl-2,3-dihydro-indol-1-yl)-2-phenoxy-propan-1-one : diastéréoisomère A

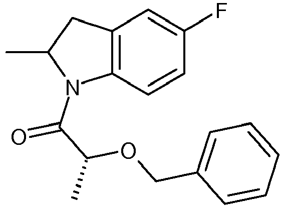

### Et

### (R)-1-(5-Fluoro-2-methyl-2,3-dihydro-indol-1-yl)-2-phenoxy-propan-1-one : diastéréoisomère B

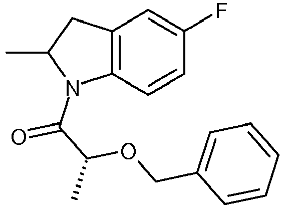

A une solution de 4.17 g d'acide o-benzyl-D-lactique dans 17 mL de DMF et 3.43 mL de pyridine sont ajoutés 2.9 g de 5-fluoro-2-methyl-2,3-dihydro-1H-indole et 5.3 g de N-[3-(diméthylamino)propyl]-N'-éthylcarbodiimide chlorhydrate.

Le milieu réactionnel est agité à la température ambiante pendant 18 heures.

Addition de 500 mL d'acétate d'éthyle et 500 mL d'eau. Après décantation, la phase organique est séchée sur sulfate de magnésium, filtrée puis concentrée sous pression réduite. Le résidu obtenu est purifié sur cartouche de silice : éluant : heptane, puis heptane/acétate d'éthyle 95/05, puis heptane/acétate d'éthyle 90/10 pour donner 2.8 g de (R)-1-(5-fluoro-2-methyl-2,3-dihydro-indol-1-yl)-2-phenoxy-propan-1-one : diastéréoisomère A sous forme d'une huile jaune dont les caractéristiques sont les suivantes :
Spectrométrie de Masse : méthode A
Temps de rétention Tr (min) = 1,08 ;
[M+H]+ : m/z 314

Et 2.63 g de (R)-1-(5-fluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,06 ;
[M+H]+ : m/z 314 ; pic de base : m/z 242

### Etape (3a)a :

### (+)-5-Fluoro-2-methyl-2,3-dihydro-1H-indole

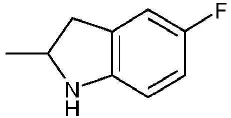

A une solution de 2.8 g de (R)-1-(5-fluoro-2-methyl-2,3-dihydro-indol-1-yl)-2-phenoxy-propan-1-one : diastéréoisomère A dans 28 mL d'éthanol sont ajoutés 28 mL d'acide chlorhydrique 37%.

Le milieu réactionnel est chauffé à reflux pendant 5 heures.

Le milieu réactionnel est concentré sous pression réduite. Le résidu obtenu est repris avec 260 mL d'eau. Addition de la soude 2N jusqu'au PH=10. Le milieu est extrait avec 200 mL de dichlorométhane. Après décantation, la phase organique est séchée sur sulfate de magnésium, filtrée puis concentrée sous pression réduite. Le résidu obtenu est purifié sur cartouche de silice : éluant : heptane/acétate d'éthyle 90/10 pour donner 1.29 g de (+)-5-fluoro-2-methyl-2,3-dihydro-1H-indole sous forme d'une huile jaune dont les caractéristiques sont les suivantes :
Spectrométrie de Masse : méthode A
Temps de rétention Tr (min) = 0,25 ; [M+H]+ : m/z 152
Pouvoir rotatoire : α_{D} = +8.2+/-0.7. C=1.801mg/0.5ML DMSO

### Etape (3b)a :

### (-)-5-Fluoro-2-methyl-2,3-dihydro-1H-indole

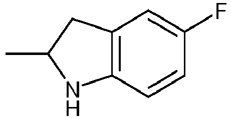

A une solution de 2.63 g (R)-1-(5-fluoro-2-methyl-2,3-dihydro-indol-1-yl)-2-phenoxy-propan-1-one : diastéréoisomère B de dans 26.5 mL d'éthanol sont ajoutés 26.5 mL d'acide chlorhydrique 37%.

Le milieu réactionnel est chauffé à reflux pendant 4 heures.

Le milieu réactionnel est concentré sous pression réduite. Le résidu obtenu est repris avec 250 mL d'eau. Addition de la soude 2N jusqu'au PH=10. Le milieu est extrait avec 200 mL de dichlorométhane. Après décantation, la phase organique est séchée sur sulfate de magnésium, filtrée puis concentrée sous pression réduite. Le résidu obtenu est purifié sur cartouche de silice : éluant : heptane/acétate d'éthyle 90/10 pour donner 1.11g de (-)-5-fluoro-2-methyl-2,3-dihydro-1H-indole sous forme d'une huile jaune dont les caractéristiques sont les suivantes :
Spectrométrie de Masse : méthode A
Temps de rétention Tr (min) = 0,24 ; [M+H]+ : m/z 152 ;
Pouvoir rotatoire : α_{D} = -7.9+/-0.4. C=3.023mg/0.5ML DMSO

### Exemple de référence 2a : 4,5-difluoro-2-méthyl-2,3-dihydro-1H-indole

### Etape 1a :

### 4,5-Difluoro-2-methyl-2,3-dihydro-1H-indole

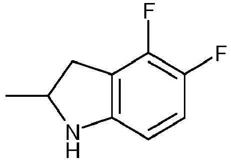

A une solution de 4.5 g du 4,5-diluoro-2-methyl-indole dans 180 mL d'acide acétique refroidit à 15°C, sont ajoutés en 3 fois 5.07 g de cyanoborohydrure de sodium. Le milieu réactionnel est agité à 15°C pendant 30 minutes puis 4 heures à la température ambiante.

Le milieu réactionnel est refroidit de nouveau à 5°C. Addition de l'eau glacée. Addition de l'ammoniaque 30% jusqu'au PH = 9. Extraction 3 fois au dichlorométhane. Les phases organique sont réunies, séchées sur sulfate de magnésium, filtrées puis concentrée sous pression réduite. Le résidu obtenu est purifié sur colonne de silice, éluant : cyclohexane/acétate d'éthyle 90/10 pour donner 4.4 g de 4,5-difluoro-2-methyl-2,3-dihydro-1H-indole dont les caractéristiques sont les suivantes :
Spectrométrie de Masse : méthode A
Temps de rétention Tr (min) = 0,60 ; [M+H]+ : m/z 170

### Etape 2a :

### (R)-1-(4,5-Difluoro-2-methyl-2,3-dihydro-indol-1-yl)-2-phenoxy-propan-1-one : diastéréoisomère A

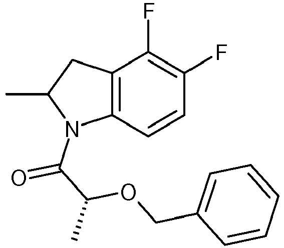

### Et

### (R)-1-(4,5-Difluoro-2-methyl-2,3-dihydro-indol-1-yl)-2-phenoxy-propan-1-one: diastéréoisomère B

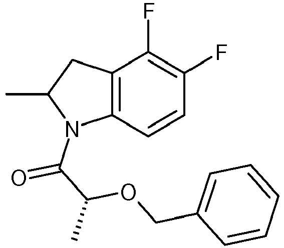

Les produits sont préparés en suivant le mode opératoire décrit à l'exemple de référence 1a (Etape 2a) à partir de 4.4 g du (4,5-difluoro-2-methyl-2,3-dihydro-1H-indole et de 5.62 g d'acide o-benzyl-D-lactique. Après purification sur colonne de silice, éluant : cyclohexane/acétate d'éthyle 90/10 puis 80/20, on obtient 4.2 g de (R)-1-(4,5-difluoro-2-methyl-2,3-dihydro-indol-1-yl)-2-phenoxy-propan-1-one : diastéréoisomère A sous forme d'une huile jaune dont les caractéristiques sont les suivantes :
Spectrométrie de Masse : méthode A
Temps de rétention Tr (min) = 1,11 ; [M+H]+ : m/z 332 ; pic de base : m/z 260
Pouvoir rotatoire : α_{D} = +41.6+/-0.9. C= 2.266mg/0.5ML DMSO

Et 4.1 g de (R)-1-(4,5-difluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,10 ; [M+H]+ : m/z 332 ; pic de base: m/z 260
Pouvoir rotatoire : α_{D} = +120.1+/-1.8. C= 2.252mg/0.5ML DMSO

### Etape 3a :

### (+)-4,5-Difluoro-2-methyl-2,3-dihydro-1H-indole

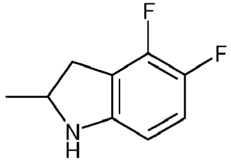

Le produit est préparé en suivant le mode opératoire décrit à l'exemple de référence 1a (Etape 3a) à partir de 4.2 g du (R)-1-(4,5-difluoro-2-methyl-2,3-dihydro-indol-1-yl)-2-phenoxy-propan-1-one : diastéréoisomère A et de 40 mL d'acide chlorhydrique 37%.

Après purification sur colonne de silice, éluant : cyclohexane/acétate d'éthyle 90/10, on obtient 1.6 g de (+)-4,5-difluoro-2-methyl-2,3-dihydro-1H-indole dont les caractéristiques sont les suivantes :
Spectrométrie de Masse : méthode A
Temps de rétention Tr (min) = 0,58 ; [M+H]+ : m/z 170 ;

### Etape 3a :

### (-)-4,5-Difluoro-2-methyl-2,3-dihydro-1H-indole

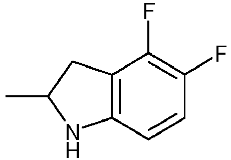

Le produit est préparé en suivant le mode opératoire décrit à l'exemple de référence1a (Etape 3a) à partir de 4.1 g du (R)-1-(4,5-difluoro-2-methyl-2,3-dihydro-indol-1-yl)-2-phenoxy-propan-1-one : diastéréoisomère B et de 41 mL d'acide chlorhydrique 37%.

Après purification sur colonne de silice, éluant : heptane/acétate d'éthyle 90/10 , on obtient 1.5 g de (-)-4,5-difluoro-2-methyl-2,3-dihydro-1H-indole dont les caractéristiques sont les suivantes :
Spectrométrie de Masse : méthode A
Temps de rétention Tr (min) = 0,60 ; [M+H]+ : m/z 170 ;

### Exemple de référence 3a : 2-methyl-5,6-fluoro-2,3-dihydro-1H-indole

### Etape 1a :

### 4,5-Difluoro-2-iodo-aniline

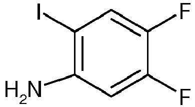

A une suspension de 6.45 g de 3,4-difluoroaniline dans 250 mL d'eau, sont ajoutés, à la température ambiante, 16.5 g d'iode et 6.3 g de bicarbonate de sodium. Le milieu réactionnel est agité à la température ambiante pendant 1 heure.

Addition d'une solution saturée de thiosulfate de sodium, puis extraction 3 fois à l'acétate d'éthyle. Les phases organique sont réunies, séchées sur sulfate de magnésium, filtrées puis concentrée sous pression réduite pour donner 12 g de 4,5-difluoro-2-iodo-ainiline dont les caractéristiques sont les suivantes :
Spectrométrie de Masse : méthode A
Temps de rétention Tr (min) = 0,90 ; [M+H]+ : m/z 256 ; pic de base : m/z 297

### Etape 2a :

### 4,5-Difluoro-2-prop-1-ynyl-phenylamine

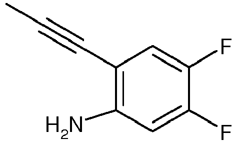

A une solution de 19 g de 4,5-difluoro-2-iodo-ainiline dans 150 mL de trietyhlamine, sont ajoutés, à la température ambiante, 426 mg d'iodure de cuivre (I) et 523 mg du bis(triphenylphosphine)palladium(II)dichloride. La suspension est refroidit à - 30°C dans un bain de carboglace/éthanol. D'autre part, 20 mL de propyne sont condensés par barbotage dans un piège refroidit à -70°C à l'aide d'un mélange de carboglace/méthanol. Le propyne est additionné sur la suspension refroidit à -30°C. Le bain refroidissant est gardé. On laisse la température monter à l'ambiante pendant la nuit.

Le milieu réactionnel est filtré. Le filtrat est concentré à sec sous pression réduite. Le résidu obtenu est repris à l'eau et à l'acétate d'éthyle. Après décantation, la phase organique est séchée sur sulfate de magnésium, filtrée puis concentrée sous pression réduite. Le résidu obtenu est purifié sur colonne de silice, éluant : cyclohexane/dichlorométhane 80/20 pour donner 10.8 g de 4,5-difluoro-2-prop-1-ynyl-phenylamine dont les caractéristiques sont les suivantes :
Spectrométrie de Masse : méthode A
Temps de rétention Tr (min) = 0,90 ; [M+H]+ : m/z 168

### Etape 3a :

### 5,6-Difluoro-2-methyl-indole

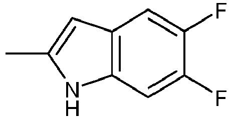

A une solution de 10.8 g du 4,5-difluoro-2-prop-1-ynyl-phenylamine dans 100 mL de DMF, sont ajoutés 246 mg d'iodure de cuivre (I). Le milieu réactionnel est chauffé à reflux pendant une heure.

Après refroidissement, le milieu réactionnel est filtré. Le filtrat est concentré sous pression réduite. Le résidu brut obtenu est purifié sur colonne de silice, éluant : cyclohexane/dichlorométhane 90/10 pour donner 7.2 g de 5,6-difluoro-2-methyl-indole dont les caractéristiques sont les suivantes :
Spectrométrie de Masse : méthode A
Temps de rétention Tr (min) = 0,94; [M-H]- : m/z 166

### Etape 4a :

### 5,6-Difluoro-2-methyl-2,3-dihydro-1H-indole

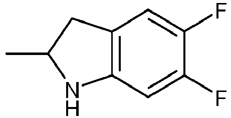

A une solution de 7.2 g du 5,6-difluoro-2-méthylindole dans 220 mL d'acide acétique refroidit à 15°C, sont ajoutés en 3 fois 8.11 g de cyanoborohydrure de sodium. Le milieu réactionnel est agité à 15°C pendant 30 minutes puis 4 heures à la température ambiante.

Le milieu réactionnel est refroidit de nouveau à 5°C. Addition de 900 mL d'eau glacée. Addition de l'ammoniaque 30% jusqu'au PH = 9. Extraction 3 fois au dichlorométhane. Les phases organique sont réunies, séchées sur sulfate de magnésium, filtrées puis concentrée sous pression réduite. Le résidu obtenu est purifié sur colonne de silice, éluant : heptane/acétate d'éthyle 90/10 pour donner 6.3 g de 5,6-difluoro-2-methyl-2,3-dihydro-1H-indole dont les caractéristiques sont les suivantes :
Spectrométrie de Masse : méthode A
Temps de rétention Tr (min) = 0,53 ; [M+H]+ : m/z 170 ;

### Etape 5a :

### (R)-1-(5,6-Difluoro-2-methyl-2,3-dihydro-indol-1-yl)-2-phenoxy-propan-1-one : diastéréoisomère A

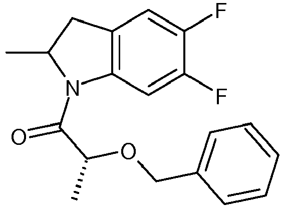

### Et

### (R)-1-(5,6-Difluoro-2-methyl-2,3-dihydro-indol-1-yl)-2-phenoxy-propan-1-one : diastéréoisomère B

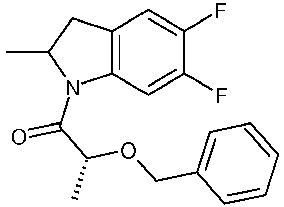

Les produits sont préparés en suivant le mode opératoire décrit à l'exemple de référence 1a (Etape 2a) à partir de 6.3 g du (5,6-difluoro-2-methyl-2,3-dihydro-1H-indole et de 6.8 g d'acide o-benzyl-D-lactique. Après purification sur colonne de silice, on obtient 6.45 g de (R)-1-(5,6-difluoro-2-methyl-2,3-dihydro-indol-1-yl)-2-phenoxy-propan-1-one : diastéréoisomère A sous forme d'une huile jaune dont les caractéristiques sont les suivantes :
Spectrométrie de Masse : méthode A
Temps de rétention Tr (min) = 1,11 ; [M+H]+ : m/z 332 ; pic de base: m/z 260

Et 6.29 g de (R)-1-(5,6-difluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,10 ; [M+H]+ : m/z 332 ; pic de base : m/z 260

### Etape (6a)a :

### (+)-5,6-Difluoro-2-methyl-2,3-dihydro-1H-indole

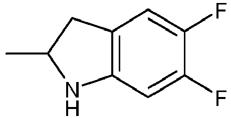

Le produit est préparé en suivant le mode opératoire décrit à l'exemple de référence 1a (Etape 3a) à partir de 6.45 g du (R)-1-(5,6-difluoro-2-methyl-2,3-dihydro-indol-1-yl)-2-phenoxy-propan-1-one : diastéréoisomère A et de 64.5 mL d'acide chlorhydrique 37%.

Après purification sur colonne de silice, éluant : heptane/acétate d'éthyle 90/10, on obtient 2.7 g de (+)-5,6-difluoro-2-methyl-2,3-dihydro-1H-indole dont les caractéristiques sont les suivantes :
Spectrométrie de Masse : méthode A
Temps de rétention Tr (min) = 0,53 ; [M+H]+ : m/z 170 ;
Pouvoir rotatoire : α_{D} = +17.6+/-0.7. C= 1.834mg/0.5ML DMSO

### Etape (6b)a :

### (-)-5,6-Difluoro-2-methyl-2,3-dihydro-1H-indole

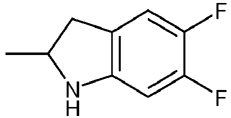

Le produit est préparé en suivant le mode opératoire décrit à l'exemple référence 1a (Etape 3a) à partir de 6.29 g du (R)-1-(5,6-difluoro-2-methyl-2,3-dihydro-indol-1-yl)-2-phenoxy-propan-1-one : diastéréoisomère B et de 63 mL d'acide chlorhydrique 37%.

Après purification sur colonne de silice, éluant : heptane/acétate d'éthyle 90/10 , on obtient 2.76 g de (-)-5,6-difluoro-2-methyl-2,3-dihydro-1H-indole dont les caractéristiques sont les suivantes :
Spectrométrie de Masse : méthode A
Temps de rétention Tr (min) = 0,55 ; [M+H]+ : m/z 170 ;
Pouvoir rotatoire : α_{D} = -6.7+/-0.6. C=1.832mg/0.5ML DMSO

### Exemple de référence 4a : 4-Bromo-2-methyl-2,3-dihydro-1H-indole

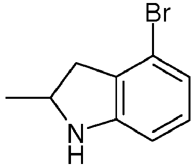

A une solution de 8,90 g de 4-bromo-2-méthyl-indole (qui peut être préparé selon le brevet US2010/160647 A1, 2010) dans 310 mL d'acide acétique sous argon refroidie à une température voisine de 14°C, sont ajoutés progressivement 7,99 g de cyanoborohydrure de sodium. Le mélange réactionnel est agité à une température voisine de 14°C pendant 15 minutes, puis il est laissé se réchauffer à température ambiante. Après 2 heures, le mélange réactionnel est versé dans un erlen contenant de l'eau glacée (200mL) puis le pH est amené jusqu'à 9 avec une solution d'ammoniac aqueux. Le milieu réactionnel est extrait avec du dichlorométhane (2x200 mL) puis les phases organiques sont réunies, lavées avec de l'eau, séchées sur sulfate de magnésium, filtrées, puis concentrées à sec sous pression réduite. Après purification du résidu par chromatographie sur colonne de silice en éluant avec un mélange d'heptane et d'acétate d'éthyle (90/10), on obtient 5,92 g de 4-bromo-2-méthyl-indoline.

### Exemple de référence 5a : 4-Fluoro-2-methyl-2,3-dihydro-1H-indole

### Etape 1 a

### (3-fluoro-2-méthyl-phényl)-carbamate de tert-butyle

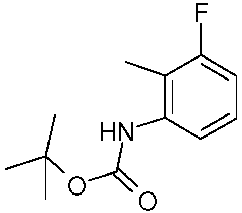

A une solution de 5 g de 3-fluoro-2-méthyl-aniline dans 25 mL de tetrahydrofuranne, sont ajoutés 9,9 g de di-*tert*-butyl-dicarbonate. Le mélange réactionnel est chauffé à reflux sous agitation pendant 16 heures, puis il est refroidi à température ambiante et concentré à sec sous pression réduite. Le résidu est trituré dans 20 mL de cyclohexane et le précipité obtenu est filtré sur verre fritté, essoré puis séché sous pression réduite à 40°C. On obtient ainsi 7,1 g de (3-fluoro-2-méthyl-phényl)-carbamate de *tert*-butyle sous forme d'un solide blanc brillant dont les caractéristiques sont les suivantes :
Spectrométrie de Masse : EI : [M]+. m/z = 225
méthode A
Temps de rétention Tr (min) = 1,04 ;
[M+H-tBu]+ : m/z 170 ; mic de base m/z : 211
Point de fusion (Kofler) : 72°C

### Etape 2a

### [3-fluoro-2-(2-oxo-propyl)-phényl]-carbamate de tert-butyle

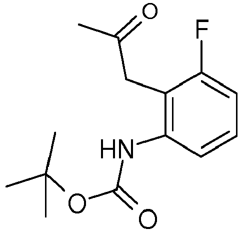

A une solution de 0,5 g de (3-fluoro-2-méthyl-phényl)-carbamate de *tert-*butyle dans 10 mL de tetrahydrofuranne sous argon et refroidie à -40°C, sont additionnés au goutte à goutte 4,3 mL d'une solution de sec-butyllithium 1,3 M dans le cyclohexane/ hexane 98/2 v/v en maintenant la température entre -40°C et -30°C. Le mélange réactionnel est ensuite refroidi à -50°C, puis une solution de 0,27 mL de N-méthoxy-N-méthyl-acétamide dans 4 mL de tetrahydrofuranne est additionnée au goutte à goutte en maintenant la température entre -50°C et -40°C. Le mélange est ensuite laissé se réchauffer à environ -10°C, puis agité à cette température pendant 0,5 heure. Il est ensuite traité avec 5,6 mL d'une solution d'acide chlorhydrique 1 N puis dilué avec 20 mL d'éther diéthylique et laissé se réchauffer à température ambiante sous agitation pendant 1 heure. Après décantation, la phase organique est séparée et la phase aqueuse est extraite avec 40 mL d'éther diéthylique. Les phases organiques sont réunies, lavées par 3x40 mL d'eau, séchées sur sulfate de magnésium, filtrées, puis concentrées à sec sous pression réduite. Le résidu est purifié par chromatographie sur une cartouche de 30 g silice 15-40 µm, en éluant avec un mélange cyclohexane/ acétate d'éthyle 90/10 v/v, à un débit de 30 mL/ min, puis sur une cartouche de 30 g silice 15-40 µm, en éluant avec du dichlorométhane pur, à un débit de 30 mL/ min. On obtient ainsi 0,38 g de [3-fluoro-2-(2-oxo-propyl)-phényl]-carbamate de *tert*-butyle sous forme d'un solide blanc dont les caractéristiques sont les suivantes :
Spectrométrie de Masse : EI : [M]+. m/z = 267
méthode A
Temps de rétention Tr (min) = 0,93 ;
[M+Na]+ : m/z 290 ;

### Etape 3a

### 4-fluoro-2-méthyl-indole

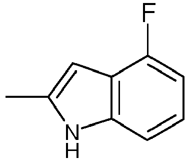

A une solution de 0,35 g de [3-fluoro-2-(2-oxo-propyl)-phényl]-carbamate de *tert-*butyle dans 13 mL de dichlorométhane anhydre à température ambiante, sont additionnés 1,43 mL d'acide trifluoroacétique. Le mélange réactionnel est ensuite agité à température ambiante pendant 24h, puis il est dilué avec 27 mL de dichlorométhane et traité avec 25 mL d'une solution d'hydrogénocarbonate de sodium à 5%. Après agitation à température ambiante pendant 1 heure puis décantation, la phase organique est séparée et la phase aqueuse est extraite avec 25 mL de dichlorométhane. Les phases organiques sont réunies, lavées avec de la saumure saturée, séchées sur sulfate de magnésium, filtrées, puis concentrées à sec sous pression réduite. On obtient ainsi 0,19 g de 4-fluoro-2-méthyl-indole sous forme d'une huile rougé foncé dont les caractéristiques sont les suivantes :
Spectre RMN 1 H (400MHz, CDCl3): 2,46 (s, 3 H) ; 6,30 (m large, 1 H) ; 6,74 (dd, *J*=7,9 et 10,6 Hz, 1 H) ; 7,02 (dt, *J*=4,9 et 7,9 Hz, 1 H) ; 7,08 (d, *J*=7,9 Hz, 1 H) ; 7,95 (m étalé, 1 H)
Spectrométrie de Masse : El : [M]+. m/z = 149

### Etape 4a

### 4-fluoro-2-methyl-2,3-dihydro-1H-indole

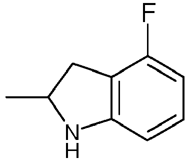

A une solution de 2,87 g de 4-fluoro-2-méthyl-indole dans 98 mL d'acide acétique sous argon refroidie à une température voisine de 14°C, sont ajoutés progressivement 3,63 g de cyanoborohydrure de sodium. Le mélange réactionnel est laissé se réchauffer à température ambiante. Après 2 heures, le mélange réactionnel est versé dans un mélange d'eau et de glace, puis il est traité avec une solution d'ammoniaque à 28% jusqu'à pH 9. Le mélange est ensuite extrait deux fois avec du dichlorométhane. Les phases organiques sont réunies, séchées sur sulfate de magnésium anhydre, filtrées puis concentrées à sec sous pression réduite. Le résidu est purifié sur une colonne de 300 g de silice, en éluant avec un gradient heptane/ acétate d'éthyle 100/0 à 90/10 v/v. On obtient ainsi 2,19 g de 4-fluoro-2-methyl-2,3-dihydro-1H-indole sous forme d'une huile incolore dont les caractéristiques sont les suivantes :
Spectre RMN 1H (400MHz): 1,18 (d, *J=*6,3 Hz, 3 H) ; 2,49 (dd partiellement masqué, J=7,6 et 15,7 Hz, 1 H) ; 3,08 (dd, *J=*9,0 et 15,7 Hz, 1 H) ; 3,92 (m, 1 H) ; 5,87 (s large, 1 H) ; 6,20 à 6,31 (m, 2 H) ; 6,90 (td, *J=*5,9 et 8,1 Hz, 1 H)
Spectrométrie de Masse : méthode A
Temps de rétention Tr (min) = 0,50 ;
[M+H]+ : m/z 152

### Etape 5a

### (R)-2-benzyloxy-1-(4-fluoro-2-méthyl-2,3-dihydro-indol-1-yl)-propan-1-one : diastéréoisomère (A)

### (R)-2-benzyloxy-1-(4-fluoro-2-méthyl-2,3-dihydro-indol-1-yl)-propan-1-one : diastéréoisomère (B)

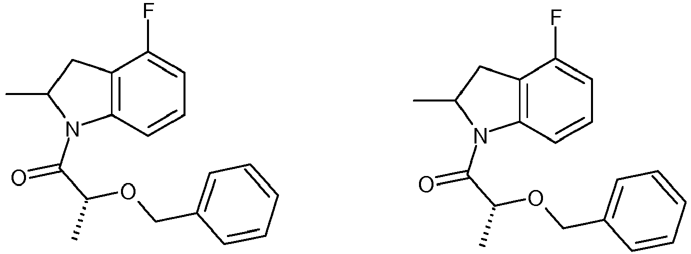

A une solution de 0,76 g d'acide o-benzyl-D-lactique dans 3,5 mL de diméthylformamide sous argon, sont ajoutés 0,69 mL de pyridine et 1,05 g de N-[3-(diméthylamino)propyl]-N'-éthylcarbodiimide chlorhydrate. Le mélange réactionnel est agité à temprérature ambiante pendant 10 minutes, puis 0,64 g de 4-fluoro-2-méthyl-indoline est additionné. Le mélange réactionnel est agité à temprérature ambiante pendant 20 heures, puis il est traité avec 20 mL d'eau et extrait avec 3x15 mL d'acétate d'éthyle. Les phases organiques sont réunies, lavées avec 15 mL d'eau, séchées sur sulfate de magnésium, filtrées puis concentrées à sec sous pression réduite. Le résidu est purifié par chromatographie sur une cartouche de 90 g de silice 15-40 µm, en éluant avec du dichlorométhane pur, puis sur une cartouche de 100 g de silice 15-40 µm, en éluant avec de l'heptane pur puis avec des mélanges heptane/ acétate d'éthyle 95/5 puis 90/10 v/v, à un débit de 85 mL/ min. On obtient ainsi 0,33 g du diastéréoisomère (A) de la
(R)-2-benzyloxy-1-(4-fluoro-2-méthyl-2,3-dihydro-indol-1-yl)-propan-1-one sous forme d'une huile incolore et 0,38 g du diastéréoisomère (B) de la (R)-2-benzyloxy-1-(-4-fluoro-2-méthyl-2,3-dihydro-indol-1-yl)-propan-1-one sous forme d'un solide blanc. Un deuxième essai dans des conditions identiques à partir de 1,85 g d'acide o-benzyl-D-lactique et de 1,55 g de 4-fluoro-2-méthyl-indoline permet d'obtenir 1,39 g du diastéréoisomère (A) de la (R)-2-benzyloxy-1-(4-fluoro-2-méthyl-2,3-dihydro-indol-1-yl)-propan-1-one sous forme d'une huile incolore et 1,34 g du diastéréoisomère (B) de la (R)-2-benzyloxy-1-(-4-fluoro-2-méthyl-2,3-dihydro-indol-1-yl)-propan-1-one sous forme d'un solide blanc.

Les deux lots de diastéréoisomère (A) de la (R)-2-benzyloxy-1-(4-fluoro-2-méthyl-2,3-dihydro-indol-1-yl)-propan-1-one sont réunis et dissous dans 75 mL d'acétate d'éthyle. Le mélange est filtré sur papier puis concentré à sec sous pression réduite. On obtient ainsi 1,66 g du diastéréoisomère (A) de la (R)-2-benzyloxy-1-(4-fluoro-2-méthyl-2,3-dihydro-indol-1-yl)-propan-1-one sous forme d'une huile visqueuse jaune très pâle dont les caractéristiques sont les suivantes :
Spectre RMN 1H (400MHz): 1,18 (d, *J*=6,4 Hz, 3 H) ; 1,39 (d, *J*=6,4 Hz, 3 H) ; 2,71 (d, *J*=16,3 Hz, 1 H) ; 3,29 (dd partiellement masqué, *J*=8,8 et 16,3 Hz, 1 H) ; 4,46 à 4,56 (m, 3 H) ; 4,75 (m, 1 H) ; 6,91 (t, *J*=8,7 Hz, 1 H) ; 7,19 à 7,37 (m, 6 H) ; 7,87 (m étalé, 1 H)
Spectrométrie de Masse : méthode A
Temps de rétention Tr (min) = 1,09 ;
[M+H]+ : m/z 314 ; [M+Na]+ : m/z 336 ; pic de base- : m/z 242

Les deux lots de diastéréoisomère (B) de la (R)-2-benzyloxy-1-(4-fluoro-2-méthyl-2,3-dihydro-indol-1-yl)-propan-1-one sont réunis et dissous dans 75 mL d'acétate d'éthyle. Le mélange est filtré sur papier puis concentré à sec sous pression réduite. On obtient ainsi 1,70 g du diastéréoisomère (B) de la (R)-2-benzyloxy-1-(4-fluoro-2-méthyl-2,3-dihydro-indol-1-yl)-propan-1-one sous forme d'un solide blanc dont les caractéristiques sont les suivantes :
Spectre RMN 1H (400MHz) : 1,18 (d, *J*=6,4 Hz, 3 H) ; 1,40 (d, *J*=6,5 Hz, 3 H) ; 2,72 (d, *J*=16,4 Hz, 1 H) ; 3,28 à 3,37 (m partiellement masqué, 1 H) ; 4,40 à 4,57 (m, 3 H) ; 4,66 (m, 1 H) ; 6,90 (dt, *J=*0,8 et 8,8 Hz, 1 H) ; 7,20 à 7,39 (m, 6 H) ; 7,91 (m large, 1 H)
Spectrométrie de Masse : méthode A
Temps de rétention Tr (min) = 1,08 ;
[M+H]+ : m/z 314 ; [M+Na]+ : m/z 336 ; pic de base : m/z 242

### Etape (6a)a

### (+)-4-fluoro-2-méthyl-indoline

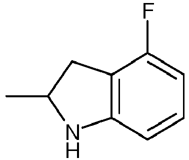

A une solution de 1,66 g du diastéréoisomère (A) de la (R)-2-benzyloxy-1-(4-fluoro-2-méthyl-2,3-dihydro-indol-1-yl)-propan-1-one dans 200 mL d'éthanol absolu, sont ajoutés 8 mL d'acide chlorhydrique concentré. Le mélange réactionnel est chauffé à reflux sous agitation pendant 40 heures, puis il est refroidi à temprérature ambiante et concentré à sec sous pression réduite. Le résidu est repris dans 250 mL d'eau, alcalinisé avec de la soude concentrée jusqu'à pH 14, puis le mélange est extrait avec 3x200 mL de dichlorométhane. Les phases organiques sont réunies, lavées avec de l'eau, séchées sur sulfate de magnésium, filtrées puis concentrées à sec sous pression réduite. Le résidu est purifié par chromatographie sur une cartouche de 70 g de silice 15-40 µm, en éluant avec de l'heptane pur puis avec un mélange heptane/ acétate d'éthyle 95/5 à un débit de 50 mL/ min, puis sur une cartouche de 70 g de silice 15-40 µm, en éluant avec du cyclohexane pur puis avec un mélange cyclohexane/ dichlorométhane 70/30, à un débit de 50 mL/ min. On obtient ainsi 0,50 g de (+)-4-fluoro-2-méthyl-indoline sous forme d'une huile incolore dont les caractéristiques sont les suivantes :
Spectre RMN 1H (400MHz): 1,18 (d, *J*=6,4 Hz, 3 H) ; 2,49 (dd partiellement masqué, *J*=7,6 et 15,7 Hz, 1 H) ; 3,08 (dd, *J*=8,8 et 15,7 Hz, 1 H) ; 3,92 (m, 1 H) ; 5,87 (s large, 1 H) ; 6,21 à 6,28 (m, 2 H) ; 6,90 (dt, *J*=5,9 et 8,1 Hz, 1 H)
Spectrométrie de Masse : méthode A
Temps de rétention Tr (min) = 0,48 ;
[M+H]+ : m/z 152
Pouvoir rotatoire : α_{D} = +40,8° +/-0,9 (c = 2,223 mg dans 0,5 mL de DMSO)

### Etape (6b)a

### (-)-4-fluoro-2-méthyl-indoline

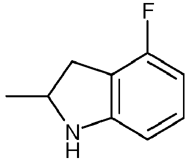

A une solution de 1,69 g du diastéréoisomère (B) de la (R)-2-benzyloxy-1-(4-fluoro-2-méthyl-2,3-dihydro-indol-1-yl)-propan-1-one dans 200 mL d'éthanol absolu, sont ajoutés 8,2 mL d'acide chlorhydrique concentré. Le mélange réactionnel est chauffé à reflux sous agitation pendant 40 heures, puis il est refroidi à temprérature ambiante et concentré à sec sous pression réduite. Le résidu est repris dans 200 mL d'eau, alcalinisé avec de la soude concentrée jusqu'à pH 14, puis le mélange est extrait avec 3x200 mL de dichlorométhane. Les phases organiques sont réunies, lavées avec de l'eau, séchées sur sulfate de magnésium, filtrées puis concentrées à sec sous pression réduite. Le résidu est purifié par chromatographie sur une cartouche de 70 g de silice 15-40 µm, en éluant avec du cyclohexane pur puis avec un mélange cyclohexane/ dichlorométhane 70/30, à un débit de 50 mL/ min. On obtient ainsi 0,56 g de (-)-4-fluoro-2-méthyl-indoline sous forme d'une huile jaune très pâle dont les caractéristiques sont les suivantes :
Spectre RMN 1H (400MHz) : 1,18 (d, *J*=6,2 Hz, 3 H) ; 2,49 (dd partiellement masqué, *J*=7,6 et 15,7 Hz, 1 H) ; 3,08 (dd, *J*=9,0 et 15,7 Hz, 1 H) ; 3,92 (m, 1 H); 5,87 (s large, 1 H) ; 6,20 à 6,29 (m, 2 H) ; 6,90 (dt, *J*=5,9 et 8,1 Hz, 1 H)
Spectrométrie de Masse : méthode A
Temps de rétention Tr (min) = 0,49 ;
[M+H]+ : m/z 152
Pouvoir rotatoire : α_{D} = -33,7° +/-0,7 (c = 2,741 mg dans 0,5 mL de DMSO)

### Exemple de référence 6a : 6-Fluoro-2-methyl-2,3-dihydro-1 H-indole

### Etape 1a :

### 5-Fluoro-2-prop-1-ynyl-phenylamine

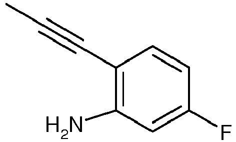

A une solution de 5 g de 5-fluoro-2-iodo-ainiline dans 150 mL de trietyhlamine, sont ajoutés, à la température ambiante, 121 mg d'iodure de cuivre (I) et 148 mg du bis(triphenylphosphine)palladium(II)dichloride. La suspension est refroidit à -30°C dans un bain de carboglace/éthanol. D'autre part, 10 mL de propyne sont condensés par barbotage dans un piège refroidit à -70°C à l'aide d'un mélange de carboglace/méthanol. Le propyne est additionné sur la suspension refroidit à -30°C. Le bain refroidissant est gardé. On laisse la température monter à l'ambiante pendant la nuit.

Le milieu réactionnel est filtré. Le filtrat est concentré à sec sous pression réduite. Le résidu obtenu est repris à l'eau et à l'acétate d'éthyle. Après décantation, la phase organique est séchée sur sulfate de magnésium, filtrée puis concentrée sous pression réduite. Le résidu obtenu est purifié sur colonne de silice, éluant : cyclohexane/acétate d'éthyle 95/05 pour donner 1.7 g de 5-difluoro-2-prop-1-ynylphenylamine dont les caractéristiques sont les suivantes :
Spectrométrie de Masse : méthode A
Temps de rétention Tr (min) = 0,84 ; [M+H]+ : m/z 150 ;

### Etape 2a :

### 6-Difluoro-2-methyl-indole

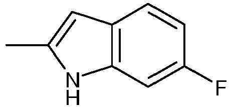

A une solution de 1.7 g du 5-difluoro-2-prop-1-ynyl-phenylamine dans 50 mL de DMF, sont ajoutés 43 mg d'iodure de cuivre (I). Le milieu réactionnel est chauffé à reflux pendant une heure.

Après refroidissement, le milieu réactionnel est filtré. Le filtrat est concentré sous pression réduite. Le résidu brut obtenu est purifié sur colonne de silice, éluant : cyclohexane/acétate d'éthyle 90/10 pour donner 1.1 g de 6-fluoro-2-methyl-indole dont les caractéristiques sont les suivantes :
Spectrométrie de Masse : méthode A
Temps de rétention Tr (min) = 0,89 ; [M+H]+ : m/z 150 ;

### Etape 3a :

### 6-Fluoro-2-methyl-2,3-dihydro-1H-indole

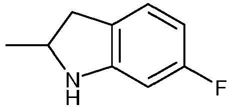

Le produit est préparé en suivant le mode opératoire décrit à l'exemple de reference 2a (Etape 1a) à partir de 1.4 g du 6-fluoro-2-methyl-indole, de 51 ml d'acide acétique et de 1.9 g de cyanoborohydrure de sodium. Après purification sur colonne de silice, éluant : heptane/acétate d'éthyle 90/10, on obtient 1.33 g de 6-fluoro-2-methyl-2,3-dihydro-1H-indole qui est utilisé tel quel dans l'étape suivante.

### Etape 4a :

### (R)-1-(6-Fluoro-2-methyl-2,3-dihydro-indol-1-yl)-2-phenoxy-propan-1-one: diastéréoisomère A

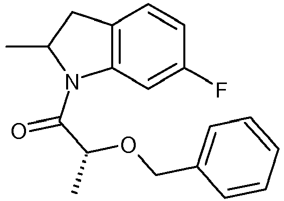

### Et

### (R)-1-(6-Fluoro-2-methyl-2,3-dihydro-indol-1-yl)-2-phenoxy-propan-1-one: diastéréoisomère B

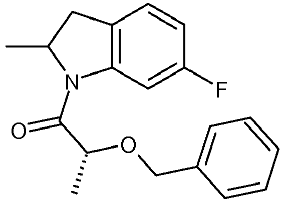

Les produits sont préparés en suivant le mode opératoire décrit à l'exemple de référence 1a (Etape 2a) à partir de 1.33 g du (6-fluoro-2-methyl-2,3-dihydro-1H-indole et de 2.1 g d'acide o-benzyl-D-lactique. Après purification sur colonne de silice, on obtient 1.3 g de (R)-1-(6-fluoro-2-methyl-2,3-dihydro-indol-1-yl)-2-phenoxy-propan-1-one : diastéréoisomère A dont les caractéristiques sont les suivantes :
Spectrométrie de Masse : méthode A
Temps de rétention Tr (min) = 1,09 ;
[M+H]+ : m/z 314 ; pic de base : m/z 242

Et 1.13 g de (R)-1-(6-fluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,08 ;
[M+H]+ : m/z 314 ; [M+Na]+ : m/z 336 ;
pic de base : m/z 242

### Etape (5a)a :

### (+)-6-Fluoro-2-methyl-2,3-dihydro-1H-indole

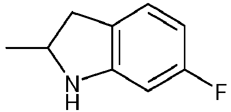

Le produit est préparé en suivant le mode opératoire décrit à l'exemple de référence 1a (Etape 3a) à partir de 1.3 g du (R)-1-(6-fluoro-2-methyl-2,3-dihydro-indol-1-yl)-2-phenoxy-propan-1-one : diastéréoisomère A et de 13 mL d'acide chlorhydrique 37%.

Après traitement, on obtient 547 mg de (+)-6-fluoro-2-methyl-2,3-dihydro-1H-indole dont les caractéristiques sont les suivantes :
Spectrométrie de Masse : méthode A
Temps de rétention Tr (min) = 0,48 ; [M+H]+ : m/z 152 ;
Pouvoir rotatoire : α_{D} =+35.0+/-0.7. C=2.899mg/0.5ML DMSO

### Etape (5b)a :

### (-)-6-Fluoro-2-methyl-2,3-dihydro-1H-indole

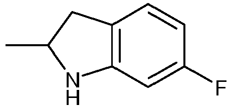

Le produit est préparé en suivant le mode opératoire décrit à l'exemple de référence 1a (Etape 3a) à partir de 1.13 g du (R)-1-(6-fluoro-2-methyl-2,3-dihydro-indol-1-yl)-2-phenoxy-propan-1-one : diastéréoisomère B et de 12 mL d'acide chlorhydrique 37%.

Après traitement, on obtient 540 mg de (R)-6-fluoro-2-methyl-2,3-dihydro-indol-1-yl)-2-phenoxy-propan-1-one dont les caractéristiques sont les suivantes :
Spectrométrie de Masse : méthode A
Temps de rétention Tr (min) = 0,48 ; [M+H]+ : m/z 152 ;
Pouvoir rotatoire : α_{D} = -32.6+/-1.0. C=1.506mg/0.5ML DMSO

### Exemple de référence 7a : 4-chloro-5-fluoro-2-methyl-2,3-dihydro-1H-indole

### Etape 1a :

### 3-chloro-4-fluoro-2-iodo-ainline

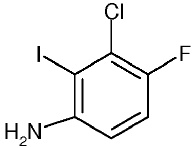

A une suspension de 50 g de 3-chloro-4-fluoroaniline dans 800 mL d'eau, sont ajoutés, à la température ambiante, 113.3 g d'iode et 43.3 g de bicarbonate de sodium. Le milieu réactionnel est agité à la température ambiante pendant 18 heures.

Addition d'une solution saturée de thiosulfate de sodium, puis extraction 3 fois à l'acétate d'éthyle. Les phases organique sont réunies, séchées sur sulfate de magnésium, filtrées puis concentrées sous pression réduite. Le résidu obtenu est purifié sur colonne de silice, éluant : cyclohexane/acétate d'éthyle 95/05 puis 85/15 pour donner 40.9 g du 5-chloro-4-fluoro-2-iodo-ainline dont les caractéristiques sont les suivantes :
Spectre RMN 1 H (400 MHz) : 5,29 (s large, 2 H) ; 6,87 (d, *J*=6,9 Hz, 1 H) ; 7,61 (d, *J*=8,8 Hz, 1 H)
Spectrométrie de Masse : méthode A
Temps de rétention Tr (min) = 0,99 ;
[M+H]+ : m/z 272 ; pic de base : m/z 313
et 12.5 g de 3-chloro-4-fluoro-2-iodo-ainline dont les caractéristiques sont les suivantes :
Spectre RMN 1 H (400 MHz) : 5,38 (s large, 2 H) ; 6,76 (dd, *J*=4,8 et 8,9 Hz, 1 H) ; 7,16 (t, *J*=8,9 Hz, 1 H)
Spectrométrie de Masse : méthode A
Temps de rétention Tr (min) = 0,95 ;
[M+H]+ : m/z 271

### Etape 2a :

### 3-chloro-4-fluoro-2-prop-1-ynyl-phenylamine

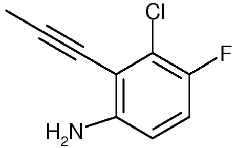

A une solution de 9 g de 3-chloro-4-fluoro-2-iodo-ainline dans 160 mL de trietyhlamine, sont ajoutés, à la température ambiante, 364 mg d'iodure de cuivre (I) et 470 mg du bis(triphenylphosphine)palladium(II)dichloride. La suspension est refroidit à-30°C dans un bain de carboglace/éthanol. D'autre part, environ 20 mL de propyne sont condensés par barbotage dans un piège refroidit à -70°C à l'aide d'un mélange de carboglace/méthanol. Le propyne est additionné sur la suspension refroidit à -30°C. Le bain refroidissant est gardé. On laisse la température monter à l'ambiante pendant la nuit.

Le milieu réactionnel est filtré. Le filtrat est concentré à sec sous pression réduite. Le résidu obtenu est purifié sur colonne de silice, éluant : heptane/acétate d'éthyle 90/10 pour donner 1.76 g de 3-chloro-4-fluoro-2-prop-1-ynyl-phenylamine
dont les caractéristiques sont les suivantes :
Spectrométrie de Masse : méthode A
Temps de rétention Tr (min) = 0,92 ; [M+H]+ : m/z 184 ; pic de base : m/z 149

### Etape 3a :

### 4-chloro-5-fluoro-2-méthylindole

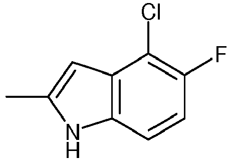

A une solution de 1.56 g du 3-chloro-4-fluoro-2-prop-1-ynyl-phenylamine dans 17 mL de DMF, sont ajoutés 32 mg d'iodure de cuivre (I). Le milieu réactionnel est chauffé à reflux pendant 45 minutes.

Après refroidissement, le milieu réactionnel est filtré. Le filtrat est concentré sous pression réduite. Le résidu brut obtenu est purifié sur colonne de silice, éluant : heptane/toluène 70/30 pour donner 0.5 g de 4-chloro-5-fluoro-2-méthylindole dont les caractéristiques sont les suivantes :
Spectrométrie de Masse : méthode A
Temps de rétention Tr (min) = 1,01 ; [M-H]- : m/z 182

### Etape 4a :

### 4-Chloro-5-fluoro-2-methyl-2,3-dihydro-1H-indole

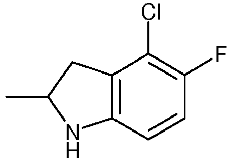

A une solution de 700 mg du 4-chloro-5-fluoro-2-méthylindole dans 16 mL d'acide acétique refroidit à 15°C, sont ajoutés en une fois 719 mg de cyanoborohydrure de sodium. Le milieu réactionnel est agité à 15°C pendant 10 minutes puis 90 minutes à la température ambiante.

Le milieu réactionnel est refroidit de nouveau à 5°C. Addition de l'eau glacée. Addition de l'ammoniaque 30% jusqu'au PH = 9. Extraction à l'acétate d'éthyle. La phase organique est séchée sur sulfate de magnésium, filtrée puis concentrée sous pression réduite pour donner 683 mg de 4-chloro-5-fluoro-2-methyl-2,3-dihydro-1H-indole dont les caractéristiques sont les suivantes :
Spectrométrie de Masse : méthode A
Temps de rétention Tr (min) = 0,75 ;
[M+H]+ : m/z 186 ;

### Exemple de référence 8a : 2-Hydroxyméthyl-2,3-dihydro-1H-indole

### Etape 1a:

### 1-((R)-2-Benzyloxy-propionyl)-2,3-dihydro-1H-indole-2-carboxylic acid ethyl ester : diastéréoisomère A

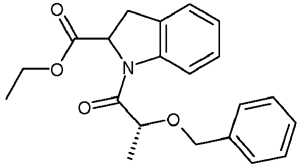

### Et

### 1-((R)-2-Benzyloxy-propionyl)-2,3-dihydro-1H-indole-2-carboxylic acid ethyl ester diastéréoisomère B

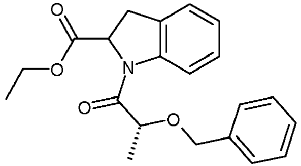

A une solution de 12.6 g d'acide o-benzyl-D-lactique dans 30 mL de DMF et 10.6 mL de pyridine sont ajoutés 16.6 g de N-[3-(diméthylamino)propyl]-N'-éthylcarbodiimide chlorhydrate puis 10 g d'indoline-2-carboxylate d'éthyle. Le milieu réactionnel est agité à la température ambiante pendant 18 heures. Le milieu réactionnel est concentré sous pression réduite au 2/3 du volume du milieu réactionnel. Addition de l'acétate d'éthyle et d'eau. Après décantation, la phase organique est séchée sur sulfate de magnésium, filtrée puis concentrée sous pression réduite. Le résidu obtenu est purifié sur cartouche de silice : éluant : gradient : heptane/acétate d'éthyle de 100/0 à 80/20, pour donner 7.24 g de 1-((R)-2-benzyloxy-propionyl)-2,3-dihydro-1H-indole-2-carboxylic acid ethyl ester : diastéréoisomère A dont les caractéristiques sont les suivantes :
Spectrométrie de Masse : méthode A
Temps de rétention Tr (min) = 1,08 ;
[M+H]+ : m/z 354 ; [M+Na]+ : m/z 376 (pic de base)

Et 7.5 g du 1-((R)-2-benzyloxy-propionyl)-2,3-dihydro-1H-indole-2-carboxylic acid ethyl ester: diastéréoisomère B sous forme d'un solide blanc dont les caractéristiques sont les suivantes :
Spectrométrie de Masse : méthode A
Temps de rétention Tr (min) = 1,06 ;
[M+H]+ : m/z 354 ; [M+Na]+ : m/z 376 ; pic de base : m/z 282

### Etape 2a:

### (R)-2-Benzyloxy-1-(2-hydroxymethyl-2,3-dihydro-indol-1-yl)-propan-1-one : diastéréoisomère A

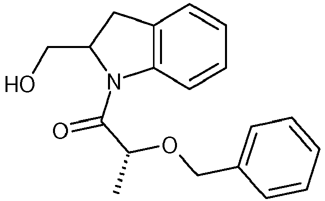

Et

### (+)-(2,3-Dihydro-1H-indol-2-yl)-methanol

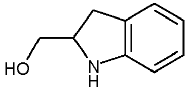

A une solution de 3.31 g du 1-((R)-2-benzyloxy-propionyl)-2,3-dihydro-1H-indole-2-carboxylic acid ethyl ester: diastéréoisomère A dans 7.5 mL de THF et 7.5 mL d'éthanol sont ajoutés 1.04 g de borohydrure de sodium.

Le milieu réactionnel est agité à la température ambiante pendant 5 heures.

Addition du dichlorométhane et de l'eau. Après décantation, la phase organique est séchée sur sulfate de magnésium, filtrée puis concentrée sous pression réduite. Le résidu obtenu est purifié sur cartouche de silice : éluant : heptane/acétate d'éthyle 50/50, pour donner 0.98 g de (R)-2-benzyloxy-1-(2-hydroxymethyl-2,3-dihydro-indol-1-yl)-propan-1-one : diastéréoisomère A dont les caractéristiques sont les suivantes :
Spectrométrie de Masse : méthode A
Temps de rétention Tr (min) = 0.85 ;
[M+H]+ : m/z 312

Et 1.65 g du (+)-(2,3-dihydro-1H-indol-2-yl)-methanol sous forme d'un solide blanc dont les caractéristiques sont les suivantes :
Spectrométrie de Masse : méthode A
Temps de rétention Tr (min) = 0,19 ;
[M+H]+ : m/z 150

### Etape 3a:

### (+)-(2,3-Dihydro-1H-indol-2-yl)-methanol

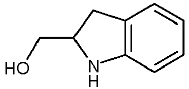

A une solution de 0.9 g du (R)-2-benzyloxy-1-(2-hydroxymethyl-2,3-dihydro-indol-1-yl)-propan-1-one : diastéréoisomère A dans 9 mL d'éthanol et 9 mL d'acide chlorhydrique 37% sont chauffés à reflux pendant deux heures. Le milieu réactionnel est concentré sous pression réduite. Le résidu obtenu est repris avec de l'eau. Addition de la soude 2N jusqu'au PH=10. Le milieu est extrait avec du dichlorométhane. Après décantation, la phase organique est séchée sur sulfate de magnésium, filtrée puis concentrée sous pression réduite pour donner 0.4 g du (+)-(2,3-Dihydro-1H-indol-2-yl)-methanol dont les caractéristiques sont les suivantes :
Spectrométrie de Masse : méthode A
Temps de rétention Tr (min) = 0,19 ;
[M+H]+ : m/z 150
PR= +38.5+/-0.9. C=1.974mg/0.5ML DMSO

### Etape 4a:

### (R)-2-Benzyloxy-1-(2-hydroxymethyl-2,3-dihydro-indol-1-yl)-propan-1-one : diastéréoisomère B

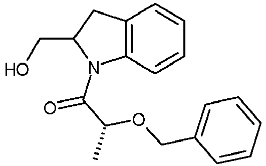

### Et

### (-)-(2,3-Dihydro-1H-indol-2-yl)-methanol

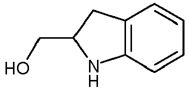

A une solution de 5.75 g du 1-((R)-2-benzyloxy-propionyl)-2,3-dihydro-1H-indole-2-carboxylic acid ethyl ester: diastéréoisomère B dans 20 mL de THF sont ajoutés 1.36 g de borohydrure de sodium.

Le milieu réactionnel est agité à la température ambiante pendant 18 heures.

Addition de 10 mL d'éthanol et 0.4 g de borohydrure de sodium. Après deux heures d'agitation à la température ambiante, addition du dichlorométhane et de l'eau.

Après décantation, la phase organique est séchée sur sulfate de magnésium, filtrée puis concentrée sous pression réduite. Le résidu obtenu est purifié sur cartouche de silice : éluant : gradient : dichlorométhane/méthanol de100/0 à 98/02, pour donner 0.51 g de (R)-2-benzyloxy-1-(2-hydroxymethyl-2,3-dihydro-indol-1-yl)-propan-1-one : diastéréoisomère B dont les caractéristiques sont les suivantes :
Spectrométrie de Masse : méthode A
Temps de rétention Tr (min) = 0,82 ; [M+H]+ : m/z 312

Et 0.96 g du (-)-(2,3-dihydro-1H-indol-2-yl)-methanol sous forme d'un solide blanc dont les caractéristiques sont les suivantes :
Spectrométrie de Masse : méthode A
Temps de rétention Tr (min) = 0,19 ; [M+H]+ : m/z 150
PR= -38.9+/-0.8. C=2.255mg/0.5ML DMSO

### Etape 3a:

### (-)-(2,3-Dihydro-1H-indol-2-yl)-methanol

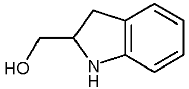

A une solution de 117 mg du (R)-2-benzyloxy-1-(2-hydroxymethyl-2,3-dihydro-indol-1-yl)-propan-1-one : diastéréoisomère B dans 1.2 mL d'éthanol et 1.2 mL d'acide chlorhydrique 37% sont chauffés à reflux pendant deux heures.

Le milieu réactionnel est concentré sous pression réduite. Le résidu obtenu est repris avec de l'eau. Addition de la soude 2N jusqu'au PH=10. Le milieu est extrait avec du dichlorométhane. Après décantation, la phase organique est séchée sur sulfate de magnésium, filtrée puis concentrée sous pression réduite pour donner 50 mg du (-)-(2,3-Dihydro-1H-indol-2-yl)-methanol dont les caractéristiques sont les suivantes :
Spectrométrie de Masse : méthode A
Temps de rétention Tr (min) = 0,19 ;
[M+H]+ : m/z 150
PR= -38.9+/-0.8. C=2.255mg/0.5ML DMSO

### Synthèse des composés de formule (Ib):

### Exemple 1b : Synthèse du N-(4-fluoro-phényl)-2-[4-(2-méthyl-morpholin-4-yl)-6-oxo-1,6-dihydro-pyrimidin-2-yl]-acétamide

### Étape 1 b : Synthèse de l'ester éthylique de l'acide [4-(2-méthyl-morpholin-4-yl)-6-oxo-1,6-dihydro-pyrimidin-2-yl]-acétique

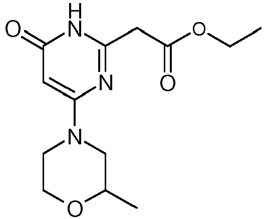

Dans un tube micro-onde, sont introduits, 20 mL d'éthanol, 1.5 g de 2-méthylmorpholine, 8.7 g de chlorhydrate de 3-éthoxy-3-iminopropanoate d'éthyle et 7.75 mL de N,N-diisopropyléthylamine. Après une heure d'irradiation aux micro-ondes à une température de 130°C sous irradiation micro-ondes, le milieu réactionnel est concentré sous pression réduite. Le résidu obtenu est purifié sur colonne de silice, éluant : dichlorométhane/méthanol : 97/03 pour donner 0.8 g de l'ester éthylique de l'acide [4-(2-méthyl-morpholin-4-yl)-6-oxo-1,6-dihydro-pyrimidin-2-yl]-acétique sous forme de solide beige dont les caractéristiques sont les suivantes :
Spectrométrie de Masse : méthode A
Temps de rétention Tr (min) = 0,56 ;
[M+H]+ : m/z 282 ; [M-H]- : m/z 280

### Étape 2b: Synthèse du sel de sodium de l'acide [4-(2-méthylmorpholin-4-yl)-6-oxo-1,6-dihydropyrimidin-2-yl]acétique

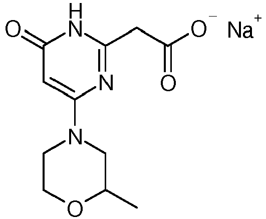

A une solution de 0.8 g de [4-(2-méthyl-morpholin-4-yl)-6-oxo-1,6-dihydro-pyrimidin-2-yl]-acetic acid éthyl ester dans 8 mL de tétrahydrofuranne, est ajouté 1.8 mL de soude 2M. Le mélange réactionnel est agité pendant 24 heures à la température ambiante. Le milieu réactionnel est concentré sous pression réduite. Le solide obtenu est alors séché à l'évaporateur rotatif pour donner 0.7 g de sel de sodium de l'acide [4-(2-méthylmorpholin-4-yl)-6-oxo-1,6-dihydropyrimidin-2-yl]acétique sous forme de solide beige dont les caractéristiques sont les suivantes:
Spectrométrie de Masse : méthode A
[M+H]+ : m/z 254 ; [M-H]- : m/z 252 ; pic de base : m/z 208

### Étape 3b : Synthèse du N-(4-fluoro-phényl)-2-[4-(2-méthyl-morpholin-4-yl)-6-oxo-1,6-dihydro-pyrimidin-2-yl]-acétamide

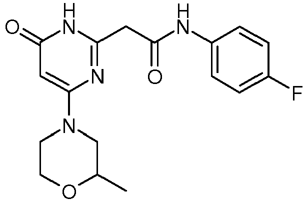

A une solution de 700 mg sel de sodium de l'acide [4-(2-méthylmorpholin-4-yl)-6-oxo-1,6-dihydropyrimidin-2-yl]acétique dans 6.7 mL de N,N-diméthylformamide sont ajoutés 1.46 g de chlorhydrate de N-[3-(diméthylamino)propyl]-N'-éthylcarbodiimide, 6.7 mL de pyridine et 0.7 mL de 4-fluoroaniline. Le mélange réactionnel est agité à la température ambiante pendant 24 heures, puis concentré sous pression réduite. On ajoute de l'eau et de l'acétate d'éthyle et agite ainsi pendant 30 minutes. Le précipité formé est filtré, lavé au dichlorométhane et à l'éther éthylique pour donner 300 mg du N-(4-fluoro-phényl)-2-[4-(2-méthyl-morpholin-4-yl)-6-oxo-1,6-dihydro-pyrimidin-2-yl]-acétamide sous forme de solide blanc dont les caractéristiques sont les suivantes:
Spectre RMN 1H (400 MHz) : 1,07 (d, J=6,1 Hz, 3 H) ; 2,47 (dd, J=10,9 et 13,0Hz, 1 H) ; 2,80 (m, 1 H) ; 3,39 à 3,50 (m, 2 H) ; 3,58 (s, 2 H) ; 3,82 (dd, J=3,0 et 10,9 Hz, 1 H) ; 3,95 (m, 1 H) ; 4,04 (m, 1 H) ; 5,21 (s, 1 H) ; 7,15 (t, J=8,9 Hz, 2 H) ; 7,58 (dd, J=5,4 et 8,9 Hz, 2 H) ; 10,19 (s large, 1 H) ; 11,62 (m étalé, 1 H)
Spectrométrie de Masse : méthode A
Temps de rétention Tr (min) = 0,66 ;
[M+H]+ : m/z 347 ; [M-H]- : m/z 345

### Exemple 2b : Synthèse du (+)-N-(4-fluoro-phényl)-2-[4-(2-méthyl-morpholin-4-yl)-6-oxo-1,6-dihydro-pyrimidin-2-yl]-acétamide

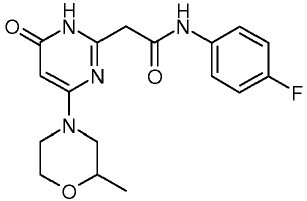

La séparation des deux énantiomères de la N-(4-fluoro-phényl)-2-[4-(2-méthyl-morpholin-4-yl)-6-oxo-1,6-dihydro-pyrimidin-2-yl]-acétamide (exemple 1b) (250 mg) a été réalisée par chromatographie chirale : Phase stationnaire : chiralapk AS 20µm; phase mobile : Heptane (80%) / EtOH (10%) / MeOH (10%) ; débit : 140 mL/mn.

L'énantiomère lévogyre est concentré pour obtenir 112 mg de la N-(4-fluorophényl)-2-[4-((-)-2-méthyl-morpholin-4-yl)-6-oxo-1,6-dihydro-pyrimidin-2-yl]-acétamide sous forme d'un solide blanc dont les caractéristiques sont les suivantes :
Spectrométrie de Masse : méthode A
Temps de rétention Tr (min) = 0,66 ; [M+H]+ : m/z 347 ; [M-H]- : m/z 345
Pouvoir rotatoire : α_{D} = -15.0+/-0.6. C= 2.243mg/0.5ML DMSO

L'énantiomère dextrogyre est concentré pour obtenir 117 mg de la N-(4-fluorophényl)-2-[4-((+)-2-méthyl-morpholin-4-yl)-6-oxo-1,6-dihydro-pyrimidin-2-yl]-acétamide sous forme d'un solide blanc dont les caractéristiques sont les suivantes :
Spectre RMN 1H (400 MHz) : 1,07 (d, J=6,4 Hz, 3 H) ; 2,47 (dd, J=10,9 et 13,0Hz, 1 H) ; 2,80 (m, 1 H) ; 3,39 à 3,50 (m, 2 H) ; 3,58 (s, 2 H) ; 3,82 (dd, J=3,5 et 10,9 Hz, 1 H) ; 3,95 (m, 1 H) ; 4,04 (m, 1 H) ; 5,21 (s, 1 H) ; 7,15 (t, J=8,9 Hz, 2 H) ; 7,58 (dd, J=5,4 et 8,9 Hz, 2 H) ; 10,21 (s large, 1 H) ; 11,62 (m étalé, 1 H)
Spectrométrie de Masse : méthode A
Temps de rétention Tr (min) = 0,66 ; [M+H]+: m/z 347 ; [M-H]- : m/z 345
Pouvoir rotatoire : α_{D}= +16.2+/-0.7. C=1.824mg/0.5ML DMSO

### Exemple 3b : Synthèse du 2-[4-(2-fluoromethyl-morpholin-4-yl)-6-oxo-1,6-dihydro-pyrimidin-2-yl]-N-(4-fluoro-phenyl)-acetamide

### Étape 1b : Synthèse de l'ester éthylique de l'acide [4-(2-fluorométhyl-morpholin-4-yl)-6-oxo-1,6-dihydro-pyrimidin-2-yl]-acétique

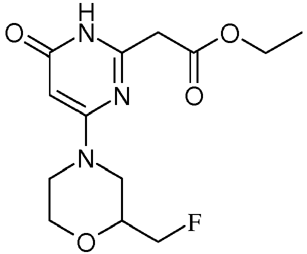

Le produit est préparé en suivant le mode opératoire décrit à l'étape 1b de l'exemple 1 b à partir de 1.5 g du chlorhydrate du 2-fluorométhylmorpholine (qui peut être préparée selon Yoshikazu J. et Coll. (J. Med. Chem.(1994), 37(17), 2791-2796; 1994) au lieu de la 2-méthylmorpholine et de 5.6 g de chlorhydrate de 3-éthoxy-3-iminopropanoate d'éthyle. On obtient 700 mg de de l'ester éthylique de l'acide [4-(2-fluorométhyl-morpholin-4-yl)-6-oxo-1,6-dihydro-pyrimidin-2-yl]-acétique sous forme de solide blanc dont les caractéristiques sont les suivantes:
Spectrométrie de Masse : méthode A
Temps de rétention Tr (min) = 0,55 ;
[M+H]+ : m/z 300 ; [M-H]- : m/z 298

### Étape 2b : Synthèse du sel de sodium de l'acide [4-(2-fluorométhyl-morpholin-4-yl)-6-oxo-1,6-dihydro-pyrimidin-2-yl]-acétique

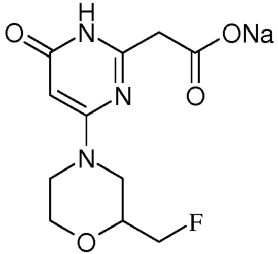

Le produit est préparé en suivant le mode opératoire décrit à l'étape 2b de l'exemple 1b à partir de 700 mg du [4-(2-fluorométhyl-morpholin-4-yl)-6-oxo-1,6-dihydropyrimidin-2-yl]-acetic acid éthyl ester et 1.4 mL de soude 2M. On obtient 670 mg du sel de sodium de l'acide [4-(2-fluorométhyl-morpholin-4-yl)-6-oxo-1,6-dihydro-pyrimidin-2-yl]-acétique dont les caractéristiques sont les suivantes:
Spectrométrie de Masse : méthode A
[M+H]+ : m/z 272 ; [M-H]- : m/z 270 ; pic de base : m/z 226

### Étape 3b: Synthèse du 2-[4-(2-fluorométhyl-morpholin-4-yl)-6-oxo-1,6-dihydropyrimidin-2-yl]-N-(4-fluoro-phényl)-acétamide

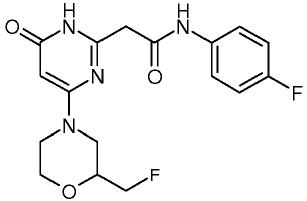

Le produit est préparé en suivant le mode opératoire décrit à l'étape 3b de l'exemple 1b mais à partir de 670 mg du sel de sodium de l'acide [4-(2-fluorométhylmorpholin-4-yl)-6-oxo-1,6-dihydro-pyrimidin-2-yl]-acétique et de 762 mg de 4-fluoroaniline. On obtient 300 mg de 2-[4-(2-fluorométhyl-morpholin-4-yl)-6-oxo-1,6-dihydro-pyrimidin-2-yl]-N-(4-fluoro-phényl)-acétamide sous forme d'un solide blanc dont les caractéristiques sont les suivantes :
Spectre RMN 1H (400 MHz) : 2,69 (dd, J=10,9 et 13,0 Hz, 1 H) ; 2,85 (m, 1 H) ; 3,49 (m, 1 H) ; 3,59 (s, 2 H) ; 3,64 (m, 1 H) ; 3,90 (dd, J=3,0 et 10,9 Hz, 1 H) ; 3,98 (m, 1 H) ; 4,09 (m, 1 H) ; 4,44 (dm, J=47,5 Hz, 2 H) ; 5,24 (s, 1 H) ; 7,15 (t, J=8,9 Hz, 2 H) ; 7,58 (dd, J=5,4 et 8,9 Hz, 2 H) ; 10,21 (s large, 1 H) ; 11,68 (m étalé, 1 H)
Spectrométrie de Masse : méthode A
Temps de rétention Tr (min) = 0,65 ;
[M+H]+ : m/z 365 ; [M-H]- : m/z 363

### Exemple 4b : Synthèse du (+)-N-(4-fluoro-phényl)-2-[4-(2-fluorométhylmorpholin-4-yl)-6-oxo-1,6-dihydro-pyrimidin-2-yl]-acétamide

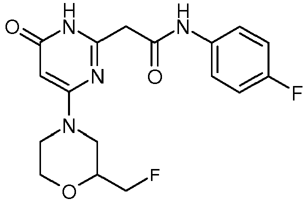

La séparation des deux énantiomères du N-(4-fluoro-phényl)-2-[4-(2-fluorométhyl-morpholin-4-yl)-6-oxo-1,6-dihydro-pyrimidin-2-yl]-acétamide (215 mg) a été réalisée par chromatographie chirale :
Phase stationnaire : Chiralpak AS 20µm; phase mobile : Heptane (70%) / EtOH (20%) / MeOH (10%) ; débit : 180 mL/mn.

L'énantiomère lévogyre est concentré pour obtenir 74 mg de la (-)-N-(4-fluorophényl)-2-[4-(2-fluorométhyl-morpholin-4-yl)-6-oxo-1,6-dihydro-pyrimidin-2-yl]-acétamide sous forme d'un solide blanc dont les caractéristiques sont les suivantes :
Spectrométrie de Masse : méthode A
Temps de rétention Tr (min) = 3,05 ;
[M+H]+ : m/z 365 ; [M-H]- : m/z 363
Pouvoir rotatoire : α_{D} = -14.3 +/-0.6 C=2.173 mg/0.5 ml/DMSO

L'énantiomère dextrogyre est concentré pour obtenir 97 mg du (+) -N-(4-fluorophényl)-2-[4-(2-fluorométhyl-morpholin-4-yl)-6-oxo-1,6-dihydro-pyrimidin-2-yl]-acétamide sous forme d'un solide blanc dont les caractéristiques sont les suivantes :
Spectre RMN 1H (400 MHz) : 2,69 (dd, J=10,9 et 13,0 Hz, 1 H) ; 2,85 (m, 1 H) ; 3,49 (m, 1 H) ; 3,59 (s, 2 H) ; 3,66 (m, 1 H) ; 3,90 (dd, J=3,5 et 10,9 Hz, 1 H) ; 3,98 (m, 1 H) ; 4,09 (m, 1 H) ; 4,44 (dm, J=47,5 Hz, 2 H) ; 5,24 (s, 1 H) ; 7,15 (t, J=8,9 Hz, 2 H) ; 7,58 (dd, J=5,4 et 8,9 Hz, 2 H) ; 10,22 (s large, 1 H); 11,68 (m étalé, 1 H)
Spectrométrie de Masse : méthode B
Temps de rétention Tr (min) = 3,05 ;
[M+H]+ : m/z 365 ; [M-H]- : m/z 363
Pouvoir rotatoire : α_{D} = +16.1+/-0.5. C=3.046mg/0.5ML DMSO

### Exemple 5b : Synthèse du (+)-N-(3-chloro-4-fluoro-phényl)-2-[4-(2-fluorométhyl-morpholin-4-yl)-1-méthyl-6-oxo-1,6-dihydro-pyrimidin-2-yl]-acétamide

### Étape 1b : Synthèse de l'ester éthylique de l'acide [4-(2-fluorométhyl-morpholin-4-yl)-1-méthyl-6-oxo-1,6-dihydro-pyrimidin-2-yl]-acétique

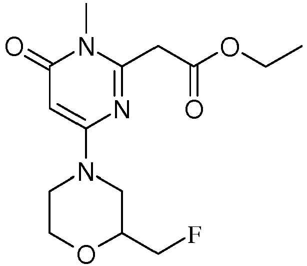

Sur un mélange de 1.5 g de l'ester éthylique de l'acide [4-(2-fluorométhylmorpholin-4-yl)-6-oxo-1,6-dihydro-pyrimidin-2-yl]-aceticque dans 35 mL de dioxane, sont ajoutés 2.1 g de carbonate de césium et 0.8 mL d'iodomethane. La suspension est agitée à la température ambiante pendant 24 heures.

Le milieu réactionnel est filtré. Le filtrat est concentré sous pression réduite. Le résidu obtenu est purifié sur colonne de silice : éluant dichlorométhane/méthanol 98/02 pour donner 253 mg de l'ester éthylique de l'acide [4-(2-fluorométhyl-morpholin-4-yl)-1-méthyl-6-oxo-1,6-dihydro-pyrimidin-2-yl]-acétique dont les caractéristiques sont les suivants :
Spectrométrie de Masse : méthode A
Temps de rétention Tr (min) = 0,60 ;
[M+H]+ : m/z 314 ; [M-H]- : m/z 312

### Étape 2b : Synthèse du sel de sodium de l'acide [4-(2-fluorométhyl-morpholin-4-yl)-1-méthyl-6-oxo-1,6-dihydro-pyrimidin-2-yl]-acétique

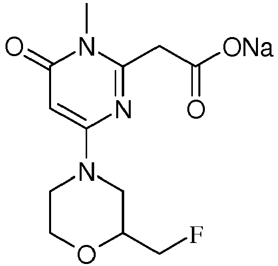

Le produit est préparé en suivant le mode opératoire décrit à l'étape 2b de l'exemple 1b à partir de 250 mg de de l'ester éthylique de l'acide [4-(2-fluorométhyl-morpholin-4-yl)-1-méthyl-6-oxo-1,6-dihydro-pyrimidin-2-yl]-acétique et 0.6 mL de soude 2M. On obtient 250 mg du sel de sodium de l'acide [4-(2-fluorométhyl-morpholin-4-yl)-1-méthyl-6-oxo-1,6-dihydro-pyrimidin-2-yl]-acétique dont les caractéristiques sont les suivantes:
Spectrométrie de Masse : méthode A
Temps de rétention Tr (min) = 0,38 ;
[M+H]+ : m/z 286 ;[M-H]- : m/z 284 ; pic de base : m/z 240

### Étape 3b : Synthèse du (+)-N-(3-chloro-4-fluoro-phényl)-2-[4-(2-fluorométhylmorpholin-4-yl)-1-méthyl-6-oxo-1,6-dihydro-pyrimidin-2-yl]-acétamide

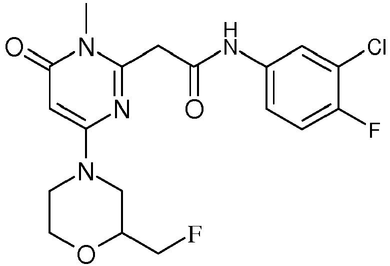

Le produit est préparé en suivant le mode opératoire décrit à l'étape 3b de l'exemple 1b mais à partir de 250 mg du sel de sodium de l'acide [4-(2-fluorométhylmorpholin-4-yl)-1-méthyl-6-oxo-1,6-dihydro-pyrimidin-2-yl]-acétique et de 237 mg de 3-chloro-4-fluoroaniline. On obtient 97 mg de 2-[4-(2-fluorométhyl-morpholin-4-yl)-1-méthyl-6-oxo-1,6-dihydro-pyrimidin-2-yl]-N-(3-chloro-4-fluoro-phényl)-acétamide sous forme d'un solide blanc dont les caractéristiques sont les suivantes :
Spectrométrie de Masse : méthode A
Temps de rétention Tr (min) = 3,58 ;
[M+H]+ : m/z 413 ; [M-H]- : m/z 411

La séparation des deux énantiomères de la N-(3-chloro-4-fluoro-phényl)-2-[4-(2-fluorométhyl-morpholin-4-yl)-1-méthyl-6-oxo-1,6-dihydro-pyrimidin-2-yl]-acétamide (80 mg) a été réalisée par chromatographie chirale : Phase stationnaire : Chiralpak AS 20µm; phase mobile : Heptane (70%) / EtOH (20%) / MeOH (10%) ; débit : 120 mL/mn.

Le premier énantiomère est concentré pour obtenir 36 mg de la (-)-N-(3-chloro-4-fluoro-phényl)-2-[4-(2-fluorométhyl-morpholin-4-yl)-1-méthyl-6-oxo-1,6-dihydro-pyrimidin-2-yl]-acétamide sous forme d'un solide blanc dont les caractéristiques sont les suivantes :
Spectrométrie de Masse : méthode A
Temps de rétention Tr (min) = 0,79 ; [M+H]+ : m/z 413 ; [M-H]- : m/z 411

Le deuxième énantiomère est concentré pour obtenir 36 mg de la (+)-N-(3-chloro-4-fluoro-phényl)-2-[4-(2-fluorométhyl-morpholin-4-yl)-1-méthyl-6-oxo-1,6-dihydropyrimidin-2-yl]-acétamide sous forme d'un solide blanc dont les caractéristiques sont les suivantes :
Spectre RMN 1 H (500 MHz,) : 2,67 (m, 1 H) ; 2,83 (m, 1 H) ; 3,34 (m partiellement masqué, 3 H) ; 3,47 (m, 1 H) ; 3,62 (m, 1 H) ; 3,85 à 3,98 (m, 4 H) ; 4,07 (m, 1 H) ; 4,40 (dm, J=47,5 Hz, 2 H) ; 5,40 (s, 1 H) ; 7,38 (t, J=9,1 Hz, 1 H) ; 7,44 (m, 1 H) ; 7,89 (dd, J=2,2 et 6,6 Hz, 1 H) ; 10,44 (s large, 1 H)
Spectrométrie de Masse : méthode
Temps de rétention Tr (min) = 0,79 ;
[M+H]+ : m/z 413 ; [M-H]- : m/z 411

### Exemple 6b : Synthèse du 2-[2-(4-fluoro-2,3-dihydro-indol-1-yl)-2-oxo-éthyl]-6-(2-fluorométhyl-morpholin-4-yl)-3H-pyrimidin-4-one

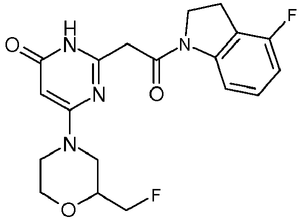

Le produit est préparé en suivant le mode opératoire décrit à l'étape 3b de l'exemple 1b mais à partir de 670 mg du sel de sodium de l'acide [4-(2-fluorométhylmorpholin-4-yl)-6-oxo-1,6-dihydro-pyrimidin-2-yl]-acétique et de 345 mg de 4-fluoro-2,3-dihydro-1H-indole. On obtient 161 mg de 2-[2-(4-fluoro-2,3-dihydro-indol-1-yl)-2-oxoéthyl]-6-(2-fluorométhyl-morpholin-4-yl)-3H-pyrimidin-4-one sous forme d'un solide blanc dont les caractéristiques sont les suivantes :
Spectre RMN 1H (400 MHz) : 2,70 (dd, J=10,6 et 12,8 Hz, 1 H) ; 2,87 (m, 1 H) ; 3,20 (t, J=8,4 Hz, 2 H) ; 3,50 (m, 1 H) ; 3,67 (m, 1 H) ; 3,77 (s, 2 H) ; 3,90 (dd, J=3,5 et 10,6 Hz, 1 H) ; 3,96 (m, 1 H) ; 4,09 (m, 1 H) ; 4,21 (t, J=8,4 Hz, 2 H) ; 4,43 (dm, J=47,5 Hz, 2 H) ; 5,25 (s, 1 H) ; 6,86 (t, J=8,7 Hz, 1 H) ; 7,22 (m, 1 H) ; 7,84 (d, J=8,1 Hz, 1 H) ; 11,63 (m étalé, 1 H)
Spectrométrie de Masse : méthode A
Temps de rétention Tr (min) = 0,74 ;
[M+H]+ : m/z 391 ; [M-H]- : m/z 389

### Exemple 7b : Synthèse du (+)-2-[2-(4-fluoro-2,3-dihydro-indol-1-yl)-2-oxo-éthyl]-6-(2-fluorométhyl-morpholin-4-yl)-3H-pyrimidin-4-one

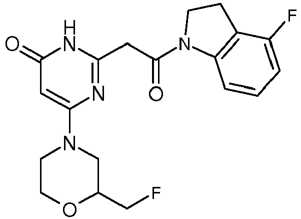

La séparation des deux énantiomères de la 2-[2-(4-fluoro-2,3-dihydro-indol-1-yl)-2-oxo-éthyl]-6-(2-fluorométhyl-morpholin-4-yl)-3H-pyrimidin-4-one (157 mg) a été réalisée par chromatographie chirale en mode SFC: Phase stationnaire : Chiralpak AS-V 20µm; phase mobile : CO2 (80%) / MeOH (20%) / TEA (0.1%); débit : 300 mL/mn.

L'énantiomère lévogyre est concentré pour obtenir 77 mg de la (-)-2-[2-(4-fluoro-2,3-dihydro-indol-1-yl)-2-oxo-éthyl]-6-(2-fluorométhyl-morpholin-4-yl)-3H-pyrimidin-4-one sous forme d'un solide blanc dont les caractéristiques sont les suivantes :
Spectre RMN 1H (400 MHz) : 2,69 (dd, J=10,6 et 12,8 Hz, 1 H) ; 2,85 (m, 1 H) ; 3,20 (t, J=8,7 Hz, 2 H) ; 3,50 (m, 1 H) ; 3,64 (m, 1 H) ; 3,77 (s, 2 H) ; 3,90 (dd, J=3,5 et 10,6 Hz, 1 H) ; 3,97 (m, 1 H) ; 4,09 (m, 1 H) ; 4,21 (t, J=8,7 Hz, 2 H) ; 4,43 (dm, J=47,5 Hz, 2 H) ; 5,25 (s, 1 H) ; 6,86 (t, J=8,7 Hz, 1 H) ; 7,22 (m, 1 H) ; 7,84 (d, J=8,1 Hz, 1 H) ; 11,65 (m étalé, 1 H)
Spectrométrie de Masse : méthode A
Temps de rétention Tr (min) = 0,77 ; [M+H]+ : m/z 391 ; [M-H]- : m/z 389

L'énantiomère dextrogyre est concentré pour obtenir 97 mg de la (+)-N-(4-fluorophényl)-2-[4-(2-fluorométhyl-morpholin-4-yl)-6-oxo-1,6-dihydro-pyrimidin-2-yl]-acétamide sous forme d'un solide blanc dont les caractéristiques sont les suivantes :
Spectre RMN 1 H (400 MHz) : 2,69 (m, 1 H) ; 2,86 (m, 1 H) ; 3,19 (t, J=8,4 Hz, 2H) ; 3,51 (m, 1 H) ; 3,65 (m, 1 H) ; 3,77 (s, 2 H) ; 3,90 (dd, J=3,5 et 10,6 Hz, 1 H) ; 3,96 (m, 1 H) ; 4,09 (m, 1 H) ; 4,21 (t, J=8,4 Hz, 2 H) ; 4,43 (dm, =47,5 Hz, 2 H) ; 5,25 (s, 1 H) ; 6,86 (t, J=8,7 Hz, 1 H) ; 7,21 (m, 1 H) ; 7,84 (d, J=8,1 Hz, 1 H) ; 11,65 (m étalé, 1 H)
Spectrométrie de Masse : méthode A
Temps de rétention Tr (min) = 0,77 ;
[M+H]+ : m/z 391 ; [M-H]- : m/z 389

### Exemple 8b : Synthèse du 2-[2-(4-fluoro-2,3-dihydro-indol-1-yl)-2-oxo-éthyl]-6-(2-méthyl-morpholin-4-yl)-3H-pyrimidin-4-one

### Étape 1b : Synthèse du 2,4-Dichloro-6-méthoxy-pyrimidine

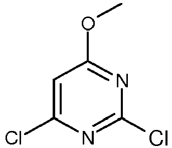

A une solution de 11 g du 2,4,6-trichloropyrimidine dans 140 mL de méthanol refroidit à 0°C dans un bain de glace, sont ajoutés, goutte à goutte, 3.24 g de méthylate de sodium préalablement dissous dans 13 mL de méthanol. Le bain de glace est retiré. Le milieu réactionnel est agité à 0°C pendant 45 minutes, puis le bain refroidissant est enlevé pour laisser la température monter à l'ambiante. Le milieu réactionnel est concentré sous pression réduite. Le résidu obtenu est repris avec 30 mL d'eau et 100 mL d'acétate d'éthyle. Après décantation, la phase organique est séchée sur sulfate de magnésium, filtrée, concentrée sous pression réduite pour donner une huile qui est laissée cristalliser pendant 24 heures à la température ambiante. Le produit cristallise sous forme d'aiguilles au milieu d'une huile. Les aiguilles sont séparées pour donner 3.94 g du 2,4-dichloro-6-méthoxy-pyrimidine dont les caractéristiques sont les suivantes :
Spectrométrie de Masse : méthode A
El : [M]+. m/z = 178 ; pic de base : m/z = 148

### Étape 2b : Synthèse de l'ester éthylique de l'acide (4-Chloro-6-méthoxypyrimidin-2-yl)-acétique

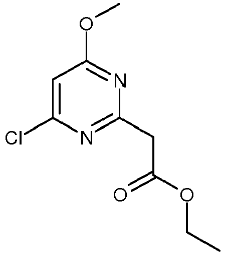

A une solution de 7.4 g du 2,4-dichloro-6-méthoxy-pyrimidine et 4.5 mL d'acétate d'éthyle dans 100 mL de THF anhydre refroidit à -75°C dans un bain de carboglace / acétone, sont additionnés, goutte à goutte, 91.4 mL de lithium bis(triméthylsilyl)amide 1 M (THF).

Le milieu réactionnel est agité à -75°C pendant une heure.

Le bain refroidissant est enlevé pour laisser la température monter à 22°C. Le milieu réactionnel est agité à 22°C pendant une heure.

Addition de 100 mL d'eau et 400 mL d'acétate d'éthyle. Après décantation, la phase organique est séchée sur sulfate de magnésium, filtrée puis concentrée sous pression réduite pour donner 9.5 g de (4-chloro-6-méthoxy-pyrimidin-2-yl)-acetic acid éthyl ester sous forme d'une huile orange dont les caractéristiques sont les suivantes :
Spectrométrie de Masse : méthode A
Temps de rétention Tr (min) = 0,80 ;
[M+H]+ : m/z 231 ;

### Étape 3b : Synthèse du sel de sodium de l'acide (4-méthoxy-6-chloro-pyrimidin-2-yl)-acétique

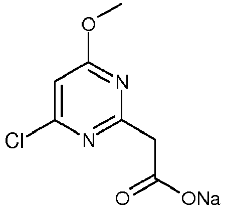

A une solution de 5.58 g du (4-chloro-6-méthoxy-pyrimidin-2-yl)-acetic acid éthyl ester dans 56 mL de THF est ajouté 12.1 mL de soude 2N. Le milieu réactionnel est agité à la température ambiante pendant 48 heures. Le milieu réactionnel est concentré sous pression réduite. Le résidu obtenu est séché à l'étuve sous vide en présence de P2O5 pour donner 5.4 g du sel de sodium de l'acide (4-méthoxy-6-chloro-pyrimidin-2-yl)-acétique qui sera utilisé tel quel dans l'étape suivante.

### Étape 4b: Synthèse du 1-(4-Fluoro-2,3-dihydro-indol-1-yl)-2-(4-méthoxy-6-chloropyrimidin-2-yl)-éthanone

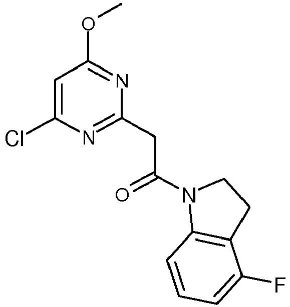

A une solution de 5.4 g du sel de sodium de l'acide (4-méthoxy-6-chloropyrimidin-2-yl)-acétique dans 50 mL de DMF et 4.3 mL de pyridine sont ajoutés 3.32 g de 4-fluoro-2,3-dihydro-1H-indole et 5.56 g de chlorhydrate de N-[3-(diméthylamino)propyl]-N'-éthylcarbodiimide. Le milieu réactionnel est agité à la température ambiante pendant une heure. Addition de 200 mL d'acétate d'éthyle, 100 mL d'eau et de l'acide chlorhydrique 1 N jusqu'au PH=5-6. Après décantation, la phase organique est séchée sur sulfate de magnésium, filtrée puis concentrée sous pression réduite. L'huile obtenue est reprise à l'éther éthylique. Le solide formé est filtré pour donner 2.2 g de 1-(4-fluoro-2,3-dihydro-indol-1-yl)-2-(4-méthoxy-6-chloropyrimidin-2-yl)-éthanone sous forme d'un solide orangé dont les caractéristiques sont les suivantes :
Spectrométrie de Masse : méthode A
Temps de rétention Tr (min) = 0,97 ;
[M+H]+ : m/z 322 ; [M-H]- : m/z 320

### Étape 5b: Synthèse du 2-[2-(4-Fluoro-2,3-dihydro-indol-1-yl)-2-oxo-éthyl]-6-chloro-3H-pyrimidin-4-one

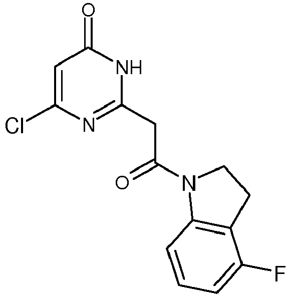

Dans un tube micro-onde, sont ajoutés, 2 g du 1-(4-fluoro-2,3-dihydro-indol-1-yl)-2-(4-méthoxy-6-chloropyrimidin-2-yl)-éthanone avec 30 mL d'acétonitrile. Addition de 3.1 g de KI et de 2.4 mL de triméthylchlorosilane. Après irradiation sous micro-ondes pendant une heure à une température de 100°C, le milieu réactionnel est dilué avec 100 mL d'acétate d'éthyle et 20 mL d'eau. Après décantation, la phase organique est séchée sur sulfate de magnésium, filtrée puis concentrée sous pression réduite. Le résidu obtenu est purifié sur colonne de silice, éluant : dichlorométhane / méthanol 95/05 pour donner 1.13 du 2-[2-(4-fluoro-2,3-dihydro-indol-1-yl)-2-oxo-éthyl]-6-chloro-3H-pyrimidin-4-one sous forme d'un solide blanc.
Spectrométrie de Masse : méthode A
Temps de rétention Tr (min) = 0,73 ;
[M+H]+ : m/z 308 ; [M-H]- : m/z 306

### Étape 6b: Synthèse du 2-[2-(4-Fluoro-2,3-dihydro-indol-1-yl)-2-oxo-éthyl]-6-(2-méthyl-morpholin-4-yl)-3H-pyrimidin-4-one

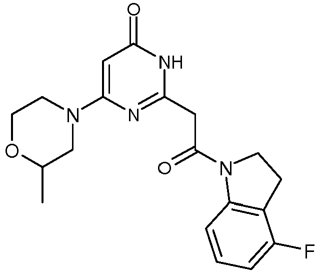

La solution de 200 mg du 2-[2-(4-fluoro-2,3-dihydro-indol-1-yl)-2-oxo-éthyl]-6-chloro-3H-pyrimidin-4-one dans 2 mL de 2-méthylmorpholine est chauffée à 100°C pendant 15 minutes. Le milieu réactionnel est repris avec 10 mL d'eau. Le solide formé est filtré et lavé avec de l'eau puis séché pour donner 216 mg de 2-[2-(4-fluoro-2,3-dihydro-indol-1-yl)-2-oxo-éthyl]-6-(2-méthyl-morpholin-4-yl)-3H-pyrimidin-4-one sous forme d'un solide beige dont les caractéristiques sont les suivantes :
Spectre RMN 1H (400 MHz) : 1,06 (d, J=6,1 Hz, 3 H) ; 2,47 (m partiellement masqué, 1 H) ; 2,80 (m, 1 H) ; 3,19 (t, J=8,4 Hz, 2 H) ; 3,39 à 3,50 (m, 2 H) ; 3,76 (s, 2 H) ; 3,81 (m, 1 H) ; 3,93 (m, 1 H) ; 4,04 (m, 1 H) ; 4,21 (t, J=8,4 Hz, 2 H) ; 5,22 (s, 1 H) ; 6,86 (t, J=8,6 Hz, 1 H) ; 7,22 (m, 1 H) ; 7,84 (d, J=8,3 Hz, 1 H) ; 11,60 (m étalé, 1 H)
Spectrométrie de Masse : méthode A
Temps de rétention Tr (min) = 0,74 ;
[M+H]+ : m/z 373 ; [M-H]- : m/z 371

### Exemple 9b : Synthèse du (-)-2-[2-(4-fluoro-2,3-dihydro-indol-1-yl)-2-oxo-éthyl]-6-(2-méthyl-morpholin-4-yl)-3H-pyrimidin-4-one Et Exemple 10b : Synthèse du (+)-2-[2-(4-fluoro-2,3-dihydro-indol-1-yl)-2-oxo-éthyl]-6-(-2-méthyl-morpholin-4-yl)-3H-pyrimidin-4-one

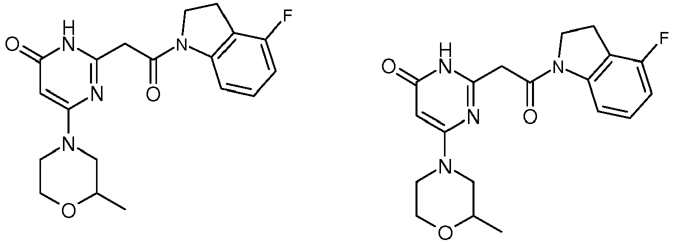

La séparation des deux énantiomères de la 2-[2-(4-fluoro-2,3-dihydro-indol-1-yl)-2-oxo-éthyl]-6-(2-méthyl-morpholin-4-yl)-3H-pyrimidin-4-one (211 mg) a été réalisée par chromatographie chirale :
Phase stationnaire : Chiralpak AS-V 20µm; phase mobile : heptane (50%) / EtOH (50%) / TEA (0.1%) ; débit : 300 mL/mn.

L'énantiomère lévogyre est concentré pour obtenir 100 mg de la (-)-2-[2-(4-fluoro-2,3-dihydro-indol-1-yl)-2-oxo-éthyl]-6-(2-méthyl-morpholin-4-yl)-3H-pyrimidin-4-one sous forme d'un solide blanc dont les caractéristiques sont les suivantes :
Spectre RMN 1 H (400 MHz) : 1,06 (d, J=6,3 Hz, 3 H) ; 2,48 (m partiellementmasqué, 1 H) ; 2,80 (m, 1 H) ; 3,20 (t, J=8,7 Hz, 2 H) ; 3,37 à 3,51 (m, 2 H) ; 3,77 (s, 2 H) ; 3,82 (m, 1 H) ; 3,94 (m, 1 H) ; 4,05 (m, 1 H) ; 4,21 (t, J=8,6 Hz, 2 H) ; 5,23 (s, 1 H) ; 6,87 (t, J=8,8 Hz, 1 H) ; 7,23 (m, 1H) ; 7,84 (d, J=8,1 Hz, 1 H) ; 11,64 (m étalé, 1 H)
Spectrométrie de Masse : méthode A
Temps de rétention Tr (min) = 0,75 ;
[M+H]+ : m/z 373 ; [M-H]- : m/z 371
Pouvoir rotatoire : α_{D} =-10 C=0.477mg/0.5ml DMSO

L'énantiomère dextrogyre est concentré pour obtenir 85 mg de la (+)-N-(4-fluorophényl)-2-[4-(2-méthyl-morpholin-4-yl)-6-oxo-1,6-dihydro-pyrimidin-2-yl]-acétamide sous forme d'un solide blanc dont les caractéristiques sont les suivantes :
Spectre RMN 1H (400 MHz) : 1,06 (d, J=6,3 Hz, 3 H) ; 2,47 (m, 1 H) ; 2,80 (m, 1 H) ; 3,19 (t, J=8,7 Hz, 2 H) ; 3,39 à 3,49 (m, 2 H) ; 3,77 (s, 2 H) ; 3,82 (m, 1 H) ; 3,94 (m, 1 H) ; 4,05 (m, 1 H) ; 4,21 (t, J=8,7 Hz, 2 H) ; 5,23 (s, 1 H) ; 6,87 (t, J=8,8 Hz, 1 H) ; 7,22 (m, 1 H) ; 7,84 (d, J=8,1 Hz, 1 H); 11,64 (m étalé, 1 H)
Spectrométrie de Masse : méthode A
Temps de rétention Tr (min) = 0,74 ;
[M-H]- : m/z 371
Pouvoir rotatoire : α_{D} =+15 ds le DMSO à 589nm C=0.388mg/0.5ml

### Exemple 11 b : Synthèse du 2-[2-(4-Fluoro-2,3-dihydro-indol-1-yl)-2-oxoéthyl]-6-(hexahydro-cyclopenta[1,4]oxazin-4-yl)-3H-pyrimidin-4-one

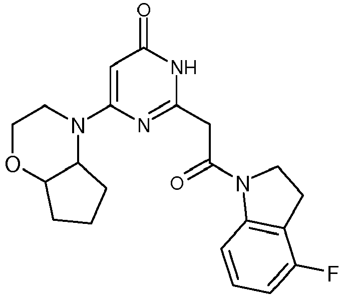

Sur une solution de 100 mg du 2-[2-(4-fluoro-2,3-dihydro-indol-1-yl)-2-oxo-éthyl]-6-chloro-3H-pyrimidin-4-one dans 5 mL de diisopropyléthylamine sont ajoutés 532 mg du chlorhydrate de hexahydro-cyclopenta[1,4]oxazine. Le milieu réactionnel est chauffé à 100°C pendant 2 heures. Le milieu réactionnel est repris avec 10 mL d'eau. Le solide formé est filtré et lavé avec de l'eau puis séché pour donner 30 mg de 2-[2-(4-fluoro-2,3-dihydro-indol-1-yl)-2-oxo-éthyl]-6-(hexahydro-cyclopenta[1,4]oxazin-4-yl)-3H-pyrimidin-4-one sous forme d'un solide beige dont les caractéristiques sont les suivantes :
Spectre RMN 1 H (400 MHz) : 1,29 à 1,55 (m, 4 H) ; 1,75 (m, 1 H) ; 2,45 (m, 1 H) ; 2,78 (m, 1 H) ; 2,88 (m, 1 H) ; 3,19 (t, J=8,1 Hz, 2 H) ; 3,37 (m, 1 H) ; 3,62 (dt, J=3,4 et 11,5 Hz, 1 H) ; 3,76 (m, 3 H) ; 3,92 (td, J=3,4 et 11,5 Hz, 1 H) ; 4,19 (m, 2 H) ; 5,30 (s, 1 H) ; 6,86 (t, J=8,7 Hz, 1 H) ; 7,22(m, 1 H) ; 7,85 (d, J=8,1 Hz, 1 H) ; 11,77 (m étalé, 1 H)
Spectrométrie de Masse : méthode A
Temps de rétention Tr (min) = 0,79 ;
[M+H]+ : m/z 399 ; [M-H]- : m/z 397

### Exemple 12b : Synthèse du 2-[2-(4-fluoro-2,3-dihydro-indol-1-yl)-2-oxo-éthyl]-6-pyridin-4-yl-3H-pyrimidin-4-one

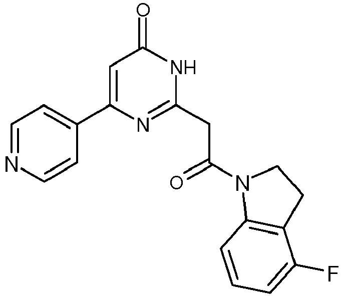

Dans un tube micro-onde, le mélange de 59 mg du 2-[2-(4-fluoro-2,3-dihydro-indol-1-yl)-2-oxo-éthyl]-6-chloro-3H-pyrimidin-4-one, 88 mg du pyridine-4-boronic acid pinacol ester, 32 mg du tetrakis(triphénylphosphine)palladium et 0.26 ml d'une solution de carbonate de césium 1.5 M dans 1.25 mL de dioxane est irradié au micro-ondes pendant une heure et demie à 100°C. Le milieu réactionnel est repris avec un mélange de méthanol , acétate d'éthyle et quelques gouttes d'eau. Le solide formé est filtré et lavé avec de l'eau puis séché pour donner 20 mg de 2-[2-(4-fluoro-2,3-dihydro-indol-1-yl)-2-oxo-éthyl]-6-pyridin-4-yl-3H-pyrimidin-4-one sous forme d'un solide beige dont les caractéristiques sont les suivantes :
Spectre RMN 1 H (400 MHz) : 3,23 (t, J=8,5 Hz, 2 H) ; 3,99 (s, 2 H) ; 4,28 (t, J=8,5Hz, 2 H) ; 6,88 (t, J=8,9 Hz, 1 H) ; 7,01 (s, 1 H) ; 7,22 (m, 1 H) ; 7,85 (d, J=8,1 Hz, 1 H) ; 7,96 (d, J=6,0Hz, 2 H) ; 8,69 (d, J=6,0 Hz, 2 H) ; 12,69 (m étalé, 1 H)
Spectrométrie de Masse : méthode A
Temps de rétention Tr (min) = 0,52 ;
[M+H]+ : m/z 351 ; [M-H]- : m/z 349

### Exemple 13b : Synthèse du 2-[2-(4-Fluoro-2,3-dihydro-indol-1-yl)-2-oxo-éthyl]-6-(2-méthoxy-pyridin-4-yl)-3H-pyrimidin-4-one

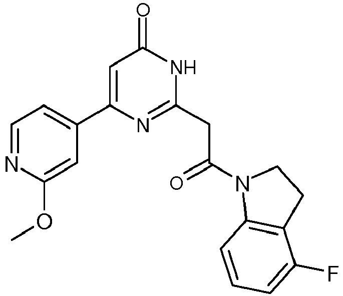

Le produit est préparé en suivant le mode opératoire décrit de l'exemple 12b à partir de 100 mg du 2-[2-(4-fluoro-2,3-dihydro-indol-1-yl)-2-oxo-éthyl]-6-chloro-3H-pyrimidin-4-one et 160 mg du 2-méthoxypyridine-4-boronic acid pinacol ester. On obtient 75 mg du 2-[2-(4-Fluoro-2,3-dihydro-indol-1-yl)-2-oxo-éthyl]-6-(2-méthoxy-pyridin-4-yl)-3H-pyrimidin-4-one sous forme d'un solide beige dont les caractéristiques sont les suivantes :
Spectre RMN 1 H (400 MHz) : 3,23 (t, J=8,6 Hz, 2 H) ; 3,88 (s, 3 H) ; 3,98 (s, 2 H); 4,27 (t, J=8,6 Hz, 2 H) ; 6,88 (t, J=8,6 Hz, 1 H) ; 7,00 (s, 1 H) ; 7,23 (m, 1 H) ; 7,39 (s large, 1 H) ; 7,56 (d large, J=5,4 Hz, 1 H) ; 7,85 (d, J=8,3 Hz, 1 H) ; 8,26 (d, J=5,4 Hz, 1 H) ; 12,66 (m étalé, 1 H)
Spectrométrie de Masse : méthode A
Temps de rétention Tr (min) = 0,82 ;
[M+H]+ : m/z 381 ; [M-H]- : m/z 379

### Exemple 14b :Synthèse du (±)-2-[4-(2-éthylmorpholin-4-yl)-6-oxo-1,6-dihydropyrimidin-2-yl]-N-(4-fluorophényl)acétamide

### Étape 1b : Synthèse de l'ester éthylique de l'acide [4-(2-éthylmorpholin-4-yl)-6-oxo-1,6-dihydropyrimidin-2-yl]acétique

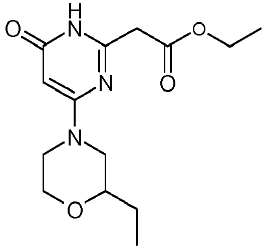

Dans un tube micro-ondes, on introduit 0.45 g de 2-éthylmorpholine dans 10 mL d'éthanol, 2,11 mL de N,N-diisopropyléthylamine et 2,29 g de chlorhydrate de 3-éthoxy-3-iminopropanoate d'éthyle. Le tube est alors chauffé sous micro-ondes à 130°C pendant 1 heure puis laissé revenir à température ambiante. Le mélange réactionnel est concentré sous pression réduite. Après purification par chromatographie sur colonne de silice, éluant : dichlorométhane/méthanol 90/05 on obtient 550 mg de l'ester éthylique de l'acide [4-(2-éthylmorpholin-4-yl)-6-oxo-1,6-dihydropyrimidin-2-yl]acétique sous forme d'une poudre beige dont les caractéristiques sont les suivantes:
Spectrométrie de Masse : méthode A,
Temps de rétention Tr (min) = 0,66 ;
[M+H]+ : m/z 296 ; [M-H]- : m/z 294

### Étape 2b: Synthèse du sel de sodium de l'acide [4-(2-éthylmorpholin-4-yl)-6-oxo-1,6-dihydropyrimidin-2-yl]acétique

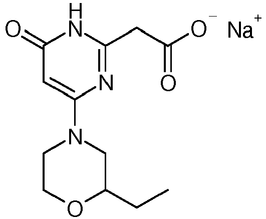

A une solution de 520 mg de l'ester éthylique de l'acide [4-(2-éthylmorpholin-4-yl)-6-oxo-1,6-dihydropyrimidin-2-yl]acétique dans 14 mL de tétrahydrofuranne, sont ajoutés 0,93 mL de soude 2M. Le mélange réactionnel est agité pendant 48 heures à température ambiante puis évaporé sous vide. On obtient 420 mg du sel de sodium de l'acide [4-(2-éthylmorpholin-4-yl)-6-oxo-1,6-dihydropyrimidin-2-yl]acétique sous forme d'une poudre jaune qui est utilisée telle quelle pour l'étape suivante.

### Étape 3b : (±)-2-[4-(2-éthylmorpholin-4-yl)-6-oxo-1,6-dihydropyrimidin-2-yl]-N-(4-fluorophényl) acétamide

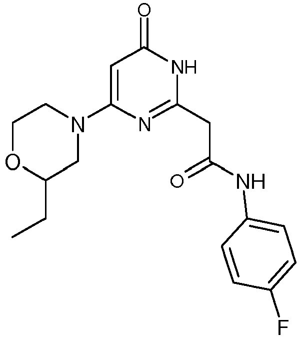

A une solution de 410 mg du sel de sodium de l'acide [4-(2-éthylmorpholin-4-yl)-6-oxo-1,6-dihydropyrimidin-2-yl]acétique dans 4 mL de N,N-diméthylformamide sont successivement ajoutés 4 mL de pyridine, 472 mg de 4-fluoroaniline et 815 mg de Chlorhydrate de N-[3-(diméthylamino)propyl]-N'-éthylcarbodiimide. Le mélange réactionnel est agité à température ambiante pendant 16 heures, puis concentré sous pression réduite. Le résidu est repris dans 25 mL d'eau puis le pH est ramené autour de 7 avec une solution d'acide chlorhydrique 2M. Ajouter 30 mL d'acétate d'éthyle, puis laisser agiter à température ambiante pendant 1 heure. Le précipité formé est filtré, puis rincé successivement à l'eau et l'éther diéthylique. On obtient ainsi 300 mg de (±)-2-[4-(2-éthylmorpholin-4-yl)-6-oxo-1,6-dihydropyrimidin-2-yl]-N-(4-fluorophényl)acétamide sous forme d'une poudre blanc-cassé dont les caractéristiques sont les suivantes:
Spectrométrie de Masse : méthode C*
Temps de rétention Tr (min) = 3,37;
[M+H]+ : m/z 361 ; [M-H]- : m/z 359

C* = ZQ XBridge C18 2.5 µm 3 x 50 mm 900µl/min 5 to 100 % B(CH3CN) with 0.1%HCO2H in 5 min

### Exemple 15b (+)-2-[4-(2-éthylmorpholin-4-yl)-6-oxo-1,6-dihydropyrimidin-2-yl]-N-(4-fluorophényl)acétamide

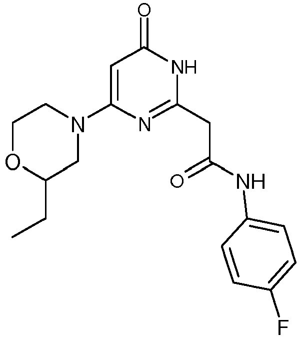

Le (±)-2-[4-(2-éthylmorpholin-4-yl)-6-oxo-1,6-dihydropyrimidin-2-yl]-N-(4-fluorophényl)acétamide (example 14, étape 3, 268 mg) est résolu en ses deux énantiomères par chromatographique chirale sur phase Chiralpak AS-V (20 µm, 6x35 cm), éluant : heptane/éthanol : 60/40 ; débit : 150 mL/min. Après élution, on rassemble les fractions contenant le deuxième énantiomère que l'on évapore sous pression réduite.

On isole l'énantiomère, dextrogyre, 93.6 mg : (+)-2-[4-(2-éthylmorpholin-4-yl)-6-oxo-1,6-dihydropyrimidin-2-yl]-N-(4-fluorophényl)acétamide sous forme d'une poudre blanche dont les caractéristiques sont les suivantes :
Spectrométrie de Masse : méthode A
Temps de rétention Tr (min) = 0,74 ;
[M+H]+ : m/z 361 ; [M-H]- : m/z 359
Pouvoir rotatoire : α_{D} = +12° (c = 1,330 mg dans 1 mL de méthanol, 589nm)

### Exemple 16b : Synthèse du 2-[2-(4-fluoro-2,3-dihydro-indol-1-yl)-2-oxo-éthyl]-6-(2,2,3,3,5,5,6,6-d8-morpholin)-4-yl-3h-pyrimidin-4-one

### Étape 1b : Synthèse de l'ester éthylique de l'acide (4-chloro-6-oxo-1,6-dihydropyrimidin-2-yl)-acétique

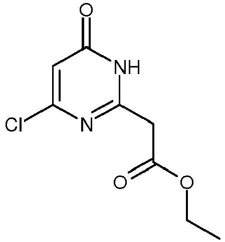

Dans un autoclave de 1000ml, on introduit 33 g de l'ester éthylique de l'acide (4-Chloro-6-méthoxy-pyrimidin-2-yl)-acétique, (exemple 8b étape 2b), 750 ml d'acétonitrile puis on ajoute 71.2 g d'iodure de potassium et 55.85 ml de triméthylchlorosilane; on obtient une solution hétérogène orangée que l'on agite en chauffant sous une pression de 10 bars d'argon à 100°C pendant 2 heures. Le milieu réactionnel est soutiré puis on filtre l'insoluble, lave avec 3 fois environ 100 ml d'AcOEt, concentre le filtrat jusqu'à obtention d'un résidu pâteux que l'on reprends dans 500 ml d'eau. Apres agitation, extraction par 3 fois environ 350 ml d'acétate d'éthyle, on lave les extraits organiques réunis avec 500 ml de solution saturée en NaCl, sèche sur MgSO4, filtre sur VF, concentre sous vide. Le composé obtenu est purifié par chromatographie sur gel de silice (40-63µM) en éluant avec un mélange de (2/8 v/v) AcOEt/n-Heptane. Les fractions contenant le composé attendu sont réunies et évaporées. On isole un solide que l'on triture dans l'oxyde de diisopropyl, filtre sur verre fritté et sèche. L' ester éthylique de l'acide (4-Chloro-6-oxo-1,6-dihydro-pyrimidin-2-yl)-acétique est isolé sous forme d'un solide beige. 25.2 g ; Rdt 81%
LCMS ES+ DMSO Tr 0.71 min ;
[MH+] m/z=217

### Étape 2b : Synthèse de l'ester éthylique de l'acide (4-(2,2,3,3,5,5,6,6,-D8)-Morpholin-4-yl-6-oxo-1,6-dihydro-pyrimidin-2-yl)-acétique

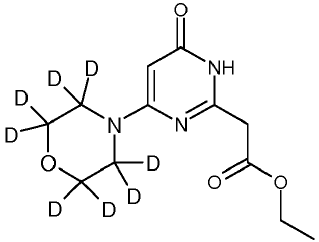

Dans le tricol on place 1.8g de l'ester éthylique de l'acide (4-Chloro-6-oxo-1,6-dihydro-pyrimidin-2-yl)-acétique 1 g de morpholine D8 (CAS, 342611-02-3), dans 35 ml de dioxane; On ajoute 1,4 ml de TEA à la température ambiante, agite en chauffant à 85°C pendant 20 heures. On concentre sous vide et reprend le résidu dans 200 ml de CH2Cl2, lave avec 100 ml de solution saturée en NaCl, sèche sur MgSO4, filtre sur VF, concentre sous vide. Le solide obtenu est trituré dans 25 ml d'eau en présence de 1 ml d'acétate d'éthyle, filtré sur VF, rincé à l'oxyde de diisopropyle et séché à l'air. On isole l'ester éthylique de l'acide (4-(2,2,3,3,5,5,6,6,-D8)-Morpholin-4-yl-6-oxo-1,6-dihydro-pyrimidin-2-yl)-acétique 1.7g Rdt 74%, que l'on utilise tel quel dans l'étape suivante.

### Étape 3b : Synthèse du sel de sodium de l'acide (4-(2,2,3,3,5,5,6,6-D8-Morpholin)-4-yl-6-oxo-1,6-dihydro-pyrimidin-2-yl)-acétique

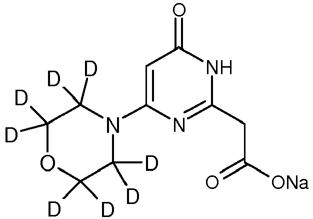

Dans un ballon on place 2.75g de l'ester éthylique de l'acide (4-(2,2,3,3,5,5,6,6,-D8)-Morpholin-4-yl-6-oxo-1,6-dihydro-pyrimidin-2-yl)-acétique dans 80ml de THF puis on ajoute 5 ml de soude 2M. Le milieu réactionnel est agité à température ambiante (20°C) pendant 6 jours. Le solide formé est filtré, le filtrat évaporé sous pression réduite. Les deux solides ainsi obtenus sont réunis et lavés à l'éther de pétrole, séchés sous vide. On isole le sel de sodium de l'acide (4-(2,2,3,3,5,5,6,6-D8-Morpholin)-4-yl-6-oxo-1,6-dihydro-pyrimidin-2-yl)-acétique. 1.45g, que l'on utilise tel quel dans l'étape suivante. (LCMS ES+ Temps de rétention Tr (min) = 2.08 ; [M+H]+ : m/z 248 (acide correrspondant))

### Étape 4b : Synthèse du 2-[2-(4-fluoro-2,3-dihydro-indol-1-yl)-2-oxo-éthyl]-6-(2,2,3,3,5,5,6,6-d8-morpholin)-4-yl-3h-pyrimidin-4-one

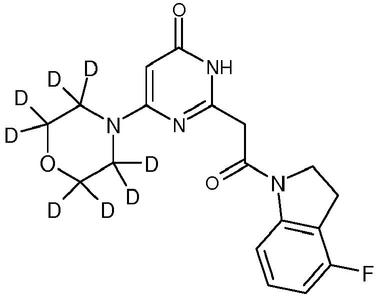

Dans un ballon on place 300mg de sel de sodium de l'acide (4-(2,2,3,3,5,5,6,6-d8-Morpholin)-4-yl-6-oxo-1,6-dihydro-pyrimidin-2-yl)-acétique en suspension dans 3ml de diméthyl formamide et 2ml de pyridine, puis on ajoute 277mg de chlorhydrate de N-[3-(diméthylamino)propyl]-N'-éthylcarbodiimide et 168 mg de 4-fluoro-2,3-dihydro-1H-indole. Le mélange réactionnel est agité à température ambiante (20°C) pendant une nuit puis évaporé aous pression réduite. Le résidu est repris dans 40ml d'eau et 5 ml d'acétate d'éthyle et agité pendant 10 minutes. Le solide formé est filtré, essoré et séche sous pression réduite à température ambiante (20°C). On isole 250mg de 2-[2-(4-fluoro-2,3-dihydro-indol-1-yl)-2-oxo-éthyl]-6-(2,2,3,3,5,5,6,6-d8-morpholin)-4-yl-3h-pyrimidin-4-one dont les caractéristiques sont les suivantes :
Spectre RMN 1 H (400 MHz) : 3,20 (t, J=8,7 Hz, 2 H) ; 3,76 (s large, 2 H) ; 4,21 (t,J=8,7 Hz, 2 H) ; 5,20 (s, 1 H) ; 6,86 (t, J=8,6 Hz, 1 H) ; 7,22 (m, 1 H) ; 7,84 (d, J=8,6 Hz, 1 H) ; 11,60 (métalé, 1 H)

### Exemple 17b: Synthèse du 2-[2-(4-Fluoro-2,3-dihydro-indol-1-yl)-2-oxo-éthyl]-6-(3,3,5,5-D4-morpholin)-4-yl-3H-pyrimidin-4-one

### Étape 1b : Synthèse du 2-(4-chloro-6-méthoxy-pyrimidin-2-yl)-1-(4-fluoro-2,3-dihydro-indol-1-yl)-éthanone

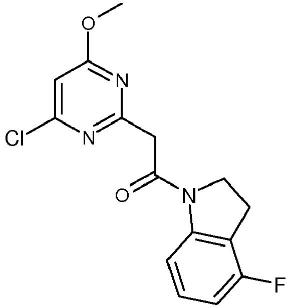

Dans un ballon de 100 ml contenant 5.44g du sel de sodium de l'acide (4-méthoxy-6-chloro-pyrimidin-2-yl)-acétique, (example 8b, étape 3b) on ajoute 50 ml de diméthyl formamide, 4.3 ml pyridine et 5.6g de chlorhydrate de N-[3-(diméthylamino)propyl]-N'-éthylcarbodiimide. On agite 10 minutes à température ambiante (20°C) puis 3.3g de 4-fluoro dihydro indole sont ajoutés. On agite la nuit à température ambiante. On ajoute au milieu réactionnel de l'acétate d'éthyle et lave avec un mélange eau/HCl 2N. Extrait à l'acétate d'éthyle, lave 3 fois avec mélange eau/HCl 2N puis avec de l'eau. Les phases organiques sont réunies, séchées sur sulfate de magnésium, filtrées ét évaporées. Le solide obtenu est trituré à l'éther éthylique puis filtré et séché. On isole le 2-(4-chloro-6-méthoxy-pyrimidin-2-yl)-1-(4-fluoro-2,3-dihydro-indol-1-yl)-éthanone (2.2 g) Rdt 28 % (LCMS ES+ Temps de rétention Tr (min) = 1.35 ; [M+H]+ : m/z 322).

### Étape 2b : Synthèse du 2-(4-Chloro-6-oxo-pyrimidin-2-yl)-1-(4-fluoro-2,3-dihydroindol-1-yl)-éthanone

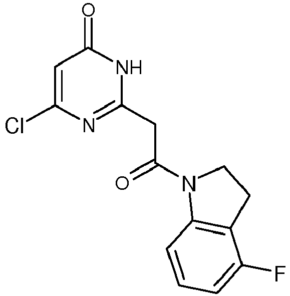

Dans un réacteur adapté au chauffage sous micro-ondes, on place 2g de 2-(4-chloro-6-méthoxy-pyrimidin-2-yl)-1-(4-fluoro-2,3-dihydro-indol-1-yl)-éthanone (exemple 17b, étape 1b), l'iodure de potassium (3.1g), 30ml d'acétonitrile et 2.03g de triméthylchlorosilane. Le réacteur est fermé et irradié 30 minutes à 100°C puis laissé la nuit à T.A. Le milieu réactionnel est dilué à l'AcOEt puis lavé deux fois à l'eau puis à la saumure. Les phases organiques sont réunies, séchées sur sulfate de magnésium, filtrées et évaporées. Le produit obtenu est chromatographié sur silice. On élue ensuite avec dichlorométhane /méthanol (de 100/0 à 95/5). Les fractions contenant l'attendu sont évaporées sous pression réduite. Le composé obtenu est trituré dans méthanol, filtré, séché. On isole 2-(4-Chloro-6-oxo-pyrimidin-2-yl)-1-(4-fluoro-2,3-dihydro-indol-1-yl)-éthanone (1.13g) Rdt = 59 %. (LCMS ES+ Temps de rétention Tr (min) = 1.04 ; [M+H]+ : m/z 308)

### Étape 3b: Synthèse du 2-[2-(4-Fluoro-2,3-dihydro-indol-1-yl)-2-oxo-éthyl]-6-(3,3,5,5-D4-morpholin)-4-yl-3H-pyrimidin-4-one

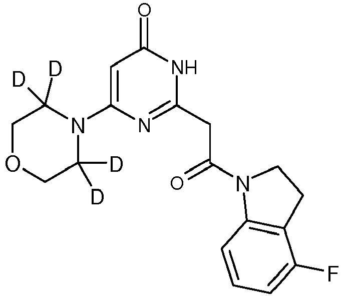

On introduit dans le tricol sous argon, 100 mg de 2-(4-Chloro-6-oxo-pyrimidin-2-yl)-1-(4-fluoro-2,3-dihydro-indol-1-yl)-éthanone, 62 mg de 3,3,5,5-D4-Morpholine (préparée selon. WO2009/23233), 2 ml de DMSO, puis 113 µl de TEA à température ambiante et on agite en chauffant à 85°C pendant 20 heures. On verse sur 20 ml de solution saturée en NaCl, extrait par 3 fois environ 20 ml de CH2Cl2, sèche sur MgSO4, filtre sur VF. Le composé obtenu est chromatographié sur gel de silice (40-63 µm) en éluant avec un mélange de dichlorométhane et de méthanol ammoniacal 7M (95/5, v/v). Les fractions contenant le composé attendu sont réunies et évaporées. Le composé obtenu est trituré dans AcOEt, filtré, séché à température ambiante (20°C). On isole 2-[2-(4-fluoro-2,3-dihydro-indol-1-yl)-2-oxo-éthyl]-6-(3,3,5,5-D4-morpholin)-4-yl-3h-pyrimidin-4-one (25mg).
Spectre RMN 1 H (400 MHz) : 3,20 (t, J=8,6 Hz, 2 H) ; 3,59 (s, 4 H) ; 3,76 (s, 2 H); 4,21 (t, J=8,6 Hz, 2 H) ; 5,20 (s, 1 H) ; 6,86 (t, J=8,7 Hz, 1 H) ; 7,22 (dt, J=6,0 et 8,7 Hz, 1 H) ; 7,84 (d,J=8,7 Hz, 1 H) ; 11,61 (s large, 1 H)
LCMS : Les spectres ont été obtenus sur un appareil Waters UPLC-SQD
lonisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;1,95 : 5 % de B

### Résultats analytiques :

Temps de rétention Tr (min) = 0,68 ;[M+H]+ : m/z 363 ; [M-H]- : m/z 361

### Exemple 18b: Synthèse du 2-[2-(3,3-Diméthyl-2,3-dihydro-indol-1-yl)-2-oxo-éthyl]-6-morpholin(D4)-4-yl-3H-pyrimidin-4-one

### Étape 1b: Synthèse du 2-(4-Chloro-6-méthoxy-pyrimidin-2-yl)-1-(3,3-diméthyl-2,3-dihydro-indol-1-yl)-éthanone

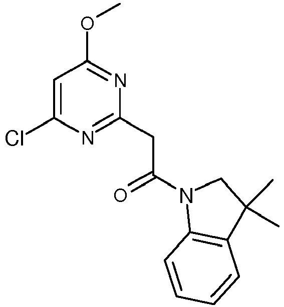

Dans un tricol on introduit 0.5 g de sel de sodium de l'acide (4-méthoxy-6-chloro-pyrimidin-2-yl)-acétique (exemple 8b, étape 3b), 0.33 g de 3,3-Diméthyl-2,3-dihydro-1H-indole (CAS 1914-02-9) 0.4 ml de pyridine et 5 ml de diméthyl formamide pour obtenir une solution homogène brune. On ajoute 512mg, le chlorhydrate de N-[3-(diméthylamino)propyl]-N'-éthylcarbodiimide à la température ambiante, agite à l'ambiante pendant 20 heures. On concentre au rotavapor sous vide, ajoute, 40 ml d'eau, extrait par 3 fois environ 25 ml de dichlorométhane, lave avec 50 ml de solution saturée en NaCl, sèche sur MgSO4, filtre sur VF. Le composé obtenu est purifié par chromatographie sur gel de silice (40-63µm) en éluant avec un mélange de dichlorométhane / méthanol 98/2. Les fractions contenant le composé attendu sont réunies et évaporées sous pression réduite. On isole, la 2-(4-Chloro-6-méthoxy-pyrimidin-2-yl)-1-(3,3-diméthyl-2,3-dihydro-indol-1-yl)-éthanone: 0.3 g d'huile jaune. Rdt 41% que l'on utilise tel quel dans l'étape suivante.

### Étape 2b: Synthèse du 6-Chloro-2-[2-(3,3-diméthyl-2,3-dihydro-indol-1-yl)-2-oxo-éthyl]-3H-pyrimidin-4-one

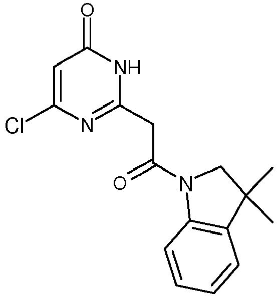

On introduit dans un réacteur adapté à l'irradiation micro onde 106 mg de 2-(4-Chloro-6-méthoxy-pyrimidin-2-yl)-1-(3,3-diméthyl-2,3-dihydro-indol-1-yl)-éthanone, 4 ml d'acétonitrile puis on ajoute 0.45 g d'iodure de potassium et 347µl de triméthylchlorosilane. La solution hétérogène orangée est agitée et irradiée à 100°C pendant 1 heure. On reprend dans 25 ml d'eau, agite, extrait par 3 fois environ 25 ml d'AcOEt, lave avec 25 ml de solution saturée en NaCl, sèche sur MgSO4, filtre sur VF et concentre sous vide. Le composé obtenu est chromatographié sur gel de silice en éluant éluant (2.5/97.5) méthanol /dichlorométhane. Les fractions contenant le composé attendu sont réunies et evaporées sous pression réduite. On isole et caractérise le 6-Chloro-2-[2-(3,3-diméthyl-2,3-dihydro-indol-1-yl)-2-oxo-éthyl]-3H-pyrimidin-4-one: 0.20 g sous forme de solide jaune. Rdt 70%. (LCMS ES+ Temps de rétention Tr (min) = 1,19 ; [M+H]+ : m/z 318)

Étape 3b : Synthèse du 2-[2-(3,3-Diméthyl-2,3-dihydro-indol-1-yl)-2-oxo-éthyl]-6-morpholin(D4)-4-yl-3H-pyrimidin-4-one

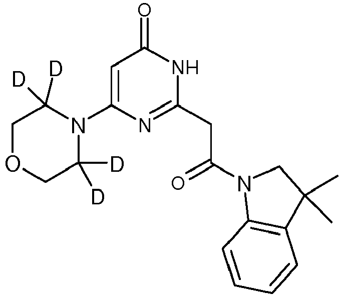

On introduit dans le tricol sous argon, 280 mg de 6-Chloro-2-[2-(3,3-diméthyl-2,3-dihydro-indol-1-yl)-2-oxo-éthyl]-3H-pyrimidin-4-one, 169 mg de 3,3,5,5-D4-Morpholine (préparée selon WO2009/23233), 5 ml de DMSO puis 307 µl de TEA à la température ambiante ; le milieu réactionnel est agité en chauffant à 85°C pendant 20 heures. On verse sur 20 ml de solution saturée en NaCl, extrait par 3 fois environ 20 ml de dichlorométhane, sèche sur MgSO4, filtre sur VF, concentre sous vide. Le composé obtenu est chromatographié sur gel de silice (40-63µm) en éluant avec un mélange 2.5/97.5 de MeOH /dichlorométhane. Les fractions contenant le composé attendu sont réunies et évaporées sous pression réduite. On isole et caractérise le 2-[2-(3,3-Diméthyl-2,3-dihydro-indol-1-yl)-2-oxo-éthyl]-6-morpholin(D4)-4-yl-3H-pyrimidin-4-one, (45 mg) sous forme d'un solide rosé Rdt 14%

### Conditions chromatographiques :

▪ colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ; 1,95:5 % de B

### Résultats analytiques :

Temps de rétention Tr (min) = 0,77 ;
[M+H]+ : m/z 373 ; [M-H]- : m/z 371
Spectre RMN 1 H (400 MHz): 1,31 (s, 6 H) ; 3,58 (s, 4 H) ; 3,75 (s, 2 H) ; 3,91 (s, 2 H) ; 5,20 (s, 1 H) ; 7,05 (t, J=7,8 Hz, 1 H) ; 7,18 (t, J=7,8 Hz, 1 H) ; 7,27 (d, J=7,8 Hz, 1 H) ; 8,00 (d, J=7,8 Hz, 1 H) ; 11,61 (s large, 1 H)

### Exemple 19b : Synthèse du (+)-2-[2-(2-méthyle-2,3-dihydro-indol-1-yl)-2-oxo-éthyl]-6-morpholin(D4)-4-yl-3H-pyrimidin-4-one et exemple 20b : (-)-2-[2-(2-méthyle-2,3-dihydro-indol-1-yl)-2-oxo-éthyl]-6-(2,2,6,6-morpholin-D4)-4-yl-3H-pyrimidin-4-one

### Étape 1b: Synthèse du 2-(4-Chloro-6-méthoxy-pyrimidin-2-yl)-1-(2-méthyl-2,3-dihydro-indol-1-yl)-éthanone

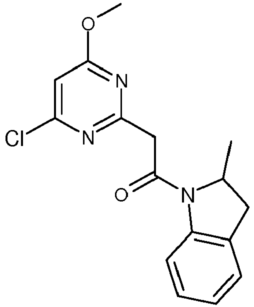

On introduit dans le tricol sous argon, 4 g de sel de sodium de l'acide (4-Chloro-6-méthoxy-pyrimidin-2-yl)-acétique (exemple 8b, étape 3b), 2.6 g de 2-Méthyl indoline (6872-06-6, Aldrich) dans 3 ml de pyridine et 60 ml de DMF. La solution hétérogène obtenue est agitée à température ambiante (20°C) puis on ajoute le chlorhydrate de N-[3-(diméthylamino)propyl]-N'-éthylcarbodiimide et maintient l'agitation pendant 20 heures. On concentre au rotavapor sous vide, ajoute, 100 ml d'eau, extrait par 3 fois environ 50 ml de dichlorométhane, lave avec 50 ml de solution saturée en NaCl, sèche sur MgSO4, filtre sur VF. Le composé obtenu est chromatographié sur gel de silice (40-63 µm) en éluant avec du dichlorométhane. Les fractions contenant le composé attendu sont réunies et évaporées. Le composé obtenu est trituré dans l'éther di-idopropylique, filtré, séché à 20°C. On isole la 2-(4-Chloro-6-méthoxy-pyrimidin-2-yl)-1-(2-méthyl-2,3-dihydro-indol-1-yl)-éthanone (2.6 g) sous forme solide (Rdt 46%) que l'on utilise tel que dans l'étape suivante.

### Étape 2b: Synthèse du 6-Chloro-2-[2-(2-méthyl-2,3-dihydro-indol-1-yl)-2-oxo-éthyl]-5H-pyrimidin-4-one

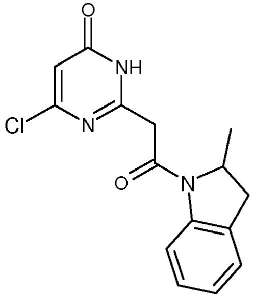

On introduit dans un réacteur adapté aux irradiations micro ondes, 1.3g de 2-(4-Chloro-6-méthoxy-pyrimidin-2-yl)-1-(2-méthyl-2,3-dihydro-indol-1-yl)-éthanone dans 20 ml d'acétonitrile ; 2 g d'iodure de potassium et 1.7ml de triméthylchlorosilane. La solution hétérogène est agitée en chauffant au micro-onde à 100°C pendant 90 minutes. On reprend dans 25 ml d'eau, agite, extrait par l'acétate d'éthyle , lave avec 25 ml de solution saturée en NaCl, sèche sur MgSO4, filtre sur VF et concentre sous vide. Le composé obtenu est chromatographié sur gel de silice (40-63 µm) en éluant avec un mélange de dichlorométhane et de méthanol (90/10, v/v). Les fractions contenant le composé attendu sont réunies et évaporées. Le composé obtenu est trituré dans un mélange d'acétate d'éthyle,d'éther di-idopropylique, filtré et séché à température ambiante (20°C). On isole le 6-Chloro-2-[2-(2-méthyl-2,3-dihydro-indol-1-yl)-2-oxo-éthyl]-5H-pyrimidin-4-one sous forme de solide beige (2.2g) Rdt 89%.

### Étape 3b : Synthèse du (±)-2-[2-(2-méthyle-2,3-dihydro-indol-1-yl)-2-oxo-éthyl]-6-(2,2,6,6-morpholin-D4)-4-yl-3H-pyrimidin-4-one

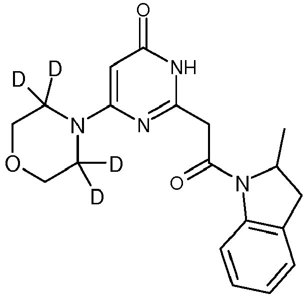

Dans un tricol n introduit, 304 mg de 6-Chloro-2-[2-(2-méthyl-2,3-dihydro-indol-1-yl)-2-oxo-éthyl]-5H-pyrimidin-4-one, 191 mg de 3,3,5,5-D4-Morpholine (préparée selon. WO2009/23233) dans 10 ml de Dioxane. On ajoute 350 µl de TEA à la température ambiante, agite en chauffant à 85°C pendant 20 heures. On concentre sous vide le mélange réactionnel, reprend dans 20 ml de dichlorométhane, ajoute 10 ml de solution saturée en NaCl, décante, sèche sur MgSO4, filtre sur VF et concentre sous vide. Le composé obtenu est chromatographié sur gel de silice (40-63 µm) en éluant avec un mélange de dichlorométhane et de méthanol (95/5, v/v). Les fractions contenant le composé attendu sont réunies et évaporées. Le composé obtenu (±)-2-[2-(2-méthyle-2,3-dihydro-indol-1-yl)-2-oxo-éthyl]-6-(2,2,6,6-morpholin-D4)-4-yl-3H-pyrimidin-4-one (0.22 g) est résolu en ses deux énantiomères dans l'étape suivante.

Le composé racémique (±)-2-[2-(2-méthyle-2,3-dihydro-indol-1-yl)-2-oxo-éthyl]-6-morpholin(D4)-4-yl-3H-pyrimidin-4-one obtenu ci-dessus est séparé en ses deux énantiomères par chromatographie chirale sur une colonne contenant 1.08 kg de phase stationaire Chiralpak AY 20µm, 7.7x35 cm, lot KLB001, en éluant avec un mélange de acétonitrile et d'isopropanol (90/10 v/v) à 200 ml/min. Les fractions contenant les énantiomères sont réunies et évaporées.

Le premier énantiomère (+)-2-[2-(2-méthyle-2,3-dihydro-indol-1-yl)-2-oxo-éthyl]-6-morpholin(D4)-4-yl-3H-pyrimidin-4-one est isolé (70.6mg) et caractérisé (+81°, c=1.422mg/0.5 ml DMSO, 589nm).
Spectrométrie de Masse : Les spectres ont été obtenus sur un appareil Waters UPLC-SQD
lonisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;
1,95 : 5 % de B

### Résultats analytiques :

Temps de rétention Tr (min) = 0,69 ;[M+H]+ : m/z 359 ; [M-H]- : m/z 357
Spectre RMN 1H (400 MHz) : 1,26 (d large, J=6,4 Hz, 3 H) ; 2,69 (d, J=16,5 Hz, 1H) ; 3,28 à 3,44 (m partiellement masqué, 1 H) ; 3,59 (s, 4 H) ; 3,72 (d, J=15,9 Hz, 1 H) ; 3,92 (d, J=15,9Hz, 1 H) ; 4,71 (m, 1 H) ; 5,20 (s, 1 H) ; 7,05 (t, J=7,8 Hz, 1 H) ; 7,18 (t, J=7,8 Hz, 1 H) ; 7,28 (d, J=7,8Hz, 1 H) ; 7,96 (d, J=7,8 Hz, 1 H) ; 11,67 (s large, 1 H)

Le deuxième énantiomère (-)-2-[2-(2-méthyle-2,3-dihydro-indol-1-yl)-2-oxo-éthyl]-6-morpholin(D4)-4-yl-3H-pyrimidin-4-one est isolé (78.4 mg) (-67.9°, c=1.609 mg/0.5ml DMSO, 589nm)

### Spectrométrie de Masse :

Les spectres ont été obtenus sur un appareil Waters UPLC-SQD
lonisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;
1,95 : 5 % de B

### Résultats analytiques :

Temps de rétention Tr (min) = 0,69 ;
[M+H]+ : m/z 359 ; [M-H]- : m/z 357
Spectre RMN 1 H (400 MHz) : 1,26 (d, J=6,4 Hz, 3 H) ; 2,69 (d, J=16,3 Hz, 1 H) ;3,25 à 3,46 (m partiellement masqué, 1 H) ; 3,59 (s, 4 H) ; 3,72 (d, J=15,9 Hz, 1 H) ; 3,92 (d, J=15,9 Hz, 1 H) ; 4,70 (m, 1 H) ; 5,20 (s, 1 H) ; 7,04 (t, J=7,9 Hz, 1 H) ; 7,18 (t, J=7,9 Hz, 1 H) ; 7,29 (d, J=7,9 Hz, 1 H); 7,96 (d, J=7,9 Hz, 1 H) ; 11,71 (s large, 1 H)

La pureté énantiomérique des deux composés obtenus ci dessus est carcatérisée par chromatographie chirale analytique réalisée sur une colonne Chiralpak AY-5µm, 250x4.6 mm en éluant avec un mélange d'acétonitrile et d'isopropanol (90/10 v/v) au débit de 1 ml/min. Les excès énantiomériques sont respectivement de 99% et de 99% pour les énantiomères lévogyre et dextrogyre.

### Exemple 21 b : Synthèse du (+)-2-[2-(2-méthyle-2,3-dihydro-indol-1-yl)-2-oxo-éthyl]-6-morpholin(D8)-4-yl-3H-pyrimidin-4-one & Exemple 22b : (-)-2-[2-(2-méthyle-2,3-dihydro-indol-1-yl)-2-oxo-éthyl]-6-morpholin(D8)-4-yl-3H-pyrimidin-4-one

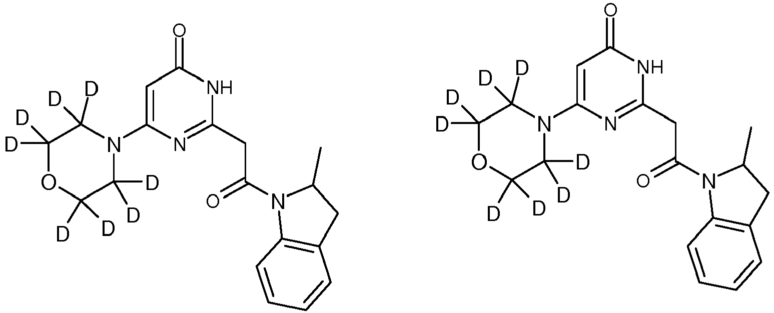

On introduit dans le tricol sous argon, 304 mg de 6-Chloro-2-[2-(2-méthyl-2,3-dihydro-indol-1-yl)-2-oxo-éthyl]-5H-pyrimidin-4-one (exemple 19b, étape 2b), 0.5g de morpholine-2,2,3,3,5,5,6,6-D8 (342611-02-3), dans 40 ml de dioxane puis on ajoute 700 µl de TEA à la température ambiante, agite en chauffant à 85°C pendant 20 heures. On concentre sous vide le melange reactionnel, reprend dans 50 ml d'AcOEt, ajoute 20 ml de solution saturée en NaCl, décante, sèche sur MgSO4, filtre sur VF. Le composé obtenu est chromatographié sur gel de silice (40-63 µm) en éluant avec un mélange de dichlorométhane et de méthanol 97/3 v/v. Les fractions contenant le composé attendu sont réunies et évaporées. Le (±)-2-[2-(2-méthyle-2,3-dihydro-indol-1-yl)-2-oxo-éthyl]-6-morpholin(D8)-4-yl-3H-pyrimidin-4-one est obtenu sous forme solide (1.2g), et résolu en ses deux énantiomères dans l'étape suivante.

Le composé racémique (±)-2-[2-(2-méthyle-2,3-dihydro-indol-1-yl)-2-oxo-éthyl]-6-morpholin(D8)-4-yl-3H-pyrimidin-4-one obtenu ci-dessus est résolu en ses deux énantiomères par chromatographie chirale sur une colonne contenant 1.08 kg de phase stationaire Chiralpak AY 20µm (7.7 x 35 cm), en éluant avec un mélange de acétonitrile et d'isopropanol (90/10 v/v) à 250 ml/min. Les fractions contenant chaque énantiomères sont évaporées. On isole exemple 21 b et exemple 22b ci dessous

Le premier enantiomère, (+)-2-[2-(2-méthyle-2,3-dihydro-indol-1-yl)-2-oxo-éthyl]-6-morpholin(D8)-4-yl-3H-pyrimidin-4-one est isolé (502mg) et caractérisé (+83.3°, c=2.003mg/0.5 ml DMSO, 589nm).

### Spectrométrie de Masse :

Les spectres ont été obtenus sur un appareil Waters UPLC-SQD
Ionisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;
1,95 : 5 % de B

### Résultats analytiques :

Temps de rétention Tr (min) = 0,69 ;
[M+H]+ : m/z 363 ; [M-H]- : m/z 361

### Résonance Magnétique Nucléaire :

Spectre RMN 1 H (400 MHz, ) : 1,26 (d, J=6,4 Hz, 3 H) ; 2,68 (d, J=16,3 Hz, 1 H) ;3,37 (dd, J=8,6 et 16,3 Hz, 1 H) ; 3,72 (d, J=15,9 Hz, 1 H) ; 3,92 (d, J=15,9 Hz, 1 H) ; 4,71 (m, 1 H) ; 5,19 (s, 1 H) ; 7,04 (t, J=7,9 Hz, 1 H) ; 7,18 (t, J=7,9 Hz, 1 H) ; 7,28 (d, J=7,9 Hz, 1 H) ; 7,96 (d, J=7,9 Hz, 1 H) ; 11,68 (s large, 1 H)

Le deuxieme énantiomère, (-)-2-[2-(2-méthyle-2,3-dihydro-indol-1-yl)-2-oxo-éthyl]-6-morpholin(D4)-4-yl-3H-pyrimidin-4-one est isolé (505mg) (-82°, c=1.670 mg/0.5ml DMSO, 589nm)

### Spectrométrie de Masse :

Les spectres ont été obtenus sur un appareil Waters UPLC-SQD
Ionisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;
1,95 : 5 % de B

### Résultats analytiques :

Temps de rétention Tr (min) = 0,69 ;[M+H]+ : m/z 363 ; [M-H]- : m/z 361
Spectre RMN 1 H (400 MHz) : 1,26 (d, J=6,3 Hz, 3 H) ; 2,68 (d, J=16,3 Hz, 1 H) ;3,37 (dd, J=8,6 et 16,3 Hz, 1 H) ; 3,72 (d, J=15,9 Hz, 1 H) ; 3,92 (d, J=15,9 Hz, 1 H) ; 4,71 (m, 1 H) ; 5,19(s, 1 H) ; 7,04 (t, J=7,9 Hz, 1 H) ; 7,18 (t, J=7,9 Hz, 1 H); 7,28 (d, J=7,9 Hz, 1 H) ; 7,96 (d, J=7,9 Hz, 1 H); 11,66 (s large, 1 H)

La pureté énantiomérique des deux composés obtenus ci dessus est carcatérisée par chromatographie chirale analytique réalisée sur une colonne Chiralpak AY-5µm, 250x4.6 mm en éluant avec un mélange de acétonitrile et de isopropanol (90/10 v/v) au débit de 1 ml/min. Les excès énantiomériques sont respectivement de >99% et de >99% pour les énantiomères lévogyre et dextrogyre.

### Exemple 23b : Synthèse du 2-[2-(4-fluoro-2,3-dihydro-indol-1-yl)-2-oxo-éthyl]-6-(2-méthyl-pyridin-4-yl)-3H-pyrimidin-4-one

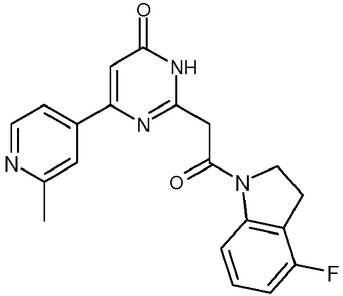

Dans un tube pour micro-ondes de 5 mL, sont introduits successivement 120 mg de 6-chloro-2-[2-(4-fluoro-2,3-dihydro-indol-1-yl)-2-oxo-éthyl]-3H-pyrimidin-4-one, 96 mg de pinacol-ester de l'acide 2-méthylpyridine-4-boronique, 45 mg de tetrakis(triphénylphosphine)palladium (0), 2,5 mL de 1,4-dioxanne et 0,52 mL d'une solution aqueuse 1,5 M de carbonate de césium. La suspension résultante est agitée sous irradiation par micro-ondes à une température de 100°C pendant 2x1 heure. Après refroidissement à température ambiante, le mélange réactionnel est dilué avec 6 mL d'acétate d'éthyle, puis filtré sur Clarcel^{®}. Le solide est lavé avec 3 mL d'acétate d'éthyle, puis le filtrat est traité avec 12 mL d'eau et agité à température ambiante pendant 1,5 heures. Après décantation, la phase organique est séparée et la phase aqueuse est extraite avec 3x10 mL d'acétate d'éthyle. Les extraits organiques sont réunis, lavés avec 10 mL de saumure saturée, séchés sur sulfate de magnésium, filtrés, puis concentrés à sec sous pression réduite. Le résidu est purifié par chromatographie sur une cartouche de 25 g silice 15-40 µm, en faisant un dépôt solide et en éluant avec un mélange dichlorométhane/ méthanol 95/5 v/v, à un débit de 25 mL/ min. Les fractons contenant le produit désiré sont réunies et concentrées à sec sous pression réduite. Le résidu est repris deux fois dans l'éther diéthylique, trituré puis concentré à sec sous pression réduite. On obtient ainsi 46 mg de 2-[2-(4-fluoro-2,3-dihydro-indol-1-yl)-2-oxo-éthyl]-6-(2-méthyl-pyridin-4-yl)-3H-pyrimidin-4-one sous forme d'une poudre cristalline blanche dont les caractéristiques sont les suivantes:
Spectre RMN 1 H (400MHz) : 2,54 (s, 3 H) ; 3,23 (t, J=8,7 Hz, 2 H) ; 3,99 (s, 2 H) ; 4,27 (t, J=8,7 Hz, 2 H) ; 6,88 (t, J=8,7 Hz, 1 H) ; 6,99 (s, 1 H) ; 7,24 (m, 1 H) ; 7,75 (d large, J=5,1 Hz, 1 H) ; 7,82 à 7,88 (m, 2 H) ; 8,54 (d, J=5,1 Hz, 1 H) ; 12,65 (s large, 1 H)
Spectrométrie de Masse : méthode A
Temps de rétention Tr (min) = 0,50 ;
[M+H]+ : m/z 365 ; [M-H]- : m/z 363
Point de fusion (Kofler) : 229°C

### Exemple 24b : 2-[2-(4-fluoro-2,3-dihydro-indol-1-yl)-2-oxo-éthyl]-6-(2-fluoro-pyridin-4-yl)-3H-pyrimidin-4-one

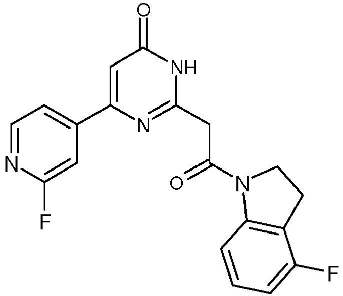

Dans un ballon tricol sous argon et sous agitation, sont introduits successivement 130 mg de 6-chloro-2-[2-(4-fluoro-2,3-dihydro-indol-1-yl)-2-oxo-éthyl]-3H-pyrimidin-4-one, 3 mL de 1,4-dioxanne, 104 mg de pinacol-ester de l'acide 2-fluoropyridine-4-boronique, 50 mg de tetrakis(triphénylphosphine)palladium (0), et 0,58 mL d'une solution aqueuse 1,5 M de carbonate de césium. Le mélange est chauffé à une température de 100°C pendant 20 heures, puis filtré à chaud. Après refroidissement à température ambiante, le filtrat est concentré à sec sous pression réduite. Le résidu est repris dans un mélange de 20 mL de dichlorométhane et 30 mL d'eau. Après décantation, la phase organique est séparée et la phase aqueuse est extraite avec du dichlorométhane. La phase aqueuse est concentrée à sec sous pression réduite. Le résidu est repris dans un mélange de 30 mL d'acétate d'éthyle et quelques gouttes d'éthanol, puis lavé avec 5 mL d'eau. Après décantation, la phase organique est séparée et la phase aqueuse est extraite avec 2x20 mL d'acétate d'éthyle. Les extraits organiques sont réunis, séchés sur sulfate de magnésium, filtrés, puis concentrés à sec sous pression réduite. Le résidu est repris dans 1 mL de dioxanne et le mélange est chauffé à reflux, puis filtré sur verre fritté. Après séchage du solide isolé, on obtient 44 mg de 2-[2-(4-fluoro-2,3-dihydro-indol-1-yl)-2-oxo-éthyl]-6-(2-fluoro-pyridin-4-yl)-3H-pyrimidin-4-one sous forme d'un solide beige dont les caractéristiques sont les suivantes:
Spectre RMN 1 H (400MHz) : 3,20 à 3,38 (m partiellement masqué, 2 H) ; 4,00 (s, 2 H) ; 4,27 (t, J=8,2 Hz, 2 H) ; 6,87 (t, J=8,1 Hz, 1 H) ; 7,11 (s, 1 H) ; 7,23 (m, 1 H) ; 7,74 (s, 1 H) ; 7,85 (d, J=8,1 Hz, 1 H) ; 7,95 (d, J=5,4 Hz, 1 H) ; 8,36 (d, J=5,4 Hz, 1 H) ; 12,86 (m étalé, 1 H)
Spectrométrie de Masse : méthode A
Temps de rétention Tr (min) = 0,80 ;
[M+H]+ : m/z 369 ; [M-H]- : m/z 367
Point de fusion (Kofler) : supérieur à 260°C

### Exemple 25b : Synthèse du 2-[2-(4-Chloro-2,3-dihydro-indol-1-yl)-2-oxo-éthyl]-6-((-)-2-hydroxyméthyl-morpholin-4-yl)-3H-pyrimidin-4-one et

### Exemple 26b Synthèse du 2-[2-(4-Chloro-2,3-dihydro-indol-1-yl)-2-oxo-éthyl]-6-((+)-2-hydroxyméthyl-morpholin-4-yl)-3H-pyrimidin-4-one

### Étape 1b : Synthèse du 2-[2-(4-Chloro-2,3-dihydro-indol-1-yl)-2-oxo-eth2-(2-Benzylamino-ethoxy)-3-chloro-propan-1-ol

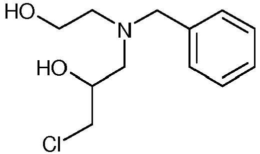

Dans un ballon de 250ml avec réfrigérant, sous argon, on place 23.6 ml de N-benzyléthanolamine (104-63-2) puis on ajoute goutte à goutte 26ml d'epichlorhydrine (106-89-8). Le milieu réactionnel est chauffé à 45°C pendant 3h. Apres retour à température ambiante (20°C), le milieu réactionnel est évaporé sous pression réduite (bain à 40°C pression 20 mbar puis à 2 mbar), pendant une heure. On isole le 2-[2-(4-Chloro-2,3-dihydro-indol-1-yl)-2-oxo-eth2-(2-Benzylamino-ethoxy)-3-chloro-propan-1-ol (41.25g) sous forme d'une huile qu'on engage l'étape suivante

### Étape 2b : Synthèse du 4-Benzyle-2-chlorométhyle-morpholine

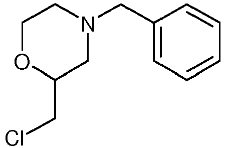

Dans un ballon de 250ml équipé d'un réfrigérant, on place 41.25g de 2-[2-(4-Chloro-2,3-dihydro-indol-1-yl)-2-oxo-eth2-(2-Benzylamino-ethoxy)-3-chloro-propan-1-ol obtenu précédemment, puis on ajoute goutte à goutte 50ml d'acide sulfurique concentré (d=1.84). La température augmente, l'eau formée se condense dans le réfrigérant. Le milieu réactionnel est chauffé ensuite une heure à 150°C, puis refroidi à température ambiante (20°C) avant d'être versé lentement sur de la glace ; le milieu réactionnel est ammené à pH=10 avec NaOH 40% puis extrait au toluène (2 x 150 ml), les phases organiques sont réunies, séchées sur sulfate de magnésium, filtrées et évaporées sour pression réduite. On isole 4-Benzyle-2-chlorométhyle-morpholine (20.30g huile marron claire Rdt=54% que l'on engage dans l'étape suivante.

### Étape 3b : Synthèse du (4-Benzyl-morpholin-2-yl)-méthanol

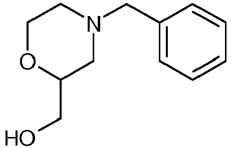

Dans un ballon de 250ml, avec réfrigérant, thermomètre et sous argon, on place 20.3g de 4-Benzyle-2-chlorométhyle-morpholine, obtenu précédemment, 3.5ml d'eau puis 45 ml de formamide (75-12-7). Le milieu réactionnel est chauffé à 215°C au reflux du formamide pendant 3h puis refoidi à 50°C avec un bain d'eau glacée. On ajoute de nouveau 3.5ml d'eau et on reprend le reflux 2h supplémentaire. Après retour à température ambiante (20°C) on verse le milieu sur de l'eau glacée (150ml), basicifier par de la soude 10M (50ml) à pH=12, extraire deux fois au toluène, réunir les phases organiques et les laver avec une solution saturée de NaCl (50ml), sécher sur sulfate de magnésium et amener à sec sous vide de 3mbar pendant 1h. On isole le (4-Benzyl-morpholin-2-yl)-méthanol (13.4g) sous forme d' une huile ambrée (Rdt=72%)

### Étape 4b : Synthèse du chlorhydrate de morpholine-2-yl-méthanol

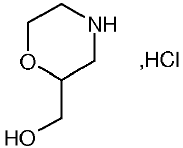

Dans un autoclave sous atmosphère d'argon à température ambiante (20°C), on place successivement 215ml de méthanol, 1.7g d'hydroxyde de Palladium (12135-22-7), 12.4g de (4-Benzyl-morpholin-2-yl)-méthanol et 15ml de HCl 4M dans le dioxane. Apres fermeture de l'autoclave, le milieu réactionnel est placé sous 6 bar d'hydrogène à 25°C pendant 24h. Le milieu réactionnel est filtré sur Clarcel, rincé plusieurs fois au méthanol ; le filtrat est concentré sous pression réduite. On isole le chlorhydrate de morpholine-2-yl-méthanol (9.65 g) sous forme d'une huile jaune.

### Étape 5b : Synthèse de l'ester éthylique de l'acide (4-Chloro-6-oxo-1,6-dihydro-pyrimidin-2-yl)-acétique

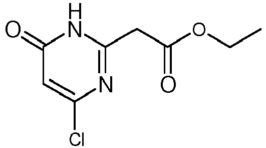

Dans un autoclave 1000ml introduit 33 g de l'ester éthylique de l'acide (4-Chloro-6-méthoxy-pyrimidin-2-yl)-acétique, (exemple 8b étape 2b) 750 ml d'acétonitrile puis on ajoute 71.2 g d'iodure de potassium et 55.85 ml de triméthylchlorosilane; on obtient une solution hétérogène orangée que l'on agite en chauffant sous une pression de 10 bars d'argon à 100°C pendant 2 heures. Le milieu réactionnel est soutiré puis on filtre l'insoluble, lave avec 3 fois environ 100 ml d'AcOEt, concentre le filtrat jusqu'à obtention d'un résidu pâteux que l'on reprend dans 500 ml d'eau. Apres agitation, extraction par 3 fois environ 350 ml d'acétate d'éthyle, on lave les extraits organiques réunis avec 500 ml de solution saturée en NaCl, sèche sur MgSO4, filtre sur VF, concentre sous vide. Le composé obtenu est purifié par chromatographie sur gel de silice (40-63µM) en éluant avec un mélange de (2/8 v/v) AcOEt/n-Heptane. Les fractions contenant le composé attendu sont réunies et évaporées. On isole un solide que l'on triture dans l'oxyde de diisopropyl, filtre sur verre fritté et sèche. L'ester éthylique de l'acide (4-Chloro-6-oxo-1,6-dihydro-pyrimidin-2-yl)-acétique est isolé sous forme d'un solide beige. 25.2 g ; Rdt 81%
LCMS ES+ DMSO Tr 0.71 min ; MH+ m/z=217

### Étape 6b: Synthèse du l'ester éthylique de l'acide [4-(2-Hydroxyméthyl-morpholin-4-yl)-6-oxo-1,6-dihydro-pyrimidin-2-yl]-acétique

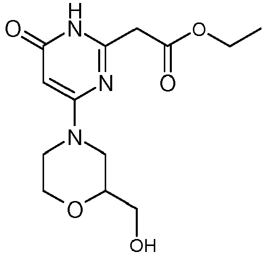

Sous argon, dans un ballon de 500ml équipé d'un thermomètre et d'un réfrigérant, on place successivement 10g de l'ester éthylique de l'acide (4-Chloro-6-oxo-1,6-dihydro-pyrimidin-2-yl)-acétique (exemple 25b, étape 5b), 9.6g de chlorhydrate de Morpholine-2-yl-méthanol obtenu précédemment dans 200ml de DMSO et 16.1ml de triéthylamine. Le milieu réactionnel est chauffé pendant 22h à 85°C. Apres retour à température ambiante (20°C), le milieu réactionnel est versé sur une solution saturée en NaCl, extrait par 8x250ml d'acétate d'éthyle. Les extraits organiques sont réunis, séchés sur sulfate de magnésium, filtrés et évaporés sous pression réduite. On obtient un solide qui est empâté dans l'éther diisopropylique contenant 10% de chlorure de méthylène. Le solide est filtré rincé deux fois puis une fois au pentane. On isole l'ester éthylique de l'acide [4-(2-Hydroxyméthyl-morpholin-4-yl)-6-oxo-1,6-dihydro-pyrimidin-2-yl]-acétique. (1.9g ; Rdt=14%) sous forme de solide beige.

### Étape 7b: Synthèse du sel de sodium de l'acide [4-(2-hydroxyméthyl-morpholin-4-yl)-6-oxo-1,6-dihydro-pyrimidin-2-yl]-acétique

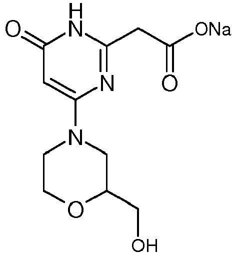

Dans un ballon, on place une solution de, 2 g de l'ester éthylique de l'acide [4-(2-Hydroxyméthyl-morpholin-4-yl)-6-oxo-1,6-dihydro-pyrimidin-2-yl]-acétique dans 15 ml de THF puis on coule gouttes à gouttes une quantité stoechiométrique de soude en solution 2M. Le milieu est agité pendant 72 heures à l'ambiante puis concentré sous pression réduite. On obtient 2.5 g de solide que l'on reprends dans 20 ml de THF, et que l'on triture ; le solide obtenu est filtré sur verre fritté, rincé à l'éther éthylique, séché à l'étuve sous vide. On isole le sel de sodium de l'acide [4-(2-hydroxyméthyl-morpholin-4-yl)-6-oxo-1,6-dihydro-pyrimidin-2-yl]-acétique, 1.6 g de solide jaune que l'on utilise dans l'étape suivante.

### Étape 8b : Séparation Chirale : Synthèse du ester éthylique de l'acide (+)-[4-(2-Hydroxyméthyl-morpholin-4-yl)-6-oxo-1,6-dihydro-pyrimidin-2-yl]-acétique et ester éthylique de l'acide (-)-[4-(2-Hydroxyméthyl-morpholin-4-yl)-6-oxo-1,6-dihydro-pyrimidin-2-yl]-acétique

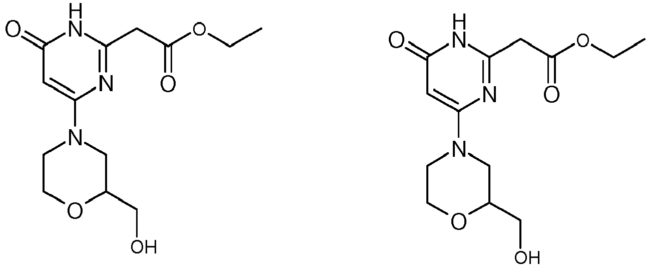

L'ester éthylique de l'acide (±)-[4-(2-Hydroxyméthyl-morpholin-4-yl)-6-oxo-1,6-dihydro-pyrimidin-2-yl]-acétique obtenu précédemment (exemple 25b, étape 6b) est résolu en ses deux énantiomères par chromatographie chirale sur phase Chiralpak T304 20µm, 7.5cm x 35cm en éluant à 300ml/min à l'aide d'un mélange (Heptane/acétate d'éthyle/Triéthyl amine : 70/30/0.1). Les fractions contenant les énantiomères sont évaporées.

On isole successivement :
L'intermédiaire 25-A : premier énantiomère de l'ester éthylique de l'acide [4-(2-Hydroxyméthyl-morpholin-4-yl)-6-oxo-1,6-dihydro-pyrimidin-2-yl]-acétique, 92mg.
L'intermédiaire 25-B: deuxième énantiomère de l'ester éthylique de l'acide [4-(2-Hydroxyméthyl-morpholin-4-yl)-6-oxo-1,6-dihydro-pyrimidin-2-yl]-acétique 72mg .
La pureté énantiomérique de ces compsés est caractérisée par analyse chirale sur Chiralpak T304 5µm 250 mm x 4.6 mm, 1ml/min, Heptane 70% EtOH 30% TEA 0.1%.

### Étape 9b : Synthèse du sel de sodium de l'acide (+)-[4-(2-hydroxyméthyl-morpholin-4-yl)-6-oxo-1,6-dihydro-pyrimidin-2-yl]-acétique

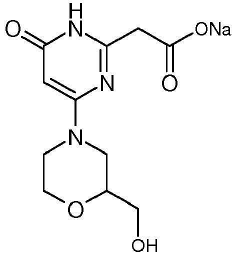

Dans un ballon de 10ml sous argon on place 92mg de l'ester éthylique de l'acide [4-(2-Hydroxyméthyl-morpholin-4-yl)-6-oxo-1,6-dihydro-pyrimidin-2-yl]-acétique, (intermédiaire 25-A, exemple 25b, étape 8b) dans 1 ml de THF que l'on additionne de 155 µl de soude 2N. Le milieu réactionnel est agité à température ambiante (20°C) pendant 96h. Evaporer le THF à température ambiante (20°C) puis on ajoute 2ml d'eau, extraire à l'éther éthylique. Evaporer l'eau à température ambiante (20°C) sous 4 mbar pendant 2h. On isole le sel de sodium de l'acide [4-(2-hydroxyméthyl-morpholin-4-yl)-6-oxo-1,6-dihydro-pyrimidin-2-yl]-acétique 71 mg solide blanc cassé Rdt. 83%, utilisé tel quel dans l'étape suivante

### Étape 10b, Exemple 25b: (-)-2-[2-(4-Chloro-2,3-dihydro-indol-1-yl)-2-oxo-éthyl]-6-(2-hydroxyméthyl-morpholin-4-yl)-3H-pyrimidin-4-one ;

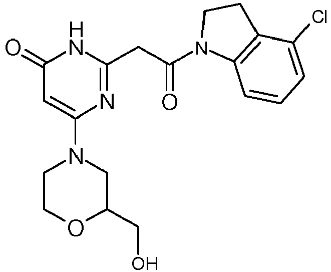

Dans un ballon de 25ml on place 71 mg du sel de sodium de l'acide [4-(2-hydroxyméthyl-morpholin-4-yl)-6-oxo-1,6-dihydro-pyrimidin-2-yl]-acétique (exemple 25b, étape 9b) 38mg de 4-chloro-2,3-dihydro-1H-indole dans 1ml de diméthyl formamide et 0.24ml de pyridine, puis on ajoute 57mg de chlorhydrate de N-[3-(diméthylamino)propyl]-N'-éthylcarbodiimide. Le milieu réactionnel est agité à température ambiante pendant 20h, puis évaporé, ajouté d'eau et trituré. Le solide formé est filtré, rincé successivement à l'eau puis à l'éther diisopropylique, au chlorure de méthylène puis au pentane. Le solide ainsi obtenu est séché sous vide 2h. On isole le (-)-2-[2-(4-Chloro-2,3-dihydro-indol-1-yl)-2-oxo-éthyl]-6-(2-hydroxyméthyl-morpholin-4-yl)-3H-pyrimidin-4-one, (62mg) solide pulvérulent (Rdt=63%).

### Spectrométrie de Masse :

Les spectres ont été obtenus sur un appareil Waters UPLC-SQD
Ionisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;1,95 : 5 % de B

### Résultats analytiques :

Temps de rétention Tr (min) = 0,66 ;[M+H]+ : m/z 405 ; [M-H]- : m/z 403
Spectre RMN 1 H (400 MHz) : 2,58 (m partiellement masqué, 1 H) ; 2,83 (m, 1 H) ;3,19 (t, J=8,7 Hz, 2 H) ; 3,27 à 3,52 (m partiellement masqué, 4 H) ; 3,77 (s, 2 H) ; 3,82 à 3,96 (m, 2 H) ;4,12 (m, 1 H) ; 4,21 (t, J=8,7 Hz, 2 H) ; 4,71 (t, J=6,1 Hz, 1 H) ; 5,20 (s, 1 H) ; 7,09 (d, J=7,9 Hz, 1 H) ;7,22 (t, J=7,9 Hz, 1 H) ; 7,97 (d, J=7,9 Hz, 1 H) ; 11,61 (s large, 1 H)
Pouvoir rotatoire : α_{D} = -22° C=0.351mg/0.5ml dans le DMSO

### Étape 11 b, Exemple 26b: (+)-2-[2-(4-Chloro-2,3-dihydro-indol-1-yl)-2-oxo-éthyl]-6-(2-hydroxyméthyl-morpholin-4-yl)-3H-pyrimidin-4-one ;

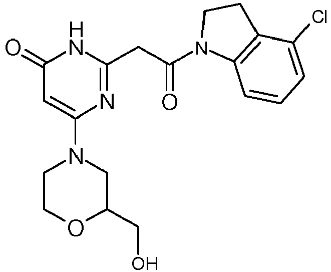

Dans un ballon de 25ml on place 42 mg du sel de sodium de l'acide [4-(2-hydroxyméthyl-morpholin-4-yl)-6-oxo-1,6-dihydro-pyrimidin-2-yl]-acétique (exemple 25b, étape 9b) 22 mg de 4-chloro-2,3-dihydro-1H-indole dans 1ml de diméthyl formamide et 0.2ml de pyridine, puis on ajoute 33mg de chlorhydrate de N-[3-(diméthylamino)propyl]-N'-éthylcarbodiimide. Le milieu réactionnel est agité à température ambiante pendant 20h, puis évaporé, ajouté d'eau et trituré. Le solide formé est filtré, rincé successivement à l'eau puis à l'éther diisopropylique, au chlorure de méthylène puis au pentane. Le solide ainsi obtenu est séché sous vide 2h. On isole le (+)-2-[2-(4-Chloro-2,3-dihydro-indol-1-yl)-2-oxo-éthyl]-6-(2-hydroxyméthyl-morpholin-4-yl)-3H-pyrimidin-4-one, (18mg) solide pulvérulent (Rdt=31%).

### Spectrométrie de Masse :

Les spectres ont été obtenus sur un appareil Waters UPLC-SQD
Ionisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;1,95 : 5 % de B

### Résultats analytiques :

Temps de rétention Tr (min) = 0,66 ;[M+H]+ : m/z 405 ; [M-H]- : m/z 403
Spectre RMN 1 H (400 MHz) : 2,58 (m partiellement masqué, 1 H) ; 2,83 (m, 1 H) ;3,19 (t, J=8,7 Hz, 2 H) ; 3,27 à 3,52 (m partiellement masqué, 4 H) ; 3,77 (s, 2 H) ; 3,82 à 3,96 (m, 2 H) ;4,12 (m, 1 H) ; 4,21 (t, J=8,7 Hz, 2 H) ; 4,71 (t, J=6,1 Hz, 1 H) ; 5,20 (s, 1 H) ; 7,09 (d, J=7,9 Hz, 1 H) ;7,22 (t, J=7,9 Hz, 1 H) ; 7,97 (d, J=7,9 Hz, 1 H) ; 11,61 (s large, 1 H)
Pouvoir rotatoire : α_{D} = +19° C=0.950mg/0.5ml dans le DMSO

### Exemple 27b : Synthèse du (±)-2-[2-(4-Fluoro-2,3-dihydro-indol-1-yl)-2-oxo-éthyl]-6-(2-hydroxyméthyl-morpholin-4-yl)-3H-pyrimidin-4-one

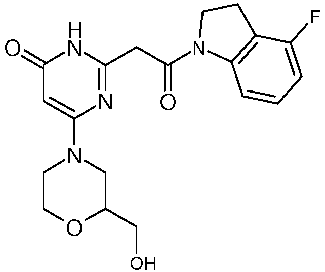

Dans un tricol, on introduit 0.975 g du sel de sodium de l'acide (±)- [4-(2-hydroxyméthyl-morpholin-4-yl)-6-oxo-1,6-dihydro-pyrimidin-2-yl]-acétique (example 25b, étape 6b), 0.534 g de 4-Fluoro-indoline; 0.57 ml de pyridine et 15 ml de DMF. On ajoute 0.9 g de chlorhydrate de N-[3-(diméthylamino)propyl]-N'-éthylcarbodiimide et agite à température ambiante (20°C) pendant 72 heures. Sous agitation, on verse le mélange réactionnel sur 50 ml d'eau, puis on filtre l'insoluble sur VF, lave 3 fois avec environ 15 ml d'eau puis avec 10 ml d'acétate d'éthyle et 2 fois environ 5 ml d'oxyde de diisopropyle. Le solide est séché à l'air sous hotte. On isole et caractérise la 2-[2-(4-Fluoro-2,3-dihydro-indol-1-yl)-2-oxo-éthyl]-6-(2-hydroxyméthyl-morpholin-4-yl)-3H-pyrimidin-4-one, (0.82 g) de solide rosé Rdt 60%

### Spectrométrie de Masse :

Les spectres ont été obtenus sur un appareil Waters UPLC-SQD
Ionisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;1,95 : 5 % de B

### Résultats analytiques :

Temps de rétention Tr (min) = 0,59 ;[M+H]+ : m/z 389 ; [M-H]- : m/z 387
Spectre RMN 1 H (400 MHz) : 2,58 (m, 1 H) ; 2,83 (m, 1 H) ; 3,20 (t, J=8,6 Hz, 2H) ; 3,32 à 3,50 (m, 4 H) ; 3,77 (s, 2 H) ; 3,83 à 3,96 (m, 2 H) ; 4,12 (m, 1 H) ; 4,22 (t, J=8,6 Hz, 2 H) ; 4,73(t, J=5,5 Hz, 1 H) ; 5,20 (s, 1 H) ; 6,86 (t, J=8,7 Hz, 1 H) ; 7,21 (m, 1 H) ; 7,84 (d, J=8,7 Hz, 1 H) ; 11,62 (slarge, 1 H)

### Exemple 28b : Synthèse du 2-[2-(4-Chloro-2,3-dihydro-indol-1-yl)-2-oxo-éthyl]-6-(2-méthyl-morpholin-4-yl)-3H-pyrimidin-4-one

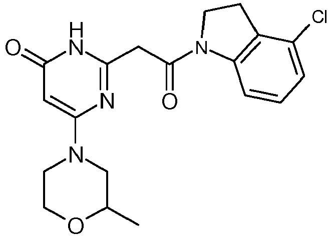

Dans un ballon, on introduit, 0.975 g de sel de sodium de l'acide [4-(2-méthyl-morpholin-4-yl)-6-oxo-1,6-dihydro-pyrimidin-2-yl]-acétique (exemple 1b, étape 2b), 0.534 g de 4-Chloro- indoline, 0.57 ml de pyridine et 15 ml de DMF puis on ajoute 0.9 g de chlorhydrate de N-[3-(diméthylamino)propyl]-N'-éthylcarbodiimide Le mélange réactionnel est agité à température ambiante pendant 72 heures. Sous agitation, on verse sur 50 ml d'eau, puis le solide formé est filtré sur verre fritté et lavé 3 fois avec environ 15 ml d'eau, lavé avec 10 ml d'AcOEt, rincé avec 2 fois environ 5 ml d'oxyde de diisopropyl, laisse sécher à l'air sous hotte. On isole et caractérise le 2-[2-(4-Chloro-2,3-dihydro-indol-1-yl)-2-oxo-éthyl]-6-(2-méthyl-morpholin-4-yl)-3H-pyrimidin-4-one (0.98 g) sous forme d'un solide blanc cassé (Rdt 71 %).
LCMS ES+ DMSO Tr 1.15 ; MH+ m/z=389

### Spectrométrie de Masse :

Les spectres ont été obtenus sur un appareil Waters UPLC-SQD
Ionisation : électrospray en mode positif et/ou négatif (ES+/-)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ;1,95 : 5 % de B

### Résultats analytiques :

Temps de rétention Tr (min) = 0,82 ;[M+H]+ : m/z 389 ; [M-H]- : m/z 387
Spectre RMN 1 H (400 MHz) : 1,06 (d, J=6,4 Hz, 3 H) ; 2,48 (m partiellement
   masqué, 1 H) ; 2,80 (m, 1 H) ; 3,19 (t, J=8,7 Hz, 2 H) ; 3,40 à 3,50 (m, 2 H) ; 3,76 (s, 2 H) ; 3,81 (dd,J=3,0 et 11,5 Hz, 1 H) ; 3,93 (m, 1 H) ; 4,03 (m, 1 H) ; 4,20 (t, J=8,7 Hz, 2 H) ; 5,22 (s, 1 H) ; 7,09 (d,J=8,1 Hz, 1 H) ; 7,22 (t, J=8,1 Hz, 1 H) ; 7,97 (d, J=8,1 Hz, 1 H) ; 11,61 (s large, 1 H)

### Exemple 29b : Synthèse du (+)-2-[2-((S)-2-Méthyl-2,3-dihydro-indol-1-yl)-2-oxo-éthyl]-6-(2-méthyl-morpholin-4-yl)-3H-pyrimidin-4-one

### Étape 1b : chromatographie chirale de l'acide (±)-[4-(2-méthyl-morpholin-4-yl)-6-oxo-1,6-dihydro-pyrimidin-2-yl]-acétique

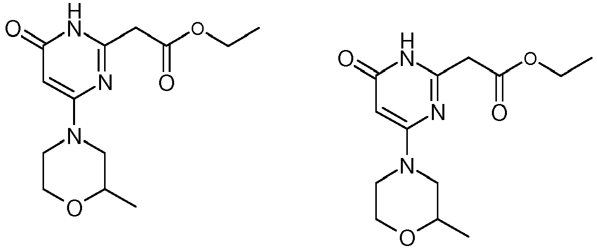

L'ester éthylique de l'acide (±)-[4-(2-méthyl-morpholin-4-yl)-6-oxo-1,6-dihydro-pyrimidin-2-yl]-acétique, (exemple 1b, étape 1b) est résolu en ses deux énantiomères par chromatographie chirale sur une colonne contenant 1.2 kg de phase stationaire Chiralpak AS 20µm (7.7 x 35 cm), en éluant avec un mélange d'heptane 80%, éthanol 20%, triéthyl amine 0.05% à 300 ml/min. Les fractions contenant les énantiomères sont réunies et évaporées. Les solides obtenus sont séchés et caractérisés par leur pouvoir rotatoire. On isole les esters intermédiaires suivants :
Intermédiaire 29-A : Un premier énantiomère (1.1g), ester éthylique de l'acide [4-(2-méthyl-morpholin-4-yl)-6-oxo-1,6-dihydro-pyrimidin-2-yl]-acétique -12°, c=2.047mg/0.5ml DMSO, 589nm dont les caractéristiques sont :
   Waters UPLC-SQD : Ionisation : électrospray en mode positif et/ou négatif (ES+/- )

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B; 1,95: 5 % de B

### Résultats analytiques :

Temps de rétention Tr (min) = 0,55 ;[M+H]+ : m/z 282 ; [M-H]- : m/z 280
**Spectre RMN 1H (400 MHz) :** 1,11 (d, J=6,3 Hz, 3 H) ; 1,19 (t, J=7,1 Hz, 3 H) ;2,48 (m partiellement masqué, 1 H) ; 2,81 (m, 1 H) ; 3,36 à 3,51 (m, 2 H) ; 3,56 (s, 2 H) ; 3,83 (m, 1 H) ;3,95 (m, 1 H) ; 4,03 (m, 1 H) ; 4,12 (q, J=7,1 Hz, 2 H) ; 5,21 (s, 1 H) ; 11,65 (m étalé, 1 H)

Intermédiaire 29-B : Un deuxième énantiomère (1g), ester éthylique de l'acide [4-(2-méthyl-morpholin-4-yl)-6-oxo-1,6-dihydro-pyrimidin-2-yl]-acétique (1 g) +19°, c=1.799mg/0.5ml DMSO, 589nm, dont les caractéristiques sont :
Waters UPLC-SQD : Ionisation : électrospray en mode positif et/ou négatif (ES+/)

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ; 1,95: 5 % de B

### Résultats analytiques :

Temps de rétention Tr (min) = 0,55 ;[M+H]+ : m/z 282 ;[M-H]- : m/z 280 ; pic de base : m/z 234
Spectre RMN 1H (400 MHz): 1,11 (d, J=6,3 Hz, 3 H) ; 1,19 (t, J=7,1 Hz, 3 H) ;2,48 (m partiellement masqué, 1 H) ; 2,81 (m, 1 H) ; 3,36 à 3,51 (m, 2 H) ; 3,56 (s, 2 H) ; 3,83 (m, 1 H) ;3,95 (m, 1 H) ; 4,03 (m, 1 H) ; 4,12 (q, J=7,1 Hz, 2 H) ; 5,21 (s, 1 H) ; 11,65 (m étalé, 1 H)

La pureté énantiomérique des deux composés obtenus ci dessus est carcatérisée par chromatographie chirale analytique réalisée sur une colonne Chiralpak AS-H-5µm, 250x4.6 mm en éluant avec un mélange Heptane 80% EtOH 20% TEA 0.05% au débit de 1 ml/min. Les ratios énantiomériques sont respectivement de 98.5/1.5% et de 1.3/98.7% pour les énantiomères lévogyre et dextrogyre.

### Étape 2b : Synthèse du sel de sodium de l'acide [4-(2-méthyl-morpholin-4-yl)-6-oxo-1,6-dihydro-pyrimidin-2-yl]-acétique

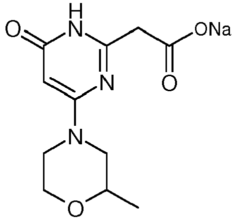

L'ester éthylique dextrogyre de l'acide [4-(2-méthyl-morpholin-4-yl)-6-oxo-1,6-dihydro-pyrimidin-2-yl]-acétique, (Intermédiaire 29-B, exemple 29b, étape 1b) 1g dans 10 ml de THF est traité dans les conditions décrites à l'étape 2b de l'exemple 1b, en présence de 1 équivalent de soude pour conduire à 1.1g de sel de sodium de l'acide [4-(2-méthyl-morpholin-4-yl)-6-oxo-1,6-dihydro-pyrimidin-2-yl]-acétique que l'on utilise dans l'étape suivante.

### Étape 3b: Synthèse du 2-[2-((S)-2-Méthyl-2,3-dihydro-indol-1-yl)-2-oxo-éthyl]-6-(2-méthyl-morpholin-4-yl)-3H-pyrimidin-4-one

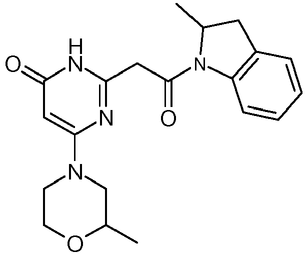

Dans un ballon on place une solution de 500mg du sel de sodium de l'acide [4-(2-méthyl-morpholin-4-yl)-6-oxo-1,6-dihydro-pyrimidin-2-yl]-acétique obtenu ci-dessus dans 7.5ml de DMF et 7.5ml de pyridine, puis on ajoute 420mg de chlorhydrate de N-[3-(diméthylamino)propyl]-N'-éthylcarbodiimide et 0.27g de (S)-2-méthyl-1H-indoline (CAS 22160-09-4). Le mélange réactionnel est agité à température ambiante pendant 48h. On ajoute, 50 ml d'eau, extrait par 3 fois environ 30 ml d'acétate d'éthyle. Les extraits organiques sont réunis et successivement lavés par 30 ml d'eau, 2 fois 30 ml de solution 1M d'acide chlorhydrique puis 30 ml de solution saturée en NaCl, séchés sur MgSO4 et concentrés sous vide. Le composé obtenu est purifié par chromatographie sur colonne Merck de 30g (15-40µm) au débit de 30ml/mn, éluant dichlorométhane/méthanol de 98/2 à 95/5. Les fractions contenant le composé attentu sont réunies et évaporées. On isole le (+)-2-[2-((S)-2-Méthyl-2,3-dihydro-indol-1-yl)-2-oxo-éthyl]-6-(2-méthyl-morpholin-4-yl)-3H-pyrimidin-4-one (237mg), dont les caractéristiques sont les suivantes :
Pouvoir rotatoire : +91°, c=0.135mg/0.5ml DMSO
Waters UPLC-SQD : Ionisation : électrospray en mode positif et/ou négatif (ES+/- )

### Conditions chromatographiques :

▪ Colonne: ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B; 1,95:5 % de B
Résultats analytiques : Temps de rétention Tr (min) = 0,75 ;[M+H]+ : m/z 369 ; [M-H]- : m/z 367
Spectre RMN 1 H (400 MHz) : 1,07 (d, *J*=6,4 Hz, 3 H) ; 1,26 (d large, *J*=6,6 Hz, 3H) ; 2,47 (m partiellement masqué, 1 H) ; 2,69 (d, *J*=16,3 Hz, 1 H) ; 2,81 (m, 1 H) ; 3,31 à 3,51 (m, 3 H) ;3,66 à 4,10 (m, 5 H) ; 4,70 (m, 1 H) ; 5,21 (s, 1 H) ; 7,04 (t, *J*=8,0 Hz, 1 H) ; 7,18 (t, *J*=8,0 Hz, 1 H); 7,28(d, *J*=8,0 Hz, 1 H) ; 7,96 (d large, *J*=8,0 Hz, 1 H) ; 11,67 (m étalé, 1 H)

### Exemple 30b :Synthèse du 6-(-2-Fluorométhyl-morpholin-4-yl)-2-[2-((S)-2-méthyl-2,3-dihydro-indol-1-yl)-2-oxo-éthyl]-3H-pyrimidin-4-one

### Étape 1b : Synthèse de l'ester éthylique de l'acide (±)-[4-(2-fluorométhyl-morpholin-4-yl)-6-oxo-1,6-dihydro-pyrimidin-2-yl]-acétique

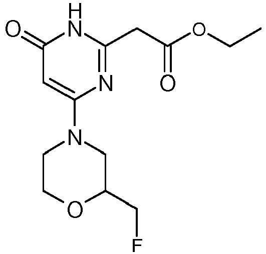

Dans un ballon de 50ml, on place 680mg de 2-Fluorométhyl-morpholine (Yoshikazu Jinho et Coll. ; J. Med. Chem., 37(17), 2791-2796; 1994), 0.63g de l'ester éthylique de l'acide (4-Chloro-6-méthoxy-pyrimidin-2-yl)-acétique, (exemple 8b étape 2b) dans 15ml de DMSO et 1.013ml de triéthylamine. Le milieu réactionnel est chauffé le milieu réactionnel pendant 18h à 85°C. On verse sur une solution saturée en NaCl et extrait par 3 fois 25ml d'acétate d'éthyle. Les extraits organiques sont réunis et lavés une solution saturée de NaCl, séchés sur sulfate de magnésium et évaporés. Le composé obtenu est chromatographié sur gel de silice (15-40 µm, merck) en éluant avec un gradient de dichlorométhane et de méthanol (98/2 à 95/5 v/v. Les fractions contenant le composé attendu sont réunies et évaporées pour conduire à l' ester éthylique de l'acide (±)-[4-(2-Fluorométhyl-morpholin-4-yl)-6-oxo-1,6-dihydro-pyrimidin-2-yl]-acétique (412mg).
LC/MS, ES+, Tr=0.78 min, m/z=299

### Étape 2b : chromatographie chirale l'ester éthylique de l'acide (±)-[4-(2-Fluorométhyl-morpholin-4-yl)-6-oxo-1,6-dihydro-pyrimidin-2-yl]-acétique

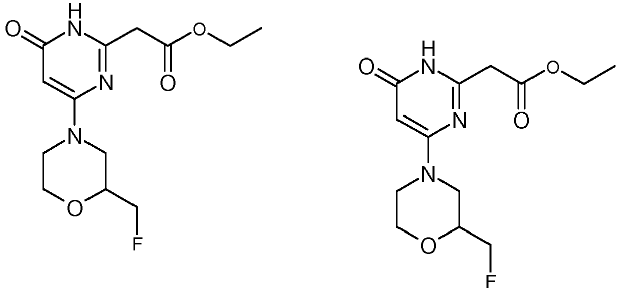

Le composé racémique ester éthylique de l'acide (±)-[4-(2-Fluorométhyl-morpholin-4-yl)-6-oxo-1,6-dihydro-pyrimidin-2-yl]-acétique (exemple 30b, étape 1b) est séparé en ses deux énantiomères par chromatographie chirale sur phase stationaire Chiralpak AS 20µm, 7.7 x 35 cm, en éluant avec un mélange de heptane 70%, isopropanol 30% et de triéthylamine à 250 ml/min. Apres évaporation des fractions d'intérêt, on isole les intermédiaires suivants :
Intermédiaire 30-A : Le premier énantiomère, ester éthylique de l'acide (-)-[4-(2-Fluorométhyl-morpholin-4-yl)-6-oxo-1,6-dihydro-pyrimidin-2-yl]-acétique, (1.17g) (-19.6°, c=1.946 mg/0.5ml DMSO, 589 nm), dont les caractéristiques sont les suivantes :
   Waters UPLC-SQD : Ionisation : électrospray en mode positif et/ou négatif (ES+/- )

### Conditions chromatographiques :

▪ Colonne : ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B 1,85 min :100%deB;1,95:5%deB

### Résultats analytiques :

Temps de rétention Tr (min) = 0,53 ;[M+H]+ : m/z 300 ;[M-H]- : m/z 298 ; pic de base : m/z 252
Spectre RMN 1H (400 MHz) : 1,19 (t, J=7,1 Hz, 3 H) ; 2,70 (m, 1 H) ; 2,86 (m, 1 H) ; 3,50 (m, 1 H) ; 3,57 (s, 2 H) ; 3,66 (m, 1 H) ; 3,87 à 4,00 (m, 2 H) ; 4,05 à 4,16 (m, 3 H) ; 4,47 (dm,*J*=47,2 Hz, 2 H) ; 5,25 (s, 1 H) ; 11,64 (m étalé, 1 H)

Intermédiaire 30-B : Le deuxième énantiomère, ester éthylique de l'acide (+)-[4-(2-Fluorométhyl-morpholin-4-yl)-6-oxo-1,6-dihydro-pyrimidin-2-yl]-acétique, (1.01 g) (+18.2°, c=1.75mg/0.5ml DMSO, 589 nm) dont les caractéristiques sont les suivantes :
Waters UPLC-SQD : Ionisation : électrospray en mode positif et/ou négatif (ES+/) Conditions chromatographiques :
   ▪ Colonne : ACQUITY BEH C18- 1,7 µm - 2,1 x 50 mm
   ▪ Solvants : A : H2O (0,1 % acide formique) B : CH3CN (0,1 % acide formique)
   ▪ Température de colonne : 50 °C
   ▪ Débit : 1 ml/min
   ▪ Gradient (2 min) : de 5 à 50 % de B en 0,8 min ; 1,2 min : 100 % de B 1,85 min : 100 % de B ; 1,95: 5 % de B

### Résultats analytiques :

Temps de rétention Tr (min) = 0,53 ;[M+H]+ : m/z 300 ;[M-H]- : m/z 298 ; pic de base : m/z 252
Spectre RMN 1H (400 MHz) : 1,19 (t, *J*=7,1 Hz, 3 H) ; 2,70 (m, 1 H) ; 2,86 (m, 1 H) ; 3,50 (m, 1 H) ; 3,57 (s, 2 H) ; 3,66 (m, 1 H) ; 3,87 à 4,00 (m, 2 H) ; 4,05 à 4,16 (m, 3 H) ; 4,47 (dm,*J*=47,2 Hz, 2 H) ; 5,25 (s, 1 H) ; 11,64 (m étalé, 1 H)

La pureté énantiomérique des deux composés obtenus ci dessus est carcatérisée par chromatographie chirale analytique réalisée sur une colonne Chiralpak AS 10µm, 250x4.6 mm en éluant avec un mélange de Heptane 70% EtOH 30% TEA 0.1% au débit de 1 ml/min. Les excès énantiomériques sont respectivement de 99.5% et de 99.1%.

### Étape 3b: Synthèse du sel de sodium de l'acide [4-(2-Fluorométhyl-morpholin-4-yl)-6-oxo-1,6-dihydro-pyrimidin-2-yl]-acétique

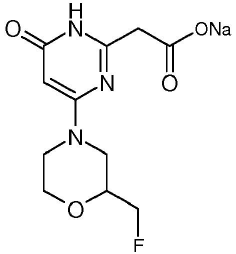

On introduit dans le ballon, 2.8 g d'ester éthylique de l'acide (+)-[4-(2-Fluorométhyl-morpholin-4-yl)-6-oxo-1,6-dihydro-pyrimidin-2-yl]-acétique (Intermédiaire 30-B, exemple 30b, étape 2b) dans 10 ml de THF, puis on coule sur un gouttes à gouttes une quantité stoechiométrique de soude 2M ; On laisse agiter pendant 5 jours à température ambiante (20°C). On concentre à sec sous vide au rotavapor l'ambiante, on obtient 1 g de solide orange mi-huileux que l'on reprend dans 20 ml de THF, triture, filtre sur VF. Le solide est rincé à l'éther éthylique, sèché à l'étuve sous vide. On isole le sel de sodium de l'acide [4-(2-Fluorométhyl-morpholin-4-yl)-6-oxo-1,6-dihydro-pyrimidin-2-yl]-acétique, 1g, solide beige, que l'on utilise tel que dans l'étape suivante.

LCMS ES+ Temps de rétention Tr (min) = 0.56 ; [M+H]+ : m/z 271 (acide correspondant)

### Étape 4b : Synthèse du (+)-6-(2-Fluorométhyl-morpholin-4-yl)-2-[2-((S)-2-méthyl-2,3-dihydro-indol-1-yl)-2-oxo-éthyl]-3H-pyrimidin-4-one

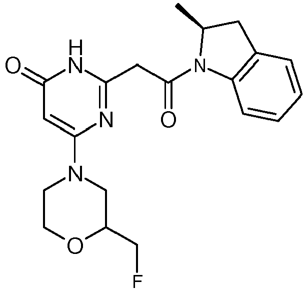

On introduit dans le tricol sous argon, 0.5 g de sel de sodium de l'acide [4-(2-fluorométhyl-morpholin-4-yl)-6-oxo-1,6-dihydro-pyrimidin-2-yl]-acétique obtenu précédemment (exemple 30b, étape 3b) dans 7 ml de pyridine et 7 ml de diméthyl formamide, puis on ajoute le chlorhydrate de N-[3-(diméthylamino)propyl]-N'-éthylcarbodiimide à la température ambiante suivi par 0.25 g de (S)-2-Méthyl indoline (CAS 22160-09-4). Le milieu réactionnel est agité à température ambiante (20°C) pendant 72 heures. On ajoute, 25 ml d'eau, extrait par 3 fois environ 25 ml de dichlorométhane, lave avec 25 ml d'eau, lave avec 2 fois environ 15 ml de solution 1 M d'acide chlorhydrique, lave avec 25 ml d'eau, lave avec 25 ml de solution saturée en NaCl, sèche sur MgSO4, filtre sur VF, concentre sous vide. Le composé obtenu est chromatographié sur gel de silice (40-63 µm) en éluant avec un mélange de dichlorométhane et de éthanol (95/5, v/v). Les fractions contenant le composé attendu sont réunies et évaporées. Le composé obtenu est trituré dans 1 ml de dichlorométhane et 10 ml d'éther di-idopropylique, filtré, séché à température ambiante (20°C). On isole le (+)-6-(2-Fluorométhyl-morpholin-4-yl)-2-[2-((S)-2-méthyl-2,3-dihydro-indol-1-yl)-2-oxo-éthyl]-3H-pyrimidin-4-one, (0.315g).

Waters UPLC-SQD : Ionisation : électrospray en mode positif et/ou négatif (ES+/)

### Conditions chromatographiques :

▪ Colonne: ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B ; 1,95:5 % de B
**Résultats analytiques :** Temps de rétention Tr (min) = 0,74 ;[M+H]+ : M/z 387 ; [M-H]- : m/z 385
**Spectre RMN 1H (400 MHz) :** 1,26 (d large, *J*=6,3 Hz, 3 H) ; 2,63 à 2,74 (m, 2 H) ;2,86 (m, 1 H) ; 3,37 (m, 1 H) ; 3,49 (m, 1 H) ; 3,56 à 3,77 (m, 2 H) ; 3,86 à 4,00 (m, 3 H) ; 4,09 (m, 1 H) ;4,44 (dm, *J*=47,2 Hz, 2 H) ; 4,71 (m, 1 H) ; 5,25 (s, 1 H) ; 7,04 (t, *J*=8,0 Hz, 1 H) ; 7,18 (t, *J*=8,0 Hz, 1 H) ;7,29 (d, *J*=8,0 Hz, 1 H) ; 7,96 (d large, *J*=8,0 Hz, 1 H) ; 11,72 (m étalé, 1 H)
Pouvoir rotatoire PR= + 76.4 +/-1.3 ds DMSO à 589nm C=0.4%
Excès diaséréoisomérique : 99.2% (Chiralpak AS-5µm, 250x4.6mm ; heptane60%, méthanel20%, méthanol20%, 1 ml/min)

### Exemple 31 b : Synthèse du (+)-6-(2-Hydroxyméthyl-morpholin-4-yl)-2-[2-((S)-2-méthyl-2,3-dihydro-indol-1-yl)-2-oxo-éthyl]-3H-pyrimidin-4-one

### Étape 1b : Ester éthylique de l'acide (-)-[4-(2-Hydroxyméthyl-morpholin-4-yl)-6-oxo-1,6-dihydro-pyrimidin-2-yl]-acétique

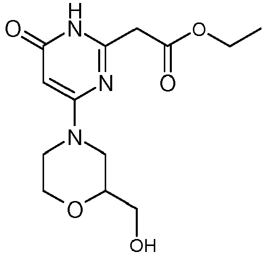

L'ester éthylique de l'acide (±)-[4-(2-Hydroxyméthyl-morpholin-4-yl)-6-oxo-1,6-dihydro-pyrimidin-2-yl]-acétique obtenu précédemment (Ex 25b, étape 6b) est séparé en ses deux énéntiomères par chromatographie chirale sur une colonne contenant 1.080 kg de phase stationaire Chiralpak AY 20µm, en éluant avec un mélange de éthanol/heptane et TEA 30/70/0.1 (%) à 300 ml/min.

Après évaporation des fractions d'intérêt, on isole

L'intermédiaire 31-A, premier enantiomère, ester éthylique de l'acide (-)-[4-(2-Hydroxyméthyl-morpholin-4-yl)-6-oxo-1,6-dihydro-pyrimidin-2-yl]-acétique, 730mg. (PR=-0.6°, c=1.587mg/0.5ml DMSO, 589nm), que l'on utilise dzns l'étape suivante.

L'intermédiaire 31-B, deuxieme énantiomère, ester éthylique de l'acide (+)-[4-(2-Hydroxyméthyl-morpholin-4-yl)-6-oxo-1,6-dihydro-pyrimidin-2-yl]-acétique, 880mg, ((PR=+4°, c=1.904mg/0.5ml DMSO, 589nm).

La pureté énantiomérique des deux composés obtenus ci dessus est carcatérisée par chromatographie chirale analytique réalisée sur une colonne Chiralpak AY-H, 5µ, 250x4.6 mm en éluant avec un mélange de heptane/éthanol/TEA 70/30/01 (%) au débit de 1 ml/min. Les excès énantiomériques sont respectivement de >99% et de >98%.

### Étape 2b : Synthèse du sel de sodium de l'acide (-)-[4-(2-Hydroxyméthyl-morpholin-4-yl)-6-oxo-1,6-dihydro-pyrimidin-2-yl]-acétique

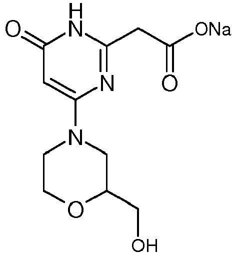

On introduit sous Argon dans le ballon, 0.73 g de l'ester éthylique de l'acide (-)-[4-(2-Hydroxyméthyl-morpholin-4-yl)-6-oxo-1,6-dihydro-pyrimidin-2-yl]-acétique

(intermédiaire 31-A, exemple 31 b, étape 1b) dans 10 ml de THF; sur la solution homogène obtenue, on coule sur un gouttes à gouttes rapide la quantité stoechiometrique de solution de soude 2M puis on laisse agiter pendant 5jours à l'ambiante. On concentre à sec sous vide au rotavapor à température ambiante (20°C), on obtient un solide orange mi-huileux que l'on reprend dans 20 ml de THF, triture, filtre sur VF. Le solide est rincé à l'éther éthylique, sèché à l'étuve sous vide. On isole le sel de sodium de l'acide (-)-[4-(2-Hydroxyméthyl-morpholin-4-yl)-6-oxo-1,6-dihydro-pyrimidin-2-yl]-acétique (0.69 g), solide jaune pâle, que l'on utilise tel que dans les étapes suivantes.

### Étape 3b :Synthèse du (+)-6-(2-Hydroxyméthyl-morpholin-4-yl)-2-[2-((S)-2-méthyl-2,3-dihydro-indol-1-yl)-2-oxo-éthyl]-3H-pyrimidin-4-one

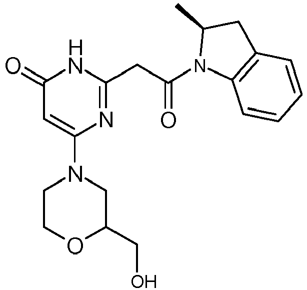

On introduit dans le tricol sous argon, 0.35 g de sel de sodium de l'acide (-)-[4-(2-Hydroxyméthyl-morpholin-4-yl)-6-oxo-1,6-dihydro-pyrimidin-2-yl]-acétique (exemple 31 b, étape 2b) dans 5 ml de Pyridine et 5ml de DMF solution hétérogène blanchâtre. On ajoute le chlorhydrate de N-[3-(diméthylamino)propyl]-N'-éthylcarbodiimide à la température ambiante, ajoute les 0.18 g de (S)-2-Méthyl indoline (CAS 22160-09-4), agite à l'ambiante pendant 72 heures. On ajoute, 25 ml d'eau, extrait par 3 fois environ 25 ml de dichlorométhane, lave avec 25 ml d'eau, lave avec 2 fois environ 15 ml de solution 1M d'acide chlorhydrique, lave avec 25 ml d'eau, lave avec 25 ml de solution saturée en NaCl, sèche sur MgSO4, filtre sur VF, concentre sous vide. Le composé obtenu est chromatographié sur gel de silice (40-63 µm) en éluant avec un mélange de dichlorométhane et d'éthanol. (95/5, v/v). Les fractions contenant le composé attendu sont réunies et évaporées. Le composé obtenu est trituré dans dichlorométhane et l'éther di-idopropylique, filtré, séché à température ambiante (20°C). Le (+)-6-(2-Hydroxyméthyl-morpholin-4-yl)-2-[2-((S)-2-méthyl-2,3-dihydro-indol-1-yl)-2-oxo-éthyl]-3H-pyrimidin-4-one est isolé (0.11 g) .

Waters UPLC-SQD : Ionisation : électrospray en mode positif et/ou négatif (ES+/)

### Conditions chromatographiques :

▪ Colonne: ACQUITY BEH C18- 1,7 µm - 2,1 x 50 mm
▪ Solvants : A : H2O (0,1 % acide formique) B : CH3CN (0,1 % acide formique)
▪ Température de colonne : 50 °C
▪ Débit : 1 ml/min
▪ Gradient (2 min) : de 5 à 50 % de B en 0,8 min ; 1,2 min : 100 % de B ; 1,85 min : 100 % de B; 1,95: 5 % de B
Résultats analytiques : Temps de rétention Tr (min) = 0,60 ;
[M+H]+ : m/z 385 ; [M-H]- : m/z 383
Spectre RMN 1H (400 MHz) : 1,26 (d large, J=6,4 Hz, 3 H) ; 2,57 (m, 1 H) ; 2,68 (d, *J*=16,3 Hz, 1 H) ; 2,84 (m, 1 H) ; 3,33 à 3,49 (m, 5 H) ; 3,72 (m, 1 H) ; 3,81 à 3,98 (m, 3 H) ; 4,12 (m, 1 H) ; 4,72 (m, 2 H) ; 5,19 (s, 1 H) ; 7,04 (t, *J*=8,0 Hz, 1 H) ; 7,18 (t, *J*=8,0 Hz, 1 H) ; 7,28 (d, *J*=8,0 Hz, 1 H); 7,96 (d, *J*=8,0 Hz, 1 H) ; 11,69 (m étalé, 1 H)
Pouvoir rotatoire : α_{D} = +72 +/- 1.5 ds le DMSO à 589nm C=0.27%

### Synthèse des composés de formule (Ic):

### Exemple 1c: Synthèse du 2-[2-(4-chloro-2-méthyl-2,3-dihydro-1H-indol-1-yl)-2-oxoéthyl]-6-(morpholin-4-yl)pyrimidin-4(3H)-one

### Etape 1c :

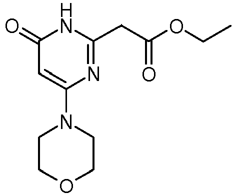

A une solution de 25 g de morpholine dans 400 mL d'éthanol chauffée à 95 °C sont ajoutés 168.5 mL de chlorhydrate de 3-éthoxy-3-iminopropanoate d'éthyle, puis 155 mL de N,N-diisopropyléthylamine dans 200 mL d'éthanol. Le mélange réactionnel est chauffé à 95 °C pendant 30 heures puis laissé revenir à température ambiante. Le précipité formé est filtré sur verre fritté puis lavé avec 100 mL d'éthanol, 2 fois 500 mL d'eau et enfin 500 mL d'éther éthylique. Le solide est séché sous vide pour donner 35 g de [4-(morpholin-4-yl)-6-oxo-1,6-dihydropyrimidin-2-yl]acétate d'éthyle sous forme de solide blanc dont les caractéristiques sont les suivantes :
Spectre RMN 1H (400MHz) : 1,19 (t, *J*=7,1 Hz, 3 H); 3,38 à 3,44 (m, 4 H); 3,56 (s, 2H); 3,61 (dd, *J*=4,0 et 5,7 Hz, 4 H); 4,12 (q, *J*=7,1 Hz, 2 H); 5,20 (s, 1 H); 11,69 (s large, 1 H)
Spectrométrie de Masse : méthode A
Temps de rétention Tr (min) = 0.48;
[M+H]+ : m/z 268 ; [M-H]- : m/z 266

### Etape 2c:

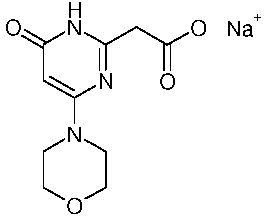

A une solution de 10 g de [4-(morpholin-4-yl)-6-oxo-1,6-dihydropyrimidin-2-yl]acétate d'éthyle dans 300 mL de tétrahydrofuranne, est ajouté 18.7 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif pour donner 8.7 g de [4-(morpholin-4-yl)-6-oxo-1,6-dihydropyrimidin-2-yl]acétate de sodium sous forme de solide blanc dont les caractéristiques sont les suivantes:
Spectre RMN 1 H (400MHz) : 3,08 (s, 2 H); 3,38 (t, *J*=4,6 Hz, 4 H); 3,61 (t, *J*=4,6Hz, 4 H); 5,08 (s, 1 H); 13,16 (s large, 1 H)
Spectrométrie de Masse : méthode A
Temps de rétention Tr (min) = 0.29;
[M+H]+ : m/z 240 ; [M-H]- : m/z 238

### Etape 3c:

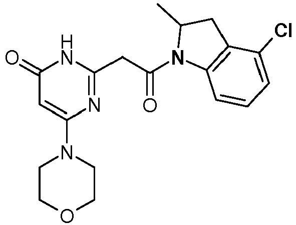

A une solution de 564 mg de [4-(morpholin-4-yl)-6-oxo-1,6-dihydropyrimidin-2-yl]acétate de sodium dans 5 mL de N,N-diméthylformamide sont ajoutés 549 mg de N-[3-(diméthylamino)propyl]-N'-éthylcarbodiimide chlorhydrate et 350 µL de pyridine. La suspension résultante est agitée à température ambiante pendant 10 minutes, puis une solution de 398 mg de 4-chloro-2-méthyl-2,3-dihydro-1H indoline [qui peut être préparée selon le brevet US 4,416,884 (1983)] dans 9 mL de N,N-diméthylformamide est ajoutée rapidement. Le mélange réactionnel est agité à température ambiante pendant 16 heures, puis concentré sous pression réduite. Le résidu est repris dans un mélange de 32 mL d'eau et 15 mL d'acétate d'éthyle, puis agité à température ambiante pendant 3 heures. Le précipité formé est filtré, puis rincé successivement à l'eau, l'éther diisopropylique et l'éther diéthylique. Le solide obtenu est essoré, puis séché sous pression réduite à 40°C. On obtient ainsi 485 mg de 2-[2-(4-chloro-2-méthyl-2,3-dihydro-1H-indol-1-yl)-2-oxoéthyl]-6-(morpholin-4-yl)pyrimidin-4(3H)-one sous forme d'une poudre cristalline blanche dont les caractéristiques sont les suivantes:
Spectre RMN 1H (400MHz) : 1,30 (d, *J*=6,4 Hz, 3 H) ; 2,73 (d, *J*=16,6 Hz, 1 H) ; 3,34 à 3,44 (m, 5 H) ; 3,60 (m, 4 H) ; 3,75 (d, *J*=16,1 Hz, 1 H) ; 3,93 (d, *J*=16,1 Hz, 1 H) ; 4,77 (m, 1 H) ; 5,21 (s, 1 H) ; 7,12 (d, *J*=7,8 Hz, 1 H) ; 7,24 (t, *J*=7,8 Hz, 1 H) ; 7,92 (d large, *J*=7,8 Hz, 1 H) ; 11,69 (m étalé, 1 H)
Spectrométrie de Masse : méthode A
Temps de rétention Tr (min) = 0,82 ;
[M+H]+ : m/z 389 ; [M-H]- : m/z 387
Point de fusion (Kofler) : 232°C

### Exemple 2c et Exemple 3c : Séparation de la (+)-2-{2-[-4-chloro-2-méthyl-2,3-dihydro-1H-indol-1-yl]-2-oxoéthyl}-6-(morpholin-4-yl)pyrimidin-4(3H)-one et de la (-)-2-{2-[-4-chloro-2-méthyl-2,3-dihydro-1H-indol-1-yl]-2-oxoéthyl}-6-(morpholin-4-yl)pyrimidin-4(3H)-one

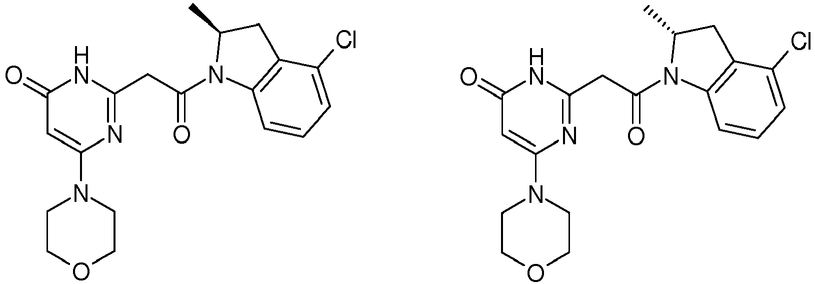

Les produits ont été obtenus par séparation chromatographique chirale de 428 mg de 2-[2-(4-chloro-2-méthyl-2,3-dihydro-1H-indol-1-yl)-2-oxoéthyl]-6-(morpholin-4-yl)pyrimidin-4(3H)-one (exemple 1c) sur colonne Whelk 01 SS (10 µm lot mixte, 7.5/40 cm), éluant : heptane/méthanol/éthanol/triéthylamine : 75/20/5/0.1 ; débit :300 mL/min. Après purification, on obtient comme premier énantiomère, 186 mg de (+)-2-[2-(4-chloro-2-méthyl-2,3-dihydro-1H-indol-1-yl)-2-oxoéthyl]-6-(morpholin-4-yl)pyrimidin-4(3H)-one, sous forme d'une poudre blanche dont les caractéristiques sont les suivantes:
Spectre RMN 1H (400MHz): 1,30 (d large, *J*=6,4 Hz, 3 H) ; 2,72 (d, *J*=16,3 Hz, 1 H) ; 3,33 à 3,44 (m, 5 H) ; 3,60 (m, 4 H) ; 3,74 (d, *J*=15,9 Hz, 1 H) ; 3,93 (d, *J*=15,9 Hz, 1 H) ; 4,78 (m, 1 H) ; 5,20 (s, 1 H) ; 7,12 (d, *J*=8,1 Hz, 1 H) ; 7,24 (t, *J*=8,1 Hz, 1 H) ; 7,92 (d large, *J*=8,1 Hz, 1 H) ; 11,68 (m étalé, 1 H)
Spectrométrie de Masse : méthode A
Temps de rétention Tr (min) = 0,82 ;
[M+H]+ : m/z 389 ; [M-H]- : m/z 387
Pouvoir rotatoire : α_{D} = +98° +/-2 (c = 0,24% dans le DMSO)

Puis le deuxième énantiomère, soit : 206 mg de (-)-2-[2-(4-chloro-2-méthyl-2,3-dihydro-1H-indol-1-yl)-2-oxoéthyl]-6-(morpholin-4-yl)pyrimidin-4(3H)-one sous forme d'une poudre blanche dont les caractéristiques sont les suivantes :
Spectre RMN 1H (400MHz): 1,30 (d large, *J*=6,4 Hz, 3 H) ; 2,73 (d, *J*=16,4 Hz, 1 H) ; 3,33 à 3,44 (m, 5 H) ; 3,60 (m, 4 H); 3,74 (d, *J*=16,1 Hz, 1 H) ; 3,93 (d, *J*=16,1 Hz, 1 H) ; 4,78 (m, 1 H); 5,20 (s, 1 H) ; 7,12 (d, *J*=8,2 Hz, 1 H) ; 7,24 (t, *J*=8,2 Hz, 1 H) ; 7,92 (d large, *J*=8,2 Hz, 1 H); 11,69 (m étalé, 1 H)
Spectrométrie de Masse : méthode A
Temps de rétention Tr (min) = 0,82 ;
[M+H]+ : m/z 389 ; [M-H]- : m/z 387
Pouvoir rotatoire : α_{D} = -85° +/-2 (c = 0,18% dans le DMSO)

### Exemple 4c: Synthèse du 2-[2-(4-chloro-3,3-diméthyl-2,3-dihydro-1H-indol-1-yl)-2-oxoéthyl]-3-méthyl-6-(morpholin-4-yl)pyrimidin-4(3H)-one

### Etape 1c:

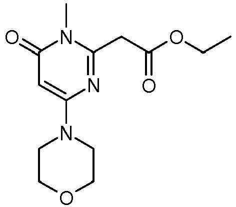

A une solution de 500 mg de [4-(morpholin-4-yl)-6-oxo-1,6-dihydropyrimidin-2-yl]acétate d'éthyle (préparé à l'étape 1c de l'exemple 1c) dans 1.5 mL de dioxanne, 330 mg de carbonate de potassium et 150 mL d'iodure de méthyle. Le mélange réactionnel est chauffé à 40 °C pendant 16 puis refroidi à température ambiante. La suspension est filtrée sur verre fritté puis rincée avec du dioxanne et le filtrat est concentré sous pression réduite. Le résidu est purifié par chromatographie sur colonne de silice, en éluant avec un mélange de dichlorométhane, d'acétonitrile et de méthanol (98/01/01, 96/02/02 puis, 90/05/05 V/V/V). On obtient 200 mg de [1-méthyl-4-(morpholin-4-yl)-6-oxo-1,6-dihydropyrimidin-2-yl]acétate d'éthyle sous forme de solide blanc dont les caractéristiques sont les suivantes :
Spectre RMN 1H (400MHz) : 1,21 (t, *J*=7,1 Hz, 3 H) ; 3,29 (m partiellement masqué, 3 H) ; 3,40 (m, 4 H) ; 3,61 (m, 4 H) ; 3,92 (s, 2 H) ; 4,15 (q, J=7,1 Hz, 2 H) ; 5,35 (s, 1 H)
Spectrométrie de Masse : méthode A
Temps de rétention Tr (min) = 0,53 ;
[M+H]+ : m/z 282 ; [M-H]- : m/z 280;

### Etape 2c:

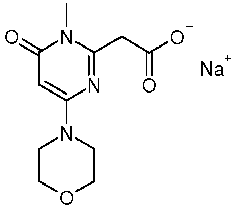

A une solution de 1.62 g de [1-méthyl-4-(morpholin-4-yl)-6-oxo-1,6-dihydropyrimidin-2-yl]acétate d'éthyle, dans 20 mL de tétrahydrofurane, sont ajoutés 2.88 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif. On obtient 730 mg de [1-méthyl-4-(morpholin-4-yl)-6-oxo-1,6-dihydropyrimidin-2-yl]acétate de sodium sous forme de solide blanc dont les caractéristiques sont les suivantes:
Spectre RMN 1 H (400MHz) : 3,27 à 3,43 (m partiellement masqué, 9 H) ; 3,61 (m, 4 H) ; 5,23 (s, 1 H)
Spectrométrie de Masse : méthode A
Temps de rétention Tr (min) = 0,31 ;
[M+H]+ : m/z 254 ; [M-H]- : m/z 252;

### Etape 3c:

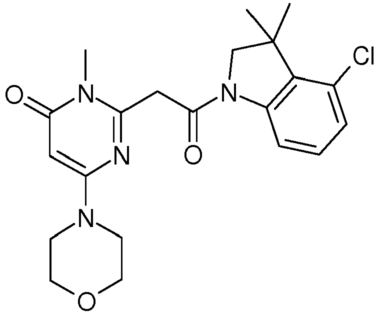

A une solution de 30 mg de [1-méthyl-4-(morpholin-4-yl)-6-oxo-1,6-dihydropyrimidin-2-yl]acétate de sodium dans 0,5 ml de N,N-diméthylformamide et 0,5 ml de pyridine sont ajoutés 20 mg de 4-chloro-3,3-dimethyl-2,3-dihydro-1H-indole [qui peut être préparé selon Tet. Let. (1987) (28), 5291-5294] et 34 mg de N-[3-(diméthylamino)propyl]-N'-éthylcarbodiimide chlorhydrate. Le mélange réactionnel est agité à température ambiante pendant 16 heures, puis 10 mL d'eau sont ajoutés et le mélange est extrait avec de l'acétate d'éthyle. La phase organique est lavée successivement avec de l'eau et une solution saturée de chlorure de sodium, séchée sur sulfate de magnésium, filtrée et concentrée sous pression réduite. Le résidu est purifié par chromatographie sur colonne de silice, en éluant avec de l'acétate d'éthyle pour donner 12 mg de 2-[2-(4-chloro-3,3-diméthyl-2,3-dihydro-1H-indol-1-yl)-2-oxoéthyl]-3-méthyl-6-(morpholin-4-yl)pyrimidin-4(3H)-one dont les caractéristiques sont les suivantes:
Spectre RMN 1 H (400MHz): 1,46 (s, 6 H) ; 3,26 à 3,45 (m partiellement masqué, 7 H) ; 3,56 (m, 4 H) ; 3,97 (s, 2 H) ; 4,12 (s, 2 H) ; 5,37 (s, 1 H) ; 7,06 (d, J=7,9 Hz, 1 H) ; 7,22 (t, J=7,9 Hz, 1 H) ; 8,06 (d, J=7,9 Hz, 1 H)
Spectrométrie de Masse : méthode A
Temps de rétention Tr (min) = 0,95 ;
[M+H]+ : m/z 417 ; [M-H]- : m/z 415

### Exemple 5c: Synthèse du 2-[2-(4-chloro-3,3-diméthyl-2,3-dihydro-1H-indol-1-yl)-2-oxoéthyl]-6-(morpholin-4-yl)pyrimidin-4(3H)-one

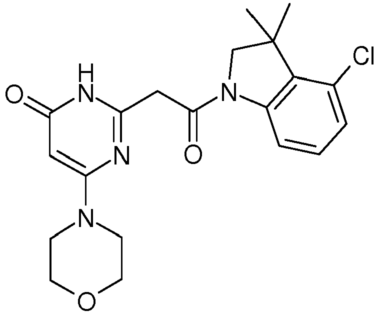

A une solution de 187 mg de [4-(morpholin-4-yl)-6-oxo-1,6-dihydropyrimidin-2-yl]acétate de sodium (obtenu à l'étape 2c de l'Exemple 1c) dans 3 ml de N,N-diméthylformamide et 3 ml de pyridine sont ajoutés 130 mg de 4-chloro-3,3-dimethyl-2,3-dihydro-1H-indole [qui peut être préparé selon Tet. Let. (1987) (28), 5291-5294] et 220 mg de N-[3-(diméthylamino)propyl]-N'-éthylcarbodiimide chlorhydrate. Le mélange réactionnel est agité à température ambiante pendant 16 heures, puis 15 mL d'eau sont ajoutés et le mélange est extrait avec de l'acétate d'éthyle. La phase organique est lavée successivement avec de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane et de méthanol (95/05 : v/v) pour donner 24 mg de 2-[2-(4-chloro-3,3-diméthyl-2,3-dihydro-1H-indol-1-yl)-2-oxoéthyl]-6-(morpholin-4-yl)pyrimidin-4(3H)-one dont les caractéristiques sont les suivantes:
Spectre RMN 1 H (400MHz): 1,48 (s, 6 H) ; 3,43 (m, 4 H) ; 3,62 (m, 4 H) ; 3,79 (s, 2 H) ; 3,97 (s, 2 H) ; 5,23 (s, 1 H) ; 7,07 (d, J=8,0 Hz, 1 H) ; 7,24 (t, J=8,0 Hz, 1 H) ; 8,07 (d, J=8,0 Hz, 1 H) ; 11,65 (s, 1 H)
Spectrométrie de Masse : méthode A
Temps de rétention Tr (min) = 0,90 ;
[M+H]+ : m/z 403 ; [M-H]- : m/z 401

### Exemple 6c : Synthèse de la 2-[2-(4-hydroxy-2-méthyl-2,3-dihydro-1H-indol-1-yl)-2-oxoéthyl]-6-(morpholin-4-yl)pyrimidin-4(3H)-one

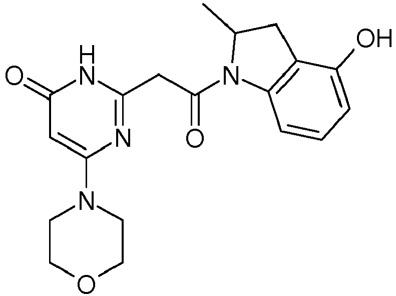

Le produit est préparé en suivant le mode opératoire décrit à l'exemple 1c à partir de 1,2 g de [4-(morpholin-4-yl)-6-oxo-1,6-dihydropyrimidin-2-yl]acétate de sodium, de 0,82 g de 2-méthyl-2,3-dihydro-1H-indol-4-ol [Exemple de référence 1c], de 1,17 g de N-[3-(diméthylamino)propyl]-N'-éthylcarbodiimide chlorhydrate dans un mélange de 1,1 mL de pyridine et de 25 mL de N,N-diméthylformamide. Après purification du résidu par chromatographie sur une cartouche de 90 g silice 15-40 µm, en éluant avec du dichlorométhane pur puis avec des mélanges dichlorométhane/ méthanol 98/2 puis 95/5 v/v, à un débit de 80 mL/ min, on obtient 0,84 g de 2-[2-(4-hydroxy-2-méthyl-2,3-dihydro-1H-indol-1-yl)-2-oxoéthyl]-6-(morpholin-4-yl)pyrimidin-4(3H)-one sous forme d'une poudre rose très pâle dont les caractéristiques sont les suivantes :
Spectre RMN 1H (400MHz) : 1,26 (d, *J*=6,4 Hz, 3 H) ; 2,60 (d, *J*=15,4 Hz, 1 H) ; 3,16 (dd, *J*=8,6 et 15,4 Hz, 1 H) ; 3,41 (m, 4 H) ; 3,60 (m, 4H) ; 3,69 (d, *J*=15,7 Hz, 1 H) ; 3,89 (d, *J*=15,7 Hz, 1 H) ; 4,69 (m, 1 H) ; 5,20 (s, 1 H) ; 6,53 (d, *J*=8,1 Hz, 1 H) ; 6,99 (t, *J*=8,1 Hz, 1 H) ; 7,45 (d, *J*=8,1 Hz, 1 H) ; 9,44 (s, 1 H) ; 11,66 (s, 1 H)
Spectrométrie de Masse : méthode A
Temps de rétention Tr (min) = 0,52 ;
[M+H]+ : m/z 371 ; [M-H]- : m/z 369
Point de fusion (Kofler) : 254°C

### Exemple 7c: Synthèse du 2-[2-(4-fluoro-3,3-diméthyl-2,3-dihydro-1H-indol-1-yl)-2-oxoéthyl]-3-méthyl-6-(morpholin-4-yl)pyrimidin-4(3H)-one

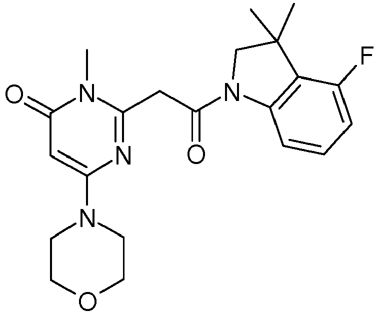

A une solution de 300 mg de [1-méthyl-4-(morpholin-4-yl)-6-oxo-1,6-dihydropyrimidin-2-yl]acétate de sodium (Obtenu à l'étape 2c de l'Exemple 4c) dans 5 ml de N,N-diméthylformamide et 5 ml de pyridine sont ajoutés 150 mg de 4-fluoro-3,3-dimethyl-2,3-dihydro-1H-indole [Exemple de référence 2c] et 279 mg de N-[3-(diméthylamino)propyl]-N'-éthylcarbodiimide chlorhydrate. Le mélange réactionnel est agité à température ambiante pendant 16 heures, puis 30 mL d'eau sont ajoutés et le mélange est extrait avec de l'acétate d'éthyle. La phase organique est lavée successivement avec de l'eau et une solution saturée de chlorure de sodium, séchée sur sulfate de magnésium, filtrée et concentrée sous pression réduite. Le résidu est purifié par trituration dans un mélange d'acétate d'éthyle et d'éther diisopropylique pour donner 260 mg de 2-[2-(4-fluoro-3,3-diméthyl-2,3-dihydro-1H-indol-1-yl)-2-oxoéthyl]-3-méthyl-6-(morpholin-4-yl)pyrimidin-4(3H)-one dont les caractéristiques sont les suivantes:
Spectre RMN 1 H (400MHz): 1,42 (s, 6 H) ; 3,30 (m partiellement masqué, 3 H) ; 3,38 (m, 4 H) ; 3,57 (m, 4 H) ; 3,98 (s, 2 H) ; 4,11 (s, 2 H) ; 5,37 (s, 1 H) ; 6,86 (t, J=8,2 Hz, 1 H) ; 7,23 (dt, J=5,7 et 8,2 Hz, 1 H) ; 7,87 (d, J=8,2 Hz, 1 H)
Spectrométrie de Masse : méthode A
Temps de rétention Tr (min) = 0,88 ;
[M+H]+ : m/z 401 ; [M-H]- : m/z 399

### Exemple 8c: Synthèse du 2-[2-(4-fluoro-3,3-diméthyl-2,3-dihydro-1H-indol-1-yl)-2-oxoéthyl]-6-(morpholin-4-yl)pyrimidin-4(3H)-one

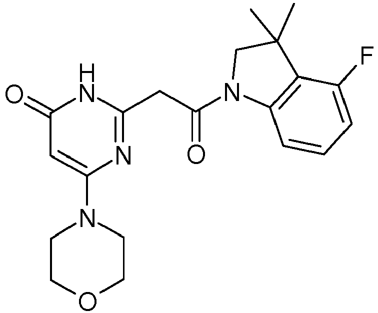

A une solution de 261 mg de [1-méthyl-4-(morpholin-4-yl)-6-oxo-1,6-dihydropyrimidin-2-yl]acétate de sodium (Obtenu à l'étape 2c de l'Exemple 1c) dans 5 ml de N,N-diméthylformamide et 5 ml de pyridine sont ajoutés 150 mg de 4-fluoro-3,3-dimethyl-2,3-dihydro-1H-indole [Exemple de reference 2c] et 279 mg de N-[3-(diméthylamino)propyl]-N'-éthylcarbodiimide chlorhydrate. Le mélange réactionnel est agité à température ambiante pendant 16 heures, puis 30 mL d'eau sont ajoutés et le mélange est extrait avec de l'acétate d'éthyle. La phase organique est lavée successivement avec de l'eau et une solution saturée de chlorure de sodium, séchée sur sulfate de magnésium, filtrée et concentrée sous pression réduite. Le résidu est purifié par trituration dans un mélange hd'acétate d'étyle et d'éther diisopropylique pour donner 194 mg de 2-[2-(4-fluoro-3,3-diméthyl-2,3-dihydro-1H-indol-1-yl)-2-oxoéthyl]-6-(morpholin-4-yl)pyrimidin-4(3H)-one dont les caractéristiques sont les suivantes:
Spectre RMN 1 H (400MHz): 1,42 (s, 6 H) ; 3,42 (m, 4 H) ; 3,60 (m, 4 H) ; 3,76 (s, 2 H) ; 3,96 (s, 2 H) ; 5,21 (s, 1 H) ; 6,85 (t, J=8,3 Hz, 1 H) ; 7,23 (dt, J=5,7 et 8,3 Hz, 1 H) ; 7,86 (d, J=8,3 Hz, 1 H) ; 11,62 (s large, 1 H)
Spectrométrie de Masse : méthode A
Temps de rétention Tr (min) = 0,83 ;
[M+H]+ : m/z 387 ; [M-H]- : m/z 385

### Exemple 9c et Exemple 10c : Séparation de la (+)-2-{2-[-4-hydroxy-2-méthyl-2,3-dihydro-1H-indol-1-yl]-2-oxoéthyl}-6-(morpholin-4-yl)pyrimidin-4(3H)-one et de la (-)-2-{2-[-4-hydroxy-2-méthyl-2,3-dihydro-1H-indol-1-yl]-2-oxoéthyl}-6-(morpholin-4-yl)pyrimidin-4(3H)-one

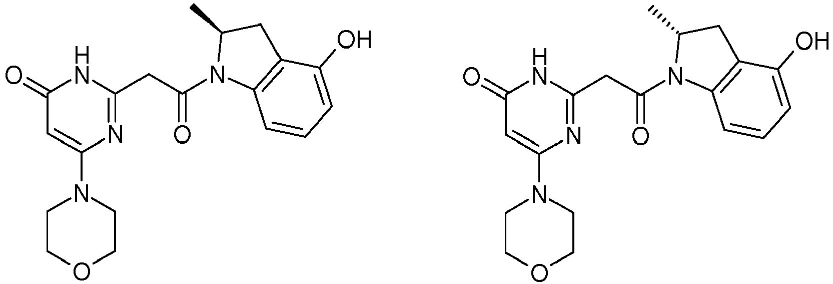

Les produits ont été obtenus par séparation chromatographique chirale de 0,73 g de 2-[2-(4-hydroxy-2-méthyl-2,3-dihydro-1H-indol-1-yl)-2-oxoéthyl]-6-(morpholin-4-yl)pyrimidin-4(3H)-one (Exemple 4c) sur colonne Whelk 01 SS (1200 g, 10 µm, 8/35 cm), éluant : heptane/dichlorométhane/éthanol/méthanol : 65/25/5/5 ; débit : 200 mL/min. Après purification, on obtient comme premier énantiomère, 337 mg de (+)-2-[2-(4-hydroxy-2-méthyl-2,3-dihydro-1H-indol-1-yl)-2-oxoéthyl]-6-(morpholin-4-yl)pyrimidin-4(3H)-one, sous forme d'un solide blanc cassé dont les caractéristiques sont les suivantes:
Spectre RMN 1 H (400MHz): 1,25 (d, *J*=6,4 Hz, 3 H) ; 2,60 (d, *J*=16,1 Hz, 1 H) ; 3,16 (dd, *J*=8,3 et 16,1 Hz, 1 H) ; 3,41 (m, 4 H) ; 3,60 (m, 4 H) ; 3,69 (d, *J*=15,9 Hz, 1 H) ; 3,89 (d, *J*=15,9 Hz, 1 H) ; 4,69 (m, 1 H) ; 5,21 (s, 1 H) ; 6,52 (d, *J*=8,1 Hz, 1 H) ; 6,99 (t, *J*=8,1 Hz, 1 H) ; 7,44 (d, *J*=8,1 Hz, 1 H) ; 9,51 (s, 1 H) ; 11,71 (m étalé, 1 H)
Spectrométrie de Masse : méthode A
Temps de rétention Tr (min) = 0,52 ;
[M+H]+ : m/z 371 ; [M-H]- : m/z 369
Pouvoir rotatoire : α_{D} = +103,6° +/-1,8 (c = 0,32% dans le DMSO)

Puis le deuxième énantiomère, soit : 335 mg de (-)-2-[2-(4-hydroxy-2-méthyl-2,3-dihydro-1H-indol-1-yl)-2-oxoéthyl]-6-(morpholin-4-yl)pyrimidin-4(3H)-one sous forme d'un solide blanc cassé dont les caractéristiques sont les suivantes :
Spectre RMN 1 H (400MHz): 1,25 (d, *J*=6,4 Hz, 3 H) ; 2,60 (d, *J*=16,3 Hz, 1 H) ; 3,16 (dd, *J*=8,7 et 16,3 Hz, 1 H) ; 3,40 (m, 4 H) ; 3,60 (m, 4 H) ; 3,69 (d, *J*=15,7 Hz, 1 H) ; 3,89 (d, *J*=15,7 Hz, 1 H) ; 4,69 (m, 1 H) ; 5,21 (s, 1 H) ; 6,52 (d, *J*=8,1 Hz, 1 H) ; 6,99 (t, *J*=8,1 Hz, 1 H) ; 7,44 (d, *J*=8,1 Hz, 1 H) ; 9,50 (s, 1 H) ; 11,71 (m étalé, 1 H)
Spectrométrie de Masse : méthode A
Temps de rétention Tr (min) = 0,52 ;
[M+H]+ : m/z 371 ; [M-H]- : m/z 369
Pouvoir rotatoire : α_{D} = -71,8° +/-1,4 (c = 0,33% dans le DMSO)

### Exemple 11c et exemple 12c : Synthèse de la (+)-2-{2-[-4-hydroxy-3-méthyl-2,3-dihydro-1H-indol-1-yl]-2-oxoéthyl}-6-(morpholin-4-yl)pyrimidin-4(3H)-one et de la (-)-2-{2-[-4-hydroxy-3-méthyl-2,3-dihydro-1H-indol-1-yl]-2-oxoéthyl}-6-(morpholin-4-yl)pyrimidin-4(3H)-one

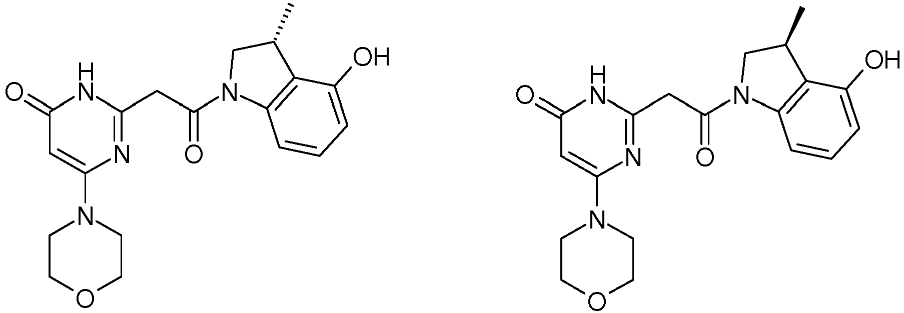

La (±)-2-{2-[4-hydroxy-3-méthyl-2,3-dihydro-1H-indol-1-yl]-2-oxoéthyl}-6-(morpholin-4-yl)pyrimidin-4(3H)-one est préparée en suivant le mode opératoire décrit à l'exemple 1c à partir de 0,70 g de [4-(morpholin-4-yl)-6-oxo-1,6-dihydropyrimidin-2-yl]acétate de sodium, de 0,44 g de 4-hydroxy-3-méthyl-indoline (Exemple de référence 3c), de 0,68 g de N-[3-(diméthylamino)propyl]-N'-éthylcarbodiimide chlorhydrate dans un mélange de 0,65 mL de pyridine et de 15 mL de N,N-diméthylformamide. On obtient ainsi 0,55 g de (±)-2-{2-[4-hydroxy-3-méthyl-2,3-dihydro-1H-indol-1-yl]-2-oxoéthyl}-6-(morpholin-4-yl)pyrimidin-4(3H)-one sous forme d'une poudre rose vif dont les caractéristiques sont les suivantes :
Spectrométrie de Masse : méthode A
Temps de rétention Tr (min) = 0,55 ;
[M+H]+ : m/z 371 ; [M-H]- : m/z 369

Les produits ont été obtenus par séparation chromatographique chirale de 530 mg de (±)-2-{2-[4-hydroxy-3-méthyl-2,3-dihydro-1H-indol-1-yl]-2-oxoéthyl}-6-(morpholin-4-yl)pyrimidin-4(3H)-one sur colonne Chiralpak AS (IK001) (1200 g, 20 µm, 8/35 cm), éluant : heptane/ éthanol/ méthanol : 60/20/20 ; débit :200 mL/min. Après purification, on obtient comme premier énantiomère, 189 mg de (+)-2-{2-[4-hydroxy-3-méthyl-2,3-dihydro-1H-indol-1-yl]-2-oxoéthyl}-6-(morpholin-4-yl)pyrimidin-4(3H)-one, sous forme d'un solide rosé dont les caractéristiques sont les suivantes:
Spectre RMN 1H (400MHz): 1,25 (d, *J*=6,8 Hz, 3 H) ; 3,41 (m, 4 H) ; 3,46 (m, 1 H) ; 3,60 (m, 4 H) ; 3,67 (d, *J*=15,9 Hz, 1 H) ; 3,72 (dd, *J*=4,3 et 10,3 Hz, 1 H) ; 3,77 (d, *J*=15,9 Hz, 1 H) ; 4,22 (t, *J*=10,3 Hz, 1 H) ; 5,20 (s, 1 H) ; 6,50 (d, *J*=8,3 Hz, 1 H) ; 6,97 (t, *J*=8,3 Hz, 1 H) ; 7,50 (d, *J*=8,3 Hz, 1 H) ; 9,45 (s large, 1 H) ; 11,58 (m étalé, 1 H)
Spectrométrie de Masse : méthode A
Temps de rétention Tr (min) = 0,54 ;
[M+H]+ : m/z 371 ; [M-H]- : m/z 369
Pouvoir rotatoire : α_{D} = +11° +/- 0.7 (c = 0,34 mg dans 0.5 mL de méthanol)

Puis le deuxième énantiomère, soit : 183 mg de (-)-2-{2-[4-hydroxy-3-méthyl-2,3-dihydro-1H-indol-1-yl]-2-oxoéthyl}-6-(morpholin-4-yl)pyrimidin-4(3H)-one sous forme d'un solide rose dont les caractéristiques sont les suivantes :
Spectre RMN 1H (400MHz): 1,25 (d, *J*=6,8 Hz, 3 H) ; 3,42 (m, 4 H) ; 3,47 (m, 1 H) ; 3,60 (m, 4 H) ; 3,67 (d, *J*=15,9 Hz, 1 H) ; 3,72 (dd, *J*=4,2 et 10,3 Hz, 1 H) ; 3,77 (d, *J*=15,9 Hz, 1 H) ; 4,22 (t, *J*=10,3 Hz, 1 H) ; 5,20 (s, 1 H) ; 6,50 (d, *J*=8,3 Hz, 1 H) ; 6,97 (t, *J*=8,3 Hz, 1 H) ; 7,50 (d, *J*=8,3 Hz, 1 H) ; 9,46 (s large, 1 H) ; 11,58 (m étalé, 1 H)
Spectrométrie de Masse : méthode A
Temps de rétention Tr (min) = 0,54 ;
[M+H]+ : m/z 371 ; [M-H]- : m/z 369
Pouvoir rotatoire : α_{D} = -2.6° +/- 0.5 (c = 0,38 mg dans 0.5 mL de méthanol)

### Exemple 13c et Exemple 14c: Synthèse du (+)-2-{2-[-5-fluoro-2-méthyl-2,3-dihydro-1H-indol-1-yl]-2-oxoéthyl}-6-(morpholin-4-yl)pyrimidin-4(3H)-one et du (-)-2-{2-[-5-fluoro-2-méthyl-2,3-dihydro-1H-indol-1-yl]-2-oxoéthyl}-6-(morpholin-4-yl)pyrimidin-4(3H)-one

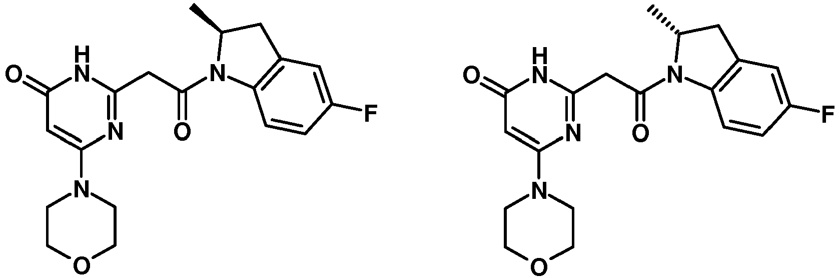

A A une solution de 743 mg de [4-(morpholin-4-yl)-6-oxo-1,6-dihydropyrimidin-2-yl]acétate de sodium (obtenu à l'étape 2c de l'Exemple 1c) dans 25 ml de N,N-diméthylformamide et 25 ml de pyridine sont ajoutés 430 mg de 5-fluoro-2-méthyl-2,3-dihydro-1H-indole (Exemple de référence 4c étape 1c) et 872 mg de N-[3-(diméthylamino)propyl]-N'-éthylcarbodiimide chlorhydrate. Le mélange réactionnel est agité à température ambiante pendant 48 heures, puis 100 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 580 mg de 2-{2-[-5-fluoro-2-méthyl-2,3-dihydro-1H-indol-1-yl]-2-oxoéthyl}-6-(morpholin-4-yl)pyrimidin-4(3H)-one.

Les énantiomères sont séparés par chromatographie chirale sur colonne Whelk 01 SS, 10 µm, (10 µm, 80x350 mm), en éluant avec un mélange de : heptane/dichlorométhane/ethanol/Methanol : 70/20/5/5 ; débit : 200 mL/min.

On obtient comme premier énantiomère, 252 mg de (+)-2-{2-[-5-fluoro-2-méthyl-2,3-dihydro-1H-indol-1-yl]-2-oxoéthyl}-6-(morpholin-4-yl)pyrimidin-4(3H)-one, sous forme d'un solide blanc dont les caractéristiques sont les suivantes:
Spectre RMN 1H (400MHz): 1,26 (d, *J*=6,4 Hz, 3 H) ; 2,70 (m, 1 H) ; 3,34 à 3,48 (m, 5 H) ; 3,60 (m, 4 H) ; 3,71 (d, *J*=15,9 Hz, 1 H) ; 3,91 (d, *J*=15,9 Hz, 1 H) ; 4,73 (m, 1 H) ; 5,20 (s, 1 H) ; 7,00 (dt, *J*=3,0 et 9,1 Hz, 1 H) ; 7,15 (dd, *J*=3,0 et 9,1 Hz, 1 H) ; 7,95 (dd, *J*=5,0 et 9,1 Hz, 1 H) ; 11,65 (m étalé, 1 H)
Spectrométrie de Masse : méthode A
Temps de rétention Tr (min) = 0,72 ;
[M+H]+ : m/z 373 ; [M-H]- : m/z 371 ;
Pouvoir rotatoire : α_{D} = +60,4° (c=1,939 mg/0,5 mL CH3OH)

Puis le deuxième énantiomère, 246 mg de (-)-2-{2-[-5-fluoro-2-méthyl-2,3-dihydro-1H-indol-1-yl]-2-oxoéthyl}-6-(morpholin-4-yl)pyrimidin-4(3H)-one sous forme d'un solide blanc dont les caractéristiques sont les suivantes :
Spectre RMN 1H (400MHz): 1,26 (d, *J*=6,5 Hz, 3 H) ; 2,69 (m, 1 H) ; 3,35 à 3,47 (m, 5 H) ; 3,60 (m, 4 H) ; 3,71 (d, J=15,9 Hz, 1 H) ; 3,91 (d, J=15,9 Hz, 1 H) ; 4,72 (m, 1 H) ; 5,20 (s, 1 H) ; 7,00 (td, J=3,0 et 9,1 Hz, 1 H) ; 7,15 (dd, J=3,0 et 9,1 Hz, 1 H) ; 7,95 (dd, J=5,0 et 9,1 Hz, 1 H) ; 11,66 (m étalé, 1 H)
Spectrométrie de Masse : méthode A
Temps de rétention Tr (min) = 0,72 ;
[M+H]+ : m/z 373 ; [M-H]- : m/z 371 ;
Pouvoir rotatoire : α_{D} = -57.9°+/-1.1. (c=1.833mg/0.5ML CH3OH)

### Exemple 15c et Exemple 16c : Synthèse du (+)-2-{2-[-5-fluoro-2-méthyl-2,3-dihydro-1H-indol-1-yl]-2-oxoéthyl}-3-méthyl-6-(morpholin-4-yl)pyrimidin-4(3H)-one et du (-)-2-{2-[-5-fluoro-2-méthyl-2,3-dihydro-1H-indol-1-yl]-2-oxoéthyl}-3-méthyl-6-(morpholin-4-yl)pyrimidin-4(3H)-one

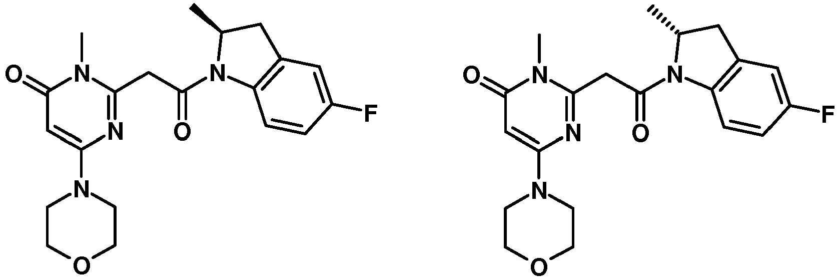

A une solution de 1,1 g de [1-méthyl-4-(morpholin-4-yl)-6-oxo-1,6-dihydropyrimidin-2-yl]acétate de sodium dans 25 ml de N,N-diméthylformamide et 25 ml de pyridine sont ajoutés 430 mg de 5-fluoro-2-méthyl-2,3-dihydro-1H-indole (Exemple de référence 4c) et 872 mg de N-[3-(diméthylamino)propyl]-N'-éthylcarbodiimide chlorhydrate. Le mélange réactionnel est agité à température ambiante pendant 48 heures, puis 100 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 538 mg de 2-{2-[(5-fluoro-2-méthyl-2,3-dihydro-1H-indol-1-yl]-2-oxoéthyl}-3-méthyl-6-(morpholin-4-yl)pyrimidin-4(3H)-one

Les énantiomères sont séparés par chromatographie chirale sur colonne Whelk 01 SS, 10 µm, (10 µm, 80x350 mm), en éluant avec un mélange de : heptane/dichlorométhane/ethanol/Methanol : 60/20/10/10 ; débit : 240 mL/min.

On obtient comme premier énantiomère, 212 mg de (+)-2-{2-[(5-fluoro-2-méthyl-2,3-dihydro-1H-indol-1-yl]-2-oxoéthyl}-3-méthyl-6-(morpholin-4-yl)pyrimidin-4(3H)-one, sous forme d'un solide blanc dont les caractéristiques sont les suivantes:
Spectre RMN 1 H (400MHz): 1,28 (d, *J*=6,6 Hz, 3 H) ; 2,70 (d, *J*=16,2 Hz, 1 H); 3,33 (s, 3 H) ; 3,36 à 3,44 (m, 5 H) ; 3,58 (m, 4 H) ; 4,02 (d, *J*=16,6 Hz, 1 H) ; 4,27 (d, *J*=16,6 Hz, 1 H) ; 4,73 (m, 1 H) ; 5,36 (s, 1 H) ; 7,00 (td, *J*=2,9 et 8,9 Hz, 1 H) ; 7,16 (dd, *J*=2,9 et 8,9 Hz, 1 H) ; 7,94 (dd, *J*=5,0 et 8,9 Hz, 1 H)
Spectrométrie de Masse : méthode A
Temps de rétention Tr (min) = 0,76 ;
[M+H]+ : m/z 387 ; [M-H]- : m/z 385;
Pouvoir rotatoire : α_{D} = +72,0° (c=1,704 mg/0,5 mL CH3OH)

Puis le deuxième énantiomère (TR =17,61 min), 210 mg de (-)-2-{2-[(5-fluoro-2-méthyl-2,3-dihydro-1H-indol-1-yl]-2-oxoéthyl}-3-méthyl-6-(morpholin-4-yl)pyrimidin-4(3H)-one sous forme d'un solide blanc dont les caractéristiques sont les suivantes :
Spectre RMN 1 H (400MHz): 1,28 (d, *J*=6,4 Hz, 3 H) ; 2,70 (d, *J*=16,2 Hz, 1 H); 3,33 (s, 3 H) ; 3,37 à 3,44 (m, 5 H) ; 3,59 (m, 4 H) ; 4,02 (d, *J*=16,6 Hz, 1 H) ; 4,27 (d, *J*=16,6 Hz, 1 H) ; 4,72 (m, 1 H) ; 5,36 (s, 1 H) ; 7,00 (td, *J*=3,0 et 8,8 Hz, 1 H) ; 7,16 (dd, *J*=3,0 et 8,8 Hz, 1 H) ; 7,93 (dd, J=5,1 et 8,8 Hz, 1 H)
Spectrométrie de Masse : méthode A
Temps de rétention Tr (min) = 0,76 ;
[M+H]+ : m/z 387 ; [M-H]- : m/z 385;
Pouvoir rotatoire : α_{D} = -59,5° (c=2,182 mg/0,5 mL CH3OH)

### Exemple 17c et Exemple 18c: Synthèse du (+)-2-{2-[-4,5-difluoro-2-méthyl-2,3-dihydro-1H-indol-1-yl]-2-oxoéthyl}-6-(morpholin-4-yl)pyrimidin-4(3H)-one et du du (-)-2-{2-[-4,5-difluoro-2-méthyl-2,3-dihydro-1H-indol-1-yl]-2-oxoéthyl}-6-(morpholin-4-yl)pyrimidin-4(3H)-one

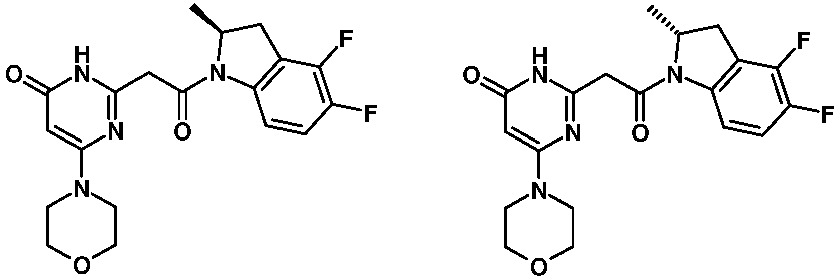

A une solution de 278 mg de [4-(morpholin-4-yl)-6-oxo-1,6-dihydropyrimidin-2-yl]acétate de sodium (obtenu à l'étape 2c de l'Exemple 1c) dans 10 ml de N,N-diméthylformamide et 10 ml de pyridine sont ajoutés 180 mg de 4,5-difluoro-2-méthyl-2,3-dihydro-1H-indole (Exemple de référence 5c) et 326 mg de N-[3-(diméthylamino)propyl]-N'-éthylcarbodiimide chlorhydrate. Le mélange réactionnel est agité à température ambiante pendant 16 heures, puis 50 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 230 mg de 2-{2-[-4,5-difluoro-2-méthyl-2,3-dihydro-1H-indol-1-yl]-2-oxoéthyl}-6-(morpholin-4-yl)pyrimidin-4(3H)-one.

Les énantiomères sont séparés par chromatographie chirale sur colonne Whelk 01 SS, 10 µm, (10 µm, 75x350 mm), en éluant avec un mélange de : heptane/dichlorométhane/ethanol/Methanol : 70/20/5/5 ; débit : 220 mL/min.

On obtient comme premier énantiomère, 100 mg de (+)-2-{2-[-4,5-difluoro-2-méthyl-2,3-dihydro-1H-indol-1-yl]-2-oxoéthyl}-6-(morpholin-4-yl)pyrimidin-4(3H)-one, sous forme d'un solide blanc dont les caractéristiques sont les suivantes:
Spectre RMN 1H (400MHz): 1,30 (d, *J*=6,1 Hz, 3 H) ; 2,82 (d, *J*=16,1 Hz, 1 H) ; 3,36 à 3,46 (m, 5 H) ; 3,60 (m, 4 H) ; 3,73 (d, *J*=16,1 Hz, 1 H) ; 3,92 (d, *J*=16,1 Hz, 1 H) ; 4,81 (m, 1 H) ; 5,21 (s, 1 H) ; 7,25 (td, *J*=8,6 et 11,2 Hz, 1 H) ; 7,74 (d, *J*=8,6 Hz, 1 H) ; 11,71 (s large, 1 H)
Spectrométrie de Masse : méthode A
Temps de rétention Tr (min) = 0,77 ;
[M+H]+ : m/z 391 ; [M-H]- : m/z 389;
Pouvoir rotatoire : α_{D} = +81° (c=2,066 mg/1 mL DMSO)

Puis le deuxième énantiomère, 101 mg de (-)-2-{2-[-4,5-difluoro-2-méthyl-2,3-dihydro-1H-indol-1-yl]-2-oxoéthyl}-6-(morpholin-4-yl)pyrimidin-4(3H)-one sous forme d'un solide blanc dont les caractéristiques sont les suivantes :
Spectre RMN 1 H (400MHz): 1,30 (d, *J*=6,4 Hz, 3 H) ; 2,82 (d, *J*=16,9 Hz, 1 H) ; 3,37 à 3,47 (m, 5 H) ; 3,57 à 3,63 (m, 4 H) ; 3,73 (d, *J*=15,7 Hz, 1 H) ; 3,92 (d, *J*=15,7 Hz, 1 H) ; 4,81 (m, 1 H) ; 5,21 (s, 1 H) ; 7,26 (td, *J*=8,6 et 11,2 Hz, 1 H) ; 7,74 (dd, *J*=3,6 et 8,6 Hz, 1 H) ; 11,71 (s large, 1 H)
Spectrométrie de Masse : méthode A
Temps de rétention Tr (min) = 0,77 ;
[M+H]+ : m/z 391 ; [M-H]- : m/z 389;
Pouvoir rotatoire : α_{D} = -80° (c=2,438 mg/1 mL DMSO)

### Exemple 19c et Exemple 20c: Synthèse du (+)-2-{2-[-4,5-difluoro-2-méthyl-2,3-dihydro-1H-indol-1-yl]-2-oxoéthyl}-3-méthyl-6-(morpholin-4-yl)pyrimidin-4(3H)-one et du (-)-2-{2-[-4,5-difluoro-2-méthyl-2,3-dihydro-1H-indol-1-yl]-2-oxoéthyl}-3-méthyl-6-(morpholin-4-yl)pyrimidin-4(3H)-one

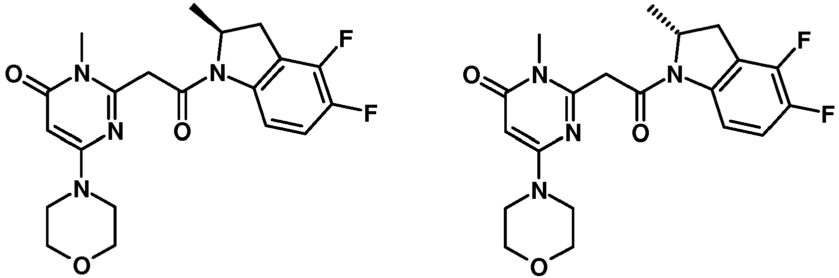

A une solution de 278 mg de [1-méthyl-4-(morpholin-4-yl)-6-oxo-1,6-dihydropyrimidin-2-yl]acétate de sodium (obtenu à l'étape 2c de l'Exemple 9c) dans 10 ml de N,N-diméthylformamide et 10 ml de pyridine sont ajoutés 180 mg de 4,5-difluoro-2-méthyl-2,3-dihydro-1H-indole [Exemple de référence 5c] et 326 mg de N-[3-(diméthylamino)propyl]-N'-éthylcarbodiimide chlorhydrate. Le mélange réactionnel est agité à température ambiante pendant 16 heures, puis 50 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 230 mg de 2-{2-[-4,5-difluoro-2-méthyl-2,3-dihydro-1H-indol-1-yl]-2-oxoéthyl}-3-méthyl-6-(morpholin-4-yl)pyrimidin-4(3H)-one.

Les énantiomères sont séparés par chromatographie chirale sur colonne Whelk 01 SS, 10 µm, (10 µm, 75x350 mm), en éluant avec un mélange de : heptane/dichlorométhane/ethanol/Methanol : 70/20/5/5 ; débit : 220 mL/min.

On obtient comme premier énantiomère, 100 mg de (+)-2-{2-[-4,5-difluoro-2-méthyl-2,3-dihydro-1H-indol-1-yl]-2-oxoéthyl}-3-méthyl-6-(morpholin-4-yl)pyrimidin-4(3H)-one, sous forme d'un solide blanc dont les caractéristiques sont les suivantes:
Spectre RMN 1H (400MHz): 1,30 (d, *J*=6,1 Hz, 3 H) ; 2,82 (d, *J*=16,1 Hz, 1 H) ; 3,36 à 3,46 (m, 5 H) ; 3,60 (m, 4 H) ; 3,73 (d, *J*=16,1 Hz, 1 H) ; 3,92 (d, *J*=16,1 Hz, 1 H) ; 4,81 (m, 1 H) ; 5,21 (s, 1 H) ; 7,25 (td, *J*=8,6 et 11,2 Hz, 1 H) ; 7,74 (d, *J*=8,6 Hz, 1 H) ; 11,71 (s large, 1 H)
Spectrométrie de Masse : méthode A
Temps de rétention Tr (min) = 0,77 ;
[M+H]+ : m/z 391 ; [M-H]- : m/z 389;
Pouvoir rotatoire : α_{D} = +81° (c=2,066 mg/1 mL de DMSO)

Puis le deuxième énantiomère, 101 mg de (-)-2-{2-[-4,5-difluoro-2-méthyl-2,3-dihydro-1H-indol-1-yl]-2-oxoéthyl}-3-méthyl-6-(morpholin-4-yl)pyrimidin-4(3H)-one sous forme d'un solide blanc dont les caractéristiques sont les suivantes :
Spectre RMN 1 H (400MHz): 1,30 (d, *J*=6,4 Hz, 3 H) ; 2,82 (d, *J*=16,9 Hz, 1 H) ; 3,37 à 3,47 (m, 5 H) ; 3,57 à 3,63 (m, 4 H) ; 3,73 (d, *J*=15,7 Hz, 1 H) ; 3,92 (d, *J*=15,7 Hz, 1 H) ; 4,81 (m, 1 H) ; 5,21 (s, 1 H) ; 7,26 (td, *J*=8,6 et 11,2 Hz, 1 H) ; 7,74 (dd, *J*=3,6 et 8,6 Hz, 1 H) ; 11,71 (s large, 1 H)
Spectrométrie de Masse : méthode A
Temps de rétention Tr (min) = 0,77 ;
[M+H]+ : m/z 391 ; [M-H]- : m/z 389;
Pouvoir rotatoire : α_{D} = -80° (c=2,438 mg/1 mL DMSO)

### Exemple 21c: Synthèse du 2-{2-[(+)-5,6-difluoro-2-méthyl-2,3-dihydro-1H-indol-1-yl]-2-oxoéthyl}-6-(morpholin-4-yl)pyrimidin-4(3H)-one

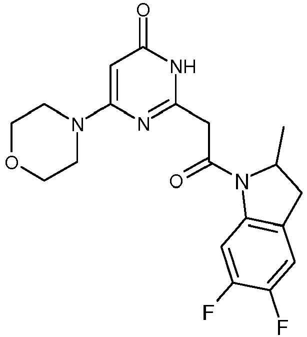

A une solution de 231 mg du (4-morpholin-4-yl-6-oxo-1,6-dihydro-pyrimidin-2-yl)-acetate de sodium (Obtenu à l'étape 2c de l'Exemple 1 c) dans 8 mL de DMF et 8 mL de pyridine sont ajoutés 150 mg de (-)-2-methyl-5,6-fluoro-2,3-dihydro-1H-indole [Exemple de référence 6c étape 6c] et 272 mg de chlorhydrate du N-[3-(diméthylamino)propyl]-N'-éthylcarbodiimide.

Le milieu réactionnel est agité à la température ambiante pendant 18 heures.

Addition de 50 mL d'acétate d'éthyle et 20 mL d'eau. Addition de l'acide chlorhydrique jusqu'au PH=5-6. Après décantation, la phase organique est séchée sur sulfate de magnésium, filtrée puis concentrée sous pression réduite. Le résidu obtenu est purifié sur colonne de silice, éluant : dichlorométhane/méthanol 98/02 puis 95/05 pour donner 170 mg de 2-[2-((+)-5,6-difluoro-2-methyl-2,3-dihydro-indol-1-yl)-2-oxo-ethyl]-6-morpholin-4-yl-3H-pyrimidin-4-one dont les caractéristiques sont les suivantes :
Spectre RMN 1H (400 MHz) : 1,27 (d, *J*=6,4 Hz, 3 H) ; 2,68 (d, *J*=17,1 Hz, 1 H) ; 3,36 (m partiellement masqué, 1 H) ; 3,40 (m, 4 H) ; 3,60 (m, 4 H) ; 3,73 (d, *J*=16,1 Hz, 1 H) ; 3,92 (d, *J*=16,1 Hz, 1 H) ; 4,76 (m, 1 H) ; 5,21 (s, 1 H) ; 7,39 (dd, *J*=8,7 et 9,8 Hz, 1 H) ; 7,90 (dd, *J*=7,5 et 12,1 Hz, 1 H) ; 11,70 (m étalé, 1 H)
Spectrométrie de Masse : méthode A
Temps de rétention Tr (min) = 0,76 ;
[M+H]+ : m/z 391 ; [M-H]- : m/z 389
Pouvoir rotatoire : α_{D} = +62.8+/-1.3 (C= 1.771 mg/0.5mL DMSO)

### Exemple 22c: Synthèse du 2-{2-[(-)-5,6-difluoro-2-méthyl-2,3-dihydro-1H-indol-1-yl]-2-oxoéthyl}-6-(morpholin-4-yl)pyrimidin-4(3H)-one

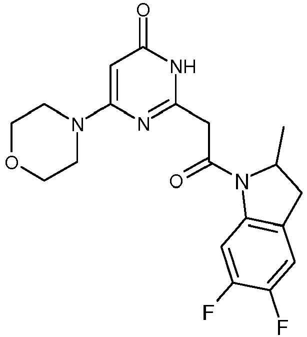

Le produit est préparé en suivant le mode opératoire décrit à l'exemple 21 c à partir de 231 mg du (4-morpholin-4-yl-6-oxo-1,6-dihydro-pyrimidin-2-yl)-acetate de sodium (Obtenu à l'étape 2c de l'Exemple 1c) et de 160 mg de (+)-2-methyl-5,6-fluoro-2,3-dihydro-1H-indole [Exemple de référence 6c étape 6c]. Après purification sur colonne de silice: éluant dichlorométhane/méthanol 98/02 puis 95/05, on obtient 247 mg du 2-[2-((-)-5,6-difluoro-2-methyl-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1H (400 MHz) : 1,27 (d, J=6,4 Hz, 3 H) ; 2,68 (d, *J*=17,1 Hz, 1 H) ; 3,35 (m partiellement masqué, 1 H) ; 3,41 (m, 4 H) ; 3,61 (m, 4 H) ; 3,73 (d, *J*=16,1 Hz, 1 H) ; 3,92 (d, *J*=16,1 Hz, 1 H) ; 4,77 (m, 1 H) ; 5,21 (s, 1 H) ; 7,39 (dd, *J*=8,7 et 9,8 Hz, 1 H) ; 7,90 (dd, *J*=7,5 et 12,1 Hz, 1 H) ; 11,68 (m étalé, 1 H)
Spectrométrie de Masse : méthode A
Temps de rétention Tr (min) = 0,76 ;
[M+H]+ : m/z 391 ; [M-H]- : m/z 389
Pouvoir rotatoire : α_{D} = -49.1+/-0.9 (C=2.157mg/0.5mL DMSO)

### Exemple 23c: Synthèse du 2-{2-[(-)-5,6-difluoro-2-méthyl-2,3-dihydro-1H-indol-1-yl]-2-oxoéthyl}-3-méthyl-6-(morpholin-4-yl)pyrimidin-4(3H)-one

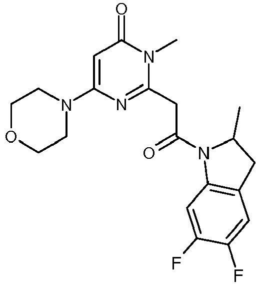

Le produit est préparé en suivant le mode opératoire décrit à l'exemple 21 c à partir de 304 mg du (1-methyl-4-morpholin-4-yl-6-oxo-1,6-dihydro-pyrimidin-2-yl)-acetate de sodium (Obtenu à l'étape 2c de l'Exemple 4c) et de 159 mg de (+)-2-methyl-5,6-fluoro-2,3-dihydro-1H-indole [Exemple de référence 6c étape 6c]. Après purification sur colonne de silice: éluant dichlorométhane/méthanol 98/02, on obtient 86 mg du 2-[2-((-)-5,6-difluoro-2-methyl-2,3-dihydro-indol-1-yl)-2-oxo-ethyl]-3-méthyl-6-morpholin-4-yl-3H-pyrimidin-4-one sous forme d'un solide dont les caractéristiques sont les suivantes :
Spectre RMN 1 H (400 MHz) : 1,29 (d, *J*=6,4 Hz, 3 H) ; 2,69 (d, *J*=16,3 Hz, 1 H) ; 3,33 (s, 3 H) ; 3,39 (m, 5 H) ; 3,60 (m, 4 H) ; 4,03 (d, *J*=16,9 Hz, 1 H) ; 4,28 (d, *J*=16,9 Hz, 1 H) ; 4,77 (m, 1 H) ; 5,36 (s, 1 H) ; 7,40 (dd, *J*=8,7 et 9,8 Hz, 1 H) ; 7,89 (dd, *J*=7,6 et 12,0 Hz, 1 H)
Temps de rétention Tr (min) = 0,81 ;
[M+H]+ : m/z 405 ; [M-H]- : m/z 403
Pouvoir rotatoire : α_{D} = -47.6+/-0.9 (C=2.131mg/0.5mL DMSO)

### Exemple 24c: Synthèse du 2-{2-[(+)-5,6-difluoro-2-méthyl-2,3-dihydro-1H-indol-1-yl]-2-oxoéthyl}-3-méthyl-6-(morpholin-4-yl)pyrimidin-4(3H)-one

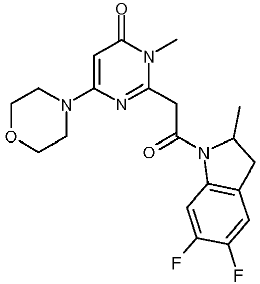

Le produit est préparé en suivant le mode opératoire décrit à l'exemple 21 c à partir de 315 mg du (1-methyl-4-morpholin-4-yl-6-oxo-1,6-dihydro-pyrimidin-2-yl)-acetate de sodium (Obtenu à l'étape 2c de l'Exemple 4c) et de 164 mg de (-)-2-methyl-5,6-fluoro-2,3-dihydro-1H-indole [Exemple de référence 6c étape 6c]. Après purification sur colonne de silice: éluant dichlorométhane/méthanol 98/02, on obtient 88 mg du 2-[2-((+)-5,6-difluoro-2-méthyl-2,3-dihydro-indol-1-yl)-2-oxo-ethyl]-3-méthyl-6-morpholin-4-yl-3H-pyrimidin-4-one sous forme d'un solide dont les caractéristiques sont les suivantes :
Spectre RMN 1 H (400 MHz) : 1,29 (d, *J*=6,6 Hz, 3 H) ; 2,69 (d, *J*=16,3 Hz, 1 H) ; 3,33 (s, 3 H) ; 3,39 (m, 5 H) ; 3,60 (m, 4 H) ; 4,03 (d, *J*=16,9 Hz, 1 H) ; 4,28 (d, *J*=16,9 Hz, 1 H) ; 4,77 (m, 1 H) ; 5,36 (s, 1 H) ; 7,40 (dd, J=8,7 et 9,8 Hz, 1 H) ; 7,89 (dd, J=7,5 et 12,1 Hz, 1 H)
Temps de rétention Tr (min) = 0,81 ;
[M+H]+ : m/z 405 ; [M-H]- : m/z 403
Pouvoir rotatoire : α_{D} = +25.9+/-0.8 (C= 2.011 mg/0.5mL DMSO)

### Exemple 25c et Exemple 26c: Synthèse du (+)-2-{2-[-4-bromo-2-méthyl-2,3-dihydro-1H-indol-1-yl]-2-oxoéthyl}-6-(morpholin-4-yl)pyrimidin-4(3H)-one et du du (-)-2-{2-[-4-bromo-2-méthyl-2,3-dihydro-1H-indol-1-yl]-2-oxoéthyl}-6-(morpholin-4-yl)pyrimidin-4(3H)-one

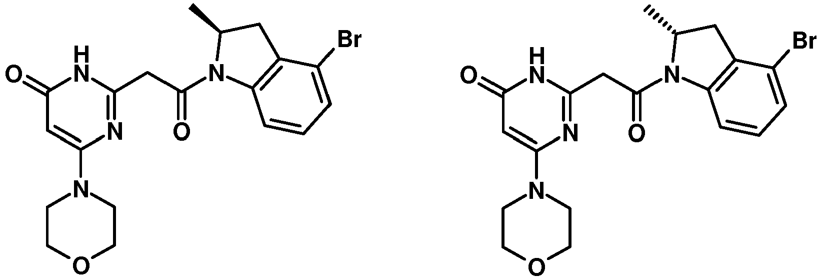

A une solution de 221 mg de [4-(morpholin-4-yl)-6-oxo-1,6-dihydropyrimidin-2-yl]acétate de sodium (obtenu à l'étape 2c de l'Exemple 1c) dans 7 ml de N,N-diméthylformamide et 7 ml de pyridine sont ajoutés 150 mg de 4-bromo-2-méthyl-2,3-dihydro-1H-indole [Exemple de référence 7c] et 217 mg de N-[3-(diméthylamino)propyl]-N'-éthylcarbodiimide chlorhydrate. Le mélange réactionnel est agité à température ambiante pendant 72 heures, puis 50 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 220 mg de 2-{2-[-4-bromo-2-méthyl-2,3-dihydro-1H-indol-1-yl]-2-oxoéthyl}-6-(morpholin-4-yl)pyrimidin-4(3H)-one.

Les énantiomères sont séparés par chromatographie chirale sur colonne Chiralpak T304 20µm, (20 µm, 77x350 mm), en éluant avec un mélange de : acetonitrile/isopropanol : 90/10 ; débit : 250 mL/min.

On obtient comme premier énantiomère, 94 mg de (+)-2-{2-[-4-bromo-2-méthyl-2,3-dihydro-1H-indol-1-yl]-2-oxoéthyl}-6-(morpholin-4-yl)pyrimidin-4(3H)-one, sous forme d'un solide blanc dont les caractéristiques sont les suivantes:
Spectre RMN 1H (400MHz): 1,30 (d, *J*=6,1 Hz, 3 H) ; 2,68 (m, 1 H) ; 3,27 à 3,45 (m partiellement masqué, 5 H) ; 3,61 (m, 4 H) ; 3,74 (d, *J*=15,2 Hz, 1 H) ; 3,93 (d, *J*=15,2 Hz, 1 H) ; 4,77 (m, 1 H) ; 5,20 (s, 1 H) ; 7,16 (t, *J*=7,8 Hz, 1 H) ; 7,27 (d, *J*=7,8 Hz, 1 H) ; 7,96 (d large, *J*=7,8 Hz, 1 H) ; 11,68 (m étalé, 1 H)
Spectrométrie de Masse : méthode A
Temps de rétention Tr (min) = 0,84 ;
[M+H]+ : m/z 433 ; [M-H]- : m/z 431;
Pouvoir rotatoire : α_{D} = +93,9° (c=1,536 mg/0,5 mL DMSO)

Puis le deuxième énantiomère, 95,5 mg de (-)-2-{2-[-4-bromo-2-méthyl-2,3-dihydro-1H-indol-1-yl]-2-oxoéthyl}-6-(morpholin-4-yl)pyrimidin-4(3H)-one sous forme d'un solide blanc dont les caractéristiques sont les suivantes :
Spectre RMN 1 H (400MHz): 1,30 (d, *J*=6,7 Hz, 3 H) ; 2,68 (m, 1 H) ; 3,25 à 3,45 (m partiellement masqué, 5 H) ; 3,60 (m, 4 H) ; 3,74 (d, *J*=16,2 Hz, 1 H) ; 3,93 (d, *J*=16,2 Hz, 1 H) ; 4,75 (m, 1 H) ; 5,20 (s, 1 H) ; 7,16 (t, *J*=7,8 Hz, 1 H) ; 7,26 (d, *J*=7,8 Hz, 1 H) ; 7,96 (d large, *J*=7,8 Hz, 1 H) ; 11,67 (m étalé, 1 H)
Spectrométrie de Masse : méthode A
Temps de rétention Tr (min) = 0,84 ;
[M+H]+ : m/z 433 ; [M-H]- : m/z 431;
Pouvoir rotatoire : α_{D} = -98° (c=0,714 mg/0,5 mL DMSO)

### Exemple 27c: Synthèse de la (-)-2-{2-[4-fluoro-2-méthyl-2,3-dihydro-1H-indol-1-yl]-2-oxoéthyl}-6-(morpholin-4-yl)pyrimidin-4(3H)-one

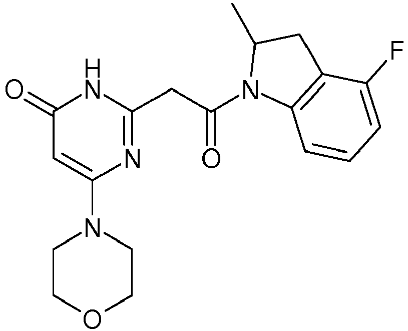

Le produit est préparé en suivant le mode opératoire décrit à l'exemple 1c à partir de 0,2 g de [4-(morpholin-4-yl)-6-oxo-1,6-dihydropyrimidin-2-yl]acétate de sodium, de 127 mg de (+)-4-fluoro-2-methyl-2,3-dihydro-1H-indole [Exemple de référence 8c étape 6c], de 0,20 g de N-[3-(diméthylamino)propyl]-N'-éthylcarbodiimide chlorhydrate dans un mélange de 123 µL de pyridine et de 3,5 mL de N,N-diméthylformamide. On obtient ainsi 0,15 g de (-)-2-{2-[4-fluoro-2-méthyl-2,3-dihydro-1H-indol-1-yl]-2-oxoéthyl}-6-(morpholin-4-yl)pyrimidin-4(3H)-one sous forme d'un solide blanc dont les caractéristiques sont les suivantes :
Spectre RMN 1 H (400MHz) : 1,29 (d large, *J*=6,4 Hz, 3 H) ; 2,75 (d, *J*=16,3 Hz, 1 H) ; 3,19 à 3,35 (m partiellement masqué, 1 H) ; 3,41 (m, 4 H) ; 3,60 (m, 4 H) ; 3,74 (d, *J*=16,1 Hz, 1 H) ; 3,93 (d, *J*=16,1 Hz, 1 H) ; 4,79 (m, 1 H) ; 5,21 (s, 1 H) ; 6,90 (t, *J*=8,6 Hz, 1 H) ; 7,24 (m, 1 H) ; 7,79 (d large, *J*=8,6 Hz, 1 H) ; 11,66 (m étalé, 1 H)
Spectrométrie de Masse : méthode A
Temps de rétention Tr (min) = 0,74 ;
[M+H]+ : m/z 373 ; [M-H]- : m/z 371
Point de fusion (Kofler) : supérieur à 260°C
Pouvoir rotatoire : α_{D} = -73,2° +/-1,4 (c = 1,834 mg dans 0,5 mL de méthanol)

### Exemple 28c: Synthèse de la (+)-2-{2-[4-fluoro-2-méthyl-2,3-dihydro-1H-indol-1-yl]-2-oxoéthyl}-6-(morpholin-4-yl)pyrimidin-4(3H)-one

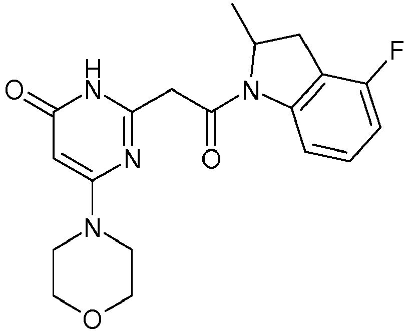

Le produit est préparé en suivant le mode opératoire décrit à l'exemple 1c à partir de 0,22 g de [4-(morpholin-4-yl)-6-oxo-1,6-dihydropyrimidin-2-yl]acétate de sodium, de 116 mg de (-)-4-fluoro-2-methyl-2,3-dihydro-1H-indole [Exemple de référence 8c étape 6c], de 0,19 g de N-[3-(diméthylamino)propyl]-N'-éthylcarbodiimide chlorhydrate dans un mélange de 123 µL de pyridine et de 3,5 mL de N,N-diméthylformamide. On obtient ainsi 0,15 g de (+)-2-{2-[4-fluoro-2-méthyl-2,3-dihydro-1H-indol-1-yl]-2-oxoéthyl}-6-(morpholin-4-yl)pyrimidin-4(3H)-one sous forme d'un solide blanc dont les caractéristiques sont les suivantes :
Spectre RMN 1 H (400MHz) : 1,29 (d large, *J*=6,5 Hz, 3 H) ; 2,76 (d, *J*=17,1 Hz, 1 H) ; 3,20 à 3,37 (m partiellement masqué, 1 H) ; 3,41 (m, 4 H) ; 3,60 (m, 4 H) ; 3,74 (d, *J*=15,9 Hz, 1 H) ; 3,93 (d, *J*=15,9 Hz, 1 H) ; 4,80 (m, 1 H) ; 5,21 (s, 1 H) ; 6,90 (t, *J*=8,3 Hz, 1 H) ; 7,24 (m, 1 H) ; 7,79 (m étalé, 1 H) ; 11,67 (m étalé, 1 H)
Spectrométrie de Masse : méthode B
Temps de rétention Tr (min) = 0,74 ;
[M+H]+ : m/z 373 ; [M-H]- : m/z 371
Point de fusion (Kofler) : supérieur à 260°C
Pouvoir rotatoire : α_{D} = +77,0° +/-1,2 (c = 2,55 mg dans 0,5 mL de méthanol)

### Exemple 29c: Synthèse de la (-)-2-{2-[4-fluoro-2-méthyl-2,3-dihydro-1H-indol-1-yl]-2-oxoéthyl}-3-méthyl-6-(morpholin-4-yl)pyrimidin-4(3H)-one

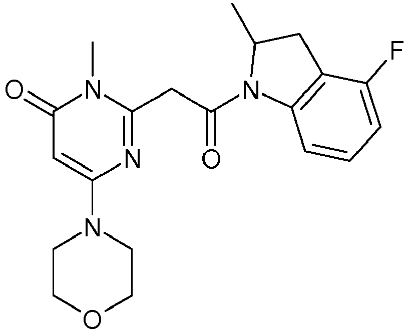

A une solution de 0,22 g de [1-méthyl-4-(morpholin-4-yl)-6-oxo-1,6-dihydropyrimidin-2-yl]acétate de sodium (préparé à l'étape 2c de l'Exemple 4c) dans 3 mL de N,N-diméthylformamide sont ajoutés 0,19 g de N-[3-(diméthylamino)propyl]-N'-éthylcarbodiimide chlorhydrate et 120 µL de pyridine. La suspension résultante est agitée à température ambiante pendant 15 minutes, puis 0,11 g de (+)-4-fluoro-2-methyl-2,3-dihydro-1H-indole [Exemple de référence 8c étape 6c] est ajouté rapidement. Le mélange réactionnel est agité à température ambiante pendant 64 heures, puis il est traité avec un mélange de 20 mL d'eau et 2 mL d'acétate d'éthyle, puis agité à température ambiante pendant 2 heures. Le précipité formé est filtré, puis rincé successivement avec 3 mL d'eau et 3x6 mL d'éther diéthylique. Le solide obtenu est essoré, puis séché sous pression réduite à 40°C. On obtient ainsi 82 mg de (-)-2-{2-[4-fluoro-2-méthyl-2,3-dihydro-1H-indol-1-yl]-2-oxoéthyl}-3-méthyl-6-(morpholin-4-yl)pyrimidin-4(3H)-one sous forme d'un solide blanc dont les caractéristiques sont les suivantes:
Spectre RMN 1H (400MHz) : 1,31 (d, *J*=6,1 Hz, 3 H) ; 2,77 (d, *J*=15,4 Hz, 1 H) ; 3,15 à 3,47 (m partiellement masqué, 8 H) ; 3,58 (m, 4 H) ; 4,04 (d, *J*=18,3 Hz, 1 H) ; 4,29 (d, *J*=18,3 Hz, 1 H) ; 4,80 (m, 1 H) ; 5,36 (s, 1 H) ; 6,91 (t, *J*=8,8 Hz, 1 H) ; 7,25 (m, 1 H) ; 7,78 (d, *J*=8,8 Hz, 1 H)
Spectrométrie de Masse : méthode A
Temps de rétention Tr (min) = 0,78 ;
[M+H]+ : m/z 387 ; [M-H]- : m/z 385
Point de fusion (Kofler) : 244°C
Pouvoir rotatoire : α_{D} = -33,0° (c = 0,64 mg dans 0,5 mL de méthanol)

### Exemple 30c: Synthèse de la (+)-2-{2-[4-fluoro-2-méthyl-2,3-dihydro-1H-indol-1-yl]-2-oxoéthyl}-3-méthyl-6-(morpholin-4-yl)pyrimidin-4(3H)-one

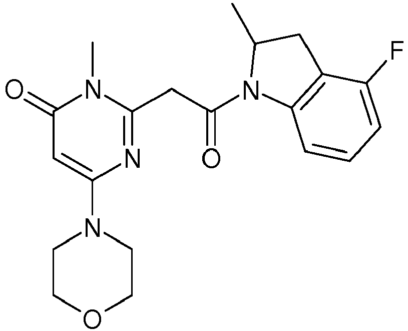

Le produit est préparé en suivant le mode opératoire décrit à l'exemple 29c à partir de 0,22 g de [1-méthyl-4-(morpholin-4-yl)-6-oxo-1,6-dihydropyrimidin-2-yl]acétate de sodium (préparé à l'étape 2c de l'Exemple 4c), de 0,11 g de (-)-4-fluoro-2-methyl-2,3-dihydro-1H-indole [Exemple de référence 8c étape 6c], de 0,19 g de N-[3-(diméthylamino)propyl]-N'-éthylcarbodiimide chlorhydrate dans un mélange de 120 µL de pyridine et de 3,0 mL de N,N-diméthylformamide. On obtient ainsi 79 mg de (+)-2-{2-[4-fluoro-2-méthyl-2,3-dihydro-1H-indol-1-yl]-2-oxoéthyl}-6-(morpholin-4-yl)pyrimidin-4(3H)-one sous forme d'un solide blanc dont les caractéristiques sont les suivantes :
Spectre RMN 1H (400MHz) : 1,31 (d, *J*=6,5 Hz, 3 H) ; 2,77 (d, *J*=15,9 Hz, 1 H) ; 3,18 à 3,41 (m partiellement masqué, 8 H) ; 3,59 (m, 4 H) ; 4,04 (d, *J*=16,3 Hz, 1 H) ; 4,29 (d, *J*=16,3 Hz, 1 H) ; 4,79 (m, 1 H) ; 5,36 (s, 1 H) ; 6,91 (t, *J*=8,6 Hz, 1 H) ; 7,24 (m, 1 H) ; 7,78 (d, *J*=8,6 Hz, 1 H)
Spectrométrie de Masse : méthode A
Temps de rétention Tr (min) = 0,78 ;
[M+H]+ : m/z 387 ; [M-H]- : m/z 385
Point de fusion (Kofler) : 252°C
Pouvoir rotatoire : α_{D} = +90,0° (c = 1,044 mg dans 0,5 mL de méthanol)

### Exemple 31c et Exemple 32c: Synthèse du (+)-2-{2-[-4-bromo-2-méthyl-2,3-dihydro-1H-indol-1-yl]-2-oxoéthyl}-3-méthyl-6-(morpholin-4-yl)pyrimidin-4(3H)-one et du (-)-2-{2-[-4-bromo-2-méthyl-2,3-dihydro-1H-indol-1-yl]-2-oxoéthyl}-3-méthyl-6-(morpholin-4-yl)pyrimidin-4(3H)-one

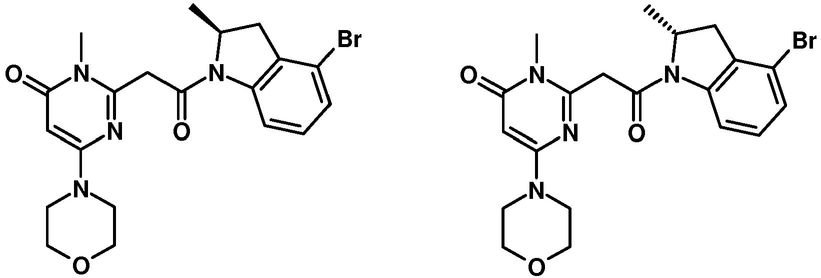

A une solution de 272 mg de [1-méthyl-4-(morpholin-4-yl)-6-oxo-1,6-dihydropyrimidin-2-yl]acétate de sodium (obtenu à l'étape 2c de l'Exemple 4c) dans 6,5 ml de N,N-diméthylformamide et 6,5 ml de pyridine sont ajoutés 150 mg de 4-bromo-2-méthyl-2,3-dihydro-1H-indole [Exemple de référence 7c] et 217 mg de N-[3-(diméthylamino)propyl]-N'-éthylcarbodiimide chlorhydrate. Le mélange réactionnel est agité à température ambiante pendant 72 heures, puis 50 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 236 mg de 2-{2-[-4-bromo-2-méthyl-2,3-dihydro-1H-indol-1-yl]-2-oxoéthyl}-3-méthyl-6-(morpholin-4-yl)pyrimidin-4(3H)-one.

Les énantiomères sont séparés par chromatographie chirale sur colonne Whelk 01 SS, 10 µm, (10 µm, 46x250 mm), en éluant avec un mélange de : heptane/éthanol/méthanol/TEA : 60/20/20/0,1 ; débit : 250 mL/min.

On obtient le premier énantiomère, 60 mg de (+)-2-{2-[-4-bromo-2-méthyl-2,3-dihydro-1H-indol-1-yl]-2-oxoéthyl}-3-méthyl-6-(morpholin-4-yl)pyrimidin-4(3H)-one, sous forme d'un solide blanc dont les caractéristiques sont les suivantes:
Spectre RMN 1 H (400MHz): 1,31 (d, *J*=6,4 Hz, 3 H) ; 2,68 (m, 1 H) ; 3,32 (s, 3 H) ; 3,35 à 3,41 (m, 5 H) ; 3,58 (m, 4 H) ; 4,03 (d, *J*=16,8 Hz, 1 H) ; 4,29 (d, *J*=16,8 Hz, 1 H) ; 4,73 (m, 1 H) ; 5,36 (s, 1 H) ; 7,16 (t, *J*=7,8 Hz, 1 H) ; 7,27 (d, *J*=7,8 Hz, 1 H) ; 7,95 (d large, *J*=7,8 Hz, 1 H)
Spectrométrie de Masse : méthode A
Temps de rétention Tr (min) = 0,89 ;
[M+H]+ : m/z 447 ; [M-H]- : m/z 445;
Pouvoir rotatoire : α_{D} = +116,2° (c=1,984 mg/0,5 mL de DMSO)

Puis le deuxième énantiomère, 61 mg de (-)-2-{2-[-4-bromo-2-méthyl-2,3-dihydro-1H-indol-1-yl]-2-oxoéthyl}-3-méthyl-6-(morpholin-4-yl)pyrimidin-4(3H)-one sous forme d'un solide blanc dont les caractéristiques sont les suivantes :
Spectre RMN 1H (400MHz): 1,31 (d, *J*=6,6 Hz, 3 H) ; 2,68 (d, *J*=15,9 Hz, 1 H) ; 3,32 (s, 3 H) ; 3,36 à 3,40 (m, 5 H) ; 3,58 (m, 4 H) ; 4,03 (d, *J*=16,9 Hz, 1 H) ; 4,29 (d, *J*=16,9 Hz, 1 H) ; 4,74 (m, 1 H) ; 5,36 (s, 1 H) ; 7,16 (t, *J*=8,1 Hz, 1 H) ; 7,27 (d, *J*=8,1 Hz, 1 H) ; 7,95 (d large, *J*=8,1 Hz, 1 H)
Spectrométrie de Masse : méthode A
Temps de rétention Tr (min) = 0,89 ;
[M+H]+ : m/z 447 ; [M-H]- : m/z 445;
Pouvoir rotatoire : α_{D} = -91,2° (c=1,706 mg/0,5 mL DMSO)

### Exemple 33c et Exemple 34c: Synthèse du (+)-2-{2-[-4-chloro-2-méthyl-2,3-dihydro-1H-indol-1-yl]-2-oxoéthyl}-3-méthyl-6-(morpholin-4-yl)pyrimidin-4(3H)-one et du (-)-2-{2-[-4-chloro-2-méthyl-2,3-dihydro-1H-indol-1-yl]-2-oxoéthyl}-3-méthyl-6-(morpholin-4-yl)pyrimidin-4(3H)-one

### Etape 1c:

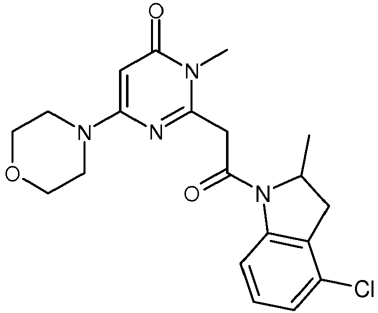

Le produit est préparé en suivant le mode opératoire décrit à l'exemple 21 c à partir de 252 mg du (1-methyl-4-morpholin-4-yl-6-oxo-1,6-dihydro-pyrimidin-2-yl)-acetate de sodium (obtenu à l'étape 2c de l'Exemple 4c) et de 152 mg de 4-chloro-2-méthyl-2,3-dihydro-1H indoline [qui peut être préparée selon le brevet US 4,416,884 (1983)]. Après purification on obtient 122 mg de 2-[2-(4-chloro-2-methyl-2,3-dihydro-indol-1-yl)-2-oxo-ethyl]-3-methyl-6-morpholin-4-yl-3H-pyrimidin-4-one sous forme d'un solide qui sera séparer sur colonne chirale en ses deux énantiomères.

Spectre RMN 1 H (400 MHz) : 1,31 (d, *J*=6,4 Hz, 3 H) ; 2,73 (d, *J*=16,3 Hz, 1 H) ; 3,36 à 3,44 (m, 8 H) ; 3,58 (m, 4 H) ; 4,04 (d, *J*=16,9 Hz, 1 H) ; 4,29 (d, *J*=16,9 Hz, 1 H) ; 4,77 (m, 1 H) ; 5,36 (s, 1 H) ; 7,13 (d, *J*=8,4 Hz, 1 H) ; 7,24 (t, *J*=8,4 Hz, 1 H) ; 7,91 (d, *J*=8,4 Hz, 1 H)

### Etape 2c :

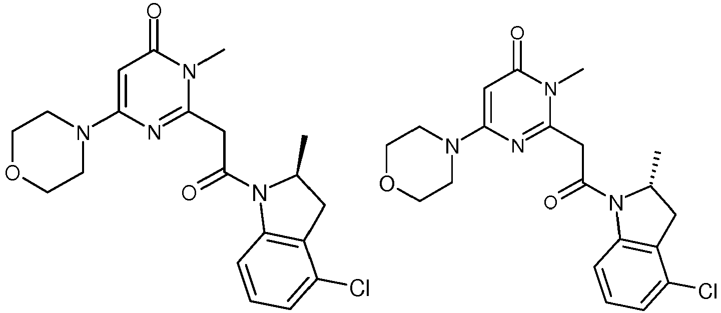

La séparation des deux énantiomères de la 2-[2-(4-chloro-2-methyl-2,3-dihydro-indol-1-yl)-2-oxo-ethyl]-3-methyl-6-morpholin-4-yl-3H-pyrimidin-4-one (195 mg) a été réalisée par chromatographie chirale :
Phase stationnaire : phase Whelk 01 SS, 5 µm ; phase mobile : heptane (60%) / dichlorométhane (20%) / méthanol (20%) ; débit 42 ml/min.

L'énantiomère dextrogyre est concentré pour obtenir 81 mg de la 2-[2-((+)-4-chloro-2-methyl-2,3-dihydro-indol-1-yl)-2-oxo-ethyl]-3-methyl-6-morpholin-4-yl-3H-pyrimidin-4-one sous forme d'un solide blanc dont les caractéristiques sont les suivantes :
Spectre RMN 1 H (400 MHz) : 1,31 (d, *J*=6,4 Hz, 3 H) ; 2,74 (d, *J*=16,3 Hz, 1 H) ; 3,33 (s, 3 H) ; 3,39 (m, 5 H) ; 3,59 (m, 4 H) ; 4,04 (d, *J*=16,9 Hz, 1 H) ; 4,29 (d, *J*=16,9 Hz, 1 H) ; 4,78 (m, 1 H) ; 5,36 (s, 1 H) ; 7,13 (d, *J*=7,9 Hz, 1 H) ; 7,24 (t, *J*=7,9 Hz, 1 H) ; 7,91 (d large, *J*=7,9 Hz, 1 H)
Spectrométrie de Masse : méthode A
Temps de rétention Tr (min) = 0,87 ;
[M+H]+ : m/z 403 ; [M-H]- : m/z 401
Pouvoir rotatoire : α_{D}= +86.2+/-1.6 (C= 1.504mg/0.5mL DMSO)

L'énantiomère lévogyre est concentré pour obtenir 81 mg de la 2-[2-((-)-4-chloro-2-methyl-2,3-dihydro-indol-1-yl)-2-oxo-ethyl]-3-méthyl-6-morpholin-4-yl-3H-pyrimidin-4-one sous forme d'un solide blanc dont les caractéristiques sont les suivantes :
Spectre RMN 1 H (400 MHz) : 1,31 (d, *J*=6,6 Hz, 3 H) ; 2,73 (d, *J*=16,3 Hz, 1 H) ; 3,33 (s, 3 H) ; 3,38 (m, 5 H) ; 3,57 (m, 4 H) ; 4,04 (d, *J*=17,1 Hz, 1 H) ; 4,29 (d, *J*=17,1 Hz, 1 H) ; 4,78 (m, 1 H) ; 5,36 (s, 1 H) ; 7,13 (d, *J*=7,9 Hz, 1 H) ; 7,24 (t, *J*=7,9 Hz, 1 H) ; 7,91 (d large, *J*=7,9 Hz, 1 H)
Spectrométrie de Masse : méthode A
Temps de rétention Tr (min) = 0,87 ;
[M+H]+ : m/z 403 ; [M-H]- : m/z 401
Pouvoir rotatoire : α_{D}= -79.3+/-1.5 (C=1.708mg/0.5mL DMSO)

### Exemple 35c: Synthèse du 2-{2-[(+)-6-fluoro-2-méthyl-2,3-dihydro-1H-indol-1-yl]-2-oxoéthyl}-6-(morpholin-4-yl)pyrimidin-4(3H)-one

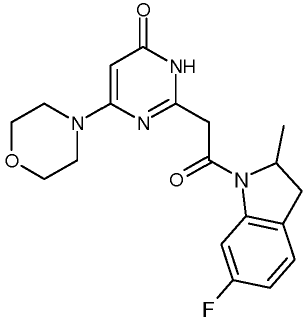

Le produit est préparé en suivant le mode opératoire décrit à l'exemple 21 c à partir de 115 mg du (4-morpholin-4-yl-6-oxo-1,6-dihydro-pyrimidin-2-yl)-acetate de sodium (obtenu à l'étape 2c de l'Exemple 1c) et de 62 mg de (-)-2-methyl-6-fluoro-2,3-dihydro-1H-indole [Exemple de référence 9c étape 5c]. Après purification sur colonne de silice: éluant dichlorométhane/méthanol 98/02 puis 95/05, on obtient 113 mg du 2-[2-((+)-6-fluoro-2-methyl-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1 H (400 MHz) : 1,27 (d, *J*=6,4 Hz, 3 H) ; 2,67 (d, *J*=16,3 Hz, 1 H) ; 3,33 (m partiellement masqué, 1 H) ; 3,41 (m, 4 H) ; 3,60 (m, 4 H) ; 3,74 (d, *J*=16,1 Hz, 1 H) ; 3,94 (d, *J*=16,1 Hz, 1 H) ; 4,76 (m, 1 H) ; 5,21 (s, 1 H) ; 6,86 (ddd, *J*=2,6 et 8,3 et 9,2 Hz, 1 H) ; 7,29 (dd, *J*=5,7 et 8,3 Hz, 1 H) ; 7,72 (d large, *J*=11,0 Hz, 1 H) ; 11,68 (m étalé, 1 H)
Spectrométrie de Masse : méthode A
Temps de rétention Tr (min) = 0,73 ;
[M+H]+ : m/z 373 ; [M-H]- : m/z 371
Pouvoir rotatoire : α_{D}= +60.0+/-1.0 (C=2.778mg/0.5mL DMSO)

### Exemple 36c: Synthèse du 2-{2-[(-)-6-fluoro-2-méthyl-2,3-dihydro-1H-indol-1-yl]-2-oxoéthyl}-6-(morpholin-4-yl)pyrimidin-4(3H)-one

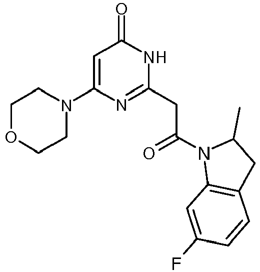

Le produit est préparé en suivant le mode opératoire décrit à l'exemple 15c à partir de 115 mg du (4-morpholin-4-yl-6-oxo-1,6-dihydro-pyrimidin-2-yl)-acetate de sodium et de 62 mg de (+)-2-methyl-6-fluoro-2,3-dihydro-1H-indole [Exemple de référence 9c étape 5c]. Après purification sur colonne de silice: éluant dichlorométhane/méthanol 98/02 puis 95/05, on obtient 84 mg du 2-[2-((-)-6-fluoro-2-methyl-2,3-dihydro-indol-1-yl)-2-oxo-ethyl]-6-morpholin-4-yl-3H-pyrimidin-4-one sous forme d'un solide dont les caractéristiques sont les suivantes :
Spectre RMN 1 H (400 MHz) : 1,27 (d, *J*=6,4 Hz, 3 H) ; 2,67 (d, *J*=16,3 Hz, 1 H) ; 3,27 à 3,38 (m partiellement masqué, 1 H) ; 3,41 (m, 4 H) ; 3,60 (m, 4 H) ; 3,73 (d, *J*=15,9 Hz, 1 H) ; 3,94 (d, *J*=15,9 Hz, 1 H) ; 4,76 (m, 1 H) ; 5,21 (s, 1) ; 6,86 (ddd, *J*=2,6 et 8,3 et 9,2 Hz, 1 H) ; 7,29 (dd, *J*=5,7 et 8,3 Hz, 1 H) ; 7,72 (d large, *J*=11,0 Hz, 1 H) ; 11,68 (m étalé, 1 H)
Spectrométrie de Masse : méthode A
Temps de rétention Tr (min) = 0,73 ;
[M+H]+ : m/z 371 ; [M-H]- : m/z 373
Pouvoir rotatoire : α_{D}= -54.6+/-0.9 (C=2.702mg/0.5mL DMSO)

### Exemple 37c et Exemple 38c : Synthèse du (+)-2-{2-[-4-chloro-5-fluoro-2-méthyl-2,3-dihydro-1H-indol-1-yl]-2-oxoéthyl}-6-(morpholin-4-yl)pyrimidin-4(3H)-one et du (-)-2-{2-[-4-chloro-5-fluoro-2-méthyl-2,3-dihydro-1H-indol-1-yl]-2-oxoéthyl}-6-(morpholin-4-yl)pyrimidin-4(3H)-one

### Etape 1c:

### 2-[2-(4-Chloro-5-fluoro-2-methyl-2,3-dihydro-indol-1-yl)-2-oxo-ethyl]1-6-morpholin-4-yl-3H-pyrimidin-4-one

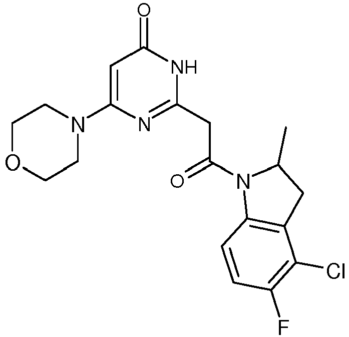

Le produit est préparé en suivant le mode opératoire décrit à l'exemple 1c étape 3c à partir de 248 mg du (4-morpholin-4-yl-6-oxo-1,6-dihydro-pyrimidin-2-yl)-acetate de sodium et de 160 mg de 4-chloro-5-fluoro-2-methyl-2,3-dihydro-1H-indole (Exemple de référence 10c). Après purification sur colonne de silice: éluant dichlorométhane/méthanol 99/01 puis 98/02, on obtient 157 mg de 2-[2-(4-chloro-5-fluoro-2-methyl-2,3-dihydro-indol-1-yl)-2-oxo-ethyl]-6-morpholin-4-yl-3H-pyrimidin-4-one sous forme d'un solide qui sera séparer sur colonne chirale en ses deux énantiomères.
Spectrométrie de Masse : méthode A
Temps de rétention Tr (min) = 0,84 ;
[M+H]+ : m/z 407 ; [M-H]- : m/z 405

### Etape 2c :

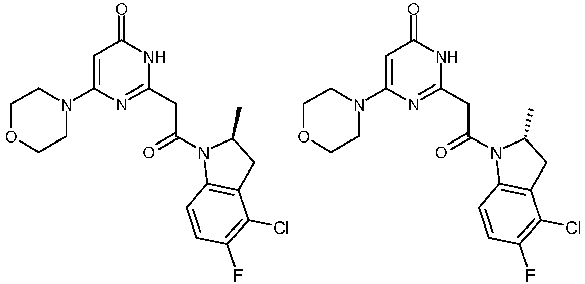

La séparation des deux énantiomères de la 2-[2-(4-chloro-5-fluoro-2-methyl-2,3-dihydro-indol-1-yl)-2-oxo-ethyl]-6-morpholin-4-yl-3H-pyrimidin-4-one (157 mg) a été réalisée par chromatographie chirale :
Phase stationnaire : phase Whelk 01 SS, 5 µm ; phase mobile : heptane (65%) / dichlorométhane (20%) / méthanol (10%) / éthanol (5%) ; débit 43 ml/min.

L'énantiomère dextrogyre est concentré pour obtenir 58 mg de la 2-[2-((+)-4-chloro-5-fluoro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (400 MHz) : 1,31 (d, *J*=6,4 Hz, 3 H) ; 2,76 (d, *J*=17,4 Hz, 1 H) ; 3,41 (m, 5 H) ; 3,60 (m, 4 H) ; 3,73 (d, *J*=15,9 Hz, 1 H) ; 3,92 (d, *J*=15,9 Hz, 1 H) ; 4,80 (m, 1 H) ; 5,20 (s, 1 H) ; 7,24 (t, *J*=9,2 Hz, 1 H) ; 7,90 (m large, 1 H); 11,68 (m étalé, 1 H)
Temps de rétention Tr (min) = 0,84 ;
[M+H]+ : m/z 407 ; [M-H]- : m/z 405
Pouvoir rotatoire : α_{D}= +25.8+/-1.0 (C=1.506 mg/0.5mL DMSO)

L'énantiomère lévogyre est concentré pour obtenir 52 mg de la 2-[2-((-)-4-chloro-5-fluoro-2-methyl-2,3-dihydro-indol-1-yl)-2-oxo-ethyl]-6-morpholin-4-yl-3H-pyrimidin-4-one sous forme d'un solide blanc dont les caractéristiques sont les suivantes :
Spectre RMN 1 H (400 MHz) : 1,31 (d, *J=*6,4 Hz, 3 H) ; 2,76 (d, *J*=17,4 Hz, 1 H) ; 3,41 (m, 5 H) ; 3,60 (m, 4 H) ; 3,73 (d, *J*=15,9 Hz, 1 H) ; 3,92 (d, *J*=15,9 Hz, 1 H) ; 4,80 (m, 1 H) ; 5,20 (s, 1 H) ; 7,24 (t, *J*=9,2 Hz, 1 H) ; 7,90 (m large, 1 H) ; 11,68 (m étalé, 1 H)
Spectrométrie de Masse : méthode A
Temps de rétention Tr (min) = 0,84 ;
[M+H]+ : m/z 407 ; [M-H]- : m/z 405
Pouvoir rotatoire : α_{D}= -24.9+/-0.9 (C= 1.704mg/0.5mL DMSO)

### Exemple 39c : Synthèse de la (-)-2-[2-(2-isopropyl-2,3-dihydro-indol-1-yl)-2-oxo-ethyl]-6-morpholin-4-yl-3H-pyrimidin-4-one :

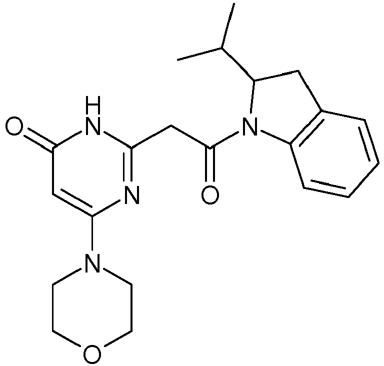

Le produit est préparé en suivant le mode opératoire décrit à l'exemple 1c à partir de 0,30 g de [4-(morpholin-4-yl)-6-oxo-1,6-dihydropyrimidin-2-yl]acétate de sodium, de 0,17 g de (-)-2-isopropyl-indoline, de 0,27 g de N-[3-(diméthylamino)propyl]-N'-éthylcarbodiimide chlorhydrate dans un mélange de 0,26 mL de pyridine et de 4,4 mL de N,N-diméthylformamide. On obtient ainsi 0,25 g de (-)-2-[2-(2-isopropyl-2,3-dihydro-indol-1-yl)-2-oxo-ethyl]-6-morpholin-4-yl-3H-pyrimidin-4-one (Exemple de référence 11c) sous forme d'un solide rose très pâle dont les caractéristiques sont les suivantes :
Spectre RMN 1 H (400MHz) : pour ce lot, tous les signaux sont larges avec : 0,58 (d, J=6,8 Hz, 3 H) ; 0,94 (d, J=6,8 Hz, 3 H) ; 2,19 (m, 1 H) ; 2,92 (d, J=16,4 Hz, 1 H) ; 3,18 (dd, J=9,8 et 16,4 Hz, 1 H) ; 3,40 (m, 4 H) ; 3,59 (m, 4 H) ; 3,75 (d, J=15,7 Hz, 1 H) ; 3,92 (d, J=15,7 Hz, 1 H) ; 4,54 (m, 1 H) ; 5,19 (s, 1 H) ; 7,01 (t, J=7,9 Hz, 1 H) ; 7,14 (t, J=7,9 Hz, 1 H) ; 7,24 (d, J=7,9 Hz, 1 H) ; 7,93 (d, J=7,9 Hz, 1 H) ; 11,66 (m, 1 H)
Spectrométrie de Masse : méthode A
Temps de rétention Tr (min) = 0,82 ;
[M+H]+ : m/z 383 ; [M-H]- : m/z 381
Point de fusion (Kofler) : 172°C
Pouvoir rotatoire : α_{D} = -110,8° +/-1,8 (c = 0,35 % dans le DMSO)

### Exemple 40c : Synthèse de la (+)-2-[2-(2-isopropyl-2,3-dihydro-indol-1-yl)-2-oxo-ethyl]-6-morpholin-4-yl-3H-pyrimidin-4-one :

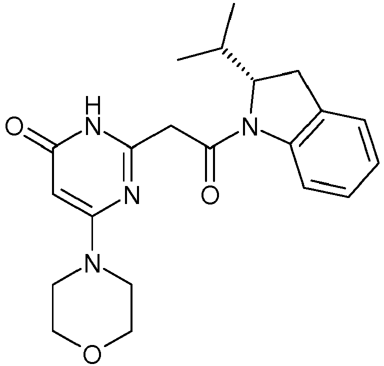

Le produit est préparé en suivant le mode opératoire décrit à l'exemple 1c à partir de 0,27 g de [4-(morpholin-4-yl)-6-oxo-1,6-dihydropyrimidin-2-yl]acétate de sodium, de 0,15 g de (+)-2-isopropyl-indoline, de 0,24 g de N-[3-(diméthylamino)propyl]-N'-éthylcarbodiimide chlorhydrate dans un mélange de 0,23 mL de pyridine et de 3,8 mL de N,N-diméthylformamide. On obtient ainsi 0,24 g de (+)-2-[2-(2-isopropyl-2,3-dihydro-indol-1-yl)-2-oxo-ethyl]-6-morpholin-4-yl-3H-pyrimidin-4-one (Exemple de référence 12c) sous forme d'un solide blanc dont les caractéristiques sont les suivantes :
Spectre RMN 1 H (400MHz) : pour ce lot, tous les signaux sont larges avec : 0,58 (d, J=6,1 Hz, 3 H) ; 0,94 (d, J=6,1 Hz, 3 H) ; 2,20 (m, 1 H) ; 2,93 (d, J=16,3 Hz, 1 H) ; 3,18 (dd, J=9,7 et 16,3 Hz, 1 H) ; 3,40 (m, 4 H) ; 3,59 (m, 4 H) ; 3,75 (d, J=15,4 Hz, 1 H) ; 3,92 (d, J=15,4 Hz, 1 H) ; 4,54 (m, 1 H) ; 5,20 (s, 1 H) ; 7,01 (t, J=7,9 Hz, 1 H) ; 7,14 (t, J=7,9 Hz, 1 H) ; 7,24 (d, J=7,9 Hz, 1 H) ; 7,93 (d, J=7,9 Hz, 1 H) ; 11,67 (s, 1 H)
Spectrométrie de Masse : méthode A
Temps de rétention Tr (min) = 0,82 ;
[M+H]+ : m/z 383 ; [M-H]- : m/z 381
Point de fusion (Kofler) : 174°C
Pouvoir rotatoire : α_{D} = +117,3° +/-1,6 (c = 2,857 mg dans 0,5 mL de méthanol)

### Exemple 41c: Synthèse du 2-[1-(4-fluoro-2,3-dihydro-1H-indol-1-yl)-1-oxopropan-2-yl]-6-(morpholin-4-yl)pyrimidin-4(3H)-one

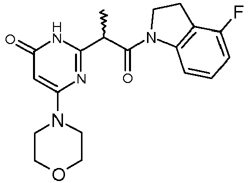

### Etape 1c :

### 2,4-dichloro-6-méthoxy-pyrimidine

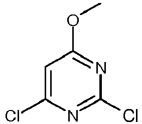

A une solution de 11 g du 2,4,6-trichloropyrimidine dans 140 mL de méthanol refroidit à 0°C dans un bain de glace, sont ajoutés, goutte à goutte, 3.24 g de méthylate de sodium préalablement dissous dans 13 mL de méthanol. Le bain de glace est retiré. Le milieu réactionnel est agité à 0°C pendant 45 minutes, puis le bain refroidissant est enlevé pour laisser la température monter à l'ambiante. Le milieu réactionnel est concentré sous pression réduite. Le résidu obtenu est repris avec 30 mL d'eau et 100 mL d'acétate d'éthyle. Après décantation, la phase organique est séchée sur sulfate de magnésium, filtrée, concentrée sous pression réduite pour donner une huile qui est laissée cristalliser pendant 24 heures à la température ambiante. Le produit cristallise sous forme d'aiguilles au milieu d'une huile. Les aiguilles sont séparées pour donner 3.94 g de 2,4-dichloro-6-méthoxy-pyrimidine dont les caractéristiques sont les suivantes :
Spectrométrie de Masse : méthode A
El : [M]+. m/z = 178 ; pic de base : m/z = 148

### Etape 2c :

### Ester éthylique de l'acide (4-chloro-6-méthoxy-pyrimidin-2-yl)-acétique

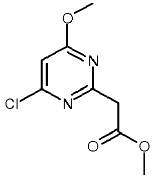

A une solution de 7.4 g du 2,4-dichloro-6-méthoxy-pyrimidine et 4.5 mL d'acétate d'éthyle dans 100 mL de THF anhydre refroidit à -75°C dans un bain de carboglace / acétone, sont additionnés, goutte à goutte, 91.4 mL de lithium bis(trimethylsilyl)amide 1 M (THF). Le milieu réactionnel est agité à -75°C pendant une heure. Le bain refroidissant est enlevé pour laisser la température monter à 22°C. Le milieu réactionnel est agité à 22°C pendant une heure. Addition de 100 mL d'eau et 400 mL d'acétate d'éthyle. Après décantation, la phase organique est séchée sur sulfate de magnésium, filtrée puis concentrée sous pression réduite pour donner 9.5 g de l'ester éthylique de l'acide (4-chloro-6-méthoxy-pyrimidin-2-yl)-acétique sous forme d'une huile orange dont les caractéristiques sont les suivantes :
Spectrométrie de Masse : méthode A
Temps de rétention Tr (min) = 0,80 ; [M+H]+ : m/z 231 ;

### Etape 3c :

### Ester éthylique de l'acide (4-méthoxy-6-morpholin-4-yl-pyrimidin-2-yl)-acétique

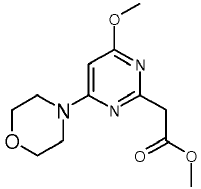

Dans un tube pour micro-ondes, sont mélangés 1,5 g de l'ester éthylique de l'acide (4-chloro-6-méthoxy-pyrimidin-2-yl)-acétique et 20 mL de morpholine. Après irradiation aux micro-ondes pendant 1h à 90°C, le mélange réactionnel est concentré sous pression réduite puis dilué avec 300 mL d'acétate d'éthyle et 300 mL d'eau. Après décantation la phase organique est séchée sur sulfate de magnésium, filtrée puis concentrée sous pression réduite. On obtient ainsi, 1,9 g de l'ester éthylique de l'acide (4-méthoxy-6-morpholin-4-yl-pyrimidin-2-yl)-acétique sous forme d'une huile visqueuse qui cristallise et qui est utilisée telle quelle dans l'étape suivante.

### Etape 4c :

### (4-méthoxy-6-morpholin-4-yl-pyrimidin-2-yl)-acétate de sodium

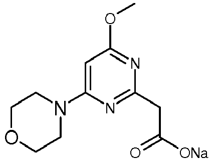

A une solution de 2,5 g de l'ester éthylique de l'acide (4-méthoxy-6-morpholin-4-yl-pyrimidin-2-yl)-acétique dans 15 mL de THF sont ajoutés 8,8 mL de soude 1N. Le milieu réactionnel est agité à la température ambiante pendant 48 heures puis concentré sous pression réduite. Après séchage à l'étuve sous vide, on obtient 2,4 g de (4-méthoxy-6-morpholin-4-yl-pyrimidin-2-yl)-acétate de sodium qui sera utilisé tel quel dans l'étape suivante.

### Etape 5c:

### 1-(4-fluoro-2,3-dihydro-indol-1-yl)-2-(4-méthoxy-6-morpholin-4-yl-pyrimidin-2-yl)-éthanone

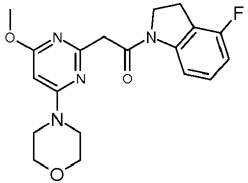

A une solution de 1,7 g du (4-méthoxy-6-morpholin-4-yl-pyrimidin-2-yl)-acétate de sodium dans 15 mL de DMF et 5 mL de pyridine sont ajoutés 1,2 g de 4-fluoro-2,3-dihydro-1H-indole et 1,9 g de N-[3-(diméthylamino)propyl]-N'-éthylcarbodiimide chlorhydrate. Le milieu réactionnel est agité à température ambiante pendant 3 heures. Le mélange réactionnel est concentré sous pression réduite puis dilué dans 80 mL d'eau et 15 mL d'acétate d'éthyle. Le précipité formé est lavé avec 15 mL d'éther éthylique puis avec 15 mL d'éther de pétrole. Après séchage sous pression réduite, on obtient 1,53 g de 1-(4-fluoro-2,3-dihydro-indol-1-yl)-2-(4-méthoxy-6-morpholin-4-yl-pyrimidin-2-yl)-éthanone sous forme de solide rosé utilisé tel quel dans l'étape suivante.

### Etape 6c:

### 1-(4-fluoro-2,3-dihydro-indol-1-yl)-2-(4-méthoxy-6-morpholin-4-yl-pyrimidin-2-yl)-propan-1-one

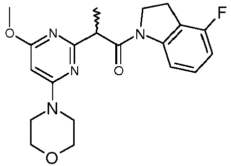

300 mg de 1-(4-Fluoro-2,3-dihydro-indol-1-yl)-2-(4-méthoxy-6-morpholin-4-yl-pyrimidin-2-yl)-éthanone dissous dans 10 mL de tétrahydrofuranne est refroidit à -78°C à l'aide d'un bain de carboglace. 2,65 mL de potassium bis(triméthylsilyl)amide 1 N dans le THF sont ajoutés lentement en maintenant la température à -75°C. Après 30 minutes d'agitation, on ajoute 0,125 mL d'iodure de méthyle et poursuit l'agitation pendant 2h15. Le mélange réactionnel est dilué avec 50 mL d'une solution saturée en chlorure d'ammonium et 30 mL d'acétate d'éthyle. Après décantation la phase organique est lavée avec 5 fois 15 mL d'eau, séchée sur sulfate de magnésium, filtrée puis concentrée sous pression réduite. Le résidu est purifié par chromatographie sur colonne de silice en éluant avec un mélange de dichlorométhane et de méthanol 99/01 pour donner 215 mg de 1-(4-fluoro-2,3-dihydro-indol-1-yl)-2-(4-méthoxy-6-morpholin-4-yl-pyrimidin-2-yl)-propan-1-one sous forme d'un solide blanc dont les caractéristiques sont les suivants :
Spectre RMN 1H (400MHz) : 1,42 (d, J=6,8 Hz, 3 H) ; 3,11 (t, J=8,4 Hz, 2 H) ; 3,48 (m, 4 H) ; 3,59 (m, 4 H) ; 3,77 (s, 3 H) ; 3,95 à 4,09 (m, 2 H) ; 4,30 (m, 1 H) ; 5,94 (s, 1 H) ; 6,81 (t, J=8,7 Hz, 1 H) ; 7,19 (m, 1 H) ; 7,92 (d large, J=8,1 Hz, 1 H)
Spectrométrie de Masse : méthode A
Temps de rétention Tr (min) = 0,96 ;
[M+H]+ : m/z 387 ;

### Etape 7c:

### 2-[1-(4-fluoro-2,3-dihydro-1H-indol-1-yl)-1-oxopropan-2-yl]-6-(morpholin-4-yl)pyrimidin-4(3H)-one

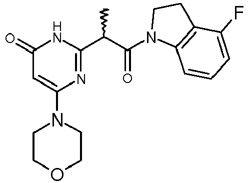

Dans un tube micro-onde, sont ajoutés, 210 mg de 1-(4-Fluoro-2,3-dihydro-indol-1-yl)-2-(4-méthoxy-6-morpholin-4-yl-pyrimidin-2-yl)-propan-1-one, 270 mg d'iodure de potassium, 7mL d'acétonitrile et 208 mL de triméthylchlorosilane. Après irradiation sous micro-ondes pendant une heure à une température de 100°C, le milieu réactionnel est dilué avec 20 mL d'acétate d'éthyle et 20 mL d'eau. Après décantation, la phase organique est séchée sur sulfate de magnésium, filtrée puis concentrée sous pression réduite. Le résidu obtenu est agité en présence de 5 mL d'acétate d'éthyle et de 20 mL d'éther de pétrole. Le solide obtenu est filtré puis séché sous pression réduite. On obtient ainsi 163 mg de 2-[1-(4-fluoro-2,3-dihydro-1H-indol-1-yl)-1-oxopropan-2-yl]-6-(morpholin-4-yl)pyrimidin-4(3H)-one sous forme d'un solide jaune pâle dont les caractéristiques sont les suivantes :
Spectre RMN 1 H (400MHz) : 1,42 (d, J=6,8 Hz, 3 H) ; 3,16 (t, J=8,4 Hz, 2 H) ; 3,39 (m, 4 H) ; 3,58 (m, 4 H) ; 3,94 à 4,10 (m, 2 H) ; 4,24 (m, 1 H) ; 5,20 (s large, 1 H) ; 6,85 (t, J=8,7 Hz, 1 H) ; 7,21 (m, 1 H) ; 7,88 (d large, J=8,1 Hz, 1 H) ; 11,73 (m étalé, 1 H)
Spectrométrie de Masse : méthode A
Temps de rétention Tr (min) = 0,71 ;
[M+H]+ : m/z 373 ; [M-H]- : m/z 371

### Exemple 42c et Exemple 43c :

### Synthèse de de la (+)-2-[2-(-2-éthyl-2,3-dihydro-indol-1-yl)-2-oxo-éthyl]-6-morpholin-4-yl-3H-pyrimidin-4-one et de la (-)-2-[2-(-2-éthyl-2,3-dihydro-indol-1-yl)-2-oxo-éthyl]-6-morpholin-4-yl-3H-pyrimidin-4-one

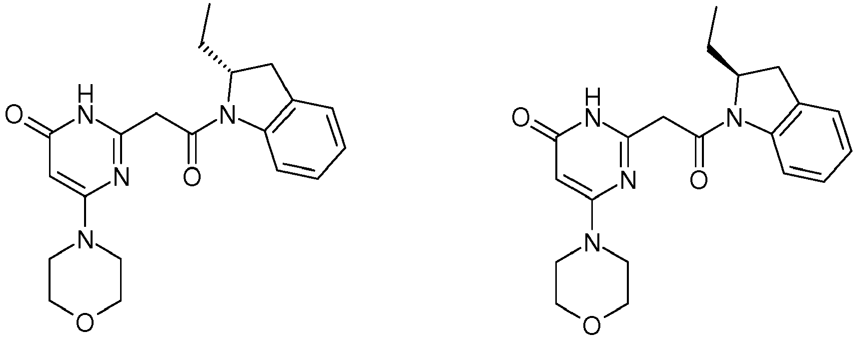

La (±)-2-[2-(2-éthyl-2,3-dihydro-indol-1-yl)-2-oxo-éthyl]-6-morpholin-4-yl-3H-pyrimidin-4-one est préparée en suivant le mode opératoire décrit à l'exemple 1c à partir de 0,40 g de [4-(morpholin-4-yl)-6-oxo-1,6-dihydropyrimidin-2-yl]acétate de sodium, de 0,25 g de 2-éthyl-indoline [qui peut être préparée selon le brevet WO2009065920], de 0,39 g de N-[3-(diméthylamino)propyl]-N'-éthylcarbodiimide chlorhydrate dans un mélange de 250 µL de pyridine et de 6,5 mL de N,N-diméthylformamide. Après purification par chromatographie sur une cartouche de 25 g de silice15-40 µm, en éluant successivement avec des mélanges dichlorométhane/ méthanol 99/1, puis 98/2 et 97/3 v/v, à un débit de 30 mL/ min, on obtient 0,35 g de (±)-2-[2-(2-éthyl-2,3-dihydro-indol-1-yl)-2-oxo-éthyl]-6-morpholin-4-yl-3H-pyrimidin-4-one sous forme d'une meringue blanche dont les caractéristiques sont les suivantes :
Spectrométrie de Masse : méthode A
Temps de rétention Tr (min) = 0,76 ;
[M+H]+ : m/z 369; [M-H]- : m/z 367
Point de fusion (Kofler) : 155°C

Les produits ont été obtenus par séparation chromatographique chirale de 350 mg de (±)-2-[2-(2-éthyl-2,3-dihydro-indol-1-yl)-2-oxo-éthyl]-6-morpholin-4-yl-3H-pyrimidin-4-one sur colonne Chiralpak AY (1080 g, 20 µm, 10/23 cm), éluant : acétonitrile/ isopropanol : 90/10 ; débit :400 mL/min. Après purification, on obtient comme premier énantiomère, 138 mg de (+)-2-[2-(2-éthyl-2,3-dihydro-indol-1-yl)-2-oxo-éthyl]-6-morpholin-4-yl-3H-pyrimidin-4-one, sous forme d'une meringue blanche dont les caractéristiques sont les suivantes:
Spectre RMN 1H (400MHz): 0,85 (t, J=7,5 Hz, 3 H) ; 1,55 (m, 1 H) ; 1,71 (m, 1 H) ; 2,84 (d, J=16,3 Hz, 1 H) ; 3,31 (m partiellement masqué, 1 H) ; 3,41 (m, 4 H) ; 3,60 (m, 4 H) ; 3,71 (d, J=16,0 Hz, 1 H) ; 3,92 (d, J=16,0 Hz, 1 H) ; 4,52 (m, 1 H) ; 5,20 (s, 1 H) ; 7,03 (t, J=7,9 Hz, 1 H) ; 7,16 (t, J=7,9 Hz, 1 H) ; 7,26 (d, J=7,9 Hz, 1 H) ; 7,94 (d large, J=7,9 Hz, 1 H) ; 11,67 (m étalé, 1 H)
Spectrométrie de Masse : méthode A
Temps de rétention Tr (min) = 0,77 ;
[M+H]+ : m/z 369 ; [M-H]- : m/z 367
Pouvoir rotatoire : α_{D} = +109° (c = 1,438 mg dans 0.5 mL de DMSO)

Puis le deuxième énantiomère, soit: 142 mg de (-)-2-[2-(2-éthyl-2,3-dihydro-indol-1-yl)-2-oxo-éthyl]-6-morpholin-4-yl-3H-pyrimidin-4-one sous forme d'une meringue blanche dont les caractéristiques sont les suivantes :
Spectre RMN 1H (400MHz): 0,85 (t, J=7,5 Hz, 3 H) ; 1,55 (m, 1 H) ; 1,73 (m, 1 H) ; 2,84 (d, J=16,3 Hz, 1 H) ; 3,30 (m partiellement masqué, 1 H) ; 3,41 (m, 4 H) ; 3,60 (m, 4 H) ; 3,71 (d, J=16,0 Hz, 1 H) ; 3,92 (d, J=16,0 Hz, 1 H) ; 4,53 (m, 1 H) ; 5,20 (s, 1 H) ; 7,03 (t, J=7,9 Hz, 1 H) ; 7,16 (t, J=7,9 Hz, 1 H) ; 7,27 (d, J=7,9 Hz, 1 H) ; 7,94 (d large, J=7,9 Hz, 1 H) ; 11,67 (m étalé, 1 H)
Spectrométrie de Masse : méthode A
Temps de rétention Tr (min) = 0,77 ;
[M+H]+ : m/z 369 ; [M-H]- : m/z 367
Pouvoir rotatoire : α_{D} = -89° (c = 0,883 mg dans 0.5 mL de DMSO)

### Exemple 44c et Exemple 45c (n'appartenant pas à l'invention) :

### Synthèse de la (-)-2-[2-fluoro-1-(4-fluoro-2,3-dihydro-1H-indol-1-yl)-1-oxopropan-2-yl]-6-(morpholin-4-yl)pyrimidin-4(3H)-one et de la (+)-2-[2-fluoro-1-(4-fluoro-2,3-dihydro-1H-indol-1-yl)-1-oxopropan-2-yl]-6-(morpholin-4-yl)pyrimidin-4(3H)-one

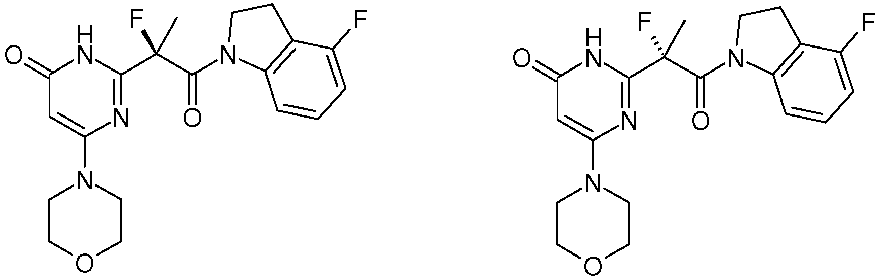

### Étape 1 c :

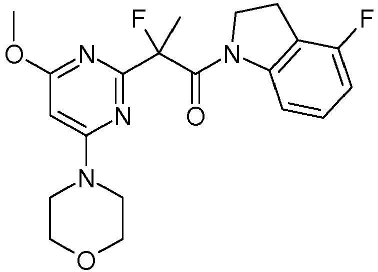

A une solution de 386 mg de 1-(4-fluoro-2,3-dihydro-indol-1-yl)-2-(4-méthoxy-6-morpholin-4-yl-pyrimidin-2-yl)-propan-1-one, qui peut être obtenue selon exemple 41c étape 6c, dissous dans 10 mL de tétrahydrofuranne et refroidie à -78°C à l'aide d'un bain de carboglace, sont addtionnés lentement 2,4 mL de potassium bis(triméthylsilyl)amide 0,91 M dans le THF en maintenant la température à -70°C. Après 30 minutes d'agitation, on ajoute une solution de 694 mg de N-fluorobenzènesulfonimide dans 5 mL de tétrahydrofuranne et on poursuit l'agitation à -70°C pendant 2 heures. Le mélange réactionnel est dilué avec 20 mL d'eau et 20 mL d'acétate d'éthyle. Après décantation la phase organique est lavée avec une solution saturée de chlorure de sodium, séchée sur sulfate de magnésium, filtrée puis concentrée sous pression réduite. Le résidu est purifié par chromatographie sur colonne de silice en éluant du dichlorométhane pour donner 340 mg de 2-fluoro-1-(4-fluoro-2,3-dihydro-1H-indol-1-yl)-2-[4-méthoxy-6-(morpholin-4-yl)pyrimidin-2-yl]propan-1-one sous forme d'un solide jaune pâle qui sera utilisé tel quel dans l'étape suivante.

### Étape 2c :

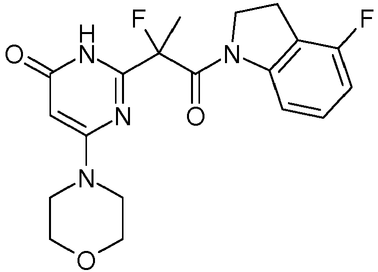

A 340 mg de 2-fluoro-1-(4-fluoro-2,3-dihydro-1*H-*indol-1-yl)-2-[4-méthoxy-6-(morpholin-4-yl)pyrimidin-2-yl]propan-1-one, obtenu selon l'étape précédente, sont additionnés 17 mL d'acide chlorhydrique (4M dans le dioxanne). Le milieu réactionnel est agité à reflux pendant 5 heures, laissé revenir à température ambiante puis concentré sous pression réduite. Le résidu obtenu est purifié sur colonne de silice, éluant : dichlorométhane/méthanol 98/02 pour donner 236 mg de 2-[2-fluoro-1-(4-fluoro-2,3-dihydro-1*H-*indol-1-yl)-1-oxopropan-2-yl]-6-(morpholin-4-yl)pyrimidin-4(3*H*-one racémique sous la forme d'un solide blanc qui sera utilisé tel quel dans l'étape suivante.
Spectre RMN 1H (400MHz): 1,86 (d, J=23,2 Hz, 3 H) ; 3,10 (m, 2 H) ; 3,42 (m, 4H) ; 3,58 (m, 4 H) ; 3,84 (m, 1 H) ; 4,21 (m, 1 H) ; 5,61 (m étalé, 1 H) ; 6,91 (t, J=8,4 Hz, 1 H) ; 7,25 (dt,J=5,9 et 8,4 Hz, 1 H) ; 7,92 (d, J=8,4 Hz, 1 H) ; 11,81 (m étalé, 1 H) Spectrométrie de Masse : méthode A
Temps de rétention Tr (min) = 0,79;
[M+H]+ : m/z 391 ;
[M-H]- : m/z 389 ; [2M-H]- : m/z 779 (pic de base)

### Étape 3c :

Les produits ont été obtenus par séparation chromatographique chirale de 200 mg de de (±)-2-[2-fluoro-1-(4-fluoro-2,3-dihydro-1*H-*indol-1-yl)-1-oxopropan-2-yl]-6-(morpholin-4-yl)pyrimidin-4(3*H*-one sur colonne Chiralpak AY-H (5 µm, 30x250 mm) éluant : heptane/éthanol/méthanol/triéthylamine : 75/20/5/0,1 ; débit : 43 mL/min. Après purification, on obtient comme premier énantiomère, 65 mg de (-)-2-[2-fluoro-1-(4-fluoro-2,3-dihydro-1*H*-indol-1-yl)-1-oxopropan-2-yl]-6-(morpholin-4-yl)pyrimidin-4(3*H*)-one, sous forme d'un lyophylisat blanc dont les caractéristiques sont les suivantes :
Spectre RMN 1H (400MHz): 1,85 (d, J=23,2 Hz, 3 H) ; 3,10 (m, 2 H) ; 3,42 (m, 4H) ; 3,59 (m, 4 H) ; 3,84 (m, 1 H) ; 4,21 (m, 1 H) ; 5,62 (m étalé, 1 H) ; 6,91 (t, J=8,4 Hz, 1 H) ; 7,25 (dt,J=5,9 et 8,4 Hz, 1 H) ; 7,92 (d, J=8,4 Hz, 1 H) ; 11,79 (m étalé, 1 H)
Spectrométrie de Masse : méthode A
Temps de rétention Tr (min) = 0,77 ;
[M+H]+ : m/z 391 ; [M-H]- : m/z 389
Pouvoir rotatoire : αD = -68,6° +/- 1,3 (c = 1,767 mg dans 0.5 mL de DMSO)

Puis le deuxième énantiomère, soit: 71 mg de (+)-2-[2-fluoro-1-(4-fluoro-2,3-dihydro-1*H-*indol-1-yl)-1-oxopropan-2-yl]-6-(morpholin-4-yl)pyrimidin-4(3*H*)-one, sous forme d'un lyophylisat blanc dont les caractéristiques sont les suivantes :
Spectre RMN 1H (400MHz): 1,85 (d, J=23,2 Hz, 3 H) ; 3,11 (m, 2 H) ; 3,42 (m, 4H) ; 3,58 (m, 4 H) ; 3,84 (m, 1 H) ; 4,21 (m, 1 H) ; 5,61 (m étalé, 1 H) ; 6,91 (t, J=8,3 Hz, 1 H) ; 7,25 (dt,J=6,1 et 8,3 Hz, 1 H) ; 7,92 (d, J=8,3 Hz, 1 H) ; 11,79 (m étalé, 1 H) Spectrométrie de Masse : méthode A
Temps de rétention Tr (min) = 0,77 ;
[M+H]+ : m/z 391 ; [M-H]- : m/z 389
Pouvoir rotatoire : αD = +75.2+/-1.5 (c= 1.532 mg dans 0,5ML de DMSO)

### Exemple 46c : Synthèse de 1-{[4-(morpholin-4-yl)-6-oxo-1,6-dihydropyrimidin-2-yl]acétyl}-2,3-dihydro-1H-indole-4-carbonitrile

### VAC.SON5.167.1

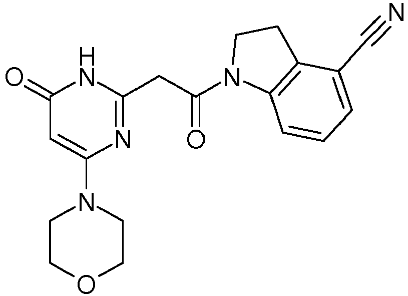

A une solution de 300 mg de [4-(morpholin-4-yl)-6-oxo-1,6-dihydropyrimidin-2-yl]acétate de sodium, obtenu selon l'exemple 1c étape 2c, dans 3 mL de N,N-diméthylformamide sont ajoutés 242 mg de N-[3-(diméthylamino)propyl]-N'-éthylcarbodiimide chlorhydrate, 371 mg de N,N-diisopropylamine et 210 mg de chlorhydrate de 2,3-dihydro-1H-indole-4-carbonitrile. Le mélange réactionnel est agité à température ambiante pendant 72 heures, puis concentré sous pression réduite. Le résidu est repris avec 100 mL d'eau et 20 mL d'acétate d'éthyle, puis agité à température ambiante pendant 1 heure. Le précipité formé est filtré, puis rincé successivement à l'eau et de l'éther de pétrole. Le solide obtenu est essoré, puis séché sous pression réduite à 40°C. On obtient ainsi 190 mg de 1-{[4-(morpholin-4-yl)-6-oxo-1,6-dihydropyrimidin-2-yl]acétyl}-2,3-dihydro-1*H-*indole-4-carbonitrile sous forme d'un solide blanc dont les caractéristiques sont les suivantes:
Spectre RMN 1 H (400MHz) : 3,36 (t, J=8,5 Hz, 2 H) ; 3,41 (m, 4 H) ; 3,60 (m, 4H) ; 3,79 (s, 2 H) ; 4,23 (t, J=8,5 Hz, 2 H) ; 5,21 (s, 1 H) ; 7,38 (t, J=8,0 Hz, 1 H) ; 7,45 (d, J=8,0 Hz, 1 H) ;8,27 (d, J=8,0 Hz, 1 H) ; 11,62 (s large, 1 H)
Spectrométrie de Masse : méthode A
Temps de rétention Tr (min) = 0,61 ;
[M+H]+ : m/z 366 ; [M-H]- : m/z 361

### Exemples de référence pour la préparation des composés de formule (Ic)

### Exemple de référence 1c : 4-hydroxy-2-méthyl-indoline :

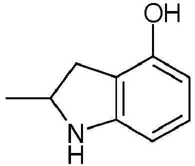

A une solution de 1 g de 4-hydroxy-2-méthyl-indole dans 35 mL d'acide acétique sous argon refroidie à une température voisine de 14°C, sont ajoutés progressivement 1,35 g de cyanoborohydrure de sodium. Le mélange réactionnel est agité à une température voisine de 14°C pendant 15 minutes, puis il est laissé se réchauffer à température ambiante. Après 1,5 heures, le mélange réactionnel est traité avec 15 mL d'eau, agité à température ambiante pendant 1 heure, puis concentré à sec sous pression réduite. Le résidu est repris dans 75 mL d'acétate d'éthyle, puis traité avec 60 mL d'une solution saturée d'hydrogénocarbonate de sodium. Après agitation pendant 1 heure puis décantation, la phase organique est séparée et la phase aqueuse est extraite avec 50 mL d'acétate d'éthyle. Les phases organiques sont réunies, lavées avec de la saumure saturée, séchées sur sulfate de magnésium anhydre, filtrées puis concentrées à sec sous pression réduite. Le résidu est purifié sur une cartouche de 70 g de silice 15-40 µm, en éluant avec un mélange cyclohexane/ acétate d'éthyle 80/20 v/v, avec un débit de 80 mL/ min. On obtient ainsi 0,87 g de 4-hydroxy-2-méthyl-indoline sous forme d'une huile jaune dont les caractéristiques sont les suivantes :
Spectre RMN 1 H (400MHz, CDCl₃) : 2,46 (s, 3 H); 6,30 (d, *J*=0,7 Hz, 1 H); 6,74 (dd, *J*=7,9 et 10,6 Hz, 1 H); 7,02 (dt, *J*=4,9 et 7,9 Hz, 1 H); 7,08 (d, *J*=7,9 Hz, 1 H); 7,95 (s large, 1 H)

### Exemple de référence 2c : 4-fluoro-3,3-diméthyl-2,3-dihydro-1H-indole

### Etape 1 c:

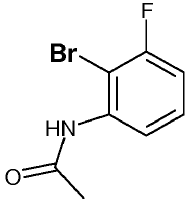

A une solution de 3 g de 2-bromo-3-fluoro aniline dans 30 mL de dichlorométhane sont ajoutés 2,7 ml de triéthylamine puis 1,2 mL de chlorure d'acétyle. Le mélange réactionnel est agité pendant 72 heures puis traité avec un mélange d'eau et de dichlorométhane. Les phases sont séparées et la phase organique concentrée pour donner 3,75 g de N-(2-bromo-3-fluoro-phényl)-acétamide.

### Etape 2c:

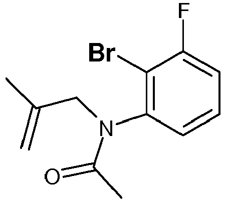

A une solution de 3 g de N-(2-bromo-3-fluoro-phényl)-acétamide dans 25 mL de toluène sont ajoutés 2 g de 3-bromo-2-méthylpropène, 1,9 g de carbonate de potassium et 570 mg d'hydrure de sodium (dispersion dans l'huile à 60%). Le mélange réactionnel est placé sous atmosphère d'argon et chauffé à 75°C pendant 16 heures. Le mélange réactionnel est traité avec un mélange d'eau et d'acétate d'éthyle, la phase organique est ensuite lavée avec de l'eau et une solution saturée de chlorure de sodium, séchée sur sulfate de magnésium, filtrée et concentrée sous pression réduite pour donner 3,8 g de N-(2-bromo-3-fluoro-phényl)-N-(2-méthyl-allyl)-acétamide.

### Etape 3c:

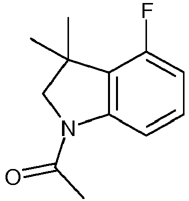

A une solution de 1,5 g de N-(2-bromo-3-fluoro-phényl)-N-(2-méthyl-allyl)-acétamide dans 10 mL de N,N-diméthylformamide sont ajoutés 59 mg d'acétate de palladium, 1,5 g de chlorhydrate de tétrabutyl ammonium et 1,8 mL de triéthylamine. Le mélange réactionnel est chauffé à 100°C sous atmosphère d'argon pendant 16 heures. Après refroidissement, 50 mL d'eau sont ajoutés et le mélange est extrait à l'acétate d'éthyle. La phase organique est ensuite lavée avec de l'eau et une solution saturée de chlorure de sodium, séchée sur sulfate de magnésium, filtrée et concentrée sous pression réduite. Le résidu est purifié par chromatographie sur colonne de silice, en éluant avec un mélange d'heptane et d'acétate d'éthyle (50/50 : v/v) pour donner 520 mg de 1-(4-fluoro-3,3-diméthyl-2,3-dihydro-indol-1-yl)-éthanone sous forme d'une huile jaune.

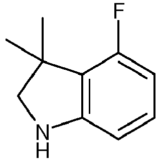

### Etape 4c:

Une solution de 520 mg de 1-(4-fluoro-3,3-diméthyl-2,3-dihydro-indol-1-yl)-éthanone dans 10 mL d'acide chlorhydrique concentré est chauffée à 90°C pendant 2 heures. La solution est ensuite traitée avec du bicarbonate de sodium jusqu'à pH7 et extraite au dichlorométhane. La phase organique est filtrée sur colonne séparatrice de phase et concentrée sous pression réduite pour donner 300 mg de 4-fluoro-3,3-dimethyl-2,3-dihydro-1H-indole sous forme d'une huile brune dont les caractéristiques sont les suivantes:
Spectre RMN 1 H (400MHz) : 1,31 (s, 6 H) ; 3,17 à 3,22 (m, 2 H) ; 5,76 (m large, 1 H) ; 6,19 à 6,31 (m, 2 H) ; 6,91 (m, 1 H)

### Exemple de référence 3c : 4-hydroxy-3-méthyl-indoline

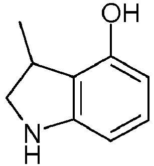

A une solution de 1,0 g de 3-méthyl-1 H-indol-4-ol dans 35 mL d'acide acétique sous argon refroidie à une température voisine de 15°C, sont ajoutés progressivement 1,35 g de cyanoborohydrure de sodium. Le mélange réactionnel est laissé se réchauffer à température ambiante. Après 16,5 heures, le mélange réactionnel est traité avec 15 mL d'eau, puis concentré à sec sous pression réduite. Le résidu est repris dans 75 mL d'acétate d'éthyle, puis traité avec 60 mL d'une solution saturée d'hydrogénocarbonate de sodium. Après agitation pendant 1 heure puis décantation, la phase organique est séparée et la phase aqueuse est extraite avec 50 mL d'acétate d'éthyle. Les phases organiques sont réunies, lavées avec de la saumure saturée, séchées sur sulfate de magnésium anhydre, filtrées puis concentrées à sec sous pression réduite. Le résidu est purifié par chromatographie sur une cartouche de 70 g de silice 15-40 µm, en éluant avec un mélange cyclohexane/ acétate d'éthyle 80/20 v/v, avec un débit de 80 mL/ min. On obtient ainsi 0,45 g de 4-hydroxy-3-méthyl-indoline sous forme d'un solide jaune pâle dont les caractéristiques sont les suivantes :
Spectrométrie de Masse : méthode A
Temps de rétention Tr (min) = 0,14 ;
[M+H]+ : m/z 150
Point de fusion (Kofler) : 115°C

### Exemple de référence 4c : 5-fluoro-2-méthyl-2,3-dihydro-1H-indole

### Etape 1c :

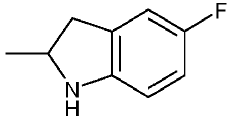

A une solution de 5 g du 5-fluoro-2-méthylindole dans 60 mL d'acide trifluoroacétique refroidit à 5°C, sont ajoutés en quatre fois 6.22 g de cyanoborohydrure de sodium. Le milieu réactionnel est agité à 0°C pendant 30 minutes puis cinq heures à la température ambiante.

Le milieu réactionnel est refroidit de nouveau à 5°C. Addition de 700 mL d'eau glacée, 150 mL de soude 31%, puis 300 mL d'acétate d'éthyle. Agitation à la température ambiante pendant une heure. Après décantation, la phase organique est séchée sur sulfate de magnésium, filtrée puis concentrée sous pression réduite. Le résidu obtenu est purifié sur colonne de silice, éluant : heptane/acétate d'éthyle 90/10 pour donner 2.14 g de 5-fluoro-2-methyl-2,3-dihydro-1H-indole dont les caractéristiques sont les suivantes :
Spectrométrie de Masse : méthode A
Temps de rétention Tr (min) = 0,24 ; [M+H]+ : m/z 152 ;

### Etape 2c :

### (R)-1-(5-Fluoro-2-methyl-2,3-dihydro-indol-1-yl)-2-phenoxy-propan-1-one : diastéréoisomère A

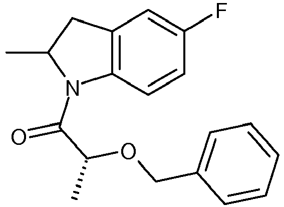

### Et

### (R)-1-(5-Fluoro-2-methyl-2,3-dihydro-indol-1-yl)-2-phenoxy-propan-1-one : diastéréoisomère B

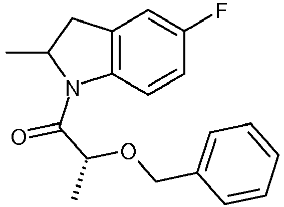

A une solution de 4.17 g d'acide o-benzyl-D-lactique dans 17 mL de DMF et 3.43 mL de pyridine sont ajoutés 2.9 g de 5-fluoro-2-methyl-2,3-dihydro-1H-indole et 5.3 g de N-[3-(diméthylamino)propyl]-N'-éthylcarbodiimide chlorhydrate.

Le milieu réactionnel est agité à la température ambiante pendant 18 heures.

Addition de 500 mL d'acétate d'éthyle et 500 mL d'eau. Après décantation, la phase organique est séchée sur sulfate de magnésium, filtrée puis concentrée sous pression réduite. Le résidu obtenu est purifié sur cartouche de silice : éluant : heptane, puis heptane/acétate d'éthyle 95/05, puis heptane/acétate d'éthyle 90/10 pour donner 2.8 g de (R)-1-(5-fluoro-2-methyl-2,3-dihydro-indol-1-yl)-2-phenoxy-propan-1-one : diastéréoisomère A sous forme d'une huile jaune dont les caractéristiques sont les suivantes :
Spectrométrie de Masse : méthode A
Temps de rétention Tr (min) = 1,08 ;
[M+H]+ : m/z 314

Et 2.63 g de (R)-1-(5-fluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,06 ;
[M+H]+ : m/z 314 ; pic de base : m/z 242

### Etape (3a)c :

### (+)-5-Fluoro-2-methyl-2,3-dihydro-1H-indole

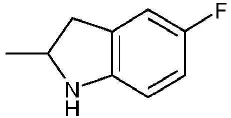

A une solution de 2.8 g de (R)-1-(5-fluoro-2-methyl-2,3-dihydro-indol-1-yl)-2-phenoxy-propan-1-one : diastéréoisomère A dans 28 mL d'éthanol sont ajoutés 28 mL d'acide chlorhydrique 37%.

Le milieu réactionnel est chauffé à reflux pendant 5 heures.

Le milieu réactionnel est concentré sous pression réduite. Le résidu obtenu est repris avec 260 mL d'eau. Addition de la soude 2N jusqu'au PH=10. Le milieu est extrait avec 200 mL de dichlorométhane. Après décantation, la phase organique est séchée sur sulfate de magnésium, filtrée puis concentrée sous pression réduite. Le résidu obtenu est purifié sur cartouche de silice : éluant : heptane/acétate d'éthyle 90/10 pour donner 1.29 g de (+)-5-fluoro-2-methyl-2,3-dihydro-1H-indole sous forme d'une huile jaune dont les caractéristiques sont les suivantes :
Spectrométrie de Masse : méthode A
Temps de rétention Tr (min) = 0,25 ; [M+H]+ : m/z 152
Pouvoir rotatoire : α_{D}= +8.2+/-0.7. C=1.801mg/0.5ML DMSO

### Etape (3b)c :

### (-)-5-Fluoro-2-methyl-2,3-dihydro-1H-indole

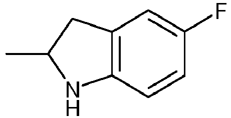

A une solution de 2.63 g (R)-1-(5-fluoro-2-methyl-2,3-dihydro-indol-1-yl)-2-phenoxy-propan-1-one : diastéréoisomère B de dans 26.5 mL d'éthanol sont ajoutés 26.5 mL d'acide chlorhydrique 37%.

Le milieu réactionnel est chauffé à reflux pendant 4 heures.

Le milieu réactionnel est concentré sous pression réduite. Le résidu obtenu est repris avec 250 mL d'eau. Addition de la soude 2N jusqu'au PH=10. Le milieu est extrait avec 200 mL de dichlorométhane. Après décantation, la phase organique est séchée sur sulfate de magnésium, filtrée puis concentrée sous pression réduite. Le résidu obtenu est purifié sur cartouche de silice : éluant : heptane/acétate d'éthyle 90/10 pour donner 1.11 g de (-)-5-fluoro-2-methyl-2,3-dihydro-1H-indole sous forme d'une huile jaune dont les caractéristiques sont les suivantes :
Spectrométrie de Masse : méthode A
Temps de rétention Tr (min) = 0,24 ; [M+H]+ : m/z 152 ;
PR=-7.9+/-0.4. C=3.023mg/0.5ML DMSO

### Exemple de référence 5c : 4,5-difluoro-2-méthyl-2,3-dihydro-1H-indole

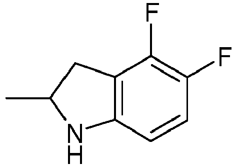

A une solution de 1 g de 4,5-difluoro-2-méthyl-indole dans 20 mL d'acide acétique sous argon refroidie à une température voisine de 5°C, sont ajoutés progressivement 750 mg de cyanoborohydrure de sodium. Le mélange réactionnel est agité à température ambiante pendant 3 heures et 5 mL d'eau sont ajoutés. Le mélange est ensuite concentré sous pression réduite et le résidu est repris dans de l'acétate d'éthyle, puis traité avec 60 mL d'une solution saturée d'hydrogénocarbonate de sodium. Après agitation pendant 1 heure puis décantation, la phase organique est séparée et la phase aqueuse est extraite avec 50 mL d'acétate d'éthyle. Les phases organiques sont réunies, lavées avec de la saumure saturée, séchées sur sulfate de magnésium anhydre, filtrées puis concentrées à sec sous pression réduite. Le résidu est purifié par chromatographie sur gel de silice, en éluant avec un mélange heptane/ acétate d'éthyle 80/20 v/v. On obtient ainsi 0,37 g de 4-hydroxy-2-méthyl-indoline sous forme d'une huile jaune dont les caractéristiques sont les suivantes :
Spectre RMN 1 H (400MHz, CDCl₃) : 1,17 (d, J=6,2 Hz, 3 H) ; 2,54 (dd, J=7,7 et 16,0 Hz, 1 H) ; 3,13 (dd, J=8,8 et 16,0 Hz, 1 H) ; 3,94 (m, 1 H) ; 5,75 (m large, 1 H) ; 6,17 (dd, J=3,5 et 8,6 Hz, 1 H) ; 6,89 (td J=8,6 et 11,4 Hz, 1 H)

### Exemple de référence 6c : 2-methyl-5,6-fluoro-2,3-dihydro-1H-indole

### Etape 1c:

### 4,5,-Difluoro-2-iodo-ainline

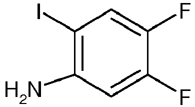

A une suspension de 6.45 g de 3,4-difluoroaniline dans 250 mL d'eau, sont ajoutés, à la température ambiante, 16.5 g d'iode et 6.3 g de bicarbonate de sodium. Le milieu réactionnel est agité à la température ambiante pendant 1 heure.

Addition d'une solution saturée de thiosulfate de sodium, puis extraction 3 fois à l'acétate d'éthyle. Les phases organique sont réunies, séchées sur sulfate de magnésium, filtrées puis concentrée sous pression réduite pour donner 12 g de 4,5,-difluoro-2-iodo-ainiline dont les caractéristiques sont les suivantes :
Spectrométrie de Masse : méthode A
Temps de rétention Tr (min) = 0,90 ;
[M+H]+ : m/z 256 ; pic de base : m/z 297

### Etape 2c :

### 4,5-Difluoro-2-prop-1-ynyl-phenylamine

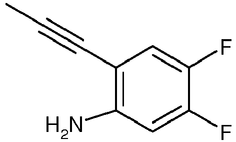

A une solution de 19 g de 4,5,-difluoro-2-iodo-ainiline dans 150 mL de trietyhlamine, sont ajoutés, à la température ambiante, 426 mg d'iodure de cuivre (I) et 523 mg du bis(triphenylphosphine)palladium(II)dichloride. La suspension est refroidit à - 30°C dans un bain de carboglace/éthanol. D'autre part, 20 mL de propyne sont condensés par barbotage dans un piège refroidit à -70°C à l'aide d'un mélange de carboglace/méthanol. Le propyne est additionné sur la suspension refroidit à -30°C. Le bain refroidissant est gardé. On laisse la température monter à l'ambiante pendant la nuit.

Le milieu réactionnel est filtré. Le filtrat est concentré à sec sous pression réduite. Le résidu obtenu est repris à l'eau et à l'acétate d'éthyle. Après décantation, la phase organique est séchée sur sulfate de magnésium, filtrée puis concentrée sous pression réduite. Le résidu obtenu est purifié sur colonne de silice, éluant : cyclohexane/dichlorométhane 80/20 pour donner 10.8 g de 4,5-difluoro-2-prop-1-ynyl-phenylamine dont les caractéristiques sont les suivantes :
Spectrométrie de Masse : méthode A
Temps de rétention Tr (min) = 0,90 ; [M+H]+ : m/z 168

### Etape 3c :

### 5,6-Difluoro-2-methyl-indole

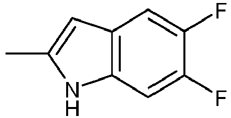

A une solution de 10.8 g du 4,5-difluoro-2-prop-1-ynyl-phenylamine dans 100 mL de DMF, sont ajoutés 246 mg d'iodure de cuivre (I). Le milieu réactionnel est chauffé à reflux pendant une heure.

Après refroidissement, le milieu réactionnel est filtré. Le filtrat est concentré sous pression réduite. Le résidu brut obtenu est purifié sur colonne de silice, éluant : cyclohexane/dichlorométhane 90/10 pour donner 7.2 g de 5,6-difluoro-2-methyl-indole dont les caractéristiques sont les suivantes :
Spectrométrie de Masse : méthode A
Temps de rétention Tr (min) = 0,94;
[M-H]- : m/z 166

### Etape 4c :

### 5,6-Difluoro-2-methyl-2,3-dihydro-1H-indole

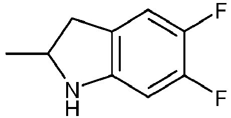

A une solution de 7.2 g du 5,6-difluoro-2-méthylindole dans 220 mL d'acide acétique refroidit à 15°C, sont ajoutés en 3 fois 8.11 g de cyanoborohydrure de sodium. Le milieu réactionnel est agité à 15°C pendant 30 minutes puis 4 heures à la température ambiante.

Le milieu réactionnel est refroidit de nouveau à 5°C. Addition de 900 mL d'eau glacée. Addition de l'ammoniaque 30% jusqu'au PH = 9. Extraction 3 fois au dichlorométhane. Les phases organique sont réunies, séchées sur sulfate de magnésium, filtrées puis concentrée sous pression réduite. Le résidu obtenu est purifié sur colonne de silice, éluant : heptane/acétate d'éthyle 90/10 pour donner 6.3 g de 5,6-difluoro-2-methyl-2,3-dihydro-1H-indole dont les caractéristiques sont les suivantes :
Spectrométrie de Masse : méthode A
Temps de rétention Tr (min) = 0,53 ;
[M+H]+ : m/z 170 ;

### Etape 5c :

### (R)-1-(5,6-Difluoro-2-methyl-2,3-dihydro-indol-1-yl)-2-phenoxy-propan-1-one : diastéréoisomère A

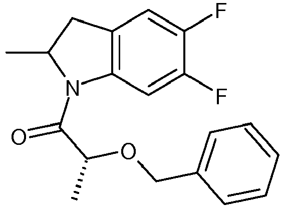

### Et

### (R)-1-(5,6-Difluoro-2-methyl-2,3-dihydro-indol-1-yl)-2-phenoxy-propan-1-one : diastéréoisomère B

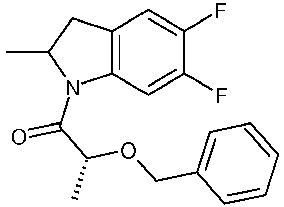

Les produits sont préparés en suivant le mode opératoire décrit à l'exemple de référence 1 c (Etape 2c) à partir de 6.3 g du (5,6-difluoro-2-methyl-2,3-dihydro-1H-indole et de 6.8 g d'acide o-benzyl-D-lactique. Après purification sur colonne de silice, on obtient 6.45 g de (R)-1-(5,6-difluoro-2-methyl-2,3-dihydro-indol-1-yl)-2-phenoxy-propan-1-one : diastéréoisomère A sous forme d'une huile jaune dont les caractéristiques sont les suivantes :
Spectrométrie de Masse : méthode A
Temps de rétention Tr (min) = 1,11 ;
M+H]+ : m/z 332 ; pic de base: m/z 260

Et 6.29 g de (R)-1-(5,6-difluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,10 ;
[M+H]+ : m/z 332 ; pic de base : m/z 260

### Etape (6a)c :

### (+)-5,6-Difluoro-2-methyl-2,3-dihydro-1H-indole

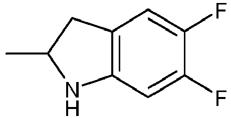

Le produit est préparé en suivant le mode opératoire décrit à l'exemple de référence 4c (Etape 3c) à partir de 6.45 g du (R)-1-(5,6-difluoro-2-methyl-2,3-dihydro-indol-1-yl)-2-phenoxy-propan-1-one : diastéréoisomère A et de 64.5 mL d'acide chlorhydrique 37%.

Après purification sur colonne de silice, éluant : heptane/acétate d'éthyle 90/10, on obtient 2.7 g de (+)-5,6-difluoro-2-methyl-2,3-dihydro-1H-indole dont les caractéristiques sont les suivantes :
Spectrométrie de Masse : méthode A
Temps de rétention Tr (min) = 0,53 ; [M+H]+ : m/z 170 ;
Pouvoir rotatoire : α_{D}= +17.6+/-0.7. C= 1.834mg/0.5ML DMSO

### Etape (6b)c :

### (-)-5,6-Difluoro-2-methyl-2,3-dihydro-1H-indole

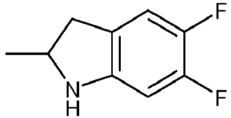

Le produit est préparé en suivant le mode opératoire décrit à l'exemple référence 4c (Etape 3c) à partir de 6.29 g du (R)-1-(5,6-difluoro-2-methyl-2,3-dihydro-indol-1-yl)-2-phenoxy-propan-1-one : diastéréoisomère B et de 63 mL d'acide chlorhydrique 37%.

Après purification sur colonne de silice, éluant : heptane/acétate d'éthyle 90/10 , on obtient 2.76 g de (-)-5,6-difluoro-2-methyl-2,3-dihydro-1H-indole dont les caractéristiques sont les suivantes :
Spectrométrie de Masse : méthode A
Temps de rétention Tr (min) = 0,55 ; [M+H]+ : m/z 170 ;
Pouvoir rotatoire : α_{D}= -6.7+/-0.6. C=1.832mg/0.5ML DMSO

### Exemple de référence 7c : 4-Bromo-2-methyl-2,3-dihydro-1H-indole

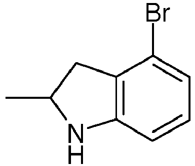

A une solution de 8,90 g de 4-bromo-2-méthyl-indole (qui peut être préparé selon le brevet US2010/160647 A1, 2010) dans 310 mL d'acide acétique sous argon refroidie à une température voisine de 14°C, sont ajoutés progressivement 7,99 g de cyanoborohydrure de sodium. Le mélange réactionnel est agité à une température voisine de 14°C pendant 15 minutes, puis il est laissé se réchauffer à température ambiante. Après 2 heures, le mélange réactionnel est versé dans un erlen contenant de l'eau glacée (200mL) puis le pH est amené jusqu'à 9 avec une solution d'ammoniaque aqueux. Le milieu réactionnel est extrait avec du dichlorométhane (2x200 mL) puis les phases organiques sont réunies, lavées avec de l'eau, séchées sur sulfate de magnésium, filtrées, puis concentrées à sec sous pression réduite. Après purification du résidu par chromatographie sur colonne de silice en éluant avec un mélange d'heptane et d'acétate d'éthyle (90/10), on obtient 5,92 g de 4-bromo-2-méthyl-indoline dont les caractéristiques sont les suivantes :
Spectre RMN 1H (400MHz) : 1,18 (d, J=6,1 Hz, 3 H); 2,47 (dd, J=7,9 et 16,0 Hz, 1 H); 3,05 (dd, J=8,9 et 16,1 Hz, 1 H); 3,84 à 3,97 (m, 1 H); 5,98 (s large, 1 H); 6,39 (d, J=7,7 Hz, 1 H); 6,62 (dd, J=0,8 et 8,0 Hz, 1 H); 6,82 (t, J=7,9 Hz, 1 H)

### Exemple de référence 8c : (+)-4-Fluoro-2-méthyl-2,3-dihydro-1H-indole et (-)-4-Fluoro-2-méthyl-2,3-dihydro-1H-indole

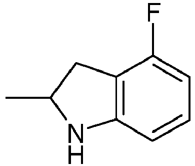

### Etape 1c

A une solution de 5 g de 3-fluoro-2-méthyl-aniline dans 25 mL de tetrahydrofuranne, sont ajoutés 9,9 g de di-*tert*-butyl-dicarbonate. Le mélange réactionnel est chauffé à reflux sous agitation pendant 16 heures, puis il est refroidi à température ambiante et concentré à sec sous pression réduite. Le résidu est trituré dans 20 mL de cyclohexane et le précipité obtenu est filtré sur verre fritté, essoré puis séché sous pression réduite à 40°C. On obtient ainsi 7,1 g de (3-fluoro-2-méthyl-phényl)-carbamate de *tert-*butyle sous forme d'un solide blanc brillant dont les caractéristiques sont les suivantes :
Spectrométrie de Masse : EI : [M]+. m/z = 225
méthode A
Temps de rétention Tr (min) = 1,04 ;
[M+H-tBu]+ : m/z 170 ; mic de base m/z : 211
Point de fusion (Kofler) : 72°C

### Etape 2c

A une solution de 0,5 g de (3-fluoro-2-méthyl-phényl)-carbamate de *tert*-butyle dans 10 mL de tetrahydrofuranne sous argon et refroidie à -40°C, sont additionnés au goutte à goutte 4,3 mL d'une solution de sec-butyllithium 1,3 M dans le cyclohexane/ hexane 98/2 v/v en maintenant la température entre -40°C et -30°C. Le mélange réactionnel est ensuite refroidi à -50°C, puis une solution de 0,27 mL de N-méthoxy-N-méthyl-acétamide dans 4 mL de tetrahydrofuranne est additionnée au goutte à goutte en maintenant la température entre -50°C et -40°C. Le mélange est ensuite laissé se réchauffer à environ -10°C, puis agité à cette température pendant 0,5 heure. Il est ensuite traité avec 5,6 mL d'une solution d'acide chlorhydrique 1 N puis dilué avec 20 mL d'éther diéthylique et laissé se réchauffer à température ambiante sous agitation pendant 1 heure. Après décantation, la phase organique est séparée et la phase aqueuse est extraite avec 40 mL d'éther diéthylique. Les phases organiques sont réunies, lavées par 3x40 mL d'eau, séchées sur sulfate de magnésium, filtrées, puis concentrées à sec sous pression réduite. Le résidu est purifié par chromatographie sur une cartouche de 30 g silice 15-40 µm, en éluant avec un mélange cyclohexane/ acétate d'éthyle 90/10 v/v, à un débit de 30 mL/ min, puis sur une cartouche de 30 g silice 15-40 µm, en éluant avec du dichlorométhane pur, à un débit de 30 mL/ min. On obtient ainsi 0,38 g de [3-fluoro-2-(2-oxo-propyl)-phényl]-carbamate de *tert*-butyle sous forme d'un solide blanc dont les caractéristiques sont les suivantes :
Spectrométrie de Masse : EI : [M]+. m/z = 267
méthode A
Temps de rétention Tr (min) = 0,93 ;
[M+Na]+ : m/z 290 ;

### Etape 3c

A une solution de 0,35 g de [3-fluoro-2-(2-oxo-propyl)-phényl]-carbamate de *tert-*butyle dans 13 mL de dichlorométhane anhydre à température ambiante, sont additionnés 1,43 mL d'acide trifluoroacétique. Le mélange réactionnel est ensuite agité à température ambiante pendant 24h, puis il est dilué avec 27 mL de dichlorométhane et traité avec 25 mL d'une solution d'hydrogénocarbonate de sodium à 5%. Après agitation à température ambiante pendant 1 heure puis décantation, la phase organique est séparée et la phase aqueuse est extraite avec 25 mL de dichlorométhane. Les phases organiques sont réunies, lavées avec de la saumure saturée, séchées sur sulfate de magnésium, filtrées, puis concentrées à sec sous pression réduite. On obtient ainsi 0,19 g de 4-fluoro-2-méthyl-indole sous forme d'une huile rougé foncé dont les caractéristiques sont les suivantes :
Spectre RMN 1 H (400MHz, CDCl3): 2,46 (s, 3 H) ; 6,30 (m large, 1 H) ; 6,74 (dd, *J*=7,9 et 10,6 Hz, 1 H) ; 7,02 (dt, *J*=4,9 et 7,9 Hz, 1 H) ; 7,08 (d, *J*=7,9 Hz, 1 H) ; 7,95 (m étalé, 1 H)
Spectrométrie de Masse : El : [M]+. m/z = 149

### Etape 4c

A une solution de 2,87 g de 4-fluoro-2-méthyl-indole dans 98 mL d'acide acétique sous argon refroidie à une température voisine de 14°C, sont ajoutés progressivement 3,63 g de cyanoborohydrure de sodium. Le mélange réactionnel est laissé se réchauffer à température ambiante. Après 2 heures, le mélange réactionnel est versé dans un mélange d'eau et de glace, puis il est traité avec une solution d'ammoniaque à 28% jusqu'à pH 9. Le mélange est ensuite extrait deux fois avec du dichlorométhane. Les phases organiques sont réunies, séchées sur sulfate de magnésium anhydre, filtrées puis concentrées à sec sous pression réduite. Le résidu est purifié sur une colonne de 300 g de silice, en éluant avec un gradient heptane/ acétate d'éthyle 100/0 à 90/10 v/v. On obtient ainsi 2,19 g de 4-fluoro-2-méthyl-2,3-dihydro-1 H-indole sous forme d'une huile incolore dont les caractéristiques sont les suivantes :
Spectre RMN 1H (400MHz): 1,18 (d, *J*=6,3 Hz, 3 H) ; 2,49 (dd partiellement masqué, *J*=7,6 et 15,7 Hz, 1 H) ; 3,08 (dd, *J*=9,0 et 15,7 Hz, 1 H) ; 3,92 (m, 1 H) ; 5,87 (s large, 1 H) ; 6,20 à 6,31 (m, 2 H) ; 6,90 (td, *J*=5,9 et 8,1 Hz, 1 H)
Spectrométrie de Masse : méthode A
Temps de rétention Tr (min) = 0,50 ;
[M+H]+ : m/z 152

### Etape 5c

A une solution de 0,76 g d'acide o-benzyl-D-lactique dans 3,5 mL de diméthylformamide sous argon, sont ajoutés 0,69 mL de pyridine et 1,05 g de N-[3-(diméthylamino)propyl]-N'-éthylcarbodiimide chlorhydrate. Le mélange réactionnel est agité à temprérature ambiante pendant 10 minutes, puis 0,64 g de 4-fluoro-2-méthyl-2,3-dihydro-1H-indole est additionné. Le mélange réactionnel est agité à temprérature ambiante pendant 20 heures, puis il est traité avec 20 mL d'eau et extrait avec 3x15 mL d'acétate d'éthyle. Les phases organiques sont réunies, lavées avec 15 mL d'eau, séchées sur sulfate de magnésium, filtrées puis concentrées à sec sous pression réduite. Le résidu est purifié par chromatographie sur une cartouche de 90 g de silice 15-40 µm, en éluant avec du dichlorométhane pur, puis sur une cartouche de 100 g de silice 15-40 µm, en éluant avec de l'heptane pur puis avec des mélanges heptane/ acétate d'éthyle 95/5 puis 90/10 v/v, à un débit de 85 mL/ min. On obtient ainsi 0,33 g du diastéréoisomère (1) de la (R)-2-benzyloxy-1-(4-fluoro-2-méthyl-2,3-dihydro-indol-1-yl)-propan-1-one sous forme d'une huile incolore et 0,38 g du diastéréoisomère (2) de la (R)-2-benzyloxy-1-(-4-fluoro-2-méthyl-2,3-dihydro-indol-1-yl)-propan-1-one sous forme d'un solide blanc. Un deuxième essai dans des conditions identiques à partir de 1,85 g d'acide o-benzyl-D-lactique et de 1,55 g de 4-fluoro-2-méthyl-indoline permet d'obtenir 1,39 g du diastéréoisomère (1) de la (R)-2-benzyloxy-1-(4-fluoro-2-méthyl-2,3-dihydro-indol-1-yl)-propan-1-one sous forme d'une huile incolore et 1,34 g du diastéréoisomère (2) de la
(R)-2-benzyloxy-1-(-4-fluoro-2-méthyl-2,3-dihydro-indol-1-yl)-propan-1-one sous forme d'un solide blanc.

Les deux lots de diastéréoisomère (1) de la (R)-2-benzyloxy-1-(4-fluoro-2-méthyl-2,3-dihydro-indol-1-yl)-propan-1-one sont réunis et dissous dans 75 mL d'acétate d'éthyle. Le mélange est filtré sur papier puis concentré à sec sous pression réduite. On obtient ainsi 1,66 g du diastéréoisomère (1) de la (R)-2-benzyloxy-1-(4-fluoro-2-méthyl-2,3-dihydro-indol-1-yl)-propan-1-one sous forme d'une huile visqueuse jaune très pâle dont les caractéristiques sont les suivantes :
Spectre RMN 1H (400MHz): 1,18 (d, *J*=6,4 Hz, 3 H) ; 1,39 (d, *J*=6,4 Hz, 3 H) ; 2,71 (d, *J*=16,3 Hz, 1 H) ; 3,29 (dd partiellement masqué, *J*=8,8 et 16,3 Hz, 1 H) ; 4,46 à 4,56 (m, 3 H) ; 4,75 (m, 1 H) ; 6,91 (t, *J*=8,7 Hz, 1 H) ; 7,19 à 7,37 (m, 6 H) ; 7,87 (m étalé, 1 H)
Spectrométrie de Masse : méthode A
Temps de rétention Tr (min) = 1,09 ;
[M+H]+ : m/z 314 ; [M+Na]+ : m/z 336 ; pic de base- : m/z 242

Les deux lots de diastéréoisomère (2) de la (R)-2-benzyloxy-1-(4-fluoro-2-méthyl-2,3-dihydro-indol-1-yl)-propan-1-one sont réunis et dissous dans 75 mL d'acétate d'éthyle. Le mélange est filtré sur papier puis concentré à sec sous pression réduite. On obtient ainsi 1,70 g du diastéréoisomère (2) de la (R)-2-benzyloxy-1-(4-fluoro-2-méthyl-2,3-dihydro-indol-1-yl)-propan-1-one sous forme d'un solide blanc dont les caractéristiques sont les suivantes :
Spectre RMN 1H (400MHz) : 1,18 (d, *J*=6,4 Hz, 3 H) ; 1,40 (d, *J*=6,5 Hz, 3 H) ; 2,72 (d, *J*=16,4 Hz, 1 H) ; 3,28 à 3,37 (m partiellement masqué, 1 H) ; 4,40 à 4,57 (m, 3 H) ; 4,66 (m, 1 H) ; 6,90 (dt, *J*=0,8 et 8,8 Hz, 1 H) ; 7,20 à 7,39 (m, 6 H) ; 7,91 (m large, 1 H)
Spectrométrie de Masse : méthode A
Temps de rétention Tr (min) = 1,08 ;
[M+H]+ : m/z 314 ; [M+Na]+ : m/z 336 ; pic de base : m/z 242

### Etape 6c

A une solution de 1,66 g du diastéréoisomère (1) de la (R)-2-benzyloxy-1-(4-fluoro-2-méthyl-2,3-dihydro-indol-1-yl)-propan-1-one dans 200 mL d'éthanol absolu, sont ajoutés 8 mL d'acide chlorhydrique concentré. Le mélange réactionnel est chauffé à reflux sous agitation pendant 40 heures, puis il est refroidi à temprérature ambiante et concentré à sec sous pression réduite. Le résidu est repris dans 250 mL d'eau, alcalinisé avec de la soude concentrée jusqu'à pH 14, puis le mélange est extrait avec 3x200 mL de dichlorométhane. Les phases organiques sont réunies, lavées avec de l'eau, séchées sur sulfate de magnésium, filtrées puis concentrées à sec sous pression réduite. Le résidu est purifié par chromatographie sur une cartouche de 70 g de silice 15-40 µm, en éluant avec de l'heptane pur puis avec un mélange heptane/ acétate d'éthyle 95/5 à un débit de 50 mL/ min, puis sur une cartouche de 70 g de silice 15-40 µm, en éluant avec du cyclohexane pur puis avec un mélange cyclohexane/ dichlorométhane 70/30, à un débit de 50 mL/ min. On obtient ainsi 0,50 g de (+)-4-fluoro-2-méthyl-indoline sous forme d'une huile incolore dont les caractéristiques sont les suivantes :
Spectre RMN 1H (400MHz): 1,18 (d, *J*=6,4 Hz, 3 H) ; 2,49 (dd partiellement masqué, J=7,6 et 15,7 Hz, 1 H) ; 3,08 (dd, *J*=8,8 et 15,7 Hz, 1 H) ; 3,92 (m, 1 H) ; 5,87 (s large, 1 H) ; 6,21 à 6,28 (m, 2 H) ; 6,90 (dt, *J*=5,9 et 8,1 Hz, 1 H)
Spectrométrie de Masse : méthode A
Temps de rétention Tr (min) = 0,48 ;
[M+H]+ : m/z 152
Pouvoir rotatoire : α_{D} = +40,8° +/-0,9 (c = 2,223 mg dans 0,5 mL de DMSO)

### Etape 6c

A une solution de 1,69 g du diastéréoisomère (2) de la (R)-2-benzyloxy-1-(4-fluoro-2-méthyl-2,3-dihydro-indol-1-yl)-propan-1-one dans 200 mL d'éthanol absolu, sont ajoutés 8,2 mL d'acide chlorhydrique concentré. Le mélange réactionnel est chauffé à reflux sous agitation pendant 40 heures, puis il est refroidi à temprérature ambiante et concentré à sec sous pression réduite. Le résidu est repris dans 200 mL d'eau, alcalinisé avec de la soude concentrée jusqu'à pH 14, puis le mélange est extrait avec 3x200 mL de dichlorométhane. Les phases organiques sont réunies, lavées avec de l'eau, séchées sur sulfate de magnésium, filtrées puis concentrées à sec sous pression réduite. Le résidu est purifié par chromatographie sur une cartouche de 70 g de silice 15-40 µm, en éluant avec du cyclohexane pur puis avec un mélange cyclohexane/ dichlorométhane 70/30, à un débit de 50 mL/ min. On obtient ainsi 0,56 g de (-)-4-fluoro-2-méthyl-indoline sous forme d'une huile jaune très pâle dont les caractéristiques sont les suivantes :
Spectre RMN 1H (400MHz) : 1,18 (d, *J*=6,2 Hz, 3 H) ; 2,49 (dd partiellement masqué, J=7,6 et 15,7 Hz, 1 H) ; 3,08 (dd, *J*=9,0 et 15,7 Hz, 1 H) ; 3,92 (m, 1 H); 5,87 (s large, 1 H) ; 6,20 à 6,29 (m, 2 H) ; 6,90 (dt, *J*=5,9 et 8,1 Hz, 1 H)
Spectrométrie de Masse : méthode A
Temps de rétention Tr (min) = 0,49 ;
[M+H]+ : m/z 152
Pouvoir rotatoire : α_{D} = -33,7° +/-0,7 (c = 2,741 mg dans 0,5 mL de DMSO)

### Exemple de référence 9c : 6-Fluoro-2-methyl-2,3-dihydro-1H-indole

### Etape 1c :

### 5-Fluoro-2-prop-1-ynyl-phenylamine

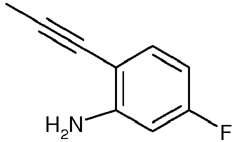

A une solution de 5 g de 5-fluoro-2-iodo-ainiline dans 150 mL de trietyhlamine, sont ajoutés, à la température ambiante, 121 mg d'iodure de cuivre (I) et 148 mg du bis(triphenylphosphine)palladium(II)dichloride. La suspension est refroidit à -30°C dans un bain de carboglace/éthanol. D'autre part, 10 mL de propyne sont condensés par barbotage dans un piège refroidit à -70°C à l'aide d'un mélange de carboglace/méthanol. Le propyne est additionné sur la suspension refroidit à -30°C. Le bain refroidissant est gardé. On laisse la température monter à l'ambiante pendant la nuit.

Le milieu réactionnel est filtré. Le filtrat est concentré à sec sous pression réduite. Le résidu obtenu est repris à l'eau et à l'acétate d'éthyle. Après décantation, la phase organique est séchée sur sulfate de magnésium, filtrée puis concentrée sous pression réduite. Le résidu obtenu est purifié sur colonne de silice, éluant : cyclohexane/acétate d'éthyle 95/05 pour donner 1.7 g de 5-difluoro-2-prop-1-ynyl-phenylamine dont les caractéristiques sont les suivantes :
Spectrométrie de Masse : méthode A
Temps de rétention Tr (min) = 0,84 ;
[M+H]+ : m/z 150 ;

### Etape 2c :

### 6-Difluoro-2-methyl-indole

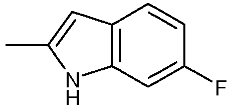

A une solution de 1.7 g du 5-difluoro-2-prop-1-ynyl-phenylamine dans 50 mL de DMF, sont ajoutés 43 mg d'iodure de cuivre (I). Le milieu réactionnel est chauffé à reflux pendant une heure.

Après refroidissement, le milieu réactionnel est filtré. Le filtrat est concentré sous pression réduite. Le résidu brut obtenu est purifié sur colonne de silice, éluant : cyclohexane/acétate d'éthyle 90/10 pour donner 1.1 g de 6-fluoro-2-methyl-indole dont les caractéristiques sont les suivantes :
Spectrométrie de Masse : méthode A
Temps de rétention Tr (min) = 0,89 ; [M+H]+ : m/z 150 ;

### Etape 3c :

### 6-Fluoro-2-methyl-2,3-dihydro-1 H-indole

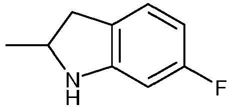

Le produit est préparé en suivant le mode opératoire décrit à l'exemple de reference 2c (Etape 1c) à partir de 1.4 g du 6-fluoro-2-methyl-indole, de 51 ml d'acide acétique et de 1.9 g de cyanoborohydrure de sodium. Après purification sur colonne de silice, éluant : heptane/acétate d'éthyle 90/10, on obtient 1.33 g de 6-fluoro-2-methyl-2,3-dihydro-1H-indole qui est utilisé tel quel dans l'étape suivante.

### Etape 4c :

### (R)-1-(6-Fluoro-2-methyl-2,3-dihydro-indol-1-yl)-2-phenoxy-propan-1-one: diastéréoisomère A

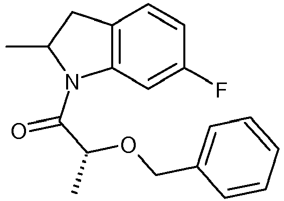

### Et

### (R)-1-(6-Fluoro-2-methyl-2,3-dihydro-indol-1-yl)-2-phenoxy-propan-1-one: diastéréoisomère B

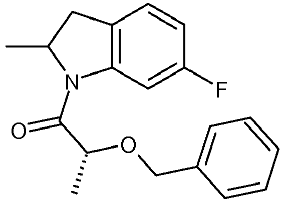

Les produits sont préparés en suivant le mode opératoire décrit à l'exemple de référence 1 c (Etape 2c) à partir de 1.33 g du (6-fluoro-2-methyl-2,3-dihydro-1 H-indole et de 2.1 g d'acide o-benzyl-D-lactique. Après purification sur colonne de silice, on obtient 1.3 g de (R)-1-(6-fluoro-2-methyl-2,3-dihydro-indol-1-yl)-2-phenoxy-propan-1-one : diastéréoisomère A dont les caractéristiques sont les suivantes :
Spectrométrie de Masse : méthode A
Temps de rétention Tr (min) = 1,09 ;
[M+H]+ : m/z 314 ; pic de base : m/z 242

Et 1.13 g de (R)-1-(6-fluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,08 ;
[M+H]+ : m/z 314 ; [M+Na]+ : m/z 336 ;
pic de base : m/z 242

### Etape (5a)c :

### (+)-6-Fluoro-2-methyl-2,3-dihydro-1 H-indole

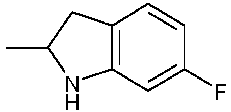

Le produit est préparé en suivant le mode opératoire décrit à l'exemple de référence 1c (Etape 3c) à partir de 1.3 g du (R)-1-(6-fluoro-2-methyl-2,3-dihydro-indol-1-yl)-2-phenoxy-propan-1-one : diastéréoisomère A et de 13 mL d'acide chlorhydrique 37%.

Après traitement, on obtient 547 mg de (+)-6-fluoro-2-methyl-2,3-dihydro-1H-indole dont les caractéristiques sont les suivantes :
Spectrométrie de Masse : méthode A
Temps de rétention Tr (min) = 0,48 ; [M+H]+ : m/z 152 ;
Pouvoir rotatoire : □D= +35.0+/-0.7. C=2.899mg/0.5ML DMSO

### Etape (5b)c :

### (-)-6-Fluoro-2-methyl-2,3-dihydro-1 H-indole

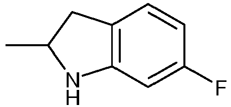

Le produit est préparé en suivant le mode opératoire décrit à l'exemple de référence 1 c (Etape 3c) à partir de 1.13 g du (R)-1-(6-fluoro-2-methyl-2,3-dihydro-indol-1-yl)-2-phenoxy-propan-1-one : diastéréoisomère B et de 12 mL d'acide chlorhydrique 37%.

Après traitement, on obtient 540 mg de (R)-6-fluoro-2-methyl-2,3-dihydro-indol-1-yl)-2-phenoxy-propan-1-one dont les caractéristiques sont les suivantes :
Spectrométrie de Masse : méthode A
Temps de rétention Tr (min) = 0,48 ; [M+H]+ : m/z 152 ;
Pouvoir Rotatoire α_{D}= -32.6+/-1.0. C=1.506mg/0.5ML DMSO

### Exemple de référence 10c : 4-chloro-5-fluoro-2-methyl-2,3-dihydro-1 H-indole

### Etape 1c :

### 3-chloro-4-fluoro-2-iodo-aniline

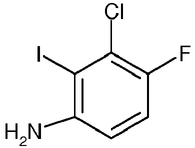

A une suspension de 50 g de 3-chloro-4-fluoroaniline dans 800 mL d'eau, sont ajoutés, à la température ambiante, 113.3 g d'iode et 43.3 g de bicarbonate de sodium. Le milieu réactionnel est agité à la température ambiante pendant 18 heures.

Addition d'une solution saturée de thiosulfate de sodium, puis extraction 3 fois à l'acétate d'éthyle. Les phases organique sont réunies, séchées sur sulfate de magnésium, filtrées puis concentrées sous pression réduite. Le résidu obtenu est purifié sur colonne de silice, éluant : cyclohexane/acétate d'éthyle 95/05 puis 85/15 pour donner 40.9 g du 5-chloro-4-fluoro-2-iodo-ainline dont les caractéristiques sont les suivantes :
Spectre RMN 1 H (400 MHz) : 5,29 (s large, 2 H) ; 6,87 (d, *J*=6,9 Hz, 1 H) ; 7,61 (d, *J*=8,8 Hz, 1 H)
Spectrométrie de Masse : méthode A
Temps de rétention Tr (min) = 0,99 ;
[M+H]+ : m/z 272 ; pic de base : m/z 313
et 12.5 g de 3-chloro-4-fluoro-2-iodo-ainline dont les caractéristiques sont les suivantes :
Spectre RMN 1 H (400 MHz) : 5,38 (s large, 2 H) ; 6,76 (dd, *J*=4,8 et 8,9 Hz, 1 H) ; 7,16 (t, *J*=8,9 Hz, 1 H)
Spectrométrie de Masse : méthode A
Temps de rétention Tr (min) = 0,95 ;
[M+H]+ : m/z 271

### Etape 2c :

### 3-chloro-4-fluoro-2-prop-1-ynyl-phenylamine

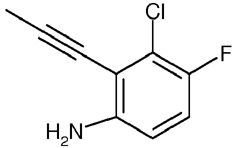

A une solution de 9 g de 3-chloro-4-fluoro-2-iodo-ainline dans 160 mL de trietyhlamine, sont ajoutés, à la température ambiante, 364 mg d'iodure de cuivre (I) et 470 mg du bis(triphenylphosphine)palladium(II)dichloride. La suspension est refroidit à - 30°C dans un bain de carboglace/éthanol. D'autre part, environ 20 mL de propyne sont condensés par barbotage dans un piège refroidit à -70°C à l'aide d'un mélange de carboglace/méthanol. Le propyne est additionné sur la suspension refroidit à -30°C. Le bain refroidissant est gardé. On laisse la température monter à l'ambiante pendant la nuit.

Le milieu réactionnel est filtré. Le filtrat est concentré à sec sous pression réduite. Le résidu obtenu est purifié sur colonne de silice, éluant : heptane/acétate d'éthyle 90/10 pour donner 1.76 g de 3-chloro-4-fluoro-2-prop-1-ynyl-phenylamine dont les caractéristiques sont les suivantes :
Spectrométrie de Masse : méthode A
Temps de rétention Tr (min) = 0,92 ;
[M+H]+ : m/z 184 ; pic de base : m/z 149

### Etape 3c :

### 4-chloro-5-fluoro-2-méthylindole

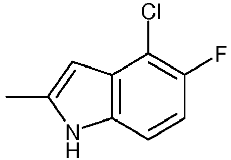

A une solution de 1.56 g du 3-chloro-4-fluoro-2-prop-1-ynyl-phenylamine dans 17 mL de DMF, sont ajoutés 32 mg d'iodure de cuivre (I). Le milieu réactionnel est chauffé à reflux pendant 45 minutes.

Après refroidissement, le milieu réactionnel est filtré. Le filtrat est concentré sous pression réduite. Le résidu brut obtenu est purifié sur colonne de silice, éluant : heptane/toluène 70/30 pour donner 0.5 g de 4-chloro-5-fluoro-2-méthylindole dont les caractéristiques sont les suivantes :
Spectrométrie de Masse : méthode A
Temps de rétention Tr (min) = 1,01 ;
[M-H]- : m/z 182

### Etape 4c :

### 4-Chloro-5-fluoro-2-methyl-2,3-dihydro-1 H-indole

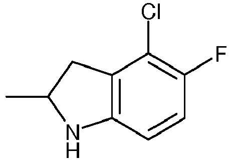

A une solution de 700 mg du 4-chloro-5-fluoro-2-méthylindole dans 16 mL d'acide acétique refroidit à 15°C, sont ajoutés en une fois 719 mg de cyanoborohydrure de sodium. Le milieu réactionnel est agité à 15°C pendant 10 minutes puis 90 minutes à la température ambiante.

Le milieu réactionnel est refroidit de nouveau à 5°C. Addition de l'eau glacée. Addition de l'ammoniaque 30% jusqu'au PH = 9. Extraction à l'acétate d'éthyle. La phase organique est séchée sur sulfate de magnésium, filtrée puis concentrée sous pression réduite pour donner 683 mg de 4-chloro-5-fluoro-2-methyl-2,3-dihydro-1 H-indole dont les caractéristiques sont les suivantes :
Spectrométrie de Masse : méthode A
Temps de rétention Tr (min) = 0,75 ;
[M+H]+ : m/z 186 ;

### Exemple de référence 11c : Synthèse de la (-)-2-isopropyl-indoline :

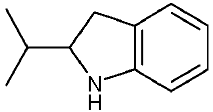

### Etape 1c

A une solution de 5,0 g de 2-isopropyl-indole dans 50 mL d'acide acétique sous argon refroidie à une température voisine de 15°C, sont ajoutés progressivement 4,65 g de cyanoborohydrure de sodium. Le mélange réactionnel est agité à une température voisine de 15°C pendant 2 heures, puis il est traité avec 25 mL d'eau. Il est ensuite refroidi à une température voisine de 5°C et alcalinisé par ajout progressif de soude en poudre. Le mélange réactionnel est laissé se réchauffer à température ambiante puis il est agité pendant 16 heures. Le précipité est filtré sur verre fritté et lavé avec 50 mL d'eau puis 50 mL d'acétate d'éthyle. Le filtrat est dilué avec 50 mL d'eau et 70 mL d'acétate d'éthyle puis décanté. La phase organique est séparée et la phase aqueuse est extraite avec 2x100 mL d'acétate d'éthyle. Les phases organiques sont réunies, lavées avec de la saumure saturée, séchées sur sulfate de magnésium anhydre, filtrées puis concentrées à sec sous pression réduite. Le résidu est purifié par chromatographie sur une cartouche de 90 g de silice 15-40 µm, en éluant avec un mélange cyclohexane/ acétate d'éthyle 95/5 v/v, à un débit de 50 mL/ min. On obtient ainsi 4,0 g de (±)-2-isopropyl-indoline sous forme d'une huile incolore qui cristallise en un solide blanc dont les caractéristiques sont les suivantes :
Spectrométrie de Masse : méthode A
Temps de rétention Tr (min) = 0,41 ;
[M+H]+ : m/z 162

### Etape 2c

A une solution de 4,36 g d'acide o-benzyl-D-lactique dans 17 mL de N,N-diméthylformamide sous argon, sont ajoutés 3,9 mL de pyridine et 6,15 g de N-[3-(diméthylamino)propyl]-N'-éthylcarbodiimide chlorhydrate. Le mélange réactionnel est agité à temprérature ambiante pendant 15 minutes, puis une solution de 3,9 g de (±)-2-isopropyl-indoline dans 3 mL de N,N-diméthylformamide est additionnée. Le mélange réactionnel est agité à temprérature ambiante pendant 16 heures, puis il est versé dans un mélange de 100 mL d'eau et 80 mL d'acétate d'éthyle et décanté. La phase organique est séparée et la phase aqueuse est extraite avec 2x80 mL d'acétate d'éthyle. Les phases organiques sont réunies, lavées avec 60 mL d'eau, séchées sur sulfate de magnésium, filtrées puis concentrées à sec sous pression réduite. Le résidu est purifié par chromatographie sur une cartouche de 400 g de silice 15-40 µm, en éluant avec de l'heptane pur, puis avec un mélange heptane/ acétate d'éthyle 95/5 v/v, à un débit de 100 mL/ min. On obtient ainsi 3,20 g du diastéréoisomère (1) de la (R)-2-benzyloxy-1-(2-isopropyl-2,3-dihydro-indol-1-yl)-propan-1-one sous forme d'une huile jaune très pâle dont les caractéristiques sont les suivantes :
Spectre RMN 1 H (400MHz): 0,52 (d large, J=6,8 Hz, 3 H) ; 0,81 (d, J=6,8 Hz, 3 H) ; 1,37 (d, J=6,4 Hz, 3 H) ; 1,84 (m étalé, 1 H) ; 2,84 (d, J=16,6 Hz, 1 H) ; 3,10 (dd, J=9,7 et 16,6 Hz, 1 H) ; 4,27 à 4,57 (m, 4 H) ; 7,01 (dt, J=1,3 et 7,8 Hz, 1 H) ; 7,15 (t, J=7,8 Hz, 1 H) ; 7,22 (d, J=7,8 Hz, 1 H) ; 7,25 à 7,38 (m, 5 H) ; 8,01 (m étalé, 1 H)
Spectrométrie de Masse : méthode A
Temps de rétention Tr (min) = 1,15 ;
[M+H]+ : m/z 324 ; [M+Na]+ : m/z 346
et 3,55 g du diastéréoisomère (2) de la (R)-2-benzyloxy-1-(2-isopropyl-2,3-dihydro-indol-1-yl)-propan-1-one sous forme d'une huile incolore dont les caractéristiques sont les suivantes :
Spectre RMN 1 H (400MHz) : pour ce lot, tous les signaux sont larges avec : 0,53 (d, J=6,8 Hz, 3 H) ; 0,83 (d, J=6,8 Hz, 3 H) ; 1,40 (d, J=6,4 Hz, 3 H) ; 1,86 (m, 1 H) ; 2,87 (d, J=16,6 Hz, 1 H) ; 3,19 (dd, J=9,7 et 16,6 Hz, 1 H) ; 4,24 à 4,62 (m, 4 H) ; 7,01 (t, J=7,8 Hz, 1 H) ; 7,15 (t, J=7,8 Hz, 1 H) ; 7,22 (d, J=7,8 Hz, 1 H) ; 7,25 à 7,42 (m, 5 H) ; 8,06 (d, J=7,8 Hz, 1 H)
Spectrométrie de Masse : méthode A
Temps de rétention Tr (min) = 1,13 ;
[M+H]+ : m/z 324 ; [M+Na]+ : m/z 346

### Etape 3c

A une solution de 1,2 g du diastéréoisomère (1) de la (R)-2-benzyloxy-1-(2-isopropyl-2,3-dihydro-indol-1-yl)-propan-1-one dans 34 mL d'éthanol absolu, sont ajoutés 5,6 mL d'acide chlorhydrique concentré. Le mélange réactionnel est chauffé à 120°C sous micro-ondes pendant 1 heure, puis il est refroidi à temprérature ambiante et concentré à sec sous pression réduite. Le résidu est refroidi dans un bain de glace et repris dans 200 mL d'eau, puis alcalinisé avec de la soude concentrée. Le mélange est extrait avec 2x150 mL de dichlorométhane. Les phases organiques sont réunies, lavées avec 150 mL d'eau, séchées sur sulfate de magnésium, filtrées puis concentrées à sec sous pression réduite. Le résidu est purifié par chromatographie sur une cartouche de 70 g de silice 15-40 µm, en éluant avec un mélange cyclohexane/ acétate d'éthyle 95/5 v/v à un débit de 50 mL/ min, puis sur une cartouche de 30 g de silice 15-40 µm, en éluant avec du cyclohexane pur puis avec un mélange cyclohexane/ acétate d'éthyle 98/2, à un débit de 30 mL/ min. On obtient ainsi 0,40 g de (-)-2-isopropyl-indoline sous forme d'une huile incolore dont les caractéristiques sont les suivantes :
Spectre RMN 1 H (400MHz): 0,87 (d, J=6,8 Hz, 3 H) ; 0,92 (d, J=6,8 Hz, 3 H) ; 1,64 (m, 1 H) ; 2,58 (dd, J=9,6 et 15,8 Hz, 1 H) ; 2,95 (dd, J=9,0 et 15,8 Hz, 1 H) ; 3,46 (m, 1 H) ; 5,62 (d large, J=3,3 Hz, 1 H) ; 6,35 à 6,49 (m, 2 H) ; 6,85 (t, J=7,5 Hz, 1H) ; 6,94 (d, J=7,5 Hz, 1 H)
Spectrométrie de Masse : méthode A
Temps de rétention Tr (min) = 0,41 ;
[M+H]+ : m/z 162
Pouvoir rotatoire : α_{D} = -17,7° +/-0,6 (c = 0,5 % dans le DMSO)

### Exemple de référence 12c : Synthèse de la (+)-2-isopropyl-indoline :

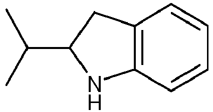

### Etape 1c

A une solution de 1,2 g du diastéréoisomère (2) de la (R)-2-benzyloxy-1-(2-isopropyl-2,3-dihydro-indol-1-yl)-propan-1-one dans 34 mL d'éthanol absolu, sont ajoutés 5,6 mL d'acide chlorhydrique concentré. Le mélange réactionnel est chauffé à 120°C sous micro-ondes pendant 1 heure, puis il est refroidi à temprérature ambiante et concentré à sec sous pression réduite. Le résidu est repris dans 100 mL d'eau, puis alcalinisé avec de la soude concentrée. Le mélange est extrait avec 2x120 mL de dichlorométhane. Les phases organiques sont réunies, lavées avec de l'eau, séchées sur sulfate de magnésium, filtrées puis concentrées à sec sous pression réduite. Le résidu est purifié par chromatographie sur une cartouche de 70 g de silice 15-40 µm, en éluant avec du cyclohexane pur, puis avec un mélange cyclohexane/ acétate d'éthyle 95/5 v/v à un débit de 50 mL/ min, puis sur une cartouche de 30 g de silice 15-40 µm, en éluant avec un mélange cyclohexane/ acétate d'éthyle 98/2, à un débit de 30 mL/ min. On obtient ainsi 0,26 g de (+)-2-isopropyl-indoline sous forme d'une huile incolore dont les caractéristiques sont les suivantes :
Spectre RMN 1 H (400MHz) : 0,87 (d, J=6,8 Hz, 3 H) ; 0,92 (d, J=6,8 Hz, 3 H) ; 1,63 (m, 1 H) ; 2,57 (dd, J=9,6 et 15,8 Hz, 1 H) ; 2,95 (dd, J=9,0 et 15,8 Hz, 1 H) ; 3,47 (m, 1 H) ; 5,65 (d large, J=3,3 Hz, 1 H) ; 6,39 à 6,51 (m, 2 H) ; 6,86 (t, J=7,5 Hz, 1 H) ; 6,94 (d, J=7,5 Hz, 1 H)
Spectrométrie de Masse : méthode A
Temps de rétention Tr (min) = 0,41 ;
[M+H]+ : m/z 162
Pouvoir rotatoire : α_{D} = +26,9° +/-0,9 (c = 0,5 % dans le DMSO)

### Synthèse des composés de formule (Id) :

### Exemple 1d Synthèse du 6-(morpholin-4-yl)-2-[2-oxo-2-(spiro[cyclopropane-1,3'-indol]-1'(2'H)-yl)éthyl]pyrimidin-4(3H)-one

### Etape 1d :

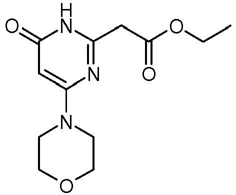

A une solution de 25 g de morpholine dans 400 mL d'éthanol chauffée à 95 °C sont ajoutés 168.5 mL de chlorhydrate de 3-éthoxy-3-iminopropanoate d'éthyle, puis 155 mL de N,N-diisopropyléthylamine dans 200 mL d'éthanol. Le mélange réactionnel est chauffé à 95 °C pendant 30 heures puis laissé revenir à température ambiante. Le précipité formé est filtré sur verre fritté puis lavé avec 100 mL d'éthanol, 2 fois 500 mL d'eau et enfin 500 mL d'éther éthylique. Le solide est séché sous vide pour donner 35 g de [4-(morpholin-4-yl)-6-oxo-1,6-dihydropyrimidin-2-yl]acétate d'éthyle sous forme de solide blanc dont les caractéristiques sont les suivantes :
Spectre RMN 1 H (400MHz, δen ppm , DMSO-d6) : 1,19 (t, *J*=7,1 Hz, 3 H); 3,38 à 3,44 (m, 4 H); 3,56 (s, 2H); 3,61 (dd, *J*=4,0 et 5,7 Hz, 4 H); 4,12 (q, *J*=7,1 Hz, 2 H); 5,20 (s, 1 H); 11,69 (s large, 1 H)
Spectrométrie de Masse : méthode A
Temps de rétention Tr (min) = 0.48;
[M+H]+ : m/z 268 ; [M-H]- : m/z 266

### Etape 2d:

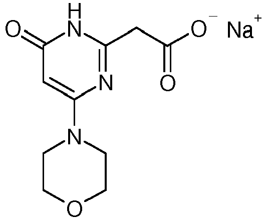

A une solution de 10 g de [4-(morpholin-4-yl)-6-oxo-1,6-dihydropyrimidin-2-yl]acétate d'éthyle dans 300 mL de tétrahydrofuranne, est ajouté 18.7 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif pour donner 8.7 g de [4-(morpholin-4-yl)-6-oxo-1,6-dihydropyrimidin-2-yl]acétate de sodium sous forme de solide blanc dont les caractéristiques sont les suivantes:
Spectre RMN 1 H (400MHz, δ en ppm , DMSO-d6) : 3,08 (s, 2 H); 3,38 (t, *J*=4,6 Hz, 4 H); 3,61 (t, *J*=4,6Hz, 4 H); 5,08 (s, 1 H); 13,16 (s large, 1 H)
Spectrométrie de Masse : méthode A
Temps de rétention Tr (min) = 0.29;
[M+H]+ : m/z 240 ; [M-H]- : m/z 238

### Etape 3d:

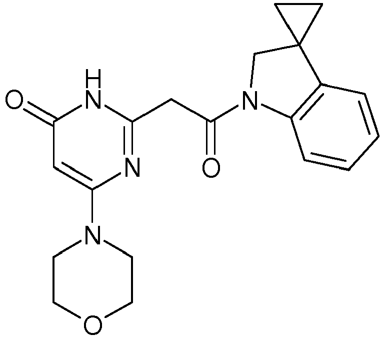

A une suspension de 222 mg de [4-(morpholin-4-yl)-6-oxo-1,6-dihydropyrimidin-2-yl]acétate de sodium obtenu selon l'étape précédente (exemple 1 d étape 2d) dans 1 mL de N,N-diméthylformamide sont successivement ajoutés 0,14 mL de pyridine et 216 mg de N-[3-(diméthylamino)propyl]-N'-éthylcarbodiimide chlorhydrate. Après agitation à température ambiante pendant 10 minutes, une solution de 136 mg de 1,2-dihydro-3-spiro-1'-cyclopropyl-1 H-indole (qui peut être préparé selon le brevet US 7,507,748 B2 (2009)) dans 4 mL de N,N-diméthylformamide est additionnée. Le mélange réactionnel est agité à température ambiante pendant 65 heures, puis 13 mL d'eau et 26 mL d'acétate d'éthyle sont additionnés. Après agitation pendant 1 heure, le précipité formé est filtré, puis rincé successivement à l'eau (2x8 mL) et l'éther diéthylique (3x13 mL). Après séchage sous pression réduite à 40°C, on obtient 208 mg de 6-(morpholin-4-yl)-2-[2-oxo-2-(spiro[cyclopropane-1,3'-indol]-1'(2'H)-yl)éthyl]pyrimidin-4(3H)-one sous forme d'une poudre cristalline rosée dont les caractéristiques sont les suivantes:
Spectre RMN 1 H (400MHz) : 1,02 à 1,14 (m, 4 H) ; 3,42 (m, 4 H) ; 3,60 (m, 4 H) ; 3,71 (s, 2 H) ; 4,17 (s, 2 H) ; 5,21 (s, 1 H) ; 6,82 (d, J=7,8 Hz, 1 H) ; 6,99 (t, J=7,8 Hz, 1 H) ; 7,13 (t, J=7,8 Hz, 1 H) ; 8,01 (d, J=7,8 Hz, 1 H) ; 11,60 (s large, 1 H)
Spectrométrie de Masse : méthode A
Temps de rétention Tr (min) = 0,74 ;
[M+H]+ : m/z 367 ; [M-H]- : m/z 365
Point de fusion (Kofler) : supérieur à 260°C

### Exemple 2d Synthèse du 6-(morpholin-4-yl)-2-[2-oxo-2-(4-phényl-2,3-dihydro-1 H-indol-1-yl)éthyl]pyrimidin-4(3H)-one

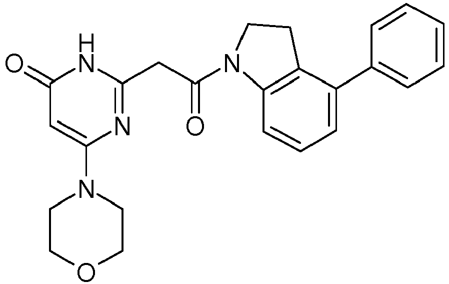

A une suspension de 250 mg de [4-(morpholin-4-yl)-6-oxo-1,6-dihydropyrimidin-2-yl]acétate de sodium (exemple 1d étape 2d) dans 5 mL de N,N-diméthylformamide sont successivement ajoutés 0,16 mL de pyridine, 240 mg de N-[3-(diméthylamino)propyl]-N'-éthylcarbodiimide chlorhydrate et 210 mg de 4-phényl-2,3-dihydro-1 H-indole (exemple de référence 1d). Le mélange réactionnel est agité à température ambiante pendant 15 heures, puis concentré à sec sous pression réduite. Le résidu est repris et trituré dans 30 mL d'eau, puis le précipité formé est filtré puis solubilisé dans un mélange dichlorométhane/méthanol 90/10 et enfin concentré à sec sous pression réduite. Le résidu est purifié par chromatographie sur une colonne de 30 g silice 20-45 µm, en éluant avec un mélange de dichlorométhane et de méthanol (90/10 : v/v). Les fractions contenant le produit attendu sont concentrées à sec sous pression réduite et le résidu est trituré dans 20 mL d'éther diisopropylique. Le solide obtenu est filtré puis séché sous pression réduite à 40 °C. On obtient ainsi 232 mg de 6-(morpholin-4-yl)-2-[2-oxo-2-(4-phényl-2,3-dihydro-1H-indol-1-yl)éthyl]pyrimidin-4(3H)-one sous forme d'un solide rose dont les caractéristiques sont les suivantes:
Spectre RMN 1 H (400MHz) : 3,22 (t, J=8,7 Hz, 2 H) ; 3,42 (m, 4 H) ; 3,61 (m, 4 H) ; 3,78 (s, 2 H) ; 4,14 (t, J=8,7 Hz, 2 H) ; 5,21 (s, 1 H) ; 7,07 (d, J=7,8 Hz, 1 H) ; 7,28 (t, J=7,8 Hz, 1 H) ; 7,36 à 7,51 (m, 5 H) ; 8,08 (d, J=7,8 Hz, 1 H) ; 11,62 (s large, 1 H)
Spectrométrie de Masse : méthode A
Temps de rétention Tr (min) = 0,89 ;
[M+H]+ : m/z 417 ; [M-H]- : m/z 415
Point de fusion (Kofler) : 266°C

### Exemple 3d Synthèse du 6-(morpholin-4-yl)-2-{2-oxo-2-[4-(trifluorométhoxy)-2,3-dihydro-1H-indol-1-yl]éthyl}pyrimidin-4(3H)-one

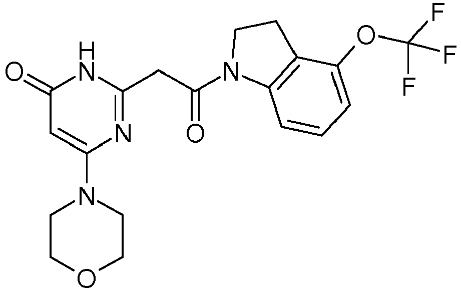

A une solution de 110 mg de [4-(morpholin-4-yl)-6-oxo-1,6-dihydropyrimidin-2-yl]acétate de sodium (exemple 1d étape 2d) dans 1 mL de N,N-diméthylformamide sont successivement ajoutés 70 µL de pyridine et 107 mg de N-[3-(diméthylamino)propyl]-N'-éthylcarbodiimide chlorhydrate. Après agitation à température ambiante pendant 10 minutes, une solution de 178 mg de 4-(trifluorométhoxy)-indoline (exemple de référence 2d) dans 1 mL de N,N-diméthylformamide est additionnée. Le mélange réactionnel est agité à température ambiante pendant 23 heures, puis 13 mL d'eau et 6 mL d'acétate d'éthyle sont additionnés. Après agitation à température ambiante pendant 2 heures, le précipité formé est filtré, puis rincé successivement à l'eau (2x4 mL) et à l'éther diéthylique (3x6 mL). Après séchage sous pression réduite à 40°C, on obtient 97 mg de 6-(morpholin-4-yl)-2-{2-oxo-2-[4-(trifluorométhoxy)-2,3-dihydro-1H-indol-1-yl]éthyl}pyrimidin-4(3H)-one sous forme d'une poudre cristalline blanche dont les caractéristiques sont les suivantes:
Spectre RMN 1 H (400MHz) : 3,21 (t, J=8,6 Hz, 2 H) ; 3,41 (m, 4 H) ; 3,60 (m, 4 H) ; 3,77 (s, 2 H) ; 4,22 (t, J=8,6 Hz, 2 H) ; 5,21 (s, 1 H) ; 7,02 (d, J=8,1 Hz, 1 H) ; 7,32 (t, J=8,1 Hz, 1 H) ; 8,02 (d, J=8,1 Hz, 1 H) ; 11,62 (s large, 1 H)
Spectrométrie de Masse : méthode A
Temps de rétention Tr (min) = 0,85 ;
[M+H]+ : m/z 425 ; [M-H]- : m/z 423
Point de fusion (Kofler) : supérieur à 260°C

### Exemple 4d Synthèse du 3-méthyl-6-(morpholin-4-yl)-2-{2-oxo-2-[4-(trifluorométhyl)-2,3-dihydro-1H-indol-1-yl]éthyl}pyrimidin-4(3H)-one

### Etape 1d:

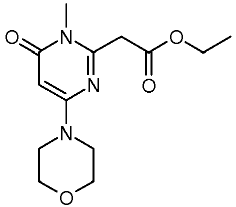

A une solution de 500 mg de [4-(morpholin-4-yl)-6-oxo-1,6-dihydropyrimidin-2-yl]acétate d'éthyle (préparé à l'étape 1d de l'exemple 1d) dans 1.5 mL de dioxanne, 330 mg de carbonate de potassium et 0,15 mL d'iodure de méthyle. Le mélange réactionnel est chauffé à 40 °C pendant 16 heures puis refroidi à température ambiante. La suspension est filtrée sur verre fritté puis rincée avec du dioxanne et le filtrat est concentré sous pression réduite. Le résidu est purifié par chromatographie sur colonne de silice, en éluant avec un mélange de dichlorométhane, d'acétonitrile et de méthanol (98/01/01, 96/02/02 puis, 90/05/05 V/V/V). On obtient 200 mg de [1-méthyl-4-(morpholin-4-yl)-6-oxo-1,6-dihydropyrimidin-2-yl]acétate d'éthyle sous forme de solide blanc dont les caractéristiques sont les suivantes :
Spectre RMN 1H (400MHz) : 1,21 (t, *J*=7,1 Hz, 3 H) ; 3,29 (m partiellement masqué, 3 H) ; 3,40 (m, 4 H) ; 3,61 (m, 4 H) ; 3,92 (s, 2 H) ; 4,15 (q, J=7,1 Hz, 2 H) ; 5,35 (s, 1 H)
Spectrométrie de Masse : méthode A
Temps de rétention Tr (min) = 0,53 ;
[M+H]+ : m/z 282 ; [M-H]- : m/z 280;

**Etape 2d:**

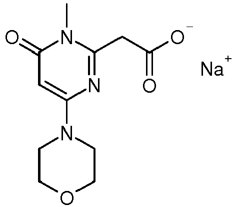

A une solution de 1.62 g de [1-méthyl-4-(morpholin-4-yl)-6-oxo-1,6-dihydropyrimidin-2-yl]acétate d'éthyle, dans 20 mL de tétrahydrofurane, sont ajoutés 2.88 mL de soude 2M. Le mélange réactionnel est agité pendant 48 heures à température ambiante. Le précipité formé est filtré sur verre fritté, lavé à l'acétate d'éthyle, rincé à l'éther diéthylique et séché pour donner 730 mg de [1-méthyl-4-(morpholin-4-yl)-6-oxo-1,6-dihydropyrimidin-2-yl]acétate de sodium sous forme de solide blanc dont les caractéristiques sont les suivantes:
Spectre RMN 1 H (400MHz) : 3,27 à 3,43 (m partiellement masqué, 9 H) ; 3,61 (m, 4 H) ; 5,23 (s, 1 H)
Spectrométrie de Masse : méthode A
Temps de rétention Tr (min) = 0,31 ;
[M+H]+ : m/z 254 ; [M-H]- : m/z 252;

### Etape 3d:

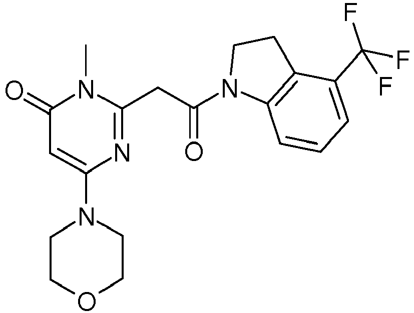

A une solution de 138 mg de [1-méthyl-4-(morpholin-4-yl)-6-oxo-1,6-dihydropyrimidin-2-yl]acétate de sodium dans 3 mL de N,N-diméthylformamide sont successivement ajoutés 3 mL de pyridine, 94 mg de 4-trifluoromethyl-2,3-dihydro-1H-indole et 153 mg de N-[3-(diméthylamino)propyl]-N'-éthylcarbodiimide chlorhydrate. Le mélange réactionnel est agité à température ambiante pendant 16 heures. Après addition de 50 mL d'eau et extraction avec de l'acétate d'éthyle (3x15 mL), les phases organiques sont rassemblées puis lavées avec de l'eau (2x15 mL), une solution d'eau saturée en chlorure de sodium (15 mL) puis séchées sur sulfate de magnésium, filtrées sur verre fritté et concentrées sous pression réduite. Le solide obtenu est lavé avec de l'éther diéthylique (5 mL) puis séché pour donner 64 mg de 3-méthyl-6-(morpholin-4-yl)-2-{2-oxo-2-[4-(trifluorométhyl)-2,3-dihydro-1H-indol-1-yl]éthyl}pyrimidin-4(3H)-one sous forme d'un solide rose dont les caractéristiques sont les suivantes:
Spectre RMN 1 H (400MHz) : 3,24 à 3,42 (m partiellement masqué, 9 H) ; 3,57 (m, 4 H) ; 4,14 (s, 2 H) ; 4,26 (t, J=8,6 Hz, 2 H) ; 5,37 (s, 1 H) ; 7,35 (d, J=8,1 Hz, 1 H) ; 7,41 (t, J=8,1 Hz, 1 H) ; 8,30 (d, J=8,1 Hz, 1 H)
Spectrométrie de Masse : méthode A
Temps de rétention Tr (min) = 0,87 ;
[M+H]+ : m/z 423 ; [M-H]- : m/z 421

### Exemple 5d Synthèse du 6-(morpholin-4-yl)-2-{2-oxo-2-[4-(trifluorométhyl)-2,3-dihydro-1H-indol-1-yl]éthyl}pyrimidin-4(3H)-one

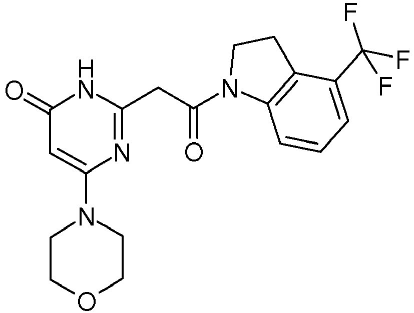

A une solution de 977 mg de [4-(morpholin-4-yl)-6-oxo-1,6-dihydropyrimidin-2-yl]acétate de sodium (obtenu à l'étape 2d de l'Exemple 1d) dans 25 ml de N,N-diméthylformamide et 25 ml de pyridine sont ajoutés 700 mg de 4-trifluoromethyl-2,3-dihydro-1H-indole et 1,15 g de N-[3-(diméthylamino)propyl]-N'-éthylcarbodiimide chlorhydrate. Le mélange réactionnel est agité à température ambiante pendant 16 heures, puis de l'eau est ajoutée et le mélange est extrait avec de l'acétate d'éthyle. La phase organique est lavée successivement avec de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 220 mg de 6-(morpholin-4-yl)-2-{2-oxo-2-[4-(trifluorométhyl)-2,3-dihydro-1H-indol-1-yl]éthyl}pyrimidin-4(3H)-one sous forme d'un solide blanc dont les caractéristiques sont les suivantes:
Spectre RMN 1 H (400MHz): 3,27 à 3,45 (m partiellement masqué, 6 H) ; 3,60 (m, 4 H) ; 3,79 (s, 2 H) ; 4,22 (t, J=8,6 Hz, 2 H) ; 5,22 (s, 1 H) ; 7,34 (d, J=8,1 Hz, 1 H) ; 7,41 (t, J=8,1 Hz, 1 H) ; 8,30 (d, J=8,1 Hz, 1 H) ; 11,64 (m large, 1 H)
Spectrométrie de Masse : méthode A
Temps de rétention Tr (min) = 0,82 ;
[M+H]+ : m/z 409 ; [M-H]- : m/z 407

### Exemple 6d Synthèse du 2-{2-[4-(2-méthoxyphényl)-2,3-dihydro-1H-indol-1-yl]-2-oxoéthyl}-6-(morpholin-4-yl)pyrimidin-4(3H)-one

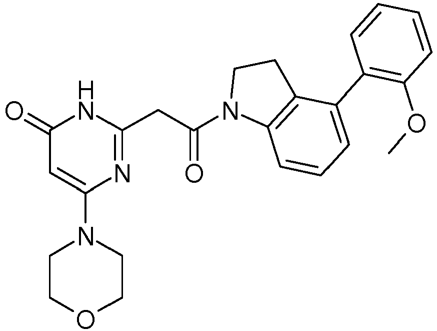

A une solution de 170 mg de [4-(morpholin-4-yl)-6-oxo-1,6-dihydropyrimidin-2-yl]acétate de sodium (exemple 1d étape 2d) dans 3 mL de N,N-diméthylformamide sont successivement ajoutés 0,105 mL de pyridine et 165 mg de N-[3-(diméthylamino)propyl]-N'-éthylcarbodiimide chlorhydrate. Après agitation à température ambiante pendant 15 minutes, 140 mg de 4-(2-méthoxy-phényl)-2,3-dihydro-1H-indole (exemple de référence 3d) sont additionnés. Le mélange réactionnel est agité à température ambiante pendant 16 heures, puis 20 mL d'eau et 3 mL d'acétate d'éthyle sont additionnés. Après agitation à température ambiante pendant 1 heure, le précipité formé est filtré, puis rincé successivement à l'eau et l'éther diéthylique. Après séchage sous pression réduite à 40°C, on obtient 202 mg de 2-{2-[4-(2-méthoxyphényl)-2,3-dihydro-1H-indol-1-yl]-2-oxoéthyl}-6-(morpholin-4-yl)pyrimidin-4(3H)-one sous forme d'un solide rose pâle dont les caractéristiques sont les suivantes:
Spectre RMN 1 H (400MHz) : 2,93 (t, J=8,7 Hz, 2 H) ; 3,42 (m, 4 H) ; 3,61 (m, 4 H) ; 3,74 (s, 3 H) ; 3,76 (s, 2 H) ; 4,11 (t, J=8,7 Hz, 2 H) ; 5,21 (s, 1 H) ; 6,91 (d, J=7,9 Hz, 1 H) ; 7,02 (t, J=7,9 Hz, 1 H) ; 7,11 (d, J=7,9 Hz, 1 H) ; 7,15 à 7,26 (m, 2 H) ; 7,38 (t large, J=7,9 Hz, 1 H) ; 8,03 (d, J=7,9 Hz, 1 H) ; 11,63 (s large, 1 H)
Spectrométrie de Masse : méthode A
Temps de rétention Tr (min) = 0,88 ;
[M+H]+ : m/z 447 ; [M-H]- : m/z 445
Point de fusion (Kofler) : supérieur à 260°C

### Exemple 7d Synthèse du 6-(morpholin-4-yl)-2-{2-oxo-2-[4-(1-propylpipéridin-3-yl)-2,3-dihydro-1H-indol-1-yl]éthyl}pyrimidin-4(3H)-one

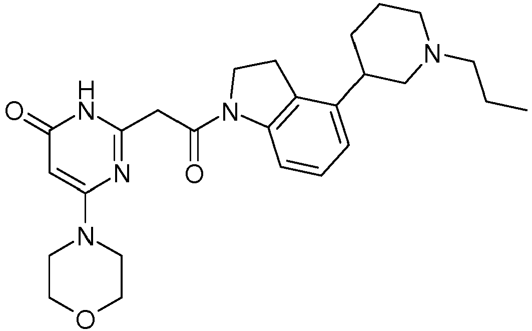

A une solution de 20,5 mg de [4-(morpholin-4-yl)-6-oxo-1,6-dihydropyrimidin-2-yl]acétate de sodium (exemple 1d étape 2d) dans 0,8 mL de N,N-diméthylformamide sont successivement ajoutés 0,2 mL de pyridine, 25,7 mg de 4-(1-propyl-piperidin-3-yl)-2,3-dihydro-1H-indole (Exemple de référence 4d) et 17,8 mg de N-[3-(diméthylamino)propyl]-N'-éthylcarbodiimide chlorhydrate. Le mélange réactionnel est agité à température ambiante pendant 24 heures, puis concentré sous pression réduite. Le résidu est repris dans 10 mL d'eau puis extrait avec du dichlorométhane (4x20 mL). Les phases organiques sont rassemblées puis lavées avec de l'eau et concentrées sous pression réduite. Après séchage à l'étuve, on obtient 16,1 mg de 6-(morpholin-4-yl)-2-{2-oxo-2-[4-(1-propylpipéridin-3-yl)-2,3-dihydro-1H-indol-1-yl]éthyl}pyrimidin-4(3H)-one sous forme d'un solide rose dont les caractéristiques sont les suivantes:

### Exemple 8d Synthèse du 2-{2-[4-(difluorométhoxy)-2,3-dihydro-1H-indol-1-yl]-2-oxoéthyl}-3-méthyl-6-(morpholin-4-yl)pyrimidin-4(3H)-one

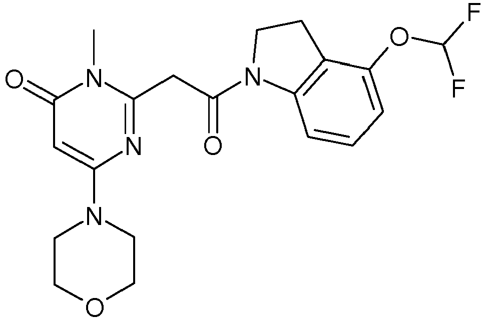

A une solution de 237 mg de [1-méthyl-4-(morpholin-4-yl)-6-oxo-1,6-dihydropyrimidin-2-yl]acétate de sodium (obtenu à l'étape 2d de l'exemple 4d) dans 7 mL de N,N-diméthylformamide et 7 mL de pyridine sont ajoutés 220 mg de 4-difluoromethoxy-2,3-dihydro-1H-indole (Exemple de référence 5d) et 364 mg de N-[3-(diméthylamino)propyl]-N'-éthylcarbodiimide chlorhydrate. Le mélange réactionnel est agité à température ambiante pendant 16 heures, puis 20 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 110 mg de 2-[2-(4-difluoromethoxy-2,3-dihydro-indol-1-yl)-2-oxo-ethyl]-3-methyl-6-morpholin-4-yl-3H-pyrimidin-4-one sous forme d'un solide rose pâle dont les caractéristiques sont les suivantes:
Spectre RMN 1 H (400MHz): 3,15 (t, J=8,6 Hz, 2 H) ; 3,30 (s partiellement masqué, 3 H) ; 3,39 (m, 4 H) ; 3,58 (m, 4 H) ; 4,12 (s, 2 H) ; 4,23 (t, J=8,6 Hz, 2 H) ; 5,37 (s, 1 H) ; 6,88 (d, J=8,1 Hz, 1 H) ; 7,24 (t, J=74,2 Hz, 1 H) ; 7,24 (t, J=8,1 Hz, 1H) ; 7,89 (d, J=8,1 Hz, 1 H)
Spectrométrie de Masse : méthode A
Temps de rétention Tr (min) = 0,77 ;
[M+H]+ : m/z 421 ; [M-H]- : m/z 419

### Exemple 9d Synthèse du 2-{2-[4-(difluorométhoxy)-2,3-dihydro-1H-indol-1-yl]-2-oxoéthyl}-6-(morpholin-4-yl)pyrimidin-4(3H)-one

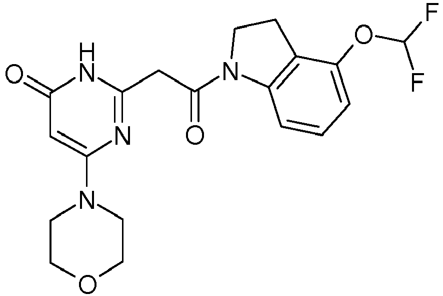

A une solution de 327 mg de [4-(morpholin-4-yl)-6-oxo-1,6-dihydropyrimidin-2-yl]acétate de sodium (obtenu à l'étape 2d de l'Exemple 1d) dans 7 ml de N,N-diméthylformamide et 7 ml de pyridine sont ajoutés 220 mg de 4-difluoromethoxy-2,3-dihydro-1H-indole (Exemple de référence 5d) et 364 mg de N-[3-(diméthylamino)propyl]-N'-éthylcarbodiimide chlorhydrate. Le mélange réactionnel est agité à température ambiante pendant 16 heures, puis 15 mL d'eau sont ajoutés et le mélange est extrait avec de l'acétate d'éthyle. La phase organique est lavée successivement avec de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 24 mg de 2-{2-[4-(difluorométhoxy)-2,3-dihydro-1H-indol-1-yl]-2-oxoéthyl}-6-(morpholin-4-yl)pyrimidin-4(3H)-one dont les caractéristiques sont les suivantes:
Spectre RMN 1 H (400MHz): 3,15 (t, J=8,6 Hz, 2 H) ; 3,42 (m, 4 H) ; 3,60 (m, 4 H) ; 3,76 (s, 2 H) ; 4,19 (t, J=8,6 Hz, 2 H) ; 5,21 (s, 1 H) ; 6,87 (d, J=8,1 Hz, 1 H) ; 7,24 (t, J=74,2 Hz, 1 H) ; 7,25 (t, J=8,1 Hz, 1 H) ; 7,89 (d, J=8,1 Hz, 1 H) ; 11,63 (m étalé, 1 H)
Spectrométrie de Masse : méthode A
Temps de rétention Tr (min) = 0,73 ;
[M+H]+ : m/z 407 ; [M-H]- : m/z 405

### Exemple 10d Synthèse du 6-(morpholin-4-yl)-2-{2-oxo-2-[4-(pyridin-4-yl)-2,3-dihydro-1H-indol-1-yl]éthyl}pyrimidin-4(3H)-one

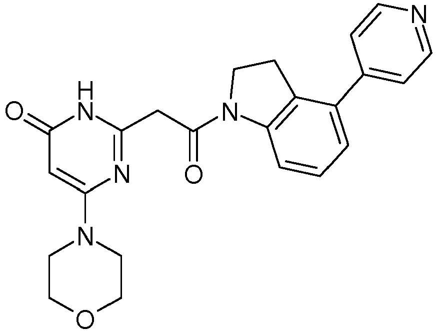

A une solution de 26,7 mg de [4-(morpholin-4-yl)-6-oxo-1,6-dihydropyrimidin-2-yl]acétate de sodium (exemple 1d étape 2d) dans 0,8 mL de N,N-diméthylformamide sont successivement ajoutés 0,2 mL de pyridine, 25 mg de 4-pyridin-4-yl-2,3-dihydro-1H-indole et 23 mg de N-[3-(diméthylamino)propyl]-N'-éthylcarbodiimide chlorhydrate. Le mélange réactionnel est agité à température ambiante pendant 24 heures, puis concentré sous pression réduite. Le résidu est repris dans 10 mL d'eau puis filtré sur verre fritté et le précipité est lavé avec de l'eau (2x1 mL) et séché à l'étuve. On obtient 27,6 mg de 6-(morpholin-4-yl)-2-{2-oxo-2-[4-(pyridin-4-yl)-2,3-dihydro-1H-indol-1-yl]éthyl}pyrimidin-4(3H)-one sous forme d'un solide rose dont les caractéristiques sont les suivantes:
Spectre RMN 1 H (400MHz) : 3,27 (m masqué, 2 H); 3,41 (m, 4 H); 3,61 (m, 4 H); 3,79 (s, 2 H); 4,16 (t, J=8,4 Hz, 2 H); 5,22 (s, 1 H); 7,18 (d, J=7,6 Hz, 1 H); 7,35 (t, J=7,9 Hz, 1 H); 7,61 (d, *J*=6,4 Hz, 2 H); 8,16 (d, *J*=8,3 Hz, 1 H); 8,69 (d, *J*=6,1 Hz, 2 H); 11,66 (s large, 1 H)
Spectrométrie de Masse : méthode A
Temps de rétention Tr (min) = 0.40;
[M+H]+ : m/z 418 ; [M-H]- : m/z 416

### Exemple 11d Synthèse du 2-[2-(1'-méthylspiro[indole-3,4'-pipéridin]-1(2H)-yl)-2-oxoéthyl]-6-(morpholin-4-yl)pyrimidin-4(3H)-one

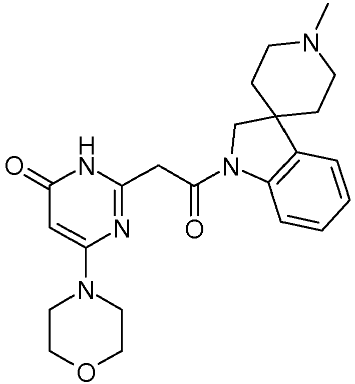

Le produit est préparé en suivant le mode opératoire décrit à l'exemple 10d à partir de 35,5 mg [4-(morpholin-4-yl)-6-oxo-1,6-dihydropyrimidin-2-yl]acétate de sodium (exemple 1 d étape 2d) et de 25 mg de 1'-méthylspiro[indoline-3,4'-piperidine à la place de 4-pyridin-4-yl-2,3-dihydro-1H-indole. On obtient 13,8 mg de 2-[2-(1'-méthylspiro[indole-3,4'-pipéridin]-1(2H)-yl)-2-oxoéthyl]-6-(morpholin-4-yl)pyrimidin-4(3H)-one sous forme d'une poudre rose pâle dont les caractéristiques sont les suivantes:
Spectre RMN 1 H (400MHz) : 1,56 (d, J=13,0 Hz, 2 H); 1,88 (td, J=3,3 et 12,8 Hz, 2 H); 2,03 (t, J=11,0 Hz, 2 H); 2,21 (s, 3 H); 2,75 (d, J=12,0 Hz, 2 H); 3,41 (t, J=4,8 Hz, 4 H); 3,60 (t, J=5,0 Hz, 4 H); 3,82 (s, 2 H); 4,00 (s, 2 H); 5,21 (s, 1 H); 7,05 (t, J=7,3 Hz, 1 H); 7,19 (t, J=7,5 Hz, 1 H); 7,27 (d, J=8,1 Hz, 1 H); 8,02 (d, J=8,1 Hz, 1 H); 11,61 (s large, 1 H)
Spectrométrie de Masse : méthode A
Temps de rétention Tr (min) = 0.40;
[M+H]+ : m/z 424 ; [M-H]- : m/z 422

### Exemple 12d Synthèse du 6-(morpholin-4-yl)-2-{2-oxo-2-[4-(pyridin-2-yl)-2,3-dihydro-1H-indol-1-yl]éthyl}pyrimidin-4(3H)-one

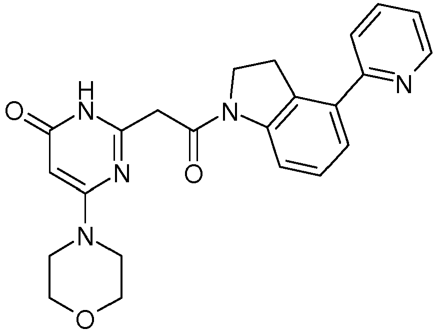

Le produit est préparé en suivant le mode opératoire décrit à l'exemple 10d à partir de 26,7 mg [4-(morpholin-4-yl)-6-oxo-1,6-dihydropyrimidin-2-yl]acétate de sodium (exemple 1 d étape 2d) et de 25 mg de 4-pyridin-2-yl-2,3-dihydro-1H-indole à la place de 4-pyridin-4-yl-2,3-dihydro-1H-indole. On obtient 26,8 mg de 6-(morpholin-4-yl)-2-{2-oxo-2-[4-(pyridin-2-yl)-2,3-dihydro-1H-indol-1-yl]éthyl}pyrimidin-4(3H)-one sous forme d'un solide pourpre dont les caractéristiques sont les suivantes:
Spectre RMN 1 H (400MHz) : 3,37 à 3,46 (m, J=5,9 Hz, 6 H); 3,61 (m, 4 H); 3,79 (s large, 2 H); 4,16 (t, J=8,6 Hz, 2 H); 5,23 (s, 1 H); 7,26 à 7,47 (m, 3 H); 7,77 (d, J=8,3 Hz, 1 H); 7,93 (t, J=8,1 Hz, 1 H); 8,16 (d, J=8,1 Hz, 1 H); 8,69 (d, J=5,6 Hz, 1 H); 11,69 (s large, 1 H)
Spectrométrie de Masse : méthode A
Temps de rétention Tr (min) = 0.53;
[M+H]+ : m/z 418 ; [M-H]- : m/z 416

### Exemple 13d Synthèse du 6-(morpholin-4-yl)-2-{2-oxo-2-[4-(pyridin-3-yl)-2,3-dihydro-1H-indol-1-yl]éthyl}pyrimidin-4(3H)-one

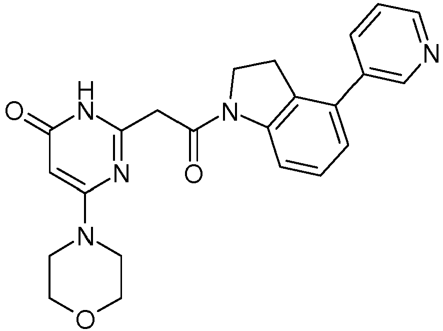

Le produit est préparé en suivant le mode opératoire décrit à l'exemple 10d à partir de 26,7 mg [4-(morpholin-4-yl)-6-oxo-1,6-dihydropyrimidin-2-yl]acétate de sodium (exemple 1 d étape 2d) et de 25 mg de 4-pyridin-3-yl-2,3-dihydro-1H-indole à la place de 4-pyridin-4-yl-2,3-dihydro-1H-indole. On obtient 30,7 mg de 6-(morpholin-4-yl)-2-{2-oxo-2-[4-(pyridin-3-yl)-2,3-dihydro-1H-indol-1-yl]éthyl}pyrimidin-4(3H)-one sous forme d'une poudre rose dont les caractéristiques sont les suivantes:
Spectre RMN 1 H (400MHz) : 3,25 (t, J=8,1 Hz, 2 H); 3,43 (m, 4 H); 3,61 (m, 4 H); 3,79 (s, 2 H); 4,16 (t, J=8,4 Hz, 2 H); 5,22 (s, 1 H); 7,14 (d, J=7,8 Hz, 1 H); 7,33 (t, J=7,9 Hz, 1 H); 7,57 (dd, J=4,9 et 7,8 Hz, 1 H); 8,02 (d, J=8,1 Hz, 1 H); 8,13 (d, J=8,1 Hz, 1 H); 8,63 (dd, J=1,7 et 4,9 Hz, 1 H); 8,76 (dd, J=0,6 et 1,8 Hz, 1 H); 11,65 (s large, 1 H)
Spectrométrie de Masse : méthode A
Temps de rétention Tr (min) = 0.45;
[M+H]+ : m/z 418 ; [M-H]- : m/z 416

### Exemple 14d Synthèse du 2-{2-[4-(2-chlorophényl)-2,3-dihydro-1H-indol-1-yl]-2-oxoéthyl}-6-(morpholin-4-yl)pyrimidin-4(3H)-one

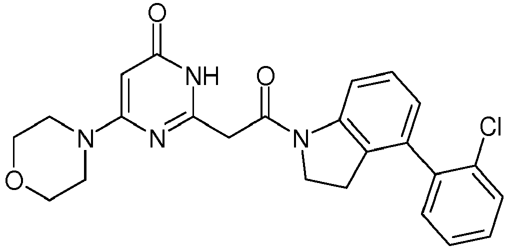

A une solution de 264 mg de 4-(2-chloro-phenyl)-2,3-dihydro-1H-indole (Exemple de référence 6d) dans 5 mL de pyridine sont successivement ajoutés 300 mg de [4-(morpholin-4-yl)-6-oxo-1,6-dihydropyrimidin-2-yl]acétate de sodium (exemple 1d étape 2d), 330 mg de N-[3-(diméthylamino)propyl]-N'-éthylcarbodiimide chlorhydrate et 6 mL de pyridine. Le mélange réactionnel est agité à température ambiante pendant 4 jours. Le milieu réactionnel est dilué avec de l'acétate d'éthyle (60 mL) puis successivement lavé avec une solution aqueuse d'acide chlorhydrique 1 M, une solution aqueuse de soude 1 M, une solution d'eau saturée en chlorure de sodium. La phase organique est ensuite séchée sur sulfate de magnésium, filtrée sur verre fritté. Un précipité apparaît au bout de quelques heures qui est ensuite filtré sur verre fritté et lavé avec de l'éther diéthylique. On obtient 294 mg de 2-{2-[4-(2-chlorophényl)-2,3-dihydro-1H-indol-1-yl]-2-oxoéthyl}-6-(morpholin-4-yl)pyrimidin-4(3H)-one sous forme d'un solide beige dont les caractéristiques sont les suivantes:
Spectre RMN 1 H (400MHz) : 2,93 (t, J=8,6 Hz, 2 H); 3,41 (m, 4 H); 3,60 (m, 4 H); 3,77 (s, 2 H); 4,14 (t, J=8,4 Hz, 2 H); 5,22 (s, 1 H); 6,92 (d, J=7,6 Hz, 1 H); 7,28 (t, J=7,8 Hz, 1 H); 7,37 (m, 1 H); 7,44 (m, 2 H); 7,59 (m, 1 H); 8,08 (d, J=7,6 Hz, 1 H); 11,65 (s large, 1 H)
Spectrométrie de Masse : méthode A
Temps de rétention Tr (min) = 0.93;
[M+H]+ : m/z 451 ; [M-H]- : m/z 449

### Exemple 15d Synthèse du 2-[2-(4-chloro-2,3-dihydro-1H-indol-1-yl)-2-oxoéthyl]-3-cyclopropyl-6-(morpholin-4-yl)pyrimidin-4(3H)-one

### Etape 1d :

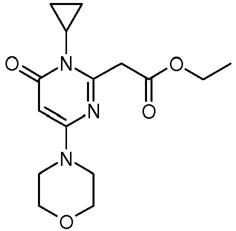

A une solution de 1 g de [4-(morpholin-4-yl)-6-oxo-1,6-dihydropyrimidin-2-yl]acétate d'éthyle préparé à l'étape 1d de l'exemple 1d dans 12 mL de toluène sont ajoutés 643 mg d'acide cyclopropylboronique, 680 mg d'acétate de cuivre (II), 1.37 g de diméthylaminopyridine et enfin 6.23 mL d'une solution de sodium bis(triméthylsilyl) amide (0,6 M dans le toluene) à l'aide d'une ampoule de coulée. Le mélange réactionnel est chauffé à 95 °C pendant 16 puis refroidi à température ambiante. Après addition de 20 mL d'une solution aqueuse 1 N d'acide chlorhydrique et extraction avec du dichlorométhane (3x50 mL), les phases organiques sont rassemblées puis séchées sur sulfate de magnésium, filtrées sur verre fritté et concentrées sous pression réduite. Le résidu est purifié par chromatographie sur colonne de silice en éluant avec un mélange de dichlorométhane et de méthanol (98/02 puis 95/05 V/V). On obtient 90 mg de (1-cyclopropyl-4-morpholin-4-yl-6-oxo-1,6-dihydro-pyrimidin-2-yl)-acetate d'éthyle sous forme d'une huile jaune utilisée telle quelle dans l'étape suivante.

### Etape 2d :

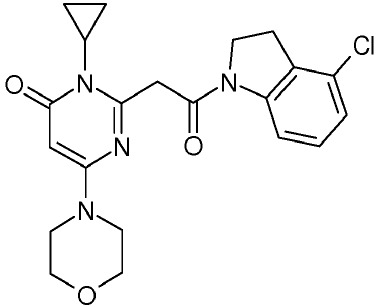

A une solution de 113 mg de 4-chloroindoline dans 4 mL de tétrahydrofuranne sont ajoutés successivement 2 mL de toluène, 90 mg de (1-cyclopropyl-4-morpholin-4-yl-6-oxo-1,6-dihydro-pyrimidin-2-yl)-acetate d'éthyle et enfin au goutte à goutte 0.55 mL d'une solution de triméthylaluminium 2M dans le toluène. Le mélange réactionnel est chauffé à 90°C pendant 4 heures, puis refroidi à température ambiante et 5 mL de méthanol sont additionnés. Après ajout de 10 g de silice, le mélange réactionnel est concentré sous pression réduite. Après purification par chromatographie sur colonne de silice (dépôt solide) en éluant avec un mélange de dichlorométhane et de méthanol (100/0 puis 98/02 V/V), on obtient 39 mg de 2-[2-(4-chloro-2,3-dihydro-1H-indol-1-yl)-2-oxoéthyl]-3-cyclopropyl-6-(morpholin-4-yl)pyrimidin-4(3H)-one sous forme d'un sous forme de solide blanc dont les caractéristiques sont les suivantes :
Spectre RMN 1 H (400MHz) : 0,83 (m, 2 H); 1,07 (m, 2 H); 2,69 (m, 1 H); 3,18 (t, J=8,6 Hz, 2 H); 3,36 (m, 4 H); 3,55 (m, 4 H); 4,18 (s, 2 H); 4,23 (t, J=8,6 Hz, 2 H); 5,27 (s, 1 H); 7,09 (d, J=8,1 Hz, 1 H); 7,21 (t, J=8,1 Hz, 1 H); 7,98 (d, J=7,9 Hz, 1 H)
Spectrométrie de Masse : méthode A
Temps de rétention Tr (min) = 0.85;
[M+H]+ : m/z 415 ; [M-H]- : m/z 413

### Exemple 16d Synthèse du 6-(morpholin-4-yl)-2-[2-oxo-2-(2,3,3a,8b-tétrahydrocyclopenta[b]indol-4(1H)-yl)éthyl]pyrimidin-4(3H)-one

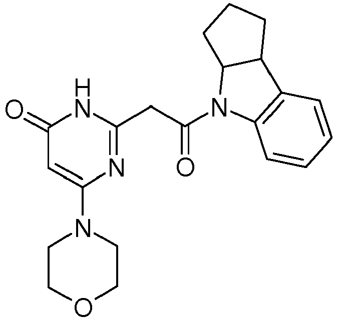

A une solution de 525 mg de [4-(morpholin-4-yl)-6-oxo-1,6-dihydropyrimidin-2-yl]acétate de sodium (obtenu à l'étape 2d de l'Exemple 1d) dans 4 ml de N,N-diméthylformamide et 4 ml de pyridine sont ajoutés 320 mg de 1,2,3,3a,4,8b-hexahydrocyclopenta[b]indole [Exemple de référence 7d] et 424 mg de N-[3-(diméthylamino)propyl]-N'-éthylcarbodiimide chlorhydrate. Le mélange réactionnel est agité à température ambiante pendant 72 heures puis concentré sous pression réduite. Le résidu est repris dans un mélange d'eau et d'acétate d'éthyle et la phase organique lavée successivement avec de l'eau, une solution d'acide chlorhydrique 1 M, de l'eau, séchée sur sulfate de magnésium, filtrée et concentrée sous pression réduite. Le résidu est trituré dans du méthanol, filtré et lavé avec de l'éther diisopropylique pour donner 375 mg de 6-(morpholin-4yl)-2-[2-oxo-2-(2,3,3a,8b-tétrahydrocyclopenta[b]indol-4(1H)-yl)éthyl]pyrimidin-4(3H)-one dont les caractéristiques sont les suivantes:
Spectre RMN 1 H (400MHz): 1,27 (m, 1 H) ; 1,61 (m, 1 H) ; 1,80 à 2,14 (m, 4 H) ; 3,41 (m, 4 H) ; 3,60 (m, 4 H) ; 3,73 (d, J=16,0 Hz, 1 H) ; 3,91 (m, 2 H) ; 4,89 (m, 1 H) ; 5,20 (s, 1 H) ; 7,05 (t, J=7,9 Hz, 1 H) ; 7,16 (t, J=7,9 Hz, 1 H) ; 7,24 (d, J=7,9 Hz, 1 H) ; 7,99 (d, J=7,9 Hz, 1 H) ; 11,67 (m étalé, 1 H)
Spectrométrie de Masse : méthode A
Temps de rétention Tr (min) = 0,79 ;
[M+H]+ : m/z 381 ; [M-H]- : m/z 379

**Exemple 17d Synthèse du 2-[2-(4-{[4-(méthylsulfonyl)pipérazin-1-yl]méthyl}-2,3-dihydro-1H-indol-1-yl)-2-oxoéthyl]-6-(morpholin-4-yl)pyrimidin-4(3H)-one**

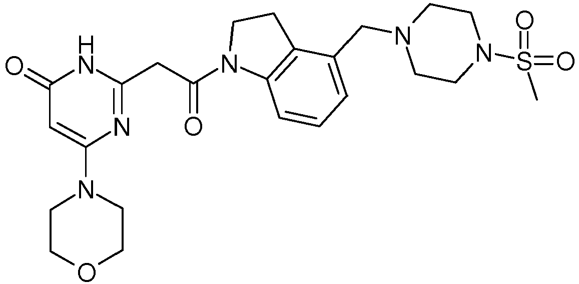

A une solution de 240 mg de [4-(morpholin-4-yl)-6-oxo-1,6-dihydropyrimidin-2-yl]acétate de sodium (exemple 1d étape 2d) dans 5 mL de N,N-diméthylformamide sont successivement ajoutés 5 mL de pyridine, 299 mg de 4-(4-méthanesulfonyl-piperazin-1-ylmethyl)-2,3-dihydro-1H-indole (Exemple de référence 8d) et 264 mg de N-[3-(diméthylamino)propyl]-N'-éthylcarbodiimide chlorhydrate. Le mélange réactionnel est agité à température ambiante pendant 16 heures, puis concentré sous pression réduite. Le résidu est repris dans 30 mL d'eau puis extrait avec du dichlorométhane (3x30 mL). Les phases organiques sont rassemblées puis lavées avec une solution d'eau saturée en chlorure de sodium puis séchées sur sulfate de magnésium, filtrées sur verre fritté et concentrées sous pression réduite. Après purification du résidu par chromatographie sur colonne de silice en éluant avec un mélange de dichlorométhane et de méthanol ammoniacal 7N (90/10), on obtient 190 mg de 2-[2-(4-{[4-(méthylsulfonyl)pipérazin-1-yl]méthyl}-2,3-dihydro-1 H-indol-1-yl)-2-oxoéthyl]-6-(morpholin-4-yl)pyrimidin-4(3H)-one sous forme d'un solide blanc dont les caractéristiques sont les suivantes:
Spectre RMN 1 H (300MHz) : 2,45 (m, 4 H); 2,86 (s, 3 H); 3,10 (m, 4 H); 3,19 (m, 2 H); 3,42 (m, 4 H); 3,48 (s, 2 H); 3,61 (m, 4 H); 3,76 (s, 2 H); 4,15 (t, J=8,6 Hz, 2 H); 5,21 (s, 1 H); 6,97 (d, J=7,0 Hz, 1 H); 7,15 (t, J=7,8 Hz, 1 H); 7,95 (d, J=8,1 Hz, 1 H); 11,60 (s large, 1 H)
Spectrométrie de Masse : méthode A
Temps de rétention Tr (min) = 0.37;
[M+H]+ : m/z 517 ; [M-H]- : m/z 515

### Exemple 18d Synthèse du 2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one

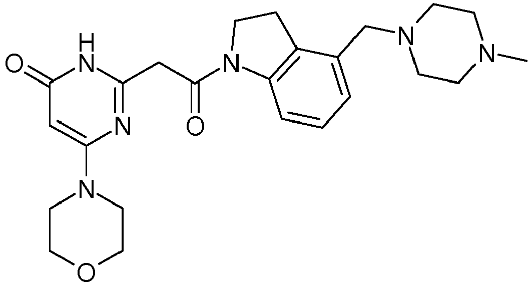

Le produit est préparé en suivant le mode opératoire décrit à l'exemple 17d à partir de 410 mg [4-(morpholin-4-yl)-6-oxo-1,6-dihydropyrimidin-2-yl]acétate de sodium (exemple 1d étape 2d) et de 400 mg de 4-(4-Methyl-piperazin-1-ylmethyl)-2,3-dihydro-1H-indole (Exemple de référence 9d) à la place de 4-(4-méthanesulfonyl-piperazin-1-ylmethyl)-2,3-dihydro-1H-indole. On obtient 120 mg de 2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one sous forme d'un solide blanc dont les caractéristiques sont les suivantes:
Spectre RMN 1 H (300MHz) : 2,14 (s, 3 H); 2,24 à 2,41 (m, 8 H); 3,17 (t, J=8,4 Hz, 2 H); 3,38 à 3,47 (m, 6 H); 3,60 (m, 4 H); 3,75 (s, 2 H); 4,15 (t, J=8,1 Hz, 2 H); 5,21 (s, 1 H); 6,95 (d, J=7,6 Hz, 1 H); 7,12 (t, J=7,8 Hz, 1 H); 7,93 (d, J=7,8 Hz, 1 H); 11,61 (s large, 1 H)
Spectrométrie de Masse : méthode A
Temps de rétention Tr (min) = 0.36;
[M+H]+ : m/z 453 ; [M-H]- : m/z 451

### Exemple 19d Synthèse du 2-{2-[4-(2-fluorophényl)-2,3-dihydro-1H-indol-1-yl]-2-oxoéthyl}-6-(morpholin-4-yl)pyrimidin-4(3H)-one

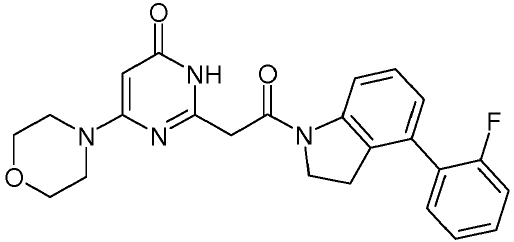

A une solution de 211 mg de [4-(morpholin-4-yl)-6-oxo-1,6-dihydropyrimidin-2-yl]acétate de sodium (exemple 1d étape 2d) dans 3 mL de pyridine sont successivement ajoutés 115 mg de 4-(2-Fluoro-phenyl)-2,3-dihydro-1H-indole (dilués dans 3 mL de pyridine) et 125 mg de N-[3-(diméthylamino)propyl]-N'-éthylcarbodiimide chlorhydrate. Le mélange réactionnel est agité à température ambiante pendant 3 jours. Le milieu réactionnel est dilué avec de l'acétate d'éthyle (100 mL) puis successivement lavé avec une solution aqueuse d'acide chlorhydrique 1 N, une solution aqueuse de soude 1 N, une solution d'eau saturée en chlorure de sodium. La phase organique est ensuite séchée sur sulfate de magnésium, filtrée sur verre fritté et concentrée sous pression réduite. On obtient 187 mg de 2-{2-[4-(2-fluorophényl)-2,3-dihydro-1H-indol-1-yl]-2-oxoéthyl}-6-(morpholin-4-yl)pyrimidin-4(3H)-one sous forme d'un solide beige dont les caractéristiques sont les suivantes:
Spectre RMN 1 H (400MHz) : 3,03 (t, J=8,4 Hz, 2 H); 3,42 (m, 4 H); 3,61 (m, 4 H); 3,77 (s, 2 H); 4,15 (t, J=8,4 Hz, 2 H); 5,21 (s, 1 H); 7,01 (d, J=7,7 Hz, 1 H); 7,22 à 7,37 (m, 3 H); 7,38 à 7,52 (m, 2 H); 8,10 (d, J=8,1 Hz, 1 H); 11,64 (s large, 1 H)
Spectrométrie de Masse : méthode A
Temps de rétention Tr (min) = 0.88;
[M+H]+ : m/z 435 ; [M-H]- : m/z 433

### Exemple 20d Synthèse du 3-méthyl-2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one

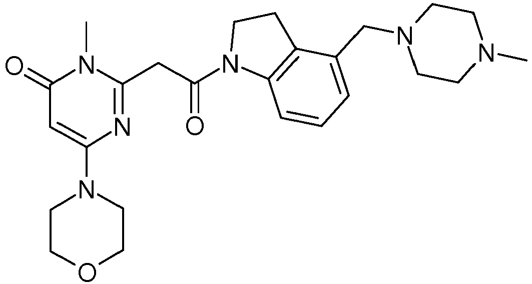

A une solution de 500 mg de [1-méthyl-4-(morpholin-4-yl)-6-oxo-1,6-dihydropyrimidin-2-yl]acétate de sodium (exemple 4d étape 2d) dans 5 mL de N,N-diméthylformamide sont successivement ajoutés 5 mL de pyridine, 404 mg de 4-(4-méthyl-piperazin-1-ylmethyl)-2,3-dihydro-1H-indole (Exemple de référence 9d) et 390 mg de N-[3-(diméthylamino)propyl]-N'-éthylcarbodiimide chlorhydrate. Le mélange réactionnel est agité à température ambiante pendant 16 heures, puis concentré sous pression réduite. Le résidu est repris dans 30 mL d'eau saturée en chlorure de sodium puis extrait avec du dichlorométhane (3x30 mL). Les phases organiques sont rassemblées puis séchées sur sulfate de magnésium, filtrées sur verre fritté et concentrées sous pression réduite. Après purification du résidu par chromatographie sur colonne de silice en éluant avec un mélange de dichlorométhane et de méthanol ammoniacal 7N (95/05), on obtient 40 mg de 3-méthyl-2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one sous forme d'un solide blanc dont les caractéristiques sont les suivantes:
Spectre RMN 1 H (400MHz) : 2,14 (s, 3 H); 2,20 à 2,44 (m, 8 H); 3,17 (t, J=8,6 Hz, 2 H); 3,31 (s, 3 H); 3,39 (m, 6 H); 3,58 (m, 4 H); 4,10 (s, 2 H); 4,18 (t, J=8,4 Hz, 2 H); 5,37 (s, 1 H); 6,95 (d, J=7,0 Hz, 1 H); 7,12 (t, J=7,9 Hz, 1 H); 7,93 (d, J=7,5 Hz, 1 H)
Spectrométrie de Masse : méthode A
Temps de rétention Tr (min) = 0.37;
[M+H]+ : m/z 467 ; [M-H]- : m/z 465

### Exemple 21 d Synthèse du 6-(morpholin-4-yl)-2-[2-oxo-2-(2',3',5',6'-tétrahydrospiro[indole-3,4'-pyran]-1(2H)-yl)éthyl]pyrimidin-4(3H)-one

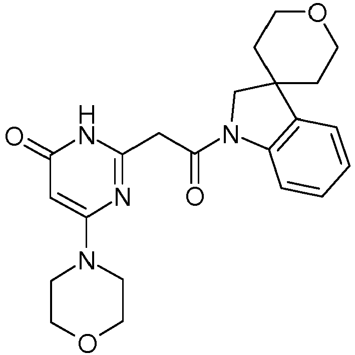

A une solution de 679 mg de [4-(morpholin-4-yl)-6-oxo-1,6-dihydropyrimidin-2-yl]acétate de sodium (exemple 1d étape 2d) dans 11,3 mL de N,N-diméthylformamide sont successivement ajoutés 3,8 mL de pyridine, 378 mg de 1,2,2',3',5',6'-hexahydrospiro[3H-indole-3,4'-[4H]pyran] et 575 mg de N-[3-(diméthylamino)propyl]-N'-éthylcarbodiimide chlorhydrate. Le mélange réactionnel est agité à température ambiante pendant 16 heures, puis concentré sous pression réduite. Le résidu est repris dans 25 mL d'eau et 20 mL d'acétate d'éthyle, puis laisser agiter à température ambiante pendant 1 heure. Le précipité formé est filtré, puis rincé successivement à l'eau et l'éther diéthylique. On obtient ainsi 640 mg de 6-(morpholin-4-yl)-2-[2-oxo-2-(2',3',5',6'-tétrahydrospiro[indole-3,4'-pyran]-1(2H)-yl)éthyl]pyrimidin-4(3H)-one sous forme d'une poudre blanc-cassé dont les caractéristiques sont les suivantes:
Spectre RMN 1 H (300MHz) : 1,53 (d, J=13,0 Hz, 2 H); 1,91 (td, J=4,8 et 12,9 Hz, 2 H); 3,41 (dd, J=4,7 et 5,0 Hz, 4 H); 3,51 (t, J=10,4 Hz, 2 H); 3,60 (m, 4 H); 3,86 (m, 4 H); 4,14 (s, 2 H); 5,21 (s, 1 H); 7,07 (t, J=7,3 Hz, 1 H); 7,20 (t, J=8,2 Hz, 1 H); 7,33 (d, J=6,7 Hz, 1 H); 8,03 (dd, J=0,3 et 7,6 Hz, 1 H); 11,63 (s large, 1 H)
Spectrométrie de Masse : méthode A
Temps de rétention Tr (min) = 0.65;
[M+H]+ : m/z 411 ; [M-H]- : m/z 409

### Exemple 22d Synthèse du 3-méthyl-6-(morpholin-4-yl)-2-[2-oxo-2-(2',3',5',6'-tétrahydrospiro[indole-3,4'-pyran]-1(2H)-yl)éthyl]pyrimidin-4(3H)-one

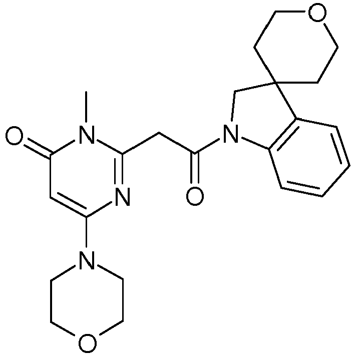

A une solution de 770 mg de [1-méthyl-4-(morpholin-4-yl)-6-oxo-1,6-dihydropyrimidin-2-yl]acétate de sodium (exemple 4d étape 2d) dans 11,3 mL de N,N-diméthylformamide sont successivement ajoutés 3,8 mL de pyridine, 378 mg de 1,2,2',3',5',6'-hexahydro-spiro[3H-indole-3,4'-[4H]pyran] et 575 mg de N-[3-(diméthylamino)propyl]-N'-éthylcarbodiimide chlorhydrate. Le mélange réactionnel est agité à température ambiante pendant 16 heures, puis concentré sous pression réduite. Le résidu est repris dans 15 mL d'eau et 20 mL d'acétate d'éthyle, puis laisser agiter à température ambiante pendant 1 heure. Le précipité formé est filtré, puis rincé successivement à l'eau et l'éther diéthylique. On obtient ainsi 45 mg de 3-méthyl-6-**(morpholin-4-yl)-2-[2-oxo-2-(2',3',5',6'-tétrahydrospiro[indole-3,4'-pyran]-1(2H)-**yl)éthyl]pyrimidin-4(3H)-one sous forme d'une poudre blanc-cassé dont les caractéristiques sont les suivantes:
Spectre RMN 1 H (400MHz) : 1,55 (d, J=12,9 Hz, 2 H); 1,91 (t, J=11,4 Hz, 2 H); 3,37 (m masqué, 8 H); 3,54 (m, 6 H); 3,88 (d, J=10,3 Hz, 2 H); 4,17 (s large, 3 H); 5,37 (s large, 1 H); 7,08 (t, J=7,9 Hz, 1 H); 7,20 (t, J=8,1 Hz, 1 H); 7,33 (d, J=8,1 Hz, 1 H); 8,03 (d, J=8,1 Hz, 1 H)
Spectrométrie de Masse : méthode A
Temps de rétention Tr (min) = 0.69;
[M+H]+ : m/z 425 ; [M-H]- : m/z 423

### Exemple 23d Synthèse du 3-méthyl-6-(morpholin-4-yl)-2-[2-oxo-2-(spiro[indole-3,4'-pipéridin]-1(2H)-yl)éthyl]pyrimidin-4(3H)-one

### Etape 1d :

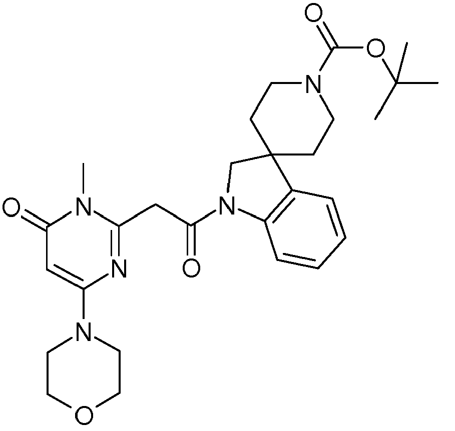

A une solution de 308 mg de [1-méthyl-4-(morpholin-4-yl)-6-oxo-1,6-dihydropyrimidin-2-yl]acétate de sodium (exemple 4d étape 2d) dans 4,5 mL de N,N-diméthylformamide sont successivement ajoutés 1,5 mL de pyridine, 231 mg de 1,2-dihydro-1'H-spiro[indole-3,4'-pipéridine]-1'-carboxylate de 2-méthylpropan-2-yle (peut être préparé selon Tetrahedron (2010), 66, 573-577) et 230 mg de N-[3-(diméthylamino)propyl]-N'-éthylcarbodiimide chlorhydrate. Le mélange réactionnel est agité à température ambiante pendant 16 heures, puis concentré sous pression réduite. Le résidu est repris dans 15 mL d'eau et 5 mL d'éther diéthylique, puis laisser agiter à température ambiante pendant 1 heure. Le précipité formé est filtré, puis rincé successivement à l'eau et l'éther diisopropylique. On obtient ainsi 230 mg de 1-{[1-méthyl-4-(morpholin-4-yl)-6-oxo-1,6-dihydropyrimidin-2-yl]acétyl}-1,2-dihydro-1'H-spiro[indole-3,4'-pipéridine]-1'-carboxylate de 2-méthylpropan-2-yle sous la forme d'un solide blanc qui est utilisé tel quel dans l'étape suivante :

### Etape 2d :

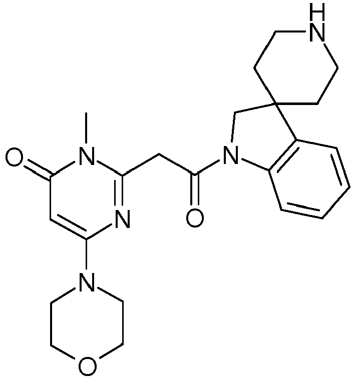

Dans un tube micro-ondes, on introduit 225 mg de 1-{[1-méthyl-4-(morpholin-4-yl)-6-oxo-1,6-dihydropyrimidin-2-yl]acétyl}-1,2-dihydro-1'H-spiro[indole-3,4'-pipéridine]-1'-carboxylate de 2-méthylpropan-2-yle dans 10 mL de dioxanne et 2 mL d'une solution aqueuse d'acide chlorhydrique 2N. Le tube est alors chauffé sous micro-ondes à 110°C pendant 10 minutes puis laissé revenir à température ambiante. Le mélange réactionnel est concentré sous pression réduite. Le résidu est repris dans 10 mL d'une solution d'une solution aqueuse saturée en hydrogénocarbonate de sodium. Après 10 minutes d'agitation, le précipité formé est filtré, puis rincé successivement à l'eau et l'éther diisopropylique. On obtient ainsi 140 mg de la 3-méthyl-6-(morpholin-4-yl)-2-[2-oxo-2-(spiro[indole-3,4'-pipéridin]-1(2H)-yl)éthyl]pyrimidin-4(3H)-one sous la forme d'une poudre blanche dont les caractéristiques sont les suivantes:
Spectre RMN 1H (400MHz) : 1,52 (d, J=12,0 Hz, 2 H); 1,70 (m, 2 H); 2,60 (t, J=12,3 Hz, 2 H); 2,90 (d, J=13,4 Hz, 2 H); 3,30 (s masqué, 3 H); 3,40 (m, 4 H); 3,57 (m, 4 H); 4,06 (s, 2 H); 4,17 (s, 2 H); 5,37 (s, 1 H); 7,06 (t, J=7,3 Hz, 1 H); 7,18 (t, J=7,8 Hz, 1 H); 7,25 (d, J=8,8 Hz, 1 H); 8,02 (d, J=7,8 Hz, 1 H)
Spectrométrie de Masse : méthode A
Temps de rétention Tr (min) = 0.42
[M+H]+ : m/z 424 ; [M-H]- : m/z 422

### Exemple 24d et Exemple 25d : Synthèse de la (+)-6-(morpholin-4-yl)-2-{2-oxo-2-[2-phényl-2,3-dihydro-1H-indol-1-yl]éthyl}pyrimidin-4(3H)-one et de la (-)-6-(morpholin-4-yl)-2-{2-oxo-2-[2-phényl-2,3-dihydro-1H-indol-1-yl]éthyl}pyrimidin-4(3H)-one

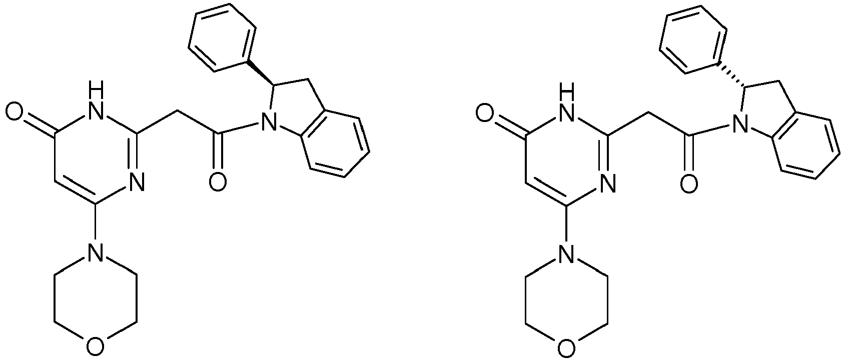

La (±)-6-(morpholin-4-yl)-2-{2-oxo-2-[2-phényl-2,3-dihydro-1H-indol-1-yl]éthyl}pyrimidin-4(3H)-one est préparée en suivant le mode opératoire décrit à l'exemple 1d étape 3d à partir de 0,50 g de [4-(morpholin-4-yl)-6-oxo-1,6-dihydropyrimidin-2-yl]acétate de sodium, de 0,42 g de 2-phényl-indoline (qui peut être préparée selon Santangelo, E.M. et al. Eur. J. Org. Chem. 2008, 5915), de 0,48 g de N-[3-(diméthylamino)propyl]-N'-éthylcarbodiimide chlorhydrate dans un mélange de 0,31 mL de pyridine et de 10 mL de N,N-diméthylformamide. Après purification par chromatographie sur une colonne de 50 g silice 20-45 µm, en éluant avec un mélange dichlorométhane/ méthanol 90/10 v/v, on obtient 0,43 g de (±)-6-(morpholin-4-yl)-2-{2-oxo-2-[2-phényl-2,3-dihydro-1H-indol-1-yl]éthyl}pyrimidin-4(3H)-one sous forme d'un solide couleur crème dont les caractéristiques sont les suivantes :
Spectrométrie de Masse : méthode A
Temps de rétention Tr (min) = 0,85 ;
[M+H]+ : m/z 417 ; [M-H]- : m/z 415

Les produits ont été obtenus par séparation chromatographique chirale de 420 mg de (±)-6-(morpholin-4-yl)-2-{2-oxo-2-[2-phényl-2,3-dihydro-1H-indol-1-yl]éthyl}pyrimidin-4(3H)-one sur colonne Chiralpak AY (T304) (1080 mg, 20 µm, 7.7/35 cm), éluant : acétonitrile/ isopropanol : 90/10 ; débit :250 mL/min. Après purification, on obtient comme premier énantiomère, 208 mg de (+)-6-(morpholin-4-yl)-2-{2-oxo-2-[2-phényl-2,3-dihydro-1H-indol-1-yl]éthyl}pyrimidin-4(3H)-one, sous forme d'une poudre rose pâle dont les caractéristiques sont les suivantes:
Spectre RMN 1H (400MHz) : 2,89 (d, J=16,3 Hz, 1 H) ; 3,13 (d, J=15,7 Hz, 1 H) ; 3,25 à 3,40 (m partiellement masqué, 4 H) ; 3,57 (m, 4 H) ; 3,81 (dd, J=9,5 et 16,3 Hz, 1 H) ; 3,86 (d, J=15,7 Hz, 1 H) ; 5,15 (s, 1 H) ; 5,77 (d, J=9,5 Hz, 1 H) ; 7,07 (t, J=7,5 Hz, 1 H) ; 7,16 à 7,40 (m, 7 H) ; 8,12 (d, J=8,3 Hz, 1 H) ; 11,60 (m étalé, 1 H)
Spectrométrie de Masse : méthode A
Temps de rétention Tr (min) = 0,84 ;
[M+H]+ : m/z 417 ; [M-H]- : m/z 415
Pouvoir rotatoire : α_{D} = +170° (c = 1,389 mg dans 0,5 mL de DMSO)

Puis le deuxième énantiomère, soit : 202 mg de (-)-6-(morpholin-4-yl)-2-{2-oxo-2-[2-phényl-2,3-dihydro-1H-indol-1-yl]éthyl}pyrimidin-4(3H)-one sous forme d'une poudre blanc cassé dont les caractéristiques sont les suivantes :
Spectre RMN 1H (400MHz): 2,89 (d, J=16,3 Hz, 1 H) ; 3,13 (d, J=15,7 Hz, 1 H) ; 3,25 à 3,38 (m partiellement masqué, 4 H) ; 3,57 (m, 4 H) ; 3,81 (dd, J=9,5 et 16,3 Hz, 1 H) ; 3,86 (d, J=15,7 Hz, 1 H) ; 5,15 (s, 1 H) ; 5,77 (d, J=9,5 Hz, 1 H) ; 7,07 (t, J=7,5 Hz, 1 H) ; 7,17 à 7,40 (m, 7 H) ; 8,12 (d, J=8,3 Hz, 1 H) ; 11,60 (m étalé, 1 H)
Spectrométrie de Masse : méthode A
Temps de rétention Tr (min) = 0,84 ;
[M+H]+ : m/z 417 ; [M-H]- : m/z 415
Pouvoir rotatoire : α_{D} = -172° (c = 0,681 mg dans 0,5 mL de DMSO)

### Exemple 26d : Synthèse de la 2-{2-[4-(4-méthylpipérazin-1-yl)-2,3-dihydro-1H-indol-1-yl]-2-oxoéthyl}-6-(morpholin-4-yl)pyrimidin-4(3H)-one :

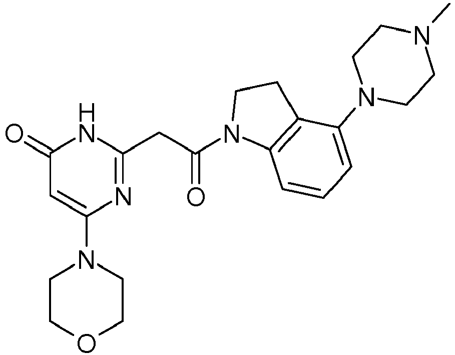

A une solution de 288 mg de [4-(morpholin-4-yl)-6-oxo-1,6-dihydropyrimidin-2-yl]acétate de sodium (exemple 1d étape 2d) dans 8 mL de N,N-diméthylformamide sont successivement ajoutés 8 mL de pyridine, 160 mg de 4-(4-méthyl-piperazin-1-yl)-2,3-dihydro-1H-indole (Exemple de référence 10d) et 212 mg de N-[3-(diméthylamino)propyl]-N'-éthylcarbodiimide chlorhydrate. Le mélange réactionnel est agité à température ambiante pendant 48 heures. Après addition de 50 mL d'eau et extraction avec de l'acétate d'éthyle, les phases organiques sont rassemblées puis séchées sur sulfate de magnésium, filtrées sur verre fritté et concentrées sous pression réduite. Après purification du résidu par chromatographie sur colonne de silice en éluant avec un mélange de dichlorométhane et de méthanol ammoniacal 7N (90/10), on obtient 29 mg de 2-{2-[4-(4-méthylpipérazin-1-yl)-2,3-dihydro-1H-indol-1-yl]-2-oxoéthyl}-6-(morpholin-4-yl)pyrimidin-4(3H)-one sous forme d'une poudre blanc-rose dont les caractéristiques sont les suivantes:
Spectre RMN 1 H (400MHz) : 2,23 (s, 3 H); 2,44 (m masqué, 4 H); 2,93 (m, J=4,2 Hz, 4 H); 3,06 (t, J=8,3 Hz, 2 H); 3,43 (m, 4 H); 3,60 (m, 4 H); 3,74 (s, 2 H); 4,11 (t, J=8,2 Hz, 2 H); 5,20 (s, 1 H); 6,67 (d, J=7,6 Hz, 1 H); 7,11 (t, J=8,4 Hz, 1 H); 7,71 (d, J=8,1 Hz, 1 H); 11,60 (s, 1 H)
Spectrométrie de Masse : méthode A
Temps de rétention Tr (min) = 0.36;
[M+H]+ : m/z 439 ; [M-H]- : m/z 437

### Exemple 27d et exemple 28d : Synthèse de la (+)-2-{2-[-2-(4-fluorophényl)-2,3-dihydro-1H-indol-1-yl]-2-oxoéthyl}-6-(morpholin-4-yl)pyrimidin-4(3H)-one et de la (-)-2-{2-[-2-(4-fluorophényl)-2,3-dihydro-1H-indol-1-yl]-2-oxoéthyl}-6-(morpholin-4-yl)pyrimidin-4(3H)-one

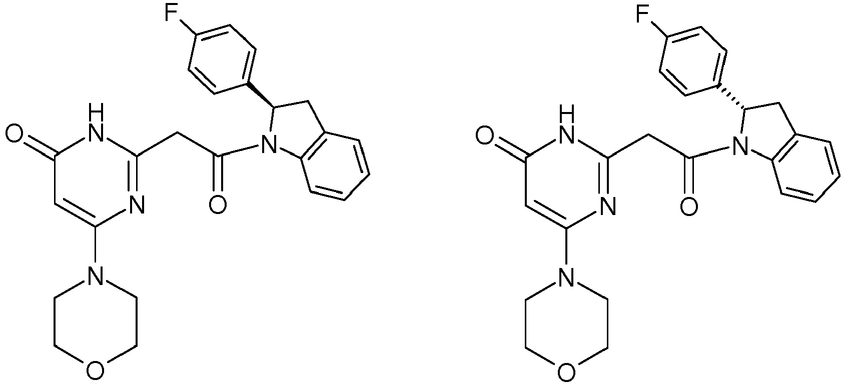

La (±)-2-{2-[2-(4-fluorophényl)-2,3-dihydro-1H-indol-1-yl]-2-oxoéthyl}-6-(morpholin-4-yl)pyrimidin-4(3H)-one est préparée en suivant le mode opératoire décrit à l'exemple 1d étape 3 à partir de 0,50 g de [4-(morpholin-4-yl)-6-oxo-1,6-dihydropyrimidin-2-yl]acétate de sodium, de 0,46 g de 2-(4-fluorophényl)-indoline, de 0,48 g de N-[3-(diméthylamino)propyl]-N'-éthylcarbodiimide chlorhydrate dans un mélange de 0,31 mL de pyridine et de 10 mL de N,N-diméthylformamide. Après purification par chromatographie sur une colonne de 50 g silice 20-45 µm, en éluant avec un mélange dichlorométhane/ méthanol 90/10 v/v, on obtient 0,46 g de (±)-2-{2-[2-(4-fluorophényl)-2,3-dihydro-1H-indol-1-yl]-2-oxoéthyl}-6-(morpholin-4-yl)pyrimidin-4(3H)-one sous forme d'une meringue couleur crème dont les caractéristiques sont les suivantes :
Spectrométrie de Masse : méthode A
Temps de rétention Tr (min) = 0,86;
[M+H]+ : m/z 435 ; [M-H]- : m/z 433

Les produits ont été obtenus par séparation chromatographique chirale de 460 mg de (±)-2-{2-[2-(4-fluorophényl)-2,3-dihydro-1H-indol-1-yl]-2-oxoéthyl}-6-(morpholin-4-yl)pyrimidin-4(3H)-one sur colonne Chiralpak AY (T304) (1080 mg, 20 µm, 7.7/35 cm), éluant : acétonitrile/ isopropanol : 90/10 ; débit : 250 mL/min. Après purification, on obtient comme premier énantiomère, 190 mg de (+)-2-{2-[2-(4-fluorophényl)-2,3-dihydro-1H-indol-1-yl]-2-oxoéthyl}-6-(morpholin-4-yl)pyrimidin-4(3H)-one, sous forme d'un lyophilisat blanc dont les caractéristiques sont les suivantes:
Spectre RMN 1H (400MHz): 2,88 (d, J=15,8 Hz, 1 H) ; 3,15 (d, J=16,3 Hz, 1 H) ; 3,33 (m, 4 H) ; 3,57 (m, 4 H) ; 3,79 (dd, J=9,5 et 16,3 Hz, 1 H) ; 3,86 (d, J=15,8 Hz, 1 H) ; 5,15 (s, 1 H) ; 5,78 (d, J=9,5 Hz, 1 H) ; 7,04 à 7,29 (m, 7 H) ; 8,11 (d, J=7,8 Hz, 1 H) ; 11,60 (m étalé, 1H)
Spectrométrie de Masse : méthode A
Temps de rétention Tr (min) = 0,86;
[M+H]+ : m/z 435 ; [M-H]- : m/z 433
Pouvoir rotatoire : α_{D} = +114.3° +/- 1.9 (c = 1,720 mg dans 0.5 mL de DMSO)

Puis le deuxième énantiomère, soit : 225 mg de (-)-2-{2-[2-(4-fluorophényl)-2,3-dihydro-1H-indol-1-yl]-2-oxoéthyl}-6-(morpholin-4-yl)pyrimidin-4(3H)-one sous forme d'un lyophilisat blanc dont les caractéristiques sont les suivantes :
Spectre RMN 1H (400MHz): 2,88 (d, J=16,3 Hz, 1 H) ; 3,15 (d, J=15,9 Hz, 1 H) ; 3,33 (m, 4 H) ; 3,57 (m, 4 H) ; 3,79 (dd, J=9,5 et 16,3 Hz, 1 H) ; 3,86 (d, J=15,9 Hz, 1 H) ; 5,15 (s, 1 H) ; 5,78 (d, J=9,5 Hz, 1 H) ; 7,03 à 7,29 (m, 7 H) ; 8,11 (d, J=7,8 Hz, 1 H) ; 11,60 (m étalé, 1 H)
Spectrométrie de Masse : méthode A
Temps de rétention Tr (min) = 0,86;
[M+H]+ : m/z 435 ; [M-H]- : m/z 433
Pouvoir rotatoire : α_{D} = -137.1° +/- 2.1 (c = 1.844 mg dans 0.5 mL de DMSO)

### Exemple 29d et Exemple 30d : Synthèse de la (+)-2-{2-[-4-(difluorométhoxy)-2-méthyl-2,3-dihydro-1H-indol-1-yl]-2-oxoéthyl}-6-(morpholin-4-yl)pyrimidin-4(3H)-one et de la (-)-2-{2-[-4-(difluorométhoxy)-2-méthyl-2,3-dihydro-1H-indol-1-yl]-2-oxoéthyl}-6-(morpholin-4-yl)pyrimidin-4(3H)-one :

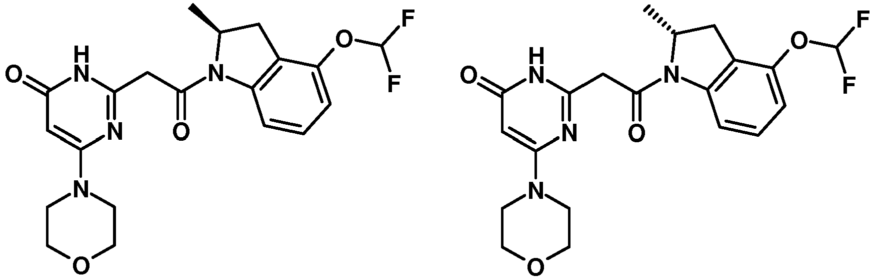

A une solution de 196,8 mg de [4-(morpholin-4-yl)-6-oxo-1,6-dihydropyrimidin-2-yl]acétate de sodium (obtenu à l'étape 2d de l'Exemple 1d) dans 6 ml de N,N-diméthylformamide et 6 ml de pyridine sont ajoutés 125 mg de 4-difluoromethoxy-2-methyl-2,3-dihydro-1H-indole (Exemple de référence 11 d) et 192,7 mg de N-[3-(diméthylamino)propyl]-N'-éthylcarbodiimide chlorhydrate. Le mélange réactionnel est agité à température ambiante pendant 16 heures, puis 25 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et de méthanol (95/05 : v/v) pour donner 220 mg de 2-{2-[-4-(difluorométhoxy)-2-méthyl-2,3-dihydro-1H-indol-1-yl]-2-oxoéthyl}-6-(morpholin-4-yl)pyrimidin-4(3H)-one.

Les énantiomères sont séparés par chromatographie chirale sur colonne phase Whelk 01 SS, (5 µm, 30x250 mm), en éluant avec un mélange de : heptane/dichlorométhane/méthanol : 60/30/10 ; débit : 43 mL/min.

On obtient comme premier énantiomère, 97 mg de (+)-2-{2-[-4-(difluorométhoxy)-2-méthyl-2,3-dihydro-1H-indol-1-yl]-2-oxoéthyl}-6-(morpholin-4-yl)pyrimidin-4(3H)-one, sous forme d'un solide blanc dont les caractéristiques sont les suivantes:
Spectre RMN 1 H (400MHz): 1,28 (d large, *J*=6,4 Hz, 3 H) ; 2,71 (d, *J*=16,3 Hz, 1 H) ; 3,35 (m partiellement masqué, 1 H) ; 3,41 (m, 4 H) ; 3,60 (m, 4 H) ; 3,73 (d, *J*=15,9 Hz, 1 H) ; 3,93 (d, *J*=15,9 Hz, 1 H) ; 4,78 (m, 1 H) ; 5,20 (s, 1 H) ; 6,90 (d, *J*=8,1 Hz, 1 H) ; 7,24 (t, J=74,2 Hz, 1 H) ; 7,26 (t, *J*=8,1 Hz, 1 H) ; 7,84 (d large, *J*=8,1 Hz, 1 H) ; 11,68 (m étalé, 1 H)
Spectrométrie de Masse : méthode A
Temps de rétention Tr (min) = 0,78 ;
[M+H]+ : m/z 421 ; [M-H]- : m/z 419;
Pouvoir rotatoire : α_{D} = +80° (c=0,25%, DMSO)

Puis le deuxième énantiomère, 97 mg de (-)-2-{2-[-4-(difluorométhoxy)-2-méthyl-2,3-dihydro-1H-indol-1-yl]-2-oxoéthyl}-6-(morpholin-4-yl)pyrimidin-4(3H)-one sous forme d'un solide blanc dont les caractéristiques sont les suivantes :
Spectre RMN 1 H (400MHz): 1,28 (d, J=6,4 Hz, 3 H) ; 2,71 (d, *J*=16,3 Hz, 1 H) ; 3,34 (m partiellement masqué, 1 H) ; 3,41 (m, 4 H) ; 3,59 (m, 4 H) ; 3,74 (d, J=15,9 Hz, 1 H) ; 3,93 (d, J=15,9 Hz, 1 H) ; 4,77 (m, 1 H) ; 5,20 (s, 1 H) ; 6,90 (d, J=8,1 Hz, 1 H) ; 7,24 (t, *J*=74,2 Hz, 1 H) ; 7,26 (t, *J*=8,1 Hz, 1 H) ; 7,84 (d large, *J*=8,1 Hz, 1 H) ; 11,69 (m étalé, 1 H)
Spectrométrie de Masse : méthode A
Temps de rétention Tr (min) = 0,78 ;
[M+H]+ : m/z 421 ; [M-H]- : m/z 419;
Pouvoir rotatoire : α_{D} = -73° (c=0,25%, DMSO)

### Exemple 31d et Exemple 32d : Synthèse de la (+)-2-{2-[-4-(difluorométhoxy)-2-méthyl-2,3-dihydro-1H-indol-1-yl]-2-oxoéthyl}-3-méthyl-6-(morpholin-4-yl)pyrimidin-4(3H)-one et de la (-)-2-{2-[-4-(difluorométhoxy)-2-méthyl-2,3-dihydro-1H-indol-1-yl]-2-oxoéthyl}-3-méthyl-6-(morpholin-4-yl)pyrimidin-4(3H)-one :

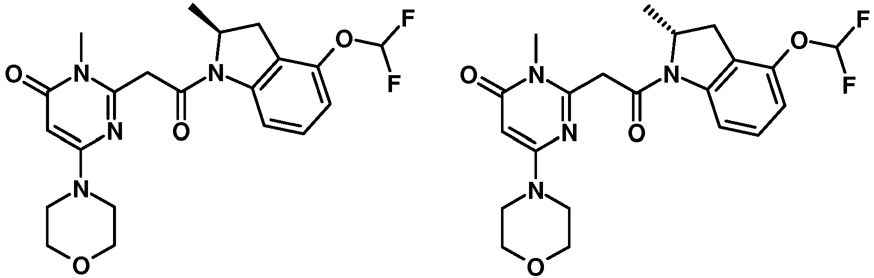

A une solution de 242 mg de [1-méthyl-4-(morpholin-4-yl)-6-oxo-1,6-dihydropyrimidin-2-yl]acétate de sodium (obtenu à l'étape 2d de l'Exemple 4d) dans 6 ml de N,N-diméthylformamide et 6 ml de pyridine sont ajoutés 125 mg de 4-difluoromethoxy-2-methyl-2,3-dihydro-1H-indole [Exemple de référence 11d] et 193 mg de N-[3-(diméthylamino)propyl]-N'-éthylcarbodiimide chlorhydrate. Le mélange réactionnel est agité à température ambiante pendant 16 heures, puis 25 mL d'eau sont ajoutés et le mélange est extrait avec de l'acétate d'éthyle. La phase organique est lavée successivement avec une solution d'acide chlorhydrique 0,1 N, de l'eau et une solution saturée de chlorure de sodium, séchée sur sulfate de magnésium, filtrée et concentrée sous pression réduite. Le résidu est purifié par chromatographie sur colonne de silice, en éluant avec un mélange de dichlorométhane, et et de méthanol (90/10 : v/v) pour donner 170 mg de 2-{2--4-(difluorométhoxy)-2-méthyl-2,3-dihydro-1H-indol-1-yl]-2-oxoéthyl}-3-méthyl-6-(morpholin-4-yl)pyrimidin-4(3H)-one.

Les énantiomères sont séparés par chromatographie chirale sur colonne Whelk 01 SS, 5 µm, (5 µm, 30x250 mm), en éluant avec un mélange de : heptane/dichlorométhane/méthanol : 50/35/15 ; débit : 40 mL/min.

On obtient le premier énantiomère, 40 mg de (+)-2-{2--4-(difluorométhoxy)-2-méthyl-2,3-dihydro-1H-indol-1-yl]-2-oxoéthyl}-3-méthyl-6-(morpholin-4-yl)pyrimidin-4(3H)-one, sous forme d'un solide blanc dont les caractéristiques sont les suivantes:
Spectre RMN 1H (400MHz): 1,30 (d, *J*=6,4 Hz, 3 H) ; 2,72 (d, *J*=15,9 Hz, 1 H) ; 3,33 (s, 3 H) ; 3,35 à 3,42 (m, 5 H) ; 3,57 (m, 4 H) ; 4,03 (d, *J*=16,4 Hz, 1 H) ; 4,29 (d, *J*=16,4 Hz, 1 H) ; 4,78 (m, 1 H) ; 5,36 (s, 1 H) ; 6,91 (d, *J*=8,1 Hz, 1 H) ; 7,24 (t, *J*=74,0 Hz, 1 H) ; 7,26 (t, *J*=8,1 Hz, 1 H) ; 7,83 (d, *J*=8,1 Hz, 1 H)
Spectrométrie de Masse : méthode A
Temps de rétention Tr (min) = 0,82 ;
[M+H]+ : m/z 435 ; [M-H]- : m/z 433;
Pouvoir rotatoire : α_{D} = +67° (c=0,3%, DMSO)

Puis le deuxième énantiomère, 61 mg de (-)-2-{2--4-(difluorométhoxy)-2-méthyl-2,3-dihydro-1H-indol-1-yl]-2-oxoéthyl}-3-méthyl-6-(morpholin-4-yl)pyrimidin-4(3H)-one, sous forme d'un solide blanc dont les caractéristiques sont les suivantes :
Spectre RMN 1 H (400MHz): 1,30 (d, *J*=6,4 Hz, 3 H) ; 2,72 (d, *J*=15,9 Hz, 1 H) ; 3,33 (s, 3 H) ; 3,35 à 3,42 (m, 5 H) ; 3,53 à 3,60 (m, 4 H) ; 4,03 (d, *J*=16,4 Hz, 1 H) ; 4,29 (d, *J*=16,4 Hz, 1 H) ; 4,78 (m, 1 H) ; 5,36 (s, 1 H) ; 6,91 (d, *J*=8,1 Hz, 1 H) ; 7,24 (t, J=74,0 Hz, 1 H) ; 7,26 (t, *J*=8,1 Hz, 1 H) ; 7,83 (d, *J*=8,1 Hz, 1 H)
Spectrométrie de Masse : méthode A
Temps de rétention Tr (min) = 0,82 ;
[M+H]+ : m/z 435 ; [M-H]- : m/z 433;
Pouvoir rotatoire : a_{D} = -91,2° (c=1,706 mg/0,5 mL DMSO)

### Exemple 33d : Synthèse de 6-(morpholin-4-yl)-2-{2-[4-(morpholin-4-yl)-2,3-dihydro-1H-indol-1-yl]-2-oxoéthyl}pyrimidin-4(3H)-one

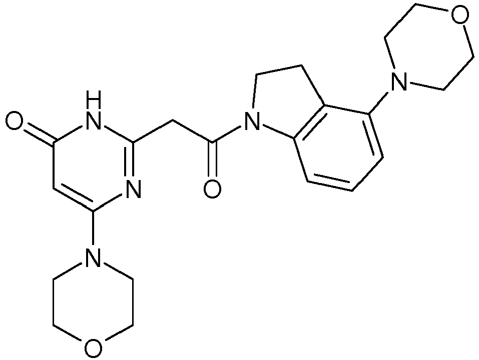

A une solution de 300 mg de [4-(morpholin-4-yl)-6-oxo-1,6-dihydropyrimidin-2-yl]acétate de sodium (exemple 1 étape 2) dans 8 mL de N,N-diméthylformamide sont successivement ajoutés 8 mL de pyridine, 200 mg de 4-morpholin-4-yl-2,3-dihydro-1H-indole (préparée à partir de la 4-bromoindoline comme décrit dans WO2007103370) et 330 mg de N-[3-(diméthylamino)propyl]-N'-éthylcarbodiimide chlorhydrate. Le mélange réactionnel est agité à température ambiante pendant 48 heures. Après addition de 50 mL d'eau et extraction avec de l'acétate d'éthyle, les phases organiques sont rassemblées puis séchées sur sulfate de magnésium, filtrées sur verre fritté et concentrées sous pression réduite. Après purification du résidu par chromatographie sur colonne de silice en éluant avec un mélange de dichlorométhane et de méthanol ammoniacal 7N (90/10), on obtient 180 mg de 6-(morpholin-4-yl)-2-{2-[4-(morpholin-4-yl)-2,3-dihydro-1H-indol-1-yl]-2-oxoéthyl}pyrimidin-4(3H)-one sous forme d'une poudre pourpre dont les caractéristiques sont les suivantes:
Spectre RMN 1 H (400MHz): 2,92 (m, 4 H); 3,08 (dd, J=8,1 et 9,3 Hz, 2 H); 3,42 (m, 4 H); 3,59 (m, 4 H); 3,72 (m, 6 H); 4,11 (m, 2 H); 5,20 (s large, 1 H); 6,68 (d, J=8,6 Hz, 1 H); 7,13 (t, J=8,9 Hz, 1 H); 7,74 (d, J=7,1 Hz, 1 H); 11,61 (s large, 1 H).
Spectrométrie de Masse : méthode A
Temps de rétention Tr (min) = 0,628 ;
[M+H]+ : m/z 426 ; [M-H]- : m/z 424

### Exemple 34d et Exemple 35d : Synthèse de la (+)-2-[2-(2-cyclopropyl-2,3-dihydro-indol-1-yl)-2-oxo-éthyl]-6-morpholin-4-yl-3H-pyrimidin-4-one et de la (-)-2-[2-(-2-cyclopropyl-2,3-dihydro-indol-1-yl)-2-oxo-éthyl]-6-morpholin-4-yl-3H-pyrimidin-4-one

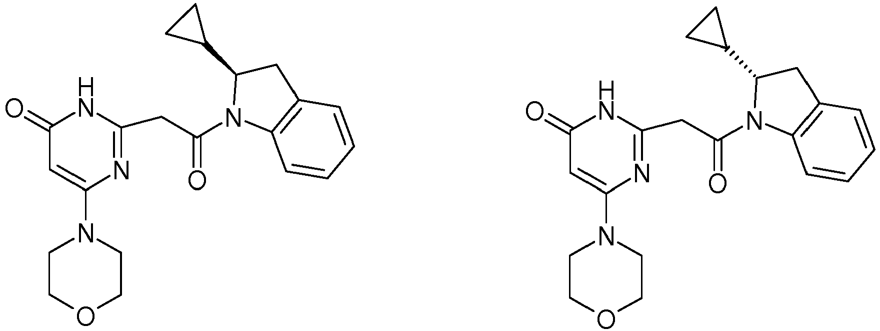

La (±)-2-[2-(-2-cyclopropyl-2,3-dihydro-indol-1-yl)-2-oxo-éthyl]-6-morpholin-4-yl-3H-pyrimidin-4-one est préparée en suivant le mode opératoire décrit à l'exemple 1 à partir de 0,90 g de [4-(morpholin-4-yl)-6-oxo-1,6-dihydropyrimidin-2-yl]acétate de sodium, de 0,60 g de (±)-2-cyclopropyl-indoline (Exemple de référence 12d), de 0,86 g de N-[3-(diméthylamino)propyl]-N'-éthylcarbodiimide chlorhydrate dans un mélange de 0,56 mL de pyridine et de 10 mL de N,N-diméthylformamide. Après purification par chromatographie sur une colonne de 100 g silice 20-45 µm, en éluant avec un mélange dichlorométhane/ méthanol 90/10 v/v, on obtient 0,88 g de (±)-2-[2-(-2-cyclopropyl-2,3-dihydro-indol-1-yl)-2-oxo-éthyl]-6-morpholin-4-yl-3H-pyrimidin-4-one sous forme d'une meringue crème dont les caractéristiques sont les suivantes :
Spectrométrie de Masse : méthode A
Temps de rétention Tr (min) = 0,79 ;
[M+H]+ : m/z 381 ; [M-H]- : m/z 379

Les produits ont été obtenus par séparation chromatographique chirale de 875 mg de (±)-2-[2-(-2-cyclopropyl-2,3-dihydro-indol-1-yl)-2-oxo-éthyl]-6-morpholin-4-yl-3H-pyrimidin-4-one sur colonne Whelk 01 SS (1200 g, 10 µm, 8/35 cm), éluant : heptane/ éthanol/ méthanol : 70/15/15; débit :250 mL/min, 2 injections. Après purification, on obtient comme premier énantiomère, 407 mg de (+)-2-[2-(-2-cyclopropyl-2,3-dihydro-indol-1-yl)-2-oxo-éthyl]-6-morpholin-4-yl-3H-pyrimidin-4-one, sous forme d'une poudre blanche dont les caractéristiques sont les suivantes:
Spectre RMN 1 H (400MHz): 0,20 à 0,59 (m, 4 H) ; 1,06 (m, 1 H) ; 2,80 (d, J=16,3 Hz, 1 H) ; 3,20 à 3,37 (m partiellement masqué, 1 H) ; 3,40 (m, 4 H) ; 3,60 (m, 4 H) ; 3,78 (d, J=15,7 Hz, 1 H) ; 3,99 (d, J=15,7 Hz, 1 H) ; 4,41 (t, J=7,8 Hz, 1 H) ; 5,20 (s, 1 H) ; 7,04 (t, J=7,9 Hz, 1 H) ; 7,17 (t, J=7,9 Hz, 1 H) ; 7,28 (d, J=7,9 Hz, 1 H) ; 7,94 (m étalé, 1 H) ; 11,67 (m étalé, 1 H)
Spectrométrie de Masse : méthode A
Temps de rétention Tr (min) = 0,78 ;
[M+H]+ : m/z 381 ; [M-H]- : m/z 379
Pouvoir rotatoire : α_{D} = +61.3° +/- 1.1 (c = 2,4 mg dans 0,5 mL de DMSO)

Puis le deuxième énantiomère, soit : 361 mg de (-)-2-[2-(-2-cyclopropyl-2,3-dihydro-indol-1-yl)-2-oxo-éthyl]-6-morpholin-4-yl-3H-pyrimidin-4-one impure sous forme d'une poudre blanche. Après repurification de 330 mg de ce produit par chromatographie chirale sur colonne Chiralpak IC (xx g, 5 µm, 2/25 cm), éluant : éthanol; débit :15 mL/min ; 16 injections, on obtient 223 mg de (-)-2-[2-(-2-cyclopropyl-2,3-dihydro-indol-1-yl)-2-oxo-éthyl]-6-morpholin-4-yl-3H-pyrimidin-4-one sous forme d'une poudre blanche dont les caractéristiques sont les suivantes :
Spectre RMN 1 H (400MHz): 0,22 à 0,59 (m, 4 H) ; 1,08 (m, 1 H) ; 2,81 (d, J=16,3 Hz, 1 H) ; 3,34 (m partiellement masqué, 1 H) ; 3,40 (m, 4 H) ; 3,60 (m, 4 H) ; 3,78 (d, J=15,9 Hz, 1 H) ; 3,99 (d, J=15,9 Hz, 1 H) ; 4,42 (t, J=7,8 Hz, 1 H) ; 5,20 (s, 1 H) ; 7,04 (t, J=7,9 Hz, 1 H) ; 7,18 (t, J=7,9 Hz, 1 H) ; 7,29 (d, J=7,9 Hz, 1 H) ; 7,94 (m étalé, 1 H) ; 11,67 (m étalé, 1 H)
Spectrométrie de Masse : méthode A
Temps de rétention Tr (min) = 0,78 ;
[M+H]+ : m/z 381 ; [M-H]- : m/z 379
Pouvoir rotatoire : α_{D} = -84.7° +/- 1.4 (c = 2,290 mg dans 0,5 mL de DMSO)

### Exemple 36d et Exemple 37d: (+)-2-{2-[-2-méthyl-4-(trifluorométhyl)-2,3-dihydro-1H-indol-1-yl]-2-oxoéthyl}-6-(morpholin-4-yl)pyrimidin-4(3H)-one et (-)-2-{2-[-2-méthyl-4-(trifluorométhyl)-2,3-dihydro-1H-indol-1-yl]-2-oxoéthyl}-6-(morpholin-4-yl)pyrimidin-4(3H)-one

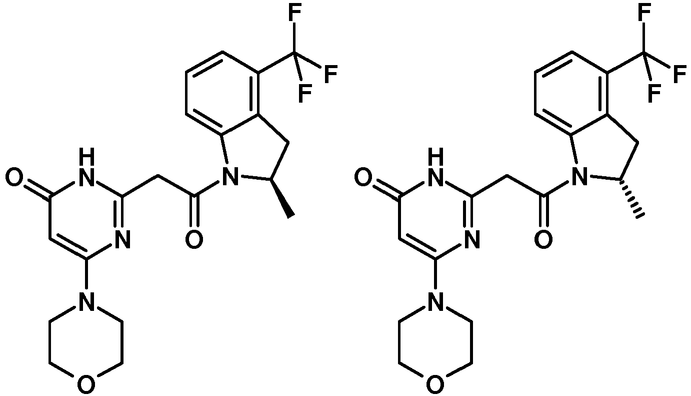

### Etape 1d: Diméthyle-[(E)-2-(2-nitro-6-trifluorométhyle-phényle)-vinyle]-amine

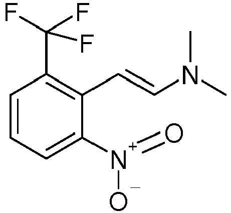

A une solution de 1g de 2-méthyle-3-nitrobenzotrifluoride dans 6 mL de N,N-diméthyleformamide sont ajoutés 3,5 mL de DMF-DMA (N,N-diméthyleformamidediméthylacétal). Le milieu réactionnel est chauffé à 110°C jusqu'à disparition du départ, puis versé sur de l'eau et extrait à l'acétate d'éthyle. Les phases organiques sont lavées successivement avec une solution de bicarbonate de sodium à 10%, une solution de chlorure d'ammonium à 10%, une solution saturée de chlorure de sodium, séchées sur sulfate de magnésium et filtrées. Le filtrat est concentré sous pression réduite pour donner 1.2g de diméthyle-[(E)-2-(2-nitro-6-trifluorométhyle-phényle)-vinyle]-amine sous forme d'une huile que l'on utilise dans l'étape suivante.

### Etape 2d: 4-Trifluorométhyle-1H-indole

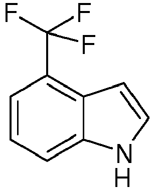

A une solution de 1.2g de diméthyle-[(E)-2-(2-nitro-6-trifluorométhyle-phényle)-vinyle]-amine dans 30ml d'acide acétique sont ajoutés 850mg de Fer en poudre. Le mélange réactionnel est chauffé au reflux pendant 16 heures puis versé sur une solution d'acide chlorhydrique (HCl, 2N), et extrait à l'acétate d'éthyle. Les phases organiques sont lavées avec une solution de carbonate de sodium à 10% puis par une solution saturée de NaCl, séchées sur sulfate de magnésium, filtrées et concentrées sous pression réduite pour donner 959mg de 4-trifluorométhyle-1H-indole que l'on utilise dans l'étape suivante.

### Etape 3d : 1-Benzenesulfonyle-4-trifluorométhyle-1H-indole

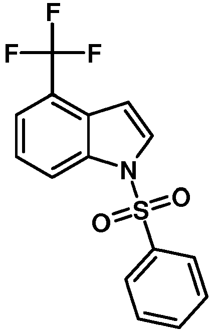

A une solution de 0.85g de 4-trifluorométhyle-1H-indole dans 15 mL de tétrahydrofurane, sous atmosphère inerte, sont ajoutés 370 mg de NaH à 60% dans l'huile. Le mélange réactionnel est agité à température ambiante pendant une heure puis 0.88 mL de chlorure de benzène-sulfonyle sont ajoutés goutte à goutte et l'agitation est poursuivie pendant 16 heures. Le mélange réactionnel est ensuite versé sur une solution à 10% de chlorure d'ammonium, extrait à l'acétate d'éthyle et séché sur sulfate de magnésium. Après filration et concentration sous pression réduite, le résidu est purifié par chromatographie sur colonne de silice, en éluant avec un mélange d'heptane et d'éther diisopropylique (98/2 puis 95/5: v/v) pour donner 1.08 g de 1-benzènesulfonyle-4-trifluorométhyle-1H-indole sous forme d'une huile jaune pâle que l'on utilise telle quelle dans l'étape suivante.

### Etape 4d : 1-Benzenesulfonyle-2-méthyle-4-trifluorométhyle-1H-indole

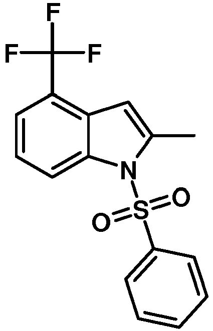

A une solution de 0.7 mL de diisopropylamine dans 10 mL de tétrahydrofurane sous argon à -30°C, sont ajoutés goutte à goutte 3 mL de n-butyllithium (1.6M dans l'hexane). La solution est agitée pendant 30 min. à température ambiante, refroidie à-60°C et une solution de 1.07 g de 1-benzènesulfonyle-4-trifluorométhyle-1H-indole dans 10 mL de tétrahydrofurane est ajoutée goutte à goutte. Le milieu réactionnel est remonté à température ambiante puis ramené à -60°C pour ajouter goutte à goutte 0,31 mL d'iodure de méthyle. Après addition, on laisse remonter à 0°C sous agitation pendant 10 min, puis à 10°C pendant 10 min avant d'ajouter 0,3 mL d'iodure de méthyle supplémentaire. Après retour à température ambiante, le mélange réactionnel est agité 1h30 puis versé sur une solution de chlorure d'ammonium à 10% et extrait à l'acétate d'éthyle. Les phases organiques sont lavées avec une solution saturée de chlorure de sodium, séchées sur sulfate de magnésium, filtrées et concentrées sous pression réduite. Le résidu est purifié par chromatographie sur colonne de silice, en éluant avec un mélange d'heptane et d'éther diisopropylique (98/2 puis 95/5 : v/v) pour donner 476 mg de 1-benzènesulfonyle-2-méthyle-4-trifluorométhyle-1H-indole, sous forme d'une huile, que l'on utilise dans l'étape suivante.

### Etape 5d : 2-Méthyle-4-trifluorométhyle-1H-indole

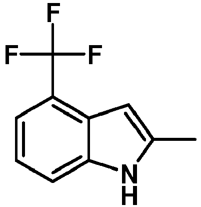

A une solution de 460 mg de 1-benzenesulfonyle-2-méthyle-4-trifluorométhyle-1 H-indole dans 1,4 mL d'éthanol et 0,44 mL de diméthoxyéthane sont ajoutés 0,271 mL de soude 5N. Le mélange réactionnel est porté au reflux pendant 6 heures et versé dans de l'eau acidifiée avec une solution d'acide chlorhydrique jusqu'à pH=1 et extrait à l'acétate d'éthyle. Les phases organiques sont réunies et lavées avec une solution de bicarbonate de sodium à 10%, une solution saturée de chlorure de sodium, séchés sur sulfate de magnésium, filtrées et concentrées sous pression réduite. Le résidu est purifié par chromatographie sur colonne de silice, en éluant avec un mélange d'heptane et d'éther diisopropylique (98/2 puis 95/5: v/v) pour donner 214 mg de 2-méthyle-4-trifluorométhyle-1H-indole, sous forme d'une huile jaune pâle, que l'on utilise dans l'étape suivante.

### Etape 6d : 2-Méthyle-4-trifluorométhyle-2,3-dihydro-1H-indole

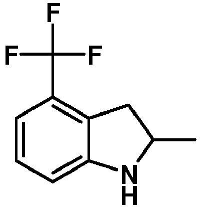

A une solution de 210 mg de 2-méthyle-4-trifluorométhyle-1 H-indole dans 8 mL d'acide acétique refroidie à 15°C sont ajoutés 209 mg de cyanoborohydrure de sodium à 95%. Le mélange réactionnel est agité pendant 16 heures à température ambiante puis versé sur une solution d'ammoniaque (pH=9). La phase aqueuse est extraite au chlorure de méthylène et les phases organiques réunies sont lavées avec une solution saturée de chlorure de sodium, séchées sur sulfate de magnésium, filtrées et concentrées sous pression réduite pour donner 196 mg de 2-méthyle-4-trifluorométhyle-2,3-dihydro-1H-indole sous forme d'un liquide jaune pâle, que l'on utilise dans l'étape suivante.

### Etape 7d : 2-[2-(2-Méthyle-4-trifluorométhyle-2,3-dihydro-indol-1-yl)-2-oxo-éthyle]-6-morpholine-4-yl-3H-pyrimidin-4-one

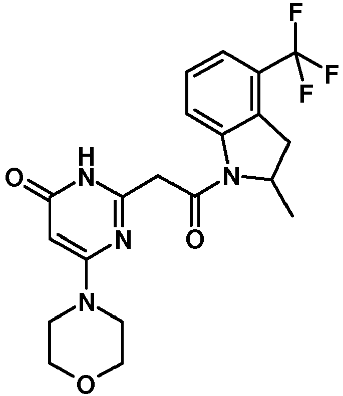

A une solution de 259 mg de [4-(morpholin-4-yl)-6-oxo-1,6-dihydropyrimidin-2-yl]acétate de sodium (exemple 1d étape 2d) dans 3 mL de N,N-diméthylformamide et 3 mL de pyridine sont ajoutés 225 mg de chlorhydrate de N-[3-(diméthylamino)propyl]-N'-éthylcarbodiimide et 196 mg de 2-méthyle-4-trifluorométhyle-2,3-dihydro-1H-indole. Le mélange réactionnel est agité à température ambiante pendant 20 heures puis dilué avec de l'eau et extrait à l'acétate d'éthyle. Les extraits sont lavés successivement avec de l'eau, une solution d'acide chlorhydrique (1 M), de l'eau et une solution saturée de chlorure de sodium. La phasea organiquea est séchée sur sulfate de magnésium filtrée et concentrée sous pression réduite pour donner 144 mg de 2-[2-(2-méthyle-4-trifluorométhyle-2,3-dihydro-indol-1-yl)-2-oxo-éthyle]-6-morpholine-4-yl-3H-pyrimidin-4-one sous forme d'un solide blanc.

La séparation des deux énantiomères de 2-[2-(2-Méthyle-4-trifluorométhyle-2,3-dihydro-indol-1-yl)-2-oxo-éthyle]-6-morpholine-4-yl-3H-pyrimidin-4-one a été réalisée par chromatographie chirale : Phase stationnaire : WHELK O1 SS 10 µm Lot P-130-84-13, 1200g, 80x350mm; phase mobile : Heptane (70%) / DCM (20%) / EtOH (10%) ; débit : 200 mL/mn.

Le premier énantiomère est concentré pour obtenir 67.2 mg de la (+)-2-[2-(2-méthyle-4-trifluorométhyle-2,3-dihydro-indol-1-yl)-2-oxo-éthyle]-6-morpholine-4-yl-3H-pyrimidin-4-one sous forme d'un solide blanc dont les caractéristiques sont les suivantes :
Spectrométrie de Masse : méthode A
Temps de rétention Tr (min) = 0,87 ;
[M+H]+ : m/z 423 ; [M-H]- : m/z 421;
RMN 1H (400 MHz) : 1,29 (d large, J=6,6 Hz, 3 H) ; 2,85 (d, J=16,4 Hz, 1H) ; 3,41 (m, 4 H) ; 3,51 (dd, J=9,0 et 16,4 Hz, 1 H) ; 3,60 (m, 4 H) ; 3,77 (d, J=16,0 Hz, 1 H) ; 3,96 (d,J=16,0 Hz, 1 H) ; 4,81 (m, 1 H) ; 5,21 (s, 1 H) ; 7,38 (d, J=7,9 Hz, 1 H) ; 7,43 (t, J=7,9 Hz, 1 H) ; 8,24 (d large, J=7,9 Hz, 1 H) ; 11,68 (m étalé, 1 H)
Pouvoir rotatoire : α_{D} = +66.7+/-1.2. C=2.090mg /0.5ML MeOH

Le deuxieme énantiomère est concentré pour obtenir 60.7 mg de la (-)-2-[2-(2-méthyle-4-trifluorométhyle-2,3-dihydro-indol-1-yl)-2-oxo-éthyle]-6-morpholine-4-yl-3H-pyrimidin-4-one sous forme d'un solide blanc dont les caractéristiques sont les suivantes :
Spectrométrie de Masse : méthode A
Temps de rétention Tr (min) = 0,87 ;
[M+H]+ : m/z 423 ; [M-H]- : m/z 421;
RMN 1H (400 MHz) : 1,29 (d large, J=6,6 Hz, 3 H) ; 2,85 (d, J=16,4 Hz, 1H) ; 3,41 (m, 4 H) ; 3,51 (dd, J=9,0 et 16,4 Hz, 1 H) ; 3,60 (m, 4 H) ; 3,77 (d, J=16,0 Hz, 1 H) ; 3,96 (d,J=16,0 Hz, 1 H) ; 4,81 (m, 1 H) ; 5,21 (s, 1 H) ; 7,38 (d, J=7,9 Hz, 1 H) ; 7,43 (t, J=7,9 Hz, 1 H) ; 8,24 (d large, J=7,9 Hz, 1 H) ; 11,68 (m étalé, 1 H)
Pouvoir rotatoire : α_{D} = -43.0+/-0.9. C=2.058mg /0.5ML MeOH

### Exemple 38d: 2-[2-((+)-2-fluoromethyl-2,3-dihydro-indol-1-yl)-2-oxo-ethyl]-6-morpholin-4-yl-3H-pyrimidin-4-one

### Etape 1 d: 2-[2-((+)-2-hydroxymethyl-2,3-dihydro-indol-1-yl)-2-oxo-ethyl]-6-morpholin-4-yl-3H-pyrimidin-4-one

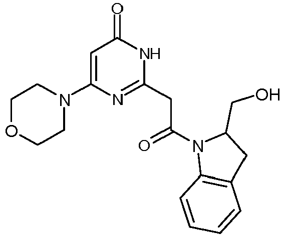

A une solution de 640 mg du (4-morpholin-4-yl-6-oxo-1,6-dihydro-pyrimidin-2-yl)-acetate de sodium dans 20 mL de DMF et 20 mL de pyridine sont ajoutés 673 mg de N-[3-(diméthylamino)propyl]-N'-éthylcarbodiimide chlorhydrate et 378 mg de (-)-(2,3-dihydro-1H-indol-2-yl)-methanol (Exemple de référence 13d). Le milieu réactionnel est agité à la température ambiante pendant 18 heures puis on ajoute un mélange d"eau et de dichlorométhane. Après décantation, la phase organique est séchée sur sulfate de magnésium, filtrée puis concentrée sous pression réduite. Le résidu obtenu est purifié sur colonne de silice, éluant : gradient: dichlorométhane/méthanol de 100/0 à 95/05 pour donner 456 mg de 2-[2-((+)-2-hydroxymethyl-2,3-dihydro-indol-1-yl)-2-oxo-ethyl]-6-morpholin-4-yl-3H-pyrimidin-4-one dont les caractéristiques sont les suivantes :
Spectrométrie de Masse : méthode A
Temps de rétention Tr (min) = 0,54 ;
[M+H]+ : m/z 371 ; [M-H]- : m/z 369
Pouvoir rotatoire : α_{D} = +80.0+/-1.4. C=2.061 mg/0.5ML DMSO

### Etape 2d:

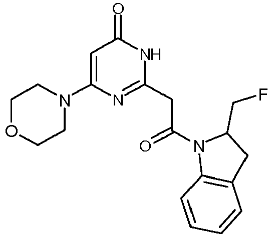

A une solution de 200 mg du 2-[2-((+)-2-hydroxymethyl-2,3-dihydro-indol-1-yl)-2-oxo-ethyl]-6-morpholin-4-yl-3H-pyrimidin-4-one dans 7 mL de dichlorométhane et 0.16 mL de triéthylamine et à 0°C, est ajouté 0.07 mL de chlorure de méthane sulfonyle. Le bain refroidissant est enlevé pour laisser la température remonter à l'ambiante. Agitation à cette même température pendant 45 minutes. Addition de l'eau froide et du dichlorométhane. Après décantation, la phase organique est lavée avec une solution saturée de bicarbonate de sodium. Après décantation, la phase organique est séchée sur sulfate de magnésium, filtrée puis concentrée sous pression réduite.

Sur le résidu obtenu, addition de 5 mL d'une solution de fluorure de tetrabutylammonium 1M dans le THF. Le milieu réactionnel est chauffé à reflux pendant une heure. Après refroidissement, addition du dichlorométhane et d'une solution saturée de bicarbonate de sodium.

Après décantation, la phase organique est séchée sur sulfate de magnésium, filtrée puis concentrée sous pression réduite. Le résidu obtenu est purifié sur colonne de silice, éluant : dichlorométhane/méthanol 97/03 pour donner 48 mg de 2-[2-((+)-2-fluoromethyl-2,3-dihydro-indol-1-yl)-2-oxo-ethyl]-6-morpholin-4-yl-3H-pyrimidin-4-one dont les caractéristiques sont les suivantes :
Spectre RMN 1 H (300 MHz) : 2,91 (d, *J*=16,3 Hz, 1 H) ; 3,32 à 3,45 (m, 5 H) ; 3,61 (m, 4 H) ; 3,78 (d, *J*=16,5 Hz, 1 H) ; 3,99 (d, *J*=16,5 Hz, 1 H) ; 4,35 à 4,68 (m, 2 H) ; 4,97 (m, 1 H) ; 5,18 (s, 1 H) ; 7,04 (t, *J*=8,0 Hz, 1 H) ; 7,18 (t, *J*=8,0 Hz, 1 H) ; 7,28 (d, J=8,0 Hz, 1 H) ; 7,83 (d large, *J*=8,0 Hz, 1 H) ; 11,40 (m étalé, 1 H)
Spectrométrie de Masse : méthode A
Temps de rétention Tr (min) = 0,66 ;
[M+H]+ : m/z 373 ; [M-H]- : m/z 371
Pouvoir rotatoire : α_{D} = +75.7+/-1.3. C=2.169mg/0.5ML DMSO

### Exemple 39d : 2-[2-(2,3-dihydro-indol-1-yl)-2-oxo-ethyl]-6-morpholin-4-yl-3-phenyl-3H-pyrimidin-4-one

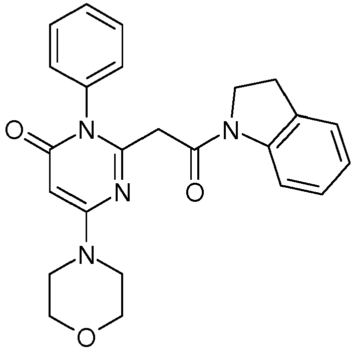

### Etape 1 d :

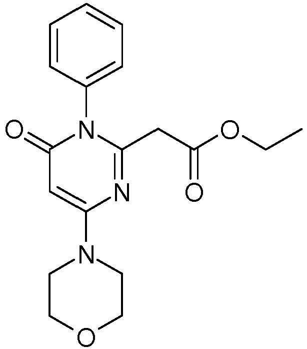

A une suspension de 1 g de [4-(morpholin-4-yl)-6-oxo-1,6-dihydropyrimidin-2-yl]acétate d'éthyle (obtenu à l'étape 1d de l'Exemple 1d), 912 mg d'acide phénylboronique, 680 mg d'acetate de cuivre (II) et 1,37 g de 4-diméthylaminopyridine dans 12 mL de toluène sont ajoutés 6,24 mL de bis-triméthylsilyl amidure de sodium 0,6 M dans le toluène. Le mélange réactionnel est chauffé à 95°C pendant 16 heures sous un courant d'air sec puis filtré sur célite. Le filtrat est concentré sous pression réduite et le résidu purifié deux fois par chromatographie sur colonne de silice, en éluant avec un mélange de dichlorométhane et de méthanol (90/10, V/V) puis avec un mélange de diisopropyléther et de méthanol (90/10, V/V) pour donner 65 mg de (4-morpholin-4-yl-6-oxo-1-phényl-1,6-dihydro-pyrimidin-2-yl)-acetate d'éthyl sous forme d'un solide blanc dont les caractéristiques sont les suivantes :
Spectre RMN 1 H (400MHz) : 1,06 (t, J=7,0 Hz, 3 H); 3,43 (s, 2 H); 3,51 (m, 4 H); 3,65 (m, 4 H); 3,94 (q, J=7,0 Hz, 2 H); 5,43 (s, 1 H); 7,22 (m, 2 H); 7,49 (m, 3 H)

### Etape 2d :

A une solution de 65 mg de (4-morpholin-4-yl-6-oxo-1-phényl 1,6-dihydropyrimidin-2-yl)-acetate d'éthyl dans 3 mL de tétrahydrofurane et 1,5 mL de toluène sont ajoutés successivement 56 mg d'indoline et 0,36 mL de triméthyl aluminium 2 M dans le toluène. Le mélange réactionnel est chauffé à 90°C pendant 4 heures puis concentré sous pression réduite. Le résidu est purifié deux fois par chromatographie sur colonne de silice, en éluant avec un mélange de dichlorométhane et de méthanol (98/2, V/V) puis avec de l'acétate d'éthyle pour donner 3 mg de 2-[2-(2,3-dihydro-indol-1-yl)-2-oxo-ethyl]-6-morpholin-4-yl-3-phenyl-3H-pyrimidin-4-one sous forme d'un solide dont les caractéristiques sont les suivantes :
Spectre RMN 1 H (400MHz) : 2,97 (t, J=8,4 Hz, 2 H); 3,48 (m, 4 H); 3,62 (td, J=3,6 et 4,5 Hz, 8 H); 5,44 (s, 1 H); 6,99 (t, J=7,2 Hz, 1 H); 7,14 (t, J=7,7 Hz, 1 H); 7,18 (d, J=7,5 Hz, 1 H); 7,26 (m, 2 H); 7,41 (m, 3 H); 7,94 (d, J=7,9 Hz, 1 H)
Spectrométrie de Masse : méthode A
Temps de rétention Tr (min) = 0.79;
[M+H]+ : m/z 417 ; [M-H]- : m/z 415

### Exempte 40d : 2-[2-((-)-2-fluoromethyl-2,3-dihydro-indol-1-yl)-2-oxo-ethyl]-6-morpholin-4-yl-3H-pyrimidin-4-one

### Etape 1d: 2-[2-((-)-2-Hydroxymethyl-2,3-dihydro-indol-1-yl)-2-oxo-ethyl]-6-morpholin-4-yl-3H-pyrimidin-4-one

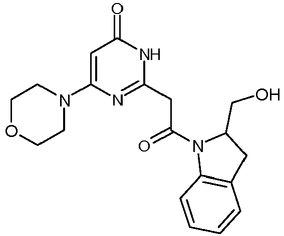

A une solution de 876 mg du (4-morpholin-4-yl-6-oxo-1,6-dihydro-pyrimidin-2-yl)-acetate de sodium dans 26 mL de DMF et 26 mL de pyridine sont ajoutés 890 mg de N-[3-(diméthylamino)propyl]-N'-éthylcarbodiimide chlorhydrate et 500 mg de (+)-(2,3-dihydro-1H-indol-2-yl)-methanol (Exemple de référence 13d). Le milieu réactionnel est agité à la température ambiante pendant 18 heures puis on ajoute un mélange d'eau et de dichlorométhane. Après décantation, la phase organique est séchée sur sulfate de magnésium, filtrée puis concentrée sous pression réduite. Le résidu obtenu est purifié sur colonne de silice, éluant : gradient: dichlorométhane/méthanol de 100/0 à 95/05 pour donner 708 mg de 2-[2-((-)-2-hydroxymethyl-2,3-dihydro-indol-1-yl)-2-oxo-ethyl]-6-morpholin-4-yl-3H-pyrimidin-4-one dont les caractéristiques sont les suivantes :
Spectrométrie de Masse : méthode A
Temps de rétention Tr (min) = 0,55 ;
[M+H]+ : m/z 371 ; [M-H]- : m/z 369
Pouvoir rotatoire :α_{D} = PR= -66.7+/-1.2. C=2.035mg/0.5ML DMSO

### Etape 2d:

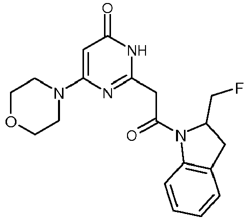

A une solution de 200 mg du 2-[2-((-)-2-hydroxymethyl-2,3-dihydro-indol-1-yl)-2-oxo-ethyl]-6-morpholin-4-yl-3H-pyrimidin-4-one dans 7 mL de dichlorométhane et 0.16 mL de triéthylamine et à 0°C, est ajouté 0.07 mL de chlorure de méthane sulfonyle. Le bain refroidissant est enlevé pour laisser la température remonter à l'ambiante. Le milieu réactionnel est agité à cette même température pendant 45 minutes. Addition de l'eau froide et du dichlorométhane. Après décantation, la phase organique est lavée avec une solution saturée de bicarbonate de sodium. Après décantation, la phase organique est séchée sur sulfate de magnésium, filtrée puis concentrée sous pression réduite.

Sur le résidu obtenu, addition de 5 mL d'une solution de fluorure de tetrabutylammonium 1M dans le THF. Le milieu réactionnel est chauffé à reflux pendant une heure. Après refroidissement, addition du dichlorométhane et d'une solution saturée de bicarbonate de sodium.

Après décantation, la phase organique est séchée sur sulfate de magnésium, filtrée puis concentrée sous pression réduite. Le résidu obtenu est purifié sur colonne de silice, éluant : dichlorométhane/méthanol 97/03 pour donner 42 mg de 2-[2-((-)-2-fluoromethyl-2,3-dihydro-indol-1-yl)-2-oxo-ethyl]-6-morpholin-4-yl-3H-pyrimidin-4-one dont les caractéristiques sont les suivantes :
Spectre RMN 1 H (300 MHz) : 2,90 (d, *J*=16,6 Hz, 1 H); 3,38 (m, 5 H); 3,59 (t, J=4,6 Hz, 4 H); 3,75 (d, *J*=15,4 Hz, 1 H); 3,98 (d, *J*=15,7 Hz, 1 H); 4,53 (m, 2 H); 4,97 (m, 1 H); 5,20 (s, 1 H); 7,05 (t, *J*=7,1 Hz, 1 H); 7,18 (t, *J*=7,7 Hz, 1 H); 7,28 (d, *J*=7,3 Hz, 1 H); 7,94 (d, J=5,4 Hz, 1 H); 11,67 (s large, 1 H)
Spectrométrie de Masse : méthode A
Temps de rétention Tr (min) = 0,66 ;
[M+H]+ : m/z 373 ; [M-H]- : m/z 371
Pouvoir rotatoire : α_{D} = PR= -91.5+/-1.7. C=1.572mg/0.5ML DMSO

### Exemple 41d : Synthèse du 3-amino-2-[2-(2,3-dihydro-1H-indol-1-yl)-2-oxoéthyl]-6-(morpholin-4-y))pyrimidin-4(3H)-one

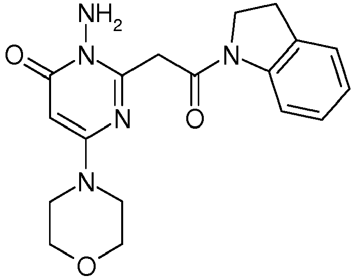

### Etape 1d :

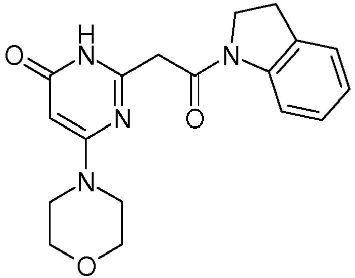

Le produit est préparé en suivant le mode opératoire décrit à l'exemple 1d étape 3d à partir de 260 mg de [4-(morpholin-4-yl)-6-oxo-1,6-dihydropyrimidin-2-yl]acétate de sodium et 238 mg de indoline au lieu d N-[3-(diméthylamino)propyl]-N'-éthylcarbodiimide chlorhydrate. On obtient 230 mg de 2-[2-(2,3-dihydro-1H-indol-1-yl)-2-oxoéthyl]-6-(morpholin-4-yl)pyrimidin-4(3H)-one sous forme d'un solide rose pâle dont les caractéristiques sont les suivantes :
Spectre RMN 1 H :3,17 (t, J=8,3 Hz, 2 H) ; 3,41 (m, 4 H) ; 3,60 (m, 4 H) ; 3,75 (s, 2 H) ; 4,14 (t, J=8,3 Hz, 2 H) ; 5,21 (s, 1 H) ; 7,01 (t, J=7,6 Hz, 1 H) ; 7,16 (t, J=7,6 Hz, 1 H) ; 7,25 (d, J=7,6 Hz, 1 H) ; 8,02 (d, J=7,6 Hz, 1 H) ; 11,61 (s large, 1 H)
Spectrométrie de Masse : méthode A
Temps de rétention Tr (min) = 0,64
[M+H]+ : m/z 341 ; [M-H]- : m/z 339

### Etape 2d :

A une suspension de 500 mg de 2-[2-(2,3-dihydro-1H-indol-1-yl)-2-oxoéthyl]-6-(morpholin-4-yl)pyrimidin-4(3H)-one (qui peut être obtenu selon l'étape précédente) dans 5 mL de diméthylformamide, sont successivement additionnés 957 mg de carbonate de césium et 685 mg d'o-diphénylphosphinylhydroxylamine. Le mélange réactionnel est agité à température ambiante pendant 2 heures puis dilué avec 10 mL d'eau et extrait par du dichlorométhane (3x25 mL). Les phases organiques sont rassemblées puis séchées sur sulfate de magnésium anhydre, filtrées puis concentrée à sec sous pression réduite. Le résidu est purifié par chromatographie sur colonne de silice, en éluant avec un mélange de dichlorométhane de méthanol (95/05). Les fractions d'intérêt contenant le produit sont réunies puis évaporées sous pression réduite et obtient 154 mg de 3-amino-2-[2-(2,3-dihydro-1*H*-indol-1-yl)-2-oxoéthyl]-6-(morpholin-4-yl)pyrimidin-4(3*H*)-one sous forme d' solide jaune pâle dont les caractéristiques sont les suivantes :
Spectre RMN 1 H : 3,17 (t, J=8,5 Hz, 2 H) ; 3,41 (m, 4 H) ; 3,60 (m, 4H) ; 4,04 (s large, 2 H) ; 4,16 (t, J=8,5 Hz, 2 H) ; 5,37 (s, 2 H) ; 5,45 (s, 1 H) ; 7,01 (td, J=1,7 et 7,9 Hz, 1 H) ; 7,15 (t large, J=7,9 Hz, 1 H) ; 7,25 (d large, J=7,9 Hz, 1 H) ; 8,02 (d, J=7,9 Hz, 1 H)
Spectrométrie de Masse : méthode A
Temps de rétention Tr (min) = 0,68 ;
[M+H]+ : m/z 356 ; [M-H]- : m/z 354

### Exemple 42d : Synthèse du 2-[2-(2,3-dihydro-1H-indol-1-yl)-2-oxoéthyl]-4-(morpholin-4-yl)-6-oxopyrimidine-1(6H)-carbonitrile

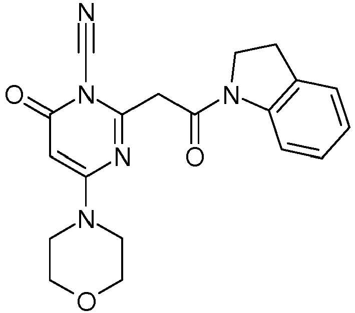

A une solution de 1 g de 2-[2-(2,3-dihydro-1H-indol-1-yl)-2-oxoéthyl]-6-(morpholin-4-yl)pyrimidin-4(3H)-one (qui peut être obtenu selon l'exemple 41 d étape 1d) dans 40 mL de dioxanne avec du tamis moléculaire 4A, sont successivement additionnés 330 mg de carbonate de césium et 404 mg de bromure de cyanogène. Le mélange réactionnel est chauffé à 40 °C pendant 2 heures puis refroidi à température ambiante. La suspension est filtrée sur verre fritté puis rincée avec du dioxanne et le filtrat est concentré sous pression réduite. Le résidu est purifié par chromatographie sur colonne de silice, en éluant avec un mélange de dichlorométhane de méthanol (95/05). Les fractions d'intérêt contenant le produit sont réunies puis évaporées sous pression réduite. Le résidu est repris par un mélange de 3 mL de méthanol et de 10 mL d'éther éthylique. Après agitation et filtration, le précipité obtenu est rincé avec de l'éther éthylique (3x2mL) et on obtient 22 mg de 2-[2-(2,3-dihydro-1*H*-indol-1-yl)-2-oxoéthyl]-4-(morpholin-4-yl)-6-oxopyrimidine-1(6*H*)-carbonitrile sous forme de solide beige dont les caractéristiques sont les suivantes :
Spectre RMN 1 H : 3,19 (t, J=8,5 Hz, 2 H) ; 3,55 à 3,69 (m, 8 H) ; 4,17(t, J=8,5 Hz, 2 H) ; 4,25 (s, 2 H) ; 5,45 (s, 1 H) ; 7,05 (t, J=8,2 Hz, 1 H) ; 7,18 (t, J=8,2 Hz, 1 H) ; 7,28 (d,J=8,2 Hz, 1 H) ; 8,00 (d, J=8,2 Hz, 1 H)
Spectrométrie de Masse : méthode A
Temps de rétention Tr (min) = 0,75 ;
[M+H]+ : m/z 366 ; [M-H]- : m/z 364

### Exemples de référence pour la préparation des composés de formule (Id)

### Exemple de référence 1d : Synthèse du 4-phényl-2,3-dihydro-1H-indole :

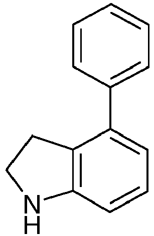

A une solution de 1,5 g de 4-phényl-indole dans 20 mL d'acide trifluoroacétique sous argon refroidie à une température voisine de -5°C, est ajouté progressivement 1,0 g de cyanoborohydrure de sodium. Le mélange réactionnel est agité à une température voisine de 0°C pendant 2 heures, puis il est jeté dans 50 g de glace et alcalinisé avec 30 mL d'une solution de soude concentrée. Après ajout de 100 mL d'acétate d'éthyle, le mélange est agité à température ambiante et traité avec 10 mL de soude concentrée, puis décanté. La phase organique est séchée sur sulfate de magnésium anhydre, filtrée puis concentrée à sec sous pression réduite. Le résidu est retraité avec un mélange de 50 mL d'eau, 50 mL d'acétate d'éthyle et 10 mL d'une solution de soude concentrée et agité à température ambiante pendant 15 minutes, puis décanté. La phase organique est séparée et la phase aqueuse est extraite avec 2x50 mL d'acétate d'éthyle. Les phases organiques sont réunies, séchées sur sulfate de magnésium anhydre, filtrées puis concentrées à sec sous pression réduite. Le résidu est trituré dans 20 mL d'éther diisopropylique puis concentré à sec sous pression réduite. Le résidu est purifié par chromatographie sur une colonne de 50 g de silice 20-45 µm, en éluant avec du dichlorométhane pur. On obtient ainsi 0,74 g de 4-phényl-2,3-dihydro-1 H-indole sous forme d'un solide couleur crème dont les caractéristiques sont les suivantes :
Spectrométrie de Masse : méthode A
Temps de rétention Tr (min) = 0,55 ;
[M+H]+ : m/z 196

### Exemple de référence 2d : 4-trifluoromethoxy-2,3-dihydro-1H-indole

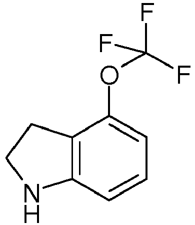

### Etape 1d :

A une solution de 1,0 g d'acide 4-(trifluorométhoxy)-1H-indole-2-carboxylique dans 4,5 mL de quinoléine sous argon sont ajoutés 0,18 g de cuivre(0). Le mélange réactionnel est chauffé à 200°C pendant 5 heures, puis il est refroidi à température ambiante. Après dilution avec 30 mL d'éther diéthylique, le mélange est filtré sur Clarcel^{®}. Le filtrat est lavé successivement avec 6x10 mL d'une solution d'acide chlorhydrique 6N, avec 10 mL d'une solution saturée d'hydrogénocarbonate de sodium, puis avec 10 mL de saumure saturée. La phase organique est séchée sur sulfate de magnésium anhydre, filtrée puis concentrée à sec sous pression réduite. Le résidu est purifié par chromatographie sur une cartouche de 25 g de silice 15-40 µm, en éluant avec un mélange cyclohexane/ acétate d'éthyle 95/5 v/v, à un débit de 20 mL/ min. On obtient ainsi 0,33 g de 4-(trifluorométhoxy)-indole sous forme d'une huile ambrée dont les caractéristiques sont les suivantes :
Spectre RMN 1 H (400MHz) : 6,48 (s large, 1 H) ; 6,96 (d, J=7,9 Hz, 1 H) ; 7,14 (t, J=7,9 Hz, 1 H) ; 7,41 à 7,47 (m, 2 H) ; 11,49 (m large, 1 H)
Spectrométrie de Masse : méthode A
Temps de rétention Tr (min) = 1,01 ;
[M-H]- : m/z 200

### Etape 2d :

A une solution de 0,87 g de 4-(trifluorométhoxy)-indole dans 12 mL d'acide trifluoroacétique sous argon refroidie à une température voisine de -5°C, est ajouté progressivement 0,57 g de cyanoborohydrure de sodium. Le mélange réactionnel est laissé se réchauffer à 0°C pendant 3 heures, puis il est jeté dans 30 g de glace et alcalinisé avec 21 mL d'une solution de soude concentrée. Après agitation pendant 19 heures, le mélange est dilué avec 60 mL d'acétate d'éthyle, puis agité à température ambiante pendant 30 minutes. Après décantation, la phase organique est séparée et la phase aqueuse est extraite avec 3x60 mL d'acétate d'éthyle. Les phases organiques sont réunies, séchées sur sulfate de magnésium anhydre, filtrées puis concentrées à sec sous pression réduite. Le résidu est retraité avec un mélange de 50 mL d'eau, 50 mL d'acétate d'éthyle et 10 mL d'une solution de soude concentrée et agité à température ambiante pendant 30 minutes, puis décanté. La phase organique est séparée et la phase aqueuse est extraite avec 3x50 mL d'acétate d'éthyle. Les phases organiques sont réunies, séchées sur sulfate de magnésium anhydre, filtrées puis concentrées à sec sous pression réduite. Le résidu est purifié par chromatographie sur une cartouche de 25 g de silice 15-40 µm, en éluant avec des mélanges cyclohexane/ acétate d'éthyle 95/5 ; 90/10 ; puis 85/15 v/v, à un débit de 25 mL/ min. On obtient ainsi 0,18 g de 4-(trifluorométhoxy)-indoline impure sous forme d'une huile jaune directement utilisée dans l'étape suivante .

### Exemple de référence 3d : Synthèse du 4-(2-méthoxy-phényl)-2,3-dihydro-1H-indole :

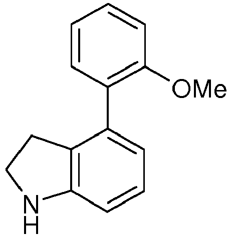

### Etape 1d :

A une solution de 0,78 g de 4-bromo-indole dans 15 mL de dioxanne et 5 mL d'eau sous argon, sont successivement ajoutés 0,73 g d'acide 2-méthoxyphénylboronique, puis 1,66 g de carbonate de potassium. Le mélange réactionnel est agité à température ambiante pendant 15 minutes, puis 0,16 g de dichlorobis(tri-o-tolylphosphine)palladium(II) est additionné. Le mélange réactionnel est agité à température ambiante pendant 16 heures, puis à 60°C pendant 18 heures, et enfin à 110°C pendant 3 heures. Après retour à température ambiante, le mélange est concentré à sec sous pression réduite. Le résidu est repris dans un mélange de 15 mL d'une solution de soude 2N et 30 mL d'acétate d'éthyle. Le mélange est filtré sur Clarcel^{®}, puis décanté. La phase organique est séparée et la phase aqueuse est extraite avec 2x30 mL d'acétate d'éthyle. Les phases organiques sont réunies, séchées sur sulfate de magnésium anhydre, filtrées puis concentrées à sec sous pression réduite. Le résidu est purifié sur une cartouche de 30 g de silice 15-40 µm, en éluant avec un mélange cyclohexane/ dichlorométhane 60/40 v/v, à un débit de 30 mL/ min. On obtient ainsi 0,44 g de 4-(2-méthoxy-phényl)-indole sous forme d'un solide jaune pâle don dont les caractéristiques sont les suivantes :
Spectrométrie de Masse : méthode A
Temps de rétention Tr (min) = 1,01 ;
[M+H]+ : m/z 224
Point de fusion (Kofler) : 172°C

### Etape 2d :

A une solution de 0,44 g de 4-(2-méthoxy-phényl)-indole dans 5 mL d'acide trifluoroacétique sous argon refroidie à une température voisine de -5°C, est ajouté progressivement 0,26 g de cyanoborohydrure de sodium. Le mélange réactionnel est agité à une température voisine de -5°C pendant 2 heures, puis il est jeté dans 15 g de glace et alcalinisé avec 8 mL d'une solution de soude concentrée. Après ajout de 30 mL d'acétate d'éthyle, le mélange est agité à température ambiante pendant 1 heure, puis décanté. La phase organique est séparée et la phase aqueuse est extraite avec 15 mL d'acétate d'éthyle. Les phases organiques sont réunies, séchées sur sulfate de magnésium anhydre, filtrées puis concentrées à sec sous pression réduite. Le résidu est retraité avec un mélange de 15 mL d'eau, 15 mL d'acétate d'éthyle et 3 mL d'une solution de soude concentrée et agité à température ambiante pendant 15 minutes, puis décanté. La phase organique est séparée et la phase aqueuse est extraite avec 2x15 mL d'acétate d'éthyle. Les phases organiques sont réunies, séchées sur sulfate de magnésium anhydre, filtrées puis concentrées à sec sous pression réduite. Le résidu est purifié par chromatographie sur une cartouche de 25 g de silice 15-40 µm, en éluant avec du dichlorométhane pur, à un débit de 30 mL/ min. On obtient ainsi 0,14 g de 4-(2-méthoxyphényl)-2,3-dihydro-1H-indole sous forme d'un solide blanc cassé dont les caractéristiques sont les suivantes :
Spectrométrie de Masse : méthode A
Temps de rétention Tr (min) = 0,55 ;
[M+H]+ : m/z 226
Point de fusion (Kofler) : 82°C

### Exemple de référence 4d : 4-(1-propyl-piperidin-3-yl)-2,3-dihydro-1H-indole

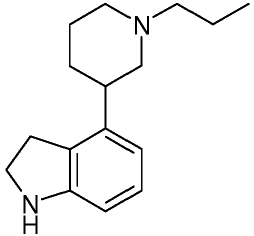

Le produit peut être préparé tel que décrit à l'Exemple de référence 1d étape 2d par réduction de l'indole correspondant (qui peut être préparé selon le brevet EP21924 Exemple 17d).

### Exemple de référence 5d : 4-difluoromethoxy-2,3-dihydro-1H-indole

### Etape 1d:

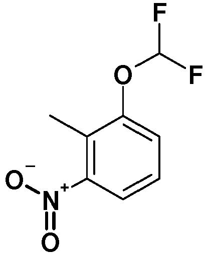

A un mélange de 23,4 g de chlorodifluoroacétate de sodium et de 22 g de carbonate de potassium dans 30 mL de N,N-diméthylformamide et 6 mL d'eau, est ajoutée une solution de 10 g de 2-méthyle-3-nitro-phénol dans 20 mL de N,N-diméthylformamide. Le mélange réactionnel est ensuite chauffé à 110°C pendant 3 heures et laissé reposer 16 heures, puis traité avec un mélange d'eau et d'acétate d'éthyle. La phase organique est lavée avec de la soude 1N, de l'eau, une solution saturée de chlorure de sodium, séchée sur sulfate de magnésium, filtrée et concentrée sous pression réduite pour donner le produit attendu sous forme d'une huile brune, utilisé tel quel dans l'étape suivante.

### Etape 2d:

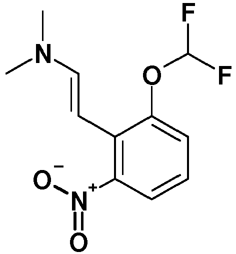

A une solution de 6 g de 1-difluoromethoxy-2-methyl-3-nitro-benzene dans 60 mL de N,N-diméthylformamide, sont ajoutés 17 g de tris(diméthylamino) méthane et le mélange réactionnel est chauffé à 100°C pendant 72 heures. Le mélange réactionnel est ensuite concentré sous pression réduite pour donner 7,6 g d'une huile brutte utilisée telle quelle dans l'étape suivante.

### Etape 3d:

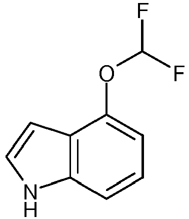

A une solution de 7,6 g de [2-(2-difluorométhoxy-6-nitro-phényl)-vinyl]-diméthyl-amine dans 35 mL de méthanol et 35 mL de tétrahydrofuranne, sous une atmosphère d'argon sont ajoutés 7 g de Nickel de Raney. Le mélange réactionnel est chauffé à 60°C et 5,6 mL d'hydrate d'hydrazine sont ajoutés en 4 portions de 1,4 mL toutes les 30 minutes et le mélange est agité pendant 16 heures.

### Etape 4d:

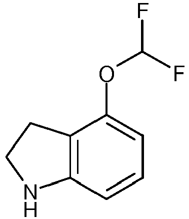

A une solution de 1 g de 4-difluoromethoxyl-1 H-indole dans 15 mL d'acide trifluoroacétique, refroidie à 0°C sont ajoutés 0,722 mg de cyanoborohydrure de sodium et l'agitation est poursuivie pendant 3 heures. Le mélange réactionnel est ensuite concentré sous pression réduite, traité avec une solution concentrée d'hydroxyde de sodium, extrait au dichlorométhane, séché sur sulfate de magnésium, filtré et concentré sous pression réduite. Le résidu est purifié par chromatographie sur colonne de silice, en éluant avec un mélange d'heptane et d'acétate d'éthyle (20/80 : v/v) pour donner 300 mg de 4-**Difluoromethoxyl-2,3-dihydro-1H-indole** sous forme d'une huile jaune dont les caractéristiques sont les suivantes :
Spectre RMN 1 H (400 MHz) : 2,91 (t, J=8,6 Hz, 2 H) ; 3,45 (dt, J=2,0 et 8,6 Hz, 2 H) ; 5,73 (m large, 1 H) ; 6,30 (d, J=7,9 Hz, 1 H) ; 6,36 (d, J=7,9 Hz, 1 H) ; 6,93 (t, J=7,9 Hz, 1 H) ; 7,10 (t, J=74,8 Hz, 1 H)

### Exemple de référence 6d : 4-(2-chloro-phényl)-2,3-dihydro-1H-indole

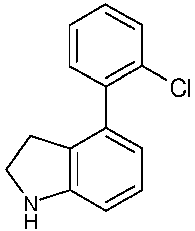

A une suspension de 960 mg de 4-(2-Chloro-phenyl)-1H-indole, dans 11 mL d'acide trifluoroacétique refroidit à 0°C, sont ajoutés par portions 558 g de cyanoborohydrure de sodium. Le milieu réactionnel est agité pendant 16 heures en le laissant remonter à la température ambiante puis le pH est amené jusqu'à la neutralité avec une solution de soude. Le mélange réactionnel est dilué avec 300 mL d'eau puis extrait avec de l'acétate d'éthyle (2x250 mL). Les phases organiques sont réunies puis concentrées à sec sous pression réduite. Après purification du résidu par chromatographie sur colonne de silice en éluant avec un mélange d'heptane et d'acétate d'éthyle (80/20), on obtient 395 mg de **4-(2-chloro-phényl)-2,3-dihydro-1H-indole** sous la forme d'une huile visqueuse incolore.

### Exemple de référence 7d: 1,2,3,3a,4,8b-hexahydro-cyclopenta[b]indole

A une solution de 1.5 g de 1,2,3,4-tétrahydro-cyclopenta[b]indole dans 40 mL d'acide acétique refroidit à 15°C, sont ajoutés par portions 1.9 g de cyanoborohydrure de sodium. Le milieu réactionnel est agité pendant 20 heures en le laissant remonter à la température ambiante puis le pH est amené jusqu'à la neutralité avec une solution d'ammoniaque à 28%. Le mélange réactionnel est dilué avec de l'eau puis extrait au dichlorométhane, la phase organique est lavée avec une solution saturée de chlorure de sodium, séchée sur sulfate de magnésium, filtrée et concentrée sous pression réduite. Le résidu est purifié par chromatographie sur colonne de silice en éluant avec un mélange d'heptane et d'acétate d'éthyle (95/05), pour donner 1.3 g de 1,2,3,3a,4,8b-hexahydrocyclopenta[b]indole sous la forme d'une huile incolore.

### Exemple de référence 8d : 4-(4-méthanesulfonyl-pipérazin-1-ylmethyl)-2,3-dihydro-1H-indole

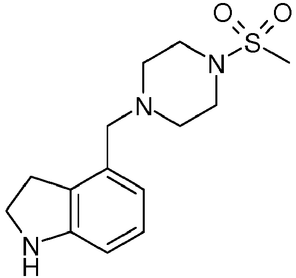

A une solution de 1 g d'indole-4-carboxaldéhyde dans 40 mL de tetrahydrofuranne sous argon, sont successivement ajoutés 2,92 g de triacétoxyborohydrure de sodium, 2,26 de chlorhydrate de 1-méthanesulfonyl-pipérazine et 545 mg de pyridine. Le mélange réactionnel est agité à température ambiante pendant 15 heures, puis concentré sous pression réduite. Le résidu est repris dans 40 mL d'acide acétique refroidis à 15°C puis, 1,30 g de cyanoborohydrure de sodium sont ajoutés par portions. Le milieu réactionnel est agité pendant 2 heures en le laissant remonter à la température ambiante, versé sur un mélange eau/glace, traité avec de l'ammoniaque à 28% jusqu'à pH neutre et extrait au dichlorométhane (4x30 mL). Les phases organiques sont rassemblées, séchées sur sulfate de magnésium, filtrées puis concentrées sous pression réduite. Le résidu obtenu est purifié sur colonne de silice (éluant : heptane/acétate d'éthyle de 0 à 100% d'acétate d'éthyle) pour donner 890 mg de **4-(4-méthanesulfonyl-pipérazin-1-ylmethyl)-2,3-dihydro-1H-indole** sous forme d'un solide jaune.

### Exemple de référence 9d : 4-(4-méthyl-piperazin-1-ylmethyl)-2,3-dihydro-1H-indole

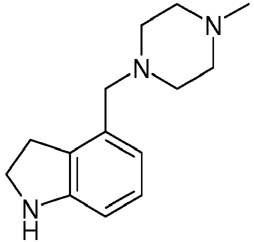

A une solution de 1 g d'indole-4-carboxaldéhyde dans 40 mL de tetrahydrofuranne sous argon, sont successivement ajoutés 1 ,38 g de 1-méthylpipérazine et 2,92 g de triacétoxyborohydrure de sodium. Le mélange réactionnel est agité à température ambiante pendant 15 heures, puis concentré sous pression réduite. Le résidu est repris dans 40 mL d'acide acétique refroidis à 15°C puis, 1,30 g de cyanoborohydrure de sodium sont ajoutés par portions. Le milieu réactionnel est agité pendant 2 heures en le laissant remonter à la température ambiante, versé sur un mélange eau/glace, traité avec de l'ammoniaque à 28% jusqu'à pH neutre et extrait au dichlorométhane (4x50 mL). Les phases organiques sont rassemblées, séchées sur sulfate de magnésium, filtrées puis concentrées sous pression réduite. Le résidu obtenu est purifié sur colonne de silice, éluant : dichlorométhane/méthanol ammoniacal 7N 95/05 pour donner 1,22 g de **4-(4-méthyl-piperazin-1-ylmethyl)-2,3-dihydro-1H-indole** sous forme d'un solide jaune.

### Exemple de référence 10d : 4-(4-méthyl-piperazin-1-yl)-2,3-dihydro-1H-indole

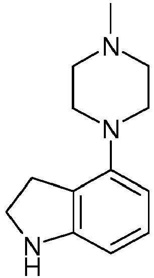

A une solution de 490 mg de 4-(4-méthyl-piperazin-1-yl)-1H-indole, dans 17 mL d'acide acétique refroidit à 14°C, sont ajoutés par portions 429 mg de cyanoborohydrure de sodium. Le milieu réactionnel est agité pendant 2 heures à température ambiante puis versé dans un mélange eau/glace et le pH est amené jusqu'à la neutralité avec une solution d'ammoniaque. Le mélange est ensuite extrait au dichlorométhane et les phases organiques réunies sont séchées sur sulfate de magnésium, filtrées et concentrées sous pression réduite. Le résidu est purifié par chromatographie sur colonne de silice en éluant avec un mélange de dichlorométhane et de méthanol (95/05), pour donner 395 mg de 4-**(4-méthyl-piperazin-1-yl)-2,3-dihydro-1H-indole** sous la forme d'une huile visqueuse incolore.

### Exemple de référence 11 d : 4-difluoromethoxy-2-methyl-2,3-dihydro-1H-indole

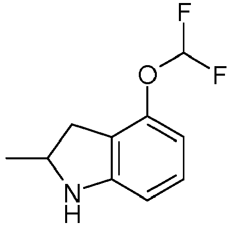

### Etape 1d:

### 4-Difluoromethoxyl-2-methyl-indole

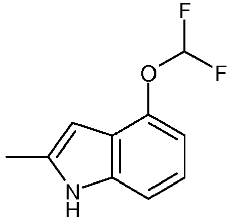

Du fréon-22 (HCF₂Cl) est mis à buller dans une solution de 3 g de 4-hydroxy-2-methyl-indole dans 90 mL de dichlorométhane à 0°C, contenant une faible quantité de bromure de tétrabutylammonium (utilisé comme agent de transfert de phase). A cette solution sont ajoutés goutte à goutte 45 mL d'une solution de soude 10M. Le mélange réactionnel est ensuite agité pendant 2 heures à 0°C puis est laissé remonter à la température ambiante. Les phases sont séparées et la phase organique concentrée sous pression réduite. Le résidu obtenu est purifié sur colonne de silice en éluant au dichlorométhane pour donner 480 mg de 4-Difluoromethoxyl-2-methyl-indole sous forme d'une huile jaune dont les caractéristiques sont les suivantes :
Spectre RMN 1H (400MHz) : 2,38 (s, 3 H); 6,15 (s, 1 H); 6,71 (d, J=7,2 Hz, 1 H); 6,97 (t, J=8,0 Hz, 1 H); 7,20 (t, J=75,0 Hz, 1 H); 7,16 (d, J=8,1 Hz, 1 H); 11,19 (d, J=0,4 Hz, 1 H)

### Etape 2d:

### 4-Difluoromethoxyl-2-methyl-2,3-dihydro-1H-indole

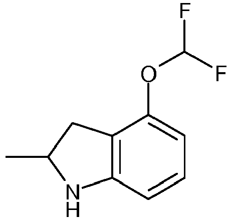

A une solution de 480 mg de 4-difluoromethoxyl-2-methyl-indole dans 15 mL d'acide acétique refroidit à 10°C, sont ajoutés par portions 459 mg de cyanoborohydrure de sodium. Le milieu réactionnel est agité pendant 16 heures en le laissant remonter à la température ambiante.

Le milieu réactionnel est versé sur un mélange eau/glace puis traité avec de l'ammoniaque à 28% et extrait à l'acétate d'éthyle. La phase organique est séchée sur sulfate de magnésium, filtrée puis concentrée sous pression réduite. Le résidu obtenu est purifié sur colonne de silice, éluant : heptane/acétate d'éthyle 80/20 pour donner 250 mg de 4-Difluoromethoxyl-2-methyl-2,3-dihydro-1H-indole sous forme d'une huile dont les caractéristiques sont les suivantes :
Spectre RMN 1 H (400MHz) : 1,17 (d, J=6,2 Hz, 3 H) ; 2,47 (dd, J=7,6 et 15,9 Hz, 1 H) ; 3,06 (dd, J=8,9 et 15,9 Hz, 1 H) ; 3,91 (m, 1 H) ; 5,85 (m large, 1 H) ; 6,25 à 6,35 (m, 2 H) ; 6,93 (t, J=7,9 Hz, 1 H) ; 7,09 (t, J=74,8 Hz, 1 H)

### Exemple de référence 12d : Synthèse de la (-)-2-isopropyl-indoline et de la (+)-2-cyclopropyl-indoline :

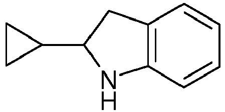

### Etape 1d

A une solution de 1,0 g de 2-iodo-aniline dans 5 mL de triéthylamine sous argon, sont successivement ajoutés 0,26 g d'iodure de cuivre(I), 0,30 g de dichlorure de bis(triphénylphosphine)palladium(II), puis 0,78 mL de cyclopropylacétylène. Le mélange réactionnel est agité à température ambiante pendant 30 minutes, puis 1 mL de N,N-diméthylformamide est ajouté. Le mélange réactionnel est agité à température ambiante pendant 30 minutes, puis 1 mL de N,N-diméthylformamide est rajouté. Le mélange réactionnel est agité à température ambiante pendant 15 heures, puis il est jeté dans 100 mL d'eau. Après ajout de 50 mL d'acétate d'éthyle, le mélange est filtré sur Celite^{®}, puis décanté. La phase organique est séparée et la phase aqueuse est extraite avec 3x40 mL d'acétate d'éthyle. Les phases organiques sont réunies, lavées avec 2x40 mL d'eau, séchées sur sulfate de magnésium anhydre, filtrées puis concentrées à sec sous pression réduite. Le résidu est purifié sur une colonne de 50 g de silice 20-45 µm, en éluant avec un mélange dichlorométhane/ cyclohexane 80/20 v/v. On obtient ainsi 0,42 g de 2-cyclopropyléthynyl-phénylamine sous forme d'une huile marron qui est utilisée directement dans l'étape suivante.

### Etape 2d

A une solution de 0,42 g de 2-cyclopropyléthynyl-phénylamine dans 20 mL de N,N-diméthylformamide sous argon, est ajouté 0,54 g d'iodure de cuivre(I). Le mélange réactionnel est chauffé à reflux pendant 2 heures, puis il est concentré à sec sous pression réduite. Le résidu est repris dans un mélange de 200 mL d'eau et 100 mL de dichlorométhane, filtré sur Celite^{®}, puis décanté. La phase organique est séparée et la phase aqueuse est extraite avec 50 mL de dichlorométhane. Les phases organiques sont réunies, lavées avec 100 mL d'eau, séchées sur sulfate de magnésium anhydre, filtrées puis concentrées à sec sous pression réduite. Le résidu est purifié sur une colonne de 50 g de silice 20-45 µm, en éluant avec un mélange cyclohexane/ dichlorométhane 50/50 v/v. On obtient ainsi 0,18 g 2-cyclopropyl-indole sous forme d'un solide jaune dont les caractéristiques sont les suivantes :
Spectrométrie de Masse : méthode A
Temps de rétention Tr (min) = 0,96 ;
[M+H]+ : m/z 158

### Etape 3d

A une solution de 1,25 g de 2-cyclopropyl-indole dans 80 mL d'acide acétique sous argon refroidie à une température voisine de 15°C, est ajouté progressivement 1,0 g de cyanoborohydrure de sodium. Le mélange réactionnel est agité à une température voisine de 15°C pendant 2 heures, puis il est traité avec 100 mL d'eau. Il est ensuite refroidi à une température voisine de 5°C et alcalinisé par ajout progressif de 140 mL d'une solution de soude à 30%. Le mélange réactionnel est dilué avec 200 mL d'acétate d'éthyle, puis il est agité à température ambiante pendant 15 heures. Après décantation, la phase organique est séparée et la phase aqueuse est extraite avec 3x200 mL d'acétate d'éthyle. Les phases organiques sont réunies, séchées sur sulfate de magnésium anhydre, filtrées puis concentrées à sec sous pression réduite. Le résidu est repris dans un mélange de 60 mL d'acétate d'éthyle, 40 mL d'eau et 10 mL de soude concentrée, puis le mélange est agité à température ambiante pendant 10 minutes. Après décantation, la phase organique est séparée et la phase aqueuse est extraite avec 2x50 mL d'acétate d'éthyle. Les phases organiques sont réunies, séchées sur sulfate de magnésium anhydre, filtrées puis concentrées à sec sous pression réduite. On obtient ainsi 1,2 g de (±)-2-cyclopropyl-indoline sous forme d'une huile marron qui est utilisée telle quelle.

### Exemple de référence 13d : 2-Hydroxyméthyl-2,3-dihydro-1H-indole

### Etape 1d:

### 1-((R)-2-Benzyloxy-propionyl)-2,3-dihydro-1H-indole-2-carboxylic acid ethyl ester : diastéréoisomère A

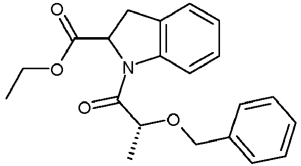

### Et

### 1-((R)-2-Benzyloxy-propionyl)-2,3-dihydro-1H-indole-2-carboxylic acid ethyl ester diastéréoisomère B

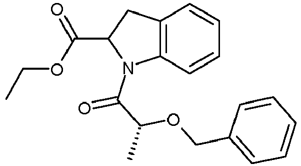

A une solution de 12.6 g d'acide o-benzyl-D-lactique dans 30 mL de DMF et 10.6 mL de pyridine sont ajoutés 16.6 g de N-[3-(diméthylamino)propyl]-N'-éthylcarbodiimide chlorhydrate puis 10 g d'indoline-2-carboxylate d'éthyle. Le milieu réactionnel est agité à la température ambiante pendant 18 heures. Le milieu réactionnel est concentré sous pression réduite au 2/3 du volume du milieu réactionnel. Addition de l'acétate d'éthyle et d'eau. Après décantation, la phase organique est séchée sur sulfate de magnésium, filtrée puis concentrée sous pression réduite. Le résidu obtenu est purifié sur cartouche de silice : éluant : gradient : heptane/acétate d'éthyle de 100/0 à 80/20, pour donner 7.24 g de 1-((R)-2-benzyloxy-propionyl)-2,3-dihydro-1H-indole-2-carboxylic acid ethyl ester : diastéréoisomère A dont les caractéristiques sont les suivantes :
Spectrométrie de Masse : méthode A
Temps de rétention Tr (min) = 1,08 ;
[M+H]+ : m/z 354 ; [M+Na]+ : m/z 376 (pic de base)

Et 7.5 g du 1-((R)-2-benzyloxy-propionyl)-2,3-dihydro-1H-indole-2-carboxylic acid ethyl ester: diastéréoisomère B sous forme d'un solide blanc dont les caractéristiques sont les suivantes :
Spectrométrie de Masse : méthode A
Temps de rétention Tr (min) = 1,06 ;
[M+H]+ : m/z 354 ; [M+Na]+ : m/z 376 ; pic de base : m/z 282

### Etape 2d:

### (R)-2-Benzyloxy-1-(2-hydroxymethyl-2,3-dihydro-indol-1-yl)-propan-1-one : diastéréoisomère A

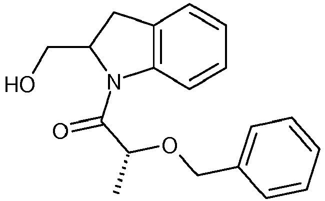

### Et

### (+)-(2,3-Dihydro-1H-indol-2-yl)-methanol

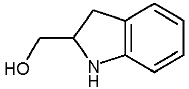

A une solution de 3.31 g du 1-((R)-2-benzyloxy-propionyl)-2,3-dihydro-1H-indole-2-carboxylic acid ethyl ester: diastéréoisomère A dans 7.5 mL de THF et 7.5 mL d'éthanol sont ajoutés 1.04 g de borohydrure de sodium.

Le milieu réactionnel est agité à la température ambiante pendant 5 heures.

Addition du dichlorométhane et de l'eau. Après décantation, la phase organique est séchée sur sulfate de magnésium, filtrée puis concentrée sous pression réduite. Le résidu obtenu est purifié sur cartouche de silice : éluant : heptane/acétate d'éthyle 50/50, pour donner 0.98 g de (R)-2-benzyloxy-1-(2-hydroxymethyl-2,3-dihydro-indol-1-yl)-propan-1-one : diastéréoisomère A dont les caractéristiques sont les suivantes :
Spectrométrie de Masse : méthode A
Temps de rétention Tr (min) = 0.85 ;
[M+H]+ : m/z 312

Et 1.65 g du (+)-(2,3-dihydro-1H-indol-2-yl)-methanol sous forme d'un solide blanc dont les caractéristiques sont les suivantes :
Spectrométrie de Masse : méthode A
Temps de rétention Tr (min) = 0,19 ;
[M+H]+ : m/z 150

### Etape 3d:

### (+)-(2,3-Dihydro-1H-indol-2-yl)-methanol

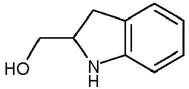

A une solution de 0.9 g du (R)-2-benzyloxy-1-(2-hydroxymethyl-2,3-dihydro-indol-1-yl)-propan-1-one : diastéréoisomère A dans 9 mL d'éthanol et 9 mL d'acide chlorhydrique 37% sont chauffés à reflux pendant deux heures.

Le milieu réactionnel est concentré sous pression réduite. Le résidu obtenu est repris avec de l'eau. Addition de la soude 2N jusqu'au PH=10. Le milieu est extrait avec du dichlorométhane. Après décantation, la phase organique est séchée sur sulfate de magnésium, filtrée puis concentrée sous pression réduite pour donner 0.4 g du (+)-(2,3-Dihydro-1H-indol-2-yl)-methanol dont les caractéristiques sont les suivantes :
Spectrométrie de Masse : méthode A
Temps de rétention Tr (min) = 0,19 ;
[M+H]+ : m/z 150
PR= +38.5+/-0.9. C=1.974mg/0.5ML DMSO

### Etape 4d:

### (R)-2-Benzyloxy-1-(2-hydroxymethyl-2,3-dihydro-indol-1-yl)-propan-1-one : diastéréoisomère B

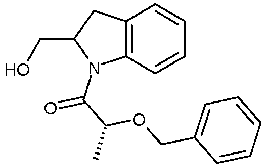

### Et

### (-)-(2,3-Dihydro-1H-indol-2-yl)-methanol

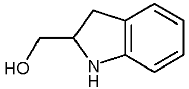

A une solution de 5.75 g du 1-((R)-2-benzyloxy-propionyl)-2,3-dihydro-1H-indole-2-carboxylic acid ethyl ester: diastéréoisomère B dans 20 mL de THF sont ajoutés 1.36 g de borohydrure de sodium.

Le milieu réactionnel est agité à la température ambiante pendant 18 heures.

Addition de 10 mL d'éthanol et 0.4 g de borohydrure de sodium. Après deux heures d'agitation à la température ambiante, addition du dichlorométhane et de l'eau.

Après décantation, la phase organique est séchée sur sulfate de magnésium, filtrée puis concentrée sous pression réduite. Le résidu obtenu est purifié sur cartouche de silice : éluant : gradient : dichlorométhane/méthanol de100/0 à 98/02, pour donner 0.51 g de (R)-2-benzyloxy-1-(2-hydroxymethyl-2,3-dihydro-indol-1-yl)-propan-1-one : diastéréoisomère B dont les caractéristiques sont les suivantes :
Spectrométrie de Masse : méthode A
Temps de rétention Tr (min) = 0,82 ; [M+H]+ : m/z 312

Et 0.96 g du (-)-(2,3-dihydro-1H-indol-2-yl)-methanol sous forme d'un solide blanc dont les caractéristiques sont les suivantes :
Spectrométrie de Masse : méthode A
Temps de rétention Tr (min) = 0,19 ; [M+H]+ : m/z 150
PR= -38.9+/-0.8. C=2.255mg/0.5ML DMSO

### Etape 5d:

### (-)-(2,3-Dihydro-1H-indol-2-yl)-methanol

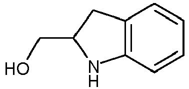

A une solution de 117 mg du (R)-2-benzyloxy-1-(2-hydroxymethyl-2,3-dihydro-indol-1-yl)-propan-1-one : diastéréoisomère B dans 1.2 mL d'éthanol et 1.2 mL d'acide chlorhydrique 37% sont chauffés à reflux pendant deux heures.

Le milieu réactionnel est concentré sous pression réduite. Le résidu obtenu est repris avec de l'eau. Addition de la soude 2N jusqu'au PH=10. Le milieu est extrait avec du dichlorométhane. Après décantation, la phase organique est séchée sur sulfate de magnésium, filtrée puis concentrée sous pression réduite pour donner 50 mg du (-)-(2,3-Dihydro-1H-indol-2-yl)-methanol dont les caractéristiques sont les suivantes :
Spectrométrie de Masse : méthode A
Temps de rétention Tr (min) = 0,19 ;
[M+H]+ : m/z 150
PR= -38.9+/-0.8. C=2.255mg/0.5ML DMSO

Les pouvoirs rotatoires (PR) ont été effectués sur un polarimètre modèle 341 de chez Perkin Elmer. longueur d'onde: raie α du sodium ( 589 nanomètres)

### Synthèse des composés de formule (le) :

### Exemple 1e : Synthèse du 1-((S)-2-Methyl-2,3-dihydro-indol-1-yl)-2-(4-morpholin-4-yl-6-thioxo-1,6-dihydro-pyrimidin-2-yl)-ethanone

### Etape 1e:

### [4-(Morpholin-4-yl)-6-oxo-1,6-dihydropyrimidin-2-yl]acétate d'éthyle

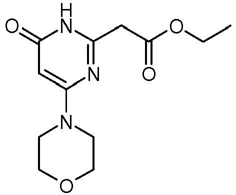

A une solution de 25 g de morpholine dans 400 mL d'éthanol chauffée à 95 °C sont ajoutés 168.5 mL de chlorhydrate de 3-éthoxy-3-iminopropanoate d'éthyle, puis 155 mL de N,N-düsopropyléthylamine dans 200 mL d'éthanol. Le mélange réactionnel est chauffé à 95 °C pendant 30 heures puis laissé revenir à température ambiante. Le précipité formé est filtré sur verre fritté puis lavé avec 100 mL d'éthanol, 2 fois 500 mL d'eau et enfin 500 mL d'éther éthylique. Le solide est séché sous vide pour donner 35 g de [4-(morpholin-4-yl)-6-oxo-1,6-dihydropyrimidin-2-yl]acétate d'éthyle sous forme de solide blanc dont les caractéristiques sont les suivantes :
Spectre RMN 1H (400MHz) : 1,19 (t, J=7,1 Hz, 3 H); 3,38 à 3,44 (m, 4 H); 3,56 (s, 2H); 3,61 (dd, J=4,0 et 5,7 Hz, 4 H); 4,12 (q, J=7,1 Hz, 2 H); 5,20 (s, 1 H); 11,69 (s large, 1 H)
Spectrométrie de Masse : méthode A
Temps de rétention Tr (min) = 0.48;
[M+H]+ : m/z 268 ; [M-H]- : m/z 266

### Etape 2e:

### (4-Morpholin-4-yl-6-thioxo-1,6-dihydro-pyrimidin-2-yl)-acetic acid ethyl ester

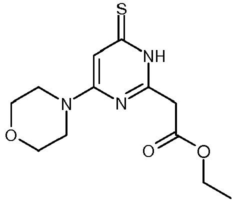

Dans un ballon, le mélange de 1 g du [4-(morpholin-4-yl)-6-oxo-1,6-dihydropyrimidin-2-yl]acétate d'éthyle obtenu à l'étape précédente avec 2.2 g de 2,4-disulfure de 2,4-bis(4-méthoxyphényl)-1,3-dithia-2,4-diphosphétane (réactif de Lawesson) dans 20 mL de toluène sont chauffés à 60°C pendant 18 heures.

Après refroidissement, le solide formé est filtré. Le filtrat est concentré sous pression réduite. Le résidu obtenu est purifié par chromatographie sur colonne de silice (éluant : dichlorométhane/acétate d'éthyle 85/15) pour donner 0.86 g du (4-morpholin-4-yl-6-thioxo-1,6-dihydro-pyrimidin-2-yl)-acetic acid ethyl ester sous forme d'un solide beige dont les caractéristiques sont les suivantes :
Spectrométrie de Masse : méthode A
Temps de rétention Tr (min) = 0,51 ;
[M+H]+ : m/z 284 ; [M-H]- : m/z 282 ; pic de base : m/z 236

### Etape 3e :

### (4-Morpholin-4-yl-6-thioxo-1,6-dihydro-pyrimidin-2-yl)-acetate de sodium

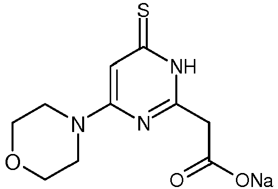

A une solution de 865 mg du (4-morpholin-4-yl-6-thioxo-1,6-dihydro-pyrimidin-2-yl)-acetic acid éthyl ester dans 15 mL de THF est ajouté 3.05 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 0.8 g de (4-morpholin-4-yl-6-thioxo-1,6-dihydro-pyrimidin-2-yl)-acetate de sodium dont les caractéristiques sont les suivantes :
Spectrométrie de Masse : méthode A
Temps de rétention Tr (min) = 0,31 ;
[M+H]+ : m/z 256 ; [M-H]- : m/z 254 ; pic de base : m/z 210

### Etape 4e:

### Synthèse du 1-((S)-2-Methyl-2,3-dihydro-indol-1-yl)-2-(4-morpholin-4-yl-6-thioxo-1,6-dihydro-pyrimidin-2-yl)-ethanone

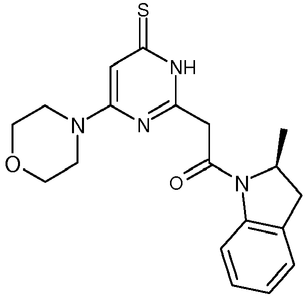

A une solution de 200 mg du (4-morpholin-4-yl-6-thioxo-1,6-dihydro-pyrimidin-2-yl)-acetate de sodium dans 5 mL de DMF et 5 mL de pyridine sont ajoutés 432 mg de N-[3-(diméthylamino)propyl]-N'-éthylcarbodiimide chlorhydrate et 106 mg de (S)-2-methyl-2,3-dihydro-1H-indole (qui peut être préparée selon Krasnov, V.P. et Coll. (Mendeleev Commun. 2002, 12(1), 27-28).

Le milieu réactionnel est agité à la température ambiante pendant 18 heures.

Addition de 50 mL de dichlorométhane et 20 mL d'eau. Après décantation, la phase organique est séchée sur sulfate de magnésium, filtrée puis concentrée sous pression réduite. Le résidu obtenu est purifié chromatographie sur colonne de silice (éluant : dichlorométhane/méthanol 99/1 puis 98/02) pour donner 57 mg du 1-((S)-2-methyl-2,3-dihydro-indol-1-yl)-2-(4-morpholin-4-yl-6-thioxo-1,6-dihydro-pyrimidin-2-yl)-ethanone sous forme d'un solide beige dont les caractéristiques sont les suivantes :
Spectre RMN 1H (400 MHz) pour ce lot tous les signaux sont larges avec : 1,26 (m, 3 H) ; 2,69 (d, *J*=15,2 Hz, 1 H) ; 3,38 (dd, *J*=9,4 et 15,2 Hz, 1 H) ; 3,50 à 3,66 (m, 8 H) ; 3,87 (d, *J*=15,4 Hz, 1 H) ; 4,06 (d, *J*=15,4 Hz, 1 H) ; 4,70 (m, 1 H) ; 6,37 (s, 1 H) ; 7,05 (t, *J*=8,0 Hz, 1 H) ; 7,18 (t, *J*=8,0 Hz, 1 H) ; 7,29 (d, *J*=8,0 Hz, 1 H) ; 7,95 (d, *J*=8,0 Hz, 1 H) ; 12,79 (m étalé, 1 H)
Spectrométrie de Masse : méthode A
Temps de rétention Tr (min) = 0,75 ;
[M+H]+ : m/z 371 ; [M-H]- : m/z 369
Pouvoir rotatoire : α_{D} =+86.0+/-1.6. C=1.606mg/0.5ML DMSO

### Etape 4'e:

Alternativement, le composé **1-((S)-2-Methyl-2,3-dihydro-indol-1-yl)-2-(4-morpholin-4-yl-6-thioxo-1,6-dihydro-pyrimidin-2-yl)-ethanone** peut également être obtenu en trois étapes :

### Etape (4'a)e:

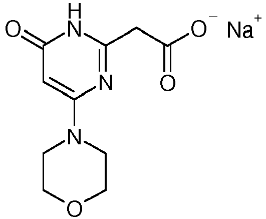

A une solution de 10 g de [4-(morpholin-4-yl)-6-oxo-1,6-dihydropyrimidin-2-yl]acétate d'éthyle dans 300 mL de tétrahydrofuranne, est ajouté 18.7 mL de soude 2M. Le mélange réactionnel est agité pendant 48 heures à température ambiante. Le précipité formé est filtré sur verre fritté, lavé à l'acétate d'éthyle et rincé plusieurs fois à l'éther éthylique. Le solide obtenu est alors séché à l'évaporateur rotatif pour donner 8.7 g de [4-(morpholin-4-yl)-6-oxo-1,6-dihydropyrimidin-2-yl]acétate de sodium sous forme de solide blanc dont les caractéristiques sont les suivantes:
Spectre RMN 1 H (400 MHz) : 3,08 (s, 2 H); 3,38 (t, J=4,6 Hz, 4 H); 3,61 (t, J=4,6Hz, 4 H); 5,08 (s, 1 H); 13,16 (s large, 1 H)
Spectrométrie de Masse : méthode A
Temps de rétention Tr (min) = 0.29;
[M+H]+ : m/z 240 ; [M-H]- : m/z 238

### Etape (4'b)e:

### Préparation de la 2-{2-[(2S)-2-méthyl-2,3-dihydro-1H-indol-1-yl]-2-oxoéthyl}-6-(morpholin-4-yl)pyrimidin-4(3H)-one et de la 2-{2-[(2R)-2-méthyl-2,3-dihydro-1H-indol-1-yl]-2-oxoéthyl}-6-(morpholin-4-yl)pyrimidin-4(3H)-one

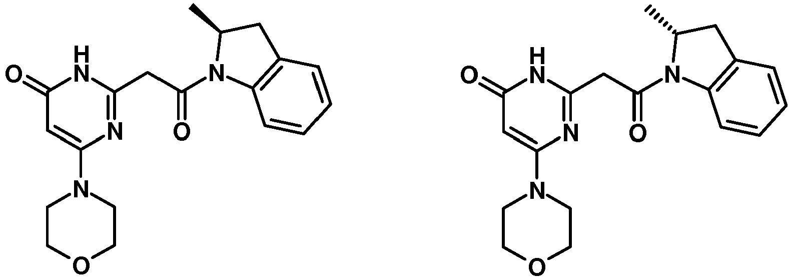

Le 2-[2-(2-méthyl-2,3-dihydro-1H-indol-1-yl)-2-oxoéthyl]-6-(morpholin-4-yl)pyrimidin-4(3H)-one est préparé en suivant le mode opératoire décrit à l'exemple 1e (étape 4e) à partir de 500 mg de [4-(morpholin-4-yl)-6-oxo-1,6-dihydropyrimidin-2-yl]acétate de sodium et de 510 mg de 2-méthyl-2,3-dihydro-1H-indole. On obtient 400 mg de 2-[2-(2-méthyl-2,3-dihydro-1H-indol-1-yl)-2-oxoéthyl]-6-(morpholin-4-yl)pyrimidin-4(3H)-one sous forme d'une poudre blanche dont les caractéristiques sont les suivantes :
Spectre RMN 1H (400MHz): 1,26 (d, J=6,1 Hz, 3 H) ; 2,65 à 2,72 (m, 1 H) ; 3,18 à 3,44 (m partiellement masqué, 5 H) ; 3,54 à 3,63 (m, 4 H) ; 3,72 (d, J=15,7 Hz, 1 H) ; 3,92 (d, J=15,7 Hz, 1 H) ; 4,71 (m, 1 H) ; 5,20 (s, 1 H) ; 7,04 (t, J=7,8 Hz, 1 H) ; 7,18 (t, J=7,8 Hz, 1 H) ; 7,29 (d, J=7,8 Hz, 1 H) ; 7,96 (d, J=7,8 Hz, 1 H) ; 11,69 (m étalé, 1 H)
Spectrométrie de Masse : méthode A
Temps de rétention Tr (min) = 0,70 ;
[M+H]+ : m/z 355 ; [M-H]- : m/z 353
Point de fusion (Kofler) : 172°C

Les énantiomères sont séparés par chromatographique chirale sur colonne : colonne chirale Chiralpak T304 20 µm (1080 g, 20 µm, 8/35 cm), éluant : Acétonitrile/Isopropanol : 90/10 ; débit :185 mL/min. Après purification, on obtient comme premier énantiomère, 160 mg de (R)-2-{2-[(2-méthyl-2,3-dihydro-1H-indol-1-yl]-2-oxoéthyl}-6-(morpholin-4-yl)pyrimidin-4(3H)-one, sous forme d'un solide amorphe rose dont les caractéristiques sont les suivantes:
Spectre RMN 1 H (400MHz): pour ce lot, les signaux sont larges avec : 1,26 (d, J=6,8 Hz, 3 H) ; 2,44 (m partiellement masqué, 1 H) ; 2,69 (d, J=15,2 Hz, 1 H) ; 3,42 (m, 4 H) ; 3,60 (m, 4H) ; 3,72 (d, J=15,7 Hz, 1 H) ; 3,92 (d, J=15,7 Hz, 1 H) ; 4,72 (m, 1 H) ; 5,20 (s, 1 H) ; 7,04 (t, J=7,8 Hz, 1 H) ; 7,18 (t, J=7,8 Hz, 1 H) ; 7,28 (d, J=7,8 Hz, 1 H) ; 7,96 (d, J=7,8 Hz, 1 H) ; 11,67 (m étalé, 1 H)
Spectrométrie de Masse : méthode A
Temps de rétention Tr (min) = 0,70 ;
[M+H]+ : m/z 355 ; [M-H]- : m/z 353;
Pouvoir rotatoire : α_{D} = +65,0° +/-1,3 (c=1,736 mg dans 0,5 mL de méthanol)

Puis le deuxième énantiomère, soit : 143 mg de (S)-2-{2-[(2-méthyl-2,3-dihydro-1H-indol-1-yl]-2-oxoéthyl}-6-(morpholin-4-yl)pyrimidin-4(3H)-one sous forme d'un solide amorphe blanc dont les caractéristiques sont les suivantes :
Spectre RMN 1 H (400MHz): pour ce lot, les signaux sont larges avec : 1,26 (d, J=6,8 Hz, 3 H) ; 2,45 (m partiellement masqué, 1 H) ; 2,69 (m, 1 H) ; 3,41 (m, 4 H) ; 3,61 (m, 4 H) ; 3,72 (d, J=15,7 Hz, 1 H) ; 3,92 (d, J=15,7 Hz, 1 H) ; 4,70 (m, 1 H) ; 5,20 (s, 1 H) ; 7,04 (t, J=7,8 Hz, 1 H) ; 7,18 (t, J=7,8 Hz, 1 H) ; 7,28 (d, J=7,8 Hz, 1 H) ; 7,96 (d, J=7,8 Hz, 1 H) ; 11,64 (m étalé, 1 H)
Spectrométrie de Masse : méthode A
Temps de rétention Tr (min) = 0,70 ;
[M+H]+ : m/z 355 ; [M-H]- : m/z 353;
Pouvoir rotatoire : α_{D} = -72,8° +/-1,2 (c=2,338 mg dans 0,5 mL de méthanol)

### Etape (4'c)e:

### Exemple 1e : 1-((S)-2-Methyl-2,3-dihydro-indol-1-yl)-2-(4-morpholin-4-yl-6-thioxo-1,6-dihydro-pyrimidin-2-yl)-ethanone

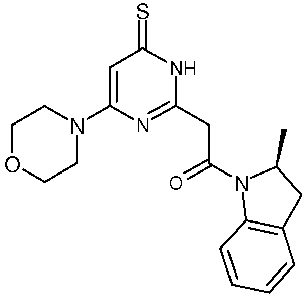

**et**

### Exemple 2e : Synthèse du 2-[2-((S)-2-Methyl-2,3-dihydro-indol-1-yl)-2-thioxo-ethyl]-6-morpholin-4-yl-3H-pyrimidine-4-thione

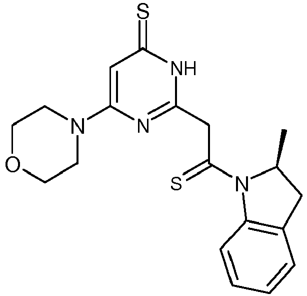

Le produit est préparé en suivant le mode opératoire décrit à l'exemple 1 e (Etape 2e) à partir de 354 mg du 2-{2-[(2S)-2-méthyl-2,3-dihydro-1H-indol-1-yl]-2-oxoéthyl}-6-(morpholin-4-yl)pyrimidin-4(3H)-one obtenu à l'exemple 1e (Etape (4'b)e) et de 0.8 g de 2,4-disulfure de 2,4-bis(4-méthoxyphényl)-1,3-dithia-2,4-diphosphétane (réactif de Lawesson). Après purification par chromatographie sur colonne de silice (éluant : dichlorométhane/méthanol 99/01 puis 98/02), on obtient 135 mg de 2-[2-((S)-2-methyl-2,3-dihydro-indol-1-yl)-2-thioxo-ethyl]-6-morpholin-4-yl-3H-pyrimidine-4-thione sous forme d'un solide jaune clair dont les caractéristiques sont les suivantes :
Spectre RMN 1 H (400 MHz) pour ce lot nous observons un dédoublement de conformères 2/3 - 1/3 avec : 1,25 (d, *J*=6,6 Hz, 2 H) ; 1,29 (d, *J*=6,6 Hz, 1 H) ; 2,65 (d, *J*=16,3 Hz, 0,65H) ; 2,73 (d, *J*=16,3 Hz, 0,35 H) ; 3,34 à 3,42 (m, 1 H) ; 3,45 à 3,66 (m, 8 H) ; 4,15 à 4,55 (m, 2 H) ; 5,18 (m, 0,35 H) ; 5,50 (m, 0,65 H) ; 6,33 (s, 0,65 H) ; 6,36 (s, 0,35 H) ; 7,15 à 7,45 (m, 3 H) ; 7,52 (d, *J*=8,0 Hz, 0,65 H) ; 9,14 (d, *J*=8,0 Hz, 0,35 H) ; 12,68 (s, 0,65 H) ; 12,79 (s, 0,35 H)
Spectrométrie de Masse : méthode A
Temps de rétention Tr (min) = 0,85 ;
[M+H]+ : m/z 387 ; [M-H]- : m/z 385
et 134 mg du 1-((S)-2-methyl-2,3-dihydro-indol-1-yl)-2-(4-morpholin-4-yl-6-thioxo-1,6-dihydro-pyrimidin-2-yl)-ethanone sous forme d'un solide jaune clair dont les caractéristiques sont les suivantes :
Spectre RMN 1H (400 MHz) pour ce lot tous les signaux sont larges avec : 1,26 (m, 3 H) ; 2,69 (d, *J*=15,2 Hz, 1 H) ; 3,38 (dd, *J*=9,4 et 15,2 Hz, 1 H) ; 3,50 à 3,66 (m, 8 H) ; 3,87 (d, *J*=15,4 Hz, 1 H) ; 4,06 (d, *J*=15,4 Hz, 1 H) ; 4,70 (m, 1 H) ; 6,37 (s, 1 H) ; 7,05 (t, *J*=8,0 Hz, 1 H) ; 7,18 (t, *J*=8,0 Hz, 1 H) ; 7,29 (d, *J*=8,0 Hz, 1 H) ; 7,95 (d, *J*=8,0 Hz, 1 H) ; 12,79 (m étalé, 1 H)
Spectrométrie de Masse : méthode A
Temps de rétention Tr (min) = 0,75 ;
[M+H]+ : m/z 371 ; [M-H]- : m/z 369

### Exemple 3e : Synthèse du 1-((R)-2-Methyl-2,3-dihydro-indol-1-yl)-2-(4-morpholin-4-yl-6-thioxo-1,6-dihydro-pyrimidin-2-yl)-ethanone

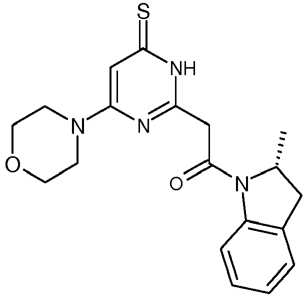

Le produit est préparé en suivant le mode opératoire décrit à l'exemple 1e (Etape 4e) à partir de 200 mg du (4-morpholin-4-yl-6-thioxo-1,6-dihydro-pyrimidin-2-yl)-acetate de sodium obtenu à l'exemple 1e (Etape 2e) et de 106 mg de (R)-2-methyl-2,3-dihydro-1H-indole (qui peut être préparée selon Krasnov, V.P. et Coll. (Mendeleev Commun. 2002, 12(1), 27-28). Après purification par chromatographie sur colonne de silice (éluant : dichlorométhane/dichlorométhane d'éthyle 98/02), on obtient 71 mg de 1-((R)-2-methyl-2,3-dihydro-indol-1-yl)-2-(4-morpholin-4-yl-6-thioxo-1,6-dihydro-pyrimidin-2-yl)-ethanone sous forme d'une poudre blanche dont les caractéristiques sont les suivantes :
Spectre RMN 1H (400 MHz) : 1,27 (d large, *J*=6,4 Hz, 3 H) ; 2,69 (d, *J*=16,3 Hz, 1 H) ; 3,33 à 3,42 (m partiellement masqué, 1 H) ; 3,53 à 3,65 (m, 8 H) ; 3,87 (d, *J*=15,7 Hz, 1 H) ; 4,06 (d, *J*=15,7 Hz, 1 H) ; 4,70 (m, 1 H) ; 6,37 (s, 1 H) ; 7,05 (t, *J*=7,8 Hz, 1 H) ; 7,18 (t, *J*=7,8 Hz, 1 H) ; 7,29 (d, *J*=7,8 Hz, 1 H) ; 7,95 (d large, *J*=7,8 Hz, 1 H) ; 12,79 (m étalé, 1 H)
Spectrométrie de Masse : méthode A
Temps de rétention Tr (min) = 0,75 ;
[M+H]+ : m/z 371 ; [M-H]- : m/z 369

### Exemple 4e: Synthèse du 1-((S)-2-Methyl-2,3-dihydro-indol-1-yl)-2-(1-methyl-4-morpholin-4-yl-6-thioxo-1,6-dihydro-pyrimidin-2-yl)-ethanone

### Etape 1e:

### (1-Methyl-4-morpholin-4-yl-6-oxo-1,6-dihydro-pyrimidin-2-yl)-acetic acid ethyl ester

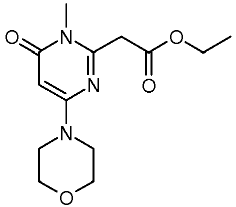

A une solution de 5 g de [4-(morpholin-4-yl)-6-oxo-1,6-dihydropyrimidin-2-yl]acétate d'éthyle (préparé à l'étape 1e de l'exemple 1e) dans 100 mL d'acétonitrile, 7,9 mg de carbonate de césium et 1,5 mL d'iodure de méthyle. Le mélange réactionnel est agité à la température ambiente pendant 24 heures. La suspension est filtrée sur verre fritté puis rincée avec de l'acétonitrile et le filtrat est concentré sous pression réduite. Le résidu est purifié par chromatographie sur colonne de silice, en éluant avec un mélange de dichlorométhane et de méthanol (98,5/1,5 V/V). On obtient 2 g d'un solide qui est de nouveau purifié sur colonne de silice, en éluant avec un mélange d'éther isopropylique et de méthanol (96/4 V/V) pour donner 1,2 g de [1-méthyl-4-(morpholin-4-yl)-6-oxo-1,6-dihydropyrimidin-2-yl]acétate d'éthyle sous forme de solide blanc dont les caractéristiques sont les suivantes :
Spectrométrie de Masse : méthode A
Temps de rétention Tr (min) = 0,53 ;
[M+H]+ : m/z 282 ; [M-H]- : m/z 280;

### Etape 2e:

### (1-Methyl-4-morpholin-4-yl-6-thioxo-1,6-dihydro-pyrimidin-2-yl)-acetic acid ethyl ester

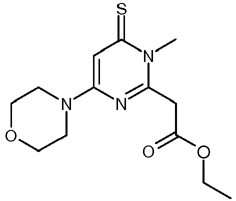

Dans un ballon, le mélange de 1.2 g du (1-methyl-4-morpholin-4-yl-6-oxo-1,6-dihydro-pyrimidin-2-yl)-acetic acid ethyl ester avec 2.6 g de 2,4-disulfure de 2,4-bis(4-méthoxyphényl)-1,3-dithia-2,4-diphosphétane (réactif de Lawesson) dans 100 mL de toluène sont chauffés à 100°C pendant 2 heures.

Après refroidissement, le solide formé est filtré. Le filtrat est concentré sous pression réduite. Le résidu obtenu est purifié par chromatographie sur colonne de silice (éluant : dichlorométhane/méthanol 99/01) pour donner 1.1 g du (1-methyl-4-morpholin-4-yl-6-thioxo-1,6-dihydro-pyrimidin-2-yl)-acetic acid ethyl ester dont les caractéristiques sont les suivantes :
Spectrométrie de Masse : méthode A
Temps de rétention Tr (min) = 0.88;
[M+H]+ : m/z 298 ; [M-H]- : m/z 296

### Etape 3e :

### (1-Methyl-4-morpholin-4-yl-6-thioxo-1,6-dihydro-pyrimidin-2-yl)-acetate de sodium

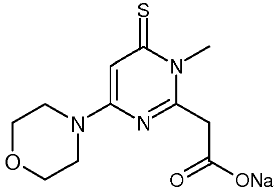

A une solution de 255 mg du (5-fluoro-4-morpholin-4-yl-6-thioxo-1,6-dihydropyrimidin-2-yl)-acetic acid ethyl ester dans 2 mL de THF est ajouté 0.85 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 0.32 g de (1-méthyl-4-morpholin-4-yl-6-thioxo-1,6-dihydro-pyrimidin-2-yl)-acetate de sodium qui est utilisé tel quel dans l'étape suivante.
Spectrométrie de Masse : méthode A
Temps de rétention Tr (min) = 0.58;
[M+H]+ : m/z 270 ; [M-H]- : m/z 268

### Etape 4e:

### Exemple 4e

### Synthèse du 1-((S)-2-Methy!-2,3-dihydro-indol-1-yl)-2-(1-methyl-4-morpholin-4-yl-6-thioxo-1,6-dihydro-pyrimidin-2-yl)-ethanone

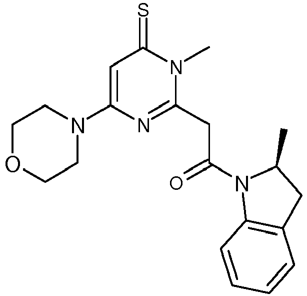

A une solution de 250 mg du (1-méthyl-4-morpholin-4-yl-6-thioxo-1,6-dihydro-pyrimidin-2-yl)-acetate de sodium dans 6 mL de DMF et 6 mL de pyridine sont ajoutés 512 mg de N-[3-(diméthylamino)propyl]-N'-éthylcarbodiimide chlorhydrate et 126 mg de (S)-2-methyl-2,3-dihydro-1H-indole (qui peut être préparée selon Krasnov, V.P. et Coll. (Mendeleev Commun. 2002, 12(1), 27-28).

Le milieu réactionnel est agité à la température ambiante pendant 18 heures.

Addition de 100 mL de dichlorométhane et 40 mL d'eau. Après décantation, la phase organique est séchée sur sulfate de magnésium, filtrée puis concentrée sous pression réduite. Le résidu obtenu est purifié chromatographie sur colonne de silice (éluant : éther isopropylique / méthanol : 80/20 en volumes) pour donner 75 mg du 1-((S)-2-Methyl-2,3-dihydro-indol-1-yl)-2-(1-methyl-4-morpholin-4-yl-6-thioxo-1,6-dihydropyrimidin-2-yl)-ethanone sous forme d'un solide blanc dont les caractéristiques sont les suivantes :
Spectre RMN 1H (400 MHz) : Spectre à 80 °C : 1,30 (d, J=6,4 Hz, 3 H) ; 2,70 (d, J=16,2 Hz, 1 H) ; 3,41 (dd, J=8,9 et 16,2 Hz, 1 H) ; 3,48 à 3,68 (m, 8 H) ; 3,87 (s, 3 H) ; 4,19 (d, J=16,8 Hz, 1 H) ; 4,43 (d, J=16,8 Hz, 1 H) ; 4,74 (m, 1 H) ; 6,68 (s, 1 H) ; 7,06 (t, J=7,7 Hz, 1 H) ; 7,19 (t, J=7,7 Hz, 1 H) ; 7,29 (d, J=7,7 Hz, 1 H) ; 7,90 (m étalé, 1 H)
Spectre de Masse (méthode A) :
   Temps de rétention Tr (min) = 1.14;
   [M+H]+ : m/z 385 ; [M-H]- : m/z 383

### Exemple 5e: 1-((R)-2-Methyl-2,3-dihydro-indol-1-yl)-2-(1-methyl-4-morpholin-4-yl-6-thioxo-1,6-dihydro-pyrimidin-2-yl)-ethanone

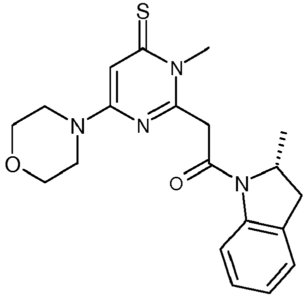

Le produit est préparé en suivant le mode opératoire décrit à l'exemple 4e (Etape 4e) à partir de 250 mg du (1-méthyl-4-morpholin-4-yl-6-thioxo-1,6-dihydropyrimidin-2-yl)-acetate de sodium dans 6 mL de DMF et 6 mL de pyridine sont ajoutés 512 mg de N-[3-(diméthylamino)propyl]-N'-éthylcarbodiimide chlorhydrate et 126 mg de (R)-2-methyl-2,3-dihydro-1H-indole (qui peut être préparée selon Krasnov, V.P. et Coll. (Mendeleev Commun. 2002, 12(1), 27-28).

Après purification par chromatographie sur colonne de silice (éluant : éther isopropylique / méthanol : 80/20 en volumes), on obtient 23 mg de 1-((R)-2-Methyl-2,3-dihydro-indol-1-yl)-2-(1-methyl-4-morpholin-4-yl-6-thioxo-1,6-dihydro-pyrimidin-2-yl)-ethanone sous forme d'un solide brun rouge dont les caractéristiques sont les suivantes :
Spectre RMN 1 H (400 MHz) : Spectre à 30°C : 1,28 (d, J=6,4 Hz, 3 H) ; 2,70 (d, J=16,1 Hz, 1 H) ; 3,40 (dd, J=8,9 et 16,1 Hz, 1 H) ; 3,45 à 3,66 (m, 8 H) ; 3,83 (s, 3 H) ; 4,18 (d, J=16,8 Hz, 1 H) ; 4,46 (d, J=16,8 Hz, 1 H) ; 4,71 (s, 1 H) ; 6,69 (s, 1 H) ; 7,06 (t, J=7,7 Hz, 1 H) ; 7,18 (t, J=7,7 Hz, 1 H) ; 7,29 (d, J=7,7 Hz, 1 H) ; 7,94 (d, J=7,7 Hz, 1 H)
Spectrométrie de Masse : méthode A
Temps de rétention Tr (min) = 1.16;
[M+H]+ : m/z 385 ; [M-H]- : m/z 383

### Exemple 6e: 1-(4-Fluoro-2,3-dihydro-indol-1-yl)-2-(1-methyl-4-morpholin-4-yl-6-thioxo-1,6-dihydro-pyrimidin-2-yl)-ethanone

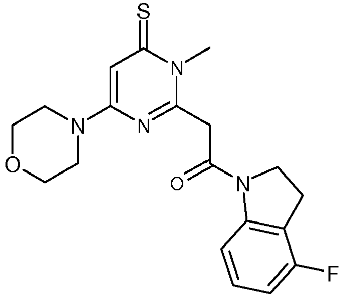

Le produit est préparé en suivant le mode opératoire décrit à l'exemple 4e (Etape 4e) à partir de 250 mg du (1-méthyl-4-morpholin-4-yl-6-thioxo-1,6-dihydropyrimidin-2-yl)-acetate de sodium dans 6 mL de DMF et 6 mL de pyridine sont ajoutés 512 mg de N-[3-(diméthylamino)propyl]-N'-éthylcarbodiimide chlorhydrate et 141 mg de 4-fluoro-2,3-dihydro-1H-indole.

Après purification, on obtient 65 mg de 1-(4-fluoro-2,3-dihydro-indol-1-yl)-2-(1-methyl-4-morpholin-4-yl-6-thioxo-1,6-dihydro-pyrimidin-2-yl)-ethanone sous forme d'un solide brun rouge dont les caractéristiques sont les suivantes :
Spectre RMN 1 H (400 MHz) : 3,20 (t, J=8,4 Hz, 2 H) ; 3,47 à 3,63 (m, 8 H) ; 3,81 (s, 3 H) ; 4,26 (t, J=8,4 Hz, 2 H) ; 4,28 (s, 2 H) ; 6,69 (s, 1 H) ; 6,88 (t, J=8,3 Hz, 1 H) ; 7,23 (m, 1 H) ; 7,84 (d, J=8,3 Hz, 1 H)
Spectrométrie de Masse : méthode A
Temps de rétention Tr (min) = 1.12;
[M+H]+ : m/z 389 ; [M-H]- : m/z 387

### Exemple 7e: 1-(4-Chloro-2,3-dihydro-indol-1-yl)-2-(1-methyl-4-morpholin-4-yl-6-thioxo-1,6-dihydro-pyrimidin-2-yl)-ethanone

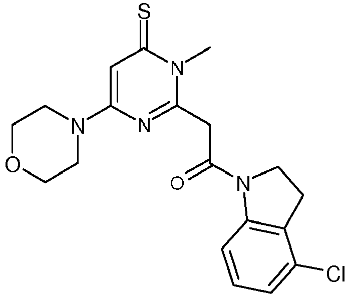

Le produit est préparé en suivant le mode opératoire décrit à l'exemple 4e (Etape 4e) à partir de 250 mg du (1-méthyl-4-morpholin-4-yl-6-thioxo-1,6-dihydropyrimidin-2-yl)-acetate de sodium dans 6 mL de DMF et 6 mL de pyridine sont ajoutés 512 mg de N-[3-(diméthylam ino)propyl]-N'-éthylcarbodiimide chlorhydrate et 158 mg de 4-chloro-2,3-dihydro-1H-indole.

Après purification, on obtient 145 mg de 1-(4-chloro-2,3-dihydro-indol-1-yl)-2-(1-methyl-4-morpholin-4-yl-6-thioxo-1,6-dihydro-pyrimidin-2-yl)-ethanone sous forme d'un solide brun rouge dont les caractéristiques sont les suivantes :
Spectre RMN 1 H (400 MHz) : 3,19 (t, J=8,4 Hz, 2 H) ; 3,46 à 3,66 (m, 8 H) ; 3,80 (s, 3 H) ; 4,24 (t, J=8,4 Hz, 2 H); 4,28 (s, 2 H) ; 6,70 (s, 1 H) ; 7,11 (d, J=7,8 Hz, 1 H) ; 7,23 (t, J=7,8 Hz, 1 H) ; 7,96 (d, J=7,8 Hz, 1 H)
Spectrométrie de Masse : méthode A
Temps de rétention Tr (min) = 1.24;
[M+H]+ : m/z 405 ; [M-H]- : m/z 403

### Exemple 8e : 1-((+)-4,5-Difluoro-2-methyl-2,3-dihydro-indol-1-yl)-2-(5-fluoro-4-morpholin-4-yl-6-thioxo-1,6-dihydro-pyrimidin-2-yl)-ethanone et

### Exemple 9e : 2-[2-((+)-4,5-Difluoro-2-methyl-2,3-dihydro-indol-1-yl)-2-thioxo-ethyl]-5-fluoro-6-morpholin-4-yl-3H-pyrimidine-4-thione

### Etape 1e :

### 4-Chloro-5-fluoro-6-méthoxy-2-méthyl-pyrimidine

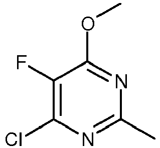

A une solution de 9.8 g du 2-méthyl-4,6-dichloro-5-fluoropyrimidine dans 80 mL de THF refroidit à 5°C dans un bain de glace sont ajoutés 3.21 g de méthylate de sodium. Le bain de glace est retiré. La suspension est agitée à la température ambiante pendant 3 heures. Le milieu réactionnel est refroidit à 5°C dans un bain de glace. Addition de 20 mL d'eau et 100 mL d'acétate d'éthyle. Après décantation, la phase organique est séchée sur sulfate de magnésium, filtrée, concentrée sous pression réduite pour donner 9 g de 4-chloro-5-fluoro-6-méthoxy-2-méthyl-pyrimidine sous forme d'une huile incolore qui cristallise dont les caractéristiques sont les suivantes :
Spectrométrie de Masse : méthode A
Temps de rétention Tr (min) = 0,82 ; [M+H]+ : m/z 177

### Etape 2e :

### (4-Chloro-5-fluoro-6-methoxy-pyrimidin-2-yl)-acetic acid methyl ester

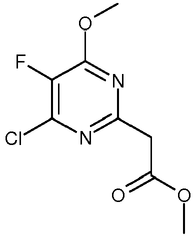

A une solution de 6.7 g du 4-chloro-5-fluoro-6-méthoxy-2-méthyl-pyrimidine et 4.83 mL de chloroformiate de méthyle dans 100 mL de THF anhydre refroidit à -60°C dans un bain de carboglace / MeOH, sont additionnés, goutte à goutte, 95 mL de LDA 2M (THF).

Le milieu réactionnel est agité à -60°C pendant une heure.

Le bain refroidissant est baissé pour laisser la température monter à 22°C. Le milieu réactionnel est agité à 22°C pendant deux heures.

Addition de 20 mL d'eau et 150 mL d'acétate d'éthyle. Après décantation, la phase organique est séchée sur sulfate de magnésium, filtrée puis concentrée sous pression réduite. Le résidu obtenu est purifié sur cartouche de silice : éluant : DCM pour donner 8.36 g de (4-chloro-5-fluoro-6-methoxy-pyrimidin-2-yl)-acetic acid methyl ester sous forme d'une huile jaune vif dont les caractéristiques sont les suivantes :
Spectrométrie de Masse : méthode A
Temps de rétention Tr (min) = 0,84 ; [M+H]+ : m/z 235 ;

### Etape 3e :

### (5-Fluoro-4-methoxy-6-morpholin-4-yl-pyrimidin-2-yl)-acetic acid methyl ester

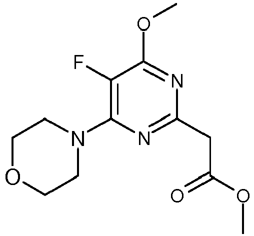

La solution de 8.36 g du (4-chloro-5-fluoro-6-methoxy-pyrimidin-2-yl)-acetic acid methyl ester dans 76 mL de morpholine est agitée à la température ambiante pendant une heure et demie. Le milieu réactionnel est concentré sous pression réduite. Le résidu obtenu est repris avec 50 mL d'eau et 200 mL d'acétate d'éthyle. Après décantation, la phase organique est séchée sur sulfate de magnésium, filtrée puis concentrée sous pression réduite pour donner 8.86 g de (5-fluoro-4-methoxy-6-morpholin-4-yl-pyrimidin-2-yl)-acetic acid methyl ester sous forme d'un solide beige dont les caractéristiques sont les suivantes :
Spectrométrie de Masse : méthode A
Temps de rétention Tr (min) = 0,84 ; [M+H]+ : m/z 286 ;

### Etape 4e:

### (5-Fluoro-4-morpholin-4-yl-6-oxo-1,6-dihydro-pyrimidin-2-yl)-acetic acid methyl ester

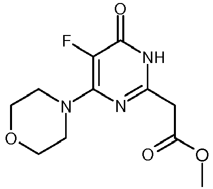

Dans un ballon, à une solution de 386 mg du (5-fluoro-4-methoxy-6-morpholin-4-yl-pyrimidin-2-yl)-acetic acid methyl ester obtenu à l'exemple 1e (Etape 3e) dans 4.7 mL d'acétonitrile, sont ajoutés 722 mg de Kl et de 0.56 mL de trimethylchlorosilane. La suspension est agitée à la température ambiante pendant 24 heures.

Le milieu réactionnel est concentré sous pression réduite.

Le résidu obtenu est repris avec de l'eau et de l'acétate d'éthyle. Après décantation, la phase organique est séchée sur sulfate de magnésium, filtrée puis concentrée sous pression réduite. Le résidu obtenu est purifié chromatographie sur colonne de silice (éluant : dichlorométhane/méthanol 98/02) pour donner 155 mg du (5-fluoro-4-morpholin-4-yl-6-oxo-1,6-dihydro-pyrimidin-2-yl)-acetic acid methyl ester sous forme d'un solide beige dont les caractéristiques sont les suivantes :
Spectrométrie de Masse : méthode A
Temps de rétention Tr (min) = 0,43 ; [M+H]+ : m/z 272 ;
[M-H]- : m/z 270 ; pic de base 238

### Etape 4'e:

Alternativement, le composé (5-Fluoro-4-morpholin-4-yl-6-oxo-1,6-dihydropyrimidin-2-yl)-acetic acid éthyl ester peut être obtenu en une étape :

### Etape (4'a)e:

### (5-Fluoro-4-morpholin-4-yl-6-oxo-1,6-dihydro-pyrimidin-2-yl)-acetic acid éthyl ester

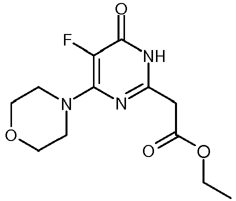

Dans un ballon, 5 g du [4-(morpholin-4-yl)-6-oxo-1,6-dihydropyrimidin-2-yl]acétate d'éthyle obtenu à l'Exemple 1e (Etape 1e) avec 50 mL d'acétonitrile sont chauffés à 74°C. Sur cette solution, et à 74°C, est ajouté, goutte à goutte, une solution de 7.67 g de Select fluor solubilisés dans un mélange de 25 mL d'eau et 25 mL d'acétonitrile.

Le milieu réactionnel est chauffé à 75°C pendant 90 minutes.

Après refroidissement, addition de 200 mL d'acétate d'éthyle puis 100 mL d'une solution saturée de bicarbonate de sodium. Après décantation, la phase organique est séchée sur sulfate de magnésium, filtrée puis concentrée sous pression réduite. Le résidu obtenu est purifié par chromatographie sur colonne de silice (éluant : gradient dichlorométhane/méthanol de 100/0 à 95/05) pour donner 0.8 g du (5-fluoro-4-morpholin-4-yl-6-oxo-1,6-dihydro-pyrimidin-2-yl)-acetic acid éthyl ester sous forme d'un solide beige dont les caractéristiques sont les suivantes :
Spectre RMN 1H (400 MHz, d en ppm, DMSO-d6) : 1,19 (t, *J*=7,1 Hz, 3 H) ; 3,56 (m, 6 H) ; 3,63 (m, 4 H) ; 4,12 (q, *J*=7,1 Hz, 2 H) ; 12,32 (m étalé, 1 H)

Etape 5e :

### (5-Fluoro-4-morpholin-4-yl-6-oxo-1,6-dihydro-pyrimidin-2-yl)-acetate de sodium

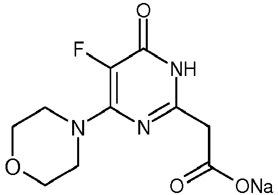

A une solution de 116 mg du (5-fluoro-4-morpholin-4-yl-6-oxo-1,6-dihydropyrimidin-2-yl)-acetic acid methyl ester dans 1.2 mL de THF est ajouté 0.43 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 110 mg de (5-fluoro-4-morpholin-4-yl-6-oxo-1,6-dihydro-pyrimidin-2-yl)-acetate de sodium dont les caractéristiques sont les suivantes :
Spectrométrie de Masse : méthode A
Temps de rétention Tr (min) = 0,32 ; [M+H]+ : m/z 258 ;
[M-H]- : m/z 256 ; pic de base : m/z 212

Etape 6e:

### 2-[2-((+)-4,5-difluoro-2-methyl-2,3-dihydro-indol-1-yl)-2-oxo-ethyl]-5-fluoro-6-morpholin-4-yl-3H-pyrimidin-4-one

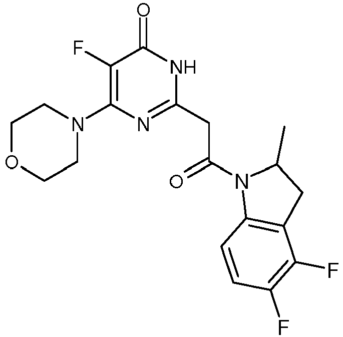

A une solution de 1.3 g du (5-fluoro-4-morpholin-4-yl-6-oxo-1,6-dihydro-pyrimidin-2-yl)-acetate de sodium dans 40 mL de DMF et 40 mL de pyridine sont ajoutés 1.46 g de N-[3-(diméthylamino)propyl]-N'-éthylcarbodiimide chlorhydrate et 1 g de (-)-2-methyl-4,5-difluoro-2,3-dihydro-1H-indole (exemple de référence 1e).

Le milieu réactionnel est agité à la température ambiante pendant 18 heures.

Addition de 200 mL de dichlorométhane et 100 mL d'eau. Après décantation, la phase organique est séchée sur sulfate de magnésium, filtrée puis concentrée sous pression réduite. Après purification sur colonne de silice: éluant dichlorométhane/méthanol 98/02, on obtient 814 mg du 2-[2-((+)-4,5-difluoro-2-methyl-2,3-dihydro-indol-1-yl)-2-oxo-ethyl]-5-fluoro-6-morpholin-4-yl-3H-pyrimidin-4-one sous forme d'un solide dont les caractéristiques sont les suivantes :
Spectre RMN 1 H (400 MHz) : 1,29 (d, *J*=6,4 Hz, 3 H) ; 2,82 (d, *J*=16,6 Hz, 1 H); 3,40 (dd, *J*=8,6 et 16,6 Hz, 1 H) ; 3,53 à 3,64 (m, 8 H) ; 3,73 (d, *J*=16,1 Hz, 1 H) ; 3,92 (d, *J*=16,1 Hz, 1 H); 4,79 (m, 1 H) ; 7,24 (m, 1 H) ; 7,74 (d, *J*=9,0 Hz, 1 H); 12,31 (m étalé, 1 H)
Spectrométrie de Masse : méthode A
Temps de rétention Tr (min) = 0,81 ; [M+H]+ : m/z 409 ; [M-H]- : m/z 407
Pouvoir rotatoire : α_{D} = +76.3+/-1.3. C=2.144mg/0.5ML DMSO

### Etape 7e:

### Exemple 8e : 1-((+)-4,5-Difluoro-2-methyl-2,3-dihydro-indol-1-yl)-2-(5-fluoro-4-morpholin-4-yl-6-thioxo-1,6-dihydro-pyrimidin-2-yl)-ethanone

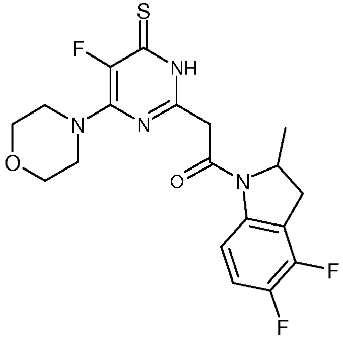

et

### Exemple 9e : 2-[2-((+)-4,5-Difluoro-2-methyl-2,3-dihydro-indol-1-yl)-2-thioxo-ethyl]-5-fluoro-6-morpholin-4-yl-3H-pyrimidine-4-thione

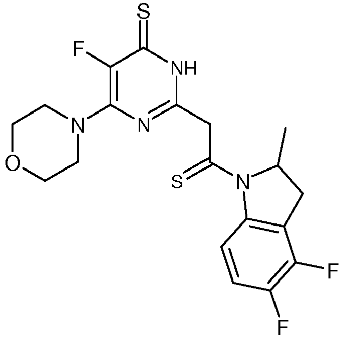

Dans un ballon, le mélange de 300 mg du 2-[2-((+)-4,5-difluoro-2-methyl-2,3-dihydro-indol-1-yl)-2-oxo-ethyl]-5-fluoro-6-morpholin-4-yl-3H-pyrimidin-4-one obtenu à l'étape précédente avec 445 mg de 2,4-disulfure de 2,4-bis(4-méthoxyphényl)-1,3-dithia-2,4-diphosphétane (réactif de Lawesson) dans 8 mL de toluène sont chauffés à 60°C pendant 18 heures.

Après refroidissement, le solide formé est filtré. Le filtrat est concentré sous pression réduite. Le résidu obtenu est purifié par chromatographie sur colonne de silice (éluant : dichlorométhane/MeOH/NH4OH 28% 98.33/1.5/0.17) pour donner 17 mg du 1-((+)-4,5-Difluoro-2-methyl-2,3-dihydro-indol-1-yl)-2-(5-fluoro-4-morpholin-4-yl-6-thioxo-1,6-dihydro-pyrimidin-2-yl)-ethanone sous forme d'un solide jaune dont les caractéristiques sont les suivantes :
Spectre RMN 1 H (400MHz) : 1,30 (d, *J*=6,1 Hz, 3 H); 2,82 (d, *J*=16,4 Hz, 1 H); 3,41 (dd, *J*=8,4 et 16,0 Hz, 1 H); 3,67 (m, 8 H); 3,86 (m, 1 H); 4,05 (d, *J=*15,9 Hz, 1 H); 4,78 (m, 1 H); 7,22 (m,1 H); 7,73 (d, *J*=5,6 Hz, 1 H); 13,24 (s large, 1 H)
Temps de rétention Tr (min) = 0.87;
[M+H]+ : m/z 425 ; [M-H]- : m/z 423
et 23 mg du 2-[2-((+)-4,5-Difluoro-2-methyl-2,3-dihydro-indol-1-yl)-2-thioxo-ethyl]-5-fluoro-6-morpholin-4-yl-3H-pyrimidine-4-thione sous forme d'un solide jaune clair dont les caractéristiques sont les suivantes :
Spectre RMN 1 H (400MHz) : 1,29 (d, *J*=6,4 Hz, 1,5 H); 1,33 (d, *J*=6,6 Hz, 1,5 H); 2,83 (m, 1 H); 3,39 (m, 1 H); 3,61 (m, 8 H); 4,11 à 4,58 (m, 2 H); 5,25 (m, 0,5 H); 5,55 (m, 0,5 H); 7,33 (m, 1,5H); 8,96 (m, 0,5 H); 13,16 (s large, 0,5 H); 13,27 (s large, 0,5 H)
Temps de rétention Tr (min) = 0.96;
[M+H]+ : m/z 441 ; [M-H]- : m/z 439

### Exemple 10e : Synthèse du 2-(5-Fluoro-4-morpholin-4-yl-6-thioxo-1,6-dihydro-pyrimidin-2-yl)-1-((S)-2-methyl-2,3-dihydro-indol-1-yl)-ethanone

### Etape 1e:

### (5-Fluoro-4-morpholin-4-yl-6-thioxo-1,6-dihydro-pyrimidin-2-yl)-acetic acid ethyl ester

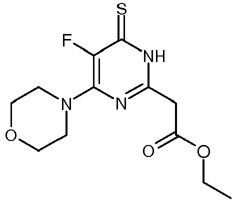

Dans un ballon, le mélange de 0.53 g du [4-(morpholin-4-yl)-6-oxo-1,6-dihydropyrimidin-2-yl]acétate d'éthyle obtenu aux Exemples 8e et 9e (Etape 4e) avec 1.12 g de 2,4-disulfure de 2,4-bis(4-méthoxyphényl)-1,3-dithia-2,4-diphosphétane (réactif de Lawesson) dans 20 mL de toluène sont chauffés à 60°C pendant 2 heures puis à reflux pendant 30 minutes.

Après refroidissement, le solide formé est filtré. Le filtrat est concentré sous pression réduite. Le résidu obtenu est purifié par chromatographie sur colonne de silice (éluant : dichlorométhane/acétate d'éthyle 85/15) pour donner 0.175 g du (5-fluoro-4-morpholin-4-yl-6-thioxo-1,6-dihydro-pyrimidin-2-yl)-acetic acid ethyl ester dont les caractéristiques sont les suivantes :
Spectrométrie de Masse : méthode A
Temps de rétention Tr (min) = 0,79;
[M+H]+ : m/z 302 ; [M-H]- : m/z 300

Et 0.33 g du (4-morpholin-4-yl-6-thioxo-1,6-dihydro-pyrimidin-2-yl)-acetic acid ethyl ester sous forme d'un solide beige dont les caractéristiques sont les suivantes:
Spectrométrie de Masse : méthode A
Temps de rétention Tr (min) = 0,74 ;
[M+H]+ : m/z 284 ; [M-H]- : m/z 282 ; pic de base : m/z 236

### Etape 2e :

### (5-fluoro-4-morpholin-4-yl-6-thioxo-1,6-dihydro-pyrimidin-2-yl)-acetate de sodium

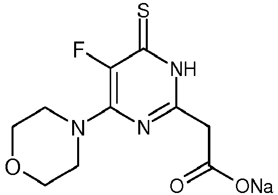

A une solution de 255 mg du (5-fluoro-4-morpholin-4-yl-6-thioxo-1,6-dihydro-pyrimidin-2-yl)-acetic acid ethyl ester dans 2 mL de THF est ajouté 0.85 mL de soude 2N. Le milieu réactionnel est agité à la température ambiante pendant 24 heures. Le milieu réactionnel est concentré sous pression réduite. Le résidu obtenu est séché à l'étuve sous vide en présence de P2O5 pour donner 0.32 g de (5-fluoro-4-morpholin-4-yl-6-thioxo-1,6-dihydro-pyrimidin-2-yl)-acetate de sodium qui est utilisé tel quel dans l'étape suivante.

### Etape 3e:

### Exemple 10e

### Synthèse du 2-(5-Fluoro-4-morpholin-4-yl-6-thioxo-1,6-dihydro-pyrimidin-2-yl)-1-((S)-2-methyl-2,3-dihydro-indol-1-yl)-ethanone

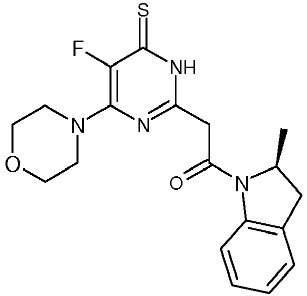

A une solution de 159 mg du (5-fluoro-4-morpholin-4-yl-6-thioxo-1,6-dihydro-pyrimidin-2-yl)-acetate de sodium dans 3.7 mL de DMF et 3.7 mL de pyridine sont ajoutés 324 mg de N-[3-(diméthylamino)propyl]- N'-éthylcarbodiimide chlorhydrate et 79 mg de (S)-2-methyl-2,3-dihydro-1H-indole (qui peut être préparée selon Krasnov, V.P. et Coll. (Mendeleev Commun. 2002, 12(1), 27-28).

Le milieu réactionnel est agité à la température ambiante pendant 18 heures.

Addition de 50 mL de dichlorométhane et 20 mL d'eau. Après décantation, la phase organique est séchée sur sulfate de magnésium, filtrée puis concentrée sous pression réduite. Le résidu obtenu est purifié chromatographie sur colonne de silice (éluant: gradient dichlorométhane / méthanol / ammoniac 28% de 98,33/1,5/0,17 à 95/4,5/0,5 en volumes) pour donner 17 mg du 2-(5-Fluoro-4-morpholin-4-yl-6-thioxo-1,6-dihydro-pyrimidin-2-yl)-1-((S)-2-methyl-2,3-dihydro-indol-1-yl)-ethanone sous forme d'un solide beige dont les caractéristiques sont les suivantes :
Spectre RMN 1H (400 MHz) : 1,27 (d large, *J*=6,3 Hz, 3 H) ; 2,69 (d, *J*=16,9 Hz, 1 H) ; 3,38 (dd, *J*=8,8 et 16,9 Hz, 1 H); 3,62 à 3,75 (m, 8 H) ; 3,88 (d, *J*=15,9 Hz, 1 H); 4,06 (d, *J*=15,9 Hz,1 H) ; 4,68 (m, 1 H); 7,05 (t, *J*=7,6 Hz, 1 H) ; 7,18 (t, *J*=7,6 Hz, 1 H); 7,29 (d, *J*=7,6 Hz, 1 H) ; 7,95 (d, *J*=7,6 Hz, 1 H) ; 13,26 (s large, 1 H)
Spectre de Masse (méthode A) :
   Temps de rétention Tr (min) = 1,05;
   [M+H]+ : m/z 389 ; [M-H]- : m/z 387

### Exemple 11e: Synthèse du 2-(5-Fluoro-4-morpholin-4-yl-6-thioxo-1,6-dihydro-pyrimidin-2-yl)-1-((R)-2-methyl-2,3-dihydro-indol-1-yl)-ethanone

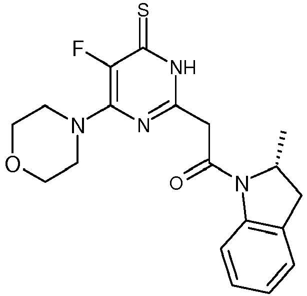

A une solution de 159 mg du (5-fluoro-4-morpholin-4-yl-6-thioxo-1,6-dihydro-pyrimidin-2-yl)-acetate de sodium dans 3.7 mL de DMF et 3.7 mL de pyridine sont ajoutés 324 mg de N-[3-(diméthylamino)propyl]-N'-éthylcarbodiimide chlorhydrate et 79 mg de (R)-2-methyl-2,3-dihydro-1H-indole (qui peut être préparée selon Krasnov, V.P. et Coll. (Mendeleev Commun. 2002, 12(1), 27-28).

Le milieu réactionnel est agité à la température ambiante pendant 18 heures.

Addition de 50 mL de dichlorométhane et 20 mL d'eau. Après décantation, la phase organique est séchée sur sulfate de magnésium, filtrée puis concentrée sous pression réduite. Le résidu obtenu est purifié chromatographie sur colonne de silice (éluant: gradient dichlorométhane / méthanol / ammoniac 28% de 98,33/1,5/0,17 à 95/4,5/0,5 en volumes) pour donner 45 mg du 2-(5-Fluoro-4-morpholin-4-yl-6-thioxo-1,6-dihydro-pyrimidin-2-yl)-1-((R)-2-methyl-2,3-dihydro-indol-1-yl)-ethanone sous forme d'un solide beige dont les caractéristiques sont les suivantes :
Spectre RMN 1H (400 MHz) : 1,27 (d large, *J*=6,3 Hz, 3 H) ; 2,69 (d, *J*=15,9 Hz, 1 H) ; 3,38 (dd, *J*=8,9 et 15,9 Hz, 1 H) ; 3,60 à 3,74 (m, 8 H) ; 3,88 (d, *J*=15,9 Hz, 1 H) ; 4,06 (d, *J*=15,9 Hz, 1 H) ; 4,68 (t, *J*=7,7 Hz, 1 H) ; 7,05 (t, *J*=7,6 Hz, 1 H) ; 7,18 (t, *J*=7,6 Hz, 1 H) ; 7,29 (d, *J*=7,6 Hz, 1 H) ; 7,95 (d, *J*=7,6 Hz, 1 H) ; 13,26 (s large, 1 H)
Spectre de Masse (méthode A) :
   Temps de rétention Tr (min) = 1,05;
   [M+H]+ : m/z 389 ; [M-H]- : m/z 387

### Exemples de référence pour la préparation des composés de formule (le)

### Exemple de référence 1e : 4,5-difluoro-2-méthyl-2,3-dihydro-1H-indole

### Etape 1e :

### 4,5-Difluoro-2-methyl-2,3-dihydro-1 H-indole

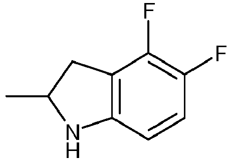

A une solution de 4.5 g du 4,5-diluoro-2-methyl-indole dans 180 mL d'acide acétique refroidit à 15°C, sont ajoutés en 3 fois 5.07 g de cyanoborohydrure de sodium. Le milieu réactionnel est agité à 15°C pendant 30 minutes puis 4 heures à la température ambiante.

Le milieu réactionnel est refroidit de nouveau à 5°C. Addition de l'eau glacée. Addition de l'ammoniaque 30% jusqu'au pH = 9. Extraction 3 fois au dichlorométhane. Les phases organique sont réunies, séchées sur sulfate de magnésium, filtrées puis concentrée sous pression réduite. Le résidu obtenu est purifié sur colonne de silice, éluant : cyclohexane/acétate d'éthyle 90/10 pour donner 4.4 g de 4,5-difluoro-2-methyl-2,3-dihydro-1H-indole dont les caractéristiques sont les suivantes :
Spectrométrie de Masse : méthode A
Temps de rétention Tr (min) = 0,60 ; [M+H]+ : m/z 170

### Etape 2e :

### (R)-1-(4,5-Difluoro-2-methyl-2,3-dihydro-indol-1-yl)-2-phenoxy-propan-1-one : diastéréoisomère A

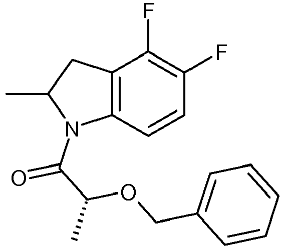

### Et

### (R)-1-(4,5-Difluoro-2-methyl-2,3-dihydro-indol-1-yl)-2-phenoxy-propan-1-one: diastéréoisomère B

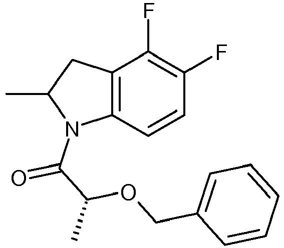

Les produits sont préparés en suivant le mode opératoire décrit à l'exemple de référence 1e (Etape 2e) à partir de 4.4 g du (4,5-difluoro-2-methyl-2,3-dihydro-1H-indole et de 5.62 g d'acide o-benzyl-D-lactique. Après purification sur colonne de silice, éluant : cyclohexane/acétate d'éthyle 90/10 puis 80/20, on obtient 4.2 g de (R)-1-(4,5-difluoro-2-methyl-2,3-dihydro-indol-1-yl)-2-phenoxy-propan-1-one : diastéréoisomère A sous forme d'une huile jaune dont les caractéristiques sont les suivantes :
Spectrométrie de Masse : méthode A
Temps de rétention Tr (min) = 1,11 ; [M+H]+ : m/z 332 ; pic de base : m/z 260
Pouvoir rotatoire : α_{D} = +41.6+/-0.9. C= 2.266mg/0.5ML DMSO

Et 4.1 g de (R)-1-(4,5-difluoro-2-methyl-2,3-dihydro-indol-1-yl)-2-phenoxy-propan-1-one : diastéréoisomère B sous forme d'un solide blanc dont les caractéristiques sont les suivantes :
Spectrométrie de Masse : méthode A
Temps de rétention Tr (min) = 1,10 ; [M+H]+ : m/z 332 ; pic de base: m/z 260
Pouvoir rotatoire : α_{D} = +120.1+/-1.8. C= 2.252mg/0.5ML DMSO

### Etape 3e :

### (+)-4,5-Difluoro-2-methyl-2,3-dihydro-1H-indole

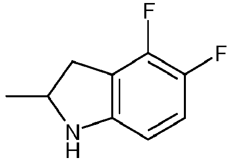

Le produit est préparé en suivant le mode opératoire décrit à l'exemple de référence 1e (Etape 3e) à partir de 4.2 g du (R)-1-(4,5-difluoro-2-methyl-2,3-dihydro-indol-1-yl)-2-phenoxy-propan-1-one : diastéréoisomère A et de 40 mL d'acide chlorhydrique 37%.

Après purification sur colonne de silice, éluant : cyclohexane/acétate d'éthyle 90/10°, on obtient 1.6 g de (+)-4,5-difluoro-2-methyl-2,3-dihydro-1 H-indole dont les caractéristiques sont les suivantes :
Spectrométrie de Masse : méthode A
Temps de rétention Tr (min) = 0,58 ; [M+H]+ : m/z 170 ;

### Etape 3e :

### (-)-4,5-Difluoro-2-methyl-2,3-dihydro-1H-indole

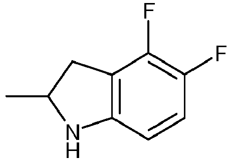

Le produit est préparé en suivant le mode opératoire décrit à l'exemple de référence1e (Etape 3e) à partir de 4.1 g du (R)-1-(4,5-difluoro-2-methyl-2,3-dihydro-indol-1-yl)-2-phenoxy-propan-1-one : diastéréoisomère B et de 41 mL d'acide chlorhydrique 37%.

Après purification sur colonne de silice, éluant : heptane/acétate d'éthyle 90/10 , on obtient 1.5 g de (-)-4,5-difluoro-2-methyl-2,3-dihydro-1H-indole dont les caractéristiques sont les suivantes :
Spectrométrie de Masse : méthode A
Temps de rétention Tr (min) = 0,60 ; [M+H]+ : m/z 170 ;
**Exemples de composition pharmaceutiques comprenant des composés**

### selon l'invention:

### Exemple a:

On a préparé des comprimés répondant à la formule suivante :

| | |
|---|---|
| Produit de l'exemple 1a... | 0,2 g |
| Excipient pour un comprimé terminé à .... | 1 g |

(détail de l'excipient : lactose, talc, amidon, stéarate de magnésium).

### Exemple b:

On a préparé des comprimés répondant à la formule suivante :

| | |
|---|---|
| Produit de l'exemple 10b... | 0,2 g |
| Excipient pour un comprimé terminé à ... | 1 g |

(détail de l'excipient : lactose, talc, amidon, stéarate de magnésium).

### Exemple c:

On a préparé des comprimés répondant à la formule suivante :

| | |
|---|---|
| Produit de l'exemple 1c... | 0,2 g |
| Excipient pour un comprimé terminé à ... | 1 g |

(détail de l'excipient : lactose, talc, amidon, stéarate de magnésium).

### Exemple d:

On a préparé des comprimés répondant à la formule suivante :

| | |
|---|---|
| Produit de l'exemple 1d... | 0,2 g |
| Excipient pour un comprimé terminé à ... | 1 g |

(détail de l'excipient : lactose, talc, amidon, stéarate de magnésium).

### Exemple e:

On a préparé des comprimés répondant à la formule suivante :

| | |
|---|---|
| Produit de l'exemple 1e... | 0,2 g |
| Excipient pour un comprimé terminé à ... | 1 g |

(détail de l'excipient : lactose, talc, amidon, stéarate de magnésium).

Les exemples cités sont pris à titre d'exemple de préparation pharmaceutique, cette préparation pouvant être réalisée si désiré avec d'autres produits en exemples dans la présente demande.

### Données expérimentales:

### Partie pharmacologique :

### Protocoles expérimentaux

### Procédures expérimentales in vitro

L'activité inhibitrice des molécules sur la phosphorylation d'AKT est mesurée par la technique MSD Multi-spot Biomarker détection de Meso Scale Discovery également décrite ci-dessous.

### Etude de l'expression de pAKT dans les cellules humaines PC3 de carcinome de prostate mesurée par la technique MSD Multi-spot Biomarker Détection de Meso Scale Discovery (Test A):

Cet essai est basé sur la mesure de l'expression de la protéine AKT phosphorylée sur la sérine 473 (P-AKT-S473), dans la lignée de carcinome de prostate humaine PC3, par la technique basée sur un immuno-essai sandwich utilisant le kit MSD Multi-spot Biomarker Détection de Meso Scale Discovery : kits phospho-Akt (Ser473) whole cell lysate (#K151CAD) ou phospho-Akt (Ser473)/Total Akt whole cell lysate (#K151OOD). L'anticorps primaire spécifique de P-AKT-S473 (Kit #K151CAD) est coaté sur une électrode dans chaque puits des plaques 96 puits du kit MSD : après ajoût d'un lysat de protéines dans chaque puits, la révélation du signal se fait par l'addition d'un anticorps secondaire de détection marqué avec un composé électrochimioluminescent. La procédure suivie est celle décrite dans le kit.

Le jour 1, les cellules PC3 sont ensemencées en plaques 96 puits (TPP, #92096) à la concentration de 35000 cellules/puits dans 200 µl de milieu DMEM (DMEM Gibco #11960-044) contenant 10% de sérum de veau foetal (SVF Gibco, #10500-056) et 1% Glutamine (L-Glu Gibco #25030-024), et incubées à 37°C, 5% CO2, pendant une nuit.

Le jour 2, les cellules sont incubées en présence ou pas des produits à tester pendant 1 à 2h à 37°C en présence de 5% CO2. Les molécules diluées dans du diméthylsulfoxyde (DMSO Sigma #D2650), sont ajoutées à partir d'une solution mère concentrée 20 fois, le pourcentage final de DMSO étant de 0.1%. Les molécules sont testées soit à une seule concentration inférieure ou égale à 10uM, soit à des concentrations croissantes dans une gamme pouvant s'étendre de moins de 1 nM à 10µM.

Après cette incubation, les cellules sont lysées pour la préparation des protéines. Pour cela, après aspiration du milieu de culture, 50µl de tampon de lyse Tris Lysis Buffer complet du kit MSD contenant les solutions d'inhibiteurs de protéases et phosphatases sont ajoutés dans les puits et les cellules sont lysées pendant 1 H à 4°C sous agitation. A ce stade les plaques contenant les lysats peuvent être congelées à -20°C ou à -80°C.

Les puits des plaques 96 puits du kit MSD sont saturés pendant 1h à température ambiante avec la solution bloquante du kit MSD. Quatre lavages sont effectués avec 150µl de solution de lavage Tris Wash Buffer du kit MSD. Les lysats préparés précédemment sont transférés dans les plaques Multi-spot 96 puits du kit MSD et incubés pendant 1h à température ambiante, sous agitation. Quatre lavages sont effectués avec 150µl de solution de lavage Tris Wash Buffer du kit MSD. 25µl de la solution MSD sulfo-tag détection antibody sont ajoutés dans les puits et incubés pendant 1h à température ambiante, sous agitation. Quatre lavages sont effectués avec 150µl de solution de lavage Tris Wash Buffer du kit MSD. 150µl de tampon de révélation Read Buffer du kit MSD sont ajoutés dans les puits et les plaques sont lues immédiatement sur l'instrument S12400 de Meso Scale Discovery.

L'appareil mesure un signal pour chaque puits. Des puits sans cellules et contenant le tampon de lyse servent à déterminer le bruit de fond qui sera soustrait à toutes les mesures (min). Les puits contenant des cellules en absence de produit et en présence de 0.1% DMSO sont considérés comme le 100 % de signal (max). Le calcul du pourcentage d'inhibition est fait pour chaque concentration de produit testé selon la formule suivante : (1- ((essai-min)/(max-min)))x100.

L'activité du produit est traduite en Cl₅₀, obtenue à partir d'une courbe dose-réponse de différentes concentrations testées et représentant la dose donnant 50 % d'inhibition spécifique (Cl₅₀ absolue). 2 expériences indépendantes permettent de calculer la moyenne des Cl₅₀ₛ.

Les résultats obtenus pour les produits en exemples dans la partie expérimentale sont donnés dans les tableaux de résultats pharmacologiques ci-après:

### Tableaux de résultats pharmacologiques :

### Exemples relatifs aux composés de formule (la):

| Exemple | Test A IC₅₀ (nM) |
|---|---|
| Exemple 1a | **22** |
| Exemple 2a | **938** |
| Exemple 3a | **158** |
| Exemple 4a | **503** |
| Exemple 5a | **15** |
| Exemple 6a | **10** |
| Exemple 7a | **3** |
| Exemple 8a | **107** |
| Exemple 9a | **120** |
| Exemple 10a | **116** |
| Exemple 11a | **24** |
| Exemple 12a | **16** |
| Exemple 13a | **162** |
| Exemple 14a | **55** |
| Exemple 15a | **65** |
| Exemple 16a | **483** |
| Exemple 17a | **45** |
| Exemple 18a | **38** |
| Exemple 19a | **19** |
| Exemple 20a | **2** |
| Exemple 21 a | **15** |
| Exemple 22a | **98** |
| Exemple 23a | **203** |
| Exemple 24a | **310** |
| Exemple 25a | **98** |
| Exemple 26a | **211** |
| Exemple 27a | **270** |
| Exemple 28a | **476** |
| Exemple 29a | **20** |
| Exemple 30a | **16** |
| Exemple 31 a | **15** |
| Exemple 32a | **40** |
| Exemple 33a | **77** |
| Exemple 34a | **2** |
| Exemple 35a | **140** |
| Exemple 36a | **55** |
| Exemple 37a | **56** |
| Exemple 38a | **1138** |
| Exemple 39a | **568** |

### Exemples relatifs aux composés de formule (Ib):

| Exemple | Test A IC50 (nM) |
|---|---|
| Exemple 1b | **26** |
| Exemple 2b | **6** |
| Exemple 3b | **271** |
| Exemple 4b | **118** |
| Exemple 5b | **127** |
| Exemple 6b | **58** |
| Exemple 7b | **21** |
| Exemple 8b | **18** |
| Exemple 9b | **156** |
| Exemple 10b | **8** |
| Exemple 11b | **12** |
| Exemple 12b | **370** |
| Exemple 13b | **3000** |
| Exemple 14b | **280** |
| Exemple 15b | **105** |
| Exemple 16b | **18** |
| Exemple 17b | **12** |
| Exemple 18b | **91** |
| Exemple 19b | **16** |
| Exemple 20b | **5** |
| Exemple 21b | **27** |
| Exemple 22b | **9** |
| Exemple 23b | **244** |
| Exemple 24b | **2855** |
| Exemple 25b | **27** |
| Exemple 26b | **240** |
| Exemple 27b | **1332** |
| Exemple 28b | **12** |
| Exemple 29b | **24** |
| Exemple 30b | **44** |
| Exemple 31b | **156** |

### Exemples relatifs aux composés de formule (Ic):

| Exemple | Test A IC50 (nM) |
|---|---|
| Exemple 1c | **5** |
| Exemple 2c | **5** |
| Exemple 3c | **1** |
| Exemple 4-c | **35** |
| Exemple 5c | **14** |
| Exemple 6c | **24** |
| Exemple 7c | **45** |
| Exemple 8c | **52** |
| Exemple 9c | **115** |
| Exemple 10c | **22** |
| Exemple 11c | |
| Exemple 12c | **57** |
| Exemple 13c | **5** |
| Exemple 14c | **2** |
| Exemple 15c | **28** |
| Exemple 16c | **13** |
| Exemple 17c | **4** |
| Exemple 18c | **1** |
| Exemple 19c | **19** |
| Exemple 20c | **13** |
| Exemple 21c | **17** |
| Exemple 22c | **2** |
| Exemple 23c | **32** |
| Exemple 24c | **114** |
| Exemple 25c | **39** |
| Exemple 26c | **3** |
| Exemple 27c | **4** |
| Exemple 28c | **98** |
| Exemple 29c | **53** |
| Exemple 30c | **73** |
| Exemple 31c | **36** |
| Exemple 32c | **14** |
| Exemple 33c | **55** |
| Exemple 34c | **242** |
| Exemple 35c | **27** |
| Exemple 36c | **9** |
| Exemple 37c | **7** |
| Exemple 38c | **1** |
| Exemple 39c | **4** |
| Exemple 40c | **243** |
| Exemple 41c | **29** |
| Exemple 42c | **380** |
| Exemple 43c | **14** |
| Exemple 44c | **140** |
| Exemple 45c | **260** |
| Exemple 46c | **101** |

### Exemples relatifs aux coposés de formule (Id):

| Exemple | Test A IC50 (nM) |
|---|---|
| Exemple 1d | **26** |
| Exemple 2d | **24** |
| Exemple 3d | **54** |
| Exemple 4d | **63** |
| Exemple 5d | **9** |
| Exemple 6d | **32** |
| Exemple 7d | **102** |
| Exemple 8d | **60** |
| Exemple 9d | **44** |
| Exemple 10d | **94** |
| Exemple 11d | **26** |
| Exemple 12d | **66** |
| Exemple 13d | **53** |
| Exemple 14d | **42** |
| Exemple 15d | **37** |
| Exemple 16d | **30** |
| Exemple 17d | **259** |
| Exemple 18d | **272** |
| Exemple 19d | **23** |
| Exemple 20d | **520** |
| Exemple 21d | **155** |
| Exemple 22d | **106** |
| Exemple 23d | **196** |
| Exemple 24d | **205** |
| Exemple 25d | **5** |
| Exemple 26d | **167** |
| Exemple 27 | **435** |
| Exemple 28d | **2** |
| Exemple 29d | **33** |
| Exemple 30d | **8** |
| Exemple 31d | **167** |
| Exemple 32d | **60** |
| Exemple 33d | **111** |
| Exemple 34d | **48** |
| Exemple 35d | **5** |
| Exemple 36d | **5** |
| Exemple 37d | **2** |
| Exemple 38d | **15** |
| Exemple 39d | **51** |
| Exemple 40d | **13** |
| Exemple 41d | **131** |
| Exemple 42d | **24** |

### Exemples relatifs aux composés de formule (Ie):

| Exemple | Test A IC50 (nM) |
|---|---|
| Exemple 1e | **22** |
| Exemple 2e | **4** |
| Exemple 3e | **8** |
| Exemple 4e | **41** |
| Exemple 5e | **71** |
| Exemple 6e | **31** |
| Exemple 7e | **33** |
| Exemple 8e | **11** |
| Exemple 9e | **1** |
| Exemple 10e | **221** |
| Exemple 11e | **61** |

## Revendications

1. Composés de formule (la): 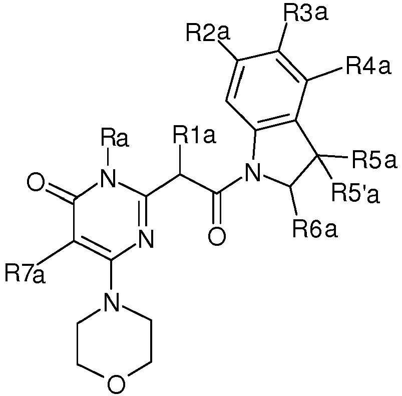 dans laquelle :
Ra représente un atome d'hydrogène ou un radical alkyle;
R1 a représente un atome d'hydrogène ou un radical méthyle;
R2a représente un atome d'hydrogène ou un atome de fluor ;
R3a représente un atome d'hydrogène ou un atome d'halogène ;
R4a représente un atome d'hydrogène, un atome d'halogène ou un radical hydroxyle, alkyle ou alcoxy, les radicaux alkyle étant éventuellement substitués par un ou plusieurs radicaux identiques ou différents choisis parmi les atomes d'halogène et le radical hydroxyle, les radicaux alcoxy étant éventuellement substitués par un ou plusieurs atomes d'halogène ; R5a et R5'a, identiques ou différents, représente un atome d'hydrogène ou un radical alkyle ;
R6a représente un atome d'hydrogène ou un radical alkyle éventuellement substitué par un ou plusieurs radicaux identiques ou différents choisis parmi les atomes d'halogène et le radical hydroxyle ;
R7a représente un atome d'halogène ;
lesdits composés de formule (la) étant sous toutes les formes isomères possibles racémiques, énantiomères et diastéréoisomères, ainsi que les sels d'addition avec les acides minéraux et organiques ou avec les bases minérales et organiques desdits composés de formule (la).

2. Composés de formule (la) tels que définis à la revendication 1, répondant aux formules suivantes :
- 5-fluoro-2-[2-(4-fluoro-2,3-dihydro-1H-indol-1-yl)-2-oxoéthyl]-6-(morpholin-4-yl)pyrimidin-4(3H)-one
- 5-fluoro-2-[2-(4-fluoro-2,3-dihydro-1H-indol-1-yl)-2-oxoéthyl]-3-méthyl-6-(morpholin-4-yl)pyrimidin-4(3H)-one
- 5-fluoro-2-[1-(4-fluoro-2,3-dihydro-1H-indol-1-yl)-1-oxopropan-2-yl]-3-méthyl-6-(morpholin-4-yl)pyrimidin-4(3H)-one
- 5-fluoro-2-{2-[(2R)-2-méthyl-2,3-dihydro-1H-indol-1-yl]-2-oxoéthyl}-6-(morpholin-4-yl)pyrimidin-4(3H)-one
- 5-fluoro-2-{2-[(2S)-2-méthyl-2,3-dihydro-1H-indol-1-yl]-2-oxoéthyl}-6-(morpholin-4-yl)pyrimidin-4(3H)-one
- 5-fluoro-2-{2-[(2R)-5-fluoro-2-méthyl-2,3-dihydro-1H-indol-1-yl]-2-oxoéthyl}-6-(morpholin-4-yl)pyrimidin-4(3H)-one
- 5-fluoro-2-{2-[(2S)-5-fluoro-2-méthyl-2,3-dihydro-1H-indol-1-yl]-2-oxoéthyl}-6-(morpholin-4-yl)pyrimidin-4(3H)-one
- 5-fluoro-3-méthyl-2-{2-[(2S)-2-méthyl-2,3-dihydro-1H-indol-1-yl]-2-oxoéthyl}-6-(morpholin-4-yl)pyrimidin-4(3H)-one
- 5-fluoro-3-méthyl-2-{2-[(2R)-2-méthyl-2,3-dihydro-1H-indol-1-yl]-2-oxoéthyl}-6-(morpholin-4-yl)pyrimidin-4(3H)-one
- 5-fluoro-2-{2-[(2R)-4-fluoro-2-méthyl-2,3-dihydro-1H-indol-1-yl]-2-oxoéthyl}-6-(morpholin-4-yl)pyrimidin-4(3H)-one
- 5-fluoro-2-{2-[(2S)-4-fluoro-2-méthyl-2,3-dihydro-1H-indol-1-yl]-2-oxoéthyl}-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-5-fluoro-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-5-fluoro-6-(morpholin-4-yl)pyrimidin-4(3H)-one
- 5-fluoro-2-{2-[(2S)-5-fluoro-2-méthyl-2,3-dihydro-1H-indol-1-yl]-2-oxoéthyl}-3-méthyl-6-(morpholin-4-yl)pyrimidin-4(3H)-one
- 5-fluoro-2-{2-[(2R)-5-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-5-fluoro-6-(morpholin-4-yl)pyrimidin-4(3H)-one
- 2-{2-[(2S)-4-bromo-2-méthyl-2,3-dihydro-1H-indol-1-yl]-2-oxoéthyl}-5-fluoro-6-(morpholin-4-yl)pyrimidin-4(3H)-one
- 2-{2-[(2R)-4-chloro-2-méthyl-2,3-dihydro-1H-indol-1-yl]-2-oxoéthyl}-5-fluoro-6-(morpholin-4-yl)pyrimidin-4(3H)-one
- 2-{2-[(2S)-4-chloro-2-méthyl-2,3-dihydro-1H-indol-1-yl]-2-oxoéthyl}-5-fluoro-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-5-fluoro-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-5-fluoro-6-(morpholin-4-yl)pyrimidin-4(3H)-one
- 5-fluoro-2-{2-[(2S)-4-fluoro-2-méthyl-2,3-dihydro-1H-indol-1-yl]-2-oxoéthyl}-3-méthyl-6-(morpholin-4-yl)pyrimidin-4(3H)-one
- 5-fluoro-2-{2-[(2R)-4-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2R)-4-chloro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-4-chloro-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-5-fluoro-3-méthyl-6-(morpholin-4-yl)pyrimidin-4(3H)-one
- 2-{2-[(2S)-4-bromo-2-méthyl-2,3-dihydro-1H-indol-1-yl]-2-oxoéthyl}-5-fluoro-3-méthyl-6-(morpholin-4-yl)pyrimidin-4(3H)-one
- 5-fluoro-2-{2-[(2S)-6-fluoro-2-méthyl-2,3-dihydro-1H-indol-1-yl]-2-oxoéthyl}-6-(morpholin-4-yl)pyrimidin-4(3H)-one
- 5-fluoro-2-{2-[(2R)-6-fluoro-2-méthyl-2,3-dihydro-1H-indol-1-yl]-2-oxoéthyl}-6-(morpholin-4-yl)pyrimidin-4(3H)-one
- 5-chloro-2-{2-[(2R)-2-méthyl-2,3-dihydro-1H-indol-1-yl]-2-oxoéthyl}-6-(morpholin-4-yl)pyrimidin-4(3H)-one
- 5-chloro-2-{2-[(2S)-2-méthyl-2,3-dihydro-1H-indol-1-yl]-2-oxoéthyl}-6-(morpholin-4-yl)pyrimidin-4(3H)-one
- 2-{2-[-4-chloro-5-fluoro-2-méthyl-2,3-dihydro-1H-indol-1-yl]-2-oxoéthyl}-5-fluoro-3-méthyl-6-(morpholin-4-yl)pyrimidin-4(3H)-one
- 5-bromo-2-[2-((S)-2-methyl-2,3-dihydro-indol-1-yl)-2-oxo-ethyl]-6-morpholin-4-yl-3H-pyrimidin-4-one
- 5-Fluoro-2-[2-((+)-2-hydroxymethyl-2,3-dihydro-indol-1-yl)-2-oxo-ethyl]-6-morpholin-4-yl-3H-pyrimidin-4-one
- 5-Fluoro-2-[2-((-)-2-hydroxymethyl-2,3-dihydro-indol-1-yl)-2-oxo-ethyl]-6-morpholin-4-yl-3H-pyrimidin-4-one
ainsi que les sels d'addition avec les acides minéraux et organiques ou avec les bases minérales et organiques desdits composés de formule (la).

3. Composés de formule (Ib): 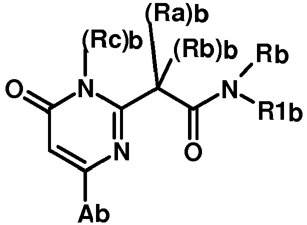 dans laquelle :
Ab représente un radical morpholine ou un radical pyridyle définis comme suit :
le radical morpholine, que peut représenter Ab, étant substitué par un ou plusieurs radicaux choisis parmi l'atome de deutérium,et les radicaux alkyles eux-mêmes éventuellement substitués par un ou plusieurs radicaux choisis parmi les atomes d'halogène et le radical hydroxyle, étant entendu que deux substituants adjacents de la morpholine peuvent former ensemble un radical cyclique avec les atomes de carbone auxquels ils sont liés ;
le radical pyridyle, que peut représenter Ab, étant éventuellement substitué par un atome d'halogène ou un radical alkyle ou alcoxy;
R1b représente un radical aryle ou hétéroaryle éventuellement substitués par un ou plusieurs radicaux identiques ou différents choisis parmi les atomes d'halogène et les radicaux hydroxyle, CN, nitro, -COOH, -COOalk, -N(Rx)b, (Ry)b, -N(Rx)bCO(Ry)b,-CO(Ry)b, -N(Rx)bCO₂(Rz)b, alcoxy, phénoxy, alkylthio, alkyle, alkényle, alkynyle, cycloalkyle, O-cycloalkyle, hétérocycloalkyle ; aryle et hétéroaryle;
ces derniers radicaux alcoxy, phénoxy, alkylthio, alkyle, alkényle, alkynyle, hétérocycloalkyle, aryle et hétéroaryle étant eux-mêmes éventuellement substitués par un ou plusieurs radicaux identiques ou différents choisis parmi les atomes d'halogène et les radicaux hydroxyle, alcoxy et N(Rv)b(Rw)b ;
les radicaux aryle et hétéroaryle étant de plus éventuellement substitués par un ou plusieurs radicaux alkyle et alcoxy eux-mêmes éventuellement substitués par un ou plusieurs atomes d'halogène ;
les radicaux hétérocycloalkyle et hétéroaryle pouvant de plus renfermer un radical oxo,
Rb représente un atome d'hydrogène ou bien forme avec R1 b un cycle
à 5 ou 6 chaînons saturé ou partiellement ou totalement insaturé fusionné à un reste aryle ou hétéroaryle et renfermant éventuellement un ou plusieurs autres hétéroatomes choisi(s) parmi O, S, N, NH et Nalk, ce radical bicyclique étant éventuellement substitué par un ou plusieurs radicaux identiques ou différents choisis parmi les atomes d'halogène et les radicaux hydroxyle, alkyle et alcoxy;
(Ra)b et (Rb)b, identiques ou différents représentent indépendamment un atome d'hydrogène, un atome d'halogène ou un radical alkyle éventuellement substitué par un ou plusieurs atomes d'halogène ;
(Rc)b représente un atome d'hydrogène ou un radical alkyle éventuellement substitué par un ou plusieurs atomes d'halogène;
N(Rx)b(Ry)b étant tel que (Rx)b représente un atome d'hydrogène ou un radical alkyle et (Ry)b représente un atome d'hydrogène, un radical cycloalkyle ou un radical alkyle éventuellement substitué par un ou plusieurs radicaux identiques ou différents choisis parmi les radicaux hydroxyle, alcoxy, N(Rv)b(Rw)b et hétérocycloalkyle ; soit (Rx)b et (Ry)b forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
N(Rv)b(Rw)b étant tel que (Rv)b représente un atome d'hydrogène ou un radical alkyle et (Rw)b représente un atome d'hydrogène, un radical cycloalkyle ou un radical alkyle éventuellement substitué par un ou plusieurs radicaux identiques ou différents choisis parmi les radicaux hydroxyle, alcoxy, hétérocycloalkyle ; soit (Rv)b et (Rw)b forment avec l'atome d'azote auquel ils sont liés un radical cyclique renfermant de 3 à 10 chaînons et éventuellement un ou plusieurs autres hétéroatomes choisi(s) parmi O, S, NH et N-alkyle, ce radical cyclique étant éventuellement substitué;
les radicaux cycliques que peuvent former (Rx)b et (Ry)b ou (Rv)b et (Rw)b respectivement avec l'atome d'azote auquel ils sont liés, étant éventuellement substitués par un ou plusieurs radicaux identiques ou différents choisis parmi les atomes d'halogène, les radicaux alkyle, hydroxyle, oxo, alcoxy, NH2; NHalk et N(alk)2 ;
(Rz)b représente les valeurs de (Ry)b à l'exception de hydrogène ;
(Rx)b, (Ry)b et (Rz)b dans les radicaux -N(Rx)bCO(Ry)b, -CO(Ry)b et N(Rx)bCO₂(Rz)b étant choisis parmi les significations indiquées ci-dessus pour (Rx)b, (Ry)b, et (Rz)b ;
tous les radicaux alkyle, alcoxy et alkylthio ci-dessus étant linéaires ou ramifiés et renfermant de 1 à 6 atomes de carbone,
lesdits composés de formule (Ib) étant sous toutes les formes isomères possibles racémiques, énantiomères et diastéréoisomères, ainsi que les sels d'addition avec les acides minéraux et organiques ou avec les bases minérales et organiques desdits composés de formule (Ib).

4. Composés de formule (Ib) tels que définis à la revendication 3, répondant aux formules suivantes :
N-(4-Fluoro-phényl)-2-[4-(2-méthyl-morpholin-4-yl)-6-oxo-1,6-dihydro-pyrimidin-2-yl]-acétamide
N-(4-Fluoro-phényl)-2-[4-((S)-2-méthyl-morpholin-4-yl)-6-oxo-1,6-dihydro-pyrimidin-2-yl]-acétamide
2-[4-(2-Fluorométhyl-morpholin-4-yl)-6-oxo-1,6-dihydro-pyrimidin-2-yl]-N-(4-fluoro-phényl)-acétamide
(+)-2-[4-(2-Fluorométhyl-morpholin-4-yl)-6-oxo-1,6-dihydro-pyrimidin-2-yl]-N-(4-fluoro-phényl)-acétamide
(+)-N-(3-Chloro-4-fluoro-phényl)-2-[4-(2-fluorométhyl-morpholin-4-yl)-1-méthyl-6-oxo-1,6-dihydro-pyrimidin-2-yl]-acétamide
2-[2-(4-Fluoro-2,3-dihydro-indol-1-yl)-2-oxo-éthyl]-6-(2-fluorométhyl-morpholin-4-yl)-3H-pyrimidin-4-one
(+)-2-[2-(4-Fluoro-2,3-dihydro-indol-1-yl)-2-oxo-éthyl]-6-(2-fluorométhyl-morpholin-4-yl)-3H-pyrimidin-4-one
2-[2-(4-Fluoro-2,3-dihydro-indol-1-yl)-2-oxo-éthyl]-6-(2-méthyl-morpholin-4-yl)-3H-pyrimidin-4-one
(-)-2-[2-(4-Fluoro-2,3-dihydro-indol-1-yl)-2-oxo-éthyl]-6-(2-méthyl-morpholin-4-yl)-3H-pyrimidin-4-one
(+)-2-[2-(4-Fluoro-2,3-dihydro-indol-1-yl)-2-oxo-éthyl]-6-(2-méthyl-morpholin-4-yl)-3H-pyrimidin-4-one
2-[2-(4-Fluoro-2,3-dihydro-indol-1-yl)-2-oxo-éthyl]-6-(hexahydro-cyclopenta[1,4]oxazin-4-yl)-3H-pyrimidin-4-one
2-[2-(4-Fluoro-2,3-dihydro-indol-1-yl)-2-oxo-éthyl]-6-pyridin-4-yl-3H-pyrimidin-4-one
2-[2-(4-Fluoro-2,3-dihydro-indol-1-yl)-2-oxo-éthyl]-6-(2-methoxy-pyridin-4-yl)-3H-pyrimidin-4-one
(±)-2-[4-(2-Éthyl-morpholin-4-yl)-6-oxo-1,6-dihydro-pyrimidin-2-yl]-N-(4-fluoro-phényl)-acétamide
(+)-2-[4-(2-Éthyl-morpholin-4-yl)-6-oxo-1,6-dihydro-pyrimidin-2-yl]-N-(4-fluoro-phényl)-acétamide
2-[2-(4-fluoro-2,3-dihydro-indol-1-yl)-2-oxo-éthyl]-6-(2,2,3,3,5,5,6,6-D8-morpholin)-4-yl-3h-pyrimidin-4-one
2-[2-(4-Fluoro-2,3-dihydro-indol-1-yl)-2-oxo-éthyl]-6-(3,3,5,5-D4-morpholin)-4-yl-3H-pyrimidin-4-one
2-(4-Chloro-6-methoxy-pyrimidin-2-yl)-1-(3,3-diméthyl-2,3-dihydro-indol-1-yl)-ethanone
(+)-2-[2-(2-méthyle-2,3-dihydro-indol-1-yl)-2-oxo-éthyl]-6-(3,3,5,5-D4-morpholin)-4-yl-3H-pyrimidin-4-one
(-)-2-[2-(2-méthyle-2,3-dihydro-indol-1-yl)-2-oxo-éthyl]-6-(3,3,5,5-D4-morpholin)-4-yl-3H-pyrimidin-4-one
(+)-2-[2-(2-méthyle-2,3-dihydro-indol-1-yl)-2-oxo-éthyl]-6-(2,2,3,3,5,5,6,6-D8-morpholin)-4-yl-3H-pyrimidin-4-one
(-)-2-[2-(2-méthyle-2,3-dihydro-indol-1-yl)-2-oxo-éthyl]-6-(2,2,3,3,5,5,6,6-D8-morpholin)-4-yl-3H-pyrimidin-4-one
2-[2-(4-Fluoro-2,3-dihydro-indol-1-yl)-2-oxo-éthyl]-6-(2-méthyl-pyridin-4-yl)-3H-pyrimidin-4-one
2-[2-(4-Fluoro-2,3-dihydro-indol-1-yl)-2-oxo-éthyl]-6-(2-fluoro-pyridin-4-yl)-3H-pyrimidin-4-one
(-)-2-[2-(4-Chloro-2,3-dihydro-indol-1-yl)-2-oxo-éthyl]-6-(2-hydroxyméthyl-morpholin-4-yl)-3H-pyrimidin-4-one
(+)-2-[2-(4-Chloro-2,3-dihydro-indol-1-yl)-2-oxo-éthyl]-6-(2-hydroxyméthyl-morpholin-4-yl)-3H-pyrimidin-4-one
(±)-2-[2-(4-Fluoro-2,3-dihydro-indol-1-yl)-2-oxo-éthyl]-6-(2-hydroxyméthyl-morpholin-4-yl)-3H-pyrimidin-4-one
(±)-2-[2-(4-Chloro-2,3-dihydro-indol-1-yl)-2-oxo-éthyl]-6-(2-méthyl-morpholin-4-yl)-3H-pyrimidin-4-one
(+)-2-[2-((S)-2-Méthyl-2,3-dihydro-indol-1-yl)-2-oxo-éthyl]-6-(-2-méthyl-morpholin-4-yl)-3H-pyrimidin-4-one
(+)-6-(2-Fluorométhyl-morpholin-4-yl)-2-[2-((S)-2-méthyl-2,3-dihydro-indol-1-yl)-2-oxo-éthyl]-3H-pyrimidin-4-one
(+)-6-(2-Hydroxyméthyl-morpholin-4-yl)-2-[2-((S)-2-méthyl-2,3-dihydro-indol-1-yl)-2-oxo-éthyl]-3H-pyrimidin-4-one
ainsi que les sels d'addition avec les acides minéraux et organiques ou avec les bases minérales et organiques desdits composés de formule (Ib).

5. Composés répondant aux formules suivantes :
- 2-[2-(4-chloro-2-méthyl-2,3-dihydro-1H-indol-1-yl)-2-oxoéthyl]-6-(morpholin-4-yl)pyrimidin-4(3H)-one
- 2-{2-[(2S)-4-chloro-2-méthyl-2,3-dihydro-1H-indol-1-yl]-2-oxoéthyl}-6-(morpholin-4-yl)pyrimidin-4(3H)-one
- 2-{2-[(2R)-4-chloro-2-méthyl-2,3-dihydro-1H-indol-1-yl]-2-oxoéthyl}-6-(morpholin-4-yl)pyrimidin-4(3H)-one
- 2-[2-(4-chloro-3,3-diméthyl-2,3-dihydro-1H-indol-1-yl)-2-oxoéthyl]-3-méthyl-6-(morpholin-4-yl)pyrimidin-4(3H)-one
- 2-[2-(4-chloro-3,3-diméthyl-2,3-dihydro-1H-indol-1-yl)-2-oxoéthyl]-6-(morpholin-4-yl)pyrimidin-4(3H)-one
- 2-[2-(4-hydroxy-2-méthyl-2,3-dihydro-1H-indol-1-yl)-2-oxoéthyl]-6-(morpholin-4-yl)pyrimidin-4(3H)-one
- 2-[2-(4-fluoro-3,3-diméthyl-2,3-dihydro-1H-indol-1-yl)-2-oxoéthyl]-3-méthyl-6-(morpholin-4-yl)pyrimidin-4(3H)-one
- 2-[2-(4-fluoro-3,3-diméthyl-2,3-dihydro-1H-indol-1-yl)-2-oxoéthyl]-6-(morpholin-4-yl)pyrimidin-4(3H)-one
- 2-{2-[(2S)-4-hydroxy-2-méthyl-2,3-dihydro-1H-indol-1-yl]-2-oxoéthyl}-6-(morpholin-4-yl)pyrimidin-4(3H)-one
- 2-{2-[(2R)-4-hydroxy-2-méthyl-2,3-dihydro-1H-indol-1-yl]-2-oxoéthyl}-6-(morpholin-4-yl)pyrimidin-4(3H)-one
- 2-{2-[(3R)-4-hydroxy-3-méthyl-2,3-dihydro-1H-indol-1-yl]-2-oxoéthyl}-6-(morpholin-4-yl)pyrimidin-4(3H)-one
- 2-{2-[(3S)-4-hydroxy-3-méthyl-2,3-dihydro-1H-indol-1-yl]-2-oxoéthyl}-6-(morpholin-4-yl)pyrimidin-4(3H)-one
- 2-{2-[(2S)-5-fluoro-2-méthyl-2,3-dihydro-1H-indol-1-yl]-2-oxoéthyl}-6-(morpholin-4-yl)pyrimidin-4(3H)-one
- 2-{2-[(2R)-5-fluoro-2-méthyl-2,3-dihydro-1H-indol-1-yl]-2-oxoéthyl}-6-(morpholin-4-yl)pyrimidin-4(3H)-one
- 2-{2-[(2S)-5-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-5-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-6-(morpholin-4-yl)pyrimidin-4(3H)-one
- 2-{2-[(2S)-4,5-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4,5-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-6-(morpholin-4-yl)pyrimidin-4(3H)-one
- 2-{2-[(2S)-5,6-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-5,6-difluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-6-(morpholin-4-yl)pyrimidin-4(3H)-one
- 2-{2-[(2S)-4-bromo-2-méthyl-2,3-dihydro-1H-indol-1-yl]-2-oxoéthyl}-6-(morpholin-4-yl)pyrimidin-4(3H)-one
- 2-{2-[(2R)-4-fluoro-2-méthyl-2,3-dihydro-1H-indol-1-yl]-2-oxoéthyl}-6-(morpholin-4-yl)pyrimidin-4(3H)-one
- 2-{2-[(2S)-4-fluoro-2-méthyl-2,3-dihydro-1H-indol-1-yl]-2-oxoéthyl}-6-(morpholin-4-yl)pyrimidin-4(3H)-one
- 2-{2-[(2R)-4-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-fluoro-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-bromo-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-bromo-2-méthyl-2,3-dihydro-1H-indol-1-yl]-2-oxoéthyl}-3-méthyl-6-(morpholin-4-yl)pyrimidin-4(3H)-one
- 2-{2-[(2R)-4-chloro-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-chloro-2-méthyl-2,3-dihydro-1H-indol-1-yl]-2-oxoéthyl}-3-méthyl-6-(morpholin-4-yl)pyrimidin-4(3H)-one
- 2-{2-[(2R)-6-fluoro-2-méthyl-2,3-dihydro-1H-indol-1-yl]-2-oxoéthyl}-6-(morpholin-4-yl)pyrimidin-4(3H)-one
- 2-{2-[(2S)-6-fluoro-2-méthyl-2,3-dihydro-1H-indol-1-yl]-2-oxoéthyl}-6-(morpholin-4-yl)pyrimidin-4(3H)-one
- 2-{2-[(2R)-4-chloro-5-fluoro-2-méthyl-2,3-dihydro-1H-indol-1-yl]-2-oxoéthyl}-6-(morpholin-4-yl)pyrimidin-4(3H)-one
- 2-{2-[(2S)-4-chloro-5-fluoro-2-méthyl-2,3-dihydro-1H-indol-1-yl]-2-oxoéthyl}-6-(morpholin-4-yl)pyrimidin-4(3H)-one
- 2-{2-[(2R)-2-isopropyl-2,3-dihydro-indol-1-yl]-2-oxo-éthyl}-6-morpholin-4-yl-3H-pyrimidin-4-one
- 2-{2-[(2S)-2-isopropyl-2,3-dihydro-indol-1-yl]-2-oxo-éthyl}-6-morpholin-4-yl-3H-pyrimidin-4-one
- 2-[1-(4-fluoro-2,3-dihydro-1H-indol-1-yl)-1-oxopropan-2-yl]-6-(morpholin-4-yl)pyrimidin-4(3H)-one
- 2-[2-((R)-2-éthyl-2,3-dihydro-indol-1-yl)-2-oxo-éthyl]-6-morpholin-4-yl-3H-pyrimidin-4-one
- 2-[2-((S)-2-éthyl-2,3-dihydro-indol-1-yl)-2-oxo-éthyl]-6-morpholin-4-yl-3H-pyrimidin-4-one
ainsi que les sels d'addition avec les acides minéraux et organiques ou avec les bases minérales et organiques desdits composés.

6. Composés répondant aux formules suivantes :
- 6-(morpholin-4-yl)-2-[2-oxo-2-(spiro[cyclopropane-1,3'-indol]-1'(2'H)-yl)éthyl]pyrimidin-4(3H)-one
- 6-(morpholin-4-yl)-2-[2-oxo-2-(4-phényl-2,3-dihydro-1H-indol-1-yl)éthyl]pyrimidin-4(3H)-one
- 6-(morpholin-4-yl)-2-{2-oxo-2-[4-(trifluorométhoxy)-2,3-dihydro-1H-indol-1-yl]éthyl}pyrimidin-4(3H)-one
- 3-méthyl-6-(morpholin-4-yl)-2-{2-oxo-2-[4-(trifluorométhyl)-2,3-dihydro-1H-indol-1-yl]éthyl}pyrimidin-4(3H)-one
- 6-(morpholin-4-yl)-2-{2-oxo-2-[4-(trifluorométhyl)-2,3-dihydro-1H-indol-1-yl]éthyl}pyrimidin-4(3H)-one
- 2-{2-[4-(2-méthoxyphényl)-2,3-dihydro-1H-indol-1-yl]-2-oxoéthyl}-6-(morpholin-4-yl)pyrimidin-4(3H)-one
- 6-(morpholin-4-yl)-2-{2-oxo-2-[4-(1-propylpipéridin-3-yl)-2,3-dihydro-1H-indol-1-yl]éthyl}pyrimidin-4(3H)-one
- 2-{2-[4-(difluorométhoxy)-2,3-dihydro-1H-indol-1-yl]-2-oxoéthyl}-3-méthyl-6-(morpholin-4-yl)pyrimidin-4(3H)-one
- 2-{2-[4-(difluorométhoxy)-2,3-dihydro-1H-indol-1-yl]-2-oxoéthyl}-6-(morpholin-4-yl)pyrimidin-4(3H)-one
- 6-(morpholin-4-yl)-2-{2-oxo-2-[4-(pyridin-4-yl)-2,3-dihydro-1H-indol-1-yl]éthyl}pyrimidin-4(3H)-one
- 2-[2-(1'-méthylspiro[indole-3,4'-pipéridin]-1(2H)-yl)-2-oxoéthyl]-6-(morpholin-4-yl)pyrimidin-4(3H)-one
- 6-(morpholin-4-yl)-2-{2-oxo-2-[4-(pyridin-2-yl)-2,3-dihydro-1H-indol-1-yl]éthyl}pyrimidin-4(3H)-one
- 6-(morpholin-4-yl)-2-{2-oxo-2-[4-(pyridin-3-yl)-2,3-dihydro-1H-indol-1-yl]éthyl}pyrimidin-4(3H)-one
- 2-{2-[4-(2-chlorophényl)-2,3-dihydro-1H-indol-1-yl]-2-oxoéthyl}-6-(morpholin-4-yl)pyrimidin-4(3H)-one
- 2-[2-(4-chloro-2,3-dihydro-1H-indol-1-yl)-2-oxoéthyl]-3-cyclopropyl-6-(morpholin-4-yl)pyrimidin-4(3H)-one
- 6-(morpholin-4-yl)-2-[2-oxo-2-(2,3,3a,8b-tétrahydrocyclopenta[b]indol-4(1H)-yl)éthyl]pyrimidin-4(3H)-one
- 2-[2-(4-{[4-(méthylsulfonyl)pipérazin-1-yl]méthyll-2,3-dihydro-1H-indol-1-yl)-2-oxoéthyl]-6-(morpholin-4-yl)pyrimidin-4(3H)-one
- 2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one
- 2-{2-[4-(2-fluorophényl)-2,3-dihydro-1H-indol-1-yl]-2-oxoéthyl}-6-(morpholin-4-yl)pyrimidin-4(3H)-one
- 3-méthyl-2-(2-{4-[(4-méthylpipérazin-1-yl)méthyl]-2,3-dihydro-1H-indol-1-yl}-2-oxoéthyl)-6-(morpholin-4-yl)pyrimidin-4(3H)-one
- 6-(morpholin-4-yl)-2-[2-oxo-2-(2',3',5',6'-tétrahydrospiro[indole-3,4'-pyran]-1(2H)-yl)éthyl]pyrimidin-4(3H)-one
- 3-méthyl-6-(morpholin-4-yl)-2-[2-oxo-2-(2',3',5',6'-tétrahydrospiro[indole-3,4'-pyran]-1(2H)-yl)éthyl]pyrimidin-4(3H)-one
- 3-méthyl-6-(morpholin-4-yl)-2-[2-oxo-2-(spiro[indole-3,4'-pipéridin]-1(2H)-yl)éthyl]pyrimidin-4(3H)-one
- 6-(morpholin-4-yl)-2-{2-oxo-2-[(2R)-2-phényl-2,3-dihydro-1H-indol-1-yl]éthyl}pyrimidin-4(3H)-one
- 6-(morpholin-4-yl)-2-{2-oxo-2-[(2S)-2-phényl-2,3-dihydro-1H-indol-1-yl]éthyl}pyrimidin-4(3H)-one
- 2-{2-[4-(4-méthylpipérazin-1-yl)-2,3-dihydro-1H-indol-1-yl]-2-oxoéthyl}-6-(morpholin-4-yl)pyrimidin-4(3H)-one
- 2-{2-[(2R)-2-(4-fluorophényl)-2,3-dihydro-1H-indol-1-yl]-2-oxoéthyl}-6-(morpholin-4-yl)pyrimidin-4(3H)-one
- 2-{2-[(2S)-2-(4-fluorophényl)-2,3-dihydro-1H-indol-1-yl]-2-oxoéthyl}-6-(morpholin-4-yl)pyrimidin-4(3H)-one
- 2-{2-[(2R)-4-(difluorométhoxy)-2-méthyl-2,3-dihydro-1H-indol-1-yl]-2-oxoéthyl}-6-(morpholin-4-yl)pyrimidin-4(3H)-one
- 2-{2-[(2S)-4-(difluorométhoxy)-2-méthyl-2,3-dihydro-1H-indol-1-yl]-2-oxoéthyl}-6-(morpholin-4-yl)pyrimidin-4(3H)-one
- 2-{2-[(2R)-4-(difluorométhoxy)-2-méthyl-2,3-dihydro-1H-indol-1-yl]-2-oxoéthyl}-3-méthyl-6-(morpholin-4-yl)pyrimidin-4(3H)-one
- 2-{2-[(2S)-4-(difluorométhoxy)-2-méthyl-2,3-dihydro-1H-indol-1-yl]-2-oxoéthyl}-3-méthyl-6-(morpholin-4-yl)pyrimidin-4(3H)-one
- 6-(morpholin-4-yl)-2-{2-[4-(morpholin-4-yl)-2,3-dihydro-1H-indol-1-yl]-2-oxoéthyl}pyrimidin-4(3H)-one
- 2-{2-[(2R)-2-cyclopropyl-2,3-dihydro-1H-indol-1-yl]-2-oxoéthyl}-6-(morpholin-4-yl)pyrimidin-4(3H)-one
- 2-{2-[(2S)-2-cyclopropyl-2,3-dihydro-1H-indol-1-yl]-2-oxoéthyl}-6-(morpholin-4-yl)pyrimidin-4(3H)-one
- 2-{2-[(2R)-2-méthyl-4-(trifluorométhyl)-2,3-dihydro-1H-indol-1-yl]-2-oxoéthyl}-6-(morpholin-4-yl)pyrimidin-4(3H)-one
- 2-{2-[(2S)-2-méthyl-4-(trifluorométhyl)-2,3-dihydro-1H-indol-1-yl]-2-oxoéthyl}-6-(morpholin-4-yl)pyrimidin-4(3H)-one
- 2-[2-((+)-2-Fluoromethyl-2,3-dihydro-indol-1-yl)-2-oxo-ethyl]-6-morpholin-4-yl-3H-pyrimidin-4-one
- 2-[2-(2,3-Dihydro-indol-1-yl)-2-oxo-ethyl]-6-morpholin-4-yl-3-phenyl-3H-pyrimidin-4-one
ainsi que les sels d'addition avec les acides minéraux et organiques ou avec les bases minérales et organiques desdits composés.

7. Composés de formule (le) : 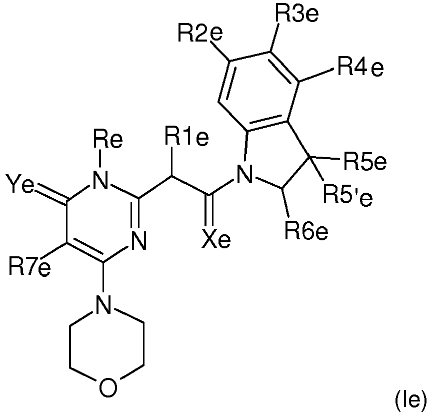 dans laquelle :
Xe et Ye, identiques ou différents, sont tels que :
Xe représente O ou S et Ye représente S,
Re représente un atome d'hydrogène ;
R1 e représente un atome d'hydrogène ou un radical méthyle ;
R2e représente un atome d'hydrogène ou un atome de fluor ;
R3e représente un atome d'hydrogène ou un atome d'halogène ;
R4e représente un atome d'hydrogène, un atome d'halogène ou un radical hydroxyle, alkyle ou alcoxy, les radicaux alkyle étant éventuellement substitués par un ou plusieurs radicaux identiques ou différents choisis parmi les atomes d'halogène et le radical hydroxyle, les radicaux alcoxy étant éventuellement substitués par un ou plusieurs atomes d'halogène ;
R5e et R5'e, identiques ou différents, représente un atome d'hydrogène ou un radical alkyle ;
R6e représente un atome d'hydrogène ou un radical alkyle éventuellement substitué par un ou plusieurs radicaux identiques ou différents choisis parmi les atomes d'halogène et le radical hydroxyle ;
R7e représente un atome d'hydrogène ;
lesdits composés de formule (le) étant sous toutes les formes isomères possibles racémiques, énantiomères et diastéréoisomères, ainsi que les sels d'addition avec les acides minéraux et organiques ou avec les bases minérales et organiques desdits composés de formule (le).

8. Composés de formule (le) tels que définis à la revendication 7, répondant aux formules suivantes :
- 1-((S)-2-Methyl-2,3-dihydro-indol-1-yl)-2-(4-morpholin-4-yl-6-thioxo-1,6-dihydro-pyrimidin-2-yl)-ethanone
- 1-((R)-2-Methyl-2,3-dihydro-indol-1-yl)-2-(4-morpholin-4-yl-6-thioxo-1,6-dihydro-pyrimidin-2-yl)-ethanone
- 2-[2-((S)-2-Methyl-2,3-dihydro-indol-1-yl)-2-thioxo-ethyl]-6-morpholin-4-yl-3H-pyrimidine-4-thione
ainsi que les sels d'addition avec les acides minéraux et organiques ou avec les bases minérales et organiques desdits composés de formule (le).

9. Composés tels que définis à l'une quelconque des revendications 1 à 8 ainsi que les sels d'addition avec les acides minéraux et organiques ou avec les bases minérales et organiques pharmaceutiquement acceptables desdits composés, pour leur utilisation en tant que médicaments.

10. Compositions pharmaceutiques contenant à titre de principe actif l'un au moins des composés tels que définis à l'une quelconque des revendications 1 à 8 ou un sel pharmaceutiquement acceptable de ce composé et un support pharmaceutiquement acceptable.

11. Composés tels que définis à l'une quelconque des revendications 1 à 8, pour leur utilisation dans le traitement ou la prévention d'une maladie choisie dans le groupe suivant : troubles de la prolifération de vaisseaux sanguins, troubles fibrotiques, troubles de la prolifération de cellules 'mesangial', désordres métaboliques, allergies, asthmes, thromboses, maladies du système nerveux, rétinopathie, psoriasis, arthrite rhumatoïde, diabète, dégénérescence musculaire et cancers.

12. Composés tels que définis à l'une quelconque des revendications 1 à 8, pour leur utilisation dans le traitement de cancers.

13. Composés tels que définis à l'une quelconque des revendications 1 à 8, pour leur utilisation selon la revendication 12 dans le traitement de cancers résistant à des agents cytotoxiques.

14. Composés tels que définis à l'une quelconque des revendications 1 à 8, pour leur utilisation selon l'une quelconque des revendications 12 ou 13 dans laquelle le cancer est choisi parmi les tumeurs primaires et/ou les métastases en particulier les cancers gastriques, hépatiques, rénaux, ovariens, du colon, de la prostate, du poumon (NSCLC et SCLC), les glioblastomes, les cancers de la thyroïde, de la vessie, du sein, le mélanome, les tumeurs hématopoietiques lymphoïdes ou myéloïdes, les sarcomes, les cancers du cerveau, du larynx, du système lymphatique, les cancers des os et du pancréas, les hamartomes.

15. Composés tels que définis aux revendications 1 à 8, pour leur utilisation dans la chimiothérapie de cancers seul ou en association.

## Patentansprüche

1. Verbindungen der Formel (Ia): 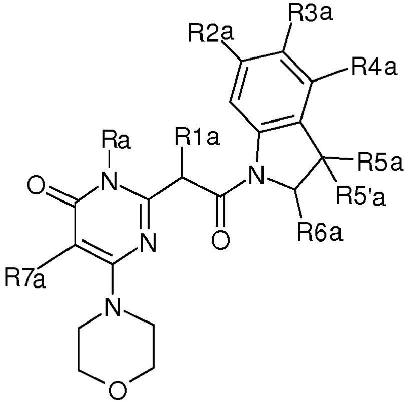 wobei:
Ra ein Wasserstoffatom oder ein Alkylradikal darstellt;
R1a ein Wasserstoffatom oder ein Methylradikal darstellt;
R2a ein Wasserstoffatom oder ein Fluoratom darstellt;
R3a ein Wasserstoffatom oder ein Halogenatom darstellt;
R4a ein Wasserstoffatom, ein Halogenatom oder ein Hydroxyl-, Alkyl- oder Alkoxyradikal, wobei die Alkylradikale wahlweise substituiert sind mit einem oder mehreren Radikalen, gleich oder verschieden, ausgewählt aus Halogenatomen und Hydroxylradikal, wobei die Alkoxyradikale wahlweise substituiert sind mit einem oder mehreren Halogenatomen, darstellt; R5a und R5'a, gleich oder verschieden, ein Wasserstoffatom oder ein Alkylradikal darstellt;
R6a ein Wasserstoffatom oder ein Alkylradikal, wahlweise substituiert mit einem oder mehreren Radikalen, gleich oder verschieden, ausgewählt aus Halogenatomen und Hydroxylradikal, darstellt;
R7a ein Halogenatom darstellt;
wobei die Verbindungen der Formel (Ia) alle möglichen racemischen, enantiomeren und diastereomeren isomeren Formen sind, sowie die Salze der Addition mit mineralischen und organischen Säuren oder mit mineralischen und organischen Basen der Verbindungen der Formel (Ia).

2. Verbindungen der Formel (Ia) gemäß Anspruch 1, entsprechend den folgenden Formeln:
- 5-Fluoro-2-[2-(4-fluoro-2,3-dihydro-1H-indol-l-yl)-2-oxoethyl]-6-(morpholin-4-yl)pyrimidin-4(3H)-on
- 5-Fluoro-2-[2-(4-fluoro-2,3-dihydro-1H-indol-l-yl)-2-oxoethyl]-3-methyl-6-(moipholin-4-yl)pyrimidin-4(3H)-on
- 5-Fluoro-2-[1-(4-fluoro-2,3-dihydro-1H-indol-1-yl)-1-oxopropan-2-yl]-3-methyl-6-(morpholin-4-yl)pyrimidin-4(3H)-on
- 5-Fluoro-2-{2-[(2R)-2-methyl-2,3-dihydro-1H-indol-1-yl]-2-oxoethyl}-6-(morpholin-4-yl)pyrimidin-4(3H)-on
- 5-Fluoro-2-{2-[(2S)-2-methyl-2,3-dihydro-1H-indol-1-yl]-2-oxoethyl}-6-(moipholin-4-yl)pyrimidin-4(3H)-on
- 5-Fluoro-2-{2-[(2R)-5-fluoro-2-methyl-2,3-dihydro-1H-indol-1-yl]-2-oxoethyl}-6-(moipholin-4-yl)pyrimidin-4(3H)-on
- 5-Fluoro-2-{2-[(2S)-5-fluoro-2-methyl-2,3-dihydro-1H-indol-1-yl]-2-oxoethyl}-6-(morpholin-4-yl)pyrimidin-4(3H)-on
- 5-Fluoro-3-methyl-2-{2-[(2S)-2-methyl-2,3-dihydro-1H-indol-1-yl]-2-oxoethyl}-6-(morpholin-4-yl)pyrimidin-4(3H)-on
- 5-Fluoro-3-methyl-2-{2-[(2R)-2-methyl-2,3-dihydro-1H-indol-1-yl]-2-oxoethyl}-6-(morpholin-4-yl)pyrimidin-4(3H)-on
- 5-Fluoro-2-{2-[(2R)-4-fluoro-2-methyl-2,3-dihydro-1H-indol-1-yl]-2-oxoethyl}-6-(morpholin-4-yl)pyrimidin-4(3H)-on
- 5-Fluoro-2-{2-[(2S)-4-fluoro-2-methyl-2,3-dihydro-1H-indol-1-yl]-2-oxoethyl}-6-(moipholin-4-yl)pyrimidin-4(3H)-on
- 2-{2-[(2S)-5,6-Difluoro-2-methyl-2,3-dihydro-1H-indol-1-yl]-2-oxoethyl}-5-fluoro-6-(morpholin-4-yl)pyrimidin-4(3H)-on
- 2-{2-[(2R)-5,6-Difluoro-2-methyl-2,3-dihydro-1H-indol-1-yl]-2-oxoethyl}-5-fluoro-6-(morpholin-4-yl)pyrimidin-4(3H)-on
- 5-Fluoro-2-{2-[(2S)-5-fluoro-2-methyl-2,3-dihydro-1H-indol-1-yl]-2-oxoethyl}-3-methyl-6-(morpholin-4-yl)pyrimidin-4(3H)-on
- 5-Fluoro-2-{2-[(2R)-5-fluoro-2-methyl-2,3-dihydro-1H-indol-1-yl]-2-oxoethyl}-3-methyl-6-(morpholin-4-yl)pyrimidin-4(3H)-on
- 2-{2-[(2R)-4-Bromo-2-methyl-2,3-dihydro-1H-indol-1-yl]-2-oxoethyl}-5-fluoro-6-(morpholin-4-yl)pyrimidin-4(3H)-on
- 2-{2-[(2S)-4-Bromo-2-methyl-2,3-dihydro-1H-indol-1-yl]-2-oxoethyl]-5-fluoro-6-(morpholin-4-yl)pyrimidin-4(3H)-on
- 2-{2-[(2R)-4-Chloro-2-methyl-2,3-dihydro-1H-indol-1-yl]-2-oxoethyl}-5-fluoro-6-(morpholin-4-yl)pyrimidin-4(3H)-on
- 2-{2-[(2S)-4-Chloro-2-methyl-2,3-dihydro-1H-indol-1-yl]-2-oxoethyl}-5-fluoro-6-(moipholin-4-yl)pyrimidin-4(3H)-on
- 2-{2-[(2R)-4,5-Difluoro-2-methyl-2,3-dihydro-1H-indol-1-yl]-2-oxoethyl}-5-fluoro-6-(morpholin-4-yl)pyrimidin-4(3H)-on
- 2-{2-[(2S)-4,5-Difluoro-2-methyl-2,3-dihydro-1H-indol-1-yl]-2-oxoethyl}-5-fluoro-6-(morpholin-4-yl)pyrimidin-4(3H)-on
- 5-Fluoro-2-{2-[(2S)-4-fluoro-2-methyl-2,3-dihydro-1H-indol-1-yl]-2-oxoethyl}-3-methyl-6-(morpholin-4-yl)pyrimidin-4(3H)-on
- 5-Fluoro-2-{2-[(2R)-4-fluoro-2-methyl-2,3-dihydro-1H-indol-1-yl]-2-oxoethyl}-3-methyl-6-(morpholin-4-yl)pyrimidin-4(3H)-on
- 2-{2-[(2R)-4,5-Difluoro-2-methyl-2,3-dihydro-1H-indol-1-yl]-2-oxoethyl}-5-fluoro-3-methyl-6-(morpholin-4-yl)pyrimidin-4(3H)-on
- 2-{2-[(2S)-4,5-Difluoro-2-methyl-2,3-dihydro-1H-indol-1-yl]-2-oxoethyl}-5-fluoro-3-methyl-6-(morpholin-4-yl)pyrimidin-4(3H)-on
- 2-{2-[(2R)-5,6-Difluoro-2-methyl-2,3-dihydro-1H-indol-1-yl]-2-oxoethyl}-5-fluoro-3-methyl-6-(morpholin-4-yl)pyrimidin-4(3H)-on
- 2-{2-[(2S)-5,6-Difluoro-2-methyl-2,3-dihydro-1H-indol-1-yl]-2-oxoethyl}-5-fluoro-3-methyl-6-(morpholin-4-yl)pyrimidin-4(3H)-on
- 2-{2-[(2R)-4-Chloro-2-methyl-2,3-dihydro-1H-indol-1-yl]-2-oxoethyl}-5-fluoro-3-methyl-6-(morpholin-4-yl)pyrimidin-4(3H)-on
- 2-{2-[(2S)-4-Chloro-2-methyl-2,3-dihydro-1H-indol-1-yl]-2-oxoethyl}-5-fluoro-3-methyl-6-(morpholin-4-yl)pyrimidin-4(3H)-on
- 2-{2-[(2R)-4-Bromo-2-methyl-2,3-dihydro-1H-indol-1-yl]-2-oxoethyl}-5-fluoro-3-methyl-6-(morpholin-4-yl)pyrimidin-4(3H)-on
- 2-{2-[(2S)-4-Bromo-2-methyl-2,3-dihydro-1H-indol-1-yl]-2-oxoethyl}-5-fluoro-3-methyl-6-(morpholin-4-yl)pyrimidin-4(3H)-on
- 5-Fluoro-2-{2-[(2S)-6-fluoro-2-methyl-2,3-dihydro-1H-indol-1-yl]-2-oxoethyl}-6-(morpholin-4-yl)pyrimidin-4(3H)-on
- 5-Fluoro-2-{2-[(2R)-6-fluoro-2-methyl-2,3-dihydro-1H-indol-l-yl]-2-oxoethyl}-6-(morpholin-4-yl)pyrimidin-4(3H)-on
- 5-Chloro-2-{2-[(2R)-2-methyl-2,3-dihydro-1H-indol-1-yl]-2-oxoethyl}-6-(morpholin-4-yl)pyrimidin-4(3H)-on
- 5-Chloro-2-{2-[(2S)-2-methyl-2,3-dihydro-1H-indol-1-yl]-2-oxoethyl}-6-(morpholin-4-yl)pyrimidin-4(3H)-on
- 2-{2-[-4-Chloro-5-fluoro-2-methyl-2,3-dihydro-1H-indol-1-yl]-2-oxoethyl}-5-fluoro-3-methyl-6-(morpholin-4-yl)pyrimidin-4(3H)-on
- 5-Bromo-2-[2-((S)-2-methyl-2,3-dihydro-indol-1-yl)-2-oxo-ethyl]-6-morpholin-4-yl-3H-pyrimidin-4-on
- 5-Fluoro-2-[2-((+)-2-hydroxymethyl-2,3-dihydro-indol-1-yl)-2-oxo-ethyl]-6-morpholin-4-yl-3H-pyrimidin-4-on
- 5-Fluoro-2-[2-((-)-2-hydroxymethyl-2,3-dihydro-indol-1-yl)-2-oxo-ethyl]-6-morpholin-4-yl-3H-pyrimidin-4-on,
sowie die Salze der Addition mit mineralischen und organischen Säuren oder mit mineralischen und organischen Basen der Verbindungen der Formel (Ia).

3. Verbindungen der Formel (Ib): 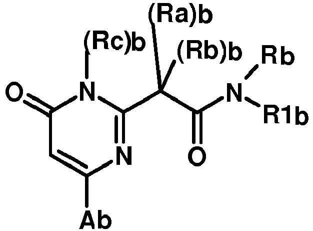 wobei:
Ab ein Morpholin-Radikal oder ein Pyridyl-Radikal, definiert wie folgt, darstellt:
das Morpholin-Radikal, das Ab darstellen kann, ist substituiert mit einem oder mehreren Radikalen ausgewählt aus Deuteriumatom und Alkylradikalen, wobei diese wahlweise selbst substituiert sind mit einem oder mehreren Radikalen ausgewählt aus Halogenatomen und Hydroxylradikal, wobei zwei benachbarte Substituenten des Morpholins gemeinsam ein cyclisches Radikal mit Kohlenstoffatomen bilden können, an die sie gebunden sind;
das Pyridyl-Radikal, das Ab darstellen kann, ist wahlweise substituiert mit einem Halogenradikal oder einem Alkyl- oder Alkoxyradikal;
R1b ein Aryl- oder Heteroarylradikal, wahlweise substituiert mit einem oder mehreren Radikalen, gleich oder verschieden, ausgewählt aus Halogenatomen und Hydroxyl-, CN-, Nitro-, -COOH-, -COOAlk-, -N(Rx)b, (Ry)b-, -N(Rx)bCO(Ry)b,-CO(Ry)b-, -N(Rx)bCO₂(Rz)b, Alkoxy-, Phenoxy-, Alkylthio-, Alkyl-, Alkenyl-, Alkinyl-, Cycloalkyl-, O-Cycloalkyl-, Heterocycloalkyl-; Aryl- und Heteroarylradikalen, darstellt;
wobei die letzten Alkoxy-, Phenoxy-, Alkylthio-, Alkyl-, Alkenyl-, Alkinyl-, Heterocycloalkyl-, Aryl- und Heteroarylradikale selbst wahlweise substituiert sind mit einem oder mehreren Radikalen, gleich oder verschieden, ausgewählt aus Halogenatomen und Hydroxyl-, Alkoxy- und N(Rv)b(Rw)b-Radikalen;
wobei die Aryl- und Heteroarylradikale darüber hinaus wahlweise substituiert sind mit einem oder mehreren Alkyl- und Alkoxyradikalen, wobei diese wahlweise selbst substituiert sind mit einem oder mehreren Halogenatomen;
wobei die Heterocycloalkyl- und Heteroarylradikale darüber hinaus ein Oxoradikal enthalten können,
Rb ein Wasserstoffatom darstellt oder aber mit R1b einen Ring mit 5 oder 6 Kettengliedern, gesättigt oder teilweise oder vollständig ungesättigt, fusioniert mit einem Aryl- oder Heteroarylrest und enthaltend wahlweise ein oder mehrere andere Heteroatome ausgewählt aus O, S, N, NH und NAlk, bildet, wobei dieses bicyclische Radikal wahlweise substituiert ist mit einem oder mehreren Radikalen,
gleich oder verschieden, ausgewählt aus Halogenatomen und Hydroxyl-, Alkyl- und Alkoxyradikalen;
(Ra)b und (Rb)b, gleich oder verschieden, unabhängig ein Wasserstoffatom, ein Halogenatom oder ein Alkylradikal, wahlweise substituiert mit einem oder mehreren Halogenatomen, darstellen;
(Rc)b ein Wasserstoffatom oder ein Alkylradikal, wahlweise substituiert mit einem oder mehreren Halogenatomen, darstellt;
N(Rx)b(Ry)b so ist, dass (Rx)b ein Wasserstoffatom oder ein Alkylradikal darstellt und (Ry)b ein Wasserstoffatom, ein Cycloalkylradikal oder ein Alkylradikal, wahlweise substituiert mit einem oder mehreren Radikalen, gleich oder verschieden, ausgewählt aus Hydroxyl-, Alkoxy-, N(Rv)b(Rw)b- und Heterocycloalkylradikalen, darstellt; oder (Rx)b und (Ry)b bilden mit dem Stickstoffatom, an welches sie gebunden sind, ein cyclisches Radikal enthaltend von 3 bis 10 Kettenglieder und wahlweise ein oder mehrere andere Heteroatome ausgewählt aus O, S, NH und N-Alkyl, wobei dieses cyclische Radikal wahlweise substituiert ist;
N(Rv)b(Rw)b so ist, dass (Rv)b ein Wasserstoffatom oder ein Alkylradikal darstellt und (Rw)b ein Wasserstoffatom, ein Cycloalkylradikal oder ein Alkylradikal, wahlweise substituiert mit einem oder mehreren Radikalen, gleich oder verschieden, ausgewählt aus Hydroxyl-, Alkoxy-, Heterocycloalkylradikalen, darstellt; oder (Rv)b und (Rw)b bilden mit dem Stickstoffatom, an welches sie gebunden sind, ein cyclisches Radikal enthaltend von 3 bis 10 Kettenglieder und wahlweise ein oder mehrere andere Heteroatome ausgewählt aus O, S, NH und N-Alkyl, wobei dieses cyclische Radikal wahlweise substituiert ist;
die cyclischen Radikale, die (Rx)b und (Ry)b oder (Rv)b und (Rw)b jeweils mit dem Stickstoffatom, an welches sie gebunden sind, bilden können, wahlweise substituiert sind mit einem oder mehreren Radikalen, gleich oder verschieden, ausgewählt aus Halogenatomen, Alkyl-, Hydroxyl-, Oxo-, Alkoxy-, NH2-, NHAlk- und N(Alk)2-Radikalen;
(Rz)b die Werte von (Ry)b, ausgenommen Wasserstoff, darstellt;
(Rx)b, (Ry)b und (Rz)b in den Radikalen -N(Rx)bCO(Ry)b, -CO(Ry)b und N(Rx)bCO₂(Rz)b ausgewählt sind aus den gleichen vorausgehenden Bezeichnungen für (Rx)b, (Ry)b und (Rz)b;
wobei die vorausgehenden Alkyl-, Alkoxy- und Alkylthioradikale linear oder verzweigt sind und von 1 bis 6 Kohlenstoffatome enthalten,
wobei die Verbindungen der Formel (Ib) alle möglichen racemischen, enantiomeren und diastereomeren isomeren Formen sind, sowie die Salze der Addition mit mineralischen und organischen Säuren oder mit mineralischen und organischen Basen der Verbindungen der Formel (Ib).

4. Verbindungen der Formel (Ib) gemäß Anspruch 3, entsprechend den folgenden Formeln:
N-(4-Fluoro-phenyl)-2-[4-(2-methyl-morpholin-4-yl)-6-oxo-1,6-dihydro-pyrimidin-2yl]-acetamid
N-(4-Fluoro-phenyl)-2-[4-((S)-2-methyl-morpholin-4-yl)-6-oxo-1,6-dihydro-pyrimidin-2-yl]-acetamid
2-[4-(2-Fluoromethyl-morpholin-4-yl)-6-oxo-1,6-dihydro-pyrimidin-2-yl]-N-(4-fluoro-phenyl)-acetamid
(+)-2-[4-(2-Fluoromethyl-morpholin-4-yl)-6-oxo-1,6-dihydro-pyrimidin-2-yl]-N-(4-fluoro-phenyl)-acetamid
(+)-N-(3-Chloro-4-fluoro-phenyl)-2 4-(2-fluoromethyl-morpholin-4-yl)-1-methyl-6-oxo-1,6-dihydro-pyrimidin-2-yl] -acetamid
2-[2-(4-Fluoro-2,3-dihydro-indol-1-yl)-2-oxo-ethyl]-6-(2-fluoromethyl-morpholin-4-yl)-3H-pyrimidin-4-on
(+)-2-[2-(4-Fluoro-2,3-dihydro-indol-1-yl)-2-oxo-ethyl]-6-(2-fluoromethyl-morpholin-4-yl)-3H-pyrimidin-4-on
2-[2-(4-Fluoro-2,3-dihydro-indol-1-yl)-2-oxo-ethyl]-6-(2-methyl-morpholin-4-yl)-3H-pyrimidin-4-on
(-)-2-[2-(4-Fluoro-2,3-dihydro-indol-1-yl)-2-oxo-ethyl]-6-(2-methyl-morpholin-4-yl)-3H-pyumidin-4-on
(+)-2-[2-(4-Fluoro-2,3-dihydro-indol-1-yl)-2-oxo-ethyl]-6-(2-methyl-morpholin-4-yl)-3H-pyumidin-4-on
2-[2-(4-Fluoro-2,3-dihydro-indol-1-yl)-2-oxo-ethyl]-6-(hexahydro-cyclopenta[1,4]oxazin-4-yl)-3H-pyumidin-4-on
2-[2-(4-Fluoro-2,3-dihydro-indol-1-yl)-2-oxo-ethyl]-6-pyridin-4-yl-3H-pyrimidin-4-on
2-[2-(4-Fluoro-2,3-dihydro-indol-1-yl)-2-oxo-ethyl]-6-(2-methoxy-pyridin-4-yl)-3H-pyrimidin-4-on
(±)-2-[4-(2-Ethyl-morpholin-4-yl)-6-oxo-1,6-dihydro-pyrimidin-2-yl]-N-(4-fluoro-phenyl)-acetamid
(+)-2-[4-(2-Ethyl-morpholin-4-yl)-6-oxo-1,6-dihydro-pyrimidin-2-yl]-N-(4-fluoro-phenyl)-acetamid
2-[2-(4-Fluoro-2,3-dihydro-indol-1-yl)-2-oxo-ethyl]-6-(2,2,3,3,5,5,6,6-D8-morpholin)-4-yl-3h-pyrimidin-4-on
2-[2-(4-Fluoro-2,3-dihydro-indol-1-yl)-2-oxo-ethyl]-6-(3,3,5,5-D4-moipholin)-4-yl-3H-pyrimidin-4-on
2-(4-Chloro-6-methoxy-pyrimidin-2-yl)-1-(3,3-dimethyl-2,3-dihydro-indol-1-yl)-ethanon
(+)-2-[2-(2-Methyle-2,3-dihydro-indol-1-yl)-2-oxo-ethyl]-6-(3,3,5,5-D4-morpholin)-4-yl-3H-pyrimidin-4-on
(-)-2-[2-(2-Methyle-2,3-dihydro-indol-1-yl)-2-oxo-ethyl]-6-(3,3,5,5-D4-morpholin)-4-yl-3H-pyrimidin-4-on
(+)-2-[2-(2-Methyle-2,3-dihydro-indol-1-yl)-2-oxo-ethyl]-6-(2,2,3,3,5,5,6,6-D8-morpholin)-4-yl-3H-pyrimidin-4-on
(-)-2-[2-(2-Methyle-2,3-dihydro-indol-1-yl)-2-oxo-ethyl]-6-(2,2,3,3,5,5,6,6-D8-morpholin)-4-yl-3H-pyrimidin-4-on
2-[2-(4-Fluoro-2,3-dihydro-indol-1-yl)-2-oxo-ethyl]-6-(2-methyl-pyridin-4-yl)-3H-pyrimidin-4-on
2-[2-(4-Fluoro-2,3-dihydro-indol-1-yl)-2-oxo-ethyl]-6-(2-fluoro-pyridin-4-yl)-3H-pyrimidin-4-on
(-)-2-[2-(4-Chloro-2,3-dihydro-indol-1-yl)-2-oxo-ethyl]-6-(2-hydroxymethyl-morpholin-4-yl)-3H-pyrimidin-4-on
(+)-2-[2-(4-Chloro-2,3-dihydro-indol-1-yl)-2-oxo-ethyl]-6-(2-hydroxymethyl-morpholin-4-yl)-3H-pyrimidin-4-on
(±)-2-[2-(4-Fluoro-2,3-dihydro-indol-1-yl)-2-oxo-ethyl]-6-(2-hydroxymethyl-morpholin-4-yl)-3H-pyrimidin-4-on
(±)-2-[2-(4-Chloro-2,3-dihydro-indol-1-yl)-2-oxo-ethyl]-6-(2-methyl-morpholin-4-yl)-3H-pyrimidin-4-on
(+)-2-[2-((S)-2-Methyl-2,3-dihydro-indol-1-yl)-2-oxo-ethyl]-6-(-2-methyl-morpholin-4-yl)-3H-pyrimidin-4-on
(+)-6-(2-Fluoromethyl-morpholin-4-yl)-2-[2-((S)-2-methyl-2,3-dihydro-indol-1-yl)-2-oxo-ethyl]-3H-pyrimidin-4-on
(+)-6-(2-Hydroxymethyl-moipholin-4-yl)-2-[2-((S)-2-methyl-2,3-dihydro-indol-1-yl)-2-oxo-ethyl]-3H-pyrimidin-4-on,
sowie die Salze der Addition mit mineralischen und organischen Säuren oder mit mineralischen und organischen Basen der Verbindungen der Formel (Ib).

5. Verbindungen entsprechend den folgenden Formeln:
- 2-[2-(4-Chloro-2-methyl-2,3-dihydro-1H-indol-1-yl)-2-oxoethyl]-6-(moipholin-4-yl)pyrimidin-4(3H)-on
- 2-{2-[(2S)-4-Chloro-2-methyl-2,3-dihydro-1H-indol-1-yl]-2-oxoethyl}-6-(morpholin-4-yl)pyrimidin-4(3H)-on
- 2-{2-[(2R)-4-Chloro-2-methyl-2,3-dihydro-1H-indol-1-yl]-2-oxoethyl}-6-(morpholin-4-yl)pyrimidin-4(3H)-on
- 2-[2-(4-Chloro-3,3-dimethyl-2,3-dihydro-1H-indol-1-yl)-2-oxoethyl]-3-methyl-6-(morpholin-4-yl)pyrimidin-4(3H)-on
- 2-[2-(4-Chloro-3,3-dimethyl-2,3-dihydro-1H-indol-1-yl)-2-oxoethyl]-6-(morpholin-4-yl)pyrimidin-4(3H)-on
- 2-[2-(4-Hydroxy-2-methyl-2,3-dihydro-1H-indol-1-yl)-2-oxoethyl]-6-(morpholin-4-yl)pyrimidin-4(3H)-on
- 2-[2-(4-Fluoro-3,3-dimethyl-2,3-dihydro-1H-indol-1-yl)-2-oxoethyl]-3-methyl-6-(morpholin-4-yl)pyrimidin-4(3H)-on
- 2-[2-(4-Fluoro-3,3-dimethyl-2,3-dihydro-1H-indol-1-yl)-2-oxoethyl]-6-(morpholin-4-yl)pyumidin-4(3H)-on
- 2-{2-[(2S)-4-Hydroxy-2-methyl-2,3-dihydro-1H-indol-1-yl]-2-oxoethyl}-6-(morpholin-4-yl)pyrimidin-4(3H)-on
- 2-{2-[(2R)-4-Hydroxy-2-methyl-2,3-dihydro-1H-indol-1-yl]-2-oxoethyl}-6-(morpholin-4-yl)pyrimidin-4(3H)-on
- 2-{2-[(3R)-4-Hydroxy-3-methyl-2,3-dihydro-1H-indol-1-yl]-2-oxoethyl}-6-(morpholin-4-yl)pyrimidin-4(3H)-on
- 2-{2-[(3S)-4-Hydroxy-3-methyl-2,3-dihydro-1H-indol-1-yl]-2-oxoethyl}-6-(morpholin-4-yl)pyrimidin-4(3H)-on
- 2-{2-[(2S)-5-Fluoro-2-methyl-2,3-dihydro-1H-indol-1-yl]-2-oxoethyl}-6-(morpholin-4-yl)pyrimidin-4(3H)-on
- 2-{2-[(2R)-5-Fluoro-2-methyl-2,3-dihydro-1H-indol-1-yl]-2-oxoethyl}-6-(morpholin- 4-yl)pyrimidin-4(3H)-on
- 2-{2-[(2S)-5-Fluoro-2-methyl-2,3-dihydro-1H-indol-1-yl]-2-oxoethyl}-3-methyl-6-(morpholin-4-yl)pyrimidin-4(3H)-on
- 2-{2-[(2R)-5-Fluoro-2-methyl-2,3-dihydro-1H-indol-1-yl]-2-oxoethyl}-3-methyl-6-(morpholin-4-yl)pyrimidin-4(3H)-on
- 2-{2-[(2S)-4,5-Difluoro-2-methyl-2,3-dihydro-1H-indol-1-yl]-2-oxoethyl}-6-(morpholin-4-yl)pyrimidin-4(3H)-on
- 2-{2-[(2R)-4,5-Difluoro-2-methyl-2,3-dihydro-1H-indol-1-yl]-2-oxoethyl}-6-(morpholin-4-yl)pyrimidin-4(3H)-on
- 2-{2-[(2S)-4,5-Difluoro-2-methyl-2,3-dihydro-1H-indol-1-yl]-2-oxoethyl}-3-methyl-6-(morpholin-4-yl)pyrimidin-4(3H)-on
- 2-{2-[(2R)-4,5-Difluoro-2-methyl-2,3-dihydro-1H-indol-1-yl]-2-oxoethyl}-3-methyl-6-(morpholin-4-yl)pyrimidin-4(3H)-on
- 2-{2-[(2R)-5,6-Difluoro-2-methyl-2,3-dihydro-1H-indol-1-yl]-2-oxoethyl}-6-(morpholin-4-yl)pyrimidin-4(3H)-on
- 2-{2-[(2S)-5,6-Difluoro-2-methyl-2,3-dihydro-1H-indol-1-yl]-2-oxoethyl}-6-(morpholin-4-yl)pyrimidin-4(3H)-on
- 2-{2-[(2S)-5,6-Difluoro-2-methyl-2,3-dihydro-1H-indol-1-yl]-2-oxoethyl}-3-methyl-6-(morpholin-4-yl)pyrimidin-4(3H)-on
- 2-{2-[(2R)-5,6-Difluoro-2-methyl-2,3-dihydro-1H-indol-1-yl]-2-oxoethyl}-3-methyl-6-(morpholin-4-yl)pyrimidin-4(3H)-on
- 2-{2-[(2R)-4-Bromo-2-methyl-2,3-dihydro-1H-indol-1-yl]-2-oxoethyl}-6-(morpholin 4-yl)pyrimidin-4(3H)-on
- 2-{2-[(2S)-4-Bromo-2-methyl-2,3-dihydro-1H-indol-1-yl]-2-oxoethyl}-6-(morpholin 4-yl)pyrimidin-4(3H)-on
- 2-{2-[(2R)-4-Fluoro-2-methyl-2,3-dihydro-1H-indol-1-yl]-2-oxoethyl}-6-(morpholin-4-yl)pyrimidin-4(3H)-on
- 2-{2-[(2S)-4-Fluoro-2-methyl-2,3-dihydro-1H-indol-1-yl]-2-oxoethyl}-6-(morpholin-4-yl)pyrimidin-4(3H)-on
- 2-{2-[(2R)-4-Fluoro-2-methyl-2,3-dihydro-1H-indol-1-yl]-2-oxoethyl}-3-methyl-6-(morpholin-4-yl)pyrimidin-4(3H)-on
- 2-{2-[(2S)-4-Fluoro-2-methyl-2,3-dihydro-1H-indol-1-yl]-2-oxoethyl}-3-methyl-6- (morpholin-4-yl)pyrimidin-4(3H)-on
- 2-{2-[(2R)-4-Bromo-2-methyl-2,3-dihydro-1H-indol-1-yl]-2-oxoethyl}-3-methyl-6-(morpholin-4-yl)pyrimidin-4(3H)-on
- 2-{2-[(2S)-4-Bromo-2-methyl-2,3-dihydro-1H-indol-1-yl]-2-oxoethyl}-3-methyl-6-(morpholin-4-yl)pyrimidin-4(3H)-on
- 2-{2-[(2R)-4-Chloro-2-methyl-2,3-dihydro-1H-indol-1-yl]-2-oxoethyl}-3-methyl-6- (morpholin-4-yl)pyrimidin-4(3H)-on
- 2-{2-[(2S)-4-Chloro-2-methyl-2,3-dihydro-1H-indol-1-yl]-2-oxoethyl}-3-methyl-6-(morpholin-4-yl)pyrimidin-4(3H)-on
- 2-{2-[(2R)-6-Fluoro-2-methyl-2,3-dihydro-1H-indol-1-yl]-2-oxoethyl}-6-(morpholin-4-yl)pyrimidin-4(3H)-on
- 2-{2-[(2S)-6-Fluoro-2-methyl-2,3-dihydro-1H-indol-1-yl]-2-oxoethyl}-6-(morpholin-4-yl)pyrimidin-4(3H)-on
- 2-{2-[(2R)-4-Chloro-5-fluoro-2-methyl-2,3-dihydro-1H-indol-1-yl]-2-oxoethyl}-6-(morpholin-4-yl)pyrimidin-4(3H)-on
- 2-{2-[(2S)-4-Chloro-5-fluoro-2-methyl-2,3-dihydro-1H-indol-1-yl]-2-oxoethyl}-6-(morpholin-4-yl)pyrimidin-4(3H)-on
- 2-{2-[(2R)-2-Isopropyl-2,3-dihydro-indol-1-yl]-2-oxo-ethyl}-6-morpholin-4-yl-3H-pyrimidin-4-on
- 2-{2-[(2S)-2-Isopropyl-2,3-dihydro-indol-1-yl]-2-oxo-ethyl}-6-morpholin-4-yl-3H-pyrimidin-4-on
- 2-[1-(4-Fluoro-2,3-dihydro-1H-indol-1-yl)-1-oxopropan-2-yl]-6-(morpholin-4-yl)pyrimidin-4(3H)-on
- 2-[2-((R)-2-Ethyl-2,3-dihydro-indol-1-yl)-2-oxo-ethyl]-6-morpholin-4-yl-3H-pyrimidin-4-on
- 2-[2-((S)-2-Ethyl-2,3-dihydro-indol-1-yl)-2-oxo-ethyl]-6-morpholin-4-yl-3H-pyrimidin-4-on,
sowie die Salze der Addition mit mineralischen und organischen Säuren oder mit mineralischen und organischen Basen der Verbindungen.

6. Verbindungen entsprechend den folgenden Formeln:
- 6-(Moipholin-4-yl)-2-[2-oxo-2-(spiro[cyclopropan-1,3'-indol]-1'(2'H)-yl)ethyl]pyrimidin-4(3H)-on
- 6-(Moipholin-4-yl)-2-[2-oxo-2-(4-phenyl-2,3-dihydro-1H-indol-1-yl)ethyl]pyrimidin-4(3H)-on
- 6-(Morpholin-4-yl)-2-{2-oxo-2-[4-(trifluoromethoxy)-2,3-dihydro-1H-indol-1-yl]ethyl}pyrimidin-4(3H)-on
- 3-Methyl-6-(morpholin-4-yl)-2-{2-oxo-2-[4-(trifluoromethyl)-2,3-dihydro-1H-indol-1-yl]ethyl}pyrimidin-4(3H)-on
- 6-(Morpholin-4-yl)-2-{2-oxo-2-[4-(trifluoromethyl)-2,3-dihydro-1H-indol-1-yl]ethyl}pyrimidin-4(3H)-on
- 2-{2-[4-(2-Methoxyphenyl)-2,3-dihydro-1H-indol-1-yl]-2-oxoethyl}-6-(morpholin-4-yl)pyrimidin-4(3H)-on
- 6-(Morpholin-4-yl)-2-{2-oxo-2-[4-(1-propylpiperidin-3-yl)-2,3-dihydro-1H-indol-1-yl]ethyl}pyrimidin-4(3H)-on
- 2-{2-[4-(Difluoromethoxy)-2,3-dihydro-1H-indol-1-yl]-2-oxoethyl}-3-methyl-6-(morpholin-4-yl)pyrimidin-4(3H)-on
- 2-{2-[4-(Difluoromethoxy)-2,3-dihydro-1H-indol-1-yl]-2-oxoethyl}-6-(morpholin-4-yl)pyrimidin-4(3H)-on
- 6-(Morpholin-4-yl)-2-{2-oxo-2-[4-(pyridin-4-yl)-2,3-dihydro-1H-indol-1-yl]ethyl}pyrimidin-4(3H)-on
- 2-[2-(1'-Methylspiro[indol-3,4'-piperidin]-1(2H)-yl)-2-oxoethyl]-6-(morpholin-4-yl)pyrimidin-4(3H)-on
- 6-(Morpholin-4-yl)-2-{2-oxo-2-[4-(pyridin-2-yl)-2,3-dihydro-1H-indol-1-yl]ethyl}pyrimidin-4(3H)-on
- 6-(Morpholin-4-yl)-2-{2-oxo-2-[4-(pyridin-3-yl)-2,3-dihydro-1H-indol-1-yl]ethyl}pyrimidin-4(3H)-on
- 2-{2-[4-(2-Chlorophenyl)-2,3-dihydro-1H-indol-1-yl]-2-oxoethyl}-6-(morpholin-4- yl)pyrimidin-4(3H)-on
- 2-[2-(4-Chloro-2,3-dihydro-1H-indol-1-yl)-2-oxoethyl]-3-cyclopropyl-6-(morpholin- 4-yl)pyrimidin-4(3H)-on
- 6-(Morpholin-4-yl)-2-[2-oxo-2-(2,3,3a,8b-tetrahydrocyclopenta[b]indol-4(1H)-yl)ethyl]pyrimidin-4(3H)-on
- 2-[2-(4-{[4-(Methylsulfonyl)piperazin-1-yl]methyl}-2,3-dihydro-1H-indol-1-yl)-2-oxoethyl]-6-(morpholin-4-yl)pyrimidin-4(3H)-on
- 2-(2-{4-[(4-Methylpiperazin-1-yl)methyl]-2,3-dihydro-1H-indol-1-yl}-2-oxoethyl)-6-(moipholin-4-yl)pyrimidin-4(3H)-on
- 2-{2-[4-(2-Fluorophenyl)-2,3-dihydro-1H-indol-1-yl]-2-oxoethyl}-6-(morpholin-4- yl)pyrimidin-4(3H)-on
- 3-Methyl-2-(2-{4-[(4-methylpiperazin-1-yl)methyl]-2,3-dihydro-1H-indol-1-yl}-2-oxoethyl)-6-(morpholin-4-yl)pyrimidin-4(3H)-on
- 6-(Moipholin-4-yl)-2-[2-oxo-2-(2',3',5',6'-tetrahydrospiro[indol-3,4'-pyran]-1(2H)-yl)ethyl]pyrimidin-4(3H)-on
- 3-Methyl-6-(morpholin-4-yl)-2-[2-oxo-2-(2',3',5',6'-tetrahydrospiro[indol-3,4'-pyran]-1(2H)-yl)ethyl]pyrimidin-4(3H)-on
- 3-Methyl-6-(morpholin-4-yl)-2-[2-oxo-2-(spiro[indol-3,4'-piperidin]-1(2H)-yl)ethyl]pyrimidin-4(3H)-on
- 6-(Moipholin-4-yl)-2-12-oxo-2-[(2R)-2-phenyl-2,3-dihydro-1H-indol-1-yl]ethyl}pyrimidin-4(3H)-on
- 6-(Morpholin-4-yl)-2-{2-oxo-2-[(2S)-2-phenyl-2,3-dihydro-1H-indol-1-yl]ethyl}pyrimidin-4(3H)-on
- 2-{2-[4-(4-Methylpiperazin-1-yl)-2,3-dihydro-1H-indol-1-yl]-2-oxoethyl}-6-(morpholin-4-yl)pyrimidin-4(3H)-on
- 2-{2-[(2R)-2-(4-Fluorophenyl)-2,3-dihydro-1H-indol-1-yl]-2-oxoethyl}-6-(morpholin- 4-yl)pyrimidin-4(3H)-on
- 2-{2-[(2S)-2-(4-Fluorophenyl)-2,3-dihydro-1H-indol-1-yl]-2-oxoethyl}-6-(morpholin- 4-yl)pyrimidin-4(3H)-on
- 2-{2-[(2R)-4-(Difluoromethoxy)-2-methyl-2,3-dihydro-1H-indol-1-yl]-2-oxoethyl}-6-(morpholin-4-yl)pyrimidin-4(3H)-on
- 2-{2-[(2S)-4-(Difluoromethoxy)-2-methyl-2,3-dihydro-1H-indol-1-yl]-2-oxoethyl}-6-(morpholin-4-yl)pyrimidin-4(3H)-on
- 2-{2-[(2R)-4-(Difluoromethoxy)-2-methyl-2,3-dihydro-1H-indol-1-yl]-2-oxoethyl}-3-methyl-6-(morpholin-4-yl)pyrimidin-4(3H)-on
- 2-{2-[(2S)-4-(Difluoromethoxy)-2-methyl-2,3-dihydro-1H-indol-1-yl]-2-oxoethyl}-3-methyl-6-(morpholin-4-yl)pyrimidin-4(3H)-on
- 6-(Morpholin-4-yl)-2-{2-[4-(morpholin-4-yl)-2,3-dihydro-1H-indol-1-yl]-2-oxoethyl}pyrimidin-4(3H)-on
- 2-{2-[(2R)-2-Cyclopropyl-2,3-dihydro-1H-indol-1-yl]-2-oxoethyl}-6-(morpholin-4- yl)pyrimidin-4(3H)-on
- 2-{2(2S)-2-Cyclopropyl-2,3-dihydro-1H-indol-1-yl]-2-oxoethyl}-6-(morpholin-4-yl)pyrimidin-4(3H)-on
- 2-{2-[(2R)-2-Methyl-4-(trifluoromethyl)-2,3-dihydro-1H-mdol-1-yl]-2-oxoethyl}-6- (morpholin-4-yl)pyrimidin-4(3H)-on
- 2-{2-[(2S)-2-Methyl-4-(trifluoromethyl)-2,3-dihydro-1H-indol-1-yl]-2-oxoethyl}-6-(morpholin-4-yl)pyrimidin-4(3H)-on
- 2-[2-((+)-2-Fluoromethyl-2,3-dihydro-indol-1-yl)-2-oxo-ethyl]-6-morpholin-4-yl-3H-pyrimidin-4-on
- 2-[2-(2,3-Dihydro-indol-1-yl)-2-oxo-ethyl]-6-morpholin-4-yl-3-phenyl-3H-pyrimidin-4-on,
sowie die Salze der Addition mit mineralischen und organischen Säuren oder mit mineralischen und organischen Basen der Verbindungen.

7. Verbindungen der Formel (Ie): 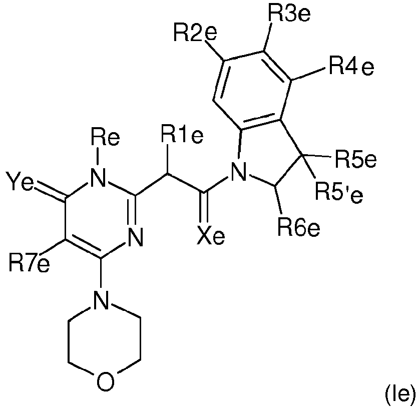 wobei:
Xe und Ye, gleich oder verschieden, diese sind:
Xe stellt O oder S dar und Ye stellt S dar;
Re ein Wasserstoffatom darstellt;
Re1 ein Wasserstoffatom oder ein Methylradikal darstellt;
R2e ein Wasserstoffatom oder ein Fluoratom darstellt;
R3e ein Wasserstoffatom oder ein Halogenatom darstellt;
R4e ein Wasserstoffatom, ein Halogenatom oder ein Hydroxyl-, Alkyl- oder Alkoxyradikal, wobei die Alkylradikale wahlweise substituiert sind mit einem oder mehreren Radikalen, gleich oder verschieden, ausgewählt aus Halogenatomen und Hydroxylradikal, wobei die Alkoxyradikale wahlweise substituiert sind mit einem oder mehreren Halogenatomen, darstellt;
R5e und R5'e, gleich oder verschieden, ein Wasserstoffatom oder ein Alkylradikal darstellt;
R6a ein Wasserstoffatom oder ein Alkylradikal, wahlweise substituiert mit einem oder mehreren Radikalen, gleich oder verschieden, ausgewählt aus Halogenatomen und Hydroxylradikal, darstellt;
R7e ein Wasserstoffatom darstellt;
wobei die Verbindungen der Formel (Ie) alle möglichen racemischen, enantiomeren und diastereomeren isomeren Formen sind, sowie die Salze der Addition mit mineralischen und organischen Säuren oder mit mineralischen und organischen Basen der Verbindungen der Formel (Ie).

8. Verbindungen der Formel (Ie) gemäß Anspruch 7, entsprechend den folgenden Formeln:
- 1-((S)-2-Methyl-2,3-dihydro-indol-1-yl)-2-(4-morpholin-4-yl-6-thioxo-1,6-dihydro-pyrimidin-2-yl)-ethanon
- 1-((R)-2-Methyl-2,3-dihydro-indol-1-yl)-2-(4-morpholin-4-yl-6-thioxo-1,6-dihydro-pyrimidin-2-yl)-ethanon
- 2-[2-((S)-2-Methyl-2,3-dihydro-indol-1-yl)-2-thioxo-ethyl]-6-morpholin-4-yl-3H-pyrimidin-4-thion,
sowie die Salze der Addition mit mineralischen und organischen Säuren oder mit mineralischen und organischen Basen der Verbindungen der Formel (Ie).

9. Verbindungen gemäß einem der Ansprüche 1 bis 8 sowie die Salze der Addition mit mineralischen und organischen Säuren oder mit pharmazeutisch annehmbaren mineralischen und organischen Basen dieser Verbindungen, zu ihrer Verwendung als Medikamente.

10. Pharmazeutische Zusammensetzungen enthaltend als Aktivstoff mindestens eins von den Verbindungen gemäß einem der Ansprüche 1 bis 8 oder ein pharmazeutisch annehmbares Salz dieser Verbindung und einen pharmazeutisch annehmbaren Trägerstoff.

11. Verbindungen gemäß einem der Ansprüche 1 bis 8 zu Verwendung in der Behandlung oder Prävention einer Erkrankung ausgewählt aus der folgenden Gruppe: Proliferationsstörungen der Blutgefäße, fibrotische Erkrankungen, Proliferationsstörungen der ,mesangialen' Zellen, metabolischen, allergischen, asthmatischen, thrombotischen Erkrankungen, Erkrankungen des Nervensystems, Retinopathie, Psoriasis, rheumatische Arthritis, Diabetes, muskuläre Degeneration und Krebs.

12. Verbindungen gemäß einem der Ansprüche 1 bis 8 zur Verwendung in der Behandlung von Krebs.

13. Verbindungen gemäß einem der Ansprüche 1 bis 8 zur Verwendung gemäß Anspruch 12 in der Behandlung von Krebsarten, die gegen zytotoxische Wirkstoffe resistent sind.

14. Verbindungen gemäß einem der Ansprüche 1 bis 8 zur Verwendung gemäß einem der Ansprüche 12 oder 13, wobei der Krebs ausgewählt ist aus primären Tumoren und/oder Metastasen, insbesondere gastutischen, hepatischen, Nieren, Eierstock-, Darm-, Prostata-, Lungen- (NSCLC und SCLC) Krebsarten, Glioblastomen, Schilddrüsen-, Harnblasen-, Brustkrebs, Melanom, lymphoiden oder myeloiden, hämotopoetischen Tumoren, Sarkomen, Hirn-, Kehlkopfkrebs, Krebs des lymphatischen Systems, Knochen- und Pankreaskrebsarten, Hamartomen.

15. Verbindungen gemäß einem der Ansprüche 1 bis 8 zur Verwendung in der Chemotherapie von Krebs, alleine oder in Assoziation.

## Claims

1. Compounds of formula (Ia): 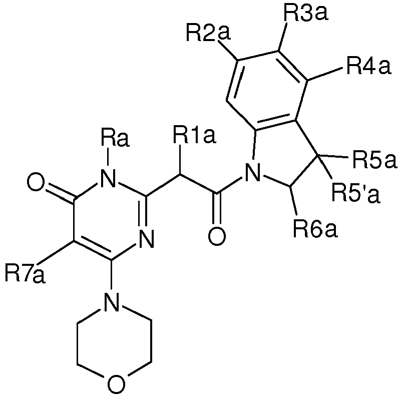 wherein:
Ra is a hydrogen atom or an alkyl radical;
R1 a is a hydrogen atom or a methyl radical;
R2a is a hydrogen atom or a fluorine atom;
R3a is a hydrogen atom or a halogen atom;
R4a is a hydrogen atom, a halogen atom or a hydroxyl, alkyl or alkoxy radical, the alkyl radicals being optionally substituted with one or more radicals, which may be identical or different, selected from halogen atoms and the hydroxyl radical, the alkoxy radicals being optionally substituted with one or more halogen atoms;
R5a and R5'a, which may be identical or different, are a hydrogen atom or an alkyl radical;
R6a is a hydrogen atom or an alkyl radical optionally substituted with one or more radicals, which may be identical or different, selected from halogen atoms and the hydroxyl radical;
R7a is a halogen atom;
said compounds of formula (Ia) being in any of the possible racemic, enantiomeric and diastereoisomeric isomer forms, and also the addition salts with inorganic and organic acids or with inorganic and organic bases of said compounds of formula (Ia).

2. The compounds of formula (Ia) as defined in claim 1, corresponding to the following formulae:
- 5-fluoro-2-[2-(4-fluoro-2,3-dihydro-1H-indol-1-yl)-2-oxoethyl]-6-(morpholin-4-yl)pyrimidin-4(3H)-one
- 5-fluoro-2-[2-(4-fluoro-2,3-dihydro-1H-indol-1-yl)-2-oxoethyl]-3-methyl-6-(morpholin-4-yl)pyrimidin-4(3H)-one
- 5-fluoro-2-[1-(4-fluoro-2,3-dihydro-1H-indol-1-yl)-1-oxopropan-2-yl]-3-methyl-6-(morpholin-4-yl)pyrimidin-4(3H)-one
- 5-fluoro-2-{2-[(2R)-2-methyl-2,3-dihydro-1H-indol-1-yl]-2-oxoethyl}-6-(morpholin-4-yl)pyrimidin-4(3H)-one
- 5-fluoro-2-{2-[(2S)-2-methyl-2,3-dihydro-1H-indol-1-yl]-2-oxoethyl}-6-(morpholin-4-yl)pyrimidin-4(3H)-one
- 5-fluoro-2-{2-[(2R)-5-fluoro-2-methyl-2,3-dihydro-1H-indol-1-yl]-2-oxoethyl}-6-(morpholin-4-yl)pyrimidin-4(3H)-one
- 5-fluoro-2-{2-[(2S)-5-fluoro-2-methyl-2,3-dihydro-1H-indol-1-yl]-2-oxoethyl}-6-(morpholin-4-yl)pyrimidin-4(3H)-one
- 5-fluoro-3-methyl-2-{2-[(2S)-2-methyl-2,3-dihydro-1H-indol-1-yl]-2-oxoethyl}-6-(morpholin-4-yl)pyrimidin-4(3H)-one
- 5-fluoro-3-methyl-2-{2-[(2R)-2-methyl-2,3-dihydro-1H-indol-1-yl]-2-oxoethyl}-6-(morpholin-4-yl)pyrimidin-4(3H)-one
- 5-fluoro-2-{2-[(2R)-4-fluoro-2-methyl-2,3-dihydro-1H-indol-1-yl]-2-oxoethyl}-6-(morpholin-4-yl)pyrimidin-4(3H)-one
- 5-fluoro-2-{2-[(2S)-4-fluoro-2-methyl-2,3-dihydro-1H-indol-1-yl]-2-oxoethyl}-6-(morpholin-4-yl)pyrimidin-4(3H)-one
- 2-{2-[(2S)-5,6-difluoro-2-methyl-2,3-dihydro-1H-indol-1-yl]-2-oxoethyl}-5-fluoro-6-(morpholin-4-yl)pyrimidin-4(3H)-one
- 2-{2-[(2R)-5,6-difluoro-2-methyl-2,3-dihydro-1H-indol-1-yl]-2-oxoethyl}-5-fluoro-6-(morpholin-4-yl)pyrimidin-4(3H)-one
- 5-fluoro-2-{2-[(2S)-5-fluoro-2-methyl-2,3-dihydro-1H-indol-1-yl]-2-oxoethyl}-3-methyl-6-(morpholin-4-yl)pyrimidin-4(3H)-one
- 5-fluoro-2-{2-[(2R)-5-fluoro-2-methyl-2,3-dihydro-1H-indol-1-yl]-2-oxoethyl}-3-methyl-6-(morpholin-4-yl)pyrimidin-4(3H)-one
- 2-{2-[(2R)-4-bromo-2-methyl-2,3-dihydro-1H-indol-1-yl]-2-oxoethyl}-5-fluoro-6-(morpholin-4-yl)pyrimidin-4(3H)-one
- 2-{2-[(2S)-4-bromo-2-methyl-2,3-dihydro-1H-indol-1-yl]-2-oxoethyl}-5-fluoro-6-(morpholin-4-yl)pyrimidin-4(3H)-one
- 2-{2-[(2R)-4-chloro-2-methyl-2,3-dihydro-1H-indol-1-yl]-2-oxoethyl}-5-fluoro-6-(morpholin-4-yl)pyrimidin-4(3H)-one
- 2-{2-[(2S)-4-chloro-2-methyl-2,3-dihydro-1H-indol-1-yl]-2-oxoethyl}-5-fluoro-6-(morpholin-4-yl)pyrimidin-4(3H)-one
- 2-{2-[(2R)-4,5-difluoro-2-methyl-2,3-dihydro-1H-indol-1-yl]-2-oxoethyl}-5-fluoro-6-(morpholin-4-yl)pyrimidin-4(3H)-one
- 2-{2-[(2S)-4,5-difluoro-2-methyl-2,3-dihydro-1H-indol-1-yl]-2-oxoethyl}-5-fluoro-6-(morpholin-4-yl)pyrimidin-4(3H)-one
- 5-fluoro-2-{2-[(2S)-4-fluoro-2-methyl-2,3-dihydro-1H-indol-1-yl]-2-oxoethyl}-3-methyl-6-(morpholin-4-yl)pyrimidin-4(3H)-one
- 5-fluoro-2-{2-[(2R)-4-fluoro-2-methyl-2,3-dihydro-1H-indol-1-yl]-2-oxoethyl}-3-methyl-6-(morpholin-4-yl)pyrimidin-4(3H)-one
- 2-{2-[(2R)-4,5-difluoro-2-methyl-2,3-dihydro-1H-indol-1-yl]-2-oxoethyl}-5-fluoro-3-methyl-6-(morpholin-4-yl)pyrimidin-4(3H)-one
- 2-{2-[(2S)-4,5-difluoro-2-methyl-2,3-dihydro-1H-indol-1-yl]-2-oxoethyl}-5-fluoro-3-methyl-6-(morpholin-4-yl)pyrimidin-4(3H)-one
- 2-{2-[(2R)-5,6-difluoro-2-methyl-2,3-dihydro-1H-indol-1-yl]-2-oxoethyl}-5-fluoro-3-methyl-6-(morpholin-4-yl)pyrimidin-4(3H)-one
- 2-{2-[(2S)-5,6-difluoro-2-methyl-2,3-dihydro-1H-indol-1-yl]-2-oxoethyl}-5-fluoro-3-methyl-6-(morpholin-4-yl)pyrimidin-4(3H)-one
- 2-{2-[(2R)-4-chloro-2-methyl-2,3-dihydro-1H-indol-1-yl]-2-oxoethyl}-5-fluoro-3-methyl-6-(morpholin-4-yl)pyrimidin-4(3H)-one
- 2-{2-[(2S)-4-chloro-2-methyl-2,3-dihydro-1H-indol-1-yl]-2-oxoethyl}-5-fluoro-3-methyl-6-(morpholin-4-yl)pyrimidin-4(3H)-one
- 2-{2-[(2R)-4-bromo-2-methyl-2,3-dihydro-1H-indol-1-yl]-2-oxoethyl}-5-fluoro-3-methyl-6-(morpholin-4-yl)pyrimidin-4(3H)-one
- 2-{2-[(2S)-4-bromo-2-methyl-2,3-dihydro-1H-indol-1-yl]-2-oxoethyl}-5-fluoro-3-methyl-6-(morpholin-4-yl)pyrimidin-4(3H)-one
- 5-fluoro-2-{2-[(2S)-6-fluoro-2-methyl-2,3-dihydro-1H-indol-1-yl]-2-oxoethyl}-6-(morpholin-4-yl)pyrimidin-4(3H)-one
- 5-fluoro-2-{2-[(2R)-6-fluoro-2-methyl-2,3-dihydro-1H-indol-1-yl]-2-oxoethyl}-6-(morpholin-4-yl)pyrimidin-4(3H)-one
- 5-chloro-2-{2-[(2R)-2-methyl-2,3-dihydro-1H-indol-1-yl]-2-oxoethyl}-6-(morpholin-4-yl)pyrimidin-4(3H)-one
- 5-chloro-2-{2-[(2S)-2-methyl-2,3-dihydro-1H-indol-1-yl]-2-oxoethyl}-6-(morpholin-4-yl)pyrimidin-4(3H)-one
- 2-{2-[4-chloro-5-fluoro-2-methyl-2,3-dihydro-1H-indol-1-yl]-2-oxoethyl}-5-fluoro-3-methyl-6-(morpholin-4-yl)pyrimidin-4(3H)-one
- 5-bromo-2-[2-((S)-2-methyl-2,3-dihydroindol-1-yl)-2-oxoethyl]-6-morpholin-4-yl-3H-pyrimidin-4-one
- 5-fluoro-2-[2-((+)-2-hydroxymethyl-2,3-dihydroindol-1-yl)-2-oxoethyl]-6-morpholin-4-yl-3H-pyrimidin-4-one
- 5-fluoro-2-[2-((-)-2-hydroxymethyl-2,3-dihydroindol-1-yl)-2-oxoethyl]-6-morpholin-4-yl-3H-pyrimidin-4-one
and also the addition salts with inorganic and organic acids or with inorganic and organic bases of said compounds of formula (Ia).

3. Compounds of formula (Ib): 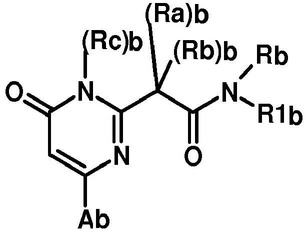 wherein:
Ab is a morpholine radical or a pyridyl radical, defined as follows:
the morpholine radical, that Ab may represent, being substituted with one or more radicals selected from a deuterium atom, and alkyl radicals which are themselves optionally substituted with one or more radicals selected from halogen atoms and the hydroxyl radical, it being understood that two adjacent substituents of the morpholine can together form a cyclic radical with the carbon atoms to which they are bonded;
the pyridyl radical, that Ab may represent, being optionally substituted with a halogen atom or an alkyl or alkoxy radical;
R1b is an aryl or heteroaryl radical optionally substituted with one or more radicals, which may be identical or different, selected from halogen atoms and hydroxyl, CN, nitro, -COOH, -COOalk, -N(Rx)b, (Ry)b, -N(Rx)bCO(Ry)b, -CO(Ry)b, - N(Rx)bCO₂(Rz)b, alkoxy, phenoxy, alkylthio, alkyl, alkenyl, alkynyl, cycloalkyl, O-cycloalkyl, heterocycloalkyl, aryl and heteroaryl radicals;
the latter alkoxy, phenoxy, alkylthio, alkyl, alkenyl, alkynyl, heterocycloalkyl, aryl and heteroaryl radicals being themselves optionally substituted with one or more radicals, which may be identical or different, selected from halogen atoms and hydroxyl, alkoxy and N(Rv)b(Rw)b radicals;
the aryl and heteroaryl radicals being, in addition, optionally substituted with one or more alkyl and alkoxy radicals which are themselves optionally substituted with one or more halogen atoms;
it being possible for the heterocycloalkyl and heteroaryl radicals, in addition, to contain an oxo radical;
Rb is a hydrogen atom or else forms, with R1b, a saturated or partially or totally unsaturated 5- or 6-membered ring fused to an aryl or heteroaryl residue and optionally containing one or more other heteroatoms selected from O, S, N, NH and Nalk, this bicyclic radical being optionally substituted with one or more radicals, which may be identical or different, selected from halogen atoms and hydroxyl, alkyl and alkoxy radicals;
(Ra)b and (Rb)b, which may be identical or different, are independently a hydrogen atom, a halogen atom or an alkyl radical optionally substituted with one or more halogen atoms;
(Rc)b is a hydrogen atom or an alkyl radical optionally substituted with one or more halogen atoms;
N(Rx)b(Ry)b being such that (Rx)b is a hydrogen atom or an alkyl radical and (Ry)b is a hydrogen atom, a cycloalkyl radical or an alkyl radical optionally substituted with one or more radicals, which may be identical or different, selected from hydroxyl, alkoxy, N(Rv)b(Rw)b and heterocycloalkyl radicals; or (Rx)b and (Ry)b form, with the nitrogen atom to which they are bonded, a cyclic radical containing from 3 to 10 ring members and optionally one or more other heteroatoms selected from O, S, NH and N-alkyl, this cyclic radical being optionally substituted;
N(Rv)b(Rw)b being such that (Rv)b is a hydrogen atom or an alkyl radical and (Rw)b is a hydrogen atom, a cycloalkyl radical or an alkyl radical optionally substituted with one or more radicals, which may be identical or different, selected from hydroxyl, alkoxy and heterocycloalkyl radicals; or (Rv)b and (Rw)b form, with the nitrogen atom to which they are bonded, a cyclic radical containing from 3 to 10 ring members and optionally one or more heteroatoms selected from O, S, NH and N-alkyl, this cyclic radical being optionally substituted;
the cyclic radicals that (Rx)b and (Ry)b or (Rv)b and (Rw)b can respectively form with the nitrogen atom to which they are bonded, being optionally substituted with one or more radicals, which may be identical or different, selected from halogen atoms, and alkyl, hydroxyl, oxo, alkoxy, NH₂, NHalk and N(alk)₂ radicals;
(Rz)b is the values for (Ry)b with the exception of hydrogen;
(Rx)b, (Ry)b and (Rz)b in the N(Rx)bCO(Ry)b, -CO(Ry)b and N(Rx)bCO₂(Rz)b radicals being selected from the meanings indicated above for (Rx)b, (Ry)b and (Rz)b;
all the alkyl (alk), alkoxy and alkylthio radicals above being linear or branched and containing from 1 to 6 carbon atoms,
said compounds of formula (Ib) being in any of the possible racemic, enantiomeric and diastereoisomeric isomer forms, and also the addition salts with inorganic and organic acids or with inorganic and organic bases of said compounds of formula (Ib).

4. The compounds of formula (Ib) as defined in claim 3, corresponding to the following formulae:
N-(4-fluorophenyl)-2-[4-(2-methylmorpholin-4-yl)-6-oxo-1,6-dihydropyrimidin-2-yl]acetamide
N-(4-fluorophenyl)-2-[4-((S)-2-methylmorpholin-4-yl)-6-oxo-1,6-dihydropyrimidin-2-yl]acetam ide
2-[4-(2-fluoromethylmorpholin-4-yl)-6-oxo-1,6-dihydropyrimidin-2-yl]-N-(4-fluorophenyl)acetamide
(+)-2-[4-(2-fluoromethylmorpholin-4-yl)-6-oxo-1,6-dihydropyrimidin-2-yl]-N-(4-fluorophenyl)acetamide
(+)-N-(3-chloro-4-fluorophenyl)-2-[4-(2-fluoromethylmorpholin-4-yl)-1-methyl-6-oxo-1,6-dihydropyrimidin-2-yl]acetamide
2-[2-(4-fluoro-2,3-dihydroindol-1-yl)-2-oxoethyl]-6-(2-fluoromethylmorpholin-4-yl)-3H-pyrimidin-4-one
(+)-2-[2-(4-fluoro-2,3-dihydroindol-1-yl)-2-oxoethyl]-6-(2-fluoromethylmorpholin-4-yl)-3H-pyrimidin-4-one
2-[2-(4-fluoro-2,3-dihydroindol-1-yl)-2-oxoethyl]-6-(2-methylmorpholin-4-yl)-3H-pyrimidin-4-one
(-)-2-[2-(4-fluoro-2,3-dihydroindol-1-yl)-2-oxoethyl]-6-(2-methylmorpholin-4-yl)-3H-pyrimidin-4-one
(+)-2-[2-(4-fluoro-2,3-dihydroindol-1-yl)-2-oxoethyl]-6-(2-methylmorpholin-4-yl)-3H-pyrimidin-4-one
2-[2-(4-fluoro-2,3-dihydroindol-1-yl)-2-oxoethyl]-6-(hexahydro-cyclopenta[1,4]oxazin-4-yl)-3H-pyrimidin-4-one
2-[2-(4-fluoro-2,3-dihydroindol-1-yl)-2-oxoethyl]-6-pyridin-4-yl-3H-pyrimidin-4-one
2-[2-(4-fluoro-2,3-dihydroindol-1-yl)-2-oxoethyl]-6-(2-methoxypyridin-4-yl)-3H-pyrimidin-4-one
(±)-2-[4-(2-ethylmorpholin-4-yl)-6-oxo-1,6-dihydropyrimidin-2-yl]-N-(4-fluorophenyl)acetamide
(+)-2-[4-(2-ethylmorpholin-4-yl)-6-oxo-1,6-dihydropyrimidin-2-yl]-N-(4-fluorophenyl)acetamide
2-[2-(4-fluoro-2,3-dihydroindol-1-yl)-2-oxoethyl]-6-(2,2,3,3,5,5,6,6-D8-morpholin)-4-yl-3H-pyrimidin-4-one
2-[2-(4-fluoro-2,3-dihydroindol-1-yl)-2-oxoethyl]-6-(3,3,5,5-D4-morpholin)-4-yl-3H-pyrimidin-4-one
2-(4-chloro-6-methoxypyrimidin-2-yl)-1-(3,3-dimethyl-2,3-dihydroindol-1-yl)ethanone
(+)-2-[2-(2-methyl-2,3-dihydroindol-1-yl)-2-oxoethyl]-6-(3,3,5,5-D4-morpholin)-4-yl-3H-pyrimidin-4-one
(-)-2-[2-(2-methyl-2,3-dihydroindol-1-yl)-2-oxoethyl]-6-(3,3,5,5-D4-morpholin)-4-yl-3H-pyrimidin-4-one
(+)-2-[2-(2-methyl-2,3-dihydroindol-1-yl)-2-oxoethyl]-6-(2,2,3,3,5,5,6,6-D8-morpholin)-4-yl-3H-pyrimidin-4-one
(-)-2-[2-(2-methyl-2,3-dihydroindol-1-yl)-2-oxoethyl]-6-(2,2,3,3,5,5,6,6-D8-morpholin)-4-yl-3H-pyrimidin-4-one
2-[2-(4-fluoro-2,3-dihydroindol-1-yl)-2-oxoethyl]-6-(2-methylpyridin-4-yl)-3H-pyrimidin-4-one
2-[2-(4-fluoro-2,3-dihydroindol-1-yl)-2-oxoethyl]-6-(2-fluoropyridin-4-yl)-3H-pyrimidin-4-one
(-)-2-[2-(4-chloro-2,3-dihydroindol-1-yl)-2-oxoethyl]-6-(2-hydroxymethylmorpholin-4-yl)-3H-pyrimidin-4-one
(+)-2-[2-(4-chloro-2,3-dihydroindol-1-yl)-2-oxoethyl]-6-(2-hydroxymethylmorpholin-4-yl)-3H-pyrimidin-4-one
(±)-2-[2-(4-fluoro-2,3-dihydroindol-1-yl)-2-oxoethyl]-6-(2-hydroxymethylmorpholin-4-yl)-3H-pyrimidin-4-one
(±)-2-[2-(4-chloro-2,3-dihydroindol-1-yl)-2-oxoethyl]-6-(2-methylmorpholin-4-yl)-3H-pyrimidin-4-one
(+)-2-[2-((S)-2-methyl-2,3-dihydroindol-1-yl)-2-oxoethyl]-6-(2-methylmorpholin-4-yl)-3H-pyrimidin-4-one
(+)-6-(2-fluoromethylmorpholin-4-yl)-2-[2-((S)-2-methyl-2,3-dihydroindol-1-yl)-2-oxoethyl]-3H-pyrimidin-4-one
(+)-6-(2-hydroxymethylmorpholin-4-yl)-2-[2-((S)-2-methyl-2,3-dihydroindol-1-yl)-2-oxoethyl]-3H-pyrimidin-4-one
and also the addition salts with inorganic and organic acids or with inorganic and organic bases of said compounds of formula (Ib).

5. Compounds having one of the following formulae:
- 2-[2-(4-chloro-2-methyl-2,3-dihydro-1H-indol-1-yl)-2-oxoethyl]-6-(morpholin-4-yl)pyrimidin-4(3H)-one
- 2-{2-[(2S)-4-chloro-2-methyl-2,3-dihydro-1H-indol-1-yl]-2-oxoethyl}-6-(morpholin-4-yl)pyrimidin-4(3H)-one
- 2-{2-[(2R)-4-chloro-2-methyl-2,3-dihydro-1H-indol-1-yl]-2-oxoethyl}-6-(morpholin-4-yl)pyrimidin-4(3H)-one
- 2-[2-(4-chloro-3,3-dimethyl-2,3-dihydro-1H-indol-1-yl)-2-oxoethyl]-3-methyl-6-(morpholin-4-yl)pyrimidin-4(3H)-one
- 2-[2-(4-chloro-3,3-dimethyl-2,3-dihydro-1H-indol-1-yl)-2-oxoethyl]-6-(morpholin-4-yl)pyrimidin-4(3H)-one
- 2-[2-(4-hydroxy-2-methyl-2,3-dihydro-1H-indol-1-yl)-2-oxoethyl]-6-(morpholin-4-yl)pyrimidin-4(3H)-one
- 2-[2-(4-fluoro-3,3-dimethyl-2,3-dihydro-1H-indol-1-yl)-2-oxoethyl]-3-methyl-6-(morpholin-4-yl)pyrimidin-4(3H)-one
- 2-[2-(4-fluoro-3,3-dimethyl-2,3-dihydro-1H-indol-1-yl)-2-oxoethyl]-6-(morpholin-4-yl)pyrimidin-4(3H)-one
- 2-{2-[(2S)-4-hydroxy-2-methyl-2,3-dihydro-1H-indol-1-yl]-2-oxoethyl}-6-(morpholin-4-yl)pyrimidin-4(3H)-one
- 2-{2-[(2R)-4-hydroxy-2-methyl-2,3-dihydro-1H-indol-1-yl]-2-oxoethyl}-6-(morpholin-4-yl)pyrimidin-4(3H)-one
- 2-{2-[(3R)-4-hydroxy-3-methyl-2,3-dihydro-1H-indol-1-yl]-2-oxoethyl}-6-(morpholin-4-yl)pyrimidin-4(3H)-one
- 2-{2-[(3S)-4-hydroxy-3-methyl-2,3-dihydro-1H-indol-1-yl]-2-oxoethyl}-6-(morpholin-4-yl)pyrimidin-4(3H)-one
- 2-{2-[(2S)-5-fluoro-2-methyl-2,3-dihydro-1H-indol-1-yl]-2-oxoethyl}-6-(morpholin-4-yl)pyrimidin-4(3H)-one
- 2-{2-[(2R)-5-fluoro-2-methyl-2,3-dihydro-1H-indol-1-yl]-2-oxoethyl}-6-(morpholin-4-yl)pyrimidin-4(3H)-one
- 2-{2-[(2S)-5-fluoro-2-methyl-2,3-dihydro-1H-indol-1-yl]-2-oxoethyl}-3-methyl-6-(morpholin-4-yl)pyrimidin-4(3H)-one
- 2-{2-[(2R)-5-fluoro-2-methyl-2,3-dihydro-1H-indol-1-yl]-2-oxoethyl}-3-methyl-6-(morpholin-4-yl)pyrimidin-4(3H)-one
- 2-{2-[(2S)-4,5-difluoro-2-methyl-2,3-dihydro-1H-indol-1-yl]-2-oxoethyl}-6-(morpholin-4-yl)pyrimidin-4(3H)-one
- 2-{2-[(2R)-4,5-difluoro-2-methyl-2,3-dihydro-1H-indol-1-yl]-2-oxoethyl}-6-(morpholin-4-yl)pyrimidin-4(3H)-one
- 2-{2-[(2S)-4,5-difluoro-2-methyl-2,3-dihydro-1H-indol-1-yl]-2-oxoethyl}-3-methyl-6-(morpholin-4-yl)pyrimidin-4(3H)-one
- 2-{2-[(2R)-4,5-difluoro-2-methyl-2,3-dihydro-1H-indol-1-yl]-2-oxoethyl}-3-methyl-6-(morpholin-4-yl)pyrimidin-4(3H)-one
- 2-{2-[(2R)-5,6-difluoro-2-methyl-2,3-dihydro-1H-indol-1-yl]-2-oxoethyl}-6-(morpholin-4-yl)pyrimidin-4(3H)-one
- 2-{2-[(2S)-5,6-difluoro-2-methyl-2,3-dihydro-1H-indol-1-yl]-2-oxoethyl}-6-(morpholin-4-yl)pyrimidin-4(3H)-one
- 2-{2-[(2S)-5,6-difluoro-2-methyl-2,3-dihydro-1H-indol-1-yl]-2-oxoethyl}-3-methyl-6-(morpholin-4-yl)pyrimidin-4(3H)-one
- 2-{2-[(2R)-5,6-difluoro-2-methyl-2,3-dihydro-1H-indol-1-yl]-2-oxoethyl}-3-methyl-6-(morpholin-4-yl)pyrimidin-4(3H)-one
- 2-{2-[(2R)-4-bromo-2-methyl-2,3-dihydro-1H-indol-1-yl]-2-oxoethyl}-6-(morpholin-4-yl)pyrimidin-4(3H)-one
- 2-{2-[(2S)-4-bromo-2-methyl-2,3-dihydro-1H-indol-1-yl]-2-oxoethyl}-6-(morpholin-4-yl)pyrimidin-4(3H)-one
- 2-{2-[(2R)-4-fluoro-2-methyl-2,3-dihydro-1H-indol-1-yl]-2-oxoethyl}-6-(morpholin-4-yl)pyrimidin-4(3H)-one
- 2-{2-[(2S)-4-fluoro-2-methyl-2,3-dihydro-1H-indol-1-yl]-2-oxoethyl}-6-(morpholin-4-yl)pyrimidin-4(3H)-one
- 2-{2-[(2R)-4-fluoro-2-methyl-2,3-dihydro-1H-indol-1-yl]-2-oxoethyl}-3-methyl-6-(morpholin-4-yl)pyrimidin-4(3H)-one
- 2-{2-[(2S)-4-fluoro-2-methyl-2,3-dihydro-1H-indol-1-yl]-2-oxoethyl}-3-methyl-6-(morpholin-4-yl)pyrimidin-4(3H)-one
- 2-{2-[(2R)-4-bromo-2-methyl-2,3-dihydro-1H-indol-1-yl]-2-oxoethyl}-3-methyl-6-(morpholin-4-yl)pyrimidin-4(3H)-one
- 2-{2-[(2S)-4-bromo-2-methyl-2,3-dihydro-1H-indol-1-yl]-2-oxoethyl}-3-methyl-6-(morpholin-4-yl)pyrimidin-4(3H)-one
- 2-{2-[(2R)-4-chloro-2-methyl-2,3-dihydro-1H-indol-1-yl]-2-oxoethyl}-3-methyl-6-(morpholin-4-yl)pyrimidin-4(3H)-one
- 2-{2-[(2S)-4-chloro-2-methyl-2,3-dihydro-1H-indol-1-yl]-2-oxoethyl}-3-methyl-6-(morpholin-4-yl)pyrimidin-4(3H)-one
- 2-{2-[(2R)-6-fluoro-2-methyl-2,3-dihydro-1H-indol-1-yl]-2-oxoethyl}-6-(morpholin-4-yl)pyrimidin-4(3H)-one
- 2-{2-[(2S)-6-fluoro-2-methyl-2,3-dihydro-1H-indol-1-yl]-2-oxoethyl}-6-(morpholin-4-yl)pyrimidin-4(3H)-one
- 2-{2-[(2R)-4-chloro-5-fluoro-2-methyl-2,3-dihydro-1H-indol-1-yl]-2-oxoethyl}-6-(morpholin-4-yl)pyrimidin-4(3H)-one
- 2-{2-[(2S)-4-chloro-5-fluoro-2-methyl-2,3-dihydro-1H-indol-1-yl]-2-oxoethyl}-6-(morpholin-4-yl)pyrimidin-4(3H)-one
- 2-{2-[(2R)-2-isopropyl-2,3-dihydroindol-1-yl]-2-oxoethyl}-6-morpholin-4-yl-3H-pyrimidin-4-one
- 2-{2-[(2S)-2-isopropyl-2,3-dihydroindol-1-yl]-2-oxoethyl}-6-morpholin-4-yl-3H-pyrimidin-4-one
- 2-[1-(4-fluoro-2,3-dihydro-1H-indol-1-yl)-1-oxopropan-2-yl]-6-(morpholin-4-yl)pyrimidin-4(3H)-one
- 2-[2-((R)-2-ethyl-2,3-dihydroindol-1-yl)-2-oxoethyl]-6-morpholin-4-yl-3H-pyrimidin-4-one
- 2-[2-((S)-2-ethyl-2,3-dihydroindol-1-yl)-2-oxoethyl]-6-morpholin-4-yl-3H-pyrimidin-4-one
and also the addition salts with inorganic and organic acids or with inorganic and organic bases of said compounds.

6. Compounds having one of the following formulae:
-6-(morpholin-4-yl)-2-[2-oxo-2-(spiro[cyclopropane-1,3'-indol]-1'(2'H)-yl)ethyl]pyrimidin-4(3H)-one
-6-(morpholin-4-yl)-2-[2-oxo-2-(4-phenyl-2,3-dihydro-1H-indol-1-yl)ethyl]pyrimidin-4(3H)-one
-6-(morpholin-4-yl)-2-{2-oxo-2-[4-(trifluoromethoxy)-2,3-dihydro-1H-indol-1-yl]ethyl}pyrimidin-4(3H)-one
-3-methyl-6-(morpholin-4-yl)-2-{2-oxo-2-[4-(trifluoromethyl)-2,3-dihydro-1H-indol-1-yl]ethyl}pyrimidin-4(3H)-one
-6-(morpholin-4-yl)-2-{2-oxo-2-[4-(trifluoromethyl)-2,3-dihydro-1H-indol-1-yl]ethyl}pyrimidin-4(3H)-one
-2-{2-[4-(2-methoxyphenyl)-2,3-dihydro-1H-indol-1-yl]-2-oxoethyl}-6-(morpholin-4-yl)pyrimidin-4(3H)-one
-6-(morpholin-4-yl)-2-{2-oxo-2-[4-(1-propylpiperidin-3-yl)-2,3-dihydro-1H-indol-1-yl]ethyl}pyrimidin-4(3H)-one
-2-{2-[4-(difluoromethoxy)-2,3-dihydro-1H-indol-1-yl]-2-oxoethyl}-3-methyl-6-(morpholin-4-yl)pyrimidin-4(3H)-one
-2-{2-[4-(difluoromethoxy)-2,3-dihydro-1H-indol-1-yl]-2-oxoethyl}-6-(morpholin-4-yl)pyrimidin-4(3H)-one
-6-(morpholin-4-yl)-2-{2-oxo-2-[4-(pyridin-4-yl)-2,3-dihydro-1H-indol-1-yl]ethyl}pyrimidin-4(3H)-one
-2-[2-(1'-methylspiro[indole-3,4'-piperidin]-1(2H)-yl)-2-oxoethyl]-6-(morpholin-4-yl)pyrimidin-4(3H)-one
-6-(morpholin-4-yl)-2-{2-oxo-2-[4-(pyridin-2-yl)-2,3-dihydro-1H-indol-1-yl]ethyl}pyrimidin-4(3H)-one
-6-(morpholin-4-yl)-2-{2-oxo-2-[4-(pyridin-3-yl)-2,3-dihydro-1H-indol-1-yl]ethyl}pyrimidin-4(3H)-one
-2-{2-[4-(2-chlorophenyl)-2,3-dihydro-1H-indol-1-yl]-2-oxoethyl}-6-(morpholin-4-yl)pyrimidin-4(3H)-one
-2-[2-(4-chloro-2,3-dihydro-1H-indol-1-yl)-2-oxoethyl]-3-cyclopropyl-6-(morpholin-4-yl)pyrimidin-4(3H)-one
-6-(morpholin-4-yl)-2-[2-oxo-2-(2,3,3a,8b-tetrahydrocyclopenta[b]indol-4(1H)-yl)ethyl]pyrimidin-4(3H)-one
-2-[2-(4-{[4-(methylsulfonyl)piperazin-1-yl]methyl}-2,3-dihydro-1H-indol-1-yl)-2-oxoethyl]-6-(morpholin-4-yl)pyrimidin-4(3H)-one
-2-(2-{4-[(4-methylpiperazin-1-yl)methyl]-2,3-dihydro-1H-indol-1-yl}-2-oxoethyl)-6-(morpholin-4-yl)pyrimidin-4(3H)-one
-2-{2-[4-(2-fluorophenyl)-2,3-dihydro-1H-indol-1-yl]-2-oxoethyl}-6-(morpholin-4-yl)pyrimidin-4(3H)-one
-3-methyl-2-(2-{4-[(4-methylpiperazin-1-yl)methyl]-2,3-dihydro-1H-indol-1-yl}-2-oxoethyl)-6-(morpholin-4-yl)pyrimidin-4(3H)-one
-6-(morpholin-4-yl)-2-[2-oxo-2-(2',3',5',6'-tetrahydrospiro[indole-3,4'-pyran]-1(2H)-yl)ethyl]pyrimidin-4(3H)-one
-3-methyl-6-(morpholin-4-yl)-2-[2-oxo-2-(2',3',5',6'-tetrahydrospiro[indole-3,4'-pyran]-1(2H)-yl)ethyl]pyrimidin-4(3H)-one
-3-methyl-6-(morpholin-4-yl)-2-[2-oxo-2-(spiro[indole-3,4'-piperidin]-1(2H)-yl)ethyl]pyrimidin-4(3H)-one
-6-(morpholin-4-yl)-2-{2-oxo-2-[(2R)-2-phenyl-2,3-dihydro-1H-indol-1-yl]ethyl}pyrimidin-4(3H)-one
-6-(morpholin-4-yl)-2-{2-oxo-2-[(2S)-2-phenyl-2,3-dihydro-1H-indol-1-yl]ethyl}pyrimidin-4(3H)-one
-2-{2-[4-(4-methylpiperazin-1-yl)-2,3-dihydro-1H-indol-1-yl]-2-oxoethyl}-6-(morpholin-4-yl)pyrimidin-4(3H)-one
-2-{2-[(2R)-2-(4-fluorophenyl)-2,3-dihydro-1H-indol-1-yl]-2-oxoethyl}-6-(morpholin-4-yl)pyrimidin-4(3H)-one
-2-{2-[(2S)-2-(4-fluorophenyl)-2,3-dihydro-1H-indol-1-yl]-2-oxoethyl}-6-(morpholin-4-yl)pyrimidin-4(3H)-one
-2-{2-[(2R)-4-(difluoromethoxy)-2-methyl-2,3-dihydro-1H-indol-1-yl]-2-oxoethyl}-6-(morpholin-4-yl)pyrimidin-4(3H)-one
-2-{2-[(2S)-4-(difluoromethoxy)-2-methyl-2,3-dihydro-1H-indol-1-yl]-2-oxoethyl}-6-(morpholin-4-yl)pyrimidin-4(3H)-one
-2-{2-[(2R)-4-(difluoromethoxy)-2-methyl-2,3-dihydro-1H-indol-1-yl]-2-oxoethyl}-3-methyl-6-(morpholin-4-yl)pyrimidin-4(3H)-one
-2-{2-[(2S)-4-(difluoromethoxy)-2-methyl-2,3-dihydro-1H-indol-1-yl]-2-oxoethyl}-3-methyl-6-(morpholin-4-yl)pyrimidin-4(3H)-one
-6-(morpholin-4-yl)-2-{2-[4-(morpholin-4-yl)-2,3-dihydro-1H-indol-1-yl]-2-oxoethyl}pyrimidin-4(3H)-one
-2-{2-[(2R)-2-cyclopropyl-2,3-dihydro-1H-indol-1-yl]-2-oxoethyl}-6-(morpholin-4-yl)pyrimidin-4(3H)-one
-2-{2-[(2S)-2-cyclopropyl-2,3-dihydro-1H-indol-1-yl]-2-oxoethyl}-6-(morpholin-4-yl)pyrimidin-4(3H)-one
-2-{2-[(2R)-2-methyl-4-(trifluoromethyl)-2,3-dihydro-1H-indol-1-yl]-2-oxoethyl}-6-(morpholin-4-yl)pyrimidin-4(3H)-one
-2-{2-[(2S)-2-methyl-4-(trifluoromethyl)-2,3-dihydro-1H-indol-1-yl]-2-oxoethyl}-6-(morpholin-4-yl)pyrimidin-4(3H)-one
-2-[2-((+)-2-fluoromethyl-2,3-dihydroindol-1-yl)-2-oxoethyl]-6-morpholin-4-yl-3H-pyrimidin-4-one
-2-[2-(2,3-dihydroindol-1-yl)-2-oxoethyl]-6-morpholin-4-yl-3-phenyl-3H-pyrimidin-4-one
and also the addition salts with inorganic and organic acids or with inorganic and organic bases of said compounds.

7. Compounds of formula (Ie): 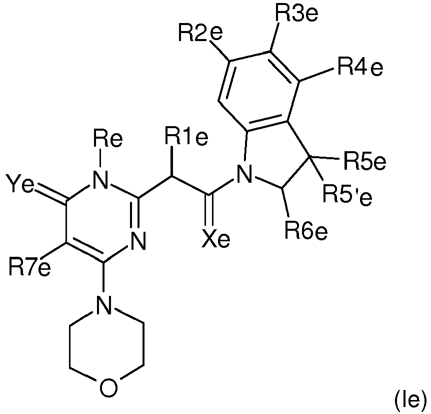 wherein:
Xe and Ye, which may be identical or different, are such that:
Xe is O or S and Ye is S;
Re is a hydrogen atom;
R1 e is a hydrogen atom or a methyl radical;
R2e is a hydrogen atom or a fluorine atom;
R3e is a hydrogen atom or a halogen atom;
R4e is a hydrogen atom, a halogen atom or a hydroxyl, alkyl or alkoxy radical, the alkyl radicals being optionally substituted with one or more radicals, which may be identical or different, selected from halogen atoms and the hydroxyl radical, the alkoxy radicals being optionally substituted with one or more halogen atoms;
R5e and R5'e, which may be identical or different, are a hydrogen atom or an alkyl radical;
R6e is a hydrogen atom or an alkyl radical optionally substituted with one or more radicals, which may be identical or different, selected from halogen atoms and the hydroxyl radical;
R7e is a hydrogen atom;
said compounds of formula (Ie) being in any of the possible racemic, enantiomeric and diastereoisomeric isomer forms, and also the addition salts with inorganic and organic acids or with inorganic and organic bases of said compounds of formula (Ie).

8. The compounds of formula (Ie) as defined in claim 7, corresponding to the following formulae:
-1-((S)-2-methyl-2,3-dihydroindol-1-yl)-2-(4-morpholin-4-yl-6-thioxo-1,6-dihydropyrimidin-2-yl)ethanone
-1-((R)-2-methyl-2,3-dihydroindol-1-yl)-2-(4-morpholin-4-yl-6-thioxo-1,6-dihydropyrimidin-2-yl)ethanone
-2-[2-((S)-2-methyl-2,3-dihydroindol-1-yl)-2-thioxoethyl]-6-morpholin-4-yl-3H-pyrimidine-4-thione
and also the addition salts with inorganic and organic acids or with inorganic and organic bases of said compounds of formula (Ie).

9. Compounds as defined in any one of claims 1 to 8, and also the pharmaceutically acceptable addition salts with inorganic and organic acids or with inorganic and organic bases of said compounds for use as a medicament.

10. Pharmaceutical compositions containing, as active ingredient, at least one of the compounds as defined in any one of claims 1 to 8, or a pharmaceutically acceptable salt of this compound and a pharmaceutically acceptable support.

11. Compounds as defined in any one of claims 1 to 8, for use in the treatment or prevention of a disease selected from the following group: blood vessel proliferation disorders, fibrotic disorders, "mesangial" cell proliferation disorders, metabolic disorders, allergies, asthma, thrombosis, nervous system diseases, retinopathy, psoriasis, rheumatoid arthritis, diabetes, muscle degeneration and cancers.

12. Compounds as defined in any one of claims 1 to 8, for use in the treatment of cancers.

13. Compounds as defined in any one of claims 1 to 8, for use according to claim 12, in the treatment of cancers resistant to cytotoxic agents.

14. Compounds as defined in any one of claims 1 to 8, for use in the treatment of primary tumors and/or of metastases, in particular in gastric, hepatic, renal, ovarian, colon, prostate and lung (NSCLC and SCLC) cancers, glioblastomas, thyroid, bladder and breast cancers, in melanoma, in lymphoid or myeloid hematopoietic tumors, in sarcomas, in brain, larynx and lymphatic system cancers, bone and pancreatic cancers, and in hamartomas.

15. Compounds as defined in claims 1 to 8, for use in cancer chemotherapy alone or in combination.
